(19) Europäisches Patentamt
European Patent Office
Office européen des brevets

(11) EP 4 722 205 A1

(12) EUROPEAN PATENT APPLICATION
published in accordance with Art. 153(4) EPC

(43) Date of publication:
**08.04.2026 Bulletin 2026/15**

(21) Application number: **24815497.3**

(22) Date of filing: **29.05.2024**

(51) International Patent Classification (IPC):
***C07D 401/12*** (2006.01) ***A01N 25/00*** (2006.01)
***A01N 37/24*** (2006.01) ***A01N 37/32*** (2006.01)
***A01N 37/38*** (2006.01) ***A01N 37/46*** (2006.01)
***A01N 37/50*** (2006.01) ***A01N 43/32*** (2006.01)
***A01N 43/36*** (2006.01) ***A01N 43/40*** (2006.01)
***A01N 43/50*** (2006.01) ***A01N 43/52*** (2006.01)
***A01N 43/54*** (2006.01) ***A01N 43/56*** (2006.01)
***A01N 43/76*** (2006.01) ***A01N 43/78*** (2006.01)
***A01N 43/80*** (2006.01) ***A01N 43/88*** (2006.01)
***A01N 43/90*** (2006.01) ***A01N 43/653*** (2006.01)
***A01N 43/713*** (2006.01)

(52) Cooperative Patent Classification (CPC):
**A01N 25/00; A01N 37/24; A01N 37/32; A01N 37/38; A01N 37/46; A01N 37/50; A01N 43/32; A01N 43/36; A01N 43/40; A01N 43/50; A01N 43/52; A01N 43/54; A01N 43/56; A01N 43/653; A01N 43/713;** (Cont.)

(86) International application number:
**PCT/JP2024/019624**

(87) International publication number:
**WO 2024/248015 (05.12.2024 Gazette 2024/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.05.2023 JP 2023088493**
**26.12.2023 JP 2023218891**

(71) Applicant: **Sumitomo Chemical Company, Limited
Tokyo 103-6020 (JP)**

(72) Inventors:
- **NISHIKAWA, Bunta
Takarazuka-shi, Hyogo 665-8555 (JP)**
- **MAEHATA, Ryota
Takarazuka-shi, Hyogo 665-8555 (JP)**
- **MINEGISHI, Hidemitsu
Takarazuka-shi, Hyogo 665-8555 (JP)**
- **SHINOMIYA, Hiroto
Takarazuka-shi, Hyogo 665-8555 (JP)**

(74) Representative: **Vossius & Partner
Patentanwälte Rechtsanwälte mbB
Siebertstraße 3
81675 München (DE)**

# (54) HETEROCYCLIC COMPOUND AND NOXIOUS ARTHROPOD-CONTROLLING COMPOSITION CONTAINING SAME

(57) The present invention provides a compound having an excellent control efficacy against harmful arthropods. A compound represented by formula (I) [in the formula (I), the symbols have the same meanings described in the specification] or N-oxide thereof has an excellent control efficacy against harmful arthropods.

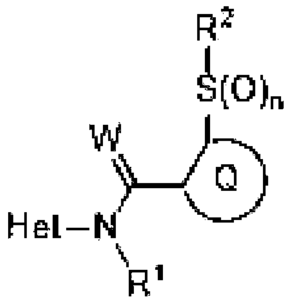

(I)

EP 4 722 205 A1

(52) Cooperative Patent Classification (CPC): (Cont.)
**A01N 43/76; A01N 43/78; A01N 43/80;
A01N 43/82; A01N 43/88; A01N 43/90;
A01N 47/02; A01N 47/04; A01N 47/14;
A01N 47/18; A01N 47/22; A01N 47/24;
A01N 47/26; A01N 47/40; A01N 51/00;
A01N 53/00; A01N 55/00; A01N 57/14;
A01N 59/16; A01N 63/20; A01N 65/12; A01P 7/00;
A01P 7/02; A01P 7/04; A61K 31/437;
A61K 31/4439; A61K 45/00; A61P 33/14;
C07D 401/12; C07D 413/12; C07D 417/12;
C07D 471/04; C07D 519/00**

## Description

TECHNICAL FIELD

This application claims priority to and the benefit of Japanese Patent Application No. 2023-088493 filed May 30, 2023, and Japanese Patent Application No. 2023-218891 filed December 26, 2023, the entire contents of which are incorporated herein by reference.

The present invention is related to a heterocyclic compound and a composition for controlling harmful arthropods comprising the same.

BACKGROUND ART

To date, in order to control harmful arthropods, some compounds have been studied. For example, a certain class of compound has been known to have an effect on controlling pests (see Patent Documents 1 and 2).

CITATION LIST

PATENT DOCUMENT

Patent Document 1: WO 2013/191041
Patent Document 2: WO 2015/117912

SUMMARY OF THE INVENTION

(PROBLEMS TO BE SOLVED BY INVENTION)

An object of the present invention is to provide a compound having an excellent efficacy for controlling harmful arthropods.

(MEANS TO SOLVE PROBLEMS)

The present compound encompasses the followings.

[1] A compound represented by formula (I):

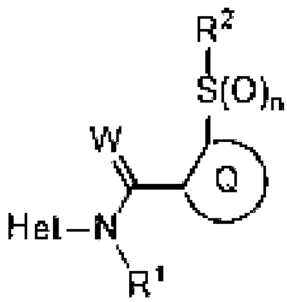

(I)

[wherein

Q represented by the following formula:

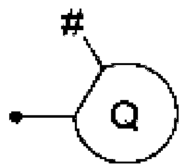

represents a group represented by formula Q1, a group represented by formula Q2, a group represented by formula Q3, a group represented by formula Q4, a group represented by formula Q5, a group represented by formula Q6, or a group represented by formula Q7 (# represents a binding site to a sulfur atom, and • represents a binding site to a carbon atom to which W is attached),

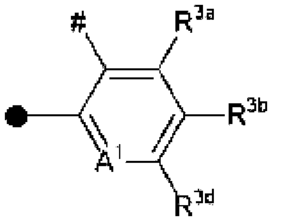

Q1

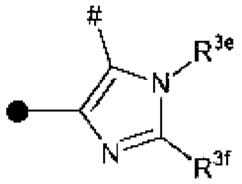

Q2

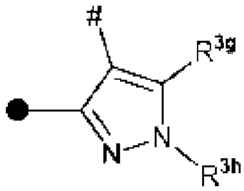

Q3

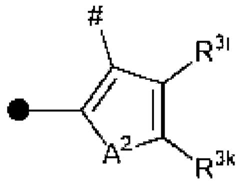

Q4

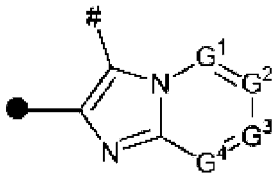

Q5

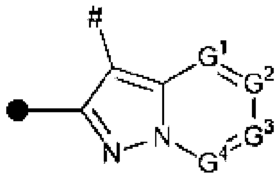

Q6

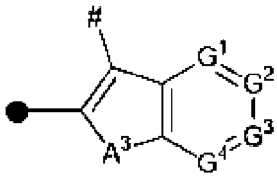

Q7

$A^1$ represents a nitrogen atom or $CR^{3c}$,
$A^2$ represents an oxygen atom, a sulfur atom or $NR^6$,
$A^3$ represents an oxygen atom or a sulfur atom,
$G^1$ represents a nitrogen atom or $CR^{10a}$,
$G^2$ represents a nitrogen atom or $CR^{10b}$,
$G^3$ represents a nitrogen atom or $CR^{10c}$,
$G^4$ represents a nitrogen atom or $CR^{10d}$,
$R^{3a}$, $R^{3b}$, $R^{3c}$, $R^{3d}$, $R^{3f}$, $R^{3g}$, $R^{3i}$, $R^{3k}$, $R^{10a}$, $R^{10b}$, $R^{10c}$ and $R^{10d}$ are identical to or different from each other and each represents a C1-C6 chain hydrocarbon group which may be optionally substituted with one or more substituents selected from Group B, a C3-C7 cycloalkyl group which may be optionally substituted with one or more substituents selected from Group K, a phenyl group which may be optionally substituted with one or more substituents selected from Group M, a five(5) or six(6) membered aromatic heterocyclic group which may be optionally substituted with one or more substituents selected from Group M, $OR^{12}$ , $NR^{11}R^{12}$ , $NR^{11a}R^{12a}$, $NR^{19}NR^{11}R^{12}$, $NR^{19}OR^{11}$, $NR^{11}C(O)R^{13}$, $NR^{19}NR^{11}C(O)R^{13}$, $NR^{11}C(O)OR^{14}$, $NR^{19}NR^{11}C(O)OR^{14}$, $NR^{11}C(O)NR^{21}R^{22}$, $NR^{19}NR^{11}C(O)NR^{21}R^{22}$, $N=CHNR^{21}R^{22}$, $N=S(O)_pR^{15}R^{16}$, $C(O)R^{13}$, $C(O)OR^{17}$, $C(O)NR^{21}R^{22}$, $C(O)NR^{11}S(O)_2R^{18}$, $CR^{20}=NOR^{17}$, $NR^{11}CR^{19}=NOR^{17}$, $S(O)_mR^{18}$, a cyano group, a nitro group, a hydrogen atom or a halogen atom (with the proviso that when Q represents a group represented by formula Q1 and $A^1$ represents a $CR^{3c}$, all of the existing $R^{3a}$, $R^{3b}$, $R^{3c}$ and $R^{3d}$ don't represent a hydrogen atom),
$R^{3e}$ and $R^{3h}$ are identical to or different from each other and each represents a C1-C6 alkyl group which may be optionally substituted with one or more halogen atoms, or a phenyl group which may be optionally substituted with one or more substituents selected from Group M,
when Q represents a group represented by formula Q1, neighboring two substituents among $R^{3a}$, $R^{3b}$ and $R^{3d}$ may combine with two carbon atoms to which they are attached to form a benzene ring, a pyrrole ring, a furan ring, a thiophene ring, a pyrazole ring, an imidazole ring, an oxazole ring, an isoxazole ring, a thiazole ring, an isothiazole ring, an oxadiazole ring, a thiadiazole ring, a pyridine ring, a pyridazine ring, a pyrimidine ring, a pyrazine ring {the benzene ring, the pyrrole ring, the furan ring, the thiophene ring, the pyrazole ring, the imidazole ring, the oxazole ring, the isoxazole ring, the thiazole ring, the isothiazole ring, the pyridine ring, the pyridazine ring, the pyrimidine ring, and the pyrazine ring may be optionally substituted with one or more substituents selected from Group M}, or a triazole ring which may be optionally substituted with one or more substituents selected from Group P,
p is 0 or 1,
m is 0, 1 or 2,
$R^{20}$ represents a C1-C6 chain hydrocarbon group which may be optionally substituted with one or more halogen

atoms, a halogen atom, $OR^{23}$, $NR^{24}R^{25}$, or a hydrogen atom,

$R^{17}$ represents a C1-C6 chain hydrocarbon group which may be optionally substituted with one or more halogen atoms, a phenyl group which may be optionally substituted with one or more substituents selected from Group J, or a hydrogen atom,

$R^{12}$ represents a C1-C6 chain hydrocarbon group which may be optionally substituted with one or more substituents selected from Group L, a C3-C7 cycloalkyl group which may be optionally substituted with one or more substituents selected from Group T, a C3-C7 cycloalkenyl group which may be optionally substituted with one or more substituents selected from Group T, a phenyl group which may be optionally substituted with one or more substituents selected from Group J, a six(6) membered aromatic heterocyclic group which may be optionally substituted with one or more substituents selected from Group J, a hydrogen atom, or $S(O)_2R^{18}$,

$R^{18}$ represents a C1-C6 chain hydrocarbon group which may be optionally substituted with one or more halogen atoms, or a phenyl group which may be optionally substituted with one or more substituents selected from Group J,

$R^{11a}$ and $R^{12a}$ combine with a nitrogen atom to which they are attached to form a three(3) to seven(7) membered non-aromatic heterocyclic group which may be optionally substituted with one or more substituents selected from Group K,

$R^{13}$ represents a C1-C6 chain hydrocarbon group which may be optionally substituted with one or more halogen atoms, a C3-C7 cycloalkyl group which may be optionally substituted with one or more halogen atoms, a (C3-C6 cycloalkyl) C1-C3 alkyl group which may be optionally substituted with one or more halogen atoms, a phenyl group which may be optionally substituted with one or more substituents selected from Group J, a five(5) or six(6) membered aromatic heterocyclic group which may be optionally substituted with one or more substituents selected from Group J, or a hydrogen atom,

$R^{14}$ represents a C1-C6 chain hydrocarbon group which may be optionally substituted with one or more halogen atoms, a C3-C7 cycloalkyl group which may be optionally substituted with one or more halogen atoms, a (C3-C6 cycloalkyl) C1-C3 alkyl group which may be optionally substituted with one or more halogen atoms, or a phenyl C1-C3 alkyl group {the phenyl moiety in the phenyl C1-C3 alkyl group may be optionally substituted with one or more substituents selected from Group J},

$R^{15}$ and $R^{16}$ are identical to or different from each other and each represents a C1-C6 alkyl group which may be optionally substituted with one or more halogen atoms,

$R^{21}$ represents a C1-C6 alkyl group which may be optionally substituted with one or more halogen atoms, or a hydrogen atom,

$R^{22}$ represents a C1-C6 chain hydrocarbon group which may be optionally substituted with one or more substituents selected from Group L, a C3-C7 cycloalkyl group which may be optionally substituted with one or more substituents selected from Group T, or a hydrogen atom,

$R^{23}$ represents a C1-C6 chain hydrocarbon group which may be optionally substituted with one or more halogen atoms,

$R^{11}$, $R^{19}$, $R^{24}$, and $R^{25}$ are identical to or different from each other and each represents a C1-C6 chain hydrocarbon group which may be optionally substituted with one or more halogen atoms, or a hydrogen atom,

W is an oxygen atom or a sulfur atom,

$R^1$ represents a C1-C6 chain hydrocarbon group which may be optionally substituted with one or more substituents selected from Group D, a C3-C7 cycloalkyl group which may be optionally substituted with one or more substituents selected from Group E, $C(O)R^4$, $C(O)OR^4$, or $C(O)NR^4R^5$,

$R^4$ and $R^5$ are identical to or different from each other and each represents a C1-C6 chain hydrocarbon group which may be optionally substituted with one or more halogen atoms, a phenyl group which may be optionally substituted with one or more substituents selected from Group F, a five(5) or six(6) membered heterocyclic group which may be optionally substituted with one or more substituents selected from Group F, or a hydrogen atom,

$R^2$ represents a C1-C6 alkyl group which may be optionally substituted with one or more halogen atoms, a cyclopropyl group, or a cyclopropylmethyl group,

n is 0, 1 or 2,

Het represents a group represented by formula Het1 , a group represented by formula Het2, a group represented by formula Het3, a group represented by formula Het4, a group represented by formula Het5, or a group represented by formula Het6,

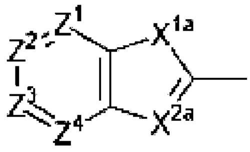

Het1

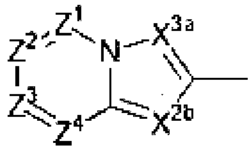

Het2

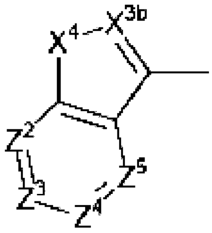

Het3

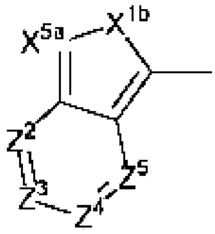

Het4

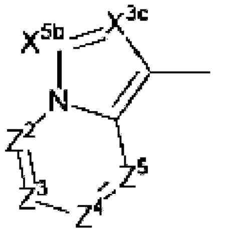

Het5

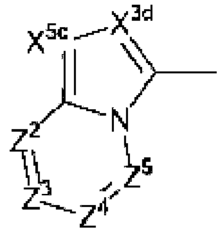

Het6

$X^{1a}$ and $X^{1b}$ are identical to or different from each other and each represents $NR^{7a}$, an oxygen atom or a sulfur atom,

$X^4$ represents $NR^{7b}$, an oxygen atom or a sulfur atom,

$X^{2a}$ and $X^{2b}$ are identical to or different from each other and each represents a nitrogen atom or $CR^{8a}$,

$X^{3a}$, $X^{3b}$, $X^{3c}$ and $X^{3d}$ are identical to or different from each other and each represents a nitrogen atom or $CR^{8b}$,

$X^{5a}$, $X^{5b}$ and $X^{5c}$ are identical to or different from each other and each represents a nitrogen atom or $CR^{8c}$,

$R^{7a}$ and $R^{7b}$ are identical to or different from each other and each represents a C1-C6 chain hydrocarbon group which may be optionally substituted with one or more halogen atoms, a C1-C6 alkoxy group which may be optionally substituted with one or more halogen atoms, a C3-C7 cycloalkyl group which may be optionally substituted with one or more halogen atoms, a (C3-C7 cycloalkyl) C1-C6 alkyl group which may be optionally substituted with one or more halogen atoms, or a hydrogen atom,

$R^{8a}$, $R^{8b}$ and $R^{8c}$ are identical to or different from each other and each represents a C1-C6 chain hydrocarbon group which may be optionally substituted with one or more substituents selected from Group $B^2$, a C3-C7 cycloalkyl group which may be optionally substituted with one or more substituents selected from Group $K^2$, a phenyl group which may be optionally substituted with one or more substituents selected from Group $M^2$, a five(5) or six(6) membered aromatic heterocyclic group which may be optionally substituted with one or more substituents selected from Group $M^2$, $OR^{32}$, $NR^{31}R^{32}$, $NR^{31a}R^{32a}$, $NR^{39}NR^{31}R^{32}$, $NR^{39}OR^{31}$, $NR^{31}C(O)R^{33}$, $NR^{39}NR^{31}C(O)R^{33}$, $NR^{31}C(O)OR^{34}$, $NR^{39}NR^{31}C(O)OR^{34}$, $NR^{31}C(O)NR^{41}R^{42}$, $NR^{39}NR^{31}C(O)NR^{41}R^{42}$, $N=CHNR^{41}R^{42}$, $N=S(O)_kR^{35}R^{36}$, $C(O)R^{33}$, $C(O)OR^{37}$, $C(O)NR^{41}R^{42}$, $C(O)NR^{31}S(O)_2R^{38}$, $CR^{40}=NOR^{37}$, $NR^{31}CR^{39}=NOR^{37}$, $S(O)_tR^{38}$, a cyano group, a nitro group, a hydrogen atom, or a halogen atom,

k is 0 or 1,

t is 0, 1 or 2,

$R^{40}$ represents a C1-C6 chain hydrocarbon group which may be optionally substituted with one or more halogen atoms, a halogen atom, $OR^{43}$, $NR^{44}R^{45}$, or a hydrogen atom,

$R^{37}$ represents a C1-C6 chain hydrocarbon group which may be optionally substituted with one or more halogen atoms, a phenyl group which may be optionally substituted with one or more substituents selected from Group $J^2$, or a hydrogen atom,

$R^{32}$ represents a C1-C6 chain hydrocarbon group which may be optionally substituted with one or more substituents selected from Group $L^2$, a C3-C7 cycloalkyl group which may be optionally substituted with one or more substituents selected from Group $T^2$, a C3-C7 cycloalkenyl group which may be optionally substituted with one or more substituents selected from Group $T^2$, a phenyl group which may be optionally substituted with one or more substituents selected from Group $J^2$, a six(6) membered aromatic heterocyclic group which may be optionally substituted with one or more substituents selected from Group $J^2$, a hydrogen atom, or $S(O)_2R^{38}$,

$R^{38}$ represents a C1-C6 chain hydrocarbon group which may be optionally substituted with one or more halogen atoms, or a phenyl group which may be optionally substituted with one or more substituents selected from Group $J^2$,

$R^{31a}$ and $R^{32a}$ combine with a nitrogen atom to which they are attached to form a three(3) to seven(7) membered non-aromatic heterocyclic group which may be optionally substituted with one or more substituents selected from

Group $K^2$,
$R^{33}$ represents a C1-C6 chain hydrocarbon group which may be optionally substituted with one or more halogen atoms, a C3-C7 cycloalkyl group which may be optionally substituted with one or more halogen atoms, a (C3-C6 cycloalkyl) C1-C3 alkyl group which may be optionally substituted with one or more halogen atoms, a phenyl group which may be optionally substituted with one or more substituents selected from Group $J^2$, a five(5) or six(6) membered aromatic heterocyclic group which may be optionally substituted with one or more substituents selected from Group $J^2$, or a hydrogen atom,
$R^{34}$ represents a C1-C6 chain hydrocarbon group which may be optionally substituted with one or more halogen atoms, a C3-C7 cycloalkyl group which may be optionally substituted with one or more halogen atoms, a (C3-C6 cycloalkyl) C1-C3 alkyl group which may be optionally substituted with one or more halogen atoms, or a phenyl C1-C3 alkyl group {the phenyl moiety in the phenyl C1-C3 alkyl group may be optionally substituted with one or more substituents selected from Group $J^2$},
$R^{35}$ and $R^{36}$ are identical to or different from each other and each represents a C1-C6 alkyl group which may be optionally substituted with one or more halogen atoms,
$R^{41}$ represents a C1-C6 alkyl group which may be optionally substituted with one or more halogen atoms, or a hydrogen atom,
$R^{42}$ represents a C1-C6 chain hydrocarbon group which may be optionally substituted with one or more substituents selected from Group $L^2$, a C3-C7 cycloalkyl group which may be optionally substituted with one or more substituents selected from Group $T^2$, or a hydrogen atom,
$R^{43}$ represents a C1-C6 chain hydrocarbon group which may be optionally substituted with one or more halogen atoms,
$R^{31}$, $R^{39}$, $R^{44}$ and $R^{45}$ are identical to or different from each other and each represents a C1-C6 chain hydrocarbon group which may be optionally substituted with one or more halogen atoms, or a hydrogen atom,
$Z^1$ represents a nitrogen atom or $CR^{9a}$,
$Z^2$ represents a nitrogen atom or $CR^{9b}$,
$Z^3$ represents a nitrogen atom or $CR^{9c}$,
$Z^4$ represents a nitrogen atom or $CR^{9d}$,
$Z^5$ represents a nitrogen atom or $CR^{9e}$,
$R^{9a}$, $R^{9d}$ and $R^{9e}$ are identical to or different from each other and each represents a C1-C6 chain hydrocarbon group which may be optionally substituted with one or more substituents selected from Group $B^3$, a C3-C7 cycloalkyl group which may be optionally substituted with one or more substituents selected from Group $K^3$, a phenyl group which may be optionally substituted with one or more substituents selected from Group $M^3$, a five(5) or six(6) membered aromatic heterocyclic group which may be optionally substituted with one or more substituents selected from Group $M^3$, $OR^{52}$, $NR^{51}R^{52}$, $NR^{51a}R^{52a}$, $NR^{59}NR^{51}R^{52}$, $NR^{59}OR^{51}$, $NR^{51}C(O)R^{53}$, $NR^{59}NR^{51}C(O)R^{53}$, $NR^{51}C(O)OR^{54}$, $NR^{59}NR^{51}C(O)OR^{54}$, $NR^{51}C(O)NR^{51}R^{52}$, $NR^{59}NR^{51}C(O)NR^{61}R^{62}$, $N=CHNR^{61}R^{62}$, $N=S(O)_qR^{55}R^{56}$, $C(O)R^{53}$, $C(O)OR^{57}$, $C(O)NR^{61}R^{62}$, $C(O)NR^{51}S(O)_2R^{58}$, $CR^{60}=NOR^{57}$, $NR^{51}CR^{59}=NOR^{57}$, $S(O)_vR^{58}$, a cyano group, a nitro group, a hydrogen atom, or a halogen atom,
q is 0 or 1,
v is 0, 1 or 2,
$R^{9b}$ and $R^{9c}$ are identical to or different from each other and each represents a C1-C6 chain hydrocarbon group which may be optionally substituted with one or more substituents selected from Group $B^3$, a C3-C7 cycloalkyl group which may be optionally substituted with one or more substituents selected from Group $K^3$, a phenyl group which may be optionally substituted with one or more substituents selected from Group $M^3$, a five(5) or six(6) membered aromatic heterocyclic group which may be optionally substituted with one or more substituents selected from Group $M^3$, $OR^{72}$, $NR^{71}R^{72}$, $NR^{71a}R^{72a}$, $NR^{79}NR^{71}R^{72}$, $NR^{79}OR^{71}$, $NR^{71}C(O)R^{73}$, $NR^{79}NR^{71}C(O)R^{73}$, $NR^{71}C(O)OR^{74}$, $NR^{79}NR^{71}C(O)OR^{74}$, $NR^{71}C(O)NR^{71}R^{72}$, $NR^{79}NR^{71}C(O)NR^{81}R^{82}$, $N=CHNR^{81}R^{82}$, $N=S(O)_uR^{75}R^{76}$, $C(O)R^{73}$, $C(O)OR^{77}$, $C(O)NR^{81}R^{82}$, $C(O)NR^{71}S(O)_2R^{78}$, $CR^{80}=NOR^{77}$, $NR^{71}CR^{79}=NOR^{77}$, $S(O)_wR^{78}$, a cyano group, a nitro group, a hydrogen atom, or a halogen atom,
u is 0 or 1,
w is 0, 1, or 2,
$R^{60}$ and $R^{80}$ are identical to or different from each other and each represents a C1-C6 chain hydrocarbon group which may be optionally substituted with one or more halogen atoms, a halogen atom, $OR^{63}$, $NR^{64}R^{65}$, or a hydrogen atom,
$R^{57}$ and $R^{77}$ are identical to or different from each other and each represents a C1-C6 chain hydrocarbon group which may be optionally substituted with one or more halogen atoms, a phenyl group which may be optionally substituted with one or more substituents selected from Group $J^3$, or a hydrogen atom,
$R^{52}$ represents a C1-C6 chain hydrocarbon group which may be optionally substituted with one or more substituents selected from Group $L^3$, a C3-C7 cycloalkyl group which may be optionally substituted with one

or more substituents selected from Group $T^3$, a C3-C7 cycloalkenyl group which may be optionally substituted with one or more substituents selected from Group $T^3$, a phenyl group which may be optionally substituted with one or more substituents selected from Group $J^3$, a six(6) membered aromatic heterocyclic group which may be optionally substituted with one or more substituents selected from Group $J^3$, or $S(O)_2R^{58}$,

$R^{72}$ represents a C1-C6 chain hydrocarbon group which may be optionally substituted with one or more substituents selected from Group $L^3$, a C3-C7 cycloalkyl group which may be optionally substituted with one or more substituents selected from Group $T^3$, a C3-C7 cycloalkenyl group which may be optionally substituted with one or more substituents selected from Group $T^3$, a hydrogen atom, or $S(O)_2R^{78}$,

$R^{58}$ represents a C1-C6 chain hydrocarbon group which may be optionally substituted with one or more halogen atoms, or a phenyl group which may be optionally substituted with one or more substituents selected from Group $J^3$,

$R^{78}$ represents a C1-C6 chain hydrocarbon group which may be optionally substituted with one or more halogen atoms,

$R^{51a}$ and $R^{52a}$ combine with a nitrogen atom to which they are attached to form a three(3) to seven(7) membered non-aromatic heterocyclic group which may be optionally substituted with one or more substituents selected from Group $K^3$,

$R^{71a}$ and $R^{72a}$ combine with a nitrogen atom to which they are attached to form a three(3) to seven(7) membered non-aromatic heterocyclic group which may be optionally substituted with one or more substituents selected from Group $K^3$,

$R^{53}$ and $R^{73}$ are identical to or different from each other and each represents a C1-C6 chain hydrocarbon group which may be optionally substituted with one or more halogen atoms, a C3-C7 cycloalkyl group which may be optionally substituted with one or more halogen atoms, a (C3-C6 cycloalkyl) C1-C3 alkyl group which may be optionally substituted with one or more halogen atoms, a phenyl group which may be optionally substituted with one or more substituents selected from Group $J^3$, a five(5) or six(6) membered aromatic heterocyclic group which may be optionally substituted with one or more substituents selected from Group $J^3$, or a hydrogen atom,

$R^{54}$ and $R^{74}$ are identical to or different from each other and each represents a C1-C6 chain hydrocarbon group which may be optionally substituted with one or more halogen atoms, a C3-C7 cycloalkyl group which may be optionally substituted with one or more halogen atoms, a (C3-C6 cycloalkyl) C1-C3 alkyl group which may be optionally substituted with one or more halogen atoms, or a phenyl C1-C3 alkyl group {the phenyl moiety in the phenyl C1-C3 alkyl group may be optionally substituted with one or more substituents selected from Group $J^3$},

$R^{55}$, $R^{56}$, $R^{75}$ and $R^{76}$ are identical to or different from each other and each represents a C1-C6 alkyl group which may be optionally substituted with one or more halogen atoms,

$R^{61}$ and $R^{81}$ are identical to or different from each other and each represents a C1-C6 alkyl group which may be optionally substituted with one or more halogen atoms, or a hydrogen atom,

$R^{62}$ and $R^{82}$ are identical to or different from each other and each represents a C1-C6 chain hydrocarbon group which may be optionally substituted with one or more substituents selected from Group $L^3$, a C3-C7 cycloalkyl group which may be optionally substituted with one or more substituents selected from Group $T^3$, or a hydrogen atom,

$R^{63}$ represents a C1-C6 chain hydrocarbon group which may be optionally substituted with one or more halogen atoms,

$R^{51}$, $R^{59}$, $R^{64}$, $R^{65}$, $R^{71}$, and $R^{79}$ are identical to or different from each other and each represents a C1-C6 chain hydrocarbon group which may be optionally substituted with one or more halogen atoms, or a hydrogen atom,

Group D: a group consisting of a C3-C6 cycloalkyl group which may be optionally substituted with one or more substituents selected from halogen atom and C1-C3 alkyl group, a C1-C6 alkoxy group which may be optionally substituted with one or more halogen atoms, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, a C2-C6 alkylcarbonyl group which may be optionally substituted with one or more halogen atoms, a C2-C6 alkoxycarbonyl group which may be optionally substituted with one or more halogen atoms, a phenyl group which may be optionally substituted with one or more substituents selected from Group F, a five(5) or six(6) membered aromatic heterocyclic group which may be optionally substituted with one or more substituents selected from Group F, a hydroxy group, a cyano group and a halogen atom,

Group E: a group consisting of a C1-C6 alkyl group which may be optionally substituted with one or more halogen atoms, a C1-C6 alkoxy group which may be optionally substituted with one or more halogen atoms, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, a cyano group, and a halogen atom,

Group F: a group consisting of a C1-C6 alkyl group which may be optionally substituted with one or more halogen

atoms, a C1-C6 alkoxy group which may be optionally substituted with one or more halogen atoms, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, a C1-C6 alkylamino group which may be optionally substituted with one or more halogen atoms, di(C1-C6 alkyl which may be optionally substituted with one or more halogen atoms) amino group, a C2-C6 alkylcarbonyl group which may be optionally substituted with one or more halogen atoms, a C2-C6 alkoxycarbonyl group which may be optionally substituted with one or more halogen atoms, a hydroxy group, a sulfanyl group, an amino group, a cyano group, a nitro group, and a halogen atom,

Group B: a group consisting of a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, a C3-C6 cycloalkyl group which may be optionally substituted with one or more halogen atoms, a cyano group, hydroxy group, and a halogen atom,

Group G: a group consisting of a C1-C6 chain hydrocarbon group which may be optionally substituted with one or more halogen atoms, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, and a halogen atom,

Group J: a group consisting of a C1-C6 chain hydrocarbon group which may be optionally substituted with one or more halogen atoms, a hydroxy group, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a sulfanyl group, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, an amino group, $NHR^{91}$, $NR^{91}R^{92}$, $C(O)R^{91}$, $OC(O)R^{91}$, $C(O)OR^{91}$, a cyano group, a nitro group, and a halogen atom,

$R^{91}$ and $R^{92}$ are identical to or different from each other and each represents a C1-C6 alkyl group which may be optionally substituted with one or more halogen atoms,

Group K: a group consisting of a C1-C6 chain hydrocarbon group which may be optionally substituted with one or more halogen atoms, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a halogen atom, an oxo group, a hydroxy group, a cyano group, and a nitro group,

Group L: a group consisting of a C1-C6 alkoxy group which may be optionally substituted with one or more halogen atoms, a phenyl group which may be optionally substituted with one or more substituents selected from Group J, a five(5) or six(6) membered aromatic heterocyclic group which may be optionally substituted with one or more substituents selected from Group J, a C3-C7 cycloalkyl group which may be optionally substituted with one or more halogen atoms, a three(3) to seven(7) membered non-aromatic heterocyclic group which may be optionally substituted with one or more substituents selected from Group G, an amino group, $NHR^{91}$, $NR^{91}R^{92}$, a halogen atom, and a cyano group,

Group M: a group consisting of a C1-C6 alkyl group which may be optionally substituted with one or more halogen atoms, $OR^{94}$, $NR^{93}R^{94}$, $C(O)R^{94}$, $C(O)NR^{93}R^{94}$, $OC(O)R^{93}$, $OC(O)OR^{93}$, $NR^{94}C(O)R^{93}$, $NR^{94}C(O)OR^{93}$, $C(O)OR^{94}$, a halogen atom, a nitro group, a cyano group, an amino group, and five(5) or six(6) membered aromatic heterocyclic group,

$R^{93}$ represents a C1-C6 alkyl group which may be optionally substituted with one or more halogen atoms, or a C3-C6 cycloalkyl group which may be optionally substituted with one or more halogen atoms,

$R^{94}$ represents a C1-C6 alkyl group which may be optionally substituted with one or more halogen atoms, a C3-C6 cycloalkyl group which may be optionally substituted with one or more halogen atoms, or a hydrogen atom,

Group P: a group consisting of a C2-C6 chain hydrocarbon group which may be optionally substituted with one or more halogen atoms, a C3-C6 cycloalkyl group which may be optionally substituted with one or more halogen atoms, a phenyl group which may be optionally substituted with one or more substituents selected from Group J, a five(5) or six(6) membered aromatic heterocyclic group which may be optionally substituted with one or more substituents selected from Group J, a C2-C6 alkylcarbonyl group which may be optionally substituted with one or more halogen atoms, a C2-C6 alkoxycarbonyl group which may be optionally substituted with one or more halogen atoms, an aminocarbonyl group, a (C1-C6 alkyl) aminocarbonyl group which may be optionally substituted with one or more halogen atoms, a methyl group, and a di(C1-C4 alkyl) aminocarbonyl group which may be optionally substituted with one or more halogen atoms,

Group T: a group consisting of a C1-C6 alkyl group which may be optionally substituted with one or more halogen atoms, a halogen atom, and a cyano group,

Group $B^2$: a group consisting of a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, a C3-C6 cycloalkyl group which may be optionally substituted with one or more halogen atoms, a cyano group, a hydroxy group, and a halogen atom,

Group $G^2$: a group consisting of a C1-C6 chain hydrocarbon group which may be optionally substituted with one or more halogen atoms, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, and a halogen atom,

Group $J^2$: a group consisting of a C1-C6 chain hydrocarbon group which may be optionally substituted with one or more halogen atoms, a hydroxy group, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a sulfanyl group, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, an amino group, $NHR^{95}$, $NR^{95}R^{96}$, $C(O)R^{95}$, $OC(O)R^{95}$, $C(O)OR^{95}$, a cyano group, a nitro group, and a halogen atom,

$R^{95}$ and $R^{96}$ are identical to or different from each other and each represents a C1-C6 alkyl group which may be optionally substituted with one or more halogen atoms,

Group $K^2$: a group consisting of a C1-C6 chain hydrocarbon group which may be optionally substituted with one or more halogen atoms, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a halogen atom, an oxo group, a hydroxy group, a cyano group, and a nitro group,

Group $L^2$: a group consisting of a C1-C6 alkoxy group which may be optionally substituted with one or more halogen atoms, a phenyl group which may be optionally substituted with one or more substituents selected from Group $J^2$, a five(5) or six(6) membered aromatic heterocyclic group which may be optionally substituted with one or more substituents selected from Group $J^2$, a C3-C7 cycloalkyl group which may be optionally substituted with one or more halogen atoms, a three(3) to seven(7) membered non-aromatic heterocyclic group which may be optionally substituted with one or more substituents selected from Group $G^2$, an amino group, $NHR^{95}$, $NR^{95}R^{96}$, a halogen atom, and a cyano group,

Group $M^2$: a group consisting of a C1-C6 alkyl group which may be optionally substituted with one or more halogen atoms, $OR^{98}$, $NR^{97}R^{98}$, $C(O)R^{98}$, $C(O)NR^{97}R^{98}$, $OC(O)R^{97}$, $OC(O)OR^{97}$, $NR^{98}C(O)R^{97}$, $NR^{98}C(O)OR^{97}$, $C(O)OR^{98}$, a halogen atom, a nitro group, a cyano group, an amino group, and a five(5) or six (6) membered aromatic heterocyclic group,

$R^{97}$ represents a C1-C6 alkyl group which may be optionally substituted with one or more halogen atoms, or a C3-C6 cycloalkyl group which may be optionally substituted with one or more halogen atoms,

$R^{98}$ represents a C1-C6 alkyl group which may be optionally substituted with one or more halogen atoms, a C3-C6 cycloalkyl group which may be optionally substituted with one or more halogen atoms, or a hydrogen atom,

Group $T^2$: a group consisting of a C1-C6 alkyl group which may be optionally substituted with one or more halogen atoms, a halogen atom, and a cyano group,

Group $B^3$: a group consisting of a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, a C3-C6 cycloalkyl group which may be optionally substituted with one or more halogen atoms, a cyano group, a hydroxy group, and a halogen atom,

Group $G^3$: a group consisting of a C1-C6 chain hydrocarbon group which may be optionally substituted with one or more halogen atoms, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, and a halogen atom,

Group $J^3$: a group consisting of a C1-C6 chain hydrocarbon group which may be optionally substituted with one or more halogen atoms, a hydroxy group, a C1-C6 alkoxy group which may be optionally substituted with one or

more halogen atoms, a C3-C6 alkenyloxy group which may be optionally substituted with one or more halogen atoms, a C3-C6 alkynyloxy group which may be optionally substituted with one or more halogen atoms, a sulfanyl group, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, an amino group, $NHR^{99}$, $NR^{99}R^{100}$, $C(O)R^{99}$, $OC(O)R^{99}$, $C(O)OR^{99}$, a cyano group, a nitro group, and a halogen atom,

$R^{99}$ and $R^{100}$ are identical to or different from each other and each represents a C1-C6 alkyl group which may be optionally substituted with one or more halogen atoms,

Group $K^3$: a group consisting of a C1-C6 chain hydrocarbon group which may be optionally substituted with one or more halogen atoms, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a halogen atom, an oxo group, a hydroxy group, a cyano group, and a nitro group,

Group $L^3$: a group consisting of a C1-C6 alkoxy group which may be optionally substituted with one or more halogen atoms, a phenyl group which may be optionally substituted with one or more substituents selected from Group $J^3$, a five(5) or six(6) membered aromatic heterocyclic group which may be optionally substituted with one or more substituents selected from Group $J^3$, a C3-C7 cycloalkyl group which may be optionally substituted with one or more halogen atoms, a three(3) to seven(7) membered non-aromatic heterocyclic group which may be optionally substituted with one or more substituents selected from Group $G^3$, an amino group, $NHR^{99}$, $NR^{99}R^{100}$, a halogen atom, and a cyano group,

Group $M^3$: a group consisting of a C1-C6 alkyl group which may be optionally substituted with one or more halogen atoms, $OR^{102}$, $NR^{101}R^{102}$, $C(O)R^{102}$, $C(O)NR^{101}R^{102}$, $OC(O)R^{101}$, $OC(O)OR^{101}$, $NR^{102}C(O)R^{101}$, $NR^{102}C(O)OR^{101}$, $C(O)OR^{102}$, a halogen atom, a nitro group, a cyano group, an amino group, and a five(5) or six(6) membered aromatic heterocyclic group,

$R^{101}$ represents a C1-C6 alkyl group which may be optionally substituted with one or more halogen atoms, or a C3-C6 cycloalkyl group which may be optionally substituted with one or more halogen atoms,

$R^{102}$ represents a C1-C6 alkyl group which may be optionally substituted with one or more halogen atoms, a C3-C6 cycloalkyl group which may be optionally substituted with one or more halogen atoms, or a hydrogen atom,

Group $T^3$: a group consisting of a C1-C6 alkyl group which may be optionally substituted with one or more halogen atoms, a halogen atom, and a cyano group]

(hereinafter, referred to as "Present compound N" or "Compound N of the present invention") or N-oxide thereof

(hereinafter, the compound represented by formula (I) or N-oxide thereof is referred to as "Present compound" or "Compound of the present invention").

[2] The compound according to [1] or N-oxide thereof wherein Q represents a group represented by formula Q1 or a group represented by formula Q5.

[3] The compound according to [1] or N-oxide thereof wherein Q represents a group represented by formula Q1 and $A^1$ represents a nitrogen atom.

[4] The compound according to [1] or N-oxide thereof wherein Q represents a group represented by formula Q5, $G^1$ represents $CR^{10a}$, $G^2$ represents $CR^{10b}$, $G^3$ represents $CR^{10c}$, and $G^4$ represents a nitrogen atom or $CR^{10d}$.

[5] The compound according to [1] or N-oxide thereof wherein Q represents a group represented by formula Q5, $G^1$ represents $CR^{10a}$, $G^2$ represents $CR^{10b}$, $G^3$ represents $CR^{10c}$, and $G^4$ represents $CR^{10d}$.

[6] The compound according to any one of [1] to [5] or N-oxide thereof wherein Het represents a group represented by formula Het1 , $Z^1$ represents $CR^{9a}$, $Z^2$ represents $CR^{9b}$, $Z^3$ represents $CR^{9c}$, and $Z^4$ represents $CR^{9d}$.

[7] The compound according to any one of [1] to [5] or N-oxide thereof wherein Het represents a group represented by formula Het2, $Z^1$ represents $CR^{9a}$, $Z^2$ represents $CR^{9b}$, $Z^3$ represents $CR^{9c}$, and $Z^4$ represents $CR^{9d}$.

[8] The compound according to any one of [1] to [7] or N-oxide thereof wherein $R^2$ represents an ethyl group.

[9] The compound according to any one of [1] to [7] or N-oxide thereof wherein W is an oxygen atom.

[10] A composition for controlling harmful arthropod which comprises the compound according to any one of [1] to [9] or N-oxide thereof and an inert career.

[11] A composition comprising one or more ingredients selected from the group consisting of the following Group (a), Group (b), Group (c), and Group (d), and the compound according to any one of [1] to [9] or N-oxide thereof

Group (a): a group consisting of insecticidal active ingredients, miticidal active ingredients, and nematicidal active ingredients;

Group (b): fungicidal active ingredients,

Group (c): plant growth regulatory ingredients; and
Group (d): repellent ingredients.

[12] A method for controlling harmful arthropod which comprises applying an effective amount of the compound according to any one of [1] to [9] or N-oxide thereof, or an effective amount of the composition according to claim 11 to a harmful arthropod or a habitat where a harmful arthropod lives.
[13] A seed or vegetative reproductive organ carrying an effective amount of the compound according to any one of [1] to [9] or N-oxide thereof, or an effective amount of the composition according to [11].

[EFFECT OF INVENTION]

The present invention can control harmful arthropod.

MODE FOR CARRYING OUT THE INVENTION

The substituent(s) as described herein is/are explained.
The term "halogen atom" represents fluorine atom, chlorine atom, bromine atom, or iodine atom.
When the substituents have two or more halogen atoms or substituents, these halogen atoms or substituents may be identical to or different from each other.
The expression of "CX-CY" as used herein represents that the number of carbon atom is from X to Y. For example, the expression of "C1-C6" represents that the number of carbon atom is from 1 to 6.
The term of "chain hydrocarbon group" represents an alkyl group, an alkenyl group, or an alkynyl group.
Examples of "alkyl group" include methyl group, ethyl group, propyl group, isopropyl group, 1,1-dimethylpropyl group, 1,2-dimethylpropyl group, 1-ethylpropyl group, butyl group, sec-butyl group, tert-butyl group, pentyl group, and hexyl group.
Examples of "alkenyl group" include vinyl group, 1-propenyl group, 2-propenyl group, 1-methyl-1-propenyl group, 1-methyl-2-propenyl group, 1,2-dimethyl-1-propenyl group, 1-ethyl-1-propenyl group, 3-butenyl group, 4-pentenyl group, and 5-hexenyl group.
Examples of "alkynyl group" includes ethynyl group, 1-propynyl group, 2-propynyl group, 1-methyl-2-propynyl group, 1,1-dimethyl-2-propynyl group, 1-ethyl-2-propynyl group, 2-butynyl group, 4-pentynyl group, and 5-hexynyl group.
Examples of "alkoxy group" includes methoxy group, ethoxy group, propoxy group, isopropoxy group, butoxy group, tert-butoxy group, pentyloxy group, and hexyloxy group.
Examples of "alkenyloxy group" includes 2-propenyloxy group, 2-butenyloxy group, and 5-hexenyloxy group.
Examples of "alkynyloxy group" includes 2-propynyloxy group, 2-butynyloxy group, and 5-hexynyloxy group.
Examples of "cycloalkyl group" include cyclopropyl group, cyclobutyl group, cyclopentyl group, cyclohexyl group, and cycloheptyl group.
Examples of "cycloalkenyl group" include cyclopropenyl group, cyclobutenyl group, cyclopentenyl group, cyclohexenyl group, and cycloheptenyl group.
Examples of "three(3) to seven(7) membered non-aromatic heterocyclic group" include aziridinyl group, oxiranyl group, thiiranyl group, azetidinyl group, oxetanyl group, thietanyl group, pyrrolidinyl group, tetrahydrofuranyl group, tetrahydrothienyl group, piperidyl group, pyranyl group, dihydropyranyl group, tetrahydropyranyl group, tetrahydrothiopyranyl group, azepanyl group, oxepanyl group, thepanyl group, pyrazolyl group, pyrazolidinyl group, imidazolidinyl group, imidazolidinyl group, oxazolinyl group, thiazolinyl group, oxazolidinyl group, thiazolidinyl group, isoxazolinyl group, isoxazolidinyl group, isothiazolinyl group, isothiazolidiny group, dioxolyl group, dioxolanyl group, Dioxanyl group, morpholinyl group, thiomorpholinyl group, and piperazinyl group.
Examples of "five(5) membered or six(6) aromatic heterocyclic group" include pyrrolyl group, furyl group, thienyl group, pyrazolyl group, imidazolyl group, triazolyl group, tetrazolyl group, oxazolyl group, isoxazolyl group, thiazolyl group, isothiazolyl group, oxadiazolyl group, thiadiazolyl group, pyridyl group, pyridazinyl group, pyrimidinyl group, pyrazinyl group, triazinyl group, and tetrazinyl group.
Examples of "six(6) membered aromatic heterocyclic group" include pyridyl group, pyridazinyl group, pyrimidinyl group, pyrazinyl group, triazinyl group, and tetrazinyl group.
Examples of "(C3-C6 cycloalkyl)C1-C3 alkyl group which may be optionally substituted with one or more halogen atoms" include cyclopropylmethyl group, (2-fluorocyclopropyl)methyl group, cyclopropyl(fluoro)methyl group, and (2-fluorocyclopropyl) (fluoro)methyl group.
Examples of "C3-C7 cycloalkyl group which may be optionally substituted with one or more cyano groups" include 1-cyano-cyclopropyl group.
The term of "(C3-C7 cycloalkyl) C1-C6 alkyl group which may be optionally substituted with one or more halogen atoms" represents a group wherein (C3-C7 cycloalkyl) and / or (C1-C6 alkyl) may be optionally substituted with one or

more halogen atoms", and includes (2,2-difluorocyclopropyl)methyl group, 2-cyclopropyl-1,1,2,2-tetrafluoroethyl group, and 2-(2,2-difluorocyclopropyl)-1,1,2,2-tetrafluoroethyl group, (2,2-difluorocyclopropyl)propyl group, (2,2-difluorocyclopropyl)butyl group, (2,2-difluorocyclopropyl)pentyl group, (2,2-difluorocyclopropyl)hexyl group.

Examples of the "alkylsulfanyl group" include methylsulfanyl group, ethylsulfanyl group, propylsulfanyl group, and isopropylsulfanyl group.

Examples of the "alkylsulfinyl group" include methylsulfinyl group, ethylsulfinyl group, propylsulfinyl group, and isopropylsulfinyl group.

Examples of the "alkylsulfonyl group" include methylsulfonyl group, ethylsulfonyl group, propylsulfonyl group, and isopropylsulfonyl group.

Examples of the "alkylamino group" include methylamino group, ethylamino group, isopropylamino group, and hexylamino group.

Examples of the "dialkylamino group" include methylamino group, ethylmethylamino group, isopropylmethylamino group, and dihexylamino group.

Examples of the "alkylcarbonyl group" include acetyl group, propanoyl group, 2-methylpropanoyl group, and hexanoly group.

Examples of the "alkoxycarbonyl group" include methoxycarbonyl group, ethoxycarbonyl group, isopropxycarbonyl group, and pentyloxycarbonyl group.

Examples of the "(C1-C6 alkyl)aminocarbonyl group" include methylaminocarbonyl group, isopropylaminocarbonyl group, and hexylaminocarbonyl group.

Examples of the "di(C1-C4 alkyl)aminocarbonyl group include dimethylaminocarbonyl group, methylethylaminocarbonyl group, and isopropylmethylaminocarbonyl group.

Examples of the N-oxide of the compound represented by formula (I) include the compounds represented by the following formula.

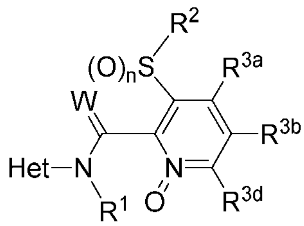

[wherein the symbols are the same as defined above]

The present compound may be existed as one or more stereoisomers. Examples of the stereoisomer include enantiomer, diastereomer, and geometric isomer. Each stereoisomer, and stereoisomer mixture(s) in an arbitrary ratio thereof are included in the present compound.

The present compound may form acid addition salts. Examples of the acid to form the acid addition salt include inorganic acids such as hydrogen chloride, phosphoric acid, sulfuric acid; and organic acids such as acetic acid, trifluoroacetic acid, benzoic acid, p-toluenesulfonic acid. The acid addition salt may be obtained by mixing the present compound with an acid.

Examples of embodiments of the present compound N include the following compounds.

[Embodiment 1] The present compound N wherein Q represents a group represented by formula Q1 or a group represented by formula Q5.

[Embodiment 2] The present compound N wherein Q represents a group represented by formula Q1 or a group represented by formula Q5, $G^1$ represents a nitrogen atom or $CR^{10a}$, $G^2$ represents $CR^{10b}$, $G^3$ represents $CR^{10c}$, $G^4$ represents $CR^{10d}$, $R^{3a}$, $R^{3b}$, $R^{3c}$, $R^{3d}$, $R^{10a}$, $R^{10b}$, $R^{10c}$, and $R^{10d}$ are identical to or different from each other and each represents a C1-C6 chain hydrocarbon group which may be optionally substituted with one or more substituents selected from Group B, a C3-C7 cycloalkyl group which may be optionally substituted with one or more substituents selected from Group K, a phenyl group which may be optionally substituted with one or more substituents selected from Group M, a five(5) or six(6) membered aromatic heterocyclic group which may be optionally substituted with one or more substituents selected from Group M, $OR^{12}$ , $CR^{20}$=$NOR^{17}$, a hydrogen atom, or a halogen atom (with the proviso that when Q represents a group represented by formula Q1 and $A^1$ represents $CR^{3c}$, all of the existing $R^{3a}$, $R^{3b}$, $R^{3c}$ and $R^{3d}$ don't represent a hydrogen atom), $R^{17}$ and $R^{20}$ are identical to or different from each other and each represents a C1-C6 chain hydrocarbon group which may be optionally substituted with one or more halogen atoms, $R^{12}$ represents a C1-C6 chain hydrocarbon group which may be optionally substituted with one or more substituents selected from Group L, a C3-C7 cycloalkyl group which may be optionally substituted with one or more substituents selected from Group T, a C3-C7 cycloalkenyl group which may be optionally substituted with one or more substituents

selected from Group T, a phenyl group which may be optionally substituted with one or more substituents selected from Group J, or a six(6) membered aromatic heterocyclic group which may be optionally substituted with one or more substituents selected from Group J.

[Embodiment 3] The present compound N wherein Q represents a group represented by formula Q1 or a group represented by formula Q5, $R^{3a}$, $R^{3c}$, $R^{10a}$ and $R^{10d}$ represent a hydrogen atom, $R^{3b}$, $R^{3d}$, $R^{10b}$ and $R^{10c}$ are identical to or different from each other and each represents a C1-C6 chain hydrocarbon group which may be optionally substituted with one or more halogen atoms, a C3-C7 cycloalkyl group which may be optionally substituted with one or more halogen atoms, a phenyl group which may be optionally substituted with one or more halogen atoms, a five(5) or six(6) membered aromatic heterocyclic group which may be optionally substituted with one or more halogen atoms, $OR^{12}$, a hydrogen atom, or a halogen atom, $R^{12}$ represents a C1-C6 chain hydrocarbon group which may be optionally substituted with one or more halogen atoms.

[Embodiment 4] The present compound N wherein Q represents a group represented by formula Q1 or a group represented by formula Q5, $R^{3a}$, $R^{3c}$, $R^{10a}$ and $R^{10d}$ represent a hydrogen atom, $R^{3b}$, $R^{3d}$, $R^{10b}$ and $R^{10c}$ are identical to or different from each other and each represents a C1-C6 chain hydrocarbon group which may be optionally substituted with one or more halogen atoms, a hydrogen atom, or a halogen atom.

[Embodiment 5] The present compound N wherein Q represent a group represented by formula Q1 or a group represented by formula Q5, $A^1$ represents a nitrogen atom, $R^{3a}$, $R^{3d}$, $R^{10a}$, $R^{10c}$ and $R^{10d}$ represent a hydrogen atom, $R^{3b}$ represents a phenyl group which may be optionally substituted with one or more substituents selected from a group consisting of a halogen and a cyano group, a hydrogen atom, or a halogen atom, and $R^{10b}$ represents a halogen atom.

[Embodiment 6] The present compound N wherein Q represents a group represented by formula Q1.

[Embodiment 7] The present compound N wherein Q represents a group represented by formula Q1, $R^{3a}$, $R^{3b}$, and $R^{3d}$ are identical to or different from each other and each represents a C1-C6 chain hydrocarbon group which may be optionally substituted with one or more substituents selected from Group B, a C3-C7 cycloalkyl group which may be optionally substituted with one or more substituents selected from Group K, a phenyl group which may be optionally substituted with one or more substituents selected from Group M, a five(5) or six(6) membered aromatic heterocyclic group which may be optionally substituted with one or more substituents selected from Group M, $OR^{12}$, $CR^{20}=NOR^{17}$, a hydrogen atom, or a halogen atom (with the proviso that when Q represents a group represented by formula Q1 and $A^1$ represents $CR^{3c}$, all of the existing $R^{3a}$, $R^{3b}$, $R^{3c}$ and $R^{3d}$ don't represent a hydrogen atom), $R^{17}$ and $R^{20}$ are identical to or different from each other and each represents a C1-C6 chain hydrocarbon group which may be optionally substituted with one or more halogen atoms, $R^{12}$ represents a C1-C6 chain hydrocarbon group which may be optionally substituted with one or more substituents selected from Group B, a C3-C7 cycloalkyl group which may be optionally substituted with one or more substituents selected from Group K, a phenyl group which may be optionally substituted with one or more substituents selected from Group M, or a five(5) or six(6) membered aromatic heterocyclic group which may be optionally substituted with one or more substituents selected from Group M.

[Embodiment 8] The present compound N wherein Q represents a group represented by formula Q1, $R^{3a}$ represents a hydrogen atom, $R^{3b}$ and $R^{3d}$ are identical to or different from each other and each represents a C1-C6 chain hydrocarbon group which may be optionally substituted with one or more halogen atoms, a C3-C7 cycloalkyl group which may be optionally substituted with one or more halogen atoms, a phenyl group which may be optionally substituted with one or more halogen atoms, a five(5) or six(6) membered aromatic heterocyclic group which may be optionally substituted with one or more halogen atoms, $OR^{12}$, a hydrogen atom, or a halogen atom, $R^{12}$ represents a C1-C6 chain hydrocarbon group which may be optionally substituted with one or more halogen atoms.

[Embodiment 1] The present compound N wherein Q represents a group represented by formula Q1, $R^{3a}$ represents a hydrogen atom, $R^{3b}$ and $R^{3d}$ are identical to or different from each other and each represents a phenyl group which may be optionally substituted with one or more halogen atoms, a five(5) or six(6) membered aromatic heterocyclic group which may be optionally substituted with one or more halogen atoms, a hydrogen atom, or a halogen atom.

[Embodiment 10] The present compound N wherein Q represents a group represented by formula Q1, $R^{3a}$ represents a hydrogen atom, $R^{3b}$ and $R^{3d}$ are identical to or different from each other and each represents a phenyl group which may be optionally substituted with one or more halogen atoms, a pyrazolyl group which may be optionally substituted with one or more halogen atoms, an imidazolyl group which may be optionally substituted with one or more halogen atoms, a triazolyl group which may be optionally substituted with one or more halogen atoms, a pyridyl group which may be optionally substituted with one or more halogen atoms, a pyrimidinyl group which may be optionally substituted with one or more halogen atoms, a hydrogen atom, or a halogen atom.

[Embodiment 11] The present compound N wherein Q represents a group represented by formula Q1, $A^1$ represents a nitrogen atom, $R^{3a}$ and $R^{3d}$ represent a hydrogen atom, $R^{3b}$ represents a phenyl group which may be optionally substituted with one or more substituents selected from a group consisting of a halogen and a cyano group, a hydrogen atom, or a halogen atom.

[Embodiment 12] The present compound N wherein Q represents a group represented by formula Q5.

[Embodiment 13] The present compound N wherein Q represents a group represented by formula Q5, $G^1$ represents a nitrogen atom or $CR^{10a}$, $G^2$ represents $CR^{10b}$, $G^3$ represents $CR^{10c}$, $G^4$ represents $CR^{10d}$, $R^{10a}$, $R^{10b}$, $R^{10c}$, and $R^{10d}$ are identical to or different from each other and each represents a C1-C6 chain hydrocarbon group which may be optionally substituted with one or more substituents selected from Group B, a C3-C7 cycloalkyl group which may be optionally substituted with one or more substituents selected from Group K, a phenyl group which may be optionally substituted with one or more substituents selected from Group M, a five(5) or six (6) membered aromatic heterocyclic group which may be optionally substituted with one or more substituents selected from Group M, $OR^{12}$, $CR^{20}$=$NOR^{17}$, a hydrogen atom, or a halogen atom, $R^{17}$ and $R^{20}$ are identical to or different from each other and each represents a C1-C6 chain hydrocarbon group which may be optionally substituted with one or more halogen atoms, $R^{12}$ represents a C1-C6 chain hydrocarbon group which may be optionally substituted with one or more substituents selected from Group L, a C3-C7 cycloalkyl group which may be optionally substituted with one or more substituents selected from Group T, a C3-C7 cycloalkenyl group which may be optionally substituted with one or more substituents selected from Group T, a phenyl group which may be optionally substituted with one or more substituents selected from Group J, or a six(6) membered aromatic heterocyclic group which may be optionally substituted with one or more substituents selected from Group J.

[Embodiment 14] The present compound N wherein Q represents a group represented by formula Q5, $G^1$ represents a nitrogen atom or $CR^{10a}$, $G^2$ represents $CR^{10b}$, $G^3$ represents $CR^{10c}$, $G^4$ represents $CR^{10d}$, $R^{10a}$ and $R^{10d}$ represent a hydrogen atom, $R^{10b}$ and $R^{10c}$ are identical to or different from each other and each represents a C1-C6 chain hydrocarbon group which may be optionally substituted with one or more halogen atoms, a C3-C7 cycloalkyl group which may be optionally substituted with one or more halogen atoms, a phenyl group which may be optionally substituted with one or more halogen atoms, a five(5) or six(6) membered aromatic heterocyclic group which may be optionally substituted with one or more halogen atoms, $OR^{12a}$, hydrogen atom, or a halogen atom, $R^{12}$ represents a C1-C6 chain hydrocarbon group which may be optionally substituted with one or more halogen atoms.

[Embodiment 15] The present compound N wherein Q represents a group represented by formula Q5, $G^1$ represents a nitrogen atom or $CR^{10a}$, $G^2$ represents $CR^{10b}$, $G^3$ represents $CR^{10c}$, $G^4$ represents $CR^{10d}$, $R^{10a}$ and $R^{10d}$ represent a hydrogen atom, $R^{10b}$ and $R^{10c}$ are identical to or different from each other and each represents a C1-C6 chain hydrocarbon group which may be optionally substituted with one or more halogen atoms, a hydrogen atom, or a halogen atom.

[Embodiment 16] The present compound N wherein Q represents a group represented by formula Q5, $G^1$ represents $CR^{10a}$, $G^2$ represents $CR^{10b}$, $G^3$ represents $CR^{10c}$, $G^4$ represents $CR^{10d}$, $R^{10a}$, $R^{10c}$ and $R^{10d}$ represent a hydrogen atom, and $R^{10b}$ represents a halogen atom.

[Embodiment 17] The present compound N wherein $R^2$ represents an ethyl group.

[Embodiment 18] The present compound N wherein $R^1$ represents a methyl group, an ethyl group, or a cyclopropylmethyl group.

[Embodiment 19] The present compound N wherein W is an oxygen atom.

[Embodiment 20] The present compound N wherein $X^{1a}$ represents $NR^{7a}$, $X^{2a}$ represents $CR^{8a}$, $R^{7a}$ represents a C1-C6 chain hydrocarbon group which may be optionally substituted with one or more halogen atoms, a C3-C7 cycloalkyl group which may be optionally substituted with one or more halogen atoms, a (C3-C7 cycloalkyl) C1-C6 alkyl group which may be optionally substituted with one or more halogen atoms, or a hydrogen atom, $R^{8a}$ represents a C1-C6 chain hydrocarbon group which may be optionally substituted with one or more halogen atoms, a C3-C7 cycloalkyl group which may be optionally substituted with one or more halogen atoms, a cyano group, a nitro group, a hydrogen atom, or a halogen atom.

[Embodiment 21] The present compound N wherein $X^{1a}$ represents $NR^{7a}$, $X^{2a}$ represents a nitrogen atom, $R^{7a}$ represents a C1-C6 chain hydrocarbon group which may be optionally substituted with one or more halogen atoms, a C3-C7 cycloalkyl group which may be optionally substituted with one or more halogen atoms, a (C3-C7 cycloalkyl) C1-C6 alkyl group which may be optionally substituted with one or more halogen atoms, or a hydrogen atom.

[Embodiment 22] The present compound N wherein $X^{1a}$ represents an oxygen atom, $X^{2a}$ represents $CR^{8a}$, and $R^{8a}$ represents a C1-C6 chain hydrocarbon group which may be optionally substituted with one or more halogen atoms, a C3-C7 cycloalkyl group which may be optionally substituted with one or more halogen atoms, a cyano group, a nitro group, a hydrogen atom, or a halogen atom.

[Embodiment 23] The present compound N wherein $X^{1a}$ represents an oxygen atom, and $X^{2a}$ represents a nitrogen atom.

[Embodiment 24] The present compound N wherein $X^{1a}$ represents a sulfur atom, $X^{2a}$ represents $CR^{8a}$, and $R^{8a}$ represents a C1-C6 chain hydrocarbon group which may be optionally substituted with one or more halogen atoms, a C3-C7 cycloalkyl group which may be optionally substituted with one or more halogen atoms, a cyano group, a nitro group, a hydrogen atom, or a halogen atom.

[Embodiment 25] The present compound N wherein $X^{1a}$ represents a sulfur atom, and $X^{2a}$ represents a nitrogen atom.

[Embodiment 26] The present compound N wherein $X^{2b}$ represents $CR^{8a}$, $X^{3a}$ represents $CR^{8b}$, and $R^{8a}$ and $R^{8b}$ are

identical to or different from each other and each represents a C1-C6 chain hydrocarbon group which may be optionally substituted with one or more halogen atoms, a C3-C7 cycloalkyl group which may be optionally substituted with one or more halogen atoms, a cyano group, a nitro group, a hydrogen atom, or a halogen atom.

[Embodiment 27] The present compound N wherein $X^{2b}$ represents a nitrogen atom, $X^{3a}$ represents $CR^{8b}$, and $R^{8b}$ represents a C1-C6 chain hydrocarbon group which may be optionally substituted with one or more halogen atoms, a C3-C7 cycloalkyl group which may be optionally substituted with one or more halogen atoms, a cyano group, a nitro group, a hydrogen atom, or a halogen atom.

[Embodiment 28] The present compound N wherein $X^{2b}$ represents $CR^{8a}$, $X^{3a}$ represents a nitrogen atom, and $R^{8a}$ represents a C1-C6 chain hydrocarbon group which may be optionally substituted with one or more halogen atoms, a C3-C7 cycloalkyl group which may be optionally substituted with one or more halogen atoms, a cyano group, a nitro group, a hydrogen atom, or a halogen atom.

[Embodiment 29] The present compound N wherein $X^{2b}$ represents a nitrogen atom, and $X^{3a}$ represents a nitrogen atom.

[Embodiment 30] The present compound N wherein $X^{1a}$ represents $NR^{7a}$, $X^{2a}$ represents $CR^{8a}$, $R^{7a}$ represents a methyl group, and $R^{8a}$ represents a methyl group, a hydrogen atom, or a halogen atom.

[Embodiment 31] The present compound N wherein $X^{1a}$ represents $NR^{7a}$, $X^{2a}$ represents a nitrogen atom, and $R^{7a}$ represents a methyl group.

[Embodiment 32] The present compound N wherein $X^{1a}$ represents an oxygen atom, $X^{2a}$ represents $CR^{8a}$, and $R^{8a}$ represents a methyl group, a hydrogen atom, or a halogen atom.

[Embodiment 33] The present compound N wherein $X^{1a}$ represents a sulfur atom, $X^{2a}$ represents $CR^{8a}$, and $R^{8a}$ represents a methyl group, a hydrogen atom, or a halogen atom.

[Embodiment 34] The present compound N wherein $X^{2b}$ represents $CR^{8a}$, $X^{3a}$ represents a nitrogen atom, and $R^{8a}$ represents a methyl group, a hydrogen atom, or a halogen atom.

[Embodiment 35] The present compound N wherein $X^{2b}$ represents $CR^{8a}$, $X^{3a}$ represents $CR^{8b}$, and $R^{8a}$ and $R^{8b}$ are identical to or different from each other and each represents a methyl group, a hydrogen atom, or a halogen atom.

[Embodiment 36] The present compound N wherein $X^{2b}$ represents a nitrogen atom, $X^{3a}$ represents $CR^{8b}$, and $R^{8b}$ represents a methyl group, a hydrogen atom, or a halogen atom.

[Embodiment 37] The present compound N wherein $X^{1a}$ represents $NR^{7a}$, an oxygen atom or a sulfur atom, $X^{2a}$ represents a nitrogen atom, and $R^{7a}$ represents a C1-C6 alkyl group.

[Embodiment 38] The present compound N wherein Het represents a group represented by formula Het1 or a group represented by formula Het2.

[Embodiment 39] The present compound N wherein Het represents a group represented by formula Het1.

[Embodiment 40] The present compound N wherein Het represents a group represented by formula Het2.

[Embodiment 41] The compound in the embodiment 1 wherein $R^2$ represents an ethyl group.

[Embodiment 42] The compound in the embodiment 2 wherein $R^2$ represents an ethyl group.

[Embodiment 43] The compound in the embodiment 3 wherein $R^2$ represents an ethyl group.

[Embodiment 44] The compound in the embodiment 4 wherein $R^2$ represents an ethyl group.

[Embodiment 45] The compound in the embodiment 5 wherein $R^2$ represents an ethyl group.

[Embodiment 46] The compound in the embodiment 6 wherein $R^2$ represents an ethyl group.

[Embodiment 47] The compound in the embodiment 7 wherein $R^2$ represents an ethyl group.

[Embodiment 48] The compound in the embodiment 8 wherein $R^2$ represents an ethyl group.

[Embodiment 49] The compound in the embodiment 9 wherein $R^2$ represents an ethyl group.

[Embodiment 50] The compound in the embodiment 10 wherein $R^2$ represents an ethyl group.

[Embodiment 51] The compound in the embodiment 11 wherein $R^2$ represents an ethyl group.

[Embodiment 52] The compound in the embodiment 12 wherein $R^2$ represents an ethyl group.

[Embodiment 53] The compound in the embodiment 13 wherein $R^2$ represents an ethyl group.

[Embodiment 54] The compound in the embodiment 14 wherein $R^2$ represents an ethyl group.

[Embodiment 55] The compound in the embodiment 15 wherein $R^2$ represents an ethyl group.

[Embodiment 56] The compound in the embodiment 16 wherein $R^2$ represents an ethyl group.

[Embodiment 57] The compound in the embodiment 18 wherein $R^2$ represents an ethyl group.

[Embodiment 58] The compound in the embodiment 19 wherein $R^2$ represents an ethyl group.

[Embodiment 59] The compound in the embodiment 20 wherein $R^2$ represents an ethyl group.

[Embodiment 60] The compound in the embodiment 21 wherein $R^2$ represents an ethyl group.

[Embodiment 61] The compound in the embodiment 22 wherein $R^2$ represents an ethyl group.

[Embodiment 62] The compound in the embodiment 23 wherein $R^2$ represents an ethyl group.

[Embodiment 63] The compound in the embodiment 24 wherein $R^2$ represents an ethyl group.

[Embodiment 64] The compound in the embodiment 25 wherein $R^2$ represents an ethyl group.

[Embodiment 65] The compound in the embodiment 26 wherein $R^2$ represents an ethyl group.

[Embodiment 66] The compound in the embodiment 27 wherein $R^2$ represents an ethyl group.
[Embodiment 67] The compound in the embodiment 28 wherein $R^2$ represents an ethyl group.
[Embodiment 68] The compound in the embodiment 29 wherein $R^2$ represents an ethyl group.
[Embodiment 69] The compound in the embodiment 30 wherein $R^2$ represents an ethyl group.
[Embodiment 70] The compound in the embodiment 31 wherein $R^2$ represents an ethyl group.
[Embodiment 71] The compound in the embodiment 32 wherein $R^2$ represents an ethyl group.
[Embodiment 72] The compound in the embodiment 33 wherein $R^2$ represents an ethyl group.
[Embodiment 73] The compound in the embodiment 34 wherein $R^2$ represents an ethyl group.
[Embodiment 74] The compound in the embodiment 35 wherein $R^2$ represents an ethyl group.
[Embodiment 75] The compound in the embodiment 36 wherein $R^2$ represents an ethyl group.
[Embodiment 76] The compound in the embodiment 37 wherein $R^2$ represents an ethyl group.
[Embodiment 77] The compound in the embodiment 38 wherein $R^2$ represents an ethyl group.
[Embodiment 78] The compound in the embodiment 39 wherein $R^2$ represents an ethyl group.
[Embodiment 79] The compound in the embodiment 40 wherein $R^2$ represents an ethyl group.
[Embodiment 80] The compound in the embodiment 1 wherein $R^1$ represents a methyl group, an ethyl group, or a cyclopropylmethyl group.
[Embodiment 81] The compound in the embodiment 2 wherein $R^1$ represents a methyl group, an ethyl group, or a cyclopropylmethyl group.
[Embodiment 82] The compound in the embodiment 3 wherein $R^1$ represents a methyl group, an ethyl group, or a cyclopropylmethyl group.
[Embodiment 83] The compound in the embodiment 4 wherein $R^1$ represents a methyl group, an ethyl group, or a cyclopropylmethyl group.
[Embodiment 84] The compound in the embodiment 5 wherein $R^1$ represents a methyl group, an ethyl group, or a cyclopropylmethyl group.
[Embodiment 85] The compound in the embodiment 6 wherein $R^1$ represents a methyl group, an ethyl group, or a cyclopropylmethyl group.
[Embodiment 86] The compound in the embodiment 7 wherein $R^1$ represents a methyl group, an ethyl group, or a cyclopropylmethyl group.
[Embodiment 87] The compound in the embodiment 8 wherein $R^1$ represents a methyl group, an ethyl group, or a cyclopropylmethyl group.
[Embodiment 88] The compound in the embodiment 9 wherein $R^1$ represents a methyl group, an ethyl group, or a cyclopropylmethyl group.
[Embodiment 89] The compound in the embodiment 10 wherein $R^1$ represents a methyl group, an ethyl group, or a cyclopropylmethyl group.
[Embodiment 90] The compound in the embodiment 11 wherein $R^1$ represents a methyl group, an ethyl group, or a cyclopropylmethyl group.
[Embodiment 91] The compound in the embodiment 12 wherein $R^1$ represents a methyl group, an ethyl group, or a cyclopropylmethyl group.
[Embodiment 92] The compound in the embodiment 13 wherein $R^1$ represents a methyl group, an ethyl group, or a cyclopropylmethyl group.
[Embodiment 93] The compound in the embodiment 14 wherein $R^1$ represents a methyl group, an ethyl group, or a cyclopropylmethyl group.
[Embodiment 94] The compound in the embodiment 15 wherein $R^1$ represents a methyl group, an ethyl group, or a cyclopropylmethyl group.
[Embodiment 95] The compound in the embodiment 16 wherein $R^1$ represents a methyl group, an ethyl group, or a cyclopropylmethyl group.
[Embodiment 96] The compound in the embodiment 19 wherein $R^1$ represents a methyl group, an ethyl group, or a cyclopropylmethyl group.
[Embodiment 97] The compound in the embodiment 20 wherein $R^1$ represents a methyl group, an ethyl group, or a cyclopropylmethyl group.
[Embodiment 98] The compound in the embodiment 21 wherein $R^1$ represents a methyl group, an ethyl group, or a cyclopropylmethyl group.
[Embodiment 99] The compound in the embodiment 22 wherein $R^1$ represents a methyl group, an ethyl group, or a cyclopropylmethyl group.
[Embodiment 100] The compound in the embodiment 23 wherein $R^1$ represents a methyl group, an ethyl group, or a cyclopropylmethyl group.
[Embodiment 101] The compound in the embodiment 24 wherein $R^1$ represents a methyl group, an ethyl group, or a cyclopropylmethyl group.

[Embodiment 102] The compound in the embodiment 25 wherein $R^1$ represents a methyl group, an ethyl group, or a cyclopropylmethyl group.
[Embodiment 103] The compound in the embodiment 26 wherein $R^1$ represents a methyl group, an ethyl group, or a cyclopropylmethyl group.
[Embodiment 104] The compound in the embodiment 27 wherein $R^1$ represents a methyl group, an ethyl group, or a cyclopropylmethyl group.
[Embodiment 105] The compound in the embodiment 28 wherein $R^1$ represents a methyl group, an ethyl group, or a cyclopropylmethyl group.
[Embodiment 106] The compound in the embodiment 29 wherein $R^1$ represents a methyl group, an ethyl group, or a cyclopropylmethyl group.
[Embodiment 107] The compound in the embodiment 30 wherein $R^1$ represents a methyl group, an ethyl group, or a cyclopropylmethyl group.
[Embodiment 108] The compound in the embodiment 31 wherein $R^1$ represents a methyl group, an ethyl group, or a cyclopropylmethyl group.
[Embodiment 109] The compound in the embodiment 32 wherein $R^1$ represents a methyl group, an ethyl group, or a cyclopropylmethyl group.
[Embodiment 110] The compound in the embodiment 33 wherein $R^1$ represents a methyl group, an ethyl group, or a cyclopropylmethyl group.
[Embodiment 111] The compound in the embodiment 34 wherein $R^1$ represents a methyl group, an ethyl group, or a cyclopropylmethyl group.
[Embodiment 112] The compound in the embodiment 35 wherein $R^1$ represents a methyl group, an ethyl group, or a cyclopropylmethyl group.
[Embodiment 113] The compound in the embodiment 36 wherein $R^1$ represents a methyl group, an ethyl group, or a cyclopropylmethyl group.
[Embodiment 114] The compound in the embodiment 37 wherein $R^1$ represents a methyl group, an ethyl group, or a cyclopropylmethyl group.
[Embodiment 115] The compound in the embodiment 38 wherein $R^1$ represents a methyl group, an ethyl group, or a cyclopropylmethyl group.
[Embodiment 116] The compound in the embodiment 39 wherein $R^1$ represents a methyl group, an ethyl group, or a cyclopropylmethyl group.
[Embodiment 117] The compound in the embodiment 40 wherein $R^1$ represents a methyl group, an ethyl group, or a cyclopropylmethyl group.
[Embodiment 118] The compound in the embodiment 41 wherein $R^1$ represents a methyl group, an ethyl group, or a cyclopropylmethyl group.
[Embodiment 119] The compound in the embodiment 42 wherein $R^1$ represents a methyl group, an ethyl group, or a cyclopropylmethyl group.
[Embodiment 120] The compound in the embodiment 43 wherein $R^1$ represents a methyl group, an ethyl group, or a cyclopropylmethyl group.
[Embodiment 121] The compound in the embodiment 44 wherein $R^1$ represents a methyl group, an ethyl group, or a cyclopropylmethyl group.
[Embodiment 122] The compound in the embodiment 45 wherein $R^1$ represents a methyl group, an ethyl group, or a cyclopropylmethyl group.
[Embodiment 123] The compound in the embodiment 46 wherein $R^1$ represents a methyl group, an ethyl group, or a cyclopropylmethyl group.
[Embodiment 124] The compound in the embodiment 47 wherein $R^1$ represents a methyl group, an ethyl group, or a cyclopropylmethyl group.
[Embodiment 125] The compound in the embodiment 48 wherein $R^1$ represents a methyl group, an ethyl group, or a cyclopropylmethyl group.
[Embodiment 126] The compound in the embodiment 49 wherein $R^1$ represents a methyl group, an ethyl group, or a cyclopropylmethyl group.
[Embodiment 127] The compound in the embodiment 50 wherein $R^1$ represents a methyl group, an ethyl group, or a cyclopropylmethyl group.
[Embodiment 128] The compound in the embodiment 51 wherein $R^1$ represents a methyl group, an ethyl group, or a cyclopropylmethyl group.
[Embodiment 129] The compound in the embodiment 52 wherein $R^1$ represents a methyl group, an ethyl group, or a cyclopropylmethyl group.
[Embodiment 130] The compound in the embodiment 53 wherein $R^1$ represents a methyl group, an ethyl group, or a cyclopropylmethyl group.

[Embodiment 131] The compound in the embodiment 54 wherein $R^1$ represents a methyl group, an ethyl group, or a cyclopropylmethyl group.
[Embodiment 132] The compound in the embodiment 55 wherein $R^1$ represents a methyl group, an ethyl group, or a cyclopropylmethyl group.
[Embodiment 133] The compound in the embodiment 56 wherein $R^1$ represents a methyl group, an ethyl group, or a cyclopropylmethyl group.
[Embodiment 134] The compound in the embodiment 57 wherein $R^1$ represents a methyl group, an ethyl group, or a cyclopropylmethyl group.
[Embodiment 135] The compound in the embodiment 58 wherein $R^1$ represents a methyl group, an ethyl group, or a cyclopropylmethyl group.
[Embodiment 136] The compound in the embodiment 59 wherein $R^1$ represents a methyl group, an ethyl group, or a cyclopropylmethyl group.
[Embodiment 137] The compound in the embodiment 60 wherein $R^1$ represents a methyl group, an ethyl group, or a cyclopropylmethyl group.
[Embodiment 138] The compound in the embodiment 61 wherein $R^1$ represents a methyl group, an ethyl group, or a cyclopropylmethyl group.
[Embodiment 139] The compound in the embodiment 62 wherein $R^1$ represents a methyl group, an ethyl group, or a cyclopropylmethyl group.
[Embodiment 140] The compound in the embodiment 63 wherein $R^1$ represents a methyl group, an ethyl group, or a cyclopropylmethyl group.
[Embodiment 141] The compound in the embodiment 64 wherein $R^1$ represents a methyl group, an ethyl group, or a cyclopropylmethyl group.
[Embodiment 142] The compound in the embodiment 65 wherein $R^1$ represents a methyl group, an ethyl group, or a cyclopropylmethyl group.
[Embodiment 143] The compound in the embodiment 66 wherein $R^1$ represents a methyl group, an ethyl group, or a cyclopropylmethyl group.
[Embodiment 144] The compound in the embodiment 67 wherein $R^1$ represents a methyl group, an ethyl group, or a cyclopropylmethyl group.
[Embodiment 145] The compound in the embodiment 68 wherein $R^1$ represents a methyl group, an ethyl group, or a cyclopropylmethyl group.
[Embodiment 146] The compound in the embodiment 69 wherein $R^1$ represents a methyl group, an ethyl group, or a cyclopropylmethyl group.
[Embodiment 147] The compound in the embodiment 70 wherein $R^1$ represents a methyl group, an ethyl group, or a cyclopropylmethyl group.
[Embodiment 148] The compound in the embodiment 71 wherein $R^1$ represents a methyl group, an ethyl group, or a cyclopropylmethyl group.
[Embodiment 149] The compound in the embodiment 72 wherein $R^1$ represents a methyl group, an ethyl group, or a cyclopropylmethyl group.
[Embodiment 150] The compound in the embodiment 73 wherein $R^1$ represents a methyl group, an ethyl group, or a cyclopropylmethyl group.
[Embodiment 151] The compound in the embodiment 74 wherein $R^1$ represents a methyl group, an ethyl group, or a cyclopropylmethyl group.
[Embodiment 152] The compound in the embodiment 75 wherein $R^1$ represents a methyl group, an ethyl group, or a cyclopropylmethyl group.
[Embodiment 153] The compound in the embodiment 76 wherein $R^1$ represents a methyl group, an ethyl group, or a cyclopropylmethyl group.
[Embodiment 154] The compound in the embodiment 77 wherein $R^1$ represents a methyl group, an ethyl group, or a cyclopropylmethyl group.
[Embodiment 155] The compound in the embodiment 78 wherein $R^1$ represents a methyl group, an ethyl group, or a cyclopropylmethyl group.
[Embodiment 156] The compound in the embodiment 79 wherein $R^1$ represents a methyl group, an ethyl group, or a cyclopropylmethyl group.
[Embodiment 157] The compound in the embodiment 1 wherein W is an oxygen atom.
[Embodiment 158] The compound in the embodiment 2 wherein W is an oxygen atom.
[Embodiment 159] The compound in the embodiment 3 wherein W is an oxygen atom.
[Embodiment 160] The compound in the embodiment 4 wherein W is an oxygen atom.
[Embodiment 161] The compound in the embodiment 5 wherein W is an oxygen atom.
[Embodiment 162] The compound in the embodiment 6 wherein W is an oxygen atom.

[Embodiment 163] The compound in the embodiment 7 wherein W is an oxygen atom.
[Embodiment 164] The compound in the embodiment 8 wherein W is an oxygen atom.
[Embodiment 165] The compound in the embodiment 9 wherein W is an oxygen atom.
[Embodiment 166] The compound in the embodiment 10 wherein W is an oxygen atom.
[Embodiment 167] The compound in the embodiment 11 wherein W is an oxygen atom.
[Embodiment 168] The compound in the embodiment 12 wherein W is an oxygen atom.
[Embodiment 169] The compound in the embodiment 13 wherein W is an oxygen atom.
[Embodiment 170] The compound in the embodiment 14 wherein W is an oxygen atom.
[Embodiment 171] The compound in the embodiment 15 wherein W is an oxygen atom.
[Embodiment 172] The compound in the embodiment 16 wherein W is an oxygen atom.
[Embodiment 173] The compound in the embodiment 20 wherein W is an oxygen atom.
[Embodiment 174] The compound in the embodiment 21 wherein W is an oxygen atom.
[Embodiment 175] The compound in the embodiment 22 wherein W is an oxygen atom.
[Embodiment 176] The compound in the embodiment 23 wherein W is an oxygen atom.
[Embodiment 177] The compound in the embodiment 24 wherein W is an oxygen atom.
[Embodiment 178] The compound in the embodiment 25 wherein W is an oxygen atom.
[Embodiment 179] The compound in the embodiment 26 wherein W is an oxygen atom.
[Embodiment 180] The compound in the embodiment 27 wherein W is an oxygen atom.
[Embodiment 181] The compound in the embodiment 28 wherein W is an oxygen atom.
[Embodiment 182] The compound in the embodiment 29 wherein W is an oxygen atom.
[Embodiment 183] The compound in the embodiment 30 wherein W is an oxygen atom.
[Embodiment 184] The compound in the embodiment 31 wherein W is an oxygen atom.
[Embodiment 185] The compound in the embodiment 32 wherein W is an oxygen atom.
[Embodiment 186] The compound in the embodiment 33 wherein W is an oxygen atom.
[Embodiment 187] The compound in the embodiment 34 wherein W is an oxygen atom.
[Embodiment 188] The compound in the embodiment 35 wherein W is an oxygen atom.
[Embodiment 189] The compound in the embodiment 36 wherein W is an oxygen atom.
[Embodiment 190] The compound in the embodiment 37 wherein W is an oxygen atom.
[Embodiment 191] The compound in the embodiment 38 wherein W is an oxygen atom.
[Embodiment 192] The compound in the embodiment 39 wherein W is an oxygen atom.
[Embodiment 193] The compound in the embodiment 40 wherein W is an oxygen atom.
[Embodiment 194] The compound in the embodiment 41 wherein W is an oxygen atom.
[Embodiment 195] The compound in the embodiment 42 wherein W is an oxygen atom.
[Embodiment 196] The compound in the embodiment 43 wherein W is an oxygen atom.
[Embodiment 197] The compound in the embodiment 44 wherein W is an oxygen atom.
[Embodiment 198] The compound in the embodiment 45 wherein W is an oxygen atom.
[Embodiment 199] The compound in the embodiment 46 wherein W is an oxygen atom.
[Embodiment 200] The compound in the embodiment 47 wherein W is an oxygen atom.
[Embodiment 201] The compound in the embodiment 48 wherein W is an oxygen atom.
[Embodiment 202] The compound in the embodiment 49 wherein W is an oxygen atom.
[Embodiment 203] The compound in the embodiment 50 wherein W is an oxygen atom.
[Embodiment 204] The compound in the embodiment 51 wherein W is an oxygen atom.
[Embodiment 205] The compound in the embodiment 52 wherein W is an oxygen atom.
[Embodiment 206] The compound in the embodiment 53 wherein W is an oxygen atom.
[Embodiment 207] The compound in the embodiment 54 wherein W is an oxygen atom.
[Embodiment 208] The compound in the embodiment 55 wherein W is an oxygen atom.
[Embodiment 209] The compound in the embodiment 56 wherein W is an oxygen atom.
[Embodiment 210] The compound in the embodiment 57 wherein W is an oxygen atom.
[Embodiment 211] The compound in the embodiment 58 wherein W is an oxygen atom.
[Embodiment 212] The compound in the embodiment 59 wherein W is an oxygen atom.
[Embodiment 213] The compound in the embodiment 60 wherein W is an oxygen atom.
[Embodiment 214] The compound in the embodiment 61 wherein W is an oxygen atom.
[Embodiment 215] The compound in the embodiment 62 wherein W is an oxygen atom.
[Embodiment 216] The compound in the embodiment 63 wherein W is an oxygen atom.
[Embodiment 217] The compound in the embodiment 64 wherein W is an oxygen atom.
[Embodiment 218] The compound in the embodiment 65 wherein W is an oxygen atom.
[Embodiment 219] The compound in the embodiment 66 wherein W is an oxygen atom.
[Embodiment 220] The compound in the embodiment 67 wherein W is an oxygen atom.

[Embodiment 221] The compound in the embodiment 68 wherein W is an oxygen atom.
[Embodiment 222] The compound in the embodiment 69 wherein W is an oxygen atom.
[Embodiment 223] The compound in the embodiment 70 wherein W is an oxygen atom.
[Embodiment 224] The compound in the embodiment 71 wherein W is an oxygen atom.
[Embodiment 225] The compound in the embodiment 72 wherein W is an oxygen atom.
[Embodiment 226] The compound in the embodiment 73 wherein W is an oxygen atom.
[Embodiment 227] The compound in the embodiment 74 wherein W is an oxygen atom.
[Embodiment 228] The compound in the embodiment 75 wherein W is an oxygen atom.
[Embodiment 229] The compound in the embodiment 76 wherein W is an oxygen atom.
[Embodiment 230] The compound in the embodiment 77 wherein W is an oxygen atom.
[Embodiment 231] The compound in the embodiment 78 wherein W is an oxygen atom.
[Embodiment 232] The compound in the embodiment 79 wherein W is an oxygen atom.
[Embodiment 233] The compound in the embodiment 80 wherein W is an oxygen atom.
[Embodiment 234] The compound in the embodiment 81 wherein W is an oxygen atom.
[Embodiment 235] The compound in the embodiment 82 wherein W is an oxygen atom.
[Embodiment 236] The compound in the embodiment 83 wherein W is an oxygen atom.
[Embodiment 237] The compound in the embodiment 84 wherein W is an oxygen atom.
[Embodiment 238] The compound in the embodiment 85 wherein W is an oxygen atom.
[Embodiment 239] The compound in the embodiment 86 wherein W is an oxygen atom.
[Embodiment 240] The compound in the embodiment 87 wherein W is an oxygen atom.
[Embodiment 241] The compound in the embodiment 88 wherein W is an oxygen atom.
[Embodiment 242] The compound in the embodiment 89 wherein W is an oxygen atom.
[Embodiment 243] The compound in the embodiment 90 wherein W is an oxygen atom.
[Embodiment 244] The compound in the embodiment 91 wherein W is an oxygen atom.
[Embodiment 245] The compound in the embodiment 92 wherein W is an oxygen atom.
[Embodiment 246] The compound in the embodiment 93 wherein W is an oxygen atom.
[Embodiment 247] The compound in the embodiment 94 wherein W is an oxygen atom.
[Embodiment 248] The compound in the embodiment 95 wherein W is an oxygen atom.
[Embodiment 249] The compound in the embodiment 96 wherein W is an oxygen atom.
[Embodiment 250] The compound in the embodiment 97 wherein W is an oxygen atom.
[Embodiment 251] The compound in the embodiment 98 wherein W is an oxygen atom.
[Embodiment 252] The compound in the embodiment 99 wherein W is an oxygen atom.
[Embodiment 253] The compound in the embodiment 100 wherein W is an oxygen atom.
[Embodiment 254] The compound in the embodiment 101 wherein W is an oxygen atom.
[Embodiment 255] The compound in the embodiment 102 wherein W is an oxygen atom.
[Embodiment 256] The compound in the embodiment 103 wherein W is an oxygen atom.
[Embodiment 257] The compound in the embodiment 104 wherein W is an oxygen atom.
[Embodiment 258] The compound in the embodiment 105 wherein W is an oxygen atom.
[Embodiment 259] The compound in the embodiment 106 wherein W is an oxygen atom.
[Embodiment 260] The compound in the embodiment 107 wherein W is an oxygen atom.
[Embodiment 261] The compound in the embodiment 108 wherein W is an oxygen atom.
[Embodiment 262] The compound in the embodiment 109 wherein W is an oxygen atom.
[Embodiment 263] The compound in the embodiment 110 wherein W is an oxygen atom.
[Embodiment 264] The compound in the embodiment 111 wherein W is an oxygen atom.
[Embodiment 265] The compound in the embodiment 112 wherein W is an oxygen atom.
[Embodiment 266] The compound in the embodiment 113 wherein W is an oxygen atom.
[Embodiment 267] The compound in the embodiment 114 wherein W is an oxygen atom.
[Embodiment 268] The compound in the embodiment 115 wherein W is an oxygen atom.
[Embodiment 269] The compound in the embodiment 116 wherein W is an oxygen atom.
[Embodiment 270] The compound in the embodiment 117 wherein W is an oxygen atom.
[Embodiment 271] The compound in the embodiment 118 wherein W is an oxygen atom.
[Embodiment 272] The compound in the embodiment 119 wherein W is an oxygen atom.
[Embodiment 273] The compound in the embodiment 120 wherein W is an oxygen atom.
[Embodiment 274] The compound in the embodiment 121 wherein W is an oxygen atom.
[Embodiment 275] The compound in the embodiment 122 wherein W is an oxygen atom.
[Embodiment 276] The compound in the embodiment 123 wherein W is an oxygen atom.
[Embodiment 277] The compound in the embodiment 124 wherein W is an oxygen atom.
[Embodiment 278] The compound in the embodiment 125 wherein W is an oxygen atom.

[Embodiment 279] The compound in the embodiment 126 wherein W is an oxygen atom.
[Embodiment 280] The compound in the embodiment 127 wherein W is an oxygen atom.
[Embodiment 281] The compound in the embodiment 128 wherein W is an oxygen atom.
[Embodiment 282] The compound in the embodiment 129 wherein W is an oxygen atom.
[Embodiment 283] The compound in the embodiment 130 wherein W is an oxygen atom.
[Embodiment 284] The compound in the embodiment 131 wherein W is an oxygen atom.
[Embodiment 285] The compound in the embodiment 132 wherein W is an oxygen atom.
[Embodiment 286] The compound in the embodiment 133 wherein W is an oxygen atom.
[Embodiment 287] The compound in the embodiment 134 wherein W is an oxygen atom.
[Embodiment 288] The compound in the embodiment 135 wherein W is an oxygen atom.
[Embodiment 289] The compound in the embodiment 136 wherein W is an oxygen atom.
[Embodiment 290] The compound in the embodiment 137 wherein W is an oxygen atom.
[Embodiment 291] The compound in the embodiment 138 wherein W is an oxygen atom.
[Embodiment 292] The compound in the embodiment 139 wherein W is an oxygen atom.
[Embodiment 293] The compound in the embodiment 140 wherein W is an oxygen atom.
[Embodiment 294] The compound in the embodiment 141 wherein W is an oxygen atom.
[Embodiment 295] The compound in the embodiment 142 wherein W is an oxygen atom.
[Embodiment 296] The compound in the embodiment 143 wherein W is an oxygen atom.
[Embodiment 297] The compound in the embodiment 144 wherein W is an oxygen atom.
[Embodiment 298] The compound in the embodiment 145 wherein W is an oxygen atom.
[Embodiment 299] The compound in the embodiment 146 wherein W is an oxygen atom.
[Embodiment 300] The compound in the embodiment 147 wherein W is an oxygen atom.
[Embodiment 301] The compound in the embodiment 148 wherein W is an oxygen atom.
[Embodiment 302] The compound in the embodiment 149 wherein W is an oxygen atom.
[Embodiment 303] The compound in the embodiment 150 wherein W is an oxygen atom.
[Embodiment 304] The compound in the embodiment 151 wherein W is an oxygen atom.
[Embodiment 305] The compound in the embodiment 152 wherein W is an oxygen atom.
[Embodiment 306] The compound in the embodiment 153 wherein W is an oxygen atom.
[Embodiment 307] The compound in the embodiment 154 wherein W is an oxygen atom.
[Embodiment 308] The compound in the embodiment 155 wherein W is an oxygen atom.
[Embodiment 309] The compound in the embodiment 156 wherein W is an oxygen atom.
[Embodiment 310] The compound in the embodiment 1 wherein Het represents a group represented by formula Het1 or a group represented by formula Het2.
[Embodiment 311] The compound in the embodiment 2 wherein Het represents a group represented by formula Het1 or a group represented by formula Het2.
[Embodiment 312] The compound in the embodiment 3 wherein Het represents a group represented by formula Het1 or a group represented by formula Het2.
[Embodiment 313] The compound in the embodiment 4 wherein Het represents a group represented by formula Het1 or a group represented by formula Het2.
[Embodiment 314] The compound in the embodiment 5 wherein Het represents a group represented by formula Het1 or a group represented by formula Het2.
[Embodiment 315] The compound in the embodiment 6 wherein Het represents a group represented by formula Het1 or a group represented by formula Het2.
[Embodiment 316] The compound in the embodiment 7 wherein Het represents a group represented by formula Het1 or a group represented by formula Het2.
[Embodiment 317] The compound in the embodiment 8 wherein Het represents a group represented by formula Het1 or a group represented by formula Het2.
[Embodiment 318] The compound in the embodiment 9 wherein Het represents a group represented by formula Het1 or a group represented by formula Het2.
[Embodiment 319] The compound in the embodiment 10 wherein Het represents a group represented by formula Het1 or a group represented by formula Het2.
[Embodiment 320] The compound in the embodiment 11 wherein Het represents a group represented by formula Het1 or a group represented by formula Het2.
[Embodiment 321] The compound in the embodiment 12 wherein Het represents a group represented by formula Het1 or a group represented by formula Het2.
[Embodiment 322] The compound in the embodiment 13 wherein Het represents a group represented by formula Het1 or a group represented by formula Het2.
[Embodiment 323] The compound in the embodiment 14 wherein Het represents a group represented by formula Het1

or a group represented by formula Het2.

[Embodiment 324] The compound in the embodiment 15 wherein Het represents a group represented by formula Het1 or a group represented by formula Het2.

[Embodiment 325] The compound in the embodiment 16 wherein Het represents a group represented by formula Het1 or a group represented by formula Het2.

[Embodiment 326] The compound in the embodiment 17 wherein Het represents a group represented by formula Het1 or a group represented by formula Het2.

[Embodiment 327] The compound in the embodiment 18 wherein Het represents a group represented by formula Het1 or a group represented by formula Het2.

[Embodiment 328] The compound in the embodiment 19 wherein Het represents a group represented by formula Het1 or a group represented by formula Het2.

[Embodiment 329] The compound in the embodiment 20 wherein Het represents a group represented by formula Het1 or a group represented by formula Het2.

[Embodiment 330] The compound in the embodiment 21 wherein Het represents a group represented by formula Het1 or a group represented by formula Het2.

[Embodiment 331] The compound in the embodiment 22 wherein Het represents a group represented by formula Het1 or a group represented by formula Het2.

[Embodiment 332] The compound in the embodiment 23 wherein Het represents a group represented by formula Het1 or a group represented by formula Het2.

[Embodiment 333] The compound in the embodiment 24 wherein Het represents a group represented by formula Het1 or a group represented by formula Het2.

[Embodiment 334] The compound in the embodiment 25 wherein Het represents a group represented by formula Het1 or a group represented by formula Het2.

[Embodiment 335] The compound in the embodiment 26 wherein Het represents a group represented by formula Het1 or a group represented by formula Het2.

[Embodiment 336] The compound in the embodiment 27 wherein Het represents a group represented by formula Het1 or a group represented by formula Het2.

[Embodiment 337] The compound in the embodiment 28 wherein Het represents a group represented by formula Het1 or a group represented by formula Het2.

[Embodiment 338] The compound in the embodiment 29 wherein Het represents a group represented by formula Het1 or a group represented by formula Het2.

[Embodiment 339] The compound in the embodiment 30 wherein Het represents a group represented by formula Het1 or a group represented by formula Het2.

[Embodiment 340] The compound in the embodiment 31 wherein Het represents a group represented by formula Het1 or a group represented by formula Het2.

[Embodiment 341] The compound in the embodiment 32 wherein Het represents a group represented by formula Het1 or a group represented by formula Het2.

[Embodiment 342] The compound in the embodiment 33 wherein Het represents a group represented by formula Het1 or a group represented by formula Het2.

[Embodiment 343] The compound in the embodiment 34 wherein Het represents a group represented by formula Het1 or a group represented by formula Het2.

[Embodiment 344] The compound in the embodiment 35 wherein Het represents a group represented by formula Het1 or a group represented by formula Het2.

[Embodiment 345] The compound in the embodiment 36 wherein Het represents a group represented by formula Het1 or a group represented by formula Het2.

[Embodiment 346] The compound in the embodiment 37 wherein Het represents a group represented by formula Het1 or a group represented by formula Het2.

[Embodiment 347] The compound in the embodiment 1 wherein Het represents a group represented by formula Het1.

[Embodiment 348] The compound in the embodiment 2 wherein Het represents a group represented by formula Het1.

[Embodiment 349] The compound in the embodiment 3 wherein Het represents a group represented by formula Het1.

[Embodiment 350] The compound in the embodiment 4 wherein Het represents a group represented by formula Het1.

[Embodiment 351] The compound in the embodiment 5 wherein Het represents a group represented by formula Het1.

[Embodiment 352] The compound in the embodiment 6 wherein Het represents a group represented by formula Het1.

[Embodiment 353] The compound in the embodiment 7 wherein Het represents a group represented by formula Het1.

[Embodiment 354] The compound in the embodiment 8 wherein Het represents a group represented by formula Het1.

[Embodiment 355] The compound in the embodiment 9 wherein Het represents a group represented by formula Het1.

[Embodiment 356] The compound in the embodiment 10 wherein Het represents a group represented by formula Het1.

[Embodiment 357] The compound in the embodiment 11 wherein Het represents a group represented by formula Het1.
[Embodiment 358] The compound in the embodiment 12 wherein Het represents a group represented by formula Het1.
[Embodiment 359] The compound in the embodiment 13 wherein Het represents a group represented by formula Het1.
[Embodiment 360] The compound in the embodiment 14 wherein Het represents a group represented by formula Het1.
[Embodiment 361] The compound in the embodiment 15 wherein Het represents a group represented by formula Het1.
[Embodiment 362] The compound in the embodiment 16 wherein Het represents a group represented by formula Het1.
[Embodiment 363] The compound in the embodiment 17 wherein Het represents a group represented by formula Het1.
[Embodiment 364] The compound in the embodiment 18 wherein Het represents a group represented by formula Het1.
[Embodiment 365] The compound in the embodiment 19 wherein Het represents a group represented by formula Het1.
[Embodiment 366] The compound in the embodiment 20 wherein Het represents a group represented by formula Het1.
[Embodiment 367] The compound in the embodiment 21 wherein Het represents a group represented by formula Het1.
[Embodiment 368] The compound in the embodiment 22 wherein Het represents a group represented by formula Het1.
[Embodiment 369] The compound in the embodiment 23 wherein Het represents a group represented by formula Het1.
[Embodiment 370] The compound in the embodiment 24 wherein Het represents a group represented by formula Het1.
[Embodiment 371] The compound in the embodiment 25 wherein Het represents a group represented by formula Het1.
[Embodiment 372] The compound in the embodiment 30 wherein Het represents a group represented by formula Het1.
[Embodiment 373] The compound in the embodiment 31 wherein Het represents a group represented by formula Het1.
[Embodiment 374] The compound in the embodiment 32 wherein Het represents a group represented by formula Het1.
[Embodiment 375] The compound in the embodiment 33 wherein Het represents a group represented by formula Het1.
[Embodiment 376] The compound in the embodiment 37 wherein Het represents a group represented by formula Het1.
[Embodiment 377] The compound in the embodiment 1 wherein Het represents a group represented by formula Het2.
[Embodiment 378] The compound in the embodiment 2 wherein Het represents a group represented by formula Het2.
[Embodiment 379] The compound in the embodiment 3 wherein Het represents a group represented by formula Het2.
[Embodiment 380] The compound in the embodiment 4 wherein Het represents a group represented by formula Het2.
[Embodiment 381] The compound in the embodiment 5 wherein Het represents a group represented by formula Het2.
[Embodiment 382] The compound in the embodiment 6 wherein Het represents a group represented by formula Het2.
[Embodiment 383] The compound in the embodiment 7 wherein Het represents a group represented by formula Het2.
[Embodiment 384] The compound in the embodiment 8 wherein Het represents a group represented by formula Het2.
[Embodiment 385] The compound in the embodiment 9 wherein Het represents a group represented by formula Het2.
[Embodiment 386] The compound in the embodiment 10 wherein Het represents a group represented by formula Het2.
[Embodiment 387] The compound in the embodiment 11 wherein Het represents a group represented by formula Het2.
[Embodiment 388] The compound in the embodiment 12 wherein Het represents a group represented by formula Het2.
[Embodiment 389] The compound in the embodiment 13 wherein Het represents a group represented by formula Het2.
[Embodiment 390] The compound in the embodiment 14 wherein Het represents a group represented by formula

Het2.

[Embodiment 391] The compound in the embodiment 15 wherein Het represents a group represented by formula Het2.

[Embodiment 392] The compound in the embodiment 16 wherein Het represents a group represented by formula Het2.

[Embodiment 393] The compound in the embodiment 17 wherein Het represents a group represented by formula Het2.

[Embodiment 394] The compound in the embodiment 18 wherein Het represents a group represented by formula Het2.

[Embodiment 395] The compound in the embodiment 19 wherein Het represents a group represented by formula Het2.

[Embodiment 396] The compound in the embodiment 26 wherein Het represents a group represented by formula Het2.

[Embodiment 397] The compound in the embodiment 27 wherein Het represents a group represented by formula Het2.

[Embodiment 398] The compound in the embodiment 28 wherein Het represents a group represented by formula Het2.

[Embodiment 399] The compound in the embodiment 29 wherein Het represents a group represented by formula Het2.

[Embodiment 400] The compound in the embodiment 34 wherein Het represents a group represented by formula Het2.

[Embodiment 401] The compound in the embodiment 35 wherein Het represents a group represented by formula Het2.

[Embodiment 402] The compound in the embodiment 36 wherein Het represents a group represented by formula Het2.

[Embodiment 403] The compound in the embodiment 37 wherein Het represents a group represented by formula Het2.

[Embodiment 404] The compound in the embodiment 1 wherein $R^2$ represents an ethyl group, $R^1$ represents a methyl group or an ethyl group, and W is an oxygen atom.

[Embodiment 405] The compound in the embodiment 2 wherein $R^2$ represents an ethyl group, $R^1$ represents a methyl group or an ethyl group, and W is an oxygen atom.

[Embodiment 406] The compound in the embodiment 3 wherein $R^2$ represents an ethyl group, $R^1$ represents a methyl group or an ethyl group, and W is an oxygen atom.

[Embodiment 407] The compound in the embodiment 4 wherein $R^2$ represents an ethyl group, $R^1$ represents a methyl group or an ethyl group, and W is an oxygen atom.

[Embodiment 408] The compound in the embodiment 5 wherein $R^2$ represents an ethyl group, $R^1$ represents a methyl group or an ethyl group, and W is an oxygen atom.

[Embodiment 409] The compound in the embodiment 6 wherein $R^2$ represents an ethyl group, $R^1$ represents a methyl group or an ethyl group, and W is an oxygen atom.

[Embodiment 410] The compound in the embodiment 7 wherein $R^2$ represents an ethyl group, $R^1$ represents a methyl group or an ethyl group, and W is an oxygen atom.

[Embodiment 411] The compound in the embodiment 8 wherein $R^2$ represents an ethyl group, $R^1$ represents a methyl group or an ethyl group, and W is an oxygen atom.

[Embodiment 412] The compound in the embodiment 9 wherein $R^2$ represents an ethyl group, $R^1$ represents a methyl group or an ethyl group, and W is an oxygen atom.

[Embodiment 413] The compound in the embodiment 10 wherein $R^2$ represents an ethyl group, $R^1$ represents a methyl group or an ethyl group, and W is an oxygen atom.

[Embodiment 414] The compound in the embodiment 11 wherein $R^2$ represents an ethyl group, $R^1$ represents a methyl group or an ethyl group, and W is an oxygen atom.

[Embodiment 415] The compound in the embodiment 12 wherein $R^2$ represents an ethyl group, $R^1$ represents a methyl group or an ethyl group, and W is an oxygen atom.

[Embodiment 416] The compound in the embodiment 13 wherein $R^2$ represents an ethyl group, $R^1$ represents a methyl group or an ethyl group, and W is an oxygen atom.

[Embodiment 417] The compound in the embodiment 14 wherein $R^2$ represents an ethyl group, $R^1$ represents a methyl group or an ethyl group, and W is an oxygen atom.

[Embodiment 418] The compound in the embodiment 15 wherein $R^2$ represents an ethyl group, $R^1$ represents a methyl group or an ethyl group, and W is an oxygen atom.

[Embodiment 419] The compound in the embodiment 16 wherein $R^2$ represents an ethyl group, $R^1$ represents a

methyl group or an ethyl group, and W is an oxygen atom.

[Embodiment 420] The compound in the embodiment 20 wherein $R^2$ represents an ethyl group, $R^1$ represents a methyl group or an ethyl group, and W is an oxygen atom.

[Embodiment 421] The compound in the embodiment 21 wherein $R^2$ represents an ethyl group, $R^1$ represents a methyl group or an ethyl group, and W is an oxygen atom.

[Embodiment 422] The compound in the embodiment 22 wherein $R^2$ represents an ethyl group, $R^1$ represents a methyl group or an ethyl group, and W is an oxygen atom.

[Embodiment 423] The compound in the embodiment 23 wherein $R^2$ represents an ethyl group, $R^1$ represents a methyl group or an ethyl group, and W is an oxygen atom.

[Embodiment 424] The compound in the embodiment 24 wherein $R^2$ represents an ethyl group, $R^1$ represents a methyl group or an ethyl group, and W is an oxygen atom.

[Embodiment 425] The compound in the embodiment 25 wherein $R^2$ represents an ethyl group, $R^1$ represents a methyl group or an ethyl group, and W is an oxygen atom.

[Embodiment 426] The compound in the embodiment 26 wherein $R^2$ represents an ethyl group, $R^1$ represents a methyl group or an ethyl group, and W is an oxygen atom.

[Embodiment 427] The compound in the embodiment 27 wherein $R^2$ represents an ethyl group, $R^1$ represents a methyl group or an ethyl group, and W is an oxygen atom.

[Embodiment 428] The compound in the embodiment 28 wherein $R^2$ represents an ethyl group, $R^1$ represents a methyl group or an ethyl group, and W is an oxygen atom.

[Embodiment 429] The compound in the embodiment 29 wherein $R^2$ represents an ethyl group, $R^1$ represents a methyl group or an ethyl group, and W is an oxygen atom.

[Embodiment 430] The compound in the embodiment 30 wherein $R^2$ represents an ethyl group, $R^1$ represents a methyl group or an ethyl group, and W is an oxygen atom.

[Embodiment 431] The compound in the embodiment 31 wherein $R^2$ represents an ethyl group, $R^1$ represents a methyl group or an ethyl group, and W is an oxygen atom.

[Embodiment 432] The compound in the embodiment 32 wherein $R^2$ represents an ethyl group, $R^1$ represents a methyl group or an ethyl group, and W is an oxygen atom.

[Embodiment 433] The compound in the embodiment 33 wherein $R^2$ represents an ethyl group, $R^1$ represents a methyl group or an ethyl group, and W is an oxygen atom.

[Embodiment 434] The compound in the embodiment 34 wherein $R^2$ represents an ethyl group, $R^1$ represents a methyl group or an ethyl group, and W is an oxygen atom.

[Embodiment 435] The compound in the embodiment 35 wherein $R^2$ represents an ethyl group, $R^1$ represents a methyl group or an ethyl group, and W is an oxygen atom.

[Embodiment 436] The compound in the embodiment 36 wherein $R^2$ represents an ethyl group, $R^1$ represents a methyl group or an ethyl group, and W is an oxygen atom.

[Embodiment 437] The compound in the embodiment 37 wherein $R^2$ represents an ethyl group, $R^1$ represents a methyl group or an ethyl group, and W is an oxygen atom.

[Embodiment 438] The compound in the embodiment 38 wherein $R^2$ represents an ethyl group, $R^1$ represents a methyl group or an ethyl group, and W is an oxygen atom.

[Embodiment 439] The compound in the embodiment 39 wherein $R^2$ represents an ethyl group, $R^1$ represents a methyl group or an ethyl group, and W is an oxygen atom.

[Embodiment 440] The compound in the embodiment 40 wherein $R^2$ represents an ethyl group, $R^1$ represents a methyl group or an ethyl group, and W is an oxygen atom.

[Embodiment 441] The compound in the embodiment 1 wherein Het represents a group represented by formula Het1, $R^2$ represents an ethyl group, $R^1$ represents a methyl group or an ethyl group, and W is an oxygen atom.

[Embodiment 442] The compound in the embodiment 2 wherein Het represents a group represented by formula Het1, $R^2$ represents an ethyl group, $R^1$ represents a methyl group or an ethyl group, and W is an oxygen atom.

[Embodiment 443] The compound in the embodiment 3 wherein Het represents a group represented by formula Het1, $R^2$ represents an ethyl group, $R^1$ represents a methyl group or an ethyl group, and W is an oxygen atom.

[Embodiment 444] The compound in the embodiment 4 wherein Het represents a group represented by formula Het1, $R^2$ represents an ethyl group, $R^1$ represents a methyl group or an ethyl group, and W is an oxygen atom.

[Embodiment 445] The compound in the embodiment 5 wherein Het represents a group represented by formula Het1, $R^2$ represents an ethyl group, $R^1$ represents a methyl group or an ethyl group, and W is an oxygen atom.

[Embodiment 446] The compound in the embodiment 6 wherein Het represents a group represented by formula Het1, $R^2$ represents an ethyl group, $R^1$ represents a methyl group or an ethyl group, and W is an oxygen atom.

[Embodiment 447] The compound in the embodiment 7 wherein Het represents a group represented by formula Het1, $R^2$ represents an ethyl group, $R^1$ represents a methyl group or an ethyl group, and W is an oxygen atom.

[Embodiment 448] The compound in the embodiment 8 wherein Het represents a group represented by formula Het1,

$R^2$ represents an ethyl group, $R^1$ represents a methyl group or an ethyl group, and W is an oxygen atom.

[Embodiment 449] The compound in the embodiment 9 wherein Het represents a group represented by formula Het1, $R^2$ represents an ethyl group, $R^1$ represents a methyl group or an ethyl group, and W is an oxygen atom.

[Embodiment 450] The compound in the embodiment 10 wherein Het represents a group represented by formula Het1, $R^2$ represents an ethyl group, $R^1$ represents a methyl group or an ethyl group, and W is an oxygen atom.

[Embodiment 451] The compound in the embodiment 11 wherein Het represents a group represented by formula Het1, $R^2$ represents an ethyl group, $R^1$ represents a methyl group or an ethyl group, and W is an oxygen atom.

[Embodiment 452] The compound in the embodiment 12 wherein Het represents a group represented by formula Het1, $R^2$ represents an ethyl group, $R^1$ represents a methyl group or an ethyl group, and W is an oxygen atom.

[Embodiment 453] The compound in the embodiment 13 wherein Het represents a group represented by formula Het1, $R^2$ represents an ethyl group, $R^1$ represents a methyl group or an ethyl group, and W is an oxygen atom.

[Embodiment 454] The compound in the embodiment 14 wherein Het represents a group represented by formula Het1, $R^2$ represents an ethyl group, $R^1$ represents a methyl group or an ethyl group, and W is an oxygen atom.

[Embodiment 455] The compound in the embodiment 15 wherein Het represents a group represented by formula Het1, $R^2$ represents an ethyl group, $R^1$ represents a methyl group or an ethyl group, and W is an oxygen atom.

[Embodiment 456] The compound in the embodiment 16 wherein Het represents a group represented by formula Het1, $R^2$ represents an ethyl group, $R^1$ represents a methyl group or an ethyl group, and W is an oxygen atom.

[Embodiment 457] The compound in the embodiment 20 wherein Het represents a group represented by formula Het1, $R^2$ represents an ethyl group, $R^1$ represents a methyl group or an ethyl group, and W is an oxygen atom.

[Embodiment 458] The compound in the embodiment 21 wherein Het represents a group represented by formula Het1, $R^2$ represents an ethyl group, $R^1$ represents a methyl group or an ethyl group, and W is an oxygen atom.

[Embodiment 459] The compound in the embodiment 22 wherein Het represents a group represented by formula Het1, $R^2$ represents an ethyl group, $R^1$ represents a methyl group or an ethyl group, and W is an oxygen atom.

[Embodiment 460] The compound in the embodiment 23 wherein Het represents a group represented by formula Het1, $R^2$ represents an ethyl group, $R^1$ represents a methyl group or an ethyl group, and W is an oxygen atom.

[Embodiment 461] The compound in the embodiment 24 wherein Het represents a group represented by formula Het1, $R^2$ represents an ethyl group, $R^1$ represents a methyl group or an ethyl group, and W is an oxygen atom.

[Embodiment 462] The compound in the embodiment 25 wherein Het represents a group represented by formula Het1, $R^2$ represents an ethyl group, $R^1$ represents a methyl group or an ethyl group, and W is an oxygen atom.

[Embodiment 463] The compound in the embodiment 30 wherein Het represents a group represented by formula Het1, $R^2$ represents an ethyl group, $R^1$ represents a methyl group or an ethyl group, and W is an oxygen atom.

[Embodiment 464] The compound in the embodiment 31 wherein Het represents a group represented by formula Het1, $R^2$ represents an ethyl group, $R^1$ represents a methyl group or an ethyl group, and W is an oxygen atom.

[Embodiment 465] The compound in the embodiment 32 wherein Het represents a group represented by formula Het1, $R^2$ represents an ethyl group, $R^1$ represents a methyl group or an ethyl group, and W is an oxygen atom.

[Embodiment 466] The compound in the embodiment 33 wherein Het represents a group represented by formula Het1, $R^2$ represents an ethyl group, $R^1$ represents a methyl group or an ethyl group, and W is an oxygen atom.

[Embodiment 467] The compound in the embodiment 37 wherein Het represents a group represented by formula Het1, $R^2$ represents an ethyl group, $R^1$ represents a methyl group or an ethyl group, and W is an oxygen atom.

[Embodiment 468] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents $CR^{9b}$, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, and $R^{78}$ represents a C1-C6 chain hydrocarbon group which may be optionally substituted with one or more halogen atoms.

[Embodiment 469] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group

represented by formula Het2, $Z^1$ represents a nitrogen atom, $Z^2$ represents $CR^{9b}$, $Z^3$ represents $CR^{9c}$, $Z^4$ represents $CR^{9d}$, $R^{9d}$ represents a C1-C6 chain hydrocarbon group which may be optionally substituted with one or more halogen atoms, a C3-C7 cycloalkyl group which may be optionally substituted with one or more halogen atoms, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, and $R^{78}$ represents a C1-C6 chain hydrocarbon group which may be optionally substituted with one or more halogen atoms.

[Embodiment 470] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents $CR^{9b}$, $Z^3$ represents $CR^{9c}$, $Z^4$ represents a nitrogen atom, $R^{9a}$ represents a C1-C6 chain hydrocarbon group which may be optionally substituted with one or more halogen atoms, a C3-C7 cycloalkyl group which may be optionally substituted with one or more halogen atoms, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, and $R^{78}$ represents a C1-C6 chain hydrocarbon group which may be optionally substituted with one or more halogen atoms.

[Embodiment 471] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents a nitrogen atom, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, a hydrogen atom, or a halogen atom, $R^{9c}$ represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, and $R^{78}$ represents a C1-C6 chain hydrocarbon group which may be optionally substituted with one or more halogen atoms.

[Embodiment 472] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents $CR^{9b}$, $Z^3$ represents a nitrogen atom, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, a hydrogen atom, or a halogen atom, $R^{9b}$ represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, and $R^{78}$ represents a C1-C6 chain hydrocarbon group which may be optionally substituted with one or more halogen atoms.

[Embodiment 473] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents $CR^{9b}$, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may

be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, a hydrogen atom, or a halogen atom, and $R^{72}$ represents a C1-C6 chain hydrocarbon group which may be optionally substituted with one or more halogen atoms.

[Embodiment 474] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents a nitrogen atom, $Z^2$ represents $CR^{9b}$, $Z^3$ represents $CR^{9c}$, $Z^4$ represents $CR^{9d}$, $R^{9d}$ represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, a hydrogen atom, or a halogen atom, $R^{72}$ represents a C1-C6 chain hydrocarbon group which may be optionally substituted with one or more halogen atoms.

[Embodiment 475] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents $CR^{9b}$, $Z^3$ represents $CR^{9c}$, $Z^4$ represents a nitrogen atom, $R^{9a}$ represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, a hydrogen atom, or a halogen atom, and $R^{72}$ represents a C1-C6 chain hydrocarbon group which may be optionally substituted with one or more halogen atoms.

[Embodiment 476] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents a nitrogen atom, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9c}$ represents a C1-C6 chain hydrocarbon group which may be optionally substituted with one or more halogen atoms, $OR^{72}$, a hydrogen atom, or a halogen atom, and $R^{72}$ represents a C1-C6 chain hydrocarbon group which may be optionally substituted with one or more halogen atoms.

[Embodiment 477] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents $CR^{9b}$, $Z^3$ represents a nitrogen atom, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ represents a C1-C6 chain hydrocarbon group which may be optionally substituted with one or more halogen atoms, $OR^{72}$, a hydrogen atom, or a halogen atom, and $R^{72}$ represents a C1-C6 chain hydrocarbon group which may be optionally substituted with one or more halogen atoms.

[Embodiment 478] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents $CR^{9b}$, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a methyl group, a trifluoromethyl group, a difluoromethyl group, a

methoxy group, a trifluoromethoxy group, a difluoromethoxy group, a hydrogen atom, or a halogen atom.

[Embodiment 479] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents a nitrogen atom, $Z^2$ represents $CR^{9b}$, $Z^3$ represents $CR^{9c}$, $Z^4$ represents $CR^{9d}$, $R^{9d}$ represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom.

[Embodiment 480] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents $CR^{9b}$, $Z^3$ represents $CR^{9c}$, $Z^4$ represents a nitrogen atom, $R^{9a}$ represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom.

[Embodiment 481] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents a nitrogen atom, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9c}$ represents a methyl group, a trifluoromethyl group, a difluoromethyl group, a methoxy group, a trifluoromethoxy group, a difluoromethoxy group, a hydrogen atom, or a halogen atom.

[Embodiment 482] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents $CR^{9b}$, $Z^3$ represents a nitrogen atom, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ represents a methyl group, a trifluoromethyl group, a difluoromethyl group, a methoxy group, a trifluoromethoxy group, a difluoromethoxy group, a hydrogen atom, or a halogen atom.

[Embodiment 483] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents $CR^{9b}$, $Z^3$ represents $CR^{9c}$, $Z^4$ represents $CR^{9d}$, $R^{9a}$ and $R^{9d}$ are identical to or different from each other and each represents a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom.

[Embodiment 484] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents a nitrogen atom, $Z^2$ represents $CR^{9b}$, $Z^3$ represents $CR^{9c}$, $Z^4$ represents $CR^{9d}$, $R^{9d}$ represents a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom.

[Embodiment 485] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents $CR^{9b}$, $Z^3$ represents $CR^{9c}$, $Z^4$ represents a nitrogen atom, $R^{9a}$ represents a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom.

[Embodiment 486] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents a nitrogen atom, $Z^3$ represents $CR^{9c}$, $Z^4$ represents $CR^{9d}$, $R^{9a}$ and $R^{9d}$ are identical to or different from each other and each represents a hydrogen atom, or a halogen atom, and $R^{9c}$ represents a methyl group, a trifluoromethyl group, a difluoromethyl group, a methoxy group, a trifluoromethoxy group, a difluoromethoxy group, a hydrogen atom, or a halogen atom.

[Embodiment 487] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents $CR^{9b}$, $Z^3$ represents a nitrogen atom, $Z^4$ represents $CR^{9d}$, $R^{9a}$ and $R^{9d}$ are identical to or different from each other and each represents a hydrogen atom, or a halogen atom, $R^{9b}$ represents a methyl group, a trifluoromethyl group, a difluoromethyl group, a methoxy group, a trifluoromethoxy group, a difluoromethoxy group, a hydrogen atom, or a halogen atom.

[Embodiment 488] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents $CR^{9a}$, $Z^2$ represents $CR^{9b}$, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, and $R^{78}$ represents a C1-C6 chain hydrocarbon group which may be optionally substituted with one or more halogen atoms.

[Embodiment 489] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1 or a group represented by formula Het2, $Z^1$ represents a nitrogen atom, $Z^2$ represents $CR^{9b}$, $Z^3$ represents $CR^{9c}$, $Z^4$ represents $CR^{9d}$, $R^{9d}$ represents a C1-C6 chain hydrocarbon group which may be optionally substituted with one or more halogen atoms, a C3-C7 cycloalkyl group which may be optionally substituted with one or more halogen atoms, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, and $R^{78}$ represents a C1-C6 chain hydrocarbon group which may be optionally substituted with one or more halogen atoms.

[Embodiment 490] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents $CR^{9b}$, $Z^3$ represents $CR^{9c}$, $Z^4$ represents a nitrogen atom, $R^{9a}$ represents a C1-C6 chain hydrocarbon

group which may be optionally substituted with one or more halogen atoms, a C3-C7 cycloalkyl group which may be optionally substituted with one or more halogen atoms, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, and $R^{78}$ represents a C1-C6 chain hydrocarbon group which may be optionally substituted with one or more halogen atoms.

[Embodiment 491] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents a nitrogen atom, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, a hydrogen atom, or a halogen atom, $R^{9c}$ represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, and $R^{78}$ represents a C1-C6 chain hydrocarbon group which may be optionally substituted with one or more halogen atoms.

[Embodiment 492] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents $CR^{9b}$, $Z^3$ represents a nitrogen atom, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, a hydrogen atom, or a halogen atom, $R^{9b}$ represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, and $R^{78}$ represents a C1-C6 chain hydrocarbon group which may be optionally substituted with one or more halogen atoms.

[Embodiment 493] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents $CR^{9b}$, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, a hydrogen atom, or a halogen atom, and $R^{72}$ represents a C1-C6 chain hydrocarbon group which may be optionally substituted with one or more halogen atoms.

[Embodiment 494] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents a nitrogen atom, $Z^2$ represents $CR^{9b}$, $Z^3$ represents $CR^{9c}$, $Z^4$ represents $CR^{9d}$, $R^{9d}$ represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, a hydrogen atom, or a halogen atom, and $R^{72}$ represents a C1-C6 chain hydrocarbon group which may be optionally substituted with one or more halogen atoms.

[Embodiment 495] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents $CR^{9b}$, $Z^3$ represents $CR^{9c}$, $Z^4$ represents a nitrogen atom, $R^{9a}$ represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, a hydrogen atom, or a halogen atom, and $R^{72}$ represents a C1-C6 chain hydrocarbon group which may be optionally substituted with one or more halogen atoms.

[Embodiment 496] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents a nitrogen atom, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9c}$ represents a C1-C6 chain hydrocarbon group which may be optionally substituted with one or more halogen atoms, $OR^{72}$, a hydrogen atom, or a halogen atom, and $R^{72}$ represents a C1-C6 chain hydrocarbon group which may be optionally substituted with one or more halogen atoms.

[Embodiment 497] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents $CR^{9b}$, $Z^3$ represents a nitrogen atom, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ represents a C1-C6 chain hydrocarbon group which may be optionally substituted with one or more halogen atoms, $OR^{72}$, a hydrogen atom, or a halogen atom, and $R^{72}$ represents a C1-C6 chain hydrocarbon group which may be optionally substituted with one or more halogen atoms.

[Embodiment 498] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents $CR^{9b}$, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ an $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom and $R^{72}$ represents a C1-C6 chain hydrocarbon group which may be optionally substituted with one or more halogen atoms.

[Embodiment 499] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents a nitrogen atom, $Z^2$ represents $CR^{9b}$, $Z^3$ represents $CR^{9c}$, $Z^4$ represents $CR^{9d}$, $R^{9d}$ represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom and $R^{72}$ represents a C1-C6 chain hydrocarbon group which may be optionally substituted with one or more halogen atoms.

[Embodiment 500] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents $CR^{9b}$, $Z^3$ represents $CR^{9c}$, $Z^4$ represents a nitrogen atom, $R^{9a}$ represents a C1-C6 chain hydrocarbon group which may be optionally substituted with one or more halogen atoms, a C3-C7 cycloalkyl group which may be

optionally substituted with one or more halogen atoms, $OR^{72}$, a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom and $R^{72}$ represents a C1-C6 chain hydrocarbon group which may be optionally substituted with one or more halogen atoms.

[Embodiment 501] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents a nitrogen atom, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, and $R^{9c}$ represents a methyl group, a trifluoromethyl group, a difluoromethyl group, a methoxy group, a trifluoromethoxy group, a difluoromethoxy group, a hydrogen atom, or a halogen atom and $R^{72}$ represents a C1-C6 chain hydrocarbon group which may be optionally substituted with one or more halogen atoms.

[Embodiment 502] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents $CR^{9b}$, $Z^3$ represents a nitrogen atom, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ represents a methyl group, a trifluoromethyl group, a difluoromethyl group, a methoxy group, a trifluoromethoxy group, a difluoromethoxy group, a hydrogen atom, or a halogen atom and $R^{72}$ represents a C1-C6 chain hydrocarbon group which may be optionally substituted with one or more halogen atoms.

[Embodiment 503] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents $CR^{9b}$, $Z^3$ represents $CR^{9c}$, $Z^4$ represents $CR^{9d}$, $R^{9a}$ and $R^{9d}$ are identical to or different from each other and each represents a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom.

[Embodiment 504] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents a nitrogen atom, $Z^2$ represents $CR^{9b}$, $Z^3$ represents $CR^{9c}$, $Z^4$ represents $CR^{9d}$, $R^{9d}$ represents a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom.

[Embodiment 505] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents $CR^{9b}$, $Z^3$ represents $CR^{9c}$, $Z^4$ represents a nitrogen atom, $R^{9a}$ represents a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom.

[Embodiment 506] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents a nitrogen atom, $Z^3$ represents $CR^{9c}$, $Z^4$ represents $CR^{9d}$, $R^{9a}$ and $R^{9d}$ are identical to or different from each other and each represents a hydrogen atom, or a halogen atom, and $R^{9c}$ represents a methyl group, a trifluoromethyl group, a difluoromethyl group, a methoxy group, a trifluoromethoxy group, a difluoromethoxy group, a

hydrogen atom, or a halogen atom.

[Embodiment 507] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents $CR^{9b}$, $Z^3$ represents a nitrogen atom, $Z^4$ represents $CR^{9d}$, $R^{9a}$ and $R^{9d}$ are identical to or different from each other and each represents a hydrogen atom, or a halogen atom, $R^{9b}$ represents a methyl group, a trifluoromethyl group, a difluoromethyl group, a methoxy group, a trifluoromethoxy group, a difluoromethoxy group, a hydrogen atom, or a halogen atom.

[Embodiment 508] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents $CR^{9b}$, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, and $R^{78}$ represents a C1-C6 chain hydrocarbon group which may be optionally substituted with one or more halogen atoms.

[Embodiment 509] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents a nitrogen atom, $Z^2$ represents $CR^{9b}$, $Z^3$ represents $CR^{9c}$, $Z^4$ represents $CR^{9d}$, $R^{9d}$ represents a C1-C6 chain hydrocarbon group which may be optionally substituted with one or more halogen atoms, a C3-C7 cycloalkyl group which may be optionally substituted with one or more halogen atoms, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, and $R^{78}$ represents a C1-C6 chain hydrocarbon group which may be optionally substituted with one or more halogen atoms.

[Embodiment 510] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents $CR^{9b}$, $Z^3$ represents $CR^{9c}$, $Z^4$ represents a nitrogen atom, $R^{9a}$ represents a C1-C6 chain hydrocarbon group which may be optionally substituted with one or more halogen atoms, a C3-C7 cycloalkyl group which may be optionally substituted with one or more halogen atoms, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, $R^{78}$ represents a C1-C6 chain hydrocarbon group which may be optionally substituted with one or more halogen atoms.

[Embodiment 511] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents a nitrogen atom, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, a

hydrogen atom, or a halogen atom, $R^{9c}$ represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, $R^{78}$ represents a C1-C6 chain hydrocarbon group which may be optionally substituted with one or more halogen atoms.

[Embodiment 512] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents $CR^{9b}$, $Z^3$ represents a nitrogen atom, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, a hydrogen atom, or a halogen atom, $R^{9b}$ represents a C1-C6 chain hydrocarbon group which may be optionally substituted with one or more halogen atoms, $OR^{72}$, $S(O)_wR^{78}$, a cyano group, a nitro group, a hydrogen atom, or a halogen atom, $R^{72}$ represents a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a hydrogen atom, or $S(O)_2R^{78}$, w is 0, 1, or 2, and $R^{78}$ represents a C1-C6 chain hydrocarbon group which may be optionally substituted with one or more halogen atoms.

[Embodiment 513] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents $CR^{9b}$, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, a hydrogen atom, or a halogen atom, and $R^{72}$ represents a C1-C6 chain hydrocarbon group which may be optionally substituted with one or more halogen atoms.

[Embodiment 514] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents a nitrogen atom, $Z^2$ represents $CR^{9b}$, $Z^3$ represents $CR^{9c}$, $Z^4$ represents $CR^{9d}$, $R^{9d}$ represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, a hydrogen atom, or a halogen atom, and $R^{72}$ represents a C1-C6 chain hydrocarbon group which may be optionally substituted with one or more halogen atoms.

[Embodiment 515] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents $CR^{9b}$, $Z^3$ represents $CR^{9c}$, $Z^4$ represents a nitrogen atom, $R^{9a}$ represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a C1-C6 chain hydrocarbon group which may be optionally substituted with one or more halogen atoms, $OR^{72}$, a hydrogen atom, or a halogen atom, and $R^{72}$ represents a C1-C6 chain hydrocarbon group which may be optionally substituted with one or more halogen atoms.

[Embodiment 516] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents a nitrogen atom, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or

more halogen atoms, a C3-C7 cycloalkyl group which may be optionally substituted with one or more halogen atoms, $OR^{72}$, a hydrogen atom, or a halogen atom, $R^{9c}$ represents a C1-C6 chain hydrocarbon group which may be optionally substituted with one or more halogen atoms, $OR^{72}$, a hydrogen atom, or a halogen atom, and $R^{72}$ represents a C1-C6 chain hydrocarbon group which may be optionally substituted with one or more halogen atoms.

[Embodiment 517] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents $CR^{9b}$, $Z^3$ represents a nitrogen atom, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ represents a C1-C6 chain hydrocarbon group which may be optionally substituted with one or more halogen atoms, $OR^{72}$, a hydrogen atom, or a halogen atom, and $R^{72}$ represents a C1-C6 chain hydrocarbon group which may be optionally substituted with one or more halogen atoms.

[Embodiment 518] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents $CR^{9b}$, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom and $R^{72}$ represents a C1-C6 chain hydrocarbon group which may be optionally substituted with one or more halogen atoms.

[Embodiment 519] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents a nitrogen atom, $Z^2$ represents $CR^{9b}$, $Z^3$ represents $CR^{9c}$, $Z^4$ represents $CR^{9d}$, $R^{9d}$ represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom and $R^{72}$ represents a C1-C6 chain hydrocarbon group which may be optionally substituted with one or more halogen atoms.

[Embodiment 520] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents $CR^{9b}$, $Z^3$ represents $CR^{9c}$, $Z^4$ represents a nitrogen atom, $R^{9a}$ represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom and $R^{72}$ represents a C1-C6 chain hydrocarbon group which may be optionally substituted with one or more halogen atoms.

[Embodiment 521] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents a nitrogen atom, $Z^3$ represents $CR^{9c}$, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, and $R^{9c}$ represents a methyl group, a trifluoromethyl group, a difluoromethyl group, a methoxy group, a trifluoromethoxy group, a difluoromethoxy group, a hydrogen atom, or a halogen atom and $R^{72}$ represents a C1-C6 chain hydrocarbon group which may be optionally substituted with one or more halogen atoms.

[Embodiment 522] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents $CR^{9b}$, $Z^3$ represents a nitrogen atom, $Z^4$ represents $CR^{9d}$, $R^{9a}$ and $R^{9d}$ are identical to or different from each other and each represents a C1-C6 chain hydrocarbon group which may be optionally substituted with one or more halogen atoms, a C3-C7 cycloalkyl group which may be optionally substituted with one or more halogen atoms, $OR^{72}$, a hydrogen atom, or a halogen atom, $R^{9b}$ represents a methyl group, a trifluoromethyl group, a difluoromethyl group, a methoxy group, a trifluoromethoxy group, a difluoromethoxy group, a hydrogen atom, or a halogen atom and $R^{72}$ represents a C1-C6 chain hydrocarbon group which may be optionally substituted with one or more halogen atoms.

[Embodiment 523] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents $CR^{9b}$, $Z^3$ represents $CR^{9c}$, $Z^4$ represents $CR^{9d}$, $R^{9a}$ and $R^{9d}$ are identical to or different from each other and each represents a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom.

[Embodiment 524] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents a nitrogen atom, $Z^2$ represents $CR^{9b}$, $Z^3$ represents $CR^{9c}$, $Z^4$ represents $CR^{9d}$, $R^{9d}$ represents a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom.

[Embodiment 525] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents $CR^{9b}$, $Z^3$ represents $CR^{9c}$, $Z^4$ represents a nitrogen atom, $R^{9a}$ represents a hydrogen atom, or a halogen atom, $R^{9b}$ and $R^{9c}$ are identical to or different from each other and each represents a methyl group, a trifluoromethyl group, a difluoromethyl group, a methoxy group, a trifluoromethoxy group, a difluoromethoxy group, a hydrogen atom, or a halogen atom.

[Embodiment 526] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents a nitrogen atom, $Z^3$ represents $CR^{9c}$, $Z^4$ represents $CR^{9d}$, $R^{9a}$ and $R^{9d}$ are identical to or different from each other and each represents a hydrogen atom, or a halogen atom, $R^{9c}$ represents a methyl group, a trifluoromethyl group, a difluoromethyl group, a methoxy group, a trifluoromethoxy group, a difluoromethoxy group, a hydrogen atom, or a halogen atom.

[Embodiment 527] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het2, $Z^1$ represents $CR^{9a}$, $Z^2$ represents $CR^{9b}$, $Z^3$ represents a nitrogen atom, $Z^4$ represents $CR^{9d}$, $R^{9a}$ and $R^{9d}$ are identical to or different from each other and each represents a hydrogen atom, or a halogen atom, $R^{9b}$ represents a methyl group, a trifluoromethyl group, a difluoromethyl group, a methoxy group, a trifluoromethoxy group, a difluoromethoxy group, a hydrogen atom, or a halogen atom.

[Embodiment 528] Any of the compound in the embodiments 1 to 37, the embodiments 41 to 76, the embodiments 80 to 114, the embodiments 118 to 153, the embodiments 157 to 190, the embodiments 194 to 229, the embodiments 233 to 267, the embodiments 271 to 306, the embodiments 310 to 437, the embodiments 441 to 467, the embodiments 529 to 619, or the present compound N wherein Het represents a group represented by formula Het1, $Z^1$ represents $CR^{9a}$, $Z^2$ represents $CR^{9b}$, $Z^3$ represents $CR^{9c}$, $Z^4$ represents $CR^{9d}$, $R^{9a}$ and $R^{9d}$ are identical to or different from each other and each represents a hydrogen atom or a halogen atom, $R^{9b}$ and $R^{9c}$ are identical to or different from each other and each represents a C1-C6 chain hydrocarbon group which may be optionally substituted with one or more halogen

atoms, a hydrogen atom, or a halogen atom.

[Embodiment 530] The present compound N wherein Q represents a group represented by formula Q1 or a group represented by formula Q5, $R^{3a}$, $R^{3c}$, $R^{10a}$ and $R^{10d}$ represent a hydrogen atom, $R^{3b}$, $R^{3d}$, $R^{10b}$ and $R^{10c}$ are identical to or different from each other and each represents a C1-C6 chain hydrocarbon group which may be optionally substituted with one or more cyano groups, a C3-C7 cycloalkyl group which may be optionally substituted with one or more cyano groups, a hydrogen atom, or a halogen atom.

[Embodiment 531] The present compound N wherein Q represents a group represented by formula Q1, $R^{3a}$ and $R^{3c}$ represent a hydrogen atom, $R^{3b}$ and $R^{3d}$ are identical to or different from each other and each represents a C1-C6 chain hydrocarbon group which may be optionally substituted with one or more cyano groups, or a C3-C7 cycloalkyl group which may be optionally substituted with one or more cyano groups, a hydrogen atom, or a halogen atom.

[Embodiment 532] The present compound N wherein Q represents a group represented by formula Q1, $A^1$ represents a nitrogen atom, $R^{3a}$, $R^{3c}$ and $R^{3d}$ represent a hydrogen atom, $R^{3b}$ represents a C3-C7 cycloalkyl group which may be optionally substituted with one or more substituents selected from a group consisting of a halogen and a cyano group, a hydrogen atom, or a halogen atom.

[Embodiment 533] The present compound N wherein Q represents a group represented by formula Q5, $G^1$ represents a nitrogen atom or $CR^{10a}$, $G^2$ represents $CR^{10b}$, $G^3$ represents $CR^{10c}$, $G^4$ represents $CR^{10d}$, $R^{10a}$ and $R^{10d}$ represent a hydrogen atom, $R^{10b}$ and $R^{10c}$ are identical to or different from each other and each represents a C1-C6 chain hydrocarbon group which may be optionally substituted with one or more cyano groups, a C3-C7 cycloalkyl group which may be optionally substituted with one or more cyano groups, a hydrogen atom, or a halogen atom.

[Embodiment 535] The present compound N wherein Q represents a group represented by formula Q5, $G^1$ represents $CR^{10a}$, $G^2$ represents $CR^{10b}$, $G^3$ represents $CR^{10c}$, $G^4$ represents $CR^{10d}$, $R^{10a}$, $R^{10c}$ and $R^{10d}$ represent a hydrogen atom, and $R^{10b}$ represents a C3-C7 cycloalkyl group which may be optionally substituted with one or more cyano groups, or a halogen atom.

[Embodiment 537] The compound in the embodiment 530 wherein $R^2$ represents an ethyl group.

[Embodiment 538] The compound in the embodiment 531 wherein $R^2$ represents an ethyl group.

[Embodiment 539] The compound in the embodiment 532 wherein $R^2$ represents an ethyl group.

[Embodiment 540] The compound in the embodiment 533 wherein $R^2$ represents an ethyl group.

[Embodiment 542] The compound in the embodiment 535 wherein $R^2$ represents an ethyl group.

[Embodiment 544] The compound in the embodiment 530 wherein $R^1$ represents a methyl group, an ethyl group, or a cyclopropylmethyl group.

[Embodiment 545] The compound in the embodiment 531 wherein $R^1$ represents a methyl group, an ethyl group, or a cyclopropylmethyl group.

[Embodiment 546] The compound in the embodiment 532 wherein $R^1$ represents a methyl group, an ethyl group, or a cyclopropylmethyl group.

[Embodiment 547] The compound in the embodiment 533 wherein $R^1$ represents a methyl group, an ethyl group, or a cyclopropylmethyl group.

[Embodiment 549] The compound in the embodiment 535 wherein $R^1$ represents a methyl group, an ethyl group, or a cyclopropylmethyl group.

[Embodiment 551] The compound in the embodiment 537 wherein $R^1$ represents a methyl group, an ethyl group, or a cyclopropylmethyl group.

[Embodiment 552] The compound in the embodiment 538 wherein $R^1$ represents a methyl group, an ethyl group, or a cyclopropylmethyl group.

[Embodiment 553] The compound in the embodiment 539 wherein $R^1$ represents a methyl group, an ethyl group, or a cyclopropylmethyl group.

[Embodiment 554] The compound in the embodiment 540 wherein $R^1$ represents a methyl group, an ethyl group, or a cyclopropylmethyl group.

[Embodiment 556] The compound in the embodiment 542 wherein $R^1$ represents a methyl group, an ethyl group, or a cyclopropylmethyl group.

[Embodiment 558] The compound in the embodiment 530 wherein W represents an oxygen atom.

[Embodiment 559] The compound in the embodiment 531 wherein W represents an oxygen atom.

[Embodiment 560] The compound in the embodiment 532 wherein W represents an oxygen atom.

[Embodiment 561] The compound in the embodiment 533 wherein W represents an oxygen atom.

[Embodiment 563] The compound in the embodiment 535 wherein W represents an oxygen atom.

[Embodiment 565] The compound in the embodiment 537 wherein W represents an oxygen atom.

[Embodiment 566] The compound in the embodiment 538 wherein W represents an oxygen atom.

[Embodiment 567] The compound in the embodiment 539 wherein W represents an oxygen atom.

[Embodiment 568] The compound in the embodiment 540 wherein W represents an oxygen atom.

[Embodiment 570] The compound in the embodiment 542 wherein W represents an oxygen atom.

[Embodiment 572] The compound in the embodiment 544 wherein W represents an oxygen atom.
[Embodiment 573] The compound in the embodiment 545 wherein W represents an oxygen atom.
[Embodiment 574] The compound in the embodiment 546 wherein W represents an oxygen atom.
[Embodiment 575] The compound in the embodiment 547 wherein W represents an oxygen atom.
[Embodiment 577] The compound in the embodiment 549 wherein W represents an oxygen atom.
[Embodiment 578] The compound in the embodiment 550 wherein W represents an oxygen atom.
[Embodiment 579] The compound in the embodiment 551 wherein W represents an oxygen atom.
[Embodiment 580] The compound in the embodiment 552 wherein W represents an oxygen atom.
[Embodiment 581] The compound in the embodiment 553 wherein W represents an oxygen atom.
[Embodiment 582] The compound in the embodiment 554 wherein W represents an oxygen atom.
[Embodiment 583] The compound in the embodiment 555 wherein W represents an oxygen atom.
[Embodiment 584] The compound in the embodiment 556 wherein W represents an oxygen atom.
[Embodiment 586] The compound in the embodiment 530 wherein Het represents a group represented by formula Het1 or a group represented by formula Het2.
[Embodiment 587] The compound in the embodiment 531 wherein Het represents a group represented by formula Het1 or a group represented by formula Het2.
[Embodiment 588] The compound in the embodiment 532 wherein Het represents a group represented by formula Het1 or a group represented by formula Het2.
[Embodiment 589] The compound in the embodiment 533 wherein Het represents a group represented by formula Het1 or a group represented by formula Het2.
[Embodiment 591] The compound in the embodiment 535 wherein Het represents a group represented by formula Het1 or a group represented by formula Het2.
[Embodiment 593] The compound in the embodiment 530 wherein Het represents a group represented by formula Het1.
[Embodiment 594] The compound in the embodiment 531 wherein Het represents a group represented by formula Het1.
[Embodiment 595] The compound in the embodiment 532 wherein Het represents a group represented by formula Het1.
[Embodiment 596] The compound in the embodiment 533 wherein Het represents a group represented by formula Het1.
[Embodiment 598] The compound in the embodiment 535 wherein Het represents a group represented by formula Het1.
[Embodiment 600] The compound in the embodiment 530 wherein Het represents a group represented by formula Het2.
[Embodiment 601] The compound in the embodiment 531 wherein Het represents a group represented by formula Het2.
[Embodiment 602] The compound in the embodiment 532 wherein Het represents a group represented by formula Het2.
[Embodiment 603] The compound in the embodiment 533 wherein Het represents a group represented by formula Het2.
[Embodiment 605] The compound in the embodiment 535 wherein Het represents a group represented by formula Het2.
[Embodiment 607] The compound in the embodiment 530 wherein $R^2$ represents an ethyl group, $R^1$ represents a methyl group or an ethyl group, and W represents an oxygen atom.
[Embodiment 608] The compound in the embodiment 531 wherein $R^2$ represents an ethyl group, $R^1$ represents a methyl group or an ethyl group, and W represents an oxygen atom.
[Embodiment 609] The compound in the embodiment 532 wherein $R^2$ represents an ethyl group, $R^1$ represents a methyl group or an ethyl group, and W represents an oxygen atom.
[Embodiment 610] The compound in the embodiment 533 wherein $R^2$ represents an ethyl group, $R^1$ represents a methyl group or an ethyl group, and W represents an oxygen atom.
[Embodiment 612] The compound in the embodiment 535 wherein $R^2$ represents an ethyl group, $R^1$ represents a methyl group or an ethyl group, and W represents an oxygen atom.
[Embodiment 614] The compound in the embodiment 530 wherein Het represents a group represented by formula Het1, $R^2$ represents an ethyl group, $R^1$ represents a methyl group or an ethyl group, and W represents an oxygen atom.
[Embodiment 615] The compound in the embodiment 531 wherein Het represents a group represented by formula Het1, $R^2$ represents an ethyl group, $R^1$ represents a methyl group or an ethyl group, and W represents an oxygen atom.

[Embodiment 616] The compound in the embodiment 532 wherein Het represents a group represented by formula Het1, $R^2$ represents an ethyl group, $R^1$ represents a methyl group or an ethyl group, and W represents an oxygen atom.

[Embodiment 617] The compound in the embodiment 533 wherein Het represents a group represented by formula Het1, $R^2$ represents an ethyl group, $R^1$ represents a methyl group or an ethyl group, and W represents an oxygen atom.

[Embodiment 619] The compound in the embodiment 535 wherein Het represents a group represented by formula Het1, $R^2$ represents an ethyl group, $R^1$ represents a methyl group or an ethyl group, and W represents an oxygen atom.

Next, a process for preparing the present compound is explained.

Process 1

A compound represented by formula (I-b) (hereinafter, referred to as compound (I-b)) or a compound represented by formula (I-c) (hereinafter, referred to as compound (I-c)) can be prepared by reacting a compound represented by formula (I-a) (hereinafter, compound (I-a)) with an oxidizing agent.

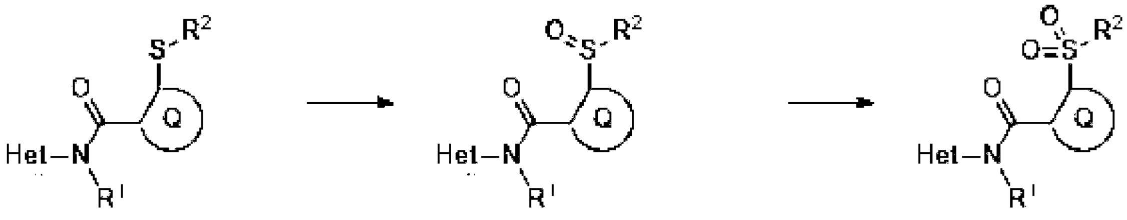

(I-a) (I-b) (I-c)

[wherein the symbols are the same as defined above.]

Firstly, a process for the compound (I-b) from the compound (I-a) is described.

The reaction is usually carried out in a solvent. Examples of the solvent to be used in the reaction include halogenated hydrocarbons such as dichloromethane and chloroform (hereinafter, collectively referred to as halogenated hydrocarbons); nitriles such as acetonitrile (hereinafter collectively referred to nitriles); alcohols such as methanol and ethanol (hereinafter, collectively referred to as alcohols); acetic acid; water; and mixed solvents of two or more kinds of the solvents.

Examples of the oxidizing agent to be used in the reaction include sodium periodate, m-chloroperbenzoic acid (hereinafter, referred to as mCPBA) and hydrogen peroxide.

When hydrogen peroxide is used as an oxidizing agent, a base or a catalyst may be added as needed.

Examples of the base include sodium carbonate.

When the base is used in the reaction, the base is usually used within a range of 0.01 to 1 molar ration(s), as opposed to 1 mole of the compound (I-a).

Examples of the catalyst to be used in the reaction include tungstic acid, and sodium tungstate. When the catalyst is used in the reaction, the catalyst is usually used within a range of 0.01 to 0.5 molar rations, as opposed to a mole of the compound (I-a).

In the reaction, the oxidizing agent is usually used within a range of 1 to 1.2 molar ratio(s), as opposed to 1 mole of the compound (I-a).

The reaction temperature of the reaction is usually within a range of -20 to 80°C. The reaction period of the reaction is usually within a range of 0.1 to 12 hours.

When the reaction is completed, water is added to reaction mixture, and the reaction mixture is extracted with organic solvent(s), and if necessary, the organic layer is washed with an aqueous solution of a reducing agent (such as sodium sulfite, and sodium thiosulfate) and an aqueous solution of a base (such as sodium hydrogen carbonate). The resulting organic layer is dried and concentrated to obtain the compound (I-b).

Next, a process for preparing the compound (I-c) from the compound (I-b) is described.

The reaction is usually carried out in a solvent. Examples of the solvents to be used in the reaction include halogenated hydrocarbons, nitriles, alcohols, acetic acid, water, and mixed solvents of two or more kinds of the solvents.

Examples of the oxidizing agent to be used in the reaction include mCPBA and peroxide hydrogen.

When hydrogen peroxide is used as an oxidizing agent, a base or a catalyst may be added as needed.

Examples of the base to be used in the reaction include sodium carbonate. When the base is used in the reaction,

the base is usually used within a range of 0.01 to 1 molar ration(s), as opposed to 1 mole of the compound (I-b).

Examples of the catalyst to be used in the reaction include sodium tungstate. When the catalyst is used in the reaction, the base is usually used within a range of 0.01 to 0.5 molar rations, as opposed to 1 mole of the compound (I-b).

In the reaction, the oxidizing agent is usually used within a range of 1 to 2 molar ratio(s), as opposed to 1 mole of the compound (I-b).

The reaction temperature of the reaction is usually within a range of -20 to 120°C. The reaction period of the reaction is usually within a range of 0.1 to 12 hours.

When the reaction is completed, water is added to reaction mixture, and the reaction mixture is extracted with organic solvent(s), and if necessary, the organic layer is washed with an aqueous solution of a reducing agent (such as sodium sulfite, and sodium thiosulfate) and an aqueous solution of a base (such as sodium hydrogen carbonate). The resulting organic layer is dried and concentrated to obtain the compound (I-c).

Also, the compound (I-c) can be prepared by reacting the compound (I-a) with an oxidizing agent in one step (one-pot).

The reaction may be carried out by using the oxidizing agent in a ratio of 2 to 5 molar ratios as opposed to 1 mole of the compound (I-a) according to the process for preparing the compound (I-c) from the compound (I-b).

Process 2

A compound represented by formula (I-1-A) (hereinafter, referred to as compound (I-1-A)) can be prepared by reacting a compound represented by formula (M1-1-A) (hereinafter, referred to as compound (M1-1-A)) with a compound represented by formula (M2-1) (hereinafter, referred to as compound (M2-1) in the presence of a condensing agent.

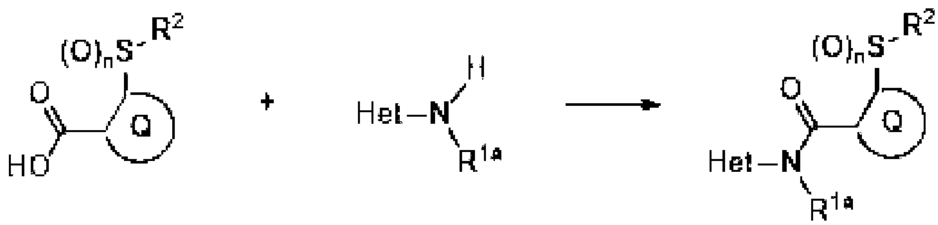

(M1-1-A) (M2-1) (I-1-A)

[wherein $R^{1a}$ represents a C1-C6 chain hydrocarbon group which may be optionally substituted with one or more substituents selected from Group D, or a C3-C7 cycloalkyl group which may be optionally substituted with one or more substituents selected from Group E, and the other symbols are the same as defined above].

The reaction is carried out in the presence or the absence of a solvent.

Examples of the solvent to be used in the reaction include ethers such as tetrahydrofuran (hereinafter, referred to as THF), methyl tert-butyl ether (hereinafter, referred to as MTBE), 1,2-dimethoxyethane (hereinafter, collectively referred to as ethers); halogenated hydrocarbons: aromatic hydrocarbons such as toluene and xylene (hereinafter, referred to as aromatic hydrocarbons); esters such as ethyl acetate and butyl acetate (hereinafter, collectively referred to as esters); nitriles; N-methylpyrrolidone (hereinafter, referred to as NMP); aprotic polar solvents such as N,N-dimethylformamide (hereinafter, referred to as DMF), dimethylsulfoxide (hereinafter, referred to as DMSO) (hereinafter, collectively referred to as aprotic polar solvents); nitrogen-containing aromatic compounds such as pyridine, picoline, lutidine, and quinoline (hereinafter, collectively referred to as nitrogen-containing aromatic compounds); and mixed solvents of two or more kinds of the solvents.

Examples of the condensing agents to be used in the reaction include 1-ethyl-3-[3-(dimethylamino)propyl] carbodiimide hydrochloride salt, and 1,3-dicyclohexylcarbodiimide.

A catalyst may be added in the reaction as needed. Examples of the catalyst include 1-hydroxybenzotriazole. When the catalyst is used in the reaction, the catalyst is usually used within a range of 0.01 to 0.5 molar ratio(s), as opposed to 1 mole of the compound (M1-1-A).

A base may be added in the reaction as needed. Examples of the base to be used in the reaction include organic bases such as triethylamine, N,N-diisopropylethylamine, pyridine, and 4-(dimethylamino)pyridine (hereinafter, collectively referred to as organic bases). When the base is used in the reaction, the base is usually used within a range of 1 to 2 molar ratio(s), as opposed to 1 mole of the compound (M1-1-A) .

In the reaction, the compound (M2-1) is usually used within a range of 1 to 2 molar ratio(s), and the condensing agent is usually used within a range of 1 to 2 molar ratio (s), as opposed to 1 mole of the compound (M1-1-A).

The reaction temperature is usually within a range of 0 to 200°C. The reaction period of the reaction is usually within a range of 0.1 to 12 hours.

When the reaction is completed, water is added to the reaction mixture, and the reaction mixture is extracted with organic solvent(s), and the organic layer is worked up (for example, drying and concentration) to obtain the compound (I-1-A).

The compound (M1-1-A) and the compound (M2-1) are commercially available compounds, or can be prepared according to a well-known method.

Process 3

A compound represented by formula (I-1-A) can be prepared by reacting a compound represented by formula (M1-2-A) (hereinafter, referred to as compound (M1-2-A)) with the compound (M2-1).

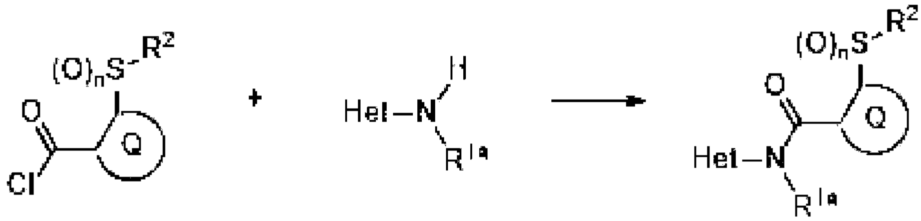

(M1-2-A) (M2-1) (I-1-A)

[wherein the symbols are the same as defined above]

The reaction is usually carried out in a solvent. Examples of the solvents to be used in the reaction include ethers; aliphatic hydrocarbons such as hexane, heptane and octane (hereinafter, collectively referred to as aliphatic hydrocarbons) ; aromatic hydrocarbons; halogenated hydrocarbons; esters; nitriles; aprotic polar solvents, and mixed solvents of two or more kinds of the solvents.

A base may be added in the reaction as needed. Examples of the bases to be used in the reaction include alkali metal carbonates such as sodium carbonate, potassium carbonate, cesium carbonate (hereinafter, collectively referred to as alkali metal carbonates); alkali metal hydrides such as sodium hydride (hereinafter, collectively referred to as alkali metal hydrides); and organic bases. When the base is used in the reaction, the base is usually used within a range of 1 to 2 molar ratio(s), as opposed to 1 mole of the compound (M1-2-A).

In the reaction, the compound (M2-1) is usually used within a range of 0.8 to 1.2 molar ratio(s), as opposed to 1 mole of the compound (M1-2-A).

The reaction temperature is usually within a range of -20 to 200°C. The reaction period of the reaction is usually within a range of 0.1 to 24 hours.

When the reaction is completed, water is added to the reaction mixture, and the reaction mixture is extracted with organic solvent(s), and the organic layer are worked up (for example, drying and concentration) to obtain the compound (I-1-A).

The compound (M1-2-A) is a commercially available compound, or can be prepared according to a well-known method.

Process 4

A compound represented by formula (I-1-B) (hereinafter, referred to as compound (I-1-B)) can be prepared by reacting a compound represented by formula (M1-3-A) (hereinafter, referred to as compound (M1-3-A) with a compound represented by formula (R-1) (hereinafter, referred to as compound (R-1)) in the presence of a base.

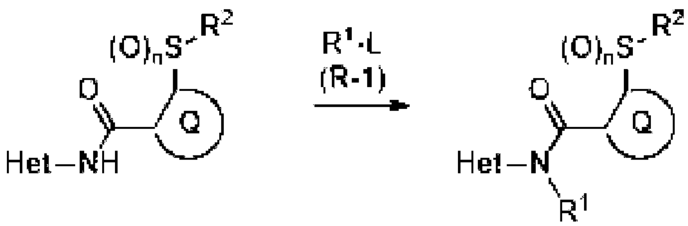


(M1-3-A) (I-1-B)

[wherein L represents a leaving group such as a chlorine atom, a bromine atom, an iodine atom, and the other symbols are

the same as defined above]

The reaction is usually carried out in a solvent. Examples of the solvents to be used in the reaction include ethers; aromatic hydrocarbons; nitriles; aprotic polar solvents; and mixed solvents of two or more kinds of the solvents.

Examples of the base include alkali metal hydrides, alkali metal carbonates, and organic bases.

In the reaction, the compound (R-1) is usually used within a range of 1 to 5 molar ratio(s), and the base is usually used within a range of 1 to 2 molar ratio(s), as opposed to 1 mole of the compound (M1-3-A).

The reaction temperature is usually within a range of 0 to 100°C. The reaction period of the reaction is usually within a range of 0.1 to 24 hours.

When the reaction is completed, water is added to the reaction mixture, and the reaction mixture is extracted with organic solvent(s), and the organic layer is worked up (for example, drying and concentration) to obtain the compound (I-1-B).

The compound (R-1) is a commercially available compound, or can be prepared according to a well-known method.

Process 5

A compound (I-1-B) can be prepared by reacting a compound represented by formula (M1-4-A) (hereinafter, referred to as compound (M1-4-A)) with a compound represented by formula (R-2) (hereinafter, referred to as compound (R-2)).

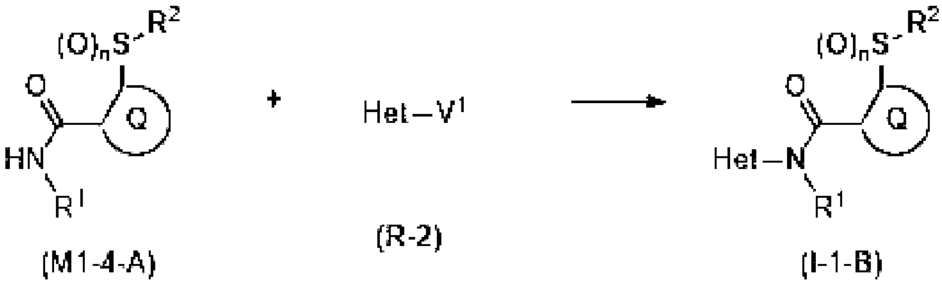

(M1-4-A) (R-2) (I-1-B)

[wherein $V^1$ represents a halogen atom, and the other symbols are the same as defined above]

When $V^1$ represents a fluorine atom, the compound (I-1-B) can be prepared by reacting the compound (M1-4-A) with the compound (R-2) in the presence of a base.

The reaction is usually carried out in a solvent. Examples of the solvents to be used in the reaction include ethers, aromatic hydrocarbons, nitriles, aprotic polar solvents, water, and mixed solvents of two or more kinds of the solvents.

Examples of the bases to be used in the reaction include alkali metal carbonates, alkali metal hydrides.

In the reaction, the compound (R-2) is usually used within a range of 0.8 to 1.2 molar ratio(s), and the base is usually used within a range of 1 to 2 molar ratio(s), as opposed to 1 mole of the compound (M1-4-A).

The reaction temperature is usually within a range of 0 to 200°C. The reaction period of the reaction is usually within a range of 0.5 to 24 hours.

When the reaction is completed, water is added to the reaction mixture, and the reaction mixture is extracted with organic solvent(s), and the organic layer is worked up (for example, drying and concentration) to give the compound (I-1-B).

When $V^1$ represents a chlorine atom, a bromine atom, or an iodine atom, the compound (I-1-B) can be prepared by reacting the compound (M1-4-A) with the compound (R-2) in the presence of the base and the metal catalyst.

The reaction is usually carried out in a solvent. Examples of the solvents to be used in the reaction include ethers, aromatic hydrocarbons, nitriles, aprotic polar solvents, water, and mixed solvents of two or more kinds of the solvents.

Examples of the bases to be used in the reaction include alkali metal carbonates; phosphates such as trisodium phosphate and tripotassium phosphate; alkali metal hydrides; organic bases; and cyclic amines such as 1,4-diazabicyclo [2,2,2]octane and diazabicyloundecene.

Examples of the metal catalysts to be used in the reaction include a cupper catalyst and a palladium catalyst. Examples of the cupper catalyst include cupper(I) iodide, cupper(I) bromide, cupper(I) chloride, and cupper(I) oxide. The copper catalyst is used in the reaction, the copper catalyst is usually used within a range of 0.01 to 0.5 molar ratios, as opposed to 1 mole of the compound (M1-4-A). Examples of the palladium catalyst include palladium catalysts such as palladium(II) acetate, and tris(dibenzylideneacetone)dipalladium(0). The palladium catalyst is used in the reaction, the palladium catalyst is usually used within a range of 0.01 to 0.2 molar ratios, as opposed to 1 mole of the compound (M1-4-A).

In the reaction, as needed, a ligand may be used. Examples of the ligand to be used in the reaction include

acetylacetone, salen, phenanthroline, triphenylphosphine, 4,5-bis(dihenylphoshino)-9,9-dimethylxanthene. When the ligand is used in the reaction, the ligand is used within a range of 0.01 to 0.5 molar ratios, as 1 mole of the compound (M1-4-A). In the reaction, the compound (R-2) is usually used within a range of 0.8 to 1.2 molar ratios, and the base is usually used within a range of 1 to 2 molar ratios, as 1 mole of the compound (M1-4-A).

The reaction temperature is usually within a range of 0 to 200°C. The reaction period of the reaction is usually within a range of 0.5 to 24 hours.

When the reaction is completed, water is added to the reaction mixture, and the reaction mixture is extracted with organic solvent(s), and the organic layer is worked up (for example, drying and concentration) to obtain the compound (I-1-B).

The compound (R-2) is a commercially available compound, or can be prepared according to a well-known method.

Process 6

The compound (I-a) can be prepared according to the scheme below.

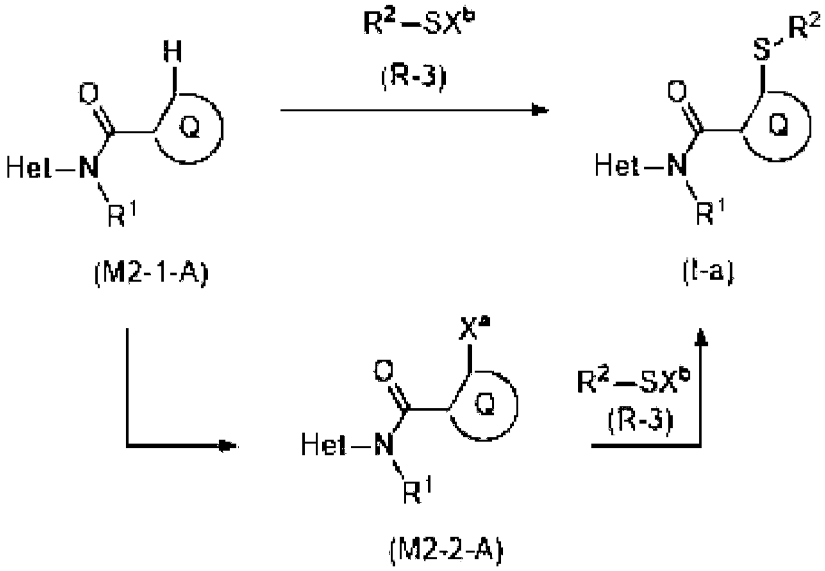


[wherein $X^a$ represents a chlorine atom, a bromine atom, or an iodine atom, $X^b$ represents $SR^2$ or a hydrogen atom, and the other symbols are the same as defined above]

Firstly, a step of preparing a compound represented by formula (M2-2-A) (hereinafter, referred to as compound (M2-2-A) from a compound represented by formula (M2-1-A) (hereinafter, referred to as compound (M2-1-A)) (hereinafter, the step is referred to as step 6-A).

The compound (M2-2-A) can be prepared by reacting the compound (M2-1-A) with a halogenating agent.

The reaction is usually carried out in a solvent. Examples of the solvent to be used in the reaction include alcohols; nitriles; ethers; aromatic hydrocarbons; aprotic polar solvents, halogenated hydrocarbons; water; mixed solvents of two or more kinds of the solvents.

Examples of the halogenating agent include chlorine, bromine, iodine, N-chlorosuccinimide, N-bromosuccinimide, and N-iodosuccinimide.

In the reaction, the halogenating agent is usually used within a range of 1 to 20 molar ratio(s), as opposed to 1 mole of the compound (M2-1-A).

The reaction temperature of the reaction is usually within a range of -20 to 200°C. The reaction period of the reaction is usually within a range of 0.1 to 72 hours.

When the reaction is completed, water is added to the reaction mixture, and the reaction mixture is extracted with organic solvent(s), and the organic layer is worked up (for example, drying and concentration) to give the compound (M2-2-A).

The compound (M2-1-A) is a commercially available compound, or can be prepared according to a well-known method.

Next, a step of preparing the compound (I-a) from the compound (M2-2-A).

The compound (I-a) can be prepared by reacting the compound (M2-2-A) with a compound represented by formula (R-3) (hereinafter, referred to as compound (R-3)) in the presence of a metal catalyst and a base.

The reaction is carried out in the presence or the absence of a solvent. Examples of the solvents to be used in the reaction include alcohols; nitriles; ethers; aromatic hydrocarbons; aprotic polar solvents, water; mixed solvents of two or more kinds of the solvents.

Examples of the metal catalyst to be used in the reaction include a palladium catalyst such as tetrakis(triphenylphosphine)palladium(0), [1,1'-bis(diphenylphoshino)ferrocene]dichlororpalladium (II) (hereinafter, referred to as $PdCl_2$(dPPF)), tris(dibenzylideneacetone)dipalladium(0), and palladium (II) acetate; a nickel catalyst such as bis(cyclooctadiene)nickel(0) and nickel(II) chloride; and a copper catalyst such as copper(I) iodide, copper(I) bromide.

Examples of the base to be used in the reaction include alkali metal hydrides, alkali metal carbonates, and organic bases.

A ligand may be used in the reaction. Examples of the ligand include triphenylphosphine, Xantphos, 2,2'-bis(diphenylphoshino)-1,1'-binaphthyl, 1,1'-bis(diphenylphoshino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, and 1,10-phenanthroline. When the ligand is used in the reaction, the ligand is used within a range of 0.01 to 1 molar ratio(s), as 1 mole of the compound (M2-2-A).

In the reaction, the compound (R-3) is usually used within a range of 1 to 20 molar ratios, the metal catalyst is usually used within a range of 0.01 to 0.5 molar ratios, and the base is usually used within a range of 0.1 to 5 molar ratios, as 1 mole of the compound (M2-2-A).

The reaction temperature of the reaction is usually within a range of -20 to 200°C. The reaction period of the reaction is usually within a range of 0.1 to 72 hours.

When the reaction is completed, water is added to the reaction mixture, and the reaction mixture is extracted with organic solvent(s), and the organic layer is worked up (for example, drying and concentration) to give the compound (I-a).

The compound (R-3) is a commercially available compound, or can be prepared according to a well-known method.

Next, a step of preparing the compound (I-a) from the compound (M2-1-A) is described.

The compound (I-a) can be prepared by reacting the compound (M2-1-A) with the compound (R-3) in the presence of a halogenating agent.

The reaction is usually carried out in a solvent. Examples of the solvent include alcohols, nitriles, ethers, aromatic hydrocarbons, ethers, aromatic hydrocarbons, aprotic polar solvents, water, and mixed solvents of two or more kinds of the solvents.

Examples of the halogenating agent include chlorine, bromine, iodine, sodium bromide, potassium bromide, sodium iodide, and potassium iodide.

An oxidizing agent may be used in the reaction as needed. Examples of the oxidizing agent include hydrogen peroxide, tert-butylhydroperoxide, DMSO. When the oxidizing agent is used in the reaction, the oxidizing agent is usually used within a range of 1 to 20 molar ration(s), as opposed to 1 mole of the compound (M2-1-A).

In the reaction, the compound (R-3) is usually used within a range of 0.5 to 10 molar ratios, and the halogenating agent is usually used within a range of 0.05 to 10 molar ratios, as opposed to 1 mole of the compound (M2-1-A).

The reaction temperature of the reaction is usually within a range of 0 to 200°C. The reaction period of the reaction is usually within a range of 0.1 to 72 hours.

When the reaction is completed, water is added to reaction mixture, and the reaction mixture is extracted with organic solvent(s), and the organic layer is worked up (for example, drying and concentration) to give the compound (I-a).

Process 7

A compound represented by formula (I-2-A) (hereinafter, referred to as compound (I-2-A)) can be prepared by reacting the compound (I-1-A) with a sulfurizing agent.

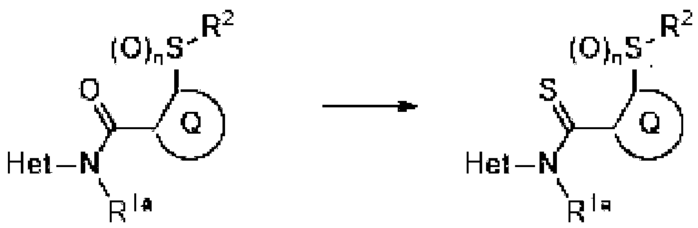

(I-1-A) (I-2-A)

[wherein the symbols are the same as defined above]

The reaction is carried out in the presence or the absence of a solvent. Examples of the solvent include ethers;

halogenated hydrocarbons; aromatic hydrocarbons; nitriles; nitrogen-containing aromatic compounds; and mixed solvents of two or more kinds of the solvents.

Examples of the sulfurizing agent include diphosphorus pentasulfide, a Lawesson's reagent (2,4-bis-(4-methoxyphenyl)-1,3-dithia-2,4-diphosphetane 2,4-disulfide).

In the reaction, the sulfurizing agent is usually used within a range of 1 to 3 molar ratio(s), as opposed to 1 mole of the compound (I-1-A).

The reaction temperature of the reaction is usually within a range of 0 to 200°C. The reaction period of the reaction is usually within a range of 1 to 24 hours.

When the reaction is completed, water is added to reaction mixture, and the reaction mixture is extracted with organic solvent(s), and the organic layer is worked up (for example, drying and concentration) to obtain the compound (I-2-A).

Process 8

A compound represented by formula (I-2-C) (hereinafter, referred to as compound (1-2-C)) can be prepared by reacting a compound represented by formula (I-1-C) (hereinafter, referred to as compound (I-1-C)) with a compound (R-4) (hereinafter, referred to compound (R-4)) or a compound represented by formula (R-5) (hereinafter, referred to as compound (R-5)) in the presence of a metal catalyst and a base.

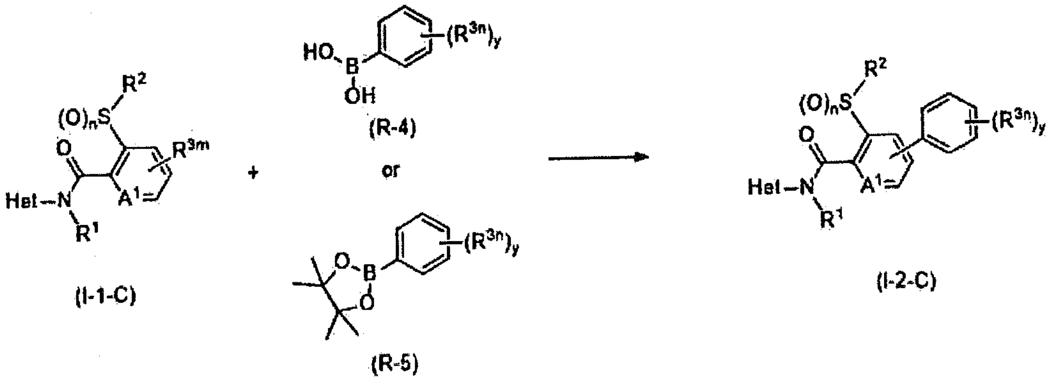

[wherein $R^{3m}$ represents a chlorine atom, a bromine atom, or an iodine atom, $R^{3n}$ represents a substituent selected from the Group M, y is 0, 1, 2, 3, 4 or 5, and the other symbols are the same as defined above]

The reaction is usually carried out in a solvent. Examples of the solvents to be used in the reaction include ethers, aromatic hydrocarbons, nitriles, aprotic polar solvents, water, and mixed solvents of two or more kinds of the solvents.

Examples of the bases to be used in the reaction include alkali metal hydrides, alkali metal carbonates, organic bases, and phosphate salts.

Examples of the metal catalyst include a nickel catalyst such as bis(cyclooctadiene)nickel(0) and nickel(II) chloride; and a palladium catalyst such as palladium (II) acetate, tetrakis(triphenylphosphine)palladium(0), tris(dibenzylideneacetone)dipalladium(0), and $PdCl_2$(dPPF).

The reaction can be conducted using a ligand as needed. Examples of the ligand include triphenylphosphine, Xantphos, 2,2'-bis(diphenylphoshino)-1,1'-binaphthyl, 1,1'-bis(diphenylphoshino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, 1,10-phenanthroline, trans-1,2-cyclohexanediamine, trans-N,N'-dimethylcycloahexan-1,2-diamine, and N,N'-dimethylethylene diamine.

When the ligand is used in the reaction, the ligand is usually used within the range of 0.01 to 0.5 molar ratio(s), as 1 mole of the compound (I-1-C).

In the reaction, the compound (R-4) or the compound (R-5) is usually used within a range of 0.01 to 0.5 molar ratio(s), the metal catalyst is usually used within a range of 0.01 to 0.5 molar ratios, and the base is usually used within a range of 1 to 3 molar ratio(s), as 1 mole of the compound (I-1-C).

The reaction temperature is usually within a range of -20 to 150°C. The reaction period of the reaction is usually within a range of 0.5 to 24 hours.

When the reaction is completed, acidic aqueous solution such as dilute hydrochloric acid is added to the reaction mixtures, and the reaction mixtures are extracted with organic solvent(s), and the organic layers are worked up (such as drying and concentration) to isolate the compound (I-2-C).

Process 9

A compound represented by formula (I-2-D) (hereinafter, referred to as compound (I-2-D)) can be prepared according to the following scheme.

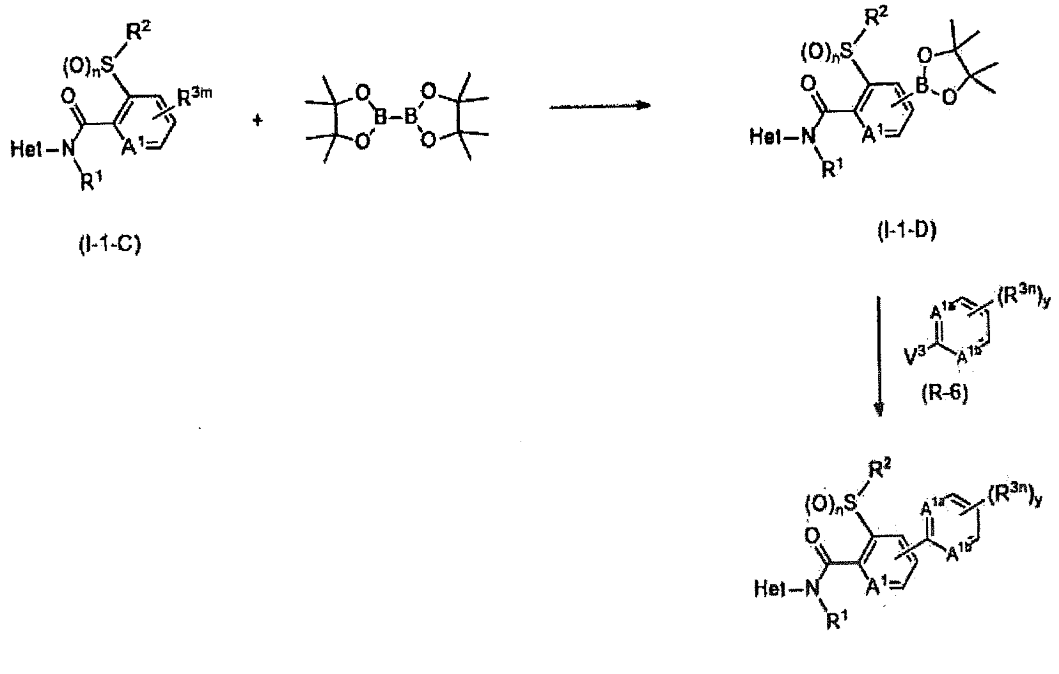

(I-1-C)

(I-1-D)

(R-6)

(I-2-D)

[wherein y is 0, 1, 2, or 3, $A^{1a}$ represents $CR^{1n}$ or a nitrogen atom, $A^{1b}$ represents $CR^{1n}$ or a nitrogen atom, $R^{1n}$ represents a substituent selected from Group M, $V^3$ represents a halogen atom, and the other symbols are the same as defined above]

A compound represented by formula (I-1-D) (hereinafter, referred to as compound (I-1-D)) can be prepared by reacting the compound (I-1-C) with bis(pinacolato)diboron in the presence of a metal catalyst and a base.

The reaction is usually carried out in a solvent. Examples of the solvents to be used in the reaction include hydrocarbons, ethers, aromatic hydrocarbons, nitriles, aprotic polar solvents, and mixed solvents of two or more kinds of the solvents.

Examples of the bases to be used in the reaction include organic bases, alkali metal carbonates, alkali metal hydrides, and phosphate salts.

Examples of the metal catalyst to be used in the reaction include a palladium catalyst such as [1,1'-bis(diphenylphoshino)ferrocene]dichlororpalladium (II).

In the reaction, bis(pinacolato)diboron is usually used within a range of 1 to 3 molar ratio(s), the metal catalyst is usually used within a range of 0.01 to 0.5 molar ratio(s), and the base is usually used within a range of 1 to 5 molar ratio(s), as opposed to 1 mole of the compound (I-1-C).

The reaction temperature is usually within a range of -20 to 150°C. The reaction period of the reaction is usually within a range of 0.5 to 24 hours.

When the reaction is completed, water is added to the reaction mixture, and the reaction mixture is extracted with organic solvent(s), and the organic layer is worked up (such as drying and concentration) to isolate the compound (I-1-D).

A compound represented by formula (I-2-D) (hereinafter, referred to as compound (I-2-D)) can be prepared by reacting the compound (I-1-D) with a compound represented by formula (R-6) (hereinafter, referred to compound (R-6)) in the presence of metal catalyst and a base.

The reaction is usually carried out in a solvent. Examples of the solvent to be used in the reaction include ethers, aromatic hydrocarbons, nitriles, aprotic polar solvents, water, and mixed solvents of two or more kinds of the solvents.

Examples of the bases to be used in the reaction include organic bases, alkali metal carbonates, alkali metal hydrides, and phosphate salts.

Examples of the metal catalyst to be used in the reaction include a palladium catalyst such as palladium (II) acetate, tetrakis(triphenylphosphine)palladium(0), tris(dibenzylideneacetone)dipalladium(0), and $PdCl_2$(dPPF).

A ligand may be used in the reaction as needed. Examples of the ligand include triphenylphosphine, Xantphos, 2,2'-bis(diphenylphoshino)-1,1'-binaphthyl, 1,1'-bis(diphenylphoshino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, 1,10-phenanthroline, trans-1,2-cyclohexanediamine, trans-N,N'-dimethylcycloahexan-1,2-diamine, and N,N'-dimethylethylene diamine. When the ligand is used in the reaction, the ligand is used within a range of 0.01 to 0.5 molar ratios, as 1 mole of the compound (I-1-D).

In the reaction, the compound (R-6) is usually used within a range of 1 to 5 molar ratio(s), the metal catalyst is usually used within a range of 0.01 to 0.5 molar ratios, and the base is usually used within a range of 1 to 5 molar ratio(s), as opposed to 1 mole of the compound (I-1-D).

The reaction temperature is usually within a range of -20 to 150°C. The reaction period of the reaction is usually within a range of 0.5 to 24 hours.

When the reaction is completed, water is added to the reaction mixture, and the reaction mixture is extracted with organic solvent(s), and the organic layer is worked up (such as drying and concentration) to isolate the compound (I-2-D).

The compound (R-6) is a commercially available compound, or can be prepared according to a well-known method.

Process 10

A compound represented by formula (I-2-E) (hereinafter, referred to as compound (I-2-E)) can be prepared by reacting a compound represented by formula (I-1-E) (hereinafter, referred to as compound (I-1-E)) with a compound represented by formula (R-7) (hereinafter, referred to as compound (R-7)) in the presence of a base.

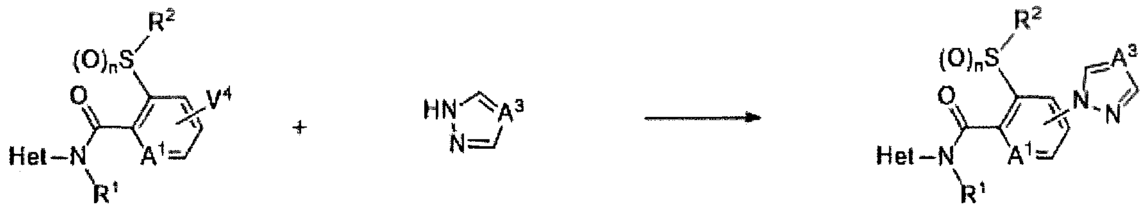

(I-1-E) (R-7) (I-2-E)

[wherein $A^3$ represents $CR^{4m}$ or a nitrogen atom, $R^{4m}$ represents a substituent selected from Group M, $V^4$ represents a fluorine atom or a chlorine atom, and the other symbols are the same as defined above]

The reaction is usually carried out in a solvent. Examples of the solvents to be used in the reaction include ethers, aromatic hydrocarbons, nitriles, aprotic polar solvents, and mixed solvents of two or more kinds of the solvents.

Examples of the bases to be used in the reaction include organic bases, alkali metal carbonates, alkali metal hydrides, and phosphate salts.

In the reaction, the compound (R-7) is usually used within a range of 1 to 5 molar ratio(s), and the base is usually used within a range of 1 to 5 molar ratio(s), as opposed to 1 mole of the compound (I-1-E).

The reaction temperature is usually within a range of -20 to 150°C. The reaction period of the reaction is usually within a range of 0.5 to 24 hours.

When the reaction is completed, water is added to the reaction mixture, and the reaction mixture is extracted with organic solvent(s), and the organic layer is worked up (such as drying and concentration) to isolate the compound (I-2-E).

The compound (R-7) is a commercially available compound, or can be prepared according to a well-known method.

Process 11

A compound represented by formula (I-2-F) (hereinafter, referred to as compound (I-2-F)) can be prepared by reacting the compound (I-1-E) with a compound represented by formula (R-8) (hereinafter, referred to as compound (R-8)) in the presence of a base.

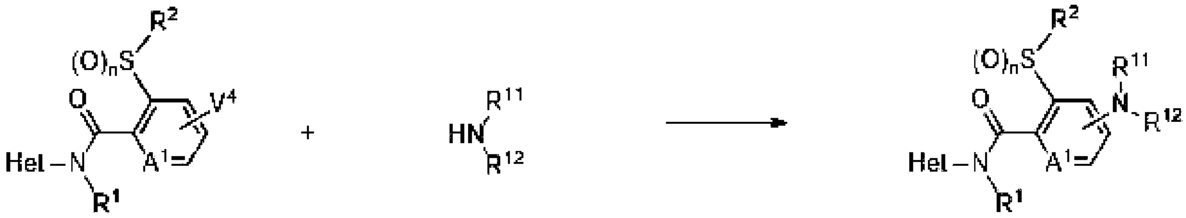

(I-1-E) (R-8) (I-2-F)

[wherein the symbols are the same as defined above]

The reaction is usually carried out in a solvent. Examples of the solvents to be used in the reaction include ethers, aromatic hydrocarbons, nitriles, aprotic polar solvents, and mixed solvents of two or more kinds of the solvents.

Examples of the bases to be used in the reaction include organic bases, alkali metal carbonates, alkali metal hydrides, and phosphate salts.

In the reaction, the compound (R-8) is usually used within a range of 1 to 5 molar ratio(s), and the base is usually used within a range of 1 to 5 molar ratio(s), as opposed to 1 mole of the compound (I-1-E).

The reaction temperature is usually within a range of -20 to 150°C. The reaction period of the reaction is usually within a range of 0.5 to 24 hours.

When the reaction is completed, water is added to the reaction mixture, and the reaction mixture is extracted with organic solvent(s), and the organic layer is worked up (such as drying and concentration) to isolate the compound (I-2-F).

The compound (R-8) is a commercially available compound, or can be prepared according to a well-known method.

Process 12

A compound represented by formula (I-2-G) (hereinafter, referred to as compound (I-2-G)) can be prepared by reacting the compound (I-1-C) with a compound represented by formula (R-9) (hereinafter, referred to as compound (R-9)) in the presence of metal catalyst and a base.

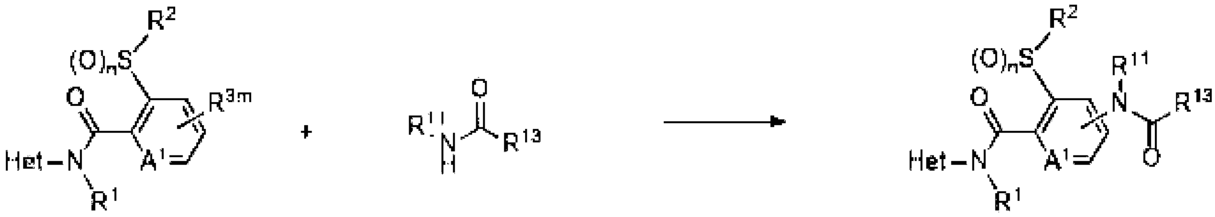

(I-1-C) (R-9) (I-2-G)

[wherein the symbols are the same as defined above]

The reaction is usually carried out in a solvent. Examples of the solvents to be used in the reaction include ethers, aromatic hydrocarbons, nitriles, aprotic polar solvents, and mixed solvents of two or more kinds of the solvents.

Examples of the bases to be used in the reaction include organic bases, alkali metal carbonates, alkali metal hydrides, and phosphate salts.

Examples of the metal catalyst to be used in the reaction include a palladium catalyst such as palladium (II) acetate, tetrakis(triphenylphosphine)palladium(0), tris(dibenzylideneacetone)dipalladium(0), and $PdCl_2$(dPPF).

The reaction can be conducted using a ligand as needed. Examples of the ligand include triphenylphosphine, Xantphos, 2,2'-bis(diphenylphoshino)-1,1'-binaphthyl, 1,1'-bis(diphenylphoshino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, 1,10-phenanthroline, trans-1,2-cyclohexanediamine, trans-N,N'-dimethylcycloahexan-1,2-diamine, and N,N'-dimethylethylene diamine.

When the ligand is used in the reaction, the ligand is used within a range of 0.01 to 0.5 molar ratios, as 1 mole of the compound (I-1-C).

In the reaction, the compound (R-9) is usually used within a range of 1 to 5 molar ratio(s), the metal catalyst is usually used within a range of 0.01 to 0.5 molar ratios, and the base is usually used within a range of 1 to 5 molar ratio(s), as opposed to 1 mole of the compound (I-1-C).

The reaction temperature is usually within a range of -20 to 150°C. The reaction period of the reaction is usually within a range of 0.5 to 24 hours.

When the reaction is completed, water is added to the reaction mixture, and the reaction mixture is extracted with organic solvent(s), and the organic layer is worked up (such as drying and concentration) to isolate the compound (I-2-G) .

The compound (R-9) is a commercially available compound, or can be prepared according to a well-known method.

Reference process 1

The compound (M-3-A) can be prepared by reacting the compound (M1-1-A) with a compound represented by formula (M2-2) (hereinafter, referred to as compound (M2-2)) in the presence of a condensing agent.

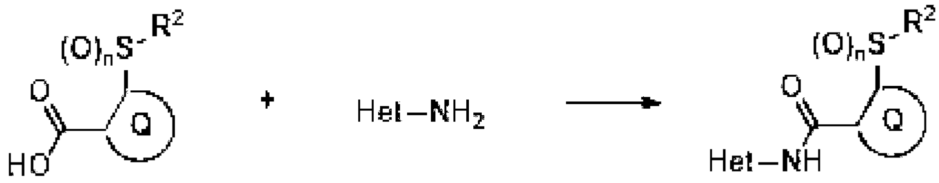

(M1-1-A) (M2-2) (M1-3-A)

[wherein the symbols are the same as defined above]

The reaction can be conducted by using the compound (M2-2) instead of the compound (M2-1) according to the method described in the Process 2.

The compound (M2-2) is a commercially available compound, or can be prepared according to a well-known method.

Reference process 2

The compound (M1-3-A) can be prepared by reacting the compound (M1-2-A) with the compound (M2-2).

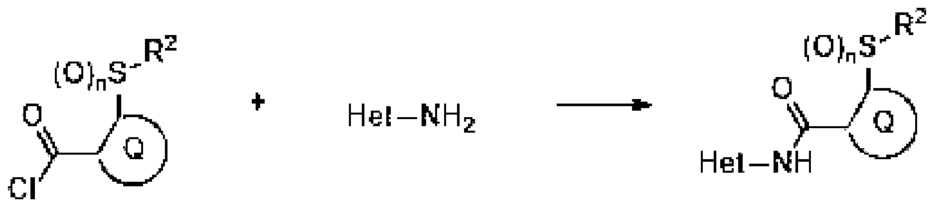

(M1-2-A) (M2-2) (M1-3-A)

[wherein the symbols are the same as defined above]

The reaction can be conducted by using the compound (M2-2) instead of the compound (M2-1) according to the method described in the Process 3.

Reference process 3

The compound (M1-4-A) can be prepared by reacting the compound (M1-1-A) with a compound represented by formula (M2-3) (hereinafter, referred to as compound (M2-3)) in the presence of a condensing agent.

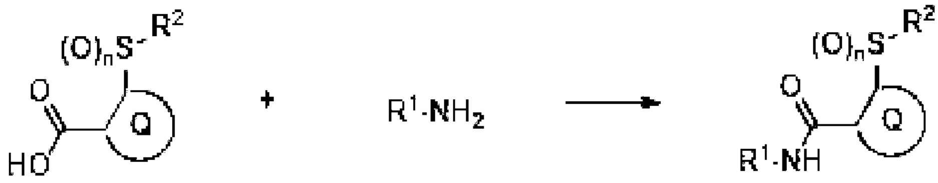

(M1-1-A) (M2-3) (M1-4-A)

[wherein the symbols are the same as defined above]

The reaction can be conducted by using the compound (M2-3) instead of the compound (M2-1) according to the method described in the Process 2.

The compound (M2-3) is a commercially available compound, or can be prepared according to a well-known method.

Reference process 4

The compound (M1-4-A) can be prepared by reacting the compound (M1-2-A) with the compound (M2-3).

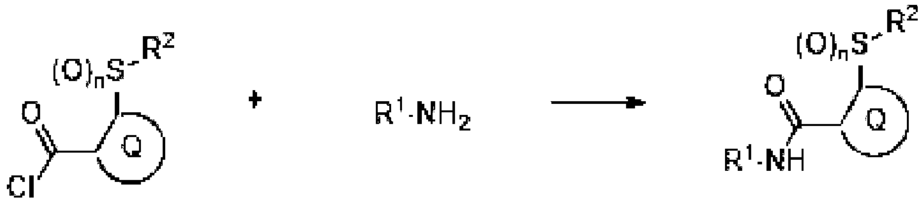

(M1-2-A) (M2-3) (M1-4-A)

[wherein the symbols are the same as defined above]

The reaction can be conducted by using the compound (M2-3) instead of the compound (M2-1) according to the method described in the Process 3.

Reference process 5

A compound represented by formula (M2-2-A) (hereinafter, referred to as compound (M2-2-A)) can be prepared by reacting a compound represented by formula (M3-1-A) (hereinafter, referred to as compound (M3-1-A)) with a thiocyanate salt in the presence of a halogenating agent.

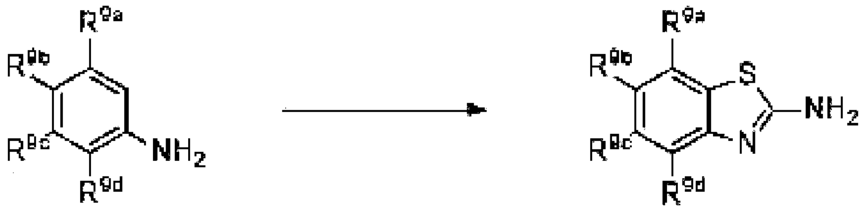

(M3-1-A) (M2-2-A)

[wherein the symbols are the same as defined above]

The reaction is usually carried out in a solvent. Examples of the solvents to be used in the reaction include alcohols, nitriles, ethers, aromatic hydrocarbons, aprotic polar solvents, halogenated hydrocarbons, water, and mixed solvents of two or more kinds of the solvents.

Examples of the thiocyanate salt to be used in the reaction include sodium thiocyanate, potassium thiocyanate, and ammonium thiocyanate.

Examples of the halogenating agent to be used in the reaction include chlorine, bromine, N-chlorosuccinimide, N-bromosuccinimide, phenyltrimethylammonium tribromide, 1,3-dichloro-5,5-dimethylhydantoin, and 1,3-dibromo-5,5-dimethylhydantoin.

In the reaction, the thiocyanate salt is usually used within a range of 1 to 5 molar ratio(s), and the halogenating agent is usually used within a range of 1 to 5 molar ratio(s), as opposed to 1 mole of the compound (M3-1-A).
The reaction temperature is usually within a range of -20 to 150°C. The reaction period of the reaction is usually within a range of 0.5 to 24 hours.
When the reaction is completed, water is added to the reaction mixture, and the reaction mixture is extracted with organic solvent(s), and the organic layer is worked up (such as drying and concentration) to isolate the compound (M2-2-A).
The compound (M3-1-A) is a commercially available compound, or can be prepared according to a well-known method.

Reference process 6

A compound represented by formula (M2-1-A) (hereinafter, referred to as compound (M-2-1-A)) can be prepared according to the following scheme.

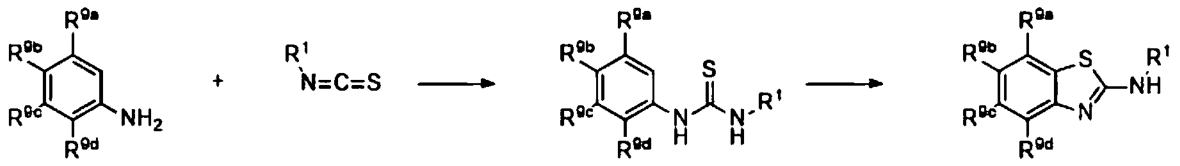

(M3-1-A) (M4-1) (M3-2-A) (M2-1-A)

[wherein the symbols are the same as defined above]
A compound represented by formula (M3-2-A) (hereinafter, referred to as compound (M3-2-A)) can be prepared by reacting a compound represented by formula (M3-1-A) (hereinafter, referred to as compound (M3-1-A)) with a compound represented by formula (M4-1) (hereinafter, referred to as compound (M4-1)).
The reaction is usually carried out in a solvent. Examples of the solvents to be used in the reaction include alcohols, nitriles, ethers, aromatic hydrocarbons, aprotic polar solvents, water, and mixed solvents of two or more kinds of the solvents.
In the reaction, the compound (M4-1) is usually used within a range of 1 to 5 molar ratio(s), as opposed to 1 mole of the compound (M3-1-A).
The reaction temperature is usually within a range of -20 to 150°C. The reaction period of the reaction is usually within a range of 0.5 to 24 hours.
When the reaction is completed, water is added to the reaction mixture, and the reaction mixture is extracted with organic solvent(s), and the organic layer is worked up (such as drying and concentration) to isolate the compound (M3-2-A) .
The compound (M4-1) is a commercially available compound, or can be prepared according to a well-known method.
The compound (M2-1-A) can be prepared by reacting the compound (M3-2-A) in the presence of a halogenating agent.
The reaction is usually carried out in a solvent. Examples of the solvents to be used in the reaction include alcohols, nitriles, ethers, aromatic hydrocarbons, aprotic polar solvents, water, and mixed solvents of two or more kinds of the solvents.
Examples of the halogenating agent to be used in the reaction include chlorine, bromine, N-chlorosuccinimide, N-bromosuccinimide, phenyltrimethylammonium tribromide, 1,3-dichloro-5,5-dimethylhydantoin, and 1,3-dibromo-5,5-dimethylhydantoin.
In the reaction, the halogenating agent is usually used within a range of 1 to 5 molar ratio(s), as opposed to 1 mole of the compound (M3-2-A).
The reaction temperature is usually within a range of -20 to 150°C. The reaction period of the reaction is usually within a range of 0.5 to 24 hours.
When the reaction is completed, water is added to the reaction mixture, and the reaction mixture is extracted with organic solvent(s), and the organic layer is worked up (such as drying and concentration) to isolate the compound (M2-1-A).

Reference process 7

A compound represented by formula (M2-1-B) (hereinafter, referred to as compound (M2-1-B)) can be prepared

according to the following scheme.

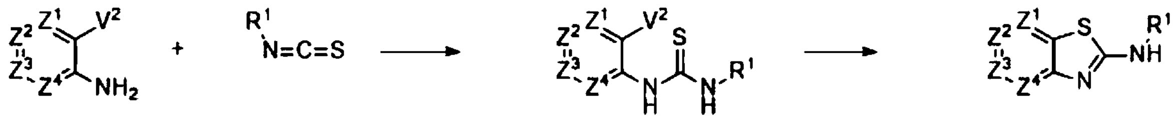

(M3-1-B) (M4-1) (M3-2-B) (M2-1-B)

[wherein $V^2$ represents a halogen atom, and the other symbols are the same as defined above]

A compound represented by formula (M3-2-B) (hereinafter, referred to as compound (M3-2-B)) can be prepared by using a compound represented by formula (M3-1-B) (hereinafter, referred to as compound (M3-1-B)) with the compound (M4-1).

The reaction can be conducted by using the compound (M3-1-B) instead of the compound (M3-1-A) according to the method described in Reference process 6.

The compound (M3-1-B) is a commercially available compound, or can be prepared according to a well-known method.

When $V^2$ represents a fluorine atom or a chloro atom, the compound (M2-1-B) can be prepared by reacting the compound (M3-2-B) in the presence of the base.

The reaction is usually carried out in a solvent. Examples of the solvents to be used in the reaction include alcohols, ethers, aromatic hydrocarbons, aprotic polar solvents, water, and mixed solvents of two or more kinds of the solvents.

Examples of the bases to be used in the reaction include alkali metal hydrides, alkali metal carbonates, organic bases, and phosphate salts.

In the reaction, the base is usually used within a range of 1 to 5 molar ratio(s), as opposed to 1 mole of the compound (M3-2-B).

The reaction temperature is usually within a range of -20 to 150°C. The reaction period of the reaction is usually within a range of 0.5 to 24 hours.

When the reaction is completed, water is added to the reaction mixture, and the reaction mixture is extracted with organic solvent(s), and the organic layer is worked up (such as drying and concentration) to isolate the compound (M2-1-B).

When $V^2$ represents a chloro atom, a bromo atom, or an iodine atom, the compound (M2-1-B) can be prepared by reacting the compound (M3-2-B) in the presence of the metal catalyst and a base.

The reaction is usually carried out in a solvent. Examples of the solvents to be used in the reaction include ethers, aromatic hydrocarbons, nitriles, aprotic polar solvents, water, and mixed solvents of two or more kinds of the solvents.

Examples of the bases to be used in the reaction include alkali metal carbonates, organic bases, and phosphate salts.

Examples of the metal catalyst to be used in the reaction include a nickel catalyst such as bis(cyclooctadiene) nickel(0) and nickel(II) chloride; a palladium catalyst palladium (II) acetate, tetrakis(triphenylphosphine)palladium(0), tris(dibenzylideneacetone)dipalladium(0), and $PdCl_2$(dPPF); and a copper catalyst such as copper(I) iodide, copper(I) chloride.

The reaction can be conducted using a ligand as needed. Examples of the ligand include triphenylphosphine, Xantphos, 2,2'-bis(diphenylphoshino)-1,1'-binaphthyl, 1,1'-bis(diphenylphoshino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, 1,10-phenanthroline, trans-1,2-cyclohexanediamine, trans-N,N'-dimethylcycloahexan-1,2-diamine, and N,N'-dimethylethylene diamine.

When the ligand is used in the reaction, the ligand is used within a range of 0.01 to 0.5 molar ratios, as 1 mole of the compound (3-2-B).

In the reaction, the metal catalyst is usually used within a range of 0.01 to 0.5 molar ratios, and the base is usually used within a range of 1 to 3 molar ratio(s), as opposed to 1 mole of the compound (3-2-B).

The reaction temperature is usually within a range of -20 to 150°C. The reaction period of the reaction is usually within a range of 0.5 to 24 hours.

When the reaction is completed, water is added to the reaction mixture, and the reaction mixture is extracted with organic solvent(s), and the organic layer is worked up (such as drying and concentration) to isolate the compound (M2-1-B).

The Present compound may be mixed with or used in combination with one or more ingredient(s) selected from the group consisting of the following Group (a), Group (b), Group (c), and Group (d) (hereinafter referred to as "Present ingredient").

When the Present compound is mixed with or used in combination with the Present ingredient, they are used simultaneously, separately, or at time intervals with each other.

When the Present compound is used simultaneously with the Present ingredient, the Present compound and the Present ingredient may be contained in separate formulations or contained in one formulation.

One aspect of the present invention provides a composition comprising one or more ingredient(s) selected from the group consisting of Group (a), Group (b), Group (c), and Group (d), and the Present compound (hereinafter referred to as "Composition A").

Group (a) is a group consisting of acetylcholinesterase inhibitors (for example, carbamate insecticides and organophosphate insecticides), GABA-gated chloride channel blockers (for example, phenylpyrazole insecticides), sodium channel modulators (for example, pyrethroid insecticides), nicotinic acetylcholine receptor competitive modulators (for example, neonicotinoid insecticides), nicotinic acetylcholine receptor allosteric modulators, glutamate-gated chloride channel allosteric modulators (for example, macrolide insecticides), juvenile hormone mimics, multisite inhibitors, chordotonal organ TRPV channel modulators, mite growth inhibitors, microbial disruptors of insect midgut membranes, inhibitors of mitochondrial ATP synthase, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, mitochondrial complexes I, II, III, and IV electron transport inhibitors, voltage-dependent sodium channel blockers, inhibitors of acetyl CoA carboxylase, ryanodine receptor modulators (for example, diamide insecticides), chordotonal organ modulators, and microbial insecticides, and other insecticidal active ingredients, miticidal active ingredients, and nematicidal active ingredients. These ingredients are described in the classification on the basis of action mechanism by IRAC.

Group (b) is a group consisting of nucleic acids synthesis inhibitors (for example, phenylamide fungicides and acylamino acid fungicides), cell division and cytoskeleton inhibitors (for example, MBC fungicides), respiration inhibitors (for example, QoI fungicides, and QiI fungicides), amino acids synthesis and protein synthesis inhibitors (for example, anilino-pyrimidine fungicides), signal transduction inhibitors, lipid synthesis and membrane synthesis inhibitors, sterol biosynthesis inhibitors (for example, DMI fungicides such as triazole fungicides), cell wall biosynthesis inhibitors, melanin synthesis inhibitors, plant defense inducers, fungicides with multi-site contact activity, microbial fungicides, and other fungicidal active ingredients. These ingredients are described in the classification on the basis of action mechanism by FRAC.

Group (c) is a group of plant growth regulatory ingredients (including mycorrhizal fungi and root nodule bacteria).

Group (d) is a group of repellent ingredients.

Hereinafter, examples of the combination of the Present ingredient and the Present compound are described. For example, "alanycarb + SX" indicates a combination of alanycarb and SX.

The abbreviation of "SX" indicates any one of the Present compounds selected from the Compound classes SX1 to SX1314 described in Examples. Also, all of the following Present ingredient are known ingredients, and may be obtained from commercially available formulations, or may be prepared by known methods. When the Present ingredient is a microorganism, it may also be available from a bacterial authority depository. Further, the number in parentheses represents the CAS RN (registered trademark).

Combinations of the Present ingredient in the above Group (a) and the Present compound:
abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, bisulflufen + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC(2-methyl-4,6-dinitrophenol) + SX, doramectin + SX, dried leaves of Dryopteris filix-mas + SX, emamectin-benzoate + SX, empenthrin + SX, endosulfan + SX, EPN(O-ethyl O-(4-nitrophenyl) phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole +

SX, extract of Artemisia absinthium + SX, extract of Azadirachta indica + SX, extract of Cassia nigricans + SX, extract of clitoria ternatea + SX, extract of Symphytum officinale + SX, extract of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, flupyroxystrobin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hvla peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, ledprona + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pioxaniliprole + SX, piperflanilide + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfiflumin + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tigolaner + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, umifoxolaner + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, N-[4-chloro-2-(pyridin-3-yl)-1,3-thiazol-5-yl]-N-ethyl-3-(methanesulfonyl)propanamide + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX, Bacillus thuringiensis Cry1Ab protein + SX, Bacillus thuringiensis Cry1Ac protein + SX, Bacillus thuringiensis Cry1Fa protein + SX, Bacillus thuringiensis Cry1A.105 protein + SX, Bacillus thuringiensis Cry2Ab protein + SX, Bacillus thuringiensis Vip3A protein + SX, Bacillus thuringiensis mCry3A protein + SX, Bacillus thuringiensis Cry3Ab protein + SX, Bacillus thuringiensis Cry3Bb protein + SX, Bacillus thuringiensis Cry34Ab1/Cry35Ab1 protein + SX, Adoxophyes orana granulosis virus strain BV-0001 + SX, Anticarsia gemmatalis MNPV (multiple nucleocapsid nucleopolyhedrovirus) + SX, Autographa californica MNPV + SX, Cydia pomonella GV(granulovirus) strain V15 + SX, Cydia pomonella GV strain V22 +

SX, Cryptophlebia leucotreta GV + SX, Dendrolimus punctatus cypovirus + SX, Helicoverpa armigera NPV(nucleopolyhedrovirus) strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua MNPV + SX, Spodoptera littoralis MNPV + SX, Spodoptera litura NPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306) + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

Combination of the Present ingredient in the above Group (b) and the Present compound:
acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper(II) acetate + SX, copper(II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Allium sativum + SX, extract of the cotyledons of lupine plantlets ("BLAD") + SX, extract of Equisetum arvense + SX, extract of Melaleuca alternifolia + SX, extract of Reynoutria sachalinensis + SX, extract of Tropaeolum majus + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin + SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX, imazalil + SX,

imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl] ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl)butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl)carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, Agrobacterium radiobactor strain K1026 + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({4-[5-(trifluoromethyl)-1,2,4-oxadiazol-3-yl]phenyl}methyl)urea + SX, N,N-diethyl-N'-({ 4-[5-(trifluoromethyl)-1,2,4-oxadiazol-3-yl]phenyl}methyl)urea + SX, N-methyl-N'-({ 4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl)(2,6-difluoropyridin-4-yl)amino]-N-(2,2-dimethylcyclobutyl)-5-methylthiazole-4-carboxamide + SX, 2-[(2-methylpropanoyl)(2,6-difluoropyridin-4-yl)aminol-N-(2,2-dimethylcyclobutyl)-5-methylthiazole-4-carboxamide + SX, 2-[(2,6-difluoropyridin-4-yl)amino]-N-(2,2-dimethylcyclobutyl)-5-methylthiazole-4-carboxamide + SX, 2-{[(oxetan-3-yl)carbonyl](2,6-difluoropyridin-4-yl)amino}-N-(2,2-dimethylcyclobutyl)-5-methylthiazole-4-carboxamide + SX, 2-{[(oxolan-3-yl)carbonyl](2,6-difluoropyridin-4-yl)amino}-N-(2,2-dimethylcyclobutyl)-5-methylthiazole-4-carboxamide + SX, 2-{[(oxan-4-yl)carbonyl](2,6-difluoropyridin-4-yl)amino}-N-(2,2-dimethylcyclobutyl)-5-methylthiazole-4-carboxamide + SX, 2-[(2,6-difluoropyridin-4-yl)amino]-N-(2,2-dimethylcyclobutyl)-5-methylthiazole-4-carboxamide + SX, 2-{[(oxetan-3-yl)carbonyl](2,6-difluoropyridin-4-yl)amino}-N-(2,2-dimethylcyclobutyl)-5-methylthiazole-4-carboxamide + SX, 2-{[(oxolan-3-yl)carbonyl](2,6-difluoropyridin-4-yl)amino}-N-(2,2-dimethylcyclobutyl)-5-methylthiazole-4-carboxamide + SX, 2-{[(oxan-4-yl)carbonyl](2,6-difluoropyridin-4-yl)amino}-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2-methylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2,2-dimethylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(5-fluoro-2-methoxyphenyl)-2-(2-cyanoethoxy)ethyl]-5-[1-(isopropoxyimino)ethyl]-2,6-dioxo-3,6-dihydropyrimidin-1(2H)-yl}propan-2-yl)-2-methylpropanamide + SX, methyl ({5-[1-(2,6-difluoro-4-isopropylphenyl)-1H-pyrazol-3-yl]-2-methylphenyl}methyl)carbamate + SX, methyl ({5-[1-(2,6-difluoro-4-cyclopropylphenyl)-1H-pyrazol-3-yl]-2-methylphenyl}methyl)carbamate + SX, methyl ({5-[1-(2,6-difluoro-4-methoxyphenyl)-1H-pyrazol-3-yl]-2-methylphenyl}methyl)carbamate + SX, methyl (Z)-2-(5-cyclopentyl-2-methylphenoxy)-3-methoxyprop-2-enoate + SX, methyl (Z)-2-(5-cyclohexyl-2-methylphenoxy)-3-methoxyprop-2-enoate + SX, methyl (Z)-2-[(3-isopropyl-1H-pyrazol-1-yl)-2-methylphenoxy]-3-methoxyprop-2-enoate + SX, 1-(4,5-dimethyl-1H-benzimidazol-1-yl)-4,4-difluoro-3,3-dimethyl-3,4-dihydroisoquinoline + SX, 1-(4,5-dimethyl-1H-benzimidazol-1-yl)-4,4,5-trifluoro-3,3-dimethyl-3,4-dihydroisoquinoline + SX, 1-(pyrazolo[1,5-a]pyridin-3-yl)-4,4-difluoro-3,3-dimethyl-3,4-dihydroisoquinoline + SX, 1-(6,7-dimethylpyrazolo[1,5-a]pyridin-3-yl)-6-fluoro-3,3-dimethylisoquinolin-4(3H)-one + SX, methyl (2Z)-3-methoxy-2-[(4-methyl[1,1'-biphenyl]-3-yl)oxy]prop-2-enoate + SX, Agrobacterium radiobactor strain K84 + SX, Bacillus amyloliquefaciens strain PTA-4838 (Aveo(registered trademark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain

742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SX.

Combination of the Present ingredient in the above Group (c) and the Present compound:
1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butylate + SX, methyl 5-(trifluoromethyl)benzo[b]thiophene-2-carboxylate + SX, 3-[(6-chloro-4-phenylquinazolin-2-yl)amino]propan-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

Combination of the Present ingredient in the above Group (d) and the Present compound:
anthraquinone + SX, deet + SX, icaridin + SX.

The ratio of the Present compound to the Present ingredient includes, but not limited thereto, as a ratio by weight (the Present compound : the Present ingredient) 1,000:1 to 1:1,000, 500:1 to 1:500, 100:1 to 1:100, 50:1, 20:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:20, and 1:50, and the others.

The Present compound has control effect on harmful arthropods such as harmful insects and harmful mites, harmful nematodes, and harmful mollusks. Examples of the harmful arthropods, harmful nematodes, and harmful mollusks include the followings.

Hemiptera:

from the family Delphacidae, for example, small brown planthopper (Laodelphax striatellus), brown planthopper (Nilaparvata lugens), white-backed planthopper (Sogatella furcifera), corn planthopper (Peregrinus maidis), cereal

leafhopper (Javesella pellucida), sugarcane leafhopper (Perkinsiella saccharicida), white-bellied planthopper (Stenocranus pacificus) and Tagosodes orizicolus;

from the family Cicadellidae, for example,

green rice leafhopper (Nephotettix cincticeps), green paddy leafhopper (Nephotettix virescens), rice leafhopper (Nephotettix nigropictus), zigzag-striped leafhopper (Recilia dorsalis), tea green leafhopper (Empoasca onukii), potato leafhopper (Empoasca fabae), corn leafhopper (Dalbulus maidis), rice leafhopper (Cofana spectra), and Amrasca biguttula biguttula;

from the family Aphrophoridae, for example, European spittlebug (Philaenus spumarius)

from the family Cercopidae, for example, Mahanarva posticata and Mahanarva fimbriolata

from the family Aphididae, for example,

bean aphid (Aphis fabae), soybean aphid (Aphis glycines), cotton aphid (Aphis gossypii), green apple aphid (Aphis pomi), apple aphid (Aphis spiraecola), green peach aphid (Myzus persicae), leaf-curling plum aphid (Brachycaudus helichrysi), cabbage aphid (Brevicoryne brassicae), rosy apple aphid(Dysaphis plantaginea), false cabbage aphid (Lipaphis erysimi), potato aphid (Macrosiphum euphorbiae), foxglove aphid (Aulacorthum solani), lettuce aphid (Nasonovia ribisnigri), grain aphid (Rhopalosiphum padi), corn aphid (Rhopalosiphum maidis), brown citrus aphid (Toxoptera citricida), mealy plum aphid (Hyalopterus pruni), cane aphid (Melanaphis sacchari), black rice root aphid (Tetraneura nigriabdominalis), sugarcane cottony aphid (Ceratovacuna lanigera), apple woolly aphid (Eriosoma lanigerum), and English grain aphid (Sitobion avenae);

from the family Phylloxeridae, for example,

grapevine phylloxera (Daktulosphaira vitifoliae), Pecan phylloxera (Phylloxera devastatrix), Pecan leaf phylloxera (Phylloxera notabilis), and Southern pecan leaf phylloxera (Phylloxera russelae);

from the family Adelgidae, for example, hemlock woolly aphid (Adelges tsugae), balsam woolly aphid (Adelges piceae), and Aphrastasia pectinatae;

from the family Pentatomidae, for example, black rice bug (Scotinophara lurida), black paddy bug (Scotinophara coarctata), common green stink bug (Nezara antennata), white-spotted spined bug (Eysarcoris aeneus), lewis spined bug (Eysarcoris lewisi), white-spotted bug (Eysarcoris ventralis), Eysarcoris annamita, brown marmorated stink bug (Halyomorpha halys), green plant bug (Nezara viridula), brown stink bug(Euschistus heros), red banded stink bug(Piezodorus guildinii), legume stink bug (Piezodorus hybneri), Oebalus pugnax, and Dichelops melacanthus;

from the family Cydnidae, for example, Scaptocoris castanea;

from the family Alydidae, for example, bean bug (Riptortus clavatus), corbett rice bug (Leptocorisa chinensis), and rice bug (Leptocorisa acuta);

from the family Coreidae, for example, Cletus punctiger and Australian leaf-footed bug (Leptoglossus australis);

from the family Lygaeidae, for example, oriental chinch bug (Cavelerius saccharivorus), seed bug (Togo hemipterus), and chinch bug (Blissus leucopterus);

from the family Miridae, for example, rice leaf bug (Trigonotylus caelestialium), sorghum plant bug (Stenotus rubrovittatus), wheat leaf bug (Stenodema calcarata), and American tarnished plant bug (Lygus lineolaris);

from the family Aleyrodidae, for example, greenhouse whitefly (Trialeurodes vaporariorum), tobacco whitefly (Bemisia tabaci), citrus whitefly (Dialeurodes citri), citrus spiny whitefly (Aleurocanthus spiniferus), tea spiny whitefly (Aleurocanthus camelliae), and Pealius euryae;

from the family Diaspididae, for example, Abgrallaspis cyanophylli, red scale (Aonidiella aurantii), San José scale (Diaspidiotus perniciosus), white peach scale (Pseudaulacaspis pentagona), arrowhead scale (Unaspis yanonensis), and citrus snow scale (Unaspis citri);

from the family Coccidae, for example, pink wax scale (Ceroplastes rubens);

from the family Margarodidae, for example, fluted scale (Icerya purchasi) and seychelles fluted scale (Icerya seychellarum);

from the family Pseudococcidae, for example, solanum mealybug (Phenacoccus solani), cotton mealybug (Phenacoccus solenopsis), Japanese mealybug (Planococcus kraunhiae), white peach scale (Pseudococcus comstocki), citrus mealybug (Planococcus citri), currant mealybug (Pseudococcus calceolariae), long-tailed mealybug (Pseudococcus longispinus), and tuttle mealybug (Brevennia rehi);

from the family Psyllidae, for example, citrus psylla (Diaphorina citri), two-spotted citrus psyllid (Trioza erytreae), pear sucker (Cacopsylla pyrisuga), Cacopsylla chinensis, potato psyllid (Bactericera cockerelli), and Pear psylla (Cacopsylla pyricola);

from the family Tingidae, for example, sycamore lace bug (Corythucha ciliata), aster tingid (Corythucha marmorata), Japanese pear lace bug (Stephanitis nashi), and azalea lace bug (Stephanitis pyrioides);

from the family Cimicidae, for example, common bed bug (Cimex lectularius) and tropical bed bug (Cimex hemipterus);

from the family Cicadidae, for example, Quesada gigas;

from the family Reduviidae, for example, Triatoma infestans, large kissing bug (Triatoma rubrofasciata), Triatoma

dimidiata, and Rhodonius prolixus;

and the others.

Lepidoptera:

from the family Crambidae, for example, rice stem borer (Chilo suppressalis), Dark-headed stem borer (Chilo polychrysus), white stem borer (Scirpophaga innotata), yellow paddy borer (Scirpophaga incertulas), Rupela albina, rice leaf roller (Cnaphalocrocis medinalis), Marasmia patnalis, rice leaf roller (Marasmia exigua), cotton leaf roller (Notarcha derogata), corn borer (Ostrinia furnacalis), European corn borer (Ostrinia nubilalis), cabbage webworm (Hellula undalis), grape leafroller (Herpetogramma luctuosale), bluegrass webworm (Parapediasia teterrellus), rice case-worm (Nymphula depunctalis), Sugarcane borer (Diatraea saccharalis), and eggplant fruit borer (Leucinodes orbonalis);
from the family Pyralidae, for example, lesser cornstalk borer (Elasmopalpus lignosellus), mealworm moth (Plodia interpunctella), persimmon bark borer (Euzophera batangensis), and fig moth (Cadra cautella);
from the family Noctuidae, for example, cotton worm (Spodoptera litura), beet armyworm (Spodoptera exigua), rice armyworm (Mythimna separata), cabbage moth (Mamestra brassicae), pink borer (Sesamia inferens), grass armyworm (Spodoptera mauritia), green rice caterpillar (Naranga aenescens), Spodoptera frugiperda, true armyworm (Spodoptera exempta), semitropical armyworm (Spodoptera eridania), black cutworm (Agrotis ipsilon), turnip moth (Agrotis segetum), beet worm (Autographa nigrisigna), rice looper (Plusia festucae), Soybean looper (Chrysodeixis includens), Trichoplusia spp., Heliothis spp. (such as tobacco budworm (Heliothis virescens)), Helicoverpa spp. (such as tobacco budworm (Helicoverpa armigera), corn earworm (Helicoverpa zea)), velvet-bean caterpillar (Anticarsia gemmatalis), cotton leafworm (Alabama argillacea), and hop vine borer (Hydraecia immanis);
from the family Pieridae, for example, common cabbage worm (Pieris rapae);
from the family Tortricidae, for example, oriental fruit moth (Grapholita molesta), Grapholita dimorpha, soybean moth (Leguminivora glycinivorella), Matsumuraeses azukivora, summer fruit tortrix (Adoxophyes orana fasciata), smaller tea tortrix (Adoxophyes honmai), Japanese tea tortrix (Homona magnanima), apple tortrix (Archips fuscocupreanus), codling moth (Cydia pomonella), sugarcane shoot borer (Tetramoera schistaceana), bean shoot borer (Epinotia aporema), citrus fruit borer (Citripestis sagittiferella), and European grapevine moth (Lobesia botrana);
from the family Gracillariidae, for example, tea leaf roller (Caloptilia theivora) and Asiatic apple leaf miner (Phyllonorycter ringoniella);
from the family Carposinidae, for example, peach fruit moth (Carposina sasakii);
from the family Lyonetiidae, for example, coffee leaf miner (Leucoptera coffeella), peach leaf miner (Lyonetia clerkella), and Lyonetia prunifoliella;
from the family Lymantriidae, for example, Lymantria spp. (such as gypsy moth (Lymantria dispar)) and Euproctis spp. (such as tea lymantriid (Euproctis pseudoconspersa));
from the family Plutellidae, for example, diamondback moth (Plutella xylostella);
from the family Gelechiidae, for example, peach worm (Anarsia lineatella), sweetpotato leaf folder (Helcystogramma triannulella), pink bollworm (Pectinophora gossypiella), potato moth (Phthorimaea operculella), and tomato leafminer (Tuta absoluta);
from the family Arctiidae, for example, American white moth (Hyphantria cunea);
from the family Castniidae, for example, giant sugarcane borer (Telchin licus);
from the family Cossidae, for example, Cossus insularis;
from the family Geometridae, for example, Ascotis selenaria;
from the family Limacodidae, for example, blue-striped nettle grub (Parasa lepida) ;
from the family Stathmopodidae, for example, persimmon fruit moth (Stathmopoda masinissa);
from the family Sphingidae, for example, tobacco hornworm (Acherontia lachesis);
from the family Sesiidae, for example, Nokona feralis, cherry borer (Synanthedon hector), and Synanthedon tenuis:

from the family Hesperiidae, for example, rice skipper (Parnara guttata);
from the family Tineidae, for example, casemaking clothes moth (Tinea translucens), common clothes moth (Tineola bisselliella);

and the others.

Thysanoptera:

from the family Thripidae, for example, western flower thrips (Frankliniella occidentalis), oriental thrips (Thrips palmi), yellow tea thrips (Scirtothrips dorsalis), onion thrips (Thrips tabaci), eastern flower thrips (Frankliniella intonsa), rice thrips (Stenchaetothrips biformis), Echinothrips americanus, and avocado thrips (Scirtothrips perseae);
from the family Phlaeothripidae, for example, aculeated rice thrips (Haplothrips aculeatus);

and the others.

Diptera:

from the family Anthomyiidae, for example, seedcorn maggot (Delia platura), onion maggot (Delia antiqua), and beet leaf miner (Pegomya cunicularia);
from the family Ulidiidae, for example, sugarbeet root maggot (Tetanops myopaeformis);
from the family Agromyzidae, for example, rice leaf miner (Agromyza oryzae), tomato leaf miner (Liriomyza sativae), chrysanthemum leaf miner (Liriomyza trifolii), and pea leafminer (Chromatomyia horticola);
from the family Chloropidae, for example, rice stem maggot (Chlorops oryzae);
from the family Tephritidae, for example, melon fly (Bactrocera cucurbitae), oriental fruit fly (Bactrocera dorsalis), Malaysian fruit fly (Bactrocera latifrons), olive fruit fly (Bactrocera oleae), Queensland fruit fly (Bactrocera tryoni), Mediterranean fruit fly (Ceratitis capitata), apple maggot (Rhagoletis pomonella), and Japanese cherry fruit fly (Rhacochlaena japonica);
from the family Ephydridae, for example, smaller rice leaf miner (Hydrellia griseola), whorl maggot (Hydrellia philippina), and paddy stem maggot (Hydrellia sasakii);
from the family Drosophilidae, for example, cherry drosophila (Drosophila suzukii), and common fruit fly (Drosophila melanogaster);
from the family Phoridae, for example, Megaselia spiracularis;
from the family Psychodidae, for example, Clogmia albipunctata;
from the family Sciaridae, for example, Bradysia difformis and Bradysia odoriphaga;
from the family Cecidomyiidae, for example, hessian fly (Mayetiola destructor) and paddy gall fly (Orseolia oryzae);
from the family Diopsidae, for example, Diopsis macrophthalma;
from the family Glossinidae, for example, Glossina palpalis and Glossina morsitans;
from the family Simuliidae, for example, Simulium japonicum and Simulium damnosum;
from the subfamily Phlebotominae;
from the family Tipulidae, for example, rice crane fly (Tipula aino), common cranefly (Tipula oleracea), and European cranefly (Tipula paludosa);
from the family Culicidae, for example, southern house mosquito (Culex pipiens pallens), Culex tritaeniorhynchus, Culex pipiens f. molestus, brown house mosquito (Culex quinquefasciatus), northern house mosquito (Culex pipiens pipiens), Culex vishnui, Asian tiger mosquito (Aedes albopictus), dengue mosquito (Aedes aegypti), Chinese malaria mosquito (Anopheles sinensis), Anopheles gambiae, Anopheles stephensi, Anopheles coluzzii, Anopheles albimanus, Anopheles sundaicus, Anopheles arabiensis, Anopheles funestus, Anopheles darlingi, Anopheles farauti, and Anopheles minimus;
from the family Simulidae, for example, Prosimulium yezoensis and Simulium ornatum;
from the family Tabanidae, for example, Tabanus trigonus;
from the family Muscidae, for example, house fly (Musca domestica), false stable fly (Muscina stabulans), biting house fly (Stomoxys calcitrans), and buffalo fly (Haematobia irritans);
from the family Calliphoridae;
from the family Sarcophagidae;
from the family Chironomidae, for example, Chironomus plumosus, Chironomus yoshimatsui, and Glyptotendipes tokunagai;
from the family Fannidae;

and the others.

Coleoptera:

from the family Chrysomelidae, for example, Diabrotica spp. (such as western corn rootworm (Diabrotica virgifera virgifera), southern corn rootworm (Diabrotica undecimpunctata howardi), northern corn rootworm (Diabrotica barberi), Mexican corn rootworm (Diabrotica virgifera zeae), banded cucumber beetle (Diabrotica balteata), cucurbit beetle(Diabrotica speciosa)), bean leaf beetle (Cerotoma trifurcata), barley leaf beetle (Oulema melanopus), cucurbit leaf beetle (Aulacophora femoralis), striped flea beetle (Phyllotreta striolata), cabbage flea beetle (Phyllotreta cruciferae), western black flea beetle (Phyllotreta pusilla), cabbage stem flea beetle (Psylliodes chrysocephala), hop flea beetle (Psylliodes punctulata), Colorado potato beetle (Leptinotarsa decemlineata), rice leaf beetle (Oulema oryzae), grape colaspis (Colaspis brunnea), corn flea beetle (Chaetocnema pulicaria), sweet-potato flea beetle (Chaetocnema confinis), potato flea beetle (Epitrix cucumeris), rice leaf beetle (Dicladispa armigera), southern corn leaf beetle (Myochrous denticollis), Laccoptera quadrimaculata, tobacco flea beetle (Epitrix hirtipennis), daikon leaf beetle (Phaedon brassicae) and two-striped leaf beetle (Medythia nigrobilineata);
from the family Carabidae, for example, Seedcorn beetle (Stenolophus lecontei) and slender seed-corn ground beetle (Clivina impressifrons);
from the family Scarabaeidae, for example, cupreus chafer (Anomala cuprea), soybean beetle (Anomala rufocuprea), Anomala albopilosa, Japanese beetle (Popillia japonica), yellowish elongate chafer (Heptophylla picea), European Chafer (Rhizotrogus majalis), Tomarus gibbosus, Holotrichia spp., Phyllophaga spp. (such as June beetle (Phyllophaga crinita)), and Diloboderus spp. (such as Diloboderus abderus);
from the family Anthriibidae, for example, coffee bean weevil (Araecerus coffeae);
from the family Aponidae, for example, sweet-potato weevil (Cylas formicarius);
from the family Bruchidae, for example, Mexican bean weevil (Zabrotes subfasciatus);
from the family Scolytidae, for example, pine beetle (Tomicus piniperda) and coffee berry borer (Hypothenemus hampei);
from the family Curculionidae, for example, West Indian sweet-potato weevil (Euscepes postfasciatus), alfalfa weevil (Hypera postica), maize wevil (Sitophilus zeamais), rice weevil (Sitophilus oryzae), grain weevil (Sitophilus granarius), rice plant weevil (Echinocnemus squameus), rice water weevil (Lissorhoptrus oryzophilus), Rhabdoscelus lineaticollis, boll weevil (Anthonomus grandis), nunting billbug (Sphenophorus venatus), southern corn billbug (Sphenophorus callosus), soybean stalk weevil (Sternechus subsignatus), sugarcane weevil (Sphenophorus levis), rusty gourd-shaped weevil (Scepticus griseus), brown gourd-shaped weevil (Scepticus uniformis), Aracanthus spp. (such as Aracanthus mourei), and cotton root borer (Eutinobothrus brasiliensis) ;
from the family Tenebrionidae, for example, red meal beetle (Tribolium castaneum), mason beetle (Tribolium confusum), and lesser mealworm (Alphitobius diaperinus);
from the family Coccinellidae, for example, twenty-eight-spotted ladybird (Epilachna vigintioctopunctata);
from the family Bostrychidae, for example, common powder-post beetle (Lyctus brunneus), and lesser grain borer (Rhizopertha dominica);
from the family Ptinidae;
from the family Cerambycidae, for example, citrus long-horned beetle (Anoplophora malasiaca), Migdolus fryanus, and peach borer (Aromia bungii);
from the family Elateridae, for example, Melanotus okinawensis, barley wireworm (Agriotes fuscicollis), Melanotus legatus, Anchastus spp., Conoderus spp., Ctenicera spp., Limonius spp., and Aeolus spp.;
from the family Staphylinidae, for example, Paederus fuscipes;
from the family Dermestidae, for example, varied carpet beetle (Anthrenus verbasci), hide beetle (Dermestes maculates), and khapra beetle (Trogoderma granarium);
from the family Anobiidae, for example, tobacco beetle (Lasioderma serricorne), and biscuit beetle (Stegobium paniceum) ;
from the family Laemophloeidae, for example, flat grain beetle (Cryptolestes ferrugineus);
from the family Silvanidae, for example, saw-toothed grain beetle (Oryzaephilus surinamensis);
from the family Nitidulidae, for example, blossom beetle (Brassicogethes aeneus);

and the others.

Orthoptera:

from the family Acrididae, for example, oriental migratory locust (Locusta migratoria), Moroccan locust (Dociostaurus maroccanus), Australian plague locust (Chortoicetes terminifera), red locust (Nomadacris septemfasciata), brown locust(Locustana pardalina), tree locust (Anacridium melanorhodon), Italian locust (Calliptamus italicus), differential grasshopper (Melanoplus differentialis), two-striped grasshopper (Melanoplus bivittatus), migratory grasshopper (Melanoplus sanguinipes), red-legged grasshopper (Melanoplus femurrubrum), clear-winged grasshopper (Camnula pellucida), desert locust (Schistocerca gregaria), Yellow-winged locust (Gastrimargus musicus), spur-throated locust (Austracris guttulosa), Japanese grasshopper (Oxya yezoensis), rice grasshopper (Oxya japonica), and Bombay locust (Patanga succincta);
from the family Gryllotalpidae, for example, oriental mole cricket (Gryllotalpa orientalis);
from the family Gryllidae, for example, house cricket (Acheta domestica), and emma field cricket (Teleogryllus emma);
from the family Tettigoniidae, for example, mormon cricket (Anabrus simplex);

and the others.

Hymenoptera :

from the family Tenthredinidae, for example, beet sawfly (Athalia rosae) and nippon cabbage sawfly (Athalia japonica);
from the family Formicidae, for example, Solenopsis spp. (such as red imported fire ant (Solenopsis invicta), tropical fire ant (Solenopsis geminata)), Atta spp. (such as brown leaf-cutting ant (Atta capiguara), Acromyrmex spp., Paraponera clavata, black house ant (Ochetellus glaber), little red ant (Monomorium pharaonis), Argentine ant (Linepithema humile), Formica japonica, Pristomyrmex punctutus, Pheidole noda, big-headed ant (Pheidole megacephala), Camponotus spp. (such as Camponotus japonicus, Camponotus obscuripes), Pogonomyrmex spp. (such as western harvester ant (Pogonomyrmex occidentalis)), Wasmania spp. (such as Wasmania auropunctata), and long-legged ant (Anoplolepis gracilipes);
from the family Vespidae, for example, Asian giant hornet (Vespa mandarinia, Vespa simillima, Vespa analis, Asian hornet(Vespa velutina), and Polistes jokahamae;
from the family Siricidae, for example, pine wood wasp (Urocerus gigas);
from the family Bethylidae;

and the others.

Blattodea :

from the family Ectobiidae, for example, German cockroach (Blattella germanica);
from the family Blattidae, for example, smoky-brown cockroach (Periplaneta fuliginosa), American cockroach (Periplaneta americana), Australian cockroach (Periplaneta australasiae), brown cockroach (Periplaneta brunnea), and black cockroach (Blatta orientalis);
from the family Termitidae, for example, Japanese termite (Reticulitermes speratus), Formosan termite (Coptotermes formosanus), western drywood termite (Incisitermes minor), Cryptotermes domesticus, Odontotermes formosanus, Neotermes koshunensis, Glyptotermes satsumensis, Glyptotermes nakajimai, Glyptotermes fuscus, Hodotermopsis sjostedti, Coptotermes guangzhouensis, Reticulitermes amamianus, Reticulitermes miyatakei, Reticulitermes kanmonensis, Nasutitermes takasagoensis, Pericapritermes nitobei, Sinocapritermes mushae, and Cornitermes cumulans;

and the others.

Siphonaptera:

from the family Pulicidae, for example, human flea (Pulex irritans), cat flea (Ctenocephalides felis), dog flea (Ctenocephalides canis), oriental rat flea (Xenopsylla cheopis), and chicken flea (Echidnophaga gallinacea);
from the family Pulicidae, for example, chigoe flea (Tunga penetrans);

from the family Ceratophyllidae, for example, European rat flea (Nosopsyllus fasciatus); and the others.

Psocodae:

from the family Pediculidae, for example, head louse (Pediculus humanus capitis);
from the family Pthiridae, for example, crab louse (Pthirus pubis);
from the family Haematopinidae, for example, short-nosed cattle louse (Haematopinus eurysternus) and pig louse (Haematopinus suis);
from the family Linognathidae, for example, blue cattle louse (Linognathus vituli), sheep face louse (Linognathus ovillus), and capillate louse (Solenopotes capillatus);
from the family Bovicoliidae, for example, cattle biting louse (Bovicola bovis), sheep biting louse (Bovicola ovis), Bovicola breviceps, Damalinia forficula, and Werneckiella spp.;
from the family Trichodectidae, for example, dog biting louse (Trichodectes canis) and cat louse (Felicola subrostratus);
from the family Menoponidae, for example, common chicken louse (Menopon gallinae), chicken body louse (Menacanthus stramineus), and Trinoton spp;
from the family Trimenoponidae, for example, Cummingsia spp.;
from the family Trogiidae, for example, death watch (Trogium pulsatorium);
from the family Liposcelidae or Liposcelididae, for example, book louse (Liposcelis corrodens), Liposcelis bostrychophila, Liposcelis pearmani and Liposcelis entomophila;

and the other.

Thysanura:

from the family Lepismatidae, for example, oriental silverfish (Ctenolepisma villosa) andmoth fish (Lepisma saccharina);
and the others.

Acari:

from the family Tetranychidae, for example, common red spider mite (Tetranychus urticae), kanzawa spider mite (Tetranychus kanzawai), red spider mite (Tetranychus evansi), citrus red mite (Panonychus citri), fruit-tree red spider mite (Panonychus ulmi), and Oligonychus spp.;
from the family Eriophyidae, for example, Japanese citrus rust mite (Aculops pelekassi), Phyllocoptruta citri, tomato mite (Aculops lycopersici), purple mite (Calacarus carinatus), tea rust mite (Acaphylla theavagrans), Eriophyes chibaensis, apple bud mite (Aculus schlechtendali), Aceria diospyri, Aceria tosichella, and Shevtchenkella sp.;
from the family Tarsonemidae, for example, broad mite (Polyphagotarsonemus latus);
from the family Tenuipalpidae, for example, Brevipalpus phoenicis;
from the family Tuckerellidae;
from the family Ixodidae, for example, Haemaphysalis longicornis, Haemaphysalis flava, Haemaphysalis japonica, Haemaphysalis campanulata, American dog tick (Dermacentor variabilis), Dermacentor taiwanensis, Rocky Mountain wood tick (Dermacentor andersoni), netted tick (Dermacentor reticulatus), Ixodes ovatus, Ixodes persulcatus, black-legged tick (Ixodes scapularis), Ixodes pacificus, Ixodes holocyclus, Ixodes ricinus, lone star tick (Amblyomma americanum), gulf coast tick (Amblyomma maculatum), Rhipicephalus microplus, cattle tick (Rhipicephalus annulatus), brown dog tick (Rhipicephalus sanguineus), Rhipicephalus appendiculatus, and Rhipicephalus decoloratus;
from the family Argasidae, for example, fowl tick (Argas persicus), Ornithodoros hermsi, and Ornithodoros turicata;
from the family Acaridae, for example, cereal mite (Tyrophagus putrescentiae) and grassland mite (Tyrophagus similis);
from the family Pyroglyphidae, for example, American house dust mite (Dermatophagoides farinae) and European house dust mite (Dermatophagoides pteronyssinus);
from the family Cheyletidae, for example, Cheyletus eruditus, Cheyletus malaccensis, Chelacaropsis moorei, and Cheyletiella yasguri;
from the family Psoroptidae, for example, sheep scab mite (Psoroptes ovis), horse psoroptic mange mite (Psoroptes

equi), Knemidocoptes mutans, ear mange mite (Otodectes cynotis), and Chorioptes spp.;
from the family Sarcoptidae, for example, Notoedres cati, Notoedres muris, and itch mite (Sarcoptes scabiei);
from the family Listrophoridae, for example, Listrophorus gibbus;
from the family Dermanyssidae, for example, bird mite (Dermanyssus gallinae);
from the family Macronyssidae, for example, feather mite (Ornithonyssus sylviarum) and tropical rat mite (Ornithonyssus bacoti);
from the family Varroidae, for example, Varroa jacobsoni;
from the family Demodicidae, for example, dog follicle mite (Demodex canis) and cat follicle mite (Demodex cati);
from the family Trombiculidae, for example, Leptotrombidium akamushi, Leptotrombidium pallidum, and Leptotrombidium scutellare;

and the others.

Araneae:

from the family Eutichuridae, for example, Cheiracanthium japonicum;
from the family Theridiidae, for example, red-back spider (Latrodectus hasseltii);

and the others

Polydesmida:

from the family Paradoxosomatidae, for example, flat-backed millipede (Oxidus gracilis) and Nedyopus tambanus; and the others.

Isopoda:

from the family Armadillidiidae, common pill bug (Armadillidium vulgare);
and the others.

Chilopoda:

from the family Scutigeridae, for example, Thereuonema hilgendorfi;
from the family Scolopendridae, giant tropical centipede (Scolopendra subspinipes);
from the family Ethopolyidae, Bothropolys rugosus; and the others.

Gastropoda:

from the family Limacidae, for example, tree slug (Limax marginatus) and garden tawny slug (Limax flavus);
from the family Philomycidae, for example, Meghimatium bilineatum;
from the family Ampullariidae, for example, golden apple snail (Pomacea canaliculata);
from the family Lymnaeidae, for example, Austropeplea ollula;

and the others.

Nematoda:

from the family Aphelenchoididae, for example, rice white-tip nematode (Aphelenchoides besseyi);
from the family Pratylenchidae, for example, root lesion nematode (Pratylenchus coffeae), Pratylenchus brachyurus, California meadow nematode (Pratylenchus neglectus), and Radopholus similis;

from the family Heteroderidae, for example, javanese root-knot nematode (Meloidogyne javanica), southern root-knot nematode (Meloidogyne incognita), guava root-knot nematodes (Meloidogyne enterolobii), northern root-knot nematode (Meloidogyne hapla), Columbia root-knot nematode (Meloidogyne chitwoodi), soybean cyst nematode (Heterodera glycines), potato cyst nematode (Globodera rostochiensis), and white potato cyst nematode (Globodera pallida);
from the family Hoplolaimidae, for example, Rotylenchulus reniformis;
from the family Anguinidae, for example, strawberry bud nematode (Nothotylenchus acris), and stem nematode (Ditylenchus dipsaci);
from the family Tylenchulidae, for example, citrus nematode (Tylenchulus semipenetrans);
from the family Longidoridae, for example, dagger nematode (Xiphinema index);
from the family Trichodoridae;

from the family Parasitaphelenchidae, for example, pine wilt disease (Bursaphelenchus xylophilus);
and the others.

The harmful arthropods such as harmful insects and harmful mites, harmful mollusks and harmful nematodes may be those having a reduced susceptibility to or a developed resistance to an insecticide, a mitecide, a molluscicide or a nematicide.

The method for controlling harmful arthropods of the present invention is carried out by applying an effective amount of the Present compound or the Composition A to a harmful arthropod directly and/or a habitat thereof (for example, plant bodies, soil, an interior of a house, animal bodies). Examples of the method for controlling harmful arthropods of the present invention include foliage treatment, soil treatment, root treatment, shower treatment, smoking treatment, water surface treatment and seed treatment.

The Present compound or the Composition A is usually used by mixing it with inert carrier(s) such as solid carrier(s), liquid carrier(s), and gaseous carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, a tablet, an aerosol formulation, a resin formulation, or the like. In addition to these formulations, the Present compound or the Composition A may be used by formulating it into a dosage form described in Manual on development and use of FAO and WHO Specifications for pesticides, FAO Plant Production and Protection Papers- 271-276, prepared by the FAO/WHO Joint Meeting on Pesticide Specifications, 2016, ISSN:0259-2517.

These formulations usually comprise 0.0001 to 99% by weight ratio of the Present compound or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the gaseous carrier include fluorocarbon, butane gas, LPG (liquefied petroleum gas), dimethyl ether, nitrogen, and carbon dioxide.

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Moreover, some adjuvants can be employed to improve or help the efficacy of the Present compound. The specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered

The plants as used herein include entire plant, foliages, flowers, ears, fruits, stems, branches, tree canopies, seeds, vegetative reproductive organs, and seedlings.

The vegetative reproductive organs represent a part of plant which have the ability to grow when the part is separated from the body and placed in soil, among the roots, stems, leaves and the like of the plant. Examples of the vegetative reproductive organs include tuberous root, creeping root, bulb, corm or solid bulb, tuber, rhizome, stolon, rhizophore, cane cuttings, propagule, and vine cutting. Here the stolon is also called runner, propagule is also called bulbils, which is divided into broad bud and bulblets. The vines represent shoots (generic name for leaves and stems) of sweet potato and Japanese yam. Discoid stem, corm, tuber, rhizome, stem fragments, rhizophore and tuberous root are also collectively referred to bulbs. For example, though a cultivation of potato begins by planting tubers in soil, the tubers used are generally called seed potatoes.

Examples of a method for controlling pests by applying an effective amount of the Present compound or the Composition A to soils include a method of applying an effective amount of the Present compound or the Composition A to soils before planting plants or after planting plants, a method of applying an effective amount of the Present compound or the Composition A to a root part of a crop to be protected from damage such as ingestion by harmful arthropods, and a method of controlling harmful arthropods that ingest a plant by permeating and transferring an effective amount of the Present compound or the Composition A from a root into the interior of the plant body. Specifically, examples of the application method include planting hole treatment (spraying into planting holes, soil mixing after planting hole treatment), plant foot treatment (plant foot spraying, soil mixing after plant foot treatment, irrigation at plant foot, plant foot treatment at a later seeding raising stage) , planting furrow treatment (planting furrow spraying, soil mixing after planting furrow treatment), planting row treatment (planting row spraying, soil mixing after planting row treatment, planting row spraying at a growing stage), planting row treatment at the time of sowing (planting row spraying at the time of sowing, soil mixing after planting row treatment at the time of sowing), broadcast treatment (overall soil surface spraying, soil mixing after broadcast treatment), side-article treatment, treatment of water surface (application to water surface, application to water surface after flooding), other soil spraying treatment (spraying of a granular formulation on leaves at a growing stage, spraying under a canopy or around a tree stem, spraying on the soil surface, mixing with surface soil, spraying into seed holes, spraying on the ground surfaces of furrows, spraying between plants), other irrigation treatment (soil irrigation, irrigation at a seedling raising stage, chemical solution injection treatment, irrigation of a plant part just above the ground, chemical solution drip irrigation, chemigation), seedling raising box treatment (spraying into a seedling raising box, irrigation of a seedling raising box, flooding into a seedling raising box with chemical solution), seedling raising tray treatment (spraying on a seedling raising tray, irrigation of a seedling raising tray, flooding into a seedling raising tray with chemical solution), seedbed treatment (spraying on a seedbed, irrigation of a seedbed, spraying on a lowland rice nursery, immersion of seedlings), seedbed soil incorporation treatment (mixing with seedbed soil, mixing with seedbed soil before sowing, spraying at sowing before covering with soils, spraying at sowing after covering with soils, mixing with covering with soils), and other treatment (mixing with culture soil, plowing under, mixing with surface soil, mixing with soil at the place where raindrops fall from a canopy, treatment at a planting position, spraying of a granule formulation on flower clusters, mixing with a paste fertilizer).

Examples of the application to seeds (or seed treatments) include an application of the Present compound or the Composition A to seeds or vegetative reproductive organs, and specific examples thereof include spraying treatment in which a suspension of the Present compound or the Composition A is sprayed onto seed surface or the vegetative reproductive organ surface in the form of mist; smearing treatment in which the Present compound or the Composition A is coated a surface of seeds or the vegetative reproductive organ; a soaking treatment in which the seeds are soaked into the solution of the Present compound or the Composition A for a certain time; and a method for coating the seeds or the vegetative reproductive organ with a carrier containing the Present compound or the Composition A (film coating treatment, pellet coating treatment). Examples of the above-described vegetative reproductive organ include particularly seed potato.

When the Composition A is applied to seeds or vegetative reproductive organs, the Composition A may be also applied to seeds or vegetative reproductive organs as a single formulation, or the Composition A may be applied to seeds or vegetative reproductive organs as multiple different formulations by multiple times. Examples of the method in which the Composition A is applied as multiple different formulations by multiple times include, for example, a method in which the formulations comprising as an active component the Present compound only are applied, and seeds or vegetative reproductive organs are air dried, followed by applying the formulations comprising the Present ingredient: and a method in which the formulations comprising as an active component the present compound and the Present ingredients are applied, and seeds or vegetative reproductive organs are air dried, followed by applying the formulations comprising the Present

ingredients other than the already-applied Present ingredients, are included.

As used herein, seeds or vegetative reproductive organs carrying the present compound or the Composition A means seeds or vegetative reproductive organs in the state where the present compound or the Composition A is adhered to a surface of the seeds or the vegetative reproductive organ. The above-described seeds or vegetative reproductive organs carrying the present compound or the Composition A may be adhered by any other materials that are different from the present compound or the Composition A before or after being adhered the present compound or the Composition A to the seeds or vegetative reproductive organs.

Also, when the Composition A is adhered in a form of layer(s) to a surface of seeds or vegetative reproductive organ, the layer(s) is/are composed of one layer or a multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs carrying the present compound or the Composition A can be obtained, for example, by applying the formulations comprising the present compound or the Composition A by the above-described application method to seeds to seeds or vegetative reproductive organs.

When the Present compound or the Composition A is applied for controlling pests in agricultural fields, the application dose thereof is usually within a range of 1 to 10,000 g of the Present compound per 10,000 $m^2$. In the case of being applied to seeds or vegetative reproductive organs, the dose of application dose thereof is usually within a range of 0.001 to 100 g of the Present compound per 1 Kg of seeds. When the Present compound or the Composition A is formulated into an emulsifiable concentrate, a wettable powder or a flowable etc., they are usually applied by diluting them with water so as to make an effective concentration of the active ingredients 0.01 to 10,000 ppm, and the dust formulation or the granular formulation, etc., is usually applied as itself without diluting them.

Also, for the Present compound or the Composition A, the resin preparation which is processed into a sheet or a string may be applied by winding a plant with a sheet or a string of the resin preparation, putting a string of the resin preparation around a crop so that the plant is surrounded by the string, or laying a sheet of the resin preparation on the soil surface near the root of a plant.

When the Present compound or the Composition A is used to control harmful arthropods that live inside a house, the application dose as an amount of the Present compound is usually within a range from 0.01 to 1,000 mg per 1 $m^2$ of an area to be treated, in the case of using it on a planar area. In the case of using it spatially, the application dose as an amount of the Present compound is usually within a range from 0.01 to 500 mg per 1 $m^3$ of the space to be treated. When the Present compound or the Composition A is formulated into emulsifiable concentrates, wettable powders, flowables or the others, such formulations are usually applied after diluting it with water in such a way that a concentration of the active ingredient is within a range from 0.1 to 10,000 ppm. In the case of being formulated into oil solutions, aerosols, smoking agents, poison baits and the others, such formulations are used as itself without diluting it.

When the Present compound or the composition A is used for controlling external parasites of livestock such as cows, horses, pigs, sheep, goats and chickens and small animals such as dogs, cats, rats and mice, the composition of the present invention may be applied to the animals by a known method in the veterinary field. Specifically, when systemic control is intended, the composition of the present invention is administered to the animals as a tablet, a mixture with feed or a suppository, or by injection (including intramuscular, subcutaneous, intravenous and intraperitoneal injections). On the other hand, when non-systemic control is intended, the composition of the present invention is applied to the animals by means of spraying of the oil solution or aqueous solution, pour-on or spot-on treatment, or washing of the animal with a shampoo formulation, or by putting a collar or ear tag made of the resin formulations to the animal. In the case of being administered to an animal body, the dose of the compound of the Present compound is usually within a range from 0.1 to 1,000 mg per 1 kg of an animal body weight.

Also, the composition of the Present compound or the Composition A may be used as an agent for controlling pests in agricultural lands such as paddy fields, fields, turfs, and orchards. Examples of the plants include the followings. corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish,

kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

EXAMPLES

Hereinafter, the present invention is explained in more detail by using Preparation examples, Formulation examples, and Test examples, however, the present invention should not be limited to these examples.

As used herein, "Me" represents a methyl group, "Et" represents an ethyl group, "Pr" represents a propyl group, "i-Pr" represents an isopropyl group, t-Bu" represents a tert-butyl group, "c-Pr" represents a cyclopropyl group, "c-Bu" represents a cyclobutyl group, "Ph" represents a phenyl group, "Bn" represents a benzyl group, "Py2" represents a 2-pyridyl group, "Py3" represents a 3-pyridyl group, "Py4" represents a 4-pyridyl group. When c-Pr, c-Bu, Ph, Py2, Py3, Py4, and Het have a substituent, the substituent is written with its substituted position before the symbol. For example, "1-CN-c-Pr" represents a 1-cyanocyclopropyl group, "3,4-$F_2$-Ph" represents a 3,4-difluorophenyl group, "4-$S(O)_2CF_3$-Ph" represents a 4-(trifluoromethanesulfonyl)phenyl group, "3,4-$(OCF_3)_2$-Ph" represents a 3,4-bis(trifluoromethoxy)phenyl group, "3-$SCF_3$-4-$OCF_3$-Ph" represents a 3-[(trifluoromethyl)thio]-4-(trifluoromethoxy)phenyl group, and "4-$CF_3$-Py2" represents a 4-(trifluoromethyl)-2-pyridyl group.

Firstly, the preparation examples of the present compound and the preparation intermediate thereof are described.

When a physical property of a compound is determined using a lipid chromatography/mass spectrography (hereinafter, referred to as "LCMS"), a determined molecular ion value $[M+H]^+$ or $[M-H]^-$ and retention time (hereinafter, referred to as "RT") are recorded. A condition for the lipid chromatography (hereinafter, referred to as "LC") is recited as follows.

[LC condition]

Column: L-column2 ODS, inner diameter 4.6 mm, length 30 mm, particle size 3 μm (manufactured by Chemicals Evaluation and Research Institute, Japan)
UV measurement wavelength: 254 nm
Mobile phase: Solution A: 0.1% formic acid in water, Solution B: 0.1% formic acid in acetonitrile
Flow rate: 2.0 mL/min
Gradient condition: the solutions are run with the concentration gradients shown in [Table LC1].

[Table LC1]

| Time (min) | Solution A (%) | Solution B (%) |
|---|---|---|
| 0.01 | 90 | 10 |

(continued)

| Time (min) | Solution A (%) | Solution B (%) |
|---|---|---|
| 2.00 | 0 | 100 |
| 4.00 | 0 | 100 |
| 4.01 | 90 | 10 |

Reference preparation example 1

To a mixture of 0.3 g sodium hydride and 40 mL THF was added 0.63 g 2-amino-5,6,7-trifluorobenzothiazole under ice-cooling, and the mixture was stirred for 30 minutes. Thereafter thereto was added 1.1 g 3-(ethylthio)-6-iodo-imidazopyridin-2-carboxylate chloride, and the mixture was stirred at room temperature for 4 hours, and water was added to the resulting mixture, and the precipitated out solids were separated by filtering, dried to obtain 0.72 g an intermediate compound 15.

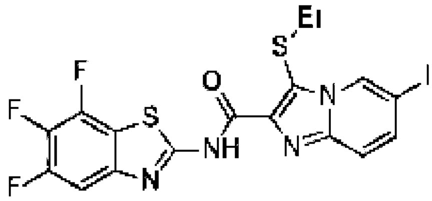

The intermediate compound 15: LCMS:535[M+H]$^+$, RT=2.419 min.

Reference preparation example 1-1

The compounds prepared according to a similar method to that described in the Reference preparation example 1, and their physical properties are shown as follows.
A compound represented by formula (A-1):

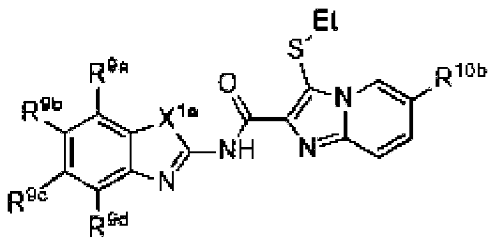

(A-1)

wherein a combination of $R^{9a}$, $R^{9b}$, $R^{9c}$, $R^{9d}$, $R^{10b}$ and $X^{1a}$ represents any of the combinations indicated in [Table A-1].

[Table A-1]

| Intermediate compound | $R^{9a}$ | $R^{9b}$ | $R^{9c}$ | $R^{9d}$ | $X^{1a}$ | $R^{10b}$ |
|---|---|---|---|---|---|---|
| 1 | F | H | H | H | S | I |
| 2 | H | F | H | H | S | I |
| 3 | H | H | F | H | S | I |
| 4 | H | H | H | F | S | I |
| 5 | H | $CF_3$ | H | H | S | I |
| 6 | Cl | H | H | H | S | I |
| 7 | H | Cl | H | H | S | I |
| 8 | H | H | Cl | H | S | I |
| 9 | Br | H | H | H | S | I |

(continued)

| Intermediate compound | $R^{9a}$ | $R^{9b}$ | $R^{9c}$ | $R^{9d}$ | $X^{1a}$ | $R^{10b}$ |
|---|---|---|---|---|---|---|
| 10 | H | H | H | H | S | I |
| 11 | H | $CH_3$ | H | H | S | I |
| 12 | F | F | H | H | S | I |
| 13 | H | F | F | H | S | I |
| 14 | H | F | H | F | S | I |
| 16 | H | Cl | Cl | H | S | I |
| 17 | H | Cl | H | Cl | S | I |
| 18 | F | H | H | H | O | I |
| 19 | H | F | H | H | O | I |
| 20 | H | H | Cl | H | O | I |
| 21 | H | F | F | H | O | I |

The intermediate compound 1: $^1$H-NMR ($CDCl_3$) δ: 10.90 (1H, s), 8.82 (1H, dd), 7.63 (1H, dd), 7.57 (1H, dd), 7.46-7.43 (1H, m), 7.42-7.38 (1H, m), 7.07-7.02 (1H, m), 3.08 (2H, q), 1.25 (3H, t).
The intermediate compound 2: $^1$H-NMR ($CDCl_3$) δ: 8.82 (1H, s), 7.77 (1H, dd), 7.58-7.53 (2H, m), 7.44 (1H, d), 7.19 (1H, td), 3.07 (2H, q), 1.25 (3H, t).
The intermediate compound 3: LCMS:499[M+H]$^+$, RT=2.290 min.
The intermediate compound 4: LCMS:499[M+H]$^+$, RT=2.239 min.
The intermediate compound 5: $^1$H-NMR ($CDCl_3$) δ: 8.82 (1H, dd), 8.14 (1H, s), 7.91 (1H, d), 7.70 (1H, d), 7.59-7.57 (1H, m), 7.45 (1H, dd), 3.07 (2H, q), 1.25 (3H, t).
The intermediate compound 6: LCMS:515[M+H]$^+$, RT=2.438 min.
The intermediate compound 7: LCMS:515[M+H]$^+$, RT=2.386 min.
The intermediate compound 8: LCMS:515[M+H]$^+$, RT=2.385 min.
The intermediate compound 9: LCMS:561[M+H]$^+$, RT=2.459 min.
The intermediate compound 10: LCMS:481[M+H]$^+$, RT=2.209 min.
The intermediate compound 11: LCMS:495[M+H]$^+$, RT=2.319 min.
The intermediate compound 12: LCMS:517[M+H]$^+$, RT=2.358 min.
The intermediate compound 13: LCMS:517[M+H]$^+$, RT=2.292 min.
The intermediate compound 14: LCMS:517[M+H]$^+$, RT=2.291 min.
The intermediate compound 16: LCMS:549[M+H]$^+$, RT=2.508 min.
The intermediate compound 17: LCMS:551[M+H]$^+$, RT=2.532 min.
The intermediate compound 18: LCMS:483[M+H]$^+$, RT=2.094 min.
The intermediate compound 19: LCMS:483[M+H]$^+$, RT=1.966 min.
The intermediate compound 20: LCMS:499[M+H]$^+$, RT=2.090 min.
The intermediate compound 21: $^1$H-NMR ($CDCl_3$) δ: 10.30 (1H, s), 8.82 (1H, s), 7.57 (1H, dd), 7.47 (1H, dd), 7.43 (1H, dd), 7.36 (1H, dd), 3.12 (2H, q), 1.23 (3H, t).

Preparation example 1

To a mixture of 0.72 g the intermediate compound 15 and 30 mL N,N-dimethylformamide were added 1.3 g cesium carbonate and 0.17 mL iodomethane under ice-cooling, and the mixture was stirred at 0°C for 7 hours, and water was added to the resulting mixture, and the precipitated out solids were separated by filtering and dried. The resulting residue was subjected to a silica gel column chromatography to obtain 0.21 g the present compound 15.

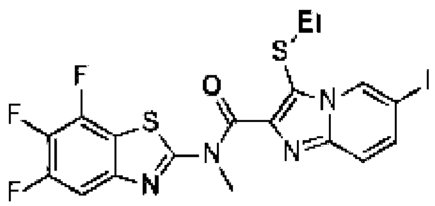

The present compound 15: $^1$H-NMR ($CDCl_3$) δ: 8.81 (1H, dd), 7.58 (1H, dd), 7.48 (2H, dd), 3.91 (3H, s), 2.96 (2H, q), 1.24 (3H, t).

Preparation example 1-1

The compounds prepared according to a similar method to that described in the Preparation example 1, and their physical properties are shown as follows.
A compound represented by formula (A-2):

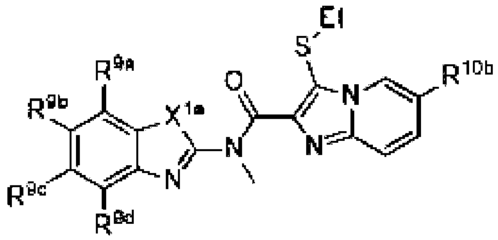

(A-2)

wherein a combination of $R^{9a}$, $R^{9b}$, $R^{9c}$, $R^{9d}$, $R^{10b}$ and $X^{1a}$ represents any of the combinations indicated in [Table A-2].

[Table A-2]

| Present compound | $R^{9a}$ | $R^{9b}$ | $R^{9c}$ | $R^{9d}$ | $X^{1a}$ | $R^{10b}$ |
|---|---|---|---|---|---|---|
| 1 | F | H | H | H | S | I |
| 2 | H | F | H | H | S | I |
| 3 | H | H | F | H | S | I |
| 4 | H | H | H | F | S | I |
| 5 | H | $CF_3$ | H | H | S | I |
| 6 | Cl | H | H | H | S | I |
| 7 | H | Cl | H | H | S | I |
| 8 | H | H | Cl | H | S | I |
| 9 | Br | H | H | H | S | I |
| 10 | H | H | H | H | S | I |
| 11 | H | $CH_3$ | H | H | S | I |
| 12 | F | F | H | H | S | I |
| 13 | H | F | F | H | S | I |
| 14 | H | F | H | F | S | I |
| 16 | H | Cl | Cl | H | S | I |
| 17 | H | Cl | H | Cl | S | I |
| 18 | F | H | H | H | O | I |
| 19 | H | F | H | H | O | I |
| 20 | H | H | Cl | H | O | I |

(continued)

| Present compound | $R^{9a}$ | $R^{9b}$ | $R^{9c}$ | $R^{9d}$ | $X^{1a}$ | $R^{10b}$ |
|---|---|---|---|---|---|---|
| 21 | H | F | F | H | O | I |

The present compound 1: $^1$H-NMR ($CDCl_3$) δ: 8.81 (1H, dd) , 7.70 (1H, d), 7.57 (1H, dd), 7.48 (1H, t), 7.44-7.39 (1H, m), 7.05 (1H, t), 3.92 (3H, s), 2.96 (2H, q), 1.25 (3H, t).
The present compound 2: $^1$H-NMR ($CDCl_3$) δ: 8.79 (1H, dd) , 7.83 (1H, dd), 7.57 (1H, dd), 7.52 (1H, dd), 7.48 (1H, dd) , 7.18 (1H, td), 3.89 (3H, s), 2.94 (2H, q), 1.23 (3H, t).
The present compound 3: $^1$H-NMR ($CDCl_3$) δ: 8.79 (1H, s), 7.76 (1H, dd), 7.60-7.55 (2H, m), 7.48 (1H, t), 7.10 (1H, td) , 3.90 (3H, s), 2.94 (2H, q), 1.23 (3H, t).
The present compound 4: $^1$H-NMR ($CDCl_3$) δ: 8.80 (1H, dd), 7.61 (1H, dd), 7.57 (1H, dd), 7.49 (1H, dd), 7.31-7.27 (1H, m), 7.17 (1H, ddd), 3.94 (3H, s), 2.93 (2H, q), 1.23 (3H, t) .
The present compound 5: $^1$H-NMR ($CDCl_3$) δ: 8.80 (1H, s), 8.12 (1H, s), 7.95 (1H, d), 7.69 (1H, d), 7.57 (1H, d), 7.47 (1H, d), 3.93 (3H, s), 2.95 (2H, q), 1.24 (3H, t).
The present compound 6: $^1$H-NMR ($CDCl_3$) δ: 8.80 (1H, d), 7.78 (1H, t), 7.57 (1H, dd), 7.48 (1H, d), 7.40 (1H, t), 7.32 (1H, t), 3.91 (3H, s), 2.96 (2H, q), 1.24 (3H, t).
The present compound 7: $^1$H-NMR ($CDCl_3$) δ: 8.80 (1H, dd), 7.80-7.79 (2H, m), 7.57 (1H, dd), 7.48 (1H, dd), 7.41 (1H, dd), 3.90 (3H, s), 2.94 (2H, q), 1.24 (3H, t).
The present compound 8: LCMS:529[M+H]$^+$, RT=2.388 min.
The present compound 9: $^1$H-NMR ($CDCl_3$) δ: 8.80 (1H, s), 7.82 (1H, t), 7.57 (1H, dd), 7.47 (2H, t), 7.34 (1H, t), 3.91 (3H, s), 2.96 (2H, q), 1.24 (3H, t).
The present compound 10: LCMS:495[M+H]$^+$, RT=2.208 min.
The present compound 11: $^1$H-NMR ($CDCl_3$) δ: 8.79 (1H, dd), 7.78 (1H, d), 7.64-7.62 (1H, m), 7.56 (1H, dd), 7.47 (1H, dd), 7.28 (1H, d), 3.88 (3H, s), 2.93 (2H, q), 2.49 (3H, s), 1.23 (3H, t).
The present compound 12: LCMS:531[M+H]$^+$, RT=2.357 min.
The present compound 13: $^1$H-NMR ($CDCl_3$) δ: 8.80 (1H, dd), 7.66-7.59 (3H, m), 7.48 (1H, dd), 3.89 (3H, s), 2.94 (2H, q), 1.23 (3H, t).
The present compound 14: LCMS:531[M+H]$^+$, RT=2.291 min.
The present compound 16: $^1$H-NMR ($CDCl_3$) δ: 8.80 (1H, dd), 7.97 (1H, s), 7.90 (1H, s), 7.58 (1H, dd), 7.48 (1H, dd), 3.90 (3H, s), 2.95 (2H, q), 1.24 (3H, t).
The present compound 17: $^1$H-NMR ($CDCl_3$) δ: 8.81-8.78 (1H, m), 7.71 (1H, d), 7.57-7.56 (1H, m), 7.50-7.48 (2H, m), 3.95 (3H, s), 2.93 (2H, q), 1.23 (3H, t).
The present compound 18: $^1$H-NMR ($CDCl_3$) δ: 8.74 (1H, s), 7.40 (1H, dd), 7.32 (1H, dd), 7.21-7.17 (1H, m), 7.16-7.13 (1H, m), 6.98-6.93 (1H, m), 3.73 (3H, s), 2.95 (2H, q), 1.25 (3H, t).
The present compound 19: LCMS:497[M+H]$^+$, RT=2.007 min.
The present compound 20: LCMS:513[M+H]$^+$, RT=2.130 min.
The present compound 21: $^1$H-NMR ($CDCl_3$) δ: 8.75 (1H, s), 7.41 (1H, dd), 7.34 (1H, dd), 7.14 (1H, d), 7.10 (1H, dd), 3.69 (3H, s), 2.96 (2H, q), 1.25 (3H, t).

Preparation example 2

To a mixture of 0.21 g the present compound 15 and 10 mL chloroform was added 0.24 g m-chloroperbenzoic acid under ice-cooling, and the mixture was stirred at room temperature for 4 hours, and to the resulting mixture were added sodium thiosulfate and saturated aqueous sodium hydrocarbonate solution, and the mixture was extracted with chloroform. The resulting organic layer was dried over magnesium sulfate, concentrated under reduced pressure to obtain 0.17 g the present compound 36.

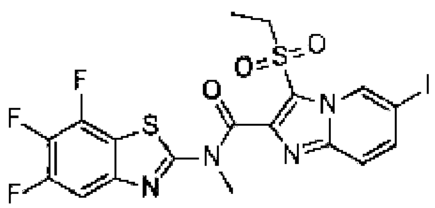

The present compound 36: $^1$H-NMR ($CDCl_3$) δ: 9.24 (1H, s), 7.77 (1H, dd), 7.60 (1H, d), 7.52-7.49 (1H, m), 3.77 (3H, s), 3.55 (2H, q), 1.43 (3H, t).

Preparation example 2-1

The compounds prepared according to a similar method to that described in the Preparation example 2, and their physical properties are shown as follows.

A compound represented by formula (A-3):

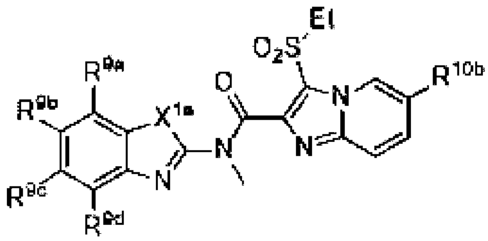

(A-3)

wherein a combination of $R^{9a}$, $R^{9b}$, $R^{9c}$, $R^{9d}$, $R^{10b}$ and $X^{1a}$ represents any of the combinations indicated in [Table A-3].

[Table A-3]

| Present compound | $R^{9a}$ | $R^{9b}$ | $R^{9c}$ | $R^{9d}$ | $X^{1a}$ | $R^{10b}$ |
|---|---|---|---|---|---|---|
| 22 | F | H | H | H | S | I |
| 23 | H | F | H | H | S | I |
| 24 | H | H | F | H | S | I |
| 25 | H | H | H | F | S | I |
| 26 | H | $CF_3$ | H | H | S | I |
| 27 | Cl | H | H | H | S | I |
| 28 | H | Cl | H | H | S | I |
| 29 | H | H | Cl | H | S | I |
| 30 | Br | H | H | H | S | I |
| 31 | H | H | H | H | S | I |
| 32 | H | $CH_3$ | H | H | S | I |
| 33 | F | F | H | H | S | I |
| 34 | H | F | F | H | S | I |
| 35 | H | F | H | F | S | I |
| 37 | H | Cl | Cl | H | S | I |
| 38 | H | Cl | H | Cl | S | I |
| 39 | F | H | H | H | O | I |
| 40 | H | F | H | H | O | I |
| 41 | H | H | Cl | H | O | I |
| 42 | H | F | F | H | O | I |
| 43 | H | F | F | H | S | Br |

(continued)

| Present compound | $R^{9a}$ | $R^{9b}$ | $R^{9c}$ | $R^{9d}$ | $X^{1a}$ | $R^{10b}$ |
|---|---|---|---|---|---|---|
| 44 | F | F | F | H | S | Br |

The present compound 22: $^1$H-NMR ($CDCl_3$) δ: 9.25 (1H, dd), 7.77 (1H, dd), 7.70 (1H, dd), 7.60 (1H, dd), 7.45-7.32 (1H, m), 7.09-7.04 (1H, m), 3.80 (3H, s), 3.56 (2H, q), 1.42 (3H, t).
The present compound 23: $^1$H-NMR ($CDCl_3$) δ: 9.24 (1H, s), 7.83 (1H, dd), 7.76 (1H, t), 7.59 (1H, d), 7.54-7.49 (1H, m), 7.20 (1H, ddt), 3.77 (3H, s), 3.55 (2H, q), 1.41 (3H, t) .
The present compound 24: $^1$H-NMR ($CDCl_3$ ) δ: 9.24 (1H, d), 7.78-7.74 (2H, m), 7.60-7.57 (2H, m), 7.12 (1H, dd), 3.78 (3H, s), 3.55 (2H, q), 1.41 (3H, t).
The present compound 25: $^1$H-NMR ($CDCl_3$) δ: 9.24 (1H, dd), 7.76 (1H, dd), 7.62-7.59 (2H, m), 7.33-7.29 (1H, m), 7.18 (1H, t), 3.83 (3H, s), 3.55 (2H, q), 1.42 (3H, t).
The present compound 26: $^1$H-NMR ($CDCl_3$) δ: 9.24 (1H, s), 8.12 (1H, s), 7.97 (1H, d), 7.77 (1H, t), 7.71 (1H, d), 7.60 (1H, d), 3.81 (3H, s), 3.56 (2H, q), 1.42 (3H, t).
The present compound 27: $^1$H-NMR ($CDCl_3$) δ: 9.25 (1H, t), 7.81-7.74 (2H, m), 7.60 (1H, d), 7.41 (1H, t), 7.33 (1H, t), 3.79 (3H, s), 3.57 (2H, q), 1.42 (3H, t).
The present compound 28: $^1$H-NMR ($CDCl_3$) δ: 9.24 (1H, s), 7.80-7.76 (3H, m), 7.59 (1H, d), 7.42 (1H, dd), 3.78 (3H, s), 3.55 (2H, q), 1.42 (3H, t).
The present compound 29: $^1$H-NMR ($CDCl_3$ ) δ: 9.24 (1H, s), 7.89 (1H, d), 7.76-7.75 (2H, m), 7.60 (1H, d), 7.32 (1H, dd), 3.78 (3H, s), 3.55 (2H, q), 1.42 (3H, t).
The present compound 30: $^1$H-NMR ($CDCl_3$ ) δ: 9.25 (1H, s), 7.83 (1H, d), 7.76 (1H, dd), 7.60 (1H, d), 7.47 (1H, d), 7.35 (1H, t), 3.78 (3H, s), 3.57 (2H, q), 1.42 (3H, t).
The present compound 31: $^1$H-NMR ($CDCl_3$) δ: 9.25 (1H, dd), 7.90 (1H, d), 7.84 (1H, d), 7.76 (1H, dd), 7.59 (1H, d), 7.48-7.46 (1H, m), 7.37-7.34 (1H, m), 3.80 (3H, s), 3.56 (2H, q), 1.41 (3H, t).
The present compound 32: $^1$H-NMR ($CDCl_3$) δ: 9.25 (1H, s), 7.78-7.75 (2H, m), 7.61-7.59 (2H, m), 7.30-7.28 (1H, m), 3.78 (3H, s), 3.56 (2H, q), 2.49 (3H, s), 1.41 (3H, t).
The present compound 33: $^1$H-NMR ($CDCl_3$) δ: 9.24 (1H, s), 7.77 (1H, dd), 7.64-7.59 (2H, m), 7.32-7.30 (1H, m), 3.77 (3H, s), 3.56 (2H, q), 1.43 (3H, t).
The present compound 34: $^1$H-NMR ($CDCl_3$) δ: 9.24 (1H, s), 7.77 (1H, dd), 7.68 (1H, dd), 7.61-7.59 (2H, m), 3.76 (3H, s), 3.55 (2H, q), 1.42 (3H, t).
The present compound 35: $^1$H-NMR ($CDCl_3$) δ: 9.23 (1H, s), 7.77 (1H, dd), 7.60 (1H, d), 7.33 (1H, d), 6.99 (1H, t), 3.80 (3H, s), 3.54 (2H, q), 1.42 (3H, t).
The present compound 37: $^1$H-NMR ($CDCl_3$) δ: 9.24 (1H, t), 7.99 (1H, d), 7.89 (1H, d), 7.77 (1H, dd), 7.59 (1H, d), 3.77 (3H, s), 3.55 (2H, q), 1.42 (3H, t).
The present compound 38: $^1$H-NMR ($CDCl_3$ ) δ: 9.23 (1H, s), 7.76-7.75 (1H, m), 7.71 (1H, d), 7.60 (1H, d), 7.49 (1H, d), 3.82 (3H, s), 3.54 (2H, q), 1.41 (3H, t).
The present compound 39: $^1$H-NMR ($CDCl_3$) δ: 9.26 (1H, dd), 7.64 (1H, dd), 7.36 (1H, d), 7.29 (1H, d), 7.17 (1H, td), 6.95 (1H, t), 3.75 (3H, s), 3.58 (2H, q), 1.40 (3H, t).
The present compound 40: $^1$H-NMR ($CDCl_3$) δ: 9.28 (1H, s), 7.63 (1H, dd), 7.46-7.44 (1H, m), 7.34 (1H, d), 7.01-6.99 (2H, m), 3.72 (3H, s), 3.62 (2H, q), 1.41 (3H, t).
The present compound 41: $^1$H-NMR ($CDCl_3$ δ: 9.27 (1H, s), 7.64 (1H, dd), 7.48 (1H, t), 7.35 (1H, d), 7.16 (2H, d), 3.73 (3H, s), 3.61 (2H, q), 1.41 (3H, t).
The present compound 42: $^1$H-NMR ($CDCl_3$) δ: 9.28 (1H, s), 7.64 (1H, dd), 7.34-7.31 (2H, m), 7.11 (1H, dd), 3.71 (3H, s), 3.62 (2H, q), 1.41 (3H, t).
The present compound 43: $^1$H-NMR ($CDCl_3$) δ: 9.15 (1H, s), 7.72-7.58 (4H, m), 3.77 (3H, s), 3.56 (2H, q), 1.42 (3H, t).
The present compound 44: $^1$H-NMR ($CDCl_3$) δ: 9.16 (1H, s), 7.73-7.65 (2H, m), 7.52-7.50 (1H, m), 3.78 (3H, s), 3.56 (2H, q), 1.43 (3H, t).

Reference preparation example 2

To a mixture of 0.25 g sodium hydride and 40 mL THF was added 0.81 g 2-amino-5,6,7-trifluorobenzothiazole under ice-cooling, and the mixture was stirred for 30 minutes. Thereafter, 1.1g 5-bromo-3-(ethylthio)picolinoyl chloride was added thereto, and the mixture was stirred at room temperature for 4 hours. Water was added to the resulting mixture, and the precipitated out solids were separated by filtering, and dried to obtain 0.79 g an intermediate compound 26.

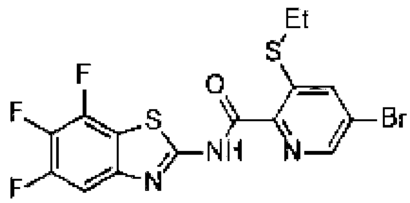

The intermediate compound 26: LCMS:450[M+H]+, RT=2.403 min.

Reference preparation example 2-1

The compounds prepared according to a similar method to that described in the Reference preparation example 2, and their physical properties are shown as follows.

A compound represented by formula (A-4):

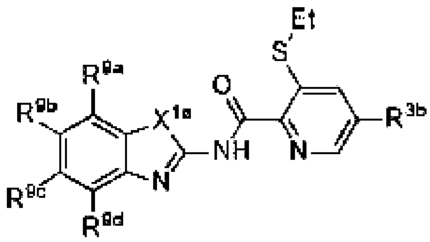 (A-4)

wherein a combination of $R^{9a}$, $R^{9b}$, $R^{9c}$, $R^{9d}$, $R^{3b}$ and $X^{1a}$ represents any of the combinations indicated in [Table A-4] .

[Table A-4]

| Intermediate compound | $R^{9a}$ | $R^{9b}$ | $R^{9c}$ | $R^{9d}$ | $X^{1a}$ | $R^{3b}$ |
|---|---|---|---|---|---|---|
| 24 | H | F | H | H | S | Br |
| 25 | H | F | F | H | S | Br |
| The intermediate compound 24: $^1$H-NMR ($CDCl_3$) δ: 11.20 (1H, s), 8.40 (1H, d), 7.85 (1H, d), 7.76 (1H, dd), 7.54 (1H, dd), 7.19 (1H, td), 2.99 (2H, q), 1.47 (3H, t). The intermediate compound 25: $^1$H-NMR ($CDCl_3$) δ: 11.22 (1H, s), 8.40 (1H, d), 7.85 (1H, d), 7.64-7.57 (2H, m), 2.99 (2H, q), 1.47 (3H, t). | | | | | | |

Reference preparation example 3

A mixture of 2.6 g 3,5-difluoropyridine-2-amine, 2.1 g methyl isothiocyanate, and 10 mL methanol was stirred at 60°C for 9 hours. The resulting mixture was concentrated under reduced pressure, water was added thereto, and the mixture was extracted with chloroform. The resulting organic layer was dried over magnesium sulfate, concentrated under reduced pressure to obtain 1.0 g an intermediate compound 36.

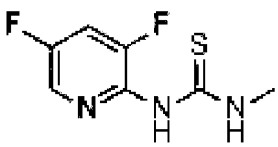

The intermediate compound 36: LCMS : 204[M+H]+, RT=1.364 min.

Reference preparation example 3-1

The compounds prepared according to a similar method to that described in the Reference preparation example 3, and their physical properties are shown as follows.

A compound represented by formula (A-10):

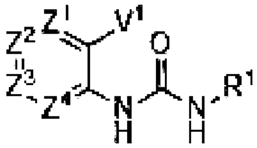

(A-10)

wherein a combination of $Z^1$, $Z^2$, $Z^3$, $Z^4$, $V^1$, and $R^1$ represents any of the combinations indicated in [Table A-10].

[Table A-10]

| Intermediate compound | $Z^1$ | $Z^2$ | $Z^3$ | $Z^4$ | $V^1$ | $R^1$ |
|---|---|---|---|---|---|---|
| 38 | N | CF | CH | CH | F | $CH_3$ |
| 39 | CF | CF | CF | CH | H | $CH_3$ |
| 40 | CH | CF | CF | CH | H | $CH_3$ |
| 41 | CH | CF | CCl | CH | F | $CH_3$ |
| 42 | CH | CF | CF | CH | H | c-Pr |

The intermediate compound 38: $^1$H-NMR ($CDCl_3$) $\delta$: 8.32 (1H, s), 7.42 (1H, s), 6.90 (1H, dd), 6.22 (1H, s), 3.15 (3H, d).
The intermediate compound 39: $^1$H-NMR ($CDCl_3$) $\delta$: 7.79 (1H, s), 6.94 (2H, t), 6.06 (1H, s), 3.16 (3H, d).
The intermediate compound 40: $^1$H-NMR ($CDCl_3$) $\delta$: 7.95 (1H, s), 7.24-7.21 (1H, m), 7.14-7.11 (1H, m), 7.02-7.00 (1H, m), 5.98 (1H, s), 3.14 (3H, d).
The intermediate compound 41: $^1$H-NMR ($CDCl_3$) $\delta$: 7.59 (1H, s), 7.38 (1H, s), 7.06 (1H, t), 6.00 (1H, s), 3.16 (3H, d).
The intermediate compound 42: $^1$H-NMR ($CDCl_3$) $\delta$: 7.91 (1H, s), 7.44 (1H, s), 7.21-7.12 (2H, m), 6.72 (1H, s), 4.78 (1H, s), 2.73 (1H, m), 0.96-0.93 (2H, m), 0.78 (2H, m).

Reference preparation example 4

To a mixture of 0.3 g sodium hydride and 10 mL N,N-dimethylformamide was added 1.0 g the intermediate compound 36 under ice-cooling, and the mixture was stirred at room temperature overnight. Water was added to the resulting mixture, and the mixture was extracted with ethyl acetate. The resulting organic layer was dried over magnesium sulfate, and concentrated under reduced pressure to obtain 0.4 g an intermediate compound 37.

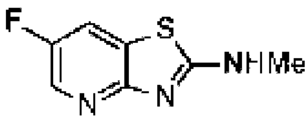

The intermediate compound 37: $^1$H-NMR ($CDCl_3$) $\delta$: 8.25 (1H, dd), 7.63 (1H, dd), 6.44 (1H, s), 3.16 (3H, s).

Reference preparation example 4-1

The compounds prepared according to a similar method to that described in the Reference preparation example 4, and their physical properties are shown as follows.
A compound represented by formula (A-11):

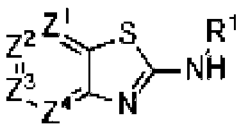

(A-11)

wherein a combination of $Z^1$, $Z^2$, $Z^3$, $Z^4$, and $R^1$ represents any of the combinations indicated in [Table A-11].

[Table A-11]

| Intermediate compound | $Z^1$ | $Z^2$ | $Z^3$ | $Z^4$ | $R^1$ |
|---|---|---|---|---|---|
| 43 | N | CF | CH | CH | $CH_3$ |

(continued)

| Intermediate compound | $Z^1$ | $Z^2$ | $Z^3$ | $Z^4$ | $R^1$ |
|---|---|---|---|---|---|
| 46 | CH | CF | CCl | CH | $CH_3$ |
| The intermediate compound 43: $^1$H-NMR ($CDCl_3$) δ: 7.79 (1H, dd), 6.86 (1H, dd), 5.45 (1H, s), 3.12 (3H, s). The intermediate compound 46: $^1$H-NMR ($CDCl_3$) δ: 7.53 (1H, d), 7.36 (1H, d), 5.47 (1H, s), 3.10 (3H, s). | | | | | |

Reference preparation example 5

To a mixture of 0.4 g 5-fluorothiazolo[5,4-b]pyridin-2-amine and 10 mL pyridine was added 1.1 g 3-(ethylthio)-6-iodo-imidazopyridin-2-carboxylic acid chloride, and the mixture was stirred at 90°C for 3 hours. Water was added to the resulting mixture and the precipitated out solids were filtered, and dried to obtain 1.0 g an intermediate compound 30.

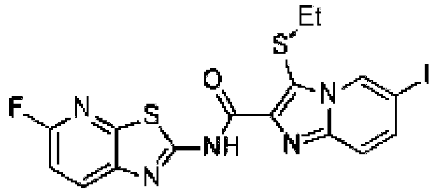

The intermediate compound 30: $^1$H-NMR ($CDCl_3$) δ: 10.78 (1H, s), 8.82 (1H, s), 8.12-8.10 (1H, m), 7.58 (1H, dd), 7.45 (1H, d), 7.03 (1H, dd), 3.07 (2H, q), 1.25 (3H, t).

Reference preparation example 5-1

The compounds prepared according to a similar method to that described in the Reference preparation example 5, and their physical properties are shown as follows.

A compound represented by formula (A-7):

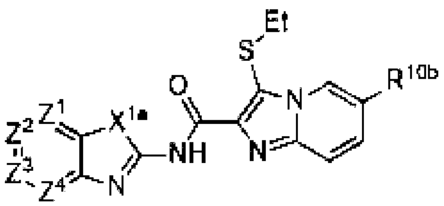

(A-7)

wherein a combination of $Z^1$, $Z^2$, $Z^3$, $Z^4$, $R^{10b}$, and $X^{1a}$ represents any of the combinations indicated in [Table A-7].

[Table A-7]

| Intermediate compound | $Z^1$ | $Z^2$ | $Z^3$ | $Z^4$ | $X^{1a}$ | $R^{10b}$ |
|---|---|---|---|---|---|---|
| 31 | CF | CF | CF | CF | S | I |
| 32 | CH | CF | CH | CH | N-Me | I |
| 33 | CH | CH | CF | CH | N-Me | I |
| 34 | CF | CH | CF | CH | S | I |

(continued)

| Intermediate compound | $Z^1$ | $Z^2$ | $Z^3$ | $Z^4$ | $X^{1a}$ | $R^{10b}$ |
|---|---|---|---|---|---|---|
| 35 | CH | CCl | CF | CH | S | I |
| The intermediate compound 31: $^1$H-NMR ($CDCl_3$) δ: 11.01 (1H, s), 8.82 (1H, s), 7.59 (1H, dd), 7.44 (1H, dd), 3.07 (2H, q), 1.25 (3H, t).<br>The intermediate compound 32: $^1$H-NMR ($CDCl_3$) δ: 8.83-8.82 (1H, m), 7.49-7.48 (2H, m), 7.30-7.29 (1H, m), 7.02-7.00 (2H, m), 3.82 (3H, s), 3.02 (2H, q), 1.21 (3H, t).<br>The intermediate compound 33: $^1$H-NMR ($CDCl_3$) δ: 8.83 (1H, m), 7.49 (2H, s), 7.22-7.20 (1H, m), 7.12 (1H, m), 7.06-7.03 (1H, m), 3.84 (3H, s), 3.02 (2H, q), 1.21 (3H, t).<br>The intermediate compound 34: $^1$H-NMR ($CDCl_3$) δ: 10.87 (1H, s), 8.82 (1H, m), 7.58 (1H, dd), 7.44 (1H, d), 7.35 (1H, dd), 6.86 (1H, td), 3.07 (2H, q), 1.25 (3H, t).<br>The intermediate compound 35: LCMS:533[M+H]$^+$, RT=2.410 min. | | | | | | |

Preparation example 3

To a mixture of 0.79 g the intermediate compound 26 and 40 mL N,N-dimethylformamide were added 1.7 g cesium carbonate and 0.22 mL iodomethane under ice-cooling, and the mixture was stirred at 0°C for 5 hours. Water was added to the resulting mixture and the precipitated out solids were separated by filtering, and dried. The resulting residue was subjected to a silica gel column chromatography to obtain 0.44 g the present compound 47.

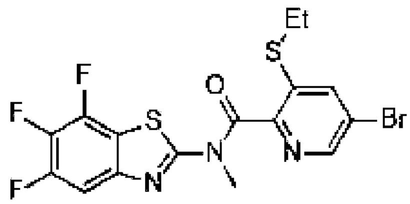

The present compound 47: LCMS:464[M+H]$^+$, RT=2.379 min.

Preparation example 3-1

The compounds prepared according to a similar method to that described in the Preparation example 3, and their physical properties are shown as follows.
A compound represented by formula (A-5):

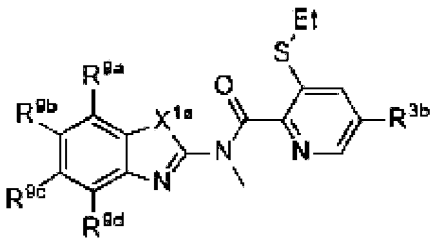

(A-5)

wherein a combination of $R^{9a}$, $R^{9b}$, $R^{9c}$, $R^{9d}$, $R^{3b}$, and $X^{1a}$ represents any of the and combinations indicated in [Table A-5].

[Table A-5]

| Present compound | $R^{9a}$ | $R^{9b}$ | $R^{9c}$ | $R^{9d}$ | $X^{1a}$ | $R^{3b}$ |
|---|---|---|---|---|---|---|
| 45 | H | F | H | H | S | Br |
| 46 | H | F | F | H | S | Br |
| 48 | H | H | $CF_3$ | H | O | H |
| 49 | H | H | $CF_3$ | H | S | H |

(continued)

| Present compound | $R^{9a}$ | $R^{9b}$ | $R^{9c}$ | $R^{9d}$ | $X^{1a}$ | $R^{3b}$ |
|---|---|---|---|---|---|---|
| 50 | H | H | $CF_3$ | H | N-Me | H |

The present compound 45: $^1$H-NMR ($CDCl_3$) δ: 8.54 (1H, d), 7.91 (1H, d), 7.82 (1H, dd), 7.53 (1H, dd), 7.19 (1H, td), 3.61 (3H, s), 2.99 (2H, q), 1.34 (3H, t).
The present compound 46: LCMS:446[M+H]$^+$, RT=2.263 min.
The present compound 48: $^1$H-NMR ($CDCl_3$) δ: 8.20 (1H, dd), 7.78 (1H, dd), 7.75-7.73 (1H, m), 7.46-7.44 (1H, m), 7.29 (1H, dd), 7.24 (1H, d), 3.74 (3H, s), 2.97 (2H, q), 1.32 (3H, t).
The present compound 49: $^1$H-NMR ($CDCl_3$) δ: 8.54 (1H, dd), 8.16 (1H, s), 7.96 (1H, d), 7.85 (1H, dd), 7.58 (1H, dd), 7.41 (1H, dd), 3.63 (3H, s), 2.98 (2H, q), 1.31 (3H, t).
The present compound 50: $^1$H-NMR ($CDCl_3$) δ: 7.82 (2H, d), 7.57-7.53 (2H, m), 7.38 (1H, s), 7.04 (1H, s), 3.76 (3H, s), 3.51 (3H, s), 2.98 (2H, q), 1.37 (3H, t).

Preparation example 4

To a mixture of 0.44 g the present compound 47 and 10 mL chloroform was added 0.53 g m-chloroperbenzoic acid under ice-cooling, and the mixture was stirred at room temperature for 2 hours, and to the resulting mixture were added sodium thiosulfate and saturated aqueous sodium hydrocarbonate solution, the mixture was extracted with chloroform. The resulting organic layer was dried over magnesium sulfate and concentrated under reduced pressure to obtain 0.42 g the present compound 53.

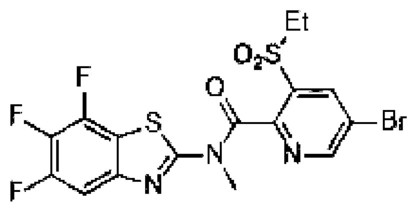

The present compound 53: $^1$H-NMR ($CDCl_3$) δ: 8.98 (1H, d), 8.53 (1H, d), 7.52-7.50 (1H, m), 3.61 (3H, s), 3.51 (2H, q), 1.38 (3H, t).

Preparation example 5

A mixture of 0.42 g the present compound 53, 0.23 g 4-chlorophenyl boronic acid, 0.08 g $PdCl_2$(dPPF), 0.46 g tripotassium phosphate, and 10 mL 1,2-dimethoxyethane was heated under reflux at 90°C for 6 hours, and water was added to the resulting mixture, and the mixture was extracted with chloroform. The resulting organic layer was dried over magnesium sulfate, and concentrated under reduced pressure. The resulting residue was subjected to a silica gel column chromatography to obtain 0.14 g the present compound 57.

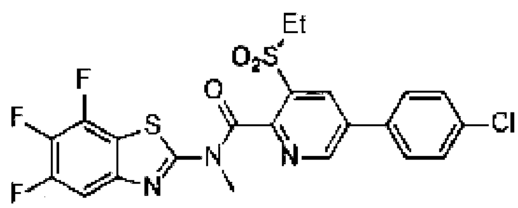

The present compound 57: $^1$H-NMR ($CDCl_3$) δ: 9.09 (1H, d), 8.52 (1H, d), 7.65-7.62 (2H, m), 7.57-7.55 (2H, m), 7.52-7.48 (1H, m), 3.65 (3H, s), 3.53 (2H, q), 1.40 (3H, t).

Preparation example 5-1

The compounds prepared according to a similar method to that described in the Preparation examples 4 and 5, and their physical properties are shown as follows.
A compound represented by formula (A-6):

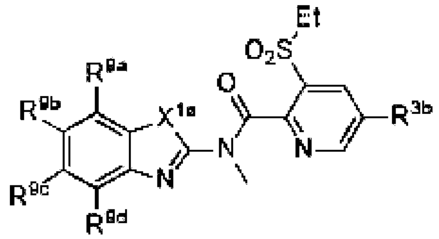

(A-6)

wherein a combination of $R^{9a}$, $R^{9b}$, $R^{9c}$, $R^{9d}$, $R^{3b}$ and $X^{1a}$ represents any of the combinations indicated in [Table A-6].

[Table A-6]

| Present compound | $R^{9a}$ | $R^{9b}$ | $R^{9c}$ | $R^{9d}$ | $X^{1a}$ | $R^{3b}$ |
|---|---|---|---|---|---|---|
| 51 | H | F | H | H | S | Br |
| 52 | H | F | F | H | S | Br |
| 54 | H | H | $CF_3$ | H | O | H |
| 55 | H | H | $CF_3$ | H | S | H |
| 56 | H | H | $CF_3$ | H | N-Me | H |
| 58 | H | F | F | H | S | 4-Cl-Ph |
| 59 | H | F | F | H | S | 4-F-Ph |
| 60 | H | F | F | H | S | 4-CN-Ph |
| 61 | H | F | F | H | S | 3, 5-$Cl_2$-Ph |
| 62 | H | F | H | H | S | 4-Cl-Ph |
| 63 | H | F | H | H | S | 4-F-Ph |

The present compound 51: $^1$H-NMR ($CDCl_3$) δ: 8.97 (1H, d), 8.53 (1H, d), 7.84 (1H, dd), 7.52 (1H, dd), 7.20 (1H, td), 3.61 (3H, s), 3.51 (2H, q), 1.37 (3H, t).
The present compound 52: $^1$H-NMR ($CDCl_3$) δ: 8.97 (1H, d), 8.53 (1H, d), 7.68 (1H, dd), 7.60 (1H, dd), 3.60 (3H, s), 3.50 (2H, q), 1.37 (3H, t).
The present compound 54: $^1$H-NMR ($CDCl_3$) δ: 8.69 (1H, d), 8.40 (1H, dd), 7.68 (1H, s), 7.61 (1H, dd), 7.44 (1H, d) , 7.22 (1H, d), 3.77 (3H, s), 3.48 (2H, q), 1.36 (3H, t).
The present compound 55: $^1$H-NMR ($CDCl_3$) δ: 8.94 (1H, d), 8.41 (1H, dd), 8.17 (1H, s), 7.95 (1H, d), 7.69 (1H, dd), 7.58 (1H, d), 3.64 (3H, s), 3.49 (2H, q), 1.36 (3H, t).
The present compound 56: $^1$H-NMR ($CDCl_3$) δ: 8.95-8.40 (1H, m), 8.37-8.27 (1H, m), 8.07-7.89 (1H, m), 7.68-7.28 (3H, m), 3.90-3.87, 3.42-4.40 (1H, m), 3.70-3.46 (2H, m), 3.61 (1H, s), 1.36-1.32 (3H, m).
The present compound 58: $^1$H-NMR ($CDCl_3$) δ: 9.09 (1H, d), 8.52 (1H, d), 7.67-7.58 (6H, m), 3.65 (3H, s), 3.53 (2H, q), 1.38 (3H, t).
The present compound 59: $^1$H-NMR ($CDCl_3$) δ: 9.08 (1H, s), 8.51 (1H, s), 7.68-7.61 (4H, m), 3.65 (3H, s), 3.53 (2H, q), 1.39 (3H, t).
The present compound 60: $^1$H-NMR ($CDCl_3$) δ: 9.13 (1H, d), 8.57 (1H, d), 7.85 (4H, dd), 7.69 (1H, dd), 7.62 (1H, t), 3.65 (3H, s), 3.55 (2H, q), 1.39 (3H, t).
The present compound 61: $^1$H-NMR ($CDCl_3$) δ: 9.08 (1H, d) , 8.51 (1H, d), 7.69 (1H, dd), 7.62 (1H, dd), 7.57-7.53 (3H, m), 3.64 (3H, s), 3.54 (2H, q), 1.39 (3H, t).
The present compound 62: $^1$H-NMR ($CDCl_3$) δ: 9.09 (1H, d), 8.52 (1H, d), 7.84 (1H, dd), 7.63 (2H, d), 7.55-7.52 (3H, m), 7.20 (1H, td), 3.66 (3H, s), 3.54 (2H, q), 1.38 (3H, t).
The present compound 63: $^1$H-NMR ($CDCl_3$) δ: 9.08 (1H, d), 8.51 (1H, d), 7.84 (1H, dd), 7.69-7.67 (2H, m), 7.53 (1H, dd), 7.30-7.28 (2H, m), 7.20 (1H, td), 3.66 (3H, s), 3.54 (2H, q), 1.38 (3H, t).

Preparation example 6

A mixture of 0.74 g 3-(ethylthio)-6-iodoimidazopyridin-2-carboxylic acid, 1.23 g 1-[bis(dimethylamino)methylene]-1H-1,2,3-triazolo[4,5-b]pyridinium-3-oxide hexafluorophosphate, 0.4 g the intermediate compound 37, 0.9 mL triethylamine, and 5 mL N,N-dimethylformamide was stirred at 50°C overnight. Water was added to the resulting mixture,

and the precipitated out solids were separated by filtering and the mixture was washed with methanol and dried to obtain 0.39 g the present compound 67.

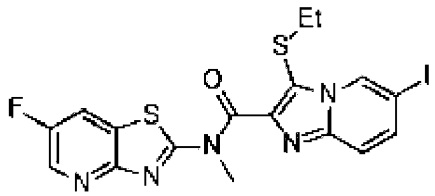

The present compound 67: $^{1}$H-NMR ($CDCl_3$) δ: 8.81 (1H, m), 8.54 (1H, dd), 7.91 (1H, dd), 7.59 (1H, dd), 7.51-7.48 (1H, m), 3.98 (3H, s), 2.95 (2H, q), 1.26-1.24 (3H, m).

Preparation example 7

To a mixture of 0.51 g the intermediate compound 2 and 30 mL N,N-dimethylformamide were added 1.1 g cesium carbonate and 0.2 mL chloromethyl methyl ether under ice-cooling, and the mixture was stirred at 0°C for 4 hours, and water was added to the resulting mixture, and the precipitated out solids were separated by filtering and dried. The resulting residue was subjected to a silica gel column chromatography to obtain 0.45 g the present compound 65.

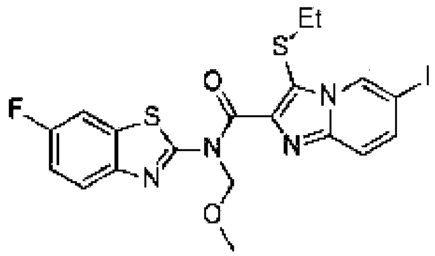

The present compound 65: $^{1}$H-NMR ($CDCl_3$) δ: 8.80-8.79 (1H, m), 7.83 (1H, dd), 7.56 (1H, dd), 7.52 (1H, dd), 7.46 (1H, dd), 7.18 (1H, td), 6.12 (2H, s), 3.32 (3H, s), 2.95 (2H, q), 1.25 (3H, t).

Preparation example 7-1

The compounds prepared according to a similar method to that described in the Preparation examples 6 and 7, and their physical properties are shown as follows.

A compound represented by formula (A-8):

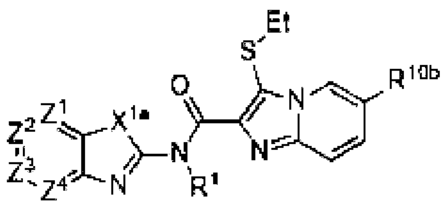

(A-8)

wherein a combination of $R^1$, $Z^1$, $Z^2$, $Z^3$, $Z^4$, $R^{10b}$, and $X^{1a}$ represents any of the and combinations indicated in [Table A-8].

[Table A-8]

| Present compound | $Z^1$ | $Z^2$ | $Z^3$ | $Z^4$ | $R^1$ | $X^{1a}$ | $R^{10b}$ |
|---|---|---|---|---|---|---|---|
| 64 | CH | CF | CH | CH | Et | S | I |
| 66 | N | CF | CH | CH | Me | S | I |
| 68 | CF | CF | CF | CF | Me | S | I |
| 69 | CH | CF | CH | CH | Me | N-Me | I |
| 70 | CH | CH | CF | CH | Me | N-Me | I |

(continued)

| Present compound | $Z^1$ | $Z^2$ | $Z^3$ | $Z^4$ | $R^1$ | $X^{1a}$ | $R^{10b}$ |
|---|---|---|---|---|---|---|---|
| 71 | CF | CH | CF | CH | Me | S | I |
| 72 | CH | CCl | CF | CH | Me | S | I |

The present compound 64: $^1$H-NMR ($CDCl_3$) δ: 8.80-8.78 (1H, m), 7.82 (1H, dd), 7.56 (1H, dd), 7.51 (1H, dd), 7.47 (1H, t), 7.18 (1H, td), 4.49 (2H, q), 2.93 (2H, q), 1.39 (3H, t), 1.24 (3H, t).
The present compound 66: $^1$H-NMR ($CDCl_3$) δ: 8.81-8.80 (1H, m), 8.17 (1H, dd), 7.58 (1H, dd), 7.48 (1H, dd), 7.03 (1H, dd), 3.89 (3H, s), 2.96 (2H, q), 1.24 (3H, t).
The present compound 68: $^1$H-NMR ($CDCl_3$) δ: 8.81 (1H, m), 7.59 (1H, dd), 7.49 (1H, d), 3.97 (3H, s), 2.96 (2H, q), 1.24 (3H, t).
The present compound 69: $^1$H-NMR ($CDCl_3$) δ: 8.67 (1H, s), 7.52-7.50 (2H, m), 6.99-6.88 (3H, m), 3.69 (3H, s), 3.52 (3H, s), 2.99-2.97 (2H, m), 1.23 (3H, t).
The present compound 70: $^1$H-NMR ($CDCl_3$) δ: 8.68 (1H, s), 7.28-7.23 (3H, m), 7.07-7.00 (1H, m), 6.86 (1H, s), 3.71 (3H, s), 3.54 (3H, s), 2.98 (2H, d), 1.23 (3H, t).
The present compound 71: $^1$H-NMR ($CDCl_3$) δ: 8.81-8.80 (1H, m), 7.58 (1H, dd), 7.48 (1H, dd), 7.41 (1H, dd), 6.86 (1H, td), 3.92 (3H, s), 2.96 (2H, q), 1.24 (3H, t).
The present compound 72: $^1$H-NMR ($CDCl_3$) δ: 8.80 (1H, m), 7.83 (1H, d), 7.65 (1H, d), 7.58 (1H, dd), 7.48 (1H, dd), 3.90 (3H, s), 2.94 (2H, q), 1.24 (3H, t).

Preparation example 8

To a mixture of 0.37 g the present compound 65 and 10 mL chloroform was added 0.43 g m-chloroperbenzoic acid under ice-cooling, and the mixture was stirred at room temperature for 5 hours, and to the resulting mixture were added sodium thiosulfate and saturated aqueous sodium hydrocarbonate solution, and the mixture was extracted with chloroform. The resulting organic layer was dried over magnesium sulfate, and concentrated under reduced pressure to obtain 0.3 g the present compound 74.

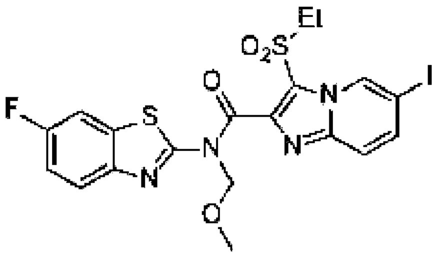

The present compound 74: $^1$H-NMR ($CDCl_3$) δ: 9.26 (1H, m), 7.84 (1H, dd), 7.74 (1H, dd), 7.58 (1H, d), 7.51 (1H, dd), 7.19 (1H, td), 5.92 (2H, s), 3.59 (2H, q), 3.40 (3H, s), 1.42 (3H, t).

Preparation example 8-1

The compounds prepared according to a similar method to that described in the Preparation example 8, and their physical properties are shown as follows.

A compound represented by formula (A-9):

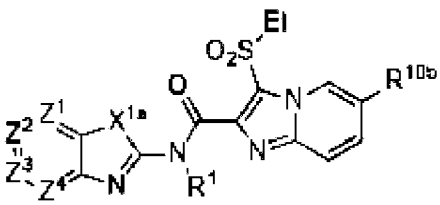

(A-9)

wherein a combination of $R^1$, $Z^1$, $Z^2$, $Z^3$, $Z^4$, $R^{10b}$, and $X^{1a}$ represents any of the and combinations indicated in [Table A-9] .

[Table A-9]

| Present compound | $Z^1$ | $Z^2$ | $Z^3$ | $Z^4$ | $R^1$ | $X^{1a}$ | $R^{10b}$ |
|---|---|---|---|---|---|---|---|
| 73 | CH | CF | CH | CH | Et | S | I |
| 75 | N | CF | CH | CH | Me | S | I |
| 76 | CH | CF | CH | N | Me | S | I |
| 77 | CF | CF | CF | CF | Me | S | I |
| 78 | CH | CF | CH | CH | Me | N-Me | I |
| 79 | CH | CH | CF | CH | Me | N-Me | I |
| 80 | CF | CH | CF | CH | Me | S | I |
| 81 | CH | CCl | CF | CH | Me | S | I |

The present compound 73: $^1$H-NMR ($CDCl_3$) δ: 9.26-9.24 (1H, m), 7.83 (1H, dd), 7.76 (1H, dd), 7.59 (1H, d), 7.51 (1H, dd), 7.19 (1H, td), 4.34 (2H, q), 3.55 (2H, q), 1.43-1.41 (6H, m).
The present compound 75: $^1$H-NMR ($CDCl_3$) δ: 9.25-9.24 (1H, m), 8.18 (1H, dd), 7.77 (1H, dd), 7.59 (1H, dd), 7.04 (1H, dd), 3.75 (3H, s), 3.56 (2H, q), 1.42 (3H, t).
The present compound 76: $^1$H-NMR ($CDCl_3$) δ: 9.24 (1H, dd), 8.55 (1H, dd), 7.90 (1H, dd), 7.78 (1H, dd), 7.60 (1H, dd), 3.83 (3H, s), 3.55 (2H, q), 1.42 (3H, t).
The present compound 77: $^1$H-NMR ($CDCl_3$) δ: 9.24-9.23 (1H, m), 7.78 (1H, dd), 7.60 (1H, dd), 3.81 (3H, s), 3.55 (2H, q), 1.43 (3H, t).
The present compound 78: $^1$H-NMR ($CDCl_3$) δ: 9.25-9.23 (1H, m), 7.77-6.90 (5H, m), 3.74 (2H, dd), 3.61-3.54 (6H, m), 1.38 (3H, t).
The present compound 79: $^1$H-NMR ($CDCl_3$) δ: 9.26-9.24 (1H, m), 7.67-7.42 (3H, m), 7.14-6.97 (2H, m), 3.79-3.72 (2H, m), 3.61-3.52 (6H, m), 1.39 (3H, t).
The present compound 80: $^1$H-NMR ($CDCl_3$) δ: 9.24 (1H, m), 7.77 (1H, dd), 7.61-7.59 (1H, m), 7.41 (1H, dd), 6.88 (1H, td), 3.78 (3H, s), 3.56 (2H, q), 1.42 (3H, t).
The present compound 81: $^1$H-NMR ($CDCl_3$) δ: 9.24 (1H, m), 7.83 (1H, d), 7.77 (1H, dd), 7.65 (1H, d), 7.59 (1H, dd), 3.77 (3H, s), 3.55 (2H, q), 1.42 (3H, t).

Reference preparation example 6

To a mixture of 7,3 g the intermediate compound 39 and 40 mL acetic acid was added 15.5 g phenyltrimethylammonium tribromide at room temperature, the mixture was stirred at 60°C for 5 hours. The resulting mixture was concentrated under reduced pressure, and saturated aqueous sodium bicarbonate solution was added thereto, and the precipitated out solids were separated by filtering. The obtained solids were washed with water, and then dried to obtain 5.5 g the intermediate compound 45.

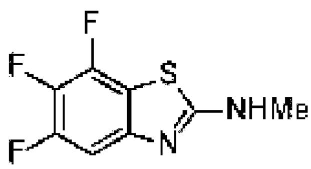

The intermediate compound 45: $^1$H-NMR ($CDCl_3$) δ: 7.15-7.12 (1H, m), 5.50 (1H, s), 3.12 (3H, s).

Reference preparation example 6-1

The compounds prepared according to a similar method to that described in the Reference preparation example 6, and their physical properties are shown as follows.
A compound represented by formula (A-12):

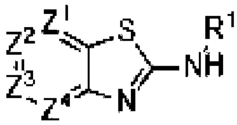

(A-12)

wherein a combination of $Z^1$, $Z^2$, $Z^3$, $Z^4$, and $R^1$ represents any of the and combinations indicated in [Table A-12].

[Table A-12]

| Intermediate compound | $Z^1$ | $Z^2$ | $Z^3$ | $Z^4$ | $R^1$ |
|---|---|---|---|---|---|
| 44 | CH | CF | CF | CH | $CH_3$ |
| 47 | CH | CF | CF | CH | c-Pr |
| The intermediate compound 44: $^1$H-NMR ($CDCl_3$) δ: 7.37-7.32 (2H, m), 5.37 (1H, s), 3.10 (3H, s).<br>The intermediate compound 47: $^1$H-NMR ($CDCl_3$) δ: 7.42 (1H, dd), 7.31 (1H, dd), 6.37 (1H, s), 2.74-2.69 (1H, m), 0.92-0.87 (2H, m), 0.76-0.74 (2H, m). | | | | | |

Reference preparation example 7

To a mixture of 2.5 g 6-chloro-3-(ethylsulfonyl)picolinic acid, 2.04 g 2-amino-5,6,7-trifluorobenzothiazole, and 30 mL ethyl acetate were added a solution of 5.1 mL diisopropylethylamine and 12.7 g propylphosphonic acid anhydride in 50 % ethyl acetate, and the mixture was stirred at 50°C. The resulting mixture was added to saturated aqueous sodium bicarbonate solution, and the mixture was extracted with ethyl acetate. The resulting organic layer was dried over magnesium sulfate, and concentrated under reduced pressure to obtain the intermediate compound 49. The intermediate compound 49 was used in the sequential reaction without further purification.

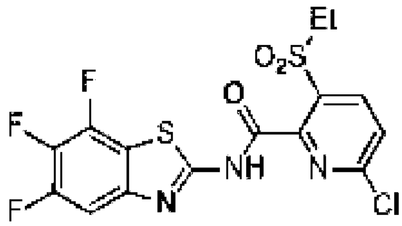

The intermediate compound 49: $^1$H-NMR ($CDCl_3$) δ: 8.59 (1H, m), 7.75 (1H, m), 7.44-7.44 (1H, m), 3.96-3.94 (2H, m), 1.39 (3H, t) .

Preparation example 1-2

The compounds prepared by using the intermediate compound 49 instead of the intermediate compound 15 according to a similar method to that described in the Preparation example 1, and its physical properties are shown as follows.

A compound represented by formula:

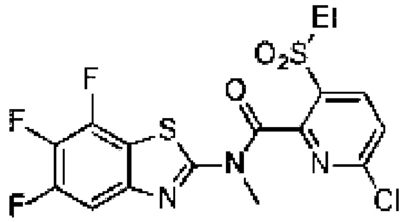

.

The present compound 82: $^1$H-NMR ($CDCl_3$) δ: 8.34 (1H, d), 7.69 (1H, d), 7.52-7.51 (1H, m), 3.63 (3H, s), 3.47 (2H, q), 1.36 (3H, t).

Preparation example 9

To a mixture of 5.1 g 3-(ethylsulfonyl)-5-fluoropicolinic acid, 60 mL chloroform, and catalytic amount of DMF was added dropwise 3.6 mL oxalyl chloride under ice-cooling. After the completion of the dropwise addition, the mixture was stirred at room temperature for 1 hour, and the resulting mixture was concentrated under reduced pressure. The resulting residue was added to a mixture of 4.2 g the intermediate compound 44 and 60 mL pyridine at room temperature, and the mixture was stirred at 90°C for 5 hours. The resulting mixture was added to water, and the precipitated out solids were separated by filtering, and washed with methanol to obtain 3.5 g the present compound 83.

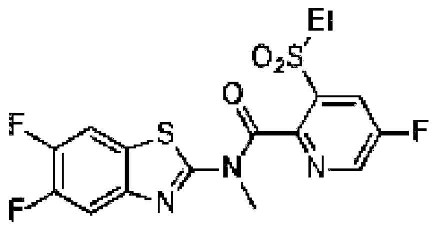

The present compound 83: $^1$H-NMR ($CDCl_3$) δ: 8.78 (1H, d), 8.13 (1H, dd), 7.68 (1H, dd), 7.61 (1H, dd), 3.60 (3H, s), 3.52 (2H, q), 1.37 (3H, t).

Preparation example 9-1

The compounds prepared by using 6-chloro-3-(ethylsulfonyl)picolinic acid instead of 3-(ethylsulfonyl)-5-fluropicolinic acid according to a similar method to that described in the Preparation example 9, and its physical properties are shown as follows.

A compound represented by formula:

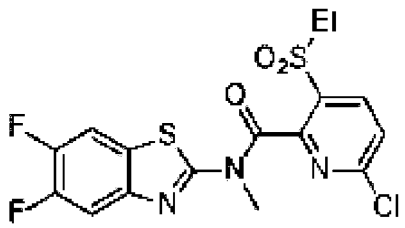

The present compound 84: $^1$H-NMR ($CDCl_3$) δ: 8.33 (1H, d), 7.70-7.66 (2H, m), 7.60 (1H, dd), 3.63 (3H, s), 3.47 (2H, q), 1.35 (3H, t).

Preparation example 5-2

The compounds prepared by using the present compound 84 instead of the present compound 53 according to a similar method to that described in the Preparation example 5, and its physical properties are shown as follows.

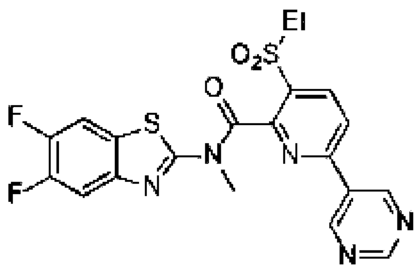

The present compound 87: $^1$H-NMR ($CDCl_3$) δ: 9.43 (2H, s), 9.37 (1H, s), 8.54 (1H, d), 8.11 (1H, d), 7.70 (1H, dd), 7.63 (1H, dd), 3.67 (3H, s), 3.52 (2H, q), 1.39 (3H, t).

Preparation example 10

A mixture of 0.49 g the present compound 53, 0.51 g bis(pinacolato)diboron, 0.07 g $PdCl_2$(dPPF), 0.29 g potassium acetate, and 5 mL toluene was stirred at 90°C for 3 hours. The resulting mixture was filtered through Celite, and water was added to the filtrate, and extracted with ethyl acetate. The resulting organic layer was dried over magnesium sulfate, and concentrated under reduced pressure. To the residue were added 0.29 g 2-bromo-6-chloropyridine, 0.07 g $PdCl_2$(dPPF), 0.53 g tripotassium phosphate, 1 mL water, and 5 mL 1,2-dimethoxyethane, and the mixture was stirred at 90°C for 3 hours. The resulting mixture was filtered through Celite, and water was added to the filtrate, and the mixture was extracted with ethyl acetate. The resulting organic layer was dried over magnesium sulfate, concentrated under reduced pressure, and subjected to a silica gel column chromatography to obtain 0.06 g the present compound 88.

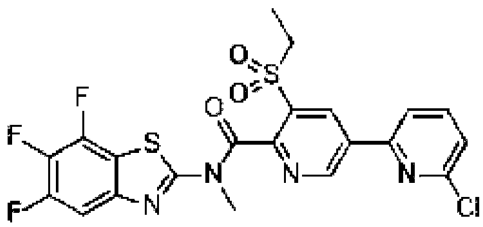

The present compound 88: $^1$H-NMR ($CDCl_3$) $\delta$: 9.52 (1H, d), 8.96 (1H, d), 7.88-7.84 (2H, m), 7.48-7.46 (2H, m), 3.63 (3H, s), 3.54 (2H, q), 1.41 (3H, t).

Preparation example 11

A mixture of 0.48 g the present compound 82, 0.46 g 2-(tributylstannyl)pyrimidine, 0.09 g $PdCl_2$(dPPF), 0.06 g copper(I) iodide, 0.05 g lithium chloride, and 3 mL toluene was stirred at 100°C for 6 hours. The resulting mixture was filtered through Celite, and water was added to the filtrate, and the mixture was extracted with ethyl acetate. The resulting organic layer was dried over magnesium sulfate, and concentrated under reduced pressure and subjected to a silica gel column chromatography to obtain 0.18 g the present compound 89.

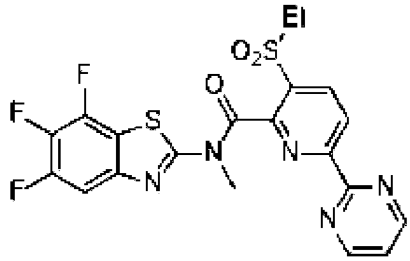

The present compound 89: $^1$H-NMR ($CDCl_3$) $\delta$: 9.02 (2H, d), 8.91 (1H, d), 8.57 (1H, d), 7.52-7.49 (1H, m), 7.46 (1H, t), 3.68 (3H, s), 3.53 (2H, q), 1.38 (3H, t).

Preparation example 12

To a mixture of 0.25 g 4-iodo-1H-pyrazole and 5 mL DMF was added 0.05 g sodium hydride under ice-cooling, and the mixture was stirred for 15 minutes. To the resulting mixture was added 0.42 g the present compound 83 under ice-cooling, and the mixture was stirred at room temperature for 4 hours. The resulting mixture was added to water, and the precipitated out solids were separated by filtering, and subjected to a silica gel column chromatography to obtain 0.3 g the present compound 90.

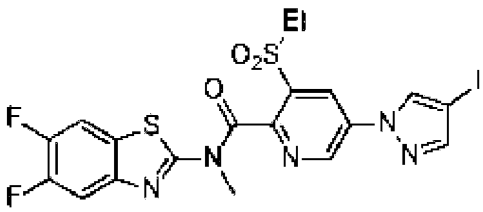

The present compound 90: $^1$H-NMR ($CDCl_3$) δ: 9.29 (1H, d), 8.63 (1H, d), 8.18 (1H, s), 7.86 (1H, s), 7.68 (1H, dd), 7.61 (1H, dd), 3.63 (3H, s), 3.55 (2H, q), 1.40 (3H, t).

Preparation example 13

To a mixture of 0.18 g 4-bromo-1H-pyrazole and 5 mL DMF was added 0.05 g sodium hydride under ice-cooling, and the mixture was stirred for 15 minutes. To the resulting mixture was added 0.44 g the present compound 84 under ice-cooling, and the mixture was stirred at room temperature for 4 hours. The resulting mixture was added to water, and the mixture was extracted with ethyl acetate. The resulting organic layer was dried over magnesium sulfate, concentrated under reduced pressure, and subjected to a silica gel column chromatography to obtain 0.13 g the present compound 91.

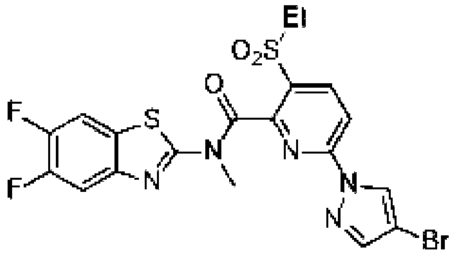

The present compound 91: $^1$H-NMR ($CDCl_3$) δ: 8.55 (1H, s), 8.47 (1H, d), 8.24 (1H, d), 7.75 (1H, s), 7.69 (1H, dd), 7.62 (1H, dd), 3.64 (3H, s), 3.47 (2H, q), 1.37 (3H, t).

Preparation example 14

To a mixture of 0.22 g the present compound 82 and 3 mL THF was added 0.5 mL 2 mol/L solution of dimethylamine in THF at room temperature, and the mixture was stirred. The resulting mixture was added to saturated aqueous ammonium chloride solution, and the mixture was extracted with ethyl acetate. The resulting organic layer was dried over magnesium sulfate, and concentrated under reduced pressure to obtain 0.23 g the present compound 92.

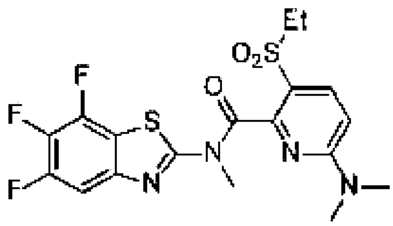

The present compound 92: $^1$H-NMR ($CDCl_3$) δ: 7.95 (1H, d), 7.49-7.47 (1H, m), 6.61 (1H, d), 3.62 (3H, s), 3.34 (2H, q), 3.20 (6H, s), 1.32 (3H, t).

Preparation example 15

A mixture of 0.43 g the present compound 84, 0.19 g nicotinamide, 0.03 g palladium (II) acetate, 0.1 g 2-dicyclohexylphosphino-2',4',6'-triisopropylbiphenyl, 0.97 g cesium carbonate, and 5 mL toluene was stirred at 100°C for 6 hours. The resulting mixture was filtered through Celite, and the filtrate was concentrated under reduced pressure and subjected to a silica gel column chromatography to obtain 0.22 g the present compound 93.

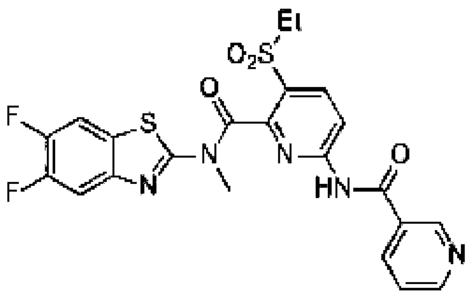

The present compound 93: $^1$H-NMR ($CDCl_3$) δ: 9.18 (1H, d), 8.86 (1H, dd), 8.80 (1H, s), 8.69 (1H, d), 8.43 (1H, d), 8.27-8.25 (1H, m), 7.68 (1H, dd), 7.61 (1H, dd), 7.51 (1H, dd), 3.62 (3H, s), 3.43 (2H, q), 1.36 (3H, t).

Reference preparation example 5-2

The compound prepared according to a similar method to that described in the Reference preparation example 5, and its physical properties are shown as follows.
A compound represented by formula:

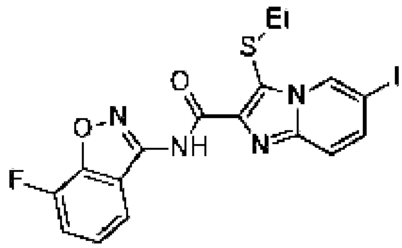

The intermediate compound 50: LCMS : 483[M+H]$^+$, RT=2.138 min.

Preparation example 16

The compound prepared by using the intermediate compound 50 instead of the intermediate compound 15 according to a similar method to that described in the Preparation example 1, and its physical properties are shown as follows.

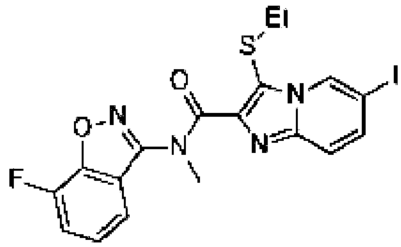

The present compound 85: $^1$H-NMR ($CDCl_3$) δ: 8.84 (1H, m), 7.99-7.97 (1H, m), 7.57 (2H, d), 7.29-7.26 (1H, m), 7.19-7.17 (1H, m), 4.10 (3H, d), 3.03 (2H, q), 1.25 (3H, t).

Preparation example 17

The compound prepared by using the present compound 85 instead of the present compound 15 according to a similar method to that described in the Preparation example 2, and its physical properties are shown as follows.

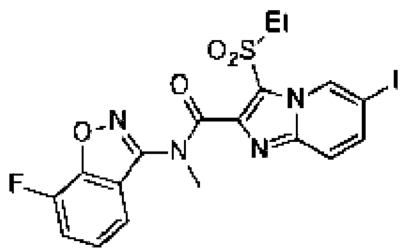

The present compound 86: $^1$H-NMR ($CDCl_3$) δ: 9.54 (1H, dd), 7.87-7.85 (1H, m), 7.77 (1H, dd), 7.68 (1H, dd), 7.31-7.29 (1H, m), 7.19-7.17 (1H, m), 4.07 (3H, d), 3.89 (2H, q), 1.45 (3H, t).

Reference preparation example 8

To a mixture of 1.4 g ethyl 2-bromo-5-ethylsulfonyl-1-methyl-inidazol-4-carboxylate (which was prepared

according to a similar method described in WO 2022/043576 A1) and 13 mL ethanol was added 13 mL aqueous 1 mol/ L sodium hydroxide solution under ice-cooling, and the mixture was stirred for 6 hours. The resulting mixture was concentrated under reduced pressure, and water was added thereto, and the mixture was stirred. To the resulting mixture was added 10 % hydrochloric acid, and the mixture was extracted with ethyl acetate. The resulting organic layer was dried over magnesium sulfate, and concentrated under reduced pressure to obtain 1.0 g the intermediate compound 51.

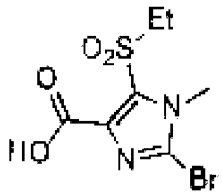

The intermediate compound 51: $^1$H-NMR ($CDCl_3$) $\delta$: 4.00 (3H, s), 3.69 (2H, q), 1.37 (3H, t).

Reference preparation example 9

To a mixture of 1.2 g ethyl 5-cyclopropyl-4-(ethylthio)-1-methyl-1H-pyrazol-3-carboxylate (which was prepared according to a similar method described in WO 2022/250069 A1) and 10 mL chloroform was added 1.7 g m-chloroperbenzoic acid under ice-cooling, and the mixture was stirred at room temperature for 3 hours. To the resulting mixture was added sodium thiosulfate and saturated aqueous sodium hydrocarbonate solution, and the mixture was extracted with chloroform. The resulting organic layer was dried over magnesium sulfate, and concentrated under reduced pressure to obtain 1.0 g the intermediate compound 52.

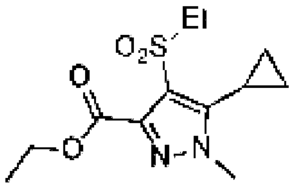

The intermediate compound 52: $^1$H-NMR ($CDCl_3$) $\delta$: 4.43 (2H, q), 4.00 (3H, s), 3.55 (2H, q), 1.89-1.87 (1H, m), 1.41-1.37 (6H, m), 1.28-1.23 (2H, m), 0.89-0.86 (2H, m).

Reference preparation example 10

The compound prepared by using the intermediate compound 52 instead of ethyl 2-bromo-5-ethylsulfonyl-1-methyl-imidazol-4-carboxylate according to a similar method to that described in the Reference preparation example 8, and its physical properties are shown as follows.

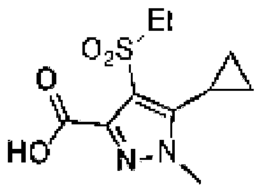

The intermediate compound 53: $^1$H-NMR ($CDCl_3$) $\delta$: 4.03 (3H, s), 3.52 (2H, q), 1.94-1.86 (1H, m), 1.37 (3H, t), 1.29-1.26 (2H, m), 0.91-0.90 (2H, m).

Preparation example 18

The compounds prepared according to a similar method to that described in any of the Preparation examples described in Examples, and their physical properties are shown as follows.

A compound represented by formula (A-13):

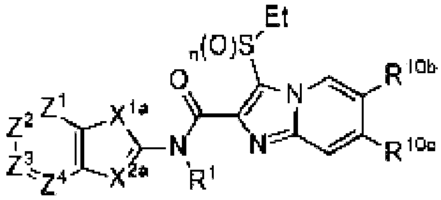

(A-13)

wherein a combination of $X^{1a}$, $X^{2a}$, $Z^1$, $Z^2$, $Z^3$, $Z^4$, n, $R^1$, $R^{10b}$, and $R^{10c}$ represents any of the combinations indicated in [Table A-13].

[Table A-13]

| Present compound | $X^{1a}$ | $X^{2a}$ | $Z^1$ | $Z^2$ | $Z^3$ | $Z^4$ | n | $R^1$ | $R^{10b}$ | $R^{10c}$ |
|---|---|---|---|---|---|---|---|---|---|---|
| 94 | S | CH | CH | CH | CF | CH | 2 | $CH_3$ | I | H |
| 95 | S | N | CH | CF | CF | CH | 0 | Et | I | H |
| 96 | S | N | CH | CF | CF | CH | 2 | Et | I | H |
| 97 | S | N | CH | CF | CF | CH | 0 | $CH_2CN$ | I | H |
| 98 | S | N | CH | CF | CF | CH | 2 | $CH_2CN$ | I | H |
| 99 | S | N | CH | CF | CF | CH | 2 | $CH_2C\equiv CH$ | I | H |
| 100 | S | N | CH | CF | CCl | CH | 0 | $CH_3$ | I | H |
| 101 | S | N | CH | CF | CCl | CH | 2 | $CH_3$ | I | H |
| 102 | S | N | CH | CF | CF | CH | 2 | $CH_3$ | c-Pr | H |
| 103 | S | N | N | CF | CH | CH | 2 | $CH_3$ | c-Pr | H |
| 104 | S | N | CH | CF | CF | CH | 2 | c-Pr | I | H |
| 105 | S | N | CH | CF | CF | CH | 2 | $CH_3$ | H | c-Pr |

The present compound 94: $^1$H-NMR ($CDCl_3$) δ: 9.17 (1H, d), 7.74-7.70 (1H, m), 7.58-7.54 (1H, m), 7.37-7.33 (1H, m), 7.25-7.23 (1H, m), 7.07-7.01 (2H, m), 3.59-3.50 (5H, m), 1.39 (3H, t).
The present compound 95: $^1$H-NMR ($CDCl_3$) δ: 8.79 (1H, m), 7.66 (1H, dd), 7.62-7.55 (2H, m), 7.47 (1H, dd), 4.49 (2H, q), 2.93 (2H, q), 1.39 (3H, t), 1.24 (3H, t).
The present compound 96: $^1$H-NMR ($CDCl_3$) δ: 9.25-9.24 (1H, m), 7.76 (1H, dd), 7.67 (1H, dd), 7.61-7.58 (2H, m), 4.33 (2H, q), 3.55 (2H, q), 1.43-1.41 (6H, m).
The present compound 97: $^1$H-NMR ($CDCl_3$) δ: 8.86 (1H, m), 7.72 (1H, dd), 7.63-7.61 (2H, m), 7.51 (1H, dd), 5.90 (2H, s), 3.03 (2H, q), 1.26 (3H, t).
The present compound 98: $^1$H-NMR ($CDCl_3$) δ: 9.33 (1H, s), 7.81 (1H, dd), 7.75 (1H, dd), 7.66-7.62 (2H, m), 5.28 (2H, s), 3.66 (2H, q), 1.44 (3H, t).
The present compound 99: $^1$H-NMR ($CDCl_3$) δ: 9.28 (1H, m), 7.77 (1H, dd), 7.73 (1H, dd), 7.63-7.58 (2H, m), 5.24 (2H, d), 3.58 (2H, q), 2.24 (1H, t), 1.42 (3H, t).
The present compound 100: $^1$H-NMR ($CDCl_3$) δ: 8.80 (1H, m), 7.91 (1H, d), 7.60-7.57 (2H, m), 7.48 (1H, dd), 3.89 (3H, s), 2.94 (2H, q), 1.24 (3H, t).
The present compound 101: $^1$H-NMR ($CDCl_3$) δ: 9.24 (1H, m), 7.92 (1H, d), 7.77 (1H, dd), 7.59 (2H, d), 3.76 (3H, s), 3.55 (2H, q), 1.42 (3H, t).
The present compound 102: $^1$H-NMR ($CDCl_3$) δ: 8.78-8.77 (1H, m), 7.70-7.65 (2H, m), 7.59 (1H, dd), 7.28-7.26 (1H, m), 3.76 (3H, s), 3.52 (2H, q), 2.06-1.99 (1H, m), 1.40 (3H, t), 1.14-1.09 (2H, m), 0.80-0.78 (2H, m).
The present compound 103: $^1$H-NMR ($CDCl_3$) δ: 8.78 (1H, m), 8.17 (1H, dd), 7.69 (1H, d), 7.28-7.26 (1H, m), 7.03 (1H, dd), 3.75 (3H, s), 3.53 (2H, q), 2.03-2.01 (1H, m), 1.41 (3H, t), 1.14-1.09 (2H, m), 0.80-0.78 (2H, m).
The present compound 104: $^1$H-NMR ($CDCl_3$) δ: 9.30 (1H, m), 7.75-7.72 (2H, m), 7.60-7.57 (2H, m), 3.64 (2H, q), 3.53-3.51 (1H, m), 1.42 (3H, t), 0.91-0.88 (4H, m).
The present compound 105: $^1$H-NMR ($CDCl_3$) δ: 8.83-8.81 (1H, m), 7.67 (1H, dd), 7.59 (1H, dd), 7.43 (1H, s), 6.85 (1H, dd), 3.77 (3H, s), 3.50 (2H, q), 2.06-2.02 (1H, m), 1.38 (3H, t), 1.21-1.18 (2H, m), 0.90-0.88 (2H, m).

A compound represented by formula (A-14):

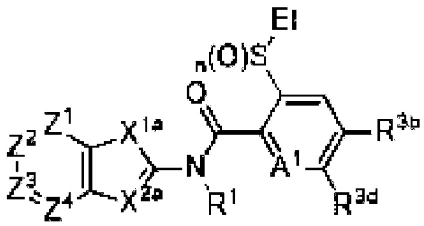

(A-14)

wherein a combination of $X^{1a}$, $X^{2a}$, $Z^1$, $Z^2$, $Z^3$, $Z^4$, $A^1$, n, $R^1$, $R^{3b}$, and $R^{3d}$ represents any of the combinations indicated in [Table A-14].

[Table A-14]

| Present compound | $X^{1a}$ | $X^{2a}$ | $Z^1$ | $Z^2$ | $Z^3$ | $Z^4$ | $A^1$ | n | $R^1$ | $R^{3b}$ | $R^{3d}$ |
|---|---|---|---|---|---|---|---|---|---|---|---|
| 106 | S | N | CH | CF | CF | CH | N | 0 | $CH_3$ | 4-F-Ph | H |
| 107 | S | N | CH | CF | CF | CH | N | 2 | Et | 4-F-Ph | H |
| 108 | S | N | CH | CF | CF | CH | N | 2 | $CH_2CN$ | 4-F-Ph | H |
| 109 | S | N | CH | CF | CF | CH | N | 2 | $CH_3$ | Py2 | H |
| 110 | S | N | CH | CF | CF | CH | N | 2 | $CH_3$ | 5-F-Py2 | H |
| 111 | S | N | CH | CF | CF | CH | N | 2 | $CH_3$ | 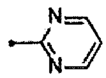 | H |
| 112 | S | N | CH | CF | CF | CH | N | 2 | $CH_3$ | 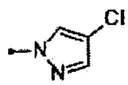 | H |
| 113 | S | N | CH | CF | CF | CH | N | 2 | $CH_3$ | 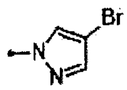 | H |
| 114 | S | N | CH | CF | CF | CH | N | 2 | $CH_3$ | H | H |
| 115 | S | N | CH | CF | CF | CH | N | 2 | $CH_3$ | c-Pr | H |
| 116 | S | N | CH | CF | CF | CH | N | 2 | $CH_3$ | 1-CN-c-Pr | H |
| 117 | S | N | CH | CF | CF | CH | N | 0 | $CH_3$ | $CF_3$ | H |
| 118 | S | N | CH | CF | CF | CH | N | 2 | $CH_3$ | $CF_3$ | H |
| 119 | S | N | CH | CF | CF | CH | CH | 0 | $CH_3$ | $CF_3$ | H |
| 120 | S | N | CH | CF | CF | CH | CH | 2 | $CH_3$ | $CF_3$ | H |
| 121 | S | N | N | CF | CH | CH | N | 2 | $CH_3$ | Br | H |
| 122 | S | N | CH | CF | CCl | CH | N | 2 | $CH_3$ | 1-CN-c-Pr | H |
| 123 | S | N | CH | CF | CF | CH | N | 0 | $CH_3$ | OBn | H |
| 124 | S | N | CH | CF | CF | CH | N | 2 | $CH_3$ | OBn | H |
| 125 | S | N | CH | CF | CF | CH | N | 2 | $CH_3$ | OEt | H |
| 126 | S | N | CH | CF | CF | CH | N | 2 | $CH_3$ | OPr | H |
| 127 | S | N | CH | CF | CF | CH | N | 0 | $CH_3$ | Ot-Bu | H |
| 128 | S | N | CH | CF | CF | CH | N | 2 | $CH_3$ | Ot-Bu | H |
| 129 | S | N | CH | CF | CF | CH | N | 2 | $CH_3$ | H | 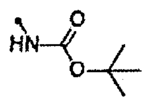 |

(continued)

| Present compound | $X^{1a}$ | $X^{2a}$ | $Z^1$ | $Z^2$ | $Z^3$ | $Z^4$ | $A^1$ | n | $R^1$ | $R^{3b}$ | $R^{3d}$ |
|---|---|---|---|---|---|---|---|---|---|---|---|
| 130 | S | N | CH | CF | CF | CH | N | 2 | $CH_3$ | H | 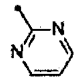 |
| 131 | S | N | CH | CF | CF | CH | N | 2 | $CH_3$ | H | 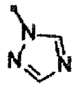 |
| 132 | S | N | CH | CF | CF | CH | N | 2 | $CH_3$ | H | 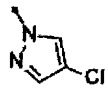 |
| 133 | S | N | CH | CF | CF | CH | N | 2 | $CH_3$ | H | 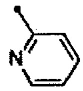 |
| 134 | S | N | CH | CF | CF | CH | N | 2 | $CH_3$ | H | $NHCH_3$ |
| 135 | S | N | CH | CF | CF | CH | N | 2 | $CH_3$ | H | $N(CH_3)_2$ |
| 136 | S | N | CH | CF | CF | CH | N | 2 | $CH_3$ | H | 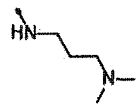 |
| 137 | S | N | CH | CF | CF | CH | N | 2 | $CH_3$ | H | 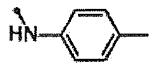 |
| 138 | S | N | CF | CF | CF | CH | N | 2 | $CH_3$ | H | $NHCH_3$ |
| 139 | S | N | CF | CF | CF | CH | N | 2 | $CH_3$ | H | NHc-Pr |

The present compound 106: $^1$H-NMR ($CDCl_3$) δ: 8.68 (1H, d), 7.93 (1H, d), 7.69-7.57 (4H, m), 7.23 (2H, t), 3.65 (3H, s), 3.03 (2H, q), 1.34 (3H, t).
The present compound 107: $^1$H-NMR ($CDCl_3$) δ: 9.08 (1H, d), 8.52 (1H, d), 7.69-7.68 (3H, m), 7.62-7.60 (1H, m), 7.29-7.28 (2H, m), 4.16-4.15 (2H, m), 3.53 (2H, q), 1.45 (3H, t), 1.38 (3H, t).
The present compound 108: $^1$H-NMR ($CDCl_3$) δ: 9.17 (1H, d), 8.54 (1H, d), 7.76-7.74 (1H, m), 7.72-7.68 (2H, m), 7.65-7.63 (1H, m), 7.31-7.28 (2H, m), 4.94 (2H, s), 3.57 (2H, q), 1.42 (3H, t).
The present compound 109: $^1$H-NMR ($CDCl_3$) δ: 9.53 (1H, d), 8.99 (1H, d), 8.81 (1H, d), 7.91 (2H, d), 7.68-7.61 (2H, m), 7.43 (1H, q), 3.64 (3H, s), 3.54 (2H, q), 1.40 (3H, t).
The present compound 110: $^1$H-NMR ($CDCl_3$) δ: 9.49 (1H, d), 8.95 (1H, d), 8.66 (1H, d), 7.93 (1H, dd), 7.68 (1H, dd), 7.63-7.60 (2H, m), 3.64 (3H, s), 3.54 (2H, q), 1.40 (3H, t).
The present compound 111: $^1$H-NMR ($CDCl_3$) δ: 9.93 (1H, d), 9.40 (1H, d), 8.92 (2H, d), 7.68 (1H, dd), 7.61 (1H, dd), 7.39 (1H, t), 3.65 (3H, s), 3.53 (2H, q), 1.41 (3H, t).
The present compound 112: $^1$H-NMR ($CDCl_3$) δ: 9.27 (1H, d), 8.62 (1H, d), 8.12 (1H, s), 7.80 (1H, s), 7.68 (1H, dd), 7.61 (1H, t), 3.63 (3H, s), 3.55 (2H, q), 1.40 (3H, t).
The present compound 113: $^1$H-NMR ($CDCl_3$) δ: 9.28 (1H, d), 8.63 (1H, d), 8.15 (1H, s), 7.83 (1H, s), 7.68 (1H, dd), 7.61 (1H, dd), 3.63 (3H, s), 3.55 (2H, q), 1.40 (3H, t).
The present compound 114: $^1$H-NMR ($CDCl_3$) δ: 8.93 (1H, dd), 8.41 (1H, dd), 7.70-7.65 (2H, m), 7.61 (1H, dd), 3.60 (3H, s), 3.48 (2H, q), 1.35 (3H, t).
The present compound 115: $^1$H-NMR ($CDCl_3$) δ: 8.66 (1H, d), 7.93 (1H, d), 7.66 (1H, dd), 7.59 (1H, dd), 3.58 (3H, s), 3.47 (2H, q), 2.11-2.06 (1H, m), 1.34 (3H, t), 1.27-1.24 (2H, m), 0.93-0.91 (2H, m).
The present compound 116: $^1$H-NMR ($CDCl_3$) δ: 8.94 (1H, d), 8.14 (1H, d), 7.68 (1H, dd), 7.61 (1H, dd), 3.59 (3H, s), 3.50 (2H, q), 2.05-2.01 (2H, m), 1.65-1.63 (2H, m), 1.36 (3H, t).
The present compound 117: $^1$H-NMR ($CDCl_3$) δ: 8.74-8.74 (1H, m), 7.98-7.98 (1H, m), 7.69-7.60 (2H, m), 3.60 (3H, s), 3.04 (2H, q), 1.36 (3H, t).
The present compound 118: $^1$H-NMR ($CDCl_3$) δ: 9.18 (1H, m), 8.64 (1H, d), 7.69 (1H, dd), 7.62 (1H, dd), 3.61 (3H, s), 3.53 (2H, q), 1.39 (3H, t).
The present compound 119: $^1$H-NMR ($CDCl_3$) δ: 7.69-7.59 (4H, m), 7.47 (1H, d), 3.58 (3H, s), 3.02 (2H, q), 1.32 (3H, t).
The present compound 120: $^1$H-NMR ($CDCl_3$) δ: 8.37 (1H, s), 8.07 (1H, d), 7.69-7.66 (2H, m), 7.61 (1H, dd), 3.59 (3H, s),

(continued)

| |
|---|
| 3.42-3.39 (2H, m), 1.35 (3H, t). |
| The present compound 121: $^1$H-NMR ($CDCl_3$) δ: 8.97 (1H, d), 8.53 (1H, d), 8.18 (1H, dd), 7.05 (1H, dd), 3.59 (3H, s), 3.51 (2H, q), 1.38 (3H, t). |
| The present compound 122: $^1$H-NMR ($CDCl_3$) δ: 8.94 (1H, d), 8.14 (1H, d), 7.92 (1H, d), 7.60 (1H, d), 3.59 (3H, s), 3.50 (2H, q), 2.04-2.02 (2H, m), 1.65-1.63 (2H, m), 1.36 (3H, t). |
| The present compound 123: $^1$H-NMR ($CDCl_3$) δ: 8.23 (1H, d), 7.65 (1H, dd), 7.60 (1H, dd), 7.43-7.39 (5H, m), 7.32 (1H, d), 5.20 (2H, s), 3.62 (3H, s), 2.92 (2H, q), 1.27 (3H, t). |
| The present compound 124: $^1$H-NMR ($CDCl_3$) δ: 8.61 (1H, d), 7.87 (1H, d), 7.66 (1H, dd), 7.59 (1H, dd), 7.44-7.40 (5H, m), 5.27 (2H, s), 3.59 (3H, s), 3.48 (2H, q), 1.28 (3H, t). |
| The present compound 125: $^1$H-NMR ($CDCl_3$) δ: 8.53 (1H, d), 7.79 (1H, d), 7.66 (1H, dd), 7.59 (1H, dd), 4.24 (2H, q), 3.59 (3H, s), 3.51 (2H, q), 1.53 (3H, t), 1.36 (3H, t). |
| The present compound 126: $^1$H-NMR ($CDCl_3$) δ: 8.54 (1H, d), 7.79 (1H, d), 7.66 (1H, dd), 7.59 (1H, dd), 4.12 (2H, t), 3.59 (3H, s), 3.51 (2H, q), 1.94-1.87 (2H, m), 1.38 (3H, t), 1.10 (3H, t). |
| The present compound 127: $^1$H-NMR ($CDCl_3$) δ: 8.20 (1H, d), 7.65 (1H, dd), 7.60 (1H, dd), 7.39 (1H, d), 3.62 (3H, s), 2.95 (2H, q), 1.45 (9H, s), 1.32 (3H, t). |
| The present compound 128: $^1$H-NMR ($CDCl_3$) δ: 8.55 (1H, d), 7.93 (1H, d), 7.67 (1H, dd), 7.61-7.58 (1H, m), 3.61 (3H, s), 3.50 (2H, q), 1.51 (9H, s), 1.35 (3H, t). |
| The present compound 129: $^1$H-NMR ($CDCl_3$) δ: 8.28-8.25 (2H, m), 7.67 (1H, dd), 7.60 (1H, dd), 7.52 (1H, s), 3.58 (3H, s), 3.39 (2H, q), 1.55 (9H, s), 1.32 (3H, t). |
| The present compound 130: $^1$H-NMR ($CDCl_3$) δ: 9.02 (2H, d), 8.90 (1H, d), 8.57 (1H, d), 7.67 (1H, dd), 7.60 (1H, dd), 7.45 (1H, t), 3.68 (3H, s), 3.53 (2H, q), 1.37 (3H, t). |
| The present compound 131: $^1$H-NMR ($CDCl_3$) δ: 9.20 (1H, s), 8.58 (1H, d), 8.24 (1H, d), 8.18 (1H, d), 7.71-7.61 (2H, m), 3.66 (3H, s), 3.49 (2H, q), 1.38 (3H, t). |
| The present compound 132: $^1$H-NMR ($CDCl_3$) δ: 8.55 (1H, s), 8.47 (1H, d), 8.24 (1H, d), 7.75 (1H, s), 7.68 (1H, dd), 7.62 (1H, dd), 3.64 (3H, s), 3.47 (2H, q), 1.37 (3H, t). |
| The present compound 133: $^1$H-NMR ($CDCl_3$) δ: 8.79 (1H, d), 8.75 (1H, d), 8.48 (2H, dd), 7.87-7.84 (1H, m), 7.67-7.64 (2H, m), 7.43 (1H, dd), 3.66 (3H, s), 3.52 (2H, q), 1.40-1.33 (3H, m). |
| The present compound 134: $^1$H-NMR ($CDCl_3$) δ: 7.96 (1H, d), 7.65 (1H, dd), 7.59 (1H, dd), 6.52 (1H, d), 5.28 (1H, s), 3.62 (3H, s), 3.34 (2H, q), 3.03 (3H, d), 1.31 (3H, t). |
| The present compound 135: $^1$H-NMR ($CDCl_3$) δ: 7.95 (1H, d), 7.65 (1H, dd), 7.59 (1H, dd), 6.60 (1H, d), 3.62 (3H, s), 3.35 (2H, q), 3.19 (6H, s), 1.31 (3H, t). |
| The present compound 136: $^1$H-NMR ($CDCl_3$) δ: 7.88-7.86 (1H, m), 7.65 (1H, dd), 7.59 (1H, dd), 6.45 (1H, d), 3.62 (3H, s), 3.47 (2H, s), 3.33 (2H, q), 2.45 (2H, t), 2.25 (6H, s), 1.77 (2H, t), 1.31 (3H, t). |
| The present compound 137: $^1$H-NMR ($CDCl_3$) δ: 7.98 (1H, d), 7.66 (1H, dd), 7.60 (1H, dd), 7.22 (4H, s), 7.05 (1H, s), 6.86 (1H, t), 3.66 (3H, s), 3.36 (2H, q), 2.37 (3H, s), 1.32 (3H, t). |
| The present compound 138: $^1$H-NMR ($CDCl_3$) δ: 7.97-7.95 (1H, m), 7.50-7.47 (1H, m), 6.53 (1H, d), 5.28 (1H, s), 3.62 (3H, s), 3.34 (2H, q), 3.03 (3H, d), 1.32 (3H, t). |
| The present compound 139: $^1$H-NMR ($CDCl_3$) δ: 8.05 (1H, d), 7.49-7.47 (1H, m), 6.90 (1H, d), 5.67 (1H, s), 3.61 (3H, s), 3.34 (2H, q), 2.64 (1H, m), 1.32 (3H, t), 0.95-0.93 (2H, m), 0.67-0.65 (2H, m). |

A compound represented by formula (A-17):

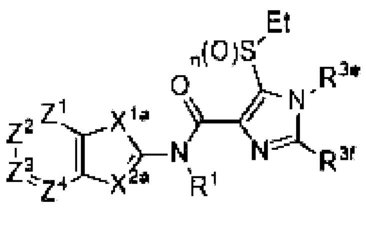

(A-17)

wherein a combination of $X^{1a}$, $X^{2a}$, $Z^1$, $Z^2$, $Z^3$, $Z^4$, n, $R^1$, $R^{3e}$, and $R^{3f}$ represents any of the combinations indicated in [Table A-17].

[Table A-17]

| Present compound | $X^{1a}$ | $X^{2a}$ | $Z^1$ | $Z^2$ | $Z^3$ | $Z^4$ | n | $R^1$ | $R^{3e}$ | $R^{3f}$ |
|---|---|---|---|---|---|---|---|---|---|---|
| 140 | S | N | CH | CF | CF | CH | 2 | $CH_3$ | $CH_3$ | Br |
| 141 | S | N | CH | CF | CF | CH | 2 | $CH_3$ | $CH_3$ | 4-F-Ph |
| 142 | S | N | CH | CF | CF | CH | 2 | $CH_3$ | $CH_3$ | 4-Cl-Ph |
| 143 | S | N | CH | CF | CF | CH | 2 | $CH_3$ | $CH_3$ | 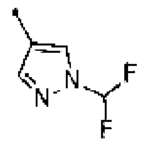 |

The present compound 140: $^1$H-NMR ($CDCl_3$) δ: 7.66 (1H, dd), 7.58 (1H, dd), 3.96 (3H, s), 3.76 (3H, s), 3.49 (2H, q), 1.43 (3H, t).
The present compound 141: $^1$H-NMR ($CDCl_3$) δ: 7.67-7.65 (3H, m), 7.58 (1H, dd), 7.27-7.25 (2H, m), 3.98 (3H, s), 3.80 (3H, s), 3.52 (2H, q), 1.47 (3H, t).
The present compound 142: $^1$H-NMR ($CDCl_3$) δ: 7.66 (1H, dd), 7.60-7.55 (5H, m), 3.98 (3H, s), 3.79 (3H, s), 3.52 (2H, q), 1.47 (3H, t).
The present compound 143: $^1$H-NMR ($CDCl_3$) δ: 8.31 (1H, s), 8.09 (1H, s), 7.66 (1H, dd), 7.59 (1H, dd), 4.08 (3H, s), 3.78 (3H, s), 3.50 (2H, q), 1.45 (3H, t), 1.24 (1H, s).

A compound represented by formula (A-18):

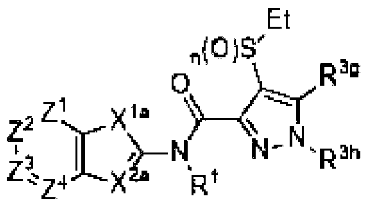

(A-18)

wherein a combination of $X^{1a}$, $X^{2a}$, $Z^1$, $Z^2$, $Z^3$, $Z^4$, n, $R^1$, $R^{3g}$, and $R^{3h}$ represents any of the combinations indicated in [Table A-18].

[Table A-18]

| Present compound | $X^{1a}$ | $X^{2a}$ | $Z^1$ | $Z^2$ | $Z^3$ | $Z^4$ | n | $R^1$ | $R^{3g}$ | $R^{3h}$ |
|---|---|---|---|---|---|---|---|---|---|---|
| 144 | S | N | CH | CF | CF | CH | 2 | $CH_3$ | c-Pr | $CH_3$ |
| 145 | S | N | CH | CF | CCl | CH | 2 | $CH_3$ | c-Pr | $CH_3$ |

The present compound 144: $^1$H-NMR ($CDCl_3$) δ: 7.65 (1H, dd), 7.57 (1H, dd), 4.00 (3H, s), 3.71 (3H, s), 3.35 (2H, q), 1.93-1.88 (1H, m), 1.39 (3H, t), 1.28-1.24 (2H, m), 1.06-1.04 (2H, m).
The present compound 145: $^1$H-NMR ($CDCl_3$) δ: 7.90 (1H, d), 7.57 (1H, d), 4.01 (3H, s), 3.71 (3H, s), 3.35 (2H, q), 1.94-1.87 (1H, m), 1.40 (3H, t), 1.28-1.24 (2H, m), 1.06-1.04 (2H, m) .

Next, examples of the present compounds that are prepared according to Preparation Examples that are described in Examples or Process that are described herein are indicated below.

Here Q1-1 to Q1-211, Q2-1 to Q2-36, Q5-1 to Q5-26, and Q6-1 to Q6-26 represent the following groups respectively (wherein # represents a binding site to a sulfur atom, and

- represents a binding site to a carbonyl group).

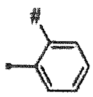
Q1-1

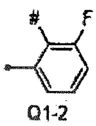
Q1-2

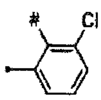
Q1-3

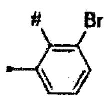
Q1-4

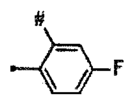
Q1-5

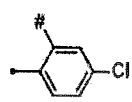
Q1-6

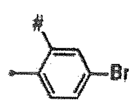
Q1-7

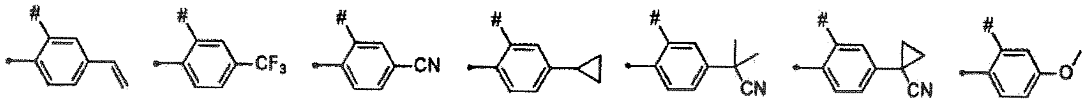

Q1-8 Q1-9 Q1-10 Q1-11 Q1-12 Q1-13 Q1-14

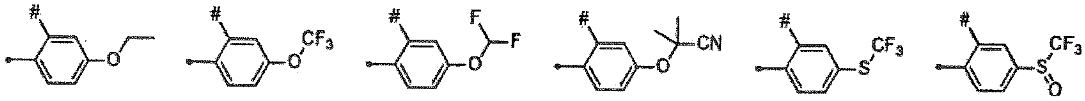

Q1-15 Q1-16 Q1-17 Q1-18 Q1-19 Q1-20

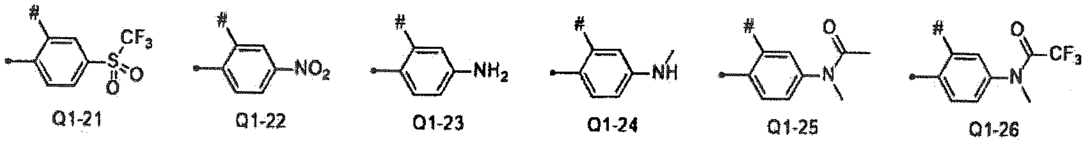

Q1-21 Q1-22 Q1-23 Q1-24 Q1-25 Q1-26

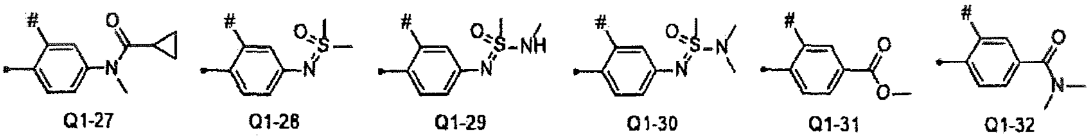

Q1-27 Q1-28 Q1-29 Q1-30 Q1-31 Q1-32

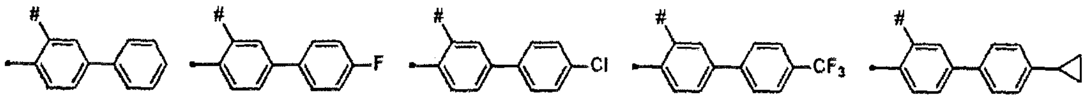

Q1-33 Q1-34 Q1-35 Q1-36 Q1-37

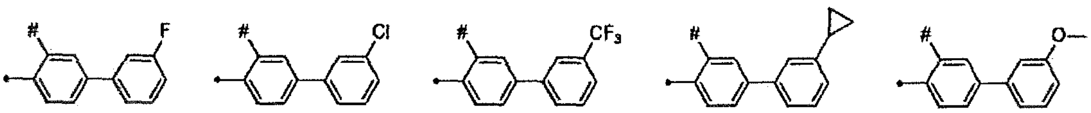

Q1-38 Q1-39 Q1-40 Q1-41 Q1-42

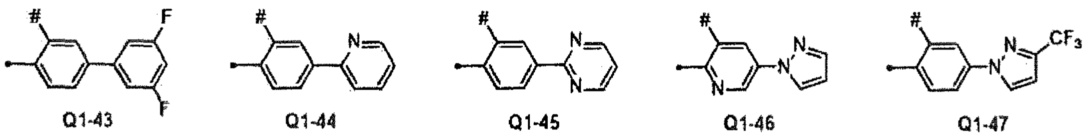

Q1-43 Q1-44 Q1-45 Q1-46 Q1-47

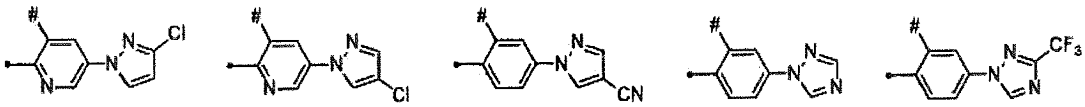

Q1-48 Q1-49 Q1-50 Q1-51 Q1-52

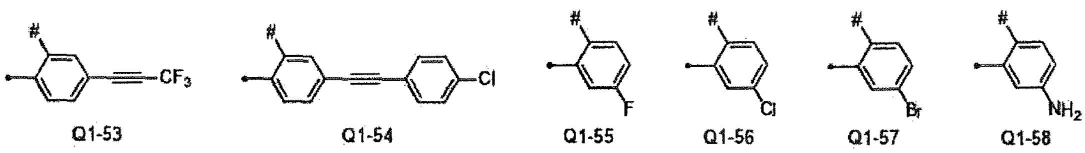

Q1-53 Q1-54 Q1-55 Q1-56 Q1-57 Q1-58

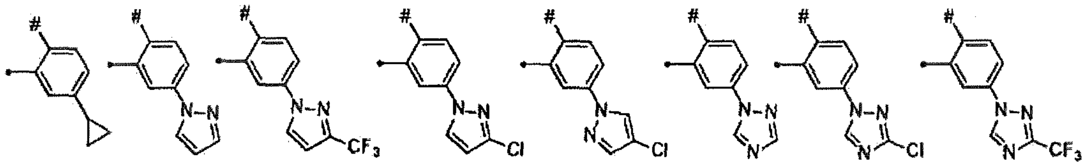

Q1-59 Q1-60 Q1-61 Q1-62 Q1-63 Q1-64 Q1-65 Q1-66

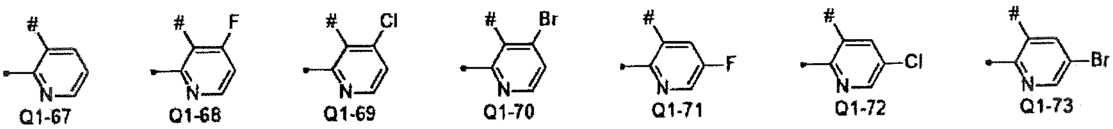

Q1-67 Q1-68 Q1-69 Q1-70 Q1-71 Q1-72 Q1-73

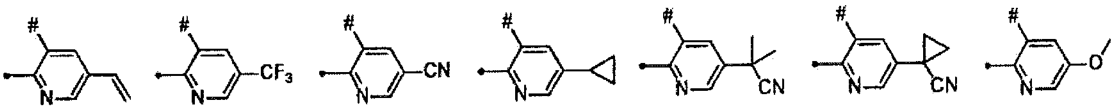

Q1-74 Q1-75 Q1-76 Q1-77 Q1-78 Q1-79 Q1-80

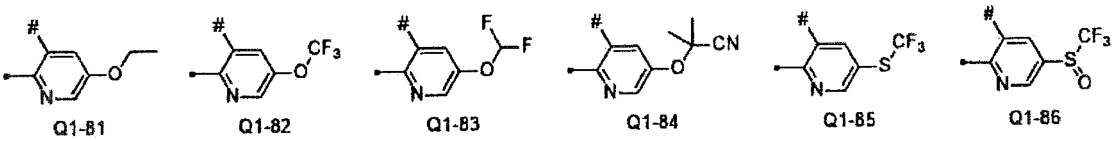

Q1-81 Q1-82 Q1-83 Q1-84 Q1-85 Q1-86

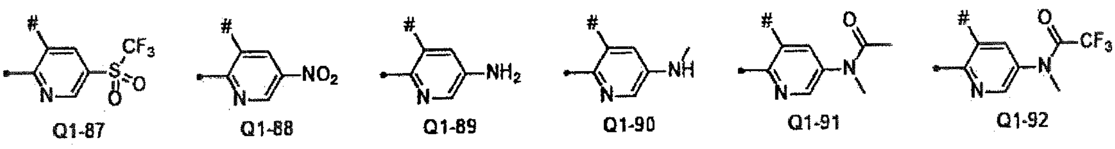

Q1-87 Q1-88 Q1-89 Q1-90 Q1-91 Q1-92

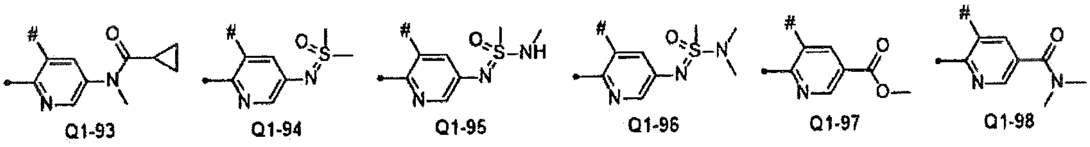

Q1-93 Q1-94 Q1-95 Q1-96 Q1-97 Q1-98

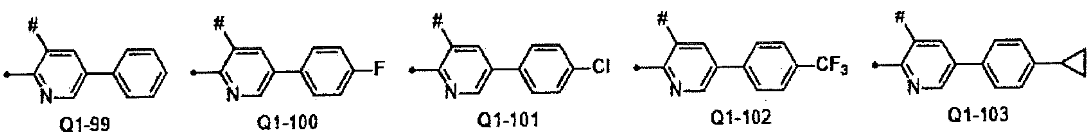

Q1-99 Q1-100 Q1-101 Q1-102 Q1-103

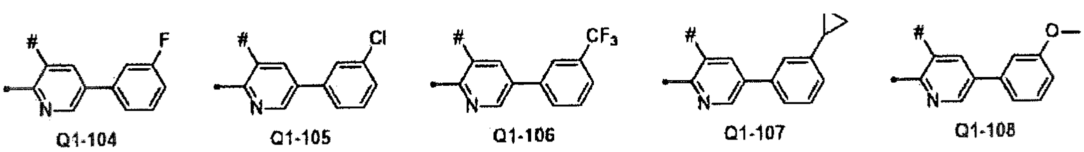

Q1-104 Q1-105 Q1-106 Q1-107 Q1-108

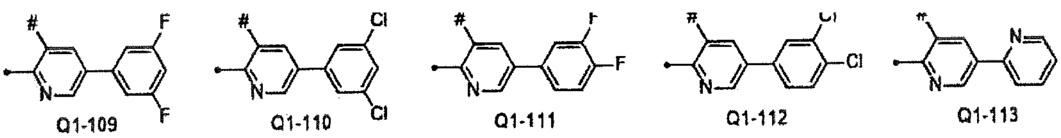

Q1-109 Q1-110 Q1-111 Q1-112 Q1-113

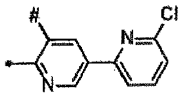
Q1-114
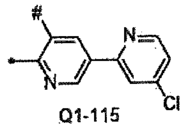
Q1-115
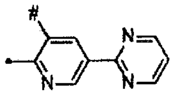
Q1-116
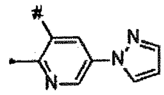
Q1-117
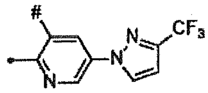
Q1-118

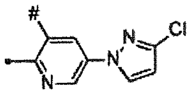
Q1-119
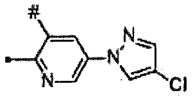
Q1-120
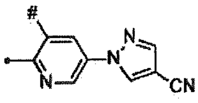
Q1-121
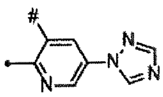
Q1-122
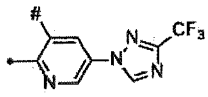
Q1-123

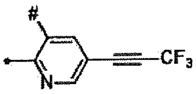
Q1-124
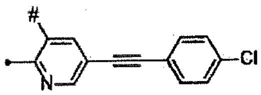
Q1-125
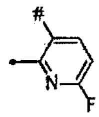
Q1-126
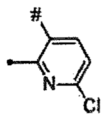
Q1-127
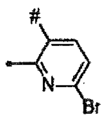
Q1-128
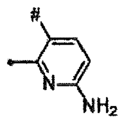
Q1-129

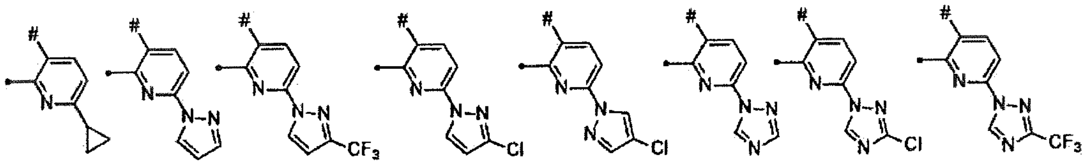
Q1-130 Q1-131 Q1-132 Q1-133 Q1-134 Q1-135 Q1-136 Q1-137

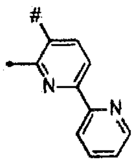
Q1-210
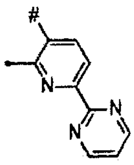
Q1-211

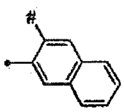
Q1-138
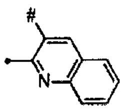
Q1-139
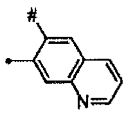
Q1-140
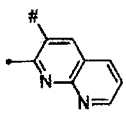
Q1-141
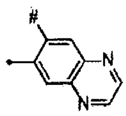
Q1-142
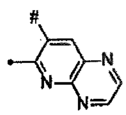
Q1-143

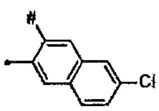
Q1-144
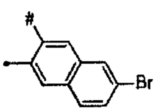
Q1-145
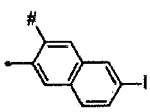
Q1-146
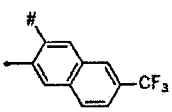
Q1-147
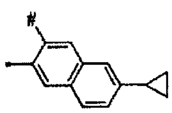
Q1-148
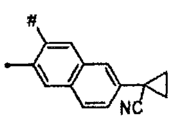
Q1-149

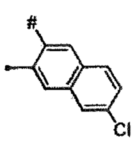
Q1-150
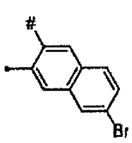
Q1-151
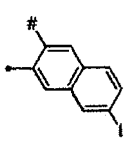
Q1-152
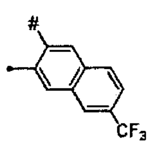
Q1-153
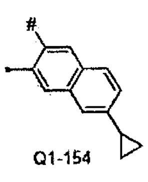
Q1-154
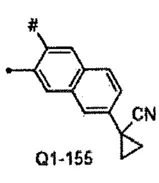
Q1-155

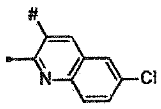
Q1-156
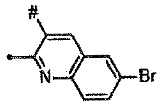
Q1-157
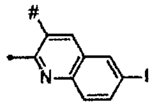
Q1-158
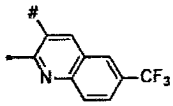
Q1-159
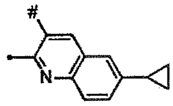
Q1-160
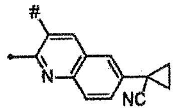
Q1-161

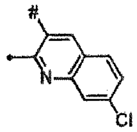
Q1-162
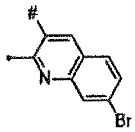
Q1-163
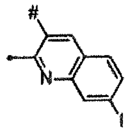
Q1-164
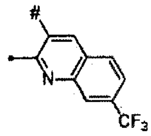
Q1-165
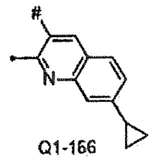
Q1-166
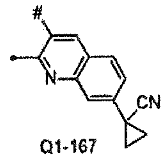
Q1-167

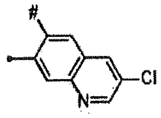
Q1-168
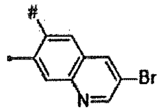
Q1-169
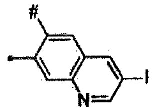
Q1-170
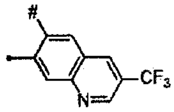
Q1-171
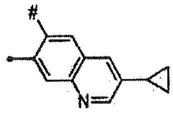
Q1-172
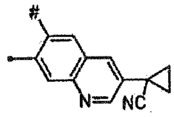
Q1-173

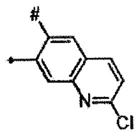
Q1-174
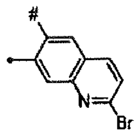
Q1-175
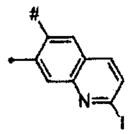
Q1-176
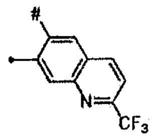
Q1-177
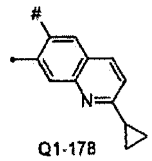
Q1-178
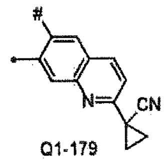
Q1-179

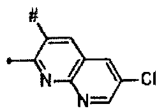
Q1-180
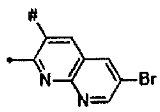
Q1-181
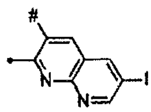
Q1-182
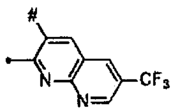
Q1-183
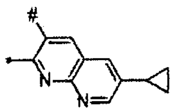
Q1-140
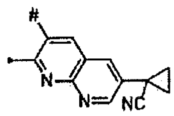
Q1-140

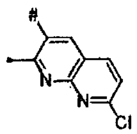
Q1-138
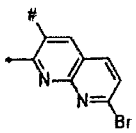
Q1-138
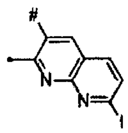
Q1-138
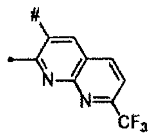
Q1-138
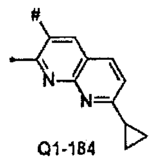
Q1-184
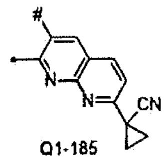
Q1-185

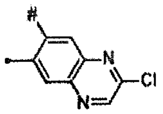
Q1-186
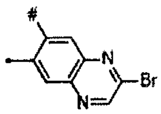
Q1-187
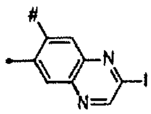
Q1-188
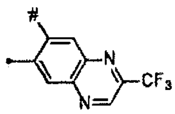
Q1-189
Q1-190
Q1-191

Q1-192
Q1-193
Q1-194
Q1-195
Q1-196
Q1-197

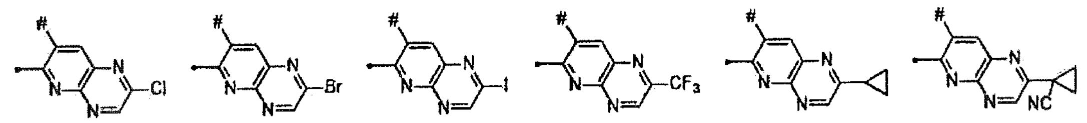
Q1-198 Q1-199 Q1-200 Q1-201 Q1-202 Q1-203

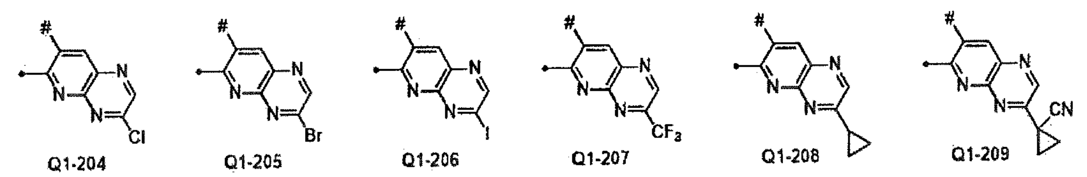
Q1-204 Q1-205 Q1-206 Q1-207 Q1-208 Q1-209

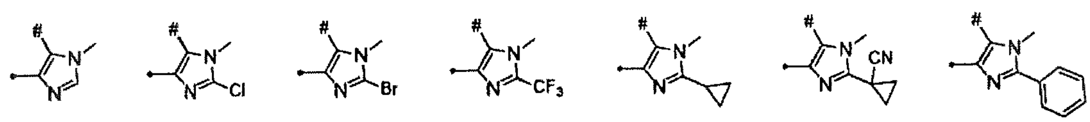
Q2-1 Q2-2 Q2-3 Q2-4 Q2-5 Q2-6 Q2-7

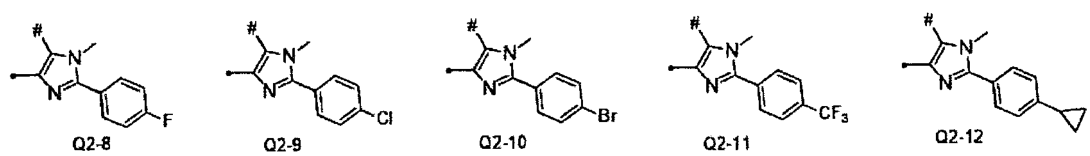
Q2-8 Q2-9 Q2-10 Q2-11 Q2-12

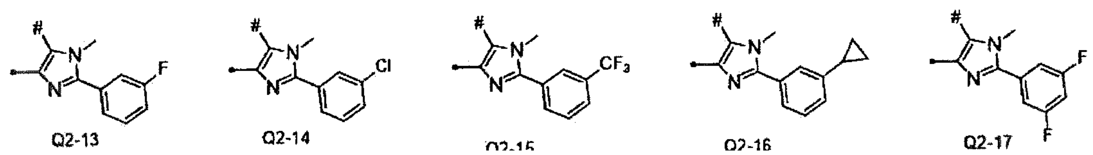
Q2-13 Q2-14 Q2-15 Q2-16 Q2-17

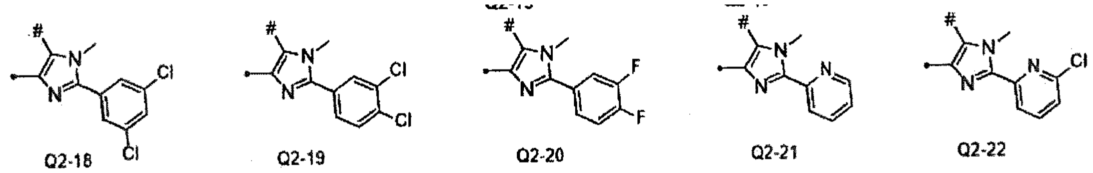
Q2-18 Q2-19 Q2-20 Q2-21 Q2-22

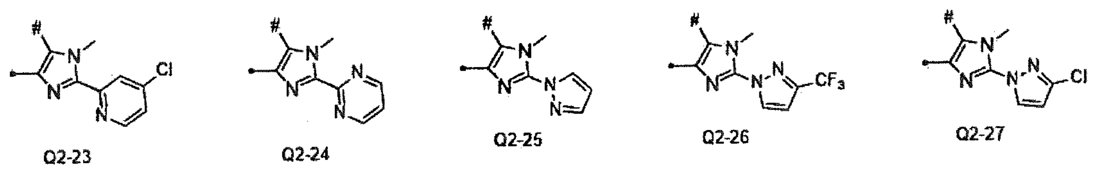
Q2-23 Q2-24 Q2-25 Q2-26 Q2-27

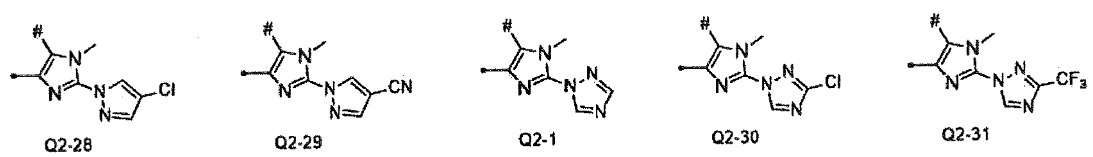
Q2-28 Q2-29 Q2-1 Q2-30 Q2-31

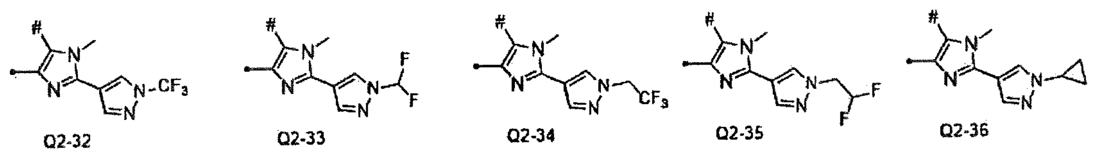
Q2-32 Q2-33 Q2-34 Q2-35 Q2-36

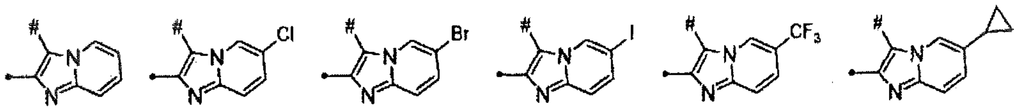

Q5-1 Q5-2 Q5-3 Q5-4 Q5-5 Q5-6

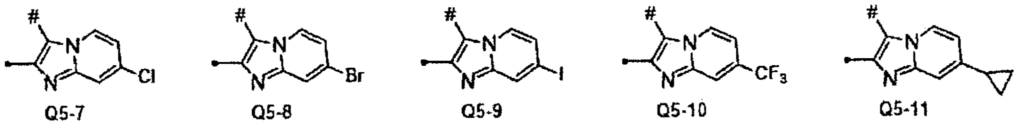

Q5-7 Q5-8 Q5-9 Q5-10 Q5-11

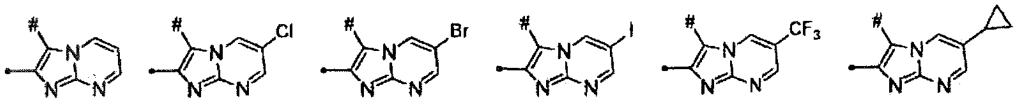

Q5-12 Q5-13 Q5-14 Q5-15 Q5-16 Q5-17

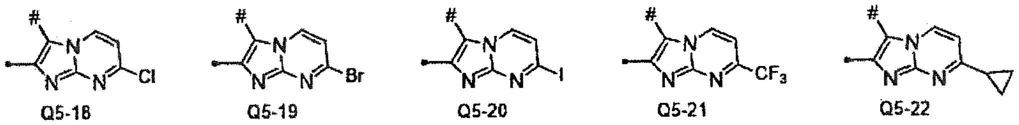

Q5-18 Q5-19 Q5-20 Q5-21 Q5-22

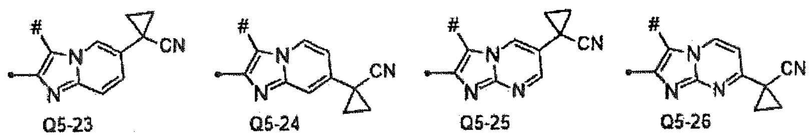

Q5-23 Q5-24 Q5-25 Q5-26

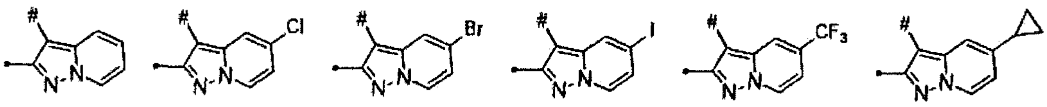

Q6-1 Q6-2 Q6-3 Q6-4 Q6-5 Q6-6

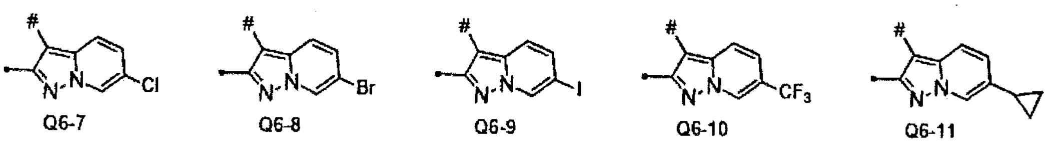

Q6-7 Q6-8 Q6-9 Q6-10 Q6-11

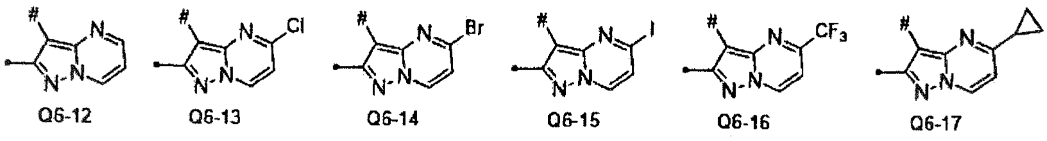

Q6-12 Q6-13 Q6-14 Q6-15 Q6-16 Q6-17

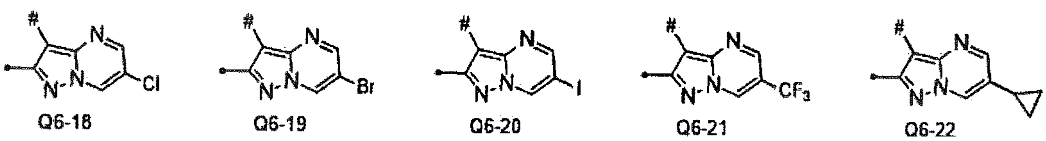

Q6-18 Q6-19 Q6-20 Q6-21 Q6-22

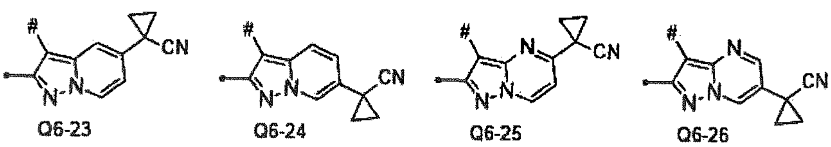

Q6-23 Q6-24 Q6-25 Q6-26

A compound represented by formula (L-1):

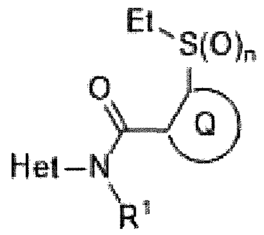

(L-1)

(hereinafter, referred to "Compound (L-1)")
wherein n is 0, $R^1$ represents a methyl group, Q represents a group represented by Q1-1, and Het represents any substituents described in any of [Table 1A] to [Table 20A].) (hereinafter, referred to as "Compound Class SX1")

[Table 1A]

| |
|---|
| 5-F-1H-indol-2-yl |
| 6-F-1H-indol-2-yl |
| 5-(CN)-1H-indol-2-yl |
| 6-(CN)-1H-indol-2-yl |
| 5-F-1-Me-1H-indol-2-yl |
| 5-F-3-Me-1H-indol-2-yl |
| 6-F-1-Me-1H-indol-2-yl |
| 6-F-3-Me-1H-indol-2-yl |
| 5-Cl-1H-indol-2-yl |
| 6-Cl-1H-indol-2-yl |
| 5-(CN)-1-Me-1H-indol-2-yl |
| 6-(CN)-1-Me-1H-indol-2-yl |
| 3-(CN)-5-F-1H-indol-2-yl |
| 3-(CN)-6-F-1H-indol-2-yl |
| 5-F-1,3-$Me_2$-1H-indol-2-yl |
| 6-F-1,3-Me-1H-indol-2-yl |
| 5-Cl-1-Me-1H-indol-2-yl |
| 6-Cl-1-Me-1H-indol-2-yl |
| 3-(CN)-5-F-1-Me-1H-indol-2-yl |
| 3-(CN)-6-F-1-Me-1H-indol-2-yl |
| 5-Cl-3-(CN)-1H-indol-2-yl |
| 6-Cl-3-(CN)-1H-indol-2-yl |
| 3-(CN)-5,6-$F_2$-1H-indol-2-yl |
| 3-(CN)-5,7-$F_2$-1H-indol-2-yl |
| 5-Cl-3-(CN)-1-Me-1H-indol-2-yl |
| 5-Cl-3-(CN)-6-Me-1H-indol-2-yl |
| 6-Cl-3-(CN)-1-Me-1H-indol-2-yl |

[Table 2A]

| |
|---|
| 6-Br-3-(CN)-1-Me-1H-indol-2-yl |
| 5-F-benzothiophen-2-yl |
| 6-F-benzothiophen-2-yl |
| 5-(CN)-benzothiophen-2-yl |
| 6-(CN)-benzothiophen-2-yl |
| 5-F-3-Me-benzothiophen-2-yl |
| 5-F-6-Me-benzothiophen-2-yl |
| 6-F-3-Me-benzothiophen-2-yl |
| 6-F-5-Me-benzothiophen-2-yl |
| 5-Cl-benzothiophen-2-yl |
| 6-Cl-benzothiophen-2-yl |
| 3,5-$F_2$-benzothiophen-2-yl |
| 3,6-$F_2$-benzothiophen-2-yl |
| 5,6-$F_2$-benzothiophen-2-yl |
| 5,7-$F_2$-benzothiophen-2-yl |
| 3-(CN)-5-F-benzothiophen-2-yl |
| 3-(CN)-6-F-benzothiophen-2-yl |
| 3-Cl-5-F-benzothiophen-2-yl |
| 3-Cl-6-F-benzothiophen-2-yl |
| 5-Cl-6-F-benzothiophen-2-yl |
| 5-Cl-7-F-benzothiophen-2-yl |
| 3-(CN)-5-F-6-Me-benzothiophen-2-yl |
| 3-(CN)-6-F-5-Me-benzothiophen-2-yl |
| 5-Cl-3-(CN)-benzothiophen-2-yl |
| 6-Cl-3-(CN)-benzothiophen-2-yl |
| 3-(CN)-5,6-$F_2$-benzothiophen-2-yl |
| 3-(CN)-5,7-$F_2$-benzothiophen-2-yl |

| |
|---|
| 3-(CN)-5,6-$F_2$-1-Me-1H-indol-2-yl |
| 3-(CN)-5,7-$F_2$-1-Me-1H-indol-2-yl |
| 5-Br-1H-indol-2-yl |
| 6-Br-1H-indol-2-yl |
| 5-Br-1-Me-1H-indol-2-yl |
| 6-Br-1-Me-1H-indol-2-yl |
| 3-(CN)-5-$CF_3$-1H-indol-2-yl |
| 3-(CN)-6-$CF_3$-1H-indol-2-yl |
| 5-Br-3-(CN)-1H-indol-2-yl |
| 6-Br-3-(CN)-1H-indol-2-yl |
| 3-(CN)-5-$CF_3$-1-Me-1H-indol-2-yl |
| 3-(CN)-6-$CF_3$-1-Me-1H-indol-2-yl |
| 5-Br-3-(CN)-1-Me-1H-indol-2-yl |

| |
|---|
| 5-$CF_3$-benzothiophen-2-yl |
| 6-$CF_3$-benzothiophen-2-yl |
| 3,5-$Cl_2$-benzothiophen-2-yl |
| 3,6-$Cl_2$-benzothiophen-2-yl |
| 5-Cl-3-(CN)-6-Me-benzothiophen-2-yl |
| 5-Cl-3-(CN)-6-F-benzothiophen-2-yl |
| 5-Cl-3-(CN)-7-F-benzothiophen-2-yl |
| 5-Br-benzothiophen-2-yl |
| 6-Br-benzothiophen-2-yl |
| 7-F-5-$CF_3$-benzothiophen-2-yl |
| 5-Br-6-Me-benzothiophen-2-yl |
| 3-(CN)-5-$CF_3$-benzothiophen-2-yl |
| 3-(CN)-6-$CF_3$-benzothiophen-2-yl |

[Table 3A]

| |
|---|
| 3-Br-5-F-benzothiophen-2-yl |
| 3-Br-6-F-benzothiophen-2-yl |
| 5-Br-6-F-benzothiophen-2-yl |
| 5-Br-7-F-benzothiophen-2-yl |
| 5-Br-3-(CN)-benzothiophen-2-yl |
| 6-Br-3-(CN)-benzothiophen-2-yl |
| 3-(CN)-7-F-5-$CF_3$-benzothiophen-2-yl |
| 3-Br-5-Cl-benzothiophen-2-yl |
| 3-Br-6-Cl-benzothiophen-2-yl |
| 5-Br-3-(CN)-6-Me-benzothiophen-2-yl |
| 5-Br-3-(CN)-6-F-benzothiophen-2-yl |
| 5-Br-3-(CN)-7-F-benzothiophen-2-yl |
| 5-I-benzothiophen-2-yl |
| 6-I-benzothiophen-2-yl |
| 5-F-benzofuran-2-yl |
| 6-F-benzofuran-2-yl |
| 5-(CN)-benzofuran-2-yl |
| 6-(CN)-benzofuran-2-yl |
| 5-F-3-Me-benzofuran-2-yl |
| 5-F-6-Me-benzofuran-2-yl |
| 6-F-3-Me-benzofuran-2-yl |
| 6-F-5-Me-benzofuran-2-yl |
| 5-Cl-benzofuran-2-yl |
| 6-Cl-benzofuran-2-yl |

[[Table 4A]

| |
|---|
| 5-Cl-3-(CN)-6-F-benzofuran-2-yl |
| 5-Br-benzofuran-2-yl |
| 6-Br-benzofuran-2-yl |
| 5-Br-6-Me-benzofuran-2-yl |
| 3-(CN)-5-$CF_3$-benzofuran-2-yl |
| 3-(CN)-6-$CF_3$-benzofuran-2-yl |
| 5-Br-6-F-benzofuran-2-yl |
| 5-Br-3-(CN)-benzofuran-2-yl |
| 6-Br-3-(CN)-benzofuran-2-yl |
| 5-Br-3-(CN)-6-F-benzofuran-2-yl |
| 5-I-benzofuran-2-yl |
| 6-I-benzofuran-2-yl |
| 1,5-$Me_2$-1H-benzoimidazol-2-yl |
| 1,6-$Me_2$-1H-benzoimidazol-2-yl |
| 4-F-1-Me-1H-benzoimidazol-2-yl |
| 5-F-1-Me-1H-benzoimidazol-2-yl |
| 6-F-1-Me-1H-benzoimidazol-2-yl |
| 7-F-1-Me-1H-benzoimidazol-2-yl |
| 5-(CN)-1-Me-1H-benzoimidazol-2-yl |
| 6-(CN)-1-Me-1H-benzoimidazol-2-yl |
| 1,5,6-$Me_3$-1H-benzoimidazol-2-yl |
| 5-(MeO)-1-Me-1H-benzoimidazol-2-yl |
| 6-(MeO)-1-Me-1H-benzoimidazol-2-yl |
| 4-F-1,6-$Me_2$-1H-benzoimidazol-2-yl |

| |
|---|
| 3,5-$F_2$-benzofuran-2-yl |
| 3,6-$F_2$-benzofuran-2-yl |
| 5,6-$F_2$-benzofuran-2-yl |
| 3-(CN)-5-F-benzofuran-2-yl |
| 3-(CN)-6-F-benzofuran-2-yl |
| 3-Cl-5-F-benzofuran-2-yl |
| 3-Cl-6-F-benzofuran-2-yl |
| 5-Cl-6-F-benzofuran-2-yl |
| 5-Cl-3-(CN)-benzofuran-2-yl |
| 6-Cl-3-(CN)-benzofuran-2-yl |
| 3-(CN)-5,6-$F_2$-benzofuran-2-yl |
| 5-$CF_3$-benzofuran-2-yl |
| 6-$CF_3$-benzofuran-2-yl |
| 3,5-$Cl_2$-benzofuran-2-yl |
| 3,6-$Cl_2$-benzofuran-2-yl |
| 5-Cl-3-(CN)-6-Me-benzofuran-2-yl |

| |
|---|
| 5-F-1,6-$Me_2$-1H-benzoimidazol-2-yl |
| 6-F-1,5-$Me_2$-1H-benzoimidazol-2-yl |
| 7-F-1,5-$Me_2$-1H-benzoimidazol-2-yl |
| 5-Cl-1-Me-1H-benzoimidazol-2-yl |
| 6-Cl-1-Me-1H-benzoimidazol-2-yl |
| 7-Cl-1-Me-1H-benzoimidazol-2-yl |
| 4,5-$F_2$-1-Me-1H-benzoimidazol-2-yl |
| 4,6-$F_2$-1-Me-1H-benzoimidazol-2-yl |
| 5,6-$F_2$-1-Me-1H-benzoimidazol-2-yl |
| 5,7-$F_2$-1-Me-1H-benzoimidazol-2-yl |
| 6,7-$F_2$-1-Me-1H-benzoimidazol-2-yl |
| 3-(CN)-5-F-1-Me-1H-benzoimidazol-2-yl |
| 5-(CN)-6-F-1-Me-1H-benzoimidazol-2-yl |
| 5-Cl-1,6-$Me_2$-1H-benzoimidazol-2-yl |
| 6-Cl-1,5-$Me_2$-1H-benzoimidazol-2-yl |
| 7-Cl-1,5-$Me_2$-1H-benzoimidazol-2-yl |

[Table 5A]

| |
|---|
| 5-($CHF_2$)-1-Me-1H-benzoimidazol-2-yl |
| 6-($CHF_2$)-1-Me-1H-benzoimidazol-2-yl |
| 5-Cl-6-F-1-Me-1H-benzoimidazol-2-yl |
| 5-Cl-7-F-1-Me-1H-benzoimidazol-2-yl |
| 6-Cl-5-F-1-Me-1H-benzoimidazol-2-yl |
| 6-Cl-7-F-1-Me-1H-benzoimidazol-2-yl |
| 7-Cl-5-F-1-Me-1H-benzoimidazol-2-yl |
| 7-Cl-6-F-1-Me-1H-benzoimidazol-2-yl |
| 4,5,6-$F_3$-1-Me-1H-benzoimidazol-2-yl |
| 4,5,7-$F_3$-1-Me-1H-benzoimidazol-2-yl |
| 4,6,7-$F_3$-1-Me-1H-benzoimidazol-2-yl |
| 5,6,7-$F_3$-1-Me-1H-benzoimidazol-2-yl |
| 5-$CF_3$-1-Me-1H-benzoimidazol-2-yl |
| 5-F-6-($CHF_2$)-1-Me-1H-benzoimidazol-2-yl |
| 6-$CF_3$-1-Me-1H-benzoimidazol-2-yl |
| 6-F-5-($CHF_2$)-1-Me-1H-benzoimidazol-2-yl |
| 7-F-5-($CHF_2$)-1-Me-1H-benzoimidazol-2-yl |
| 7-F-6-($CHF_2$)-1-Me-1H-benzoimidazol-2-yl |
| 5,6-$Cl_2$-1-Me-1H-benzoimidazol-2-yl |
| 5,7-$Cl_2$-1-Me-1H-benzoimidazol-2-yl |
| 6,7-$Cl_2$-1-Me-1H-benzoimidazol-2-yl |
| 5-Cl-6,7-$F_2$-1-Me-1H-benzoimidazol-2-yl |

[Table 6A]

| |
|---|
| 6-Br-5-F-1-Me-1H-benzoimidazol-2-yl |
| 6-Br-7-F-1-Me-1H-benzoimidazol-2-yl |
| 5-Cl-1-Me-6-$CF_3$-1H-benzoimidazol-2-yl |
| 6-Cl-5-$CF_3$-1-Me-1H-benzoimidazol-2-yl |
| 7-Cl-1-Me-6-$CF_3$-1H-benzoimidazol-2-yl |
| 7-Cl-5-$CF_3$-1-Me-1H-benzoimidazol-2-yl |
| 5,6,7-$Cl_3$-1-Me-1H-benzoimidazol-2-yl |
| 5-Br-6-Cl-1-Me-1H-benzoimidazol-2-yl |
| 5-Br-7-Cl-1-Me-1H-benzoimidazol-2-yl |
| 6-Br-5-Cl-1-Me-1H-benzoimidazol-2-yl |
| 6-Br-7-Cl-1-Me-1H-benzoimidazol-2-yl |
| 6-Br-5,7-$F_2$-1-Me-1H-benzoimidazol-2-yl |
| 5-I-1-Me-1H-benzoimidazol-2-yl |
| 6-I-1-Me-1H-benzoimidazol-2-yl |
| 5-Br-6-($CHF_2$)-1-Me-1H-benzoimidazol-2-yl |
| 6-Br-5-($CHF_2$)-1-Me-1H-benzoimidazol-2-yl |
| 5-F-6-I-1-Me-1H-benzoimidazol-2-yl |
| 6-F-5-I-1-Me-1H-benzoimidazol-2-yl |
| 5-Br-6-$CF_3$-1-Me-1H-benzoimidazol-2-yl |
| 6-Br-5-$CF_3$-1-Me-1H-benzoimidazol-2-yl |
| 5,6-BR21-Me-1H-benzoimidazol-2-yl |
| 5-Cl-6-I-1-Me-1H-benzoimidazol-2-yl |

| |
|---|
| 6-Cl-5,7-$F_2$-1-Me-1H-benzoimidazol-2-yl |
| 4,5,6,7-$F_4$-1-Me-1H-benzoimidazol-2-yl |

[Table 5A]] (continued)

| |
|---|
| 5-(CN)-6-($CHF_2$)-1-Me-1H-benzoimidazol-2-yl |
| 6-(CN)-5-($CHF_2$)-1-Me-1H-benzoimidazol-2-yl |
| 5-Br-1-Me-1H-benzoimidazol-2-yl |
| 6-Br-1-Me-1H-benzoimidazol-2-yl |
| 5-Cl-6-($CHF_2$)-1-Me-1H-benzoimidazol-2-yl |
| 6-Cl-5-($CHF_2$)-1-Me-1H-benzoimidazol-2-yl |
| 7-Cl-5-($CHF_2$)-1-Me-1H-benzoimidazol-2-yl |
| 7-Cl-6-($CHF_2$)-1-Me-1H-benzoimidazol-2-yl |
| 5-F-6-$CF_3$-1-Me-1H-benzoimidazol-2-yl |
| 6-F-5-$CF_3$-1-Me-1H-benzoimidazol-2-yl |
| 7-F-1-Me-6-$CF_3$-1H-benzoimidazol-2-yl |
| 7-F-5-$CF_3$-1-Me-1H-benzoimidazol-2-yl |
| 5-(CN)-6-$CF_3$-1-Me-1H-benzoimidazol-2-yl |
| 6-(CN)-5-$CF_3$-1-Me-1H-benzoimidazol-2-yl |
| 5-Br-6-F-1-Me-1H-benzoimidazol-2-yl |
| 5-Br-7-F-1-Me-1H-benzoimidazol-2-yl |

| |
|---|
| 6-Cl-5-I-1-Me-1H-benzoimidazol-2-yl |
| 5-Me-benzoxazol-2-yl |

[Table 6A] (continued)

| |
|---|
| 6-Me-benzoxazol-2-yl |
| 4-F-benzoxazol-2-yl |
| 5-F-benzoxazol-2-yl |
| 6-F-benzoxazol-2-yl |
| 7-F-benzoxazol-2-yl |
| 5-(CN)-benzoxazol-2-yl |
| 6-(CN)-benzoxazol-2-yl |
| 5,6-$Me_2$-benzoxazol-2-yl |
| 5-(MeO)-benzoxazol-2-yl |
| 6-(MeO)-benzoxazol-2-yl |
| 4-F-6-Me-benzoxazol-2-yl |
| 5-F-6-Me-benzoxazol-2-yl |
| 6-F-5-Me-benzoxazol-2-yl |
| 7-F-5-Me-benzoxazol-2-yl |
| 5-Cl-benzoxazol-2-yl |
| 6-Cl-benzoxazol-2-yl |

[Table 7A]

| |
|---|
| 7-Cl-benzoxazol-2-yl |
| 4,5-$F_2$-benzoxazol-2-yl |
| 4,6-$F_2$-benzoxazol-2-yl |
| 5,6-$F_2$-benzoxazol-2-yl |
| 5,7-$F_2$-benzoxazol-2-yl |
| 6,7-$F_2$-benzoxazol-2-yl |
| 3-(CN)-5-F-benzoxazol-2-yl |
| 4-F-6-(CN)-benzoxazol-2-yl |
| 5-(CN)-6-F-benzoxazol-2-yl |
| 6-(CN)-4-F-benzoxazol-2-yl |
| 5-Cl-6-Me-benzoxazol-2-yl |
| 6-Cl-5-Me-benzoxazol-2-yl |
| 7-Cl-5-Me-benzoxazol-2-yl |
| 5-($CHF_2$)-benzoxazol-2-yl |
| 6-($CHF_2$)-benzoxazol-2-yl |
| 5-Cl-4-F-benzoxazol-2-yl |
| 5-Cl-6-F-benzoxazol-2-yl |
| 5-Cl-7-F-benzoxazol-2-yl |
| 6-Cl-4-F-benzoxazol-2-yl |
| 6-Cl-5-F-benzoxazol-2-yl |
| 6-Cl-7-F-benzoxazol-2-yl |
| 7-Cl-5-F-benzoxazol-2-yl |
| 7-Cl-6-F-benzoxazol-2-yl |
| 4,5,6-$F_3$-benzoxazol-2-yl |
| 4,5,7-$F_3$-benzoxazol-2-yl |
| 4,6,7-$F_3$-benzoxazol-2-yl |
| 5,6,7-$F_3$-benzoxazol-2-yl |
| 4-F-5-($CHF_2$)-benzoxazol-2-yl |
| 4-F-6-($CHF_2$)-benzoxazol-2-yl |
| 5-$CF_3$-benzoxazol-2-yl |
| 5-F-6-($CHF_2$)-benzoxazol-2-yl |
| 6-$CF_3$-benzoxazol-2-yl |

[Table 8A]

| |
|---|
| 6-Cl-4,5-$F_2$-benzoxazol-2-yl |
| 6-Cl-5,7-$F_2$-benzoxazol-2-yl |
| 4,5,6,7-$F_4$-benzoxazol-2-yl |
| 5-(CN)-6-($CHF_2$)-benzoxazol-2-yl |
| 6-(CN)-5-($CHF_2$)-benzoxazol-2-yl |
| 5-Br-benzoxazol-2-yl |
| 6-Br-benzoxazol-2-yl |
| 5-Cl-6-($CHF_2$)-benzoxazol-2-yl |
| 6-Cl-5-($CHF_2$)-benzoxazol-2-yl |
| 7-Cl-5-($CHF_2$)-benzoxazol-2-yl |
| 7-Cl-6-($CHF_2$)-benzoxazol-2-yl |
| 4-F-5-$CF_3$-benzoxazol-2-yl |
| 4-F-6-$CF_3$-benzoxazol-2-yl |
| 5-F-6-$CF_3$-benzoxazol-2-yl |
| 6-F-5-$CF_3$-benzoxazol-2-yl |
| 7-F-5-$CF_3$-benzoxazol-2-yl |
| 7-F-6-$CF_3$-benzoxazol-2-yl |
| 5-Br-6-Me-benzoxazol-2-yl |
| 6-Br-5-Me-benzoxazol-2-yl |
| 5-(CN)-6-$CF_3$-benzoxazol-2-yl |
| 6-(CN)-5-$CF_3$-benzoxazol-2-yl |
| 5-Br-4-F-benzoxazol-2-yl |
| 5-Br-6-F-benzoxazol-2-yl |
| 5-Br-7-F-benzoxazol-2-yl |
| 6-Br-4-F-benzoxazol-2-yl |
| 6-Br-5-F-benzoxazol-2-yl |
| 6-Br-7-F-benzoxazol-2-yl |
| 5-Cl-6-$CF_3$-benzoxazol-2-yl |
| 6-Cl-5-$CF_3$-benzoxazol-2-yl |
| 7-Cl-5-$CF_3$-benzoxazol-2-yl |
| 7-Cl-6-$CF_3$-benzoxazol-2-yl |
| 5,6,7-$Cl_3$-benzoxazol-2-yl |

| |
|---|
| 6-F-5-($CHF_2$)-benzoxazol-2-yl |
| 7-F-5-($CHF_2$)-benzoxazol-2-yl |
| 7-F-6-($CHF_2$)-benzoxazol-2-yl |
| 5,6-$Cl_2$-benzoxazol-2-yl |
| 5,7-$Cl_2$-benzoxazol-2-yl |
| 6,7-$Cl_2$-benzoxazol-2-yl |
| 5-Cl-4,6-$F_2$-benzoxazol-2-yl |
| 5-Cl-6,7-$F_2$-benzoxazol-2-yl |

| |
|---|
| 5-Br-6-Cl-benzoxazol-2-yl |
| 6-Br-5-Cl-benzoxazol-2-yl |
| 6-Br-7-Cl-benzoxazol-2-yl |
| 5-Br-4,6-$F_2$-benzoxazol-2-yl |
| 6-Br-5,7-$F_2$-benzoxazol-2-yl |
| 5-I-benzoxazol-2-yl |
| 6-I-benzoxazol-2-yl |
| 5-Br-6-($CHF_2$)-benzoxazol-2-yl |

[Table 9A]

| |
|---|
| 6-Br-5-($CHF_2$)-benzoxazol-2-yl |
| 5-F-6-I-benzoxazol-2-yl |
| 6-F-5-I-benzoxazol-2-yl |
| 5-Br-6-$CF_3$-benzoxazol-2-yl |
| 6-Br-5-$CF_3$-benzoxazol-2-yl |
| 5,6-$Br_2$-benzoxazol-2-yl |
| 5-Cl-6-I-benzoxazol-2-yl |
| 6-Cl-5-I-benzoxazol-2-yl |
| 5-Me-benzothiazol-2-yl |
| 6-Me-benzothiazol-2-yl |
| 4-F-benzothiazol-2-yl |
| 5-F-benzothiazol-2-yl |
| 6-F-benzothiazol-2-yl |
| 7-F-benzothiazol-2-yl |
| 5-(CN)-benzothiazol-2-yl |
| 6-(CN)-benzothiazol-2-yl |
| 5,6-$Me_2$-benzothiazol-2-yl |
| 5-(MeO)-benzothiazol-2-yl |
| 6-(MeO)-benzothiazol-2-yl |
| 4-F-6-Me-benzothiazol-2-yl |
| 5-F-6-Me-benzothiazol-2-yl |
| 6-F-5-Me-benzothiazol-2-yl |
| 7-F-5-Me-benzothiazol-2-yl |
| 4-Cl-benzothiazol-2-yl |
| 5-Cl-benzothiazol-2-yl |
| 6-Cl-benzothiazol-2-yl |
| 7-Cl-benzothiazol-2-yl |
| 4,5-$F_2$-benzothiazol-2-yl |
| 4,6-$F_2$-benzothiazol-2-yl |
| 5,6-$F_2$-benzothiazol-2-yl |
| 5,7-$F_2$-benzothiazol-2-yl |
| 6,7-$F_2$-benzothiazol-2-yl |

[Table 10A]

| |
|---|
| 5-($CHF_2$)-benzothiazol-2-yl |
| 6-($CHF_2$)-benzothiazol-2-yl |
| 4-Cl-5-F-benzothiazol-2-yl |
| 4-Cl-6-F-benzothiazol-2-yl |
| 5-Cl-4-F-benzothiazol-2-yl |
| 5-Cl-6-F-benzothiazol-2-yl |
| 5-Cl-7-F-benzothiazol-2-yl |
| 6-Cl-4-F-benzothiazol-2-yl |
| 6-Cl-5-F-benzothiazol-2-yl |
| 6-Cl-7-F-benzothiazol-2-yl |
| 7-Cl-5-F-benzothiazol-2-yl |
| 7-Cl-6-F-benzothiazol-2-yl |
| 4,5,6-$F_3$-benzothiazol-2-yl |
| 4,5,7-$F_3$-benzothiazol-2-yl |
| 4,6,7-$F_3$-benzothiazol-2-yl |
| 5,6,7-$F_3$-benzothiazol-2-yl |
| 4-F-5-($CHF_2$)-benzothiazol-2-yl |
| 4-F-6-($CHF_2$)-benzothiazol-2-yl |
| 5-$CF_3$-benzothiazol-2-yl |
| 5-F-6-($CHF_2$)-benzothiazol-2-yl |
| 6-$CF_3$-benzothiazol-2-yl |
| 6-F-5-($CHF_2$)-benzothiazol-2-yl |
| 7-F-5-($CHF_2$)-benzothiazol-2-yl |
| 7-F-6-($CHF_2$)-benzothiazol-2-yl |
| 4,5-$Cl_2$-benzothiazol-2-yl |
| 4,6-$Cl_2$-benzothiazol-2-yl |
| 5,6-$Cl_2$-benzothiazol-2-yl |
| 5,7-$Cl_2$-benzothiazol-2-yl |
| 6,7-$Cl_2$-benzothiazol-2-yl |
| 5-Cl-4,6-$F_2$-benzothiazol-2-yl |
| 5-Cl-6,7-$F_2$-benzothiazol-2-yl |
| 6-Cl-4,5-$F_2$-benzothiazol-2-yl |

| |
|---|
| 3-(CN)-5-F-benzothiazol-2-yl |
| 4-F-6-(CN)-benzothiazol-2-yl |
| 5-(CN)-6-F-benzothiazol-2-yl |
| 6-(CN)-4-F-benzothiazol-2-yl |
| 4-Cl-6-Me-benzothiazol-2-yl |
| 5-Cl-6-Me-benzothiazol-2-yl |
| 6-Cl-5-Me-benzothiazol-2-yl |
| 7-Cl-5-Me-benzothiazol-2-yl |

| |
|---|
| 6-Cl-5,7-$F_2$-benzothiazol-2-yl |
| 4,5,6,7-$F_4$-benzothiazol-2-yl |
| 5-(CN)-6-($CHF_2$)-benzothiazol-2-yl |
| 6-(CN)-5-($CHF_2$)-benzothiazol-2-yl |
| 5-Br-benzothiazol-2-yl |
| 6-Br-benzothiazol-2-yl |
| 4-Cl-5-($CHF_2$)-benzothiazol-2-yl |
| 4-Cl-6-($CHF_2$)-benzothiazol-2-yl |

[Table 11A]

| |
|---|
| 5-Cl-6-($CHF_2$)-benzothiazol-2-yl |
| 6-Cl-5-($CHF_2$)-benzothiazol-2-yl |
| 7-Cl-5-($CHF_2$)-benzothiazol-2-yl |
| 7-Cl-6-($CHF_2$)-benzothiazol-2-yl |
| 4-F-5-$CF_3$-benzothiazol-2-yl |
| 4-F-6-$CF_3$-benzothiazol-2-yl |
| 5-F-6-$CF_3$-benzothiazol-2-yl |
| 6-F-5-$CF_3$-benzothiazol-2-yl |
| 7-F-5-$CF_3$-benzothiazol-2-yl |
| 7-F-6-$CF_3$-benzothiazol-2-yl |
| 5-Br-6-Me-benzothiazol-2-yl |
| 6-Br-5-Me-benzothiazol-2-yl |
| 5-(CN)-6-$CF_3$-benzothiazol-2-yl |
| 6-(CN)-5-$CF_3$-benzothiazol-2-yl |
| 5-Br-4-F-benzothiazol-2-yl |
| 5-Br-6-F-benzothiazol-2-yl |
| 5-Br-7-F-benzothiazol-2-yl |
| 6-Br-4-F-benzothiazol-2-yl |
| 6-Br-5-F-benzothiazol-2-yl |
| 6-Br-7-F-benzothiazol-2-yl |
| 4-Cl-5-$CF_3$-benzothiazol-2-yl |
| 4-Cl-6-$CF_3$-benzothiazol-2-yl |

[Table 12A]

| |
|---|
| 6-Br-5-($CHF_2$)-benzothiazol-2-yl |
| 5-F-6-I-benzothiazol-2-yl |
| 6-F-5-I-benzothiazol-2-yl |
| 5-Br-6-$CF_3$-benzothiazol-2-yl |
| 6-Br-5-$CF_3$-benzothiazol-2-yl |
| 5,6-$Br_2$-benzothiazol-2-yl |
| 5-Cl-6-I-benzothiazol-2-yl |
| 6-Cl-5-I-benzothiazol-2-yl |
| 5-F-1H-pyrrolo[2,3-c]pyridin-2-yl |
| 5-Cl-1H-pyrrolo[2,3-c]pyridin-2-yl |
| 5-F-thieno[2,3-b]pyridin-2-yl |
| 6-F-thieno[2,3-b]pyridin-2-yl |
| 5-Cl-thieno[2,3-b]pyridin-2-yl |
| 6-Cl-thieno[2,3-b]pyridin-2-yl |
| 5-Br-thieno[2,3-b]pyridin-2-yl |
| 6-Br-thieno[2,3-b]pyridin-2-yl |
| 2-F-thieno[2,3-d]pyrimidin-6-yl |
| 2-Cl-thieno[2,3-d]pyrimidin-6-yl |
| 6-F-thieno[3,2-b]pyridin-2-yl |
| 6-Cl-thieno[3,2-b]pyridin-2-yl |
| 6-Br-thieno[3,2-b]pyridin-2-yl |
| 6-F-thieno[3,2-c]pyridin-2-yl |

| |
|---|
| 5-Cl-6-$CF_3$-benzothiazol-2-yl |
| 6-Cl-5-$CF_3$-benzothiazol-2-yl |
| 7-Cl-5-$CF_3$-benzothiazol-2-yl |
| 7-Cl-6-$CF_3$-benzothiazol-2-yl |
| 4,5,6-$Cl_3$-benzothiazol-2-yl |
| 4,5,7-$Cl_3$-benzothiazol-2-yl |
| 4,6,7-$Cl_3$-benzothiazol-2-yl |
| 5,6,7-$Cl_3$-benzothiazol-2-yl |
| 5-Br-4-Cl-benzothiazol-2-yl |
| 5-Br-6-Cl-benzothiazol-2-yl |
| 6-Br-4-Cl-benzothiazol-2-yl |
| 6-Br-5-Cl-benzothiazol-2-yl |
| 6-Br-7-Cl-benzothiazol-2-yl |
| 5-Br-4,6-$F_2$-benzothiazol-2-yl |
| 6-Br-5,7-$F_2$-benzothiazol-2-yl |
| 5-I-benzothiazol-2-yl |
| 6-I-benzothiazol-2-yl |
| 5-Br-6-($CHF_2$)-benzothiazol-2-yl |

| |
|---|
| 6-Cl-thieno[3,2-c]pyridin-2-yl |
| 5-F-furo[2,3-b]pyridin-2-yl |
| 5-Cl-furo[2,3-b]pyridin-2-yl |
| 5-Br-furo[2,3-b]pyridin-2-yl |
| 6-F-imidazo[1,2-a]pyrazin-2-yl |
| 6-Cl-imidazo[1,2-a]pyrazin-2-yl |
| 6-($CHF_2$)-imidazo[1,2-a]pyrazin-2-yl |
| 6-$CF_3$-imidazo[1,2-a]pyrazin-2-yl |
| 6-Br-imidazo[1,2-a]pyrazin-2-yl |
| 6-F-imidazo[1,2-a]pyridin-2-yl |
| 7-F-imidazo[1,2-a]pyridin-2-yl |
| 6-(MeO)-imidazo[1,2-a]pyridin-2-yl |
| 7-(MeO)-imidazo[1,2-a]pyridin-2-yl |
| 6-F-3-Me-imidazo[1,2-a]pyridin-2-yl |
| 6-Cl-imidazo[1,2-a]pyridin-2-yl |
| 7-Cl-imidazo[1,2-a]pyridin-2-yl |
| 6-($CHF_2$)-imidazo[1,2-a]pyridin-2-yl |
| 7-($CHF_2$)-imidazo[1,2-a]pyridin-2-yl |

[Table 13A]

| |
|---|
| 6-$CF_3$-imidazo[1,2-a]pyridin-2-yl |
| 7-$CF_3$-imidazo[1,2-a]pyridin-2-yl |
| 6-Br-imidazo[1,2-a]pyridin-2-yl |
| 7-Br-imidazo[1,2-a]pyridin-2-yl |
| 6-I-imidazo[1,2-a]pyridin-2-yl |
| 7-I-imidazo[1,2-a]pyridin-2-yl |
| 6-I-3-Me-imidazo[1,2-a]pyridin-2-yl |
| 6-F-imidazo[1,2-a]pyrimidin-2-yl |
| 7-F-imidazo[1,2-a]pyrimidin-2-yl |
| 6-Cl-imidazo[1,2-a]pyrimidin-2-yl |
| 7-Cl-imidazo[1,2-a]pyrimidin-2-yl |
| 6-($CHF_2$)-imidazo[1,2-a]pyrimidin-2-yl |
| 7-($CHF_2$)-imidazo[1,2-a]pyrimidin-2-yl |
| 6-$CF_3$-imidazo[1,2-a]pyrimidin-2-yl |
| 7-$CF_3$-imidazo[1,2-a]pyrimidin-2-yl |
| 6-Br-imidazo[1,2-a]pyrimidin-2-yl |
| 7-Br-imidazo[1,2-a]pyrimidin-2-yl |
| 6-F-imidazo[1,2-b]pyridazin-2-yl |
| 6-Cl-imidazo[1,2-b]pyridazin-2-yl |
| 6-($CHF_2$)-imidazo[1,2-b]pyridazin-2-yl |
| 6-$CF_3$-imidazo[1,2-b]pyridazin-2-yl |
| 6-Br-imidazo[1,2-b]pyridazin-2-yl |

[Table 14A]

| |
|---|
| 5-Cl-6-F-1-Me-3H-imidazo[4,5-b]pyridin-2-yl |
| 5-Cl-6-F-3-Me-3H-imidazo[4,5-b]pyridin-2-yl |
| 6-Cl-5-F-1-Me-3H-imidazo[4,5-b]pyridin-2-yl |
| 6-Cl-5-F-3-Me-3H-imidazo[4,5-b]pyridin-2-yl |
| 5-$CF_3$-1-Me-3H-imidazo[4,5-b]pyridin-2-yl |
| 5-$CF_3$-3-Me-3H-imidazo[4,5-b]pyridin-2-yl |
| 6-$CF_3$-1-Me-1H-imidazo[4,5-b]pyridin-2-yl |
| 6-$CF_3$-3-Me-3H-imidazo[4,5-b]pyridin-2-yl |
| 5,6-$Cl_2$-1-Me-3H-imidazo[4,5-b]pyridin-2-yl |
| 5,6-$Cl_2$-3-Me-3H-imidazo[4,5-b]pyridin-2-yl |
| 5-Br-1-Me-3H-imidazo[4,5-b]pyridin-2-yl |
| 5-Br-3-Me-3H-imidazo[4,5-b]pyridin-2-yl |
| 6-Br-1-Me-1H-imidazo[4,5-b]pyridin-2-yl |
| 6-Br-3-Me-3H-imidazo[4,5-b]pyridin-2-yl |
| 5-$CF_3$-6-F-1-Me-3H-imidazo[4,5-b]pyridin-2-yl |
| 5-$CF_3$-6-F-3-Me-3H-imidazo[4,5-b]pyridin-2-yl |
| 6-$CF_3$-5-F-1-Me-3H-imidazo[4,5-b]pyridin-2-yl |
| 6-$CF_3$-5-F-3-Me-3H-imidazo[4,5-b]pyridin-2-yl |
| 6-F-1-Me-1H-imidazo[4,5-c]pyridin-2-yl |
| 6-Cl-1-Me-1H-imidazo[4,5-c]pyridin-2-yl |
| 4,6-$F_2$-3-Me-3H-imidazo[4,5-c]pyridin-2-yl |
| 3-Me-6-$CF_3$-3H-imidazo[4,5-c]pyridin-2-yl |

[Table 13A] (continued)

| |
|---|
| 7-F-imidazo[1,2-c]pyrimidin-2-yl |
| 7-Cl-imidazo[1,2-c]pyrimidin-2-yl |
| 7-$CF_3$-imidazo[1,2-c]pyrimidin-2-yl |
| 7-($CHF_2$)-imidazo[1,2-c]pyrimidin-2-yl |
| 7-Br-imidazo[1,2-c]pyrimidin-2-yl |
| 5-F-1-Me-1H-imidazo[4,5-b]pyrazin-2-yl |
| 5-Cl-1-Me-1H-imidazo[4,5-b]pyrazin-2-yl |
| -Br-1-Me-1H-imidazo[4,5-b]pyrazin-2-yl |
| 5-F-1-Me-3H-imidazo[4,5-b]pyridin-2-yl |
| 5-F-3-Me-3H-imidazo[4,5-b]pyridin-2-yl |
| 6-F-1-Me-1H-imidazo[4,5-b]pyridin-2-yl |
| 6-F-3-Me-3H-imidazo[4,5-b]pyridin-2-yl |
| 5-Cl-1-Me-3H-imidazo[4,5-b]pyridin-2-yl |
| 5-Cl-3-Me-3H-imidazo[4,5-b]pyridin-2-yl |
| 6-Cl-1-Me-1H-imidazo[4,5-b]pyridin-2-yl |
| 6-Cl-3-Me-3H-imidazo[4,5-b]pyridin-2-yl |
| 5,6-$F_2$-1-Me-3H-imidazo[4,5-b]pyridin-2-yl |
| 5,6-$F_2$-3-Me-3H-imidazo[4,5-b]pyridin-2-yl |

[Table 14A] (continued)

| |
|---|
| 4,6-$Cl_2$-3-Me-3H-imidazo[4,5-c]pyridin-2-yl |
| 6-Br-1-Me-1H-imidazo[4,5-c]pyridin-2-yl |
| 5-F-oxazolo[4,5-b]pyridin-2-yl |
| 6-F-oxazolo[4,5-b]pyridin-2-yl |
| 5-Cl-oxazolo[4,5-b]pyridin-2-yl |
| 6-Cl-oxazolo[4,5-b]pyridin-2-yl |
| 5,6-$F_2$-oxazolo[4,5-b]pyridin-2-yl |
| 5-($CHF_2$)-oxazolo[4,5-b]pyridin-2-yl |
| 6-($CHF_2$)-oxazolo[4,5-b]pyridin-2-yl |
| 5-Cl-6-F-oxazolo[4,5-b]pyridin-2-yl |
| 6-Cl-5-F-oxazolo[4,5-b]pyridin-2-yl |
| 5-$CF_3$-oxazolo[4,5-b]pyridin-2-yl |
| 5-F-6-($CHF_2$)-oxazolo[4,5-b]pyridin-2-yl |
| 6-$CF_3$-oxazolo[4,5-b]pyridin-2-yl |
| 6-F5-($CHF_2$)-oxazolo[4,5-b]pyridin-2-yl |
| 5,6-$Cl_2$-oxazolo[4,5-b]pyridin-2-yl |
| 5-Br-oxazolo[4,5-b]pyridin-2-yl |
| 6-Br-oxazolo[4,5-b]pyridin-2-yl |

[Table 15A]

| |
|---|
| 5-F-6-$CF_3$-oxazolo[4,5-b]pyridin-2-yl |
| 6-F5-$CF_3$-oxazolo[4,5-b]pyridin-2-yl |
| 5-Ioxazolo[4,5-b]pyridin-2-yl |
| 6-Ioxazolo[4,5-b]pyridin-2-yl |
| 6-F-oxazolo[4,5-c]pyridin-2-yl |
| 6-Cl-oxazolo[4,5-c]pyridin-2-yl |
| 6-($CHF_2$)-oxazolo[4,5-c]pyridin-2-yl |
| 6-$CF_3$-oxazolo[4,5-c]pyridin-2-yl |
| 6-Br-oxazolo[4,5-c]pyridin-2-yl |
| 5-F-oxazolo[5,4-b]pyridin-2-yl |
| 6-F-oxazolo[5,4-b]pyridin-2-yl |
| 6-F-5-Me-oxazolo[5,4-b]pyridin-2-yl |
| 5-Cl-oxazolo[5,4-b]pyridin-2-yl |
| 6-Cl-oxazolo[5,4-b]pyridin-2-yl |
| 5,6-$F_2$-oxazolo[5,4-b]pyridin-2-yl |
| 6-Cl-5-Me-oxazolo[5,4-b]pyridin-2-yl |
| 5-($CHF_2$)-oxazolo[5,4-b]pyridin-2-yl |
| 6-($CHF_2$)-oxazolo[5,4-b]pyridin-2-yl |
| 5-Cl-6-F-oxazolo[5,4-b]pyridin-2-yl |
| 5-F-6-Cl-oxazolo[5,4-b]pyridin-2-yl |
| 5-F-6-($CHF_2$)-oxazolo[5,4-b]pyridin-2-yl |
| 5-$CF_3$-oxazolo[5,4-b]pyridin-2-yl |

[Table 16A]

| |
|---|
| 6-F-thiazolo[4,5-b]pyridin-2-yl |
| 5-Cl-thiazolo[4,5-b]pyridin-2-yl |
| 6-Cl-thiazolo[4,5-b]pyridin-2-yl |
| 5,6-$F_2$-thiazolo[4,5-b]pyridin-2-yl |
| 5-($CHF_2$)-thiazolo[4,5-b]pyridin-2-yl |
| 6-($CHF_2$)-thiazolo[4,5-b]pyridin-2-yl |
| 5-Cl-6-F-thiazolo[4,5-b]pyridin-2-yl |
| 6-Cl-5-F-thiazolo[4,5-b]pyridin-2-yl |
| 5-$CF_3$-thiazolo[4,5-b]pyridin-2-yl |
| 5-F-6-($CHF_2$)-thiazolo[4,5-b]pyridin-2-yl |
| 6-$CF_3$-thiazolo[4,5-b]pyridin-2-yl |
| 6-F5-($CHF_2$)-thiazolo[4,5-b]pyridin-2-yl |
| 5,6-$Cl_2$-thiazolo[4,5-b]pyridin-2-yl |
| 5-Br-thiazolo[4,5-b]pyridin-2-yl |
| 6-Br-thiazolo[4,5-b]pyridin-2-yl |
| 5-F-6-$CF_3$-thiazolo[4,5-b]pyridin-2-yl |
| 6-F5-$CF_3$-thiazolo[4,5-b]pyridin-2-yl |
| 5-I-thiazolo[4,5-b]pyridin-2-yl |
| 6-I-thiazolo[4,5-b]pyridin-2-yl |
| 3-F-thiazolo[4,5-c]pyridazin-6-yl |
| 3-Cl-thiazolo[4,5-c]pyridazin-6-yl |
| 3-($CHF_2$)-thiazolo[4,5-c]pyridazin-6-yl |

| |
|---|
| 3-$CF_3$-thiazolo[4,5-c]pyridazin-6-yl |
| 6-F-thiazolo[4,5-c]pyridin-2-yl |
| 6-Cl-thiazolo[4,5-c]pyridin-2-yl |
| 6-($CHF_2$)-thiazolo[4,5-c]pyridin-2-yl |
| 6-$CF_3$-thiazolo[4,5-c]pyridin-2-yl |
| 6-Br-thiazolo[4,5-c]pyridin-2-yl |
| 5-F-thiazolo[4,5-d]pyrimidin-2-yl |
| 5-Cl-thiazolo[4,5-d]pyrimidin-2-yl |
| 5-Br-thiazolo[4,5-d]pyrimidin-2-yl |
| 5-F-thiazolo[5,4-b]pyridin-2-yl |

[Table 16A](continued)

| |
|---|
| 6-F-thiazolo[5,4-b]pyridin-2-yl |
| 5-ethynylthiazolo[5,4-b]pyridin-2-yl |
| 6-F-5-Methiazolo[5,4-b]pyridin-2-yl |
| 5-Cl-thiazolo[5,4-b]pyridin-2-yl |
| 6-Cl-thiazolo[5,4-b]pyridin-2-yl |
| 5,6-$F_2$-thiazolo[5,4-b]pyridin-2-yl |
| 6-Cl-5-Methiazolo[5,4-b]pyridin-2-yl |
| 5-($CHF_2$)-thiazolo[5,4-b]pyridin-2-yl |

| |
|---|
| 6-$CF_3$-oxazolo[5,4-b]pyridin-2-yl |
| 6-F-5-($CHF_2$)-oxazolo[5,4-b]pyridin-2-yl |
| 5-Br-oxazolo[5,4-b]pyridin-2-yl |
| 6-Br-oxazolo[5,4-b]pyridin-2-yl |
| 5-F-6-$CF_3$-oxazolo[5,4-b]pyridin-2-yl |
| 5-$CF_3$-6-F-oxazolo[5,4-b]pyridin-2-yl |
| 6-Br-5-Me-oxazolo[5,4-b]pyridin-2-yl |
| 5-I-oxazolo[5,4-b]pyridin-2-yl |
| 6-I-oxazolo[5,4-b]pyridin-2-yl |
| 3-F-oxazolo[5,4-c]pyridazin-6-yl |

[Table 15A] (continued)

| |
|---|
| 3-Cl-oxazolo[5,4-c]pyridazin-6-yl |
| 5-F-thiazolo[4,5-b]pyrazin-2-yl |
| 6-F-thiazolo[4,5-b]pyrazin-2-yl |
| 5-Cl-thiazolo[4,5-b]pyrazin-2-yl |
| 6-Cl-thiazolo[4,5-b]pyrazin-2-yl |
| 5-Br-thiazolo[4,5-b]pyrazin-2-yl |
| 6-Br-thiazolo[4,5-b]pyrazin-2-yl |
| 5-F-thiazolo[4,5-b]pyridin-2-yl |

[Table 17A]

| |
|---|
| 6-($CHF_2$)-thiazolo[5,4-b]pyridin-2-yl |
| 5-Cl-6-F-thiazolo[5,4-b]pyridin-2-yl |
| 5-F-6-Cl-thiazolo[5,4-b]pyridin-2-yl |
| 5-F-6-($CHF_2$)-thiazolo[5,4-b]pyridin-2-yl |
| 5-$CF_3$-thiazolo[5,4-b]pyridin-2-yl |
| 6-$CF_3$-thiazolo[5,4-b]pyridin-2-yl |
| 6-F-5-($CHF_2$)-thiazolo[5,4-b]pyridin-2-yl |
| 5-Br-thiazolo[5,4-b]pyridin-2-yl |
| 6-Br-thiazolo[5,4-b]pyridin-2-yl |
| 5-F-6-$CF_3$-thiazolo[5,4-b]pyridin-2-yl |
| 5-$CF_3$-6-F-thiazolo[5,4-b]pyridin-2-yl |
| 6-Br-5-Methiazolo[5,4-b]pyridin-2-yl |
| 5-I-thiazolo[5,4-b]pyridin-2-yl |
| 6-I-thiazolo[5,4-b]pyridin-2-yl |
| 3-F-thiazolo[5,4-c]pyridazin-6-yl |
| 3-Cl-thiazolo[5,4-c]pyridazin-6-yl |
| 6-F-thiazolo[5,4-c]pyridin-2-yl |
| 6-Cl-thiazolo[5,4-c]pyridin-2-yl |
| 6-($CHF_2$)-thiazolo[5,4-c]pyridin-2-yl |
| 6-$CF_3$-thiazolo[5,4-c]pyridin-2-yl |

[Table 18A]

| |
|---|
| 5-F-pyrazolo[1,5-a]pyrimidin-2-yl |
| 6-F-pyrazolo[1,5-a]pyrimidin-2-yl |
| 5-Cl-pyrazolo[1,5-a]pyrimidin-2-yl |
| 6-Cl-pyrazolo[1,5-a]pyrimidin-2-yl |
| 6-Br-pyrazolo[1,5-a]pyrimidin-2-yl |
| 6-F-3-Ipyrazolo[1,5-a]pyrimidin-2-yl |
| 6-Cl-3-Ipyrazolo[1,5-a]pyrimidin-2-yl |
| 6-BR3ipyrazolo[1,5-a]pyrimidin-2-yl |
| 5-F-pyrazolo[1,5-c]pyrimidin-2-yl |
| 5-Cl-pyrazolo[1,5-c]pyrimidin-2-yl |
| 6-F-[1,2,4]triazolo[1,5-a]pyrazin-2-yl |
| 6-Cl-[1,2,4]triazolo[1,5-a]pyrazin-2-yl |
| 6-Br-[1,2,4]triazolo[1,5-a]pyrazin-2-yl |
| 5-F-6-I-[1,2,4]triazolo[1,5-a]pyrazin-2-yl |
| 5-Cl-6-I-[1,2,4]triazolo[1,5-a]pyrazin-2-yl |
| 6-F-[1,2,4]triazolo[1,5-a]pyridin-2-yl |
| 7-F-[1,2,4]triazolo[1,5-a]pyridin-2-yl |
| 7-Cl-[1,2,4]triazolo[1,5-a]pyridin-2-yl |
| 5,7-$F_2$-[1,2,4]triazolo[1,5-a]pyridin-2-yl |
| 6-F-5-F-[1,2,4]triazolo[1,5-a]pyridin-2-yl |

| |
|---|
| 6-Br-thiazolo[5,4-c]pyridin-2-yl |
| 5-F-thiazolo[5,4-d]pyrimidin-2-yl |
| 5-Cl-thiazolo[5,4-d]pyrimidin-2-yl |
| 5-($CHF_2$)-thiazolo[5,4-d]pyrimidin-2-yl |
| 5-$CF_3$-thiazolo[5,4-d]pyrimidin-2-yl |

| |
|---|
| 6-F-7-F-[1,2,4]triazolo[1,5-a]pyridin-2-yl |
| 6-F-8-F-[1,2,4]triazolo[1,5-a]pyridin-2-yl |
| 6,8-$F_2$-[1,2,4]triazolo[1,5-a]pyridin-2-yl |
| 6-Cl-7-F-[1,2,4]triazolo[1,5-a]pyridin-2-yl |
| 6-Cl-8-F-[1,2,4]triazolo[1,5-a]pyridin-2-yl |

[Table 17A](continued)

| |
|---|
| 6-F-1H-pyrrolo[3,2-b]pyridin-2-yl |
| 6-Cl-1H-pyrrolo[3,2-b]pyridin-2-yl |
| 3-(CN)-5-F-1H-pyrrolo[3,2-b]pyridin-2-yl |
| 3-(CN)-5-Cl-1H-pyrrolo[3,2-b]pyridin-2-yl |
| 6-Br-1H-pyrrolo[3,2-b]pyridin-2-yl |
| 3-(CN)-5-$CF_3$-1H-pyrrolo[3,2-b]pyridin-2-yl |
| 3-(CN)-5-Br-1H-pyrrolo[3,2-b]pyridin-2-yl |
| 5-F-pyrazolo[1,5-a]pyridin-2-yl |
| 6-F-pyrazolo[1,5-a]pyridin-2-yl |
| 5-Cl-pyrazolo[1,5-a]pyridin-2-yl |
| 6-Cl-pyrazolo[1,5-a]pyridin-2-yl |
| 6-$CF_3$-pyrazolo[1,5-a]pyridin-2-yl |
| 5-Br-pyrazolo[1,5-a]pyridin-2-yl |
| 6-Br-pyrazolo[1,5-a]pyridin-2-yl |
| 6-I-pyrazolo[1,5-a]pyridin-2-yl |

[Table 18A] (continued)

| |
|---|
| 6-$CF_3$-[1,2,4]triazolo[1,5-a]pyridin-2-yl |
| 7-$CF_3$-[1,2,4]triazolo[1,5-a]pyridin-2-yl |
| 5,7-$Cl_2$-[1,2,4]triazolo[1,5-a]pyridin-2-yl |
| 6-Cl-5-Cl-[1,2,4]triazolo[1,5-a]pyridin-2-yl |
| 6-Cl-7-Cl-[1,2,4]triazolo[1,5-a]pyridin-2-yl |
| 6-Cl-8-Cl-[1,2,4]triazolo[1,5-a]pyridin-2-yl |
| 6,8-$Cl_2$-[1,2,4]triazolo[1,5-a]pyridin-2-yl |
| 6-Br-[1,2,4]triazolo[1,5-a]pyridin-2-yl |
| 7-Br-[1,2,4]triazolo[1,5-a]pyridin-2-yl |
| 5-F-7-$CF_3$-[1,2,4]triazolo[1,5-a]pyridin-2-yl |
| 6-F-7-$CF_3$-[1,2,4]triazolo[1,5-a]pyridin-2-yl |
| 7-F-6-$CF_3$-[1,2,4]triazolo[1,5-a]pyridin-2-yl |
| 8-F-6-$CF_3$-[1,2,4]triazolo[1,5-a]pyridin-2-yl |
| 6-Br-7-Me-[1,2,4]triazolo[1,5-a]pyridin-2-yl |
| 6-Br-5-F-[1,2,4]triazolo[1,5-a]pyridin-2-yl |

[Table 19A]

| |
|---|
| 6-Br-7-F-[1,2,4]triazolo[1,5-a]pyridin-2-yl |
| 6-Br-8-F-[1,2,4]triazolo[1,5-a]pyridin-2-yl |
| 5-Cl-7-$CF_3$-[1,2,4]triazolo[1,5-a]pyridin-2-yl |

(continued)

| |
|---|
| 6-Cl-7-$CF_3$-[1,2,4]triazolo[1,5-a]pyridin-2-yl |
| 7-Cl-6-$CF_3$-[1,2,4]triazolo[1,5-a]pyridin-2-yl |
| 8-Cl-6-$CF_3$-[1,2,4]triazolo[1,5-a]pyridin-2-yl |
| 6-Br-5-Cl-[1,2,4]triazolo[1,5-a]pyridin-2-yl |
| 6-Br-7-Cl-[1,2,4]triazolo[1,5-a]pyridin-2-yl |
| 6-BR8cl-[1,2,4]triazolo[1,5-a]pyridin-2-yl |
| 6-I-[1,2,4]triazolo[1,5-a]pyridin-2-yl |
| 7-I-[1,2,4]triazolo[1,5-a]pyridin-2-yl |
| 8-F-7-I-[1,2,4]triazolo[1,5-a]pyridin-2-yl |
| 6-Br-7-$CF_3$-[1,2,4]triazolo[1,5-a]pyridin-2-yl |
| 5-F-[1,2,4]triazolo[1,5-a]pyrimidin-2-yl |

[Table 20A]

| |
|---|
| 6-F-[1,2,4]triazolo[1,5-a]pyrimidin-2-yl |
| 5-Cl-[1,2,4]triazolo[1,5-a]pyrimidin-2-yl |
| 6-Cl-[1,2,4]triazolo[1,5-a]pyrimidin-2-yl |
| 6-Br-[1,2,4]triazolo[1,5-a]pyrimidin-2-yl |
| 7-F-[1,2,4]triazolo[1,5-c]pyrimidin-2-yl |
| 7-Cl-[1,2,4]triazolo[1,5-c]pyrimidin-2-yl |
| 6-F-[1,2,4]triazolo[1,5-b]pyridazin-2-yl |
| 6-Cl-[1,2,4]triazolo[1,5-b]pyridazin-2-yl |
| 6-Br-[1,2,4]triazolo[1,5-b]pyridazin-2-yl |
| 6-F-oxazolo[5,4-c]pyridin-2-yl |
| 6-Cl-oxazolo[5,4-c]pyridin-2-yl |
| 6-($CHF_2$)-oxazolo[5,4-c]pyridin-2-yl |
| 6-$CF_3$-oxazolo[5,4-c]pyridin-2-yl |
| 6-Br-oxazolo[5,4-c]pyridin-2-yl |

A compound (L-1) wherein n is 0, $R^1$ represents a methyl group, Q represents a group represented by Q1-2, and Het represents any substituents indicated in [Table 1A] to [Table 20A] (hereinafter, referred to as Compound Class SX2).

A compound (L-1) wherein n is 0, $R^1$ represents a methyl group, Q represents a group represented by Q1-3, and Het represents any substituents indicated in [Table 1A] to [Table 20A] (hereinafter, referred to as Compound Class SX3).

A compound (L-1) wherein n is 0, $R^1$ represents a methyl group, Q represents a group represented by Q1-4, and Het represents any substituents indicated in [Table 1A] to [Table 20A] (hereinafter, referred to as Compound Class SX4).

A compound (L-1) wherein n is 0, $R^1$ represents a methyl group, Q represents a group represented by Q1-5, and Het represents any substituents indicated in [Table 1A] to [Table 20A] (hereinafter, referred to as Compound Class SX5).

A compound (L-1) wherein n is 0, $R^1$ represents a methyl group, Q represents a group represented by Q1-6, and Het represents any substituents indicated in [Table 1A] to [Table 20A] (hereinafter, referred to as Compound Class SX6).

A compound (L-1) wherein n is 0, $R^1$ represents a methyl group, Q represents a group represented by Q1-7, and Het represents any substituents indicated in [Table 1A] to [Table 20A] (hereinafter, referred to as Compound Class SX7).

A compound (L-1) wherein n is 0, $R^1$ represents a methyl group, Q represents a group represented by Q1-8, and Het represents any substituents indicated in [Table 1A] to [Table 20A] (hereinafter, referred to as Compound Class SX8).

A compound (L-1) wherein n is 0, $R^1$ represents a methyl group, Q represents a group represented by Q1-9, and Het represents any substituents indicated in [Table 1A] to [Table 20A] (hereinafter, referred to as Compound Class SX9).

A compound (L-1) wherein n is 0, $R^1$ represents a methyl group, Q represents a group represented by Q1-10, and Het represents any substituents indicated in [Table 1A] to [Table 20A] (hereinafter, referred to as Compound Class SX10).

A compound (L-1) wherein n is 0, $R^1$ represents a methyl group, Q represents a group represented by Q1-11, and Het represents any substituents indicated in [Table 1A] to [Table 20A] (hereinafter, referred to as Compound Class SX11).

A compound (L-1) wherein n is 0, $R^1$ represents a methyl group, Q represents a group represented by Q1-12, and Het represents any substituents indicated in [Table 1A] to [Table 20A] (hereinafter, referred to as Compound Class SX12).

A compound (L-1) wherein n is 0, $R^1$ represents a methyl group, Q represents a group represented by Q1-13, and Het represents any substituents indicated in [Table 1A] to [Table 20A] (hereinafter, referred to as Compound Class SX13).

A compound (L-1) wherein n is 0, $R^1$ represents a methyl group, Q represents a group represented by Q1-14, and Het represents any substituents indicated in [Table 1A] to [Table 20A] (hereinafter, referred to as Compound Class SX14).

A compound (L-1) wherein n is 0, $R^1$ represents a methyl group, Q represents a group represented by Q1-15, and Het represents any substituents indicated in [Table 1A] to [Table 20A] (hereinafter, referred to as Compound Class SX15).

A compound (L-1) wherein n is 0, $R^1$ represents a methyl group, Q represents a group represented by Q1-16, and Het represents any substituents indicated in [Table 1A] to [Table 20A] (hereinafter, referred to as Compound Class SX16).

A compound (L-1) wherein n is 0, $R^1$ represents a methyl group, Q represents a group represented by Q1-17, and Het represents any substituents indicated in [Table 1A] to [Table 20A] (hereinafter, referred to as Compound Class SX17).

A compound (L-1) wherein n is 0, $R^1$ represents a methyl group, Q represents a group represented by Q1-18, and Het represents any substituents indicated in [Table 1A] to [Table 20A] (hereinafter, referred to as Compound Class SX18).

A compound (L-1) wherein n is 0, $R^1$ represents a methyl group, Q represents a group represented by Q1-19, and Het represents any substituents indicated in [Table 1A] to [Table 20A] (hereinafter, referred to as Compound Class SX19).

A compound (L-1) wherein n is 0, $R^1$ represents a methyl group, Q represents a group represented by Q1-20, and Het represents any substituents indicated in [Table 1A] to [Table 20A] (hereinafter, referred to as Compound Class SX20).

A compound (L-1) wherein n is 0, $R^1$ represents a methyl group, Q represents a group represented by Q1-21, and Het represents any substituents indicated in [Table 1A] to [Table 20A] (hereinafter, referred to as Compound Class SX21).

A compound (L-1) wherein n is 0, $R^1$ represents a methyl group, Q represents a group represented by Q1-22, and Het represents any substituents indicated in [Table 1A] to [Table 20A] (hereinafter, referred to as Compound Class SX22).

A compound (L-1) wherein n is 0, $R^1$ represents a methyl group, Q represents a group represented by Q1-23, and Het represents any substituents indicated in [Table 1A] to [Table 20A] (hereinafter, referred to as Compound Class SX23).

A compound (L-1) wherein n is 0, $R^1$ represents a methyl group, Q represents a group represented by Q1-24, and Het represents any substituents indicated in [Table 1A] to [Table 20A] (hereinafter, referred to as Compound Class SX24).

A compound (L-1) wherein n is 0, $R^1$ represents a methyl group, Q represents a group represented by Q1-25, and Het represents any substituents indicated in [Table 1A] to [Table 20A] (hereinafter, referred to as Compound Class SX25).

A compound (L-1) wherein n is 0, $R^1$ represents a methyl group, Q represents a group represented by Q1-26, and Het represents any substituents indicated in [Table 1A] to [Table 20A] (hereinafter, referred to as Compound Class SX26).

A compound (L-1) wherein n is 0, $R^1$ represents a methyl group, Q represents a group represented by Q1-27, and Het represents any substituents indicated in [Table 1A] to [Table 20A] (hereinafter, referred to as Compound Class SX27).

A compound (L-1) wherein n is 0, $R^1$ represents a methyl group, Q represents a group represented by Q1-28, and Het represents any substituents indicated in [Table 1A] to [Table 20A] (hereinafter, referred to as Compound Class SX28).

A compound (L-1) wherein n is 0, $R^1$ represents a methyl group, Q represents a group represented by Q1-29, and Het represents any substituents indicated in [Table 1A] to [Table 20A] (hereinafter, referred to as Compound Class SX29).

A compound (L-1) wherein n is 0, $R^1$ represents a methyl group, Q represents a group represented by Q1-30, and Het represents any substituents indicated in [Table 1A] to [Table 20A] (hereinafter, referred to as Compound Class SX30).

A compound (L-1) wherein n is 0, $R^1$ represents a methyl group, Q represents a group represented by Q1-31, and Het represents any substituents indicated in [Table 1A] to [Table 20A] (hereinafter, referred to as Compound Class SX31).

A compound (L-1) wherein n is 0, $R^1$ represents a methyl group, Q represents a group represented by Q1-32, and Het represents any substituents indicated in [Table 1A] to [Table 20A] (hereinafter, referred to as Compound Class SX32).

A compound (L-1) wherein n is 0, $R^1$ represents a methyl group, Q represents a group represented by Q1-33, and Het represents any substituents indicated in [Table 1A] to [Table 20A] (hereinafter, referred to as Compound Class SX33).

A compound (L-1) wherein n is 0, $R^1$ represents a methyl group, Q represents a group represented by Q1-34, and Het represents any substituents indicated in [Table 1A] to [Table 20A] (hereinafter, referred to as Compound Class SX34).

A compound (L-1) wherein n is 0, $R^1$ represents a methyl group, Q represents a group represented by Q1-35, and Het represents any substituents indicated in [Table 1A] to [Table 20A] (hereinafter, referred to as Compound Class SX35).

A compound (L-1) wherein n is 0, $R^1$ represents a methyl group, Q represents a group represented by Q1-36, and Het represents any substituents indicated in [Table 1A] to [Table 20A] (hereinafter, referred to as Compound Class SX36).

A compound (L-1) wherein n is 0, $R^1$ represents a methyl group, Q represents a group represented by Q1-37, and Het represents any substituents indicated in [Table 1A] to [Table 20A] (hereinafter, referred to as Compound Class SX37).

A compound (L-1) wherein n is 0, $R^1$ represents a methyl group, Q represents a group represented by Q1-38, and Het represents any substituents indicated in [Table 1A] to [Table 20A] (hereinafter, referred to as Compound Class SX38).

A compound (L-1) wherein n is 0, $R^1$ represents a methyl group, Q represents a group represented by Q1-39, and Het represents any substituents indicated in [Table 1A] to [Table 20A] (hereinafter, referred to as Compound Class SX39).

A compound (L-1) wherein n is 0, $R^1$ represents a methyl group, Q represents a group represented by Q1-40, and Het represents any substituents indicated in [Table 1A] to [Table 20A] (hereinafter, referred to as Compound Class SX40).

A compound (L-1) wherein n is 0, $R^1$ represents a methyl group, Q represents a group represented by Q1-41, and Het represents any substituents indicated in [Table 1A] to [Table 20A] (hereinafter, referred to as Compound Class SX41).

A compound (L-1) wherein n is 0, $R^1$ represents a methyl group, Q represents a group represented by Q1-42, and Het represents any substituents indicated in [Table 1A] to [Table 20A] (hereinafter, referred to as Compound Class SX42).

A compound (L-1) wherein n is 0, $R^1$ represents a methyl group, Q represents a group represented by Q1-43, and Het represents any substituents indicated in [Table 1A] to [Table 20A] (hereinafter, referred to as Compound Class SX43).

A compound (L-1) wherein n is 0, $R^1$ represents a methyl group, Q represents a group represented by Q1-44, and Het represents any substituents indicated in [Table 1A] to [Table 20A] (hereinafter, referred to as Compound Class SX44).

A compound (L-1) wherein n is 0, $R^1$ represents a methyl group, Q represents a group represented by Q1-45, and Het represents any substituents indicated in [Table 1A] to [Table 20A] (hereinafter, referred to as Compound Class SX45).

A compound (L-1) wherein n is 0, $R^1$ represents a methyl group, Q represents a group represented by Q1-46, and Het represents any substituents indicated in [Table 1A] to [Table 20A] (hereinafter, referred to as Compound Class SX46).

A compound (L-1) wherein n is 0, $R^1$ represents a methyl group, Q represents a group represented by Q1-47, and Het represents any substituents indicated in [Table 1A] to [Table 20A] (hereinafter, referred to as Compound Class SX47).

A compound (L-1) wherein n is 0, $R^1$ represents a methyl group, Q represents a group represented by Q1-48, and Het represents any substituents indicated in [Table 1A] to [Table 20A] (hereinafter, referred to as Compound Class SX48).

A compound (L-1) wherein n is 0, $R^1$ represents a methyl group, Q represents a group represented by Q1-49, and Het represents any substituents indicated in [Table 1A] to [Table 20A] (hereinafter, referred to as Compound Class SX49).

A compound (L-1) wherein n is 0, $R^1$ represents a methyl group, Q represents a group represented by Q1-50, and Het represents any substituents indicated in [Table 1A] to [Table 20A] (hereinafter, referred to as Compound Class SX50).

A compound (L-1) wherein n is 0, $R^1$ represents a methyl group, Q represents a group represented by Q1-51, and Het represents any substituents indicated in [Table 1A] to [Table 20A] (hereinafter, referred to as Compound Class SX51).

A compound (L-1) wherein n is 0, $R^1$ represents a methyl group, Q represents a group represented by Q1-52, and Het represents any substituents indicated in [Table 1A] to [Table 20A] (hereinafter, referred to as Compound Class SX52).

A compound (L-1) wherein n is 0, $R^1$ represents a methyl group, Q represents a group represented by Q1-53, and Het represents any substituents indicated in [Table 1A] to [Table 20A] (hereinafter, referred to as Compound Class SX53).

A compound (L-1) wherein n is 0, $R^1$ represents a methyl group, Q represents a group represented by Q1-54, and Het represents any substituents indicated in [Table 1A] to [Table 20A] (hereinafter, referred to as Compound Class SX54).

A compound (L-1) wherein n is 0, $R^1$ represents a methyl group, Q represents a group represented by Q1-55, and Het represents any substituents indicated in [Table 1A] to [Table 20A] (hereinafter, referred to as Compound Class SX55).

A compound (L-1) wherein n is 0, $R^1$ represents a methyl group, Q represents a group represented by Q1-56, and Het represents any substituents indicated in [Table 1A] to [Table 20A] (hereinafter, referred to as Compound Class SX56).

A compound (L-1) wherein n is 0, $R^1$ represents a methyl group, Q represents a group represented by Q1-57, and Het represents any substituents indicated in [Table 1A] to [Table 20A] (hereinafter, referred to as Compound Class SX57).

A compound (L-1) wherein n is 0, $R^1$ represents a methyl group, Q represents a group represented by Q1-58, and Het represents any substituents indicated in [Table 1A] to [Table 20A] (hereinafter, referred to as Compound Class SX58).

A compound (L-1) wherein n is 0, $R^1$ represents a methyl group, Q represents a group represented by Q1-59, and Het represents any substituents indicated in [Table 1A] to [Table 20A] (hereinafter, referred to as Compound Class SX59).

A compound (L-1) wherein n is 0, $R^1$ represents a methyl group, Q represents a group represented by Q1-60, and Het represents any substituents indicated in [Table 1A] to [Table 20A] (hereinafter, referred to as Compound Class SX60).

A compound (L-1) wherein n is 0, $R^1$ represents a methyl group, Q represents a group represented by Q1-61, and Het represents any substituents indicated in [Table 1A] to [Table 20A] (hereinafter, referred to as Compound Class SX61).

A compound (L-1) wherein n is 0, $R^1$ represents a methyl group, Q represents a group represented by Q1-62, and Het represents any substituents indicated in [Table 1A] to [Table 20A] (hereinafter, referred to as Compound Class SX62).

A compound (L-1) wherein n is 0, $R^1$ represents a methyl group, Q represents a group represented by Q1-63, and Het represents any substituents indicated in [Table 1A] to [Table 20A] (hereinafter, referred to as Compound Class SX63).

A compound (L-1) wherein n is 0, $R^1$ represents a methyl group, Q represents a group represented by Q1-64, and Het represents any substituents indicated in [Table 1A] to [Table 20A] (hereinafter, referred to as Compound Class SX64).

A compound (L-1) wherein n is 0, $R^1$ represents a methyl group, Q represents a group represented by Q1-65, and Het represents any substituents indicated in [Table 1A] to [Table 20A] (hereinafter, referred to as Compound Class SX65).

A compound (L-1) wherein n is 0, $R^1$ represents a methyl group, Q represents a group represented by Q1-66, and Het represents any substituents indicated in [Table 1A] to [Table 20A] (hereinafter, referred to as Compound Class SX66).

A compound (L-1) wherein n is 0, $R^1$ represents a methyl group, Q represents a group represented by Q1-67, and Het represents any substituents indicated in [Table 1A] to [Table 20A] (hereinafter, referred to as Compound Class SX67).

A compound (L-1) wherein n is 0, $R^1$ represents a methyl group, Q represents a group represented by Q1-68, and Het represents any substituents indicated in [Table 1A] to [Table 20A] (hereinafter, referred to as Compound Class SX68).

A compound (L-1) wherein n is 0, $R^1$ represents a methyl group, Q represents a group represented by Q1-69, and Het represents any substituents indicated in [Table 1A] to [Table 20A] (hereinafter, referred to as Compound Class SX69).
A compound (L-1) wherein n is 0, $R^1$ represents a methyl group, Q represents a group represented by Q1-70, and Het represents any substituents indicated in [Table 1A] to [Table 20A] (hereinafter, referred to as Compound Class SX70).
A compound (L-1) wherein n is 0, $R^1$ represents a methyl group, Q represents a group represented by Q1-71, and Het represents any substituents indicated in [Table 1A] to [Table 20A] (hereinafter, referred to as Compound Class SX71).
A compound (L-1) wherein n is 0, $R^1$ represents a methyl group, Q represents a group represented by Q1-72, and Het represents any substituents indicated in [Table 1A] to [Table 20A] (hereinafter, referred to as Compound Class SX72).
A compound (L-1) wherein n is 0, $R^1$ represents a methyl group, Q represents a group represented by Q1-73, and Het represents any substituents indicated in [Table 1A] to [Table 20A] (hereinafter, referred to as Compound Class SX73).
A compound (L-1) wherein n is 0, $R^1$ represents a methyl group, Q represents a group represented by Q1-74, and Het represents any substituents indicated in [Table 1A] to [Table 20A] (hereinafter, referred to as Compound Class SX74).
A compound (L-1) wherein n is 0, $R^1$ represents a methyl group, Q represents a group represented by Q1-75, and Het represents any substituents indicated in [Table 1A] to [Table 20A] (hereinafter, referred to as Compound Class SX75).
A compound (L-1) wherein n is 0, $R^1$ represents a methyl group, Q represents a group represented by Q1-76, and Het represents any substituents indicated in [Table 1A] to [Table 20A] (hereinafter, referred to as Compound Class SX76).
A compound (L-1) wherein n is 0, $R^1$ represents a methyl group, Q represents a group represented by Q1-77, and Het represents any substituents indicated in [Table 1A] to [Table 20A] (hereinafter, referred to as Compound Class SX77).
A compound (L-1) wherein n is 0, $R^1$ represents a methyl group, Q represents a group represented by Q1-78, and Het represents any substituents indicated in [Table 1A] to [Table 20A] (hereinafter, referred to as Compound Class SX78).
A compound (L-1) wherein n is 0, $R^1$ represents a methyl group, Q represents a group represented by Q1-79, and Het represents any substituents indicated in [Table 1A] to [Table 20A] (hereinafter, referred to as Compound Class SX79).
A compound (L-1) wherein n is 0, $R^1$ represents a methyl group, Q represents a group represented by Q1-80, and Het represents any substituents indicated in [Table 1A] to [Table 20A] (hereinafter, referred to as Compound Class SX80).
A compound (L-1) wherein n is 0, $R^1$ represents a methyl group, Q represents a group represented by Q1-81, and Het represents any substituents indicated in [Table 1A] to [Table 20A] (hereinafter, referred to as Compound Class SX81).
A compound (L-1) wherein n is 0, $R^1$ represents a methyl group, Q represents a group represented by Q1-82, and Het represents any substituents indicated in [Table 1A] to [Table 20A] (hereinafter, referred to as Compound Class SX82).
A compound (L-1) wherein n is 0, $R^1$ represents a methyl group, Q represents a group represented by Q1-83, and Het represents any substituents indicated in [Table 1A] to [Table 20A] (hereinafter, referred to as Compound Class SX83).
A compound (L-1) wherein n is 0, $R^1$ represents a methyl group, Q represents a group represented by Q1-84, and Het represents any substituents indicated in [Table 1A] to [Table 20A] (hereinafter, referred to as Compound Class SX84).
A compound (L-1) wherein n is 0, $R^1$ represents a methyl group, Q represents a group represented by Q1-85, and Het represents any substituents indicated in [Table 1A] to [Table 20A] (hereinafter, referred to as Compound Class SX85).
A compound (L-1) wherein n is 0, $R^1$ represents a methyl group, Q represents a group represented by Q1-86, and Het represents any substituents indicated in [Table 1A] to [Table 20A] (hereinafter, referred to as Compound Class SX86).
A compound (L-1) wherein n is 0, $R^1$ represents a methyl group, Q represents a group represented by Q1-87, and Het represents any substituents indicated in [Table 1A] to [Table 20A] (hereinafter, referred to as Compound Class SX87).
A compound (L-1) wherein n is 0, $R^1$ represents a methyl group, Q represents a group represented by Q1-88, and Het represents any substituents indicated in [Table 1A] to [Table 20A] (hereinafter, referred to as Compound Class SX88).
A compound (L-1) wherein n is 0, $R^1$ represents a methyl group, Q represents a group represented by Q1-89, and Het represents any substituents indicated in [Table 1A] to [Table 20A] (hereinafter, referred to as Compound Class SX89).
A compound (L-1) wherein n is 0, $R^1$ represents a methyl group, Q represents a group represented by Q1-90, and Het represents any substituents indicated in [Table 1A] to [Table 20A] (hereinafter, referred to as Compound Class SX90).
A compound (L-1) wherein n is 0, $R^1$ represents a methyl group, Q represents a group represented by Q1-91, and Het represents any substituents indicated in [Table 1A] to [Table 20A] (hereinafter, referred to as Compound Class SX91).
A compound (L-1) wherein n is 0, $R^1$ represents a methyl group, Q represents a group represented by Q1-92, and Het represents any substituents indicated in [Table 1A] to [Table 20A] (hereinafter, referred to as Compound Class SX92).
A compound (L-1) wherein n is 0, $R^1$ represents a methyl group, Q represents a group represented by Q1-93, and Het represents any substituents indicated in [Table 1A] to [Table 20A] (hereinafter, referred to as Compound Class SX93).
A compound (L-1) wherein n is 0, $R^1$ represents a methyl group, Q represents a group represented by Q1-94, and Het represents any substituents indicated in [Table 1A] to [Table 20A] (hereinafter, referred to as Compound Class SX94).
A compound (L-1) wherein n is 0, $R^1$ represents a methyl group, Q represents a group represented by Q1-95, and Het represents any substituents indicated in [Table 1A] to [Table 20A] (hereinafter, referred to as Compound Class SX95).
A compound (L-1) wherein n is 0, $R^1$ represents a methyl group, Q represents a group represented by Q1-96, and Het represents any substituents indicated in [Table 1A] to [Table 20A] (hereinafter, referred to as Compound Class SX96).
A compound (L-1) wherein n is 0, $R^1$ represents a methyl group, Q represents a group represented by Q1-97, and Het represents any substituents indicated in [Table 1A] to [Table 20A] (hereinafter, referred to as Compound Class SX97).

A compound (L-1) wherein n is 0, $R^1$ represents a methyl group, Q represents a group represented by Q1-98, and Het represents any substituents indicated in [Table 1A] to [Table 20A] (hereinafter, referred to as Compound Class SX98).

A compound (L-1) wherein n is 0, $R^1$ represents a methyl group, Q represents a group represented by Q1-99, and Het represents any substituents indicated in [Table 1A] to [Table 20A] (hereinafter, referred to as Compound Class SX99).

A compound (L-1) wherein n is 0, $R^1$ represents a methyl group, Q represents a group represented by Q1-100, and Het represents any substituents indicated in [Table 1A] to [Table 20A] (hereinafter, referred to as Compound Class SX100).

A compound (L-1) wherein n is 0, $R^1$ represents a methyl group, Q represents a group represented by Q1-101, and Het represents any substituents indicated in [Table 1A] to [Table 20A] (hereinafter, referred to as Compound Class SX101).

A compound (L-1) wherein n is 0, $R^1$ represents a methyl group, Q represents a group represented by Q1-102, and Het represents any substituents indicated in [Table 1A] to [Table 20A] (hereinafter, referred to as Compound Class SX102).

A compound (L-1) wherein n is 0, $R^1$ represents a methyl group, Q represents a group represented by Q1-103, and Het represents any substituents indicated in [Table 1A] to [Table 20A] (hereinafter, referred to as Compound Class SX103).

A compound (L-1) wherein n is 0, $R^1$ represents a methyl group, Q represents a group represented by Q1-104, and Het represents any substituents indicated in [Table 1A] to [Table 20A] (hereinafter, referred to as Compound Class SX104).

A compound (L-1) wherein n is 0, $R^1$ represents a methyl group, Q represents a group represented by Q1-105, and Het represents any substituents indicated in [Table 1A] to [Table 20A] (hereinafter, referred to as Compound Class SX105).

A compound (L-1) wherein n is 0, $R^1$ represents a methyl group, Q represents a group represented by Q1-106, and Het represents any substituents indicated in [Table 1A] to [Table 20A] (hereinafter, referred to as Compound Class SX106).

A compound (L-1) wherein n is 0, $R^1$ represents a methyl group, Q represents a group represented by Q1-107, and Het represents any substituents indicated in [Table 1A] to [Table 20A] (hereinafter, referred to as Compound Class SX107).

A compound (L-1) wherein n is 0, $R^1$ represents a methyl group, Q represents a group represented by Q1-108, and Het represents any substituents indicated in [Table 1A] to [Table 20A] (hereinafter, referred to as Compound Class SX108).

A compound (L-1) wherein n is 0, $R^1$ represents a methyl group, Q represents a group represented by Q1-109, and Het represents any substituents indicated in [Table 1A] to [Table 20A] (hereinafter, referred to as Compound Class SX109).

A compound (L-1) wherein n is 0, $R^1$ represents a methyl group, Q represents a group represented by Q1-110, and Het represents any substituents indicated in [Table 1A] to [Table 20A] (hereinafter, referred to as Compound Class SX110).

A compound (L-1) wherein n is 0, $R^1$ represents a methyl group, Q represents a group represented by Q1-111, and Het represents any substituents indicated in [Table 1A] to [Table 20A] (hereinafter, referred to as Compound Class SX111).

A compound (L-1) wherein n is 0, $R^1$ represents a methyl group, Q represents a group represented by Q1-112, and Het represents any substituents indicated in [Table 1A] to [Table 20A] (hereinafter, referred to as Compound Class SX112).

A compound (L-1) wherein n is 0, $R^1$ represents a methyl group, Q represents a group represented by Q1-113, and Het represents any substituents indicated in [Table 1A] to [Table 20A] (hereinafter, referred to as Compound Class SX113).

A compound (L-1) wherein n is 0, $R^1$ represents a methyl group, Q represents a group represented by Q1-114, and Het represents any substituents indicated in [Table 1A] to [Table 20A] (hereinafter, referred to as Compound Class SX114).

A compound (L-1) wherein n is 0, $R^1$ represents a methyl group, Q represents a group represented by Q1-115, and Het represents any substituents indicated in [Table 1A] to [Table 20A] (hereinafter, referred to as Compound Class SX115).

A compound (L-1) wherein n is 0, $R^1$ represents a methyl group, Q represents a group represented by Q1-116, and Het represents any substituents indicated in [Table 1A] to [Table 20A] (hereinafter, referred to as Compound Class SX116).

A compound (L-1) wherein n is 0, $R^1$ represents a methyl group, Q represents a group represented by Q1-117, and Het represents any substituents indicated in [Table 1A] to [Table 20A] (hereinafter, referred to as Compound Class SX117).

A compound (L-1) wherein n is 0, $R^1$ represents a methyl group, Q represents a group represented by Q1-118, and Het represents any substituents indicated in [Table 1A] to [Table 20A] (hereinafter, referred to as Compound Class SX118).
A compound (L-1) wherein n is 0, $R^1$ represents a methyl group, Q represents a group represented by Q1-119, and Het represents any substituents indicated in [Table 1A] to [Table 20A] (hereinafter, referred to as Compound Class SX119).
A compound (L-1) wherein n is 0, $R^1$ represents a methyl group, Q represents a group represented by Q1-120, and Het represents any substituents indicated in [Table 1A] to [Table 20A] (hereinafter, referred to as Compound Class SX120).
A compound (L-1) wherein n is 0, $R^1$ represents a methyl group, Q represents a group represented by Q1-121, and Het represents any substituents indicated in [Table 1A] to [Table 20A] (hereinafter, referred to as Compound Class SX121).
A compound (L-1) wherein n is 0, $R^1$ represents a methyl group, Q represents a group represented by Q1-122, and Het represents any substituents indicated in [Table 1A] to [Table 20A] (hereinafter, referred to as Compound Class SX122).
A compound (L-1) wherein n is 0, $R^1$ represents a methyl group, Q represents a group represented by Q1-123, and Het represents any substituents indicated in [Table 1A] to [Table 20A] (hereinafter, referred to as Compound Class SX123).
A compound (L-1) wherein n is 0, $R^1$ represents a methyl group, Q represents a group represented by Q1-124, and Het represents any substituents indicated in [Table 1A] to [Table 20A] (hereinafter, referred to as Compound Class SX124).
A compound (L-1) wherein n is 0, $R^1$ represents a methyl group, Q represents a group represented by Q1-125, and Het represents any substituents indicated in [Table 1A] to [Table 20A] (hereinafter, referred to as Compound Class SX125).
A compound (L-1) wherein n is 0, $R^1$ represents a methyl group, Q represents a group represented by Q1-126, and Het represents any substituents indicated in [Table 1A] to [Table 20A] (hereinafter, referred to as Compound Class SX126).
A compound (L-1) wherein n is 0, $R^1$ represents a methyl group, Q represents a group represented by Q1-127, and Het represents any substituents indicated in [Table 1A] to [Table 20A] (hereinafter, referred to as Compound Class SX127).
A compound (L-1) wherein n is 0, $R^1$ represents a methyl group, Q represents a group represented by Q1-128, and Het represents any substituents indicated in [Table 1A] to [Table 20A] (hereinafter, referred to as Compound Class SX128).
A compound (L-1) wherein n is 0, $R^1$ represents a methyl group, Q represents a group represented by Q1-129, and Het represents any substituents indicated in [Table 1A] to [Table 20A] (hereinafter, referred to as Compound Class SX129).
A compound (L-1) wherein n is 0, $R^1$ represents a methyl group, Q represents a group represented by Q1-130, and Het represents any substituents indicated in [Table 1A] to [Table 20A] (hereinafter, referred to as Compound Class SX130).
A compound (L-1) wherein n is 0, $R^1$ represents a methyl group, Q represents a group represented by Q1-131, and Het represents any substituents indicated in [Table 1A] to [Table 20A] (hereinafter, referred to as Compound Class SX131).
A compound (L-1) wherein n is 0, $R^1$ represents a methyl group, Q represents a group represented by Q1-132, and Het represents any substituents indicated in [Table 1A] to [Table 20A] (hereinafter, referred to as Compound Class SX132).
A compound (L-1) wherein n is 0, $R^1$ represents a methyl group, Q represents a group represented by Q1-133, and Het represents any substituents indicated in [Table 1A] to [Table 20A] (hereinafter, referred to as Compound Class SX133).
A compound (L-1) wherein n is 0, $R^1$ represents a methyl group, Q represents a group represented by Q1-134, and Het represents any substituents indicated in [Table 1A] to [Table 20A] (hereinafter, referred to as Compound Class SX134).
A compound (L-1) wherein n is 0, $R^1$ represents a methyl group, Q represents a group represented by Q1-135, and Het represents any substituents indicated in [Table 1A] to [Table 20A] (hereinafter, referred to as Compound Class SX135).
A compound (L-1) wherein n is 0, $R^1$ represents a methyl group, Q represents a group represented by Q1-136, and Het represents any substituents indicated in [Table 1A] to [Table 20A] (hereinafter, referred to as Compound Class SX136).
A compound (L-1) wherein n is 0, $R^1$ represents a methyl group, Q represents a group represented by Q1-137,

and Het represents any substituents indicated in [Table 1A] to [Table 20A] (hereinafter, referred to as Compound Class SX137).

A compound (L-1) wherein n is 0, $R^1$ represents a methyl group, Q represents a group represented by Q1-138, and Het represents any substituents indicated in [Table 1A] to [Table 20A] (hereinafter, referred to as Compound Class SX138).

A compound (L-1) wherein n is 0, $R^1$ represents a methyl group, Q represents a group represented by Q1-139, and Het represents any substituents indicated in [Table 1A] to [Table 20A] (hereinafter, referred to as Compound Class SX139).

A compound (L-1) wherein n is 0, $R^1$ represents a methyl group, Q represents a group represented by Q1-140, and Het represents any substituents indicated in [Table 1A] to [Table 20A] (hereinafter, referred to as Compound Class SX140).

A compound (L-1) wherein n is 0, $R^1$ represents a methyl group, Q represents a group represented by Q1-141, and Het represents any substituents indicated in [Table 1A] to [Table 20A] (hereinafter, referred to as Compound Class SX141).

A compound (L-1) wherein n is 0, $R^1$ represents a methyl group, Q represents a group represented by Q1-142, and Het represents any substituents indicated in [Table 1A] to [Table 20A] (hereinafter, referred to as Compound Class SX142).

A compound (L-1) wherein n is 0, $R^1$ represents a methyl group, Q represents a group represented by Q1-143, and Het represents any substituents indicated in [Table 1A] to [Table 20A] (hereinafter, referred to as Compound Class SX143).

A compound (L-1) wherein n is 0, $R^1$ represents a methyl group, Q represents a group represented by Q1-144, and Het represents any substituents indicated in [Table 1A] to [Table 20A] (hereinafter, referred to as Compound Class SX144).

A compound (L-1) wherein n is 0, $R^1$ represents a methyl group, Q represents a group represented by Q1-145, and Het represents any substituents indicated in [Table 1A] to [Table 20A] (hereinafter, referred to as Compound Class SX145).

A compound (L-1) wherein n is 0, $R^1$ represents a methyl group, Q represents a group represented by Q1-146, and Het represents any substituents indicated in [Table 1A] to [Table 20A] (hereinafter, referred to as Compound Class SX146).

A compound (L-1) wherein n is 0, $R^1$ represents a methyl group, Q represents a group represented by Q1-147, and Het represents any substituents indicated in [Table 1A] to [Table 20A] (hereinafter, referred to as Compound Class SX147).

A compound (L-1) wherein n is 0, $R^1$ represents a methyl group, Q represents a group represented by Q1-148, and Het represents any substituents indicated in [Table 1A] to [Table 20A] (hereinafter, referred to as Compound Class SX148).

A compound (L-1) wherein n is 0, $R^1$ represents a methyl group, Q represents a group represented by Q1-149, and Het represents any substituents indicated in [Table 1A] to [Table 20A] (hereinafter, referred to as Compound Class SX149).

A compound (L-1) wherein n is 0, $R^1$ represents a methyl group, Q represents a group represented by Q1-150, and Het represents any substituents indicated in [Table 1A] to [Table 20A] (hereinafter, referred to as Compound Class SX150).

A compound (L-1) wherein n is 0, $R^1$ represents a methyl group, Q represents a group represented by Q1-151, and Het represents any substituents indicated in [Table 1A] to [Table 20A] (hereinafter, referred to as Compound Class SX151).

A compound (L-1) wherein n is 0, $R^1$ represents a methyl group, Q represents a group represented by Q1-152, and Het represents any substituents indicated in [Table 1A] to [Table 20A] (hereinafter, referred to as Compound Class SX152).

A compound (L-1) wherein n is 0, $R^1$ represents a methyl group, Q represents a group represented by Q1-153, and Het represents any substituents indicated in [Table 1A] to [Table 20A] (hereinafter, referred to as Compound Class SX153).

A compound (L-1) wherein n is 0, $R^1$ represents a methyl group, Q represents a group represented by Q1-154, and Het represents any substituents indicated in [Table 1A] to [Table 20A] (hereinafter, referred to as Compound Class SX154).

A compound (L-1) wherein n is 0, $R^1$ represents a methyl group, Q represents a group represented by Q1-155, and Het represents any substituents indicated in [Table 1A] to [Table 20A] (hereinafter, referred to as Compound Class SX155).

A compound (L-1) wherein n is 0, $R^1$ represents a methyl group, Q represents a group represented by Q1-156, and Het represents any substituents indicated in [Table 1A] to [Table 20A] (hereinafter, referred to as Compound Class

SX156).

A compound (L-1) wherein n is 0, $R^1$ represents a methyl group, Q represents a group represented by Q1-157, and Het represents any substituents indicated in [Table 1A] to [Table 20A] (hereinafter, referred to as Compound Class SX157).

A compound (L-1) wherein n is 0, $R^1$ represents a methyl group, Q represents a group represented by Q1-158, and Het represents any substituents indicated in [Table 1A] to [Table 20A] (hereinafter, referred to as Compound Class SX158).

A compound (L-1) wherein n is 0, $R^1$ represents a methyl group, Q represents a group represented by Q1-159, and Het represents any substituents indicated in [Table 1A] to [Table 20A] (hereinafter, referred to as Compound Class SX159).

A compound (L-1) wherein n is 0, $R^1$ represents a methyl group, Q represents a group represented by Q1-160, and Het represents any substituents indicated in [Table 1A] to [Table 20A] (hereinafter, referred to as Compound Class SX160).

A compound (L-1) wherein n is 0, $R^1$ represents a methyl group, Q represents a group represented by Q1-161, and Het represents any substituents indicated in [Table 1A] to [Table 20A] (hereinafter, referred to as Compound Class SX161).

A compound (L-1) wherein n is 0, $R^1$ represents a methyl group, Q represents a group represented by Q1-162, and Het represents any substituents indicated in [Table 1A] to [Table 20A] (hereinafter, referred to as Compound Class SX162).

A compound (L-1) wherein n is 0, $R^1$ represents a methyl group, Q represents a group represented by Q1-163, and Het represents any substituents indicated in [Table 1A] to [Table 20A] (hereinafter, referred to as Compound Class SX163).

A compound (L-1) wherein n is 0, $R^1$ represents a methyl group, Q represents a group represented by Q1-164, and Het represents any substituents indicated in [Table 1A] to [Table 20A] (hereinafter, referred to as Compound Class SX164).

A compound (L-1) wherein n is 0, $R^1$ represents a methyl group, Q represents a group represented by Q1-165, and Het represents any substituents indicated in [Table 1A] to [Table 20A] (hereinafter, referred to as Compound Class SX165).

A compound (L-1) wherein n is 0, $R^1$ represents a methyl group, Q represents a group represented by Q1-166, and Het represents any substituents indicated in [Table 1A] to [Table 20A] (hereinafter, referred to as Compound Class SX166).

A compound (L-1) wherein n is 0, $R^1$ represents a methyl group, Q represents a group represented by Q1-167, and Het represents any substituents indicated in [Table 1A] to [Table 20A] (hereinafter, referred to as Compound Class SX167).

A compound (L-1) wherein n is 0, $R^1$ represents a methyl group, Q represents a group represented by Q1-168, and Het represents any substituents indicated in [Table 1A] to [Table 20A] (hereinafter, referred to as Compound Class SX168).

A compound (L-1) wherein n is 0, $R^1$ represents a methyl group, Q represents a group represented by Q1-169, and Het represents any substituents indicated in [Table 1A] to [Table 20A] (hereinafter, referred to as Compound Class SX169).

A compound (L-1) wherein n is 0, $R^1$ represents a methyl group, Q represents a group represented by Q1-170, and Het represents any substituents indicated in [Table 1A] to [Table 20A] (hereinafter, referred to as Compound Class SX170).

A compound (L-1) wherein n is 0, $R^1$ represents a methyl group, Q represents a group represented by Q1-171, and Het represents any substituents indicated in [Table 1A] to [Table 20A] (hereinafter, referred to as Compound Class SX171).

A compound (L-1) wherein n is 0, $R^1$ represents a methyl group, Q represents a group represented by Q1-172, and Het represents any substituents indicated in [Table 1A] to [Table 20A] (hereinafter, referred to as Compound Class SX172).

A compound (L-1) wherein n is 0, $R^1$ represents a methyl group, Q represents a group represented by Q1-173, and Het represents any substituents indicated in [Table 1A] to [Table 20A] (hereinafter, referred to as Compound Class SX173).

A compound (L-1) wherein n is 0, $R^1$ represents a methyl group, Q represents a group represented by Q1-174, and Het represents any substituents indicated in [Table 1A] to [Table 20A] (hereinafter, referred to as Compound Class SX174).

A compound (L-1) wherein n is 0, $R^1$ represents a methyl group, Q represents a group represented by Q1-175, and Het represents any substituents indicated in [Table 1A] to [Table 20A] (hereinafter, referred to as Compound Class SX175).

A compound (L-1) wherein n is 0, $R^1$ represents a methyl group, Q represents a group represented by Q1-176, and Het represents any substituents indicated in [Table 1A] to [Table 20A] (hereinafter, referred to as Compound Class SX176).

A compound (L-1) wherein n is 0, $R^1$ represents a methyl group, Q represents a group represented by Q1-177, and Het represents any substituents indicated in [Table 1A] to [Table 20A] (hereinafter, referred to as Compound Class SX177).

A compound (L-1) wherein n is 0, $R^1$ represents a methyl group, Q represents a group represented by Q1-178, and Het represents any substituents indicated in [Table 1A] to [Table 20A] (hereinafter, referred to as Compound Class SX178).

A compound (L-1) wherein n is 0, $R^1$ represents a methyl group, Q represents a group represented by Q1-179, and Het represents any substituents indicated in [Table 1A] to [Table 20A] (hereinafter, referred to as Compound Class SX179).

A compound (L-1) wherein n is 0, $R^1$ represents a methyl group, Q represents a group represented by Q1-180, and Het represents any substituents indicated in [Table 1A] to [Table 20A] (hereinafter, referred to as Compound Class SX180).

A compound (L-1) wherein n is 0, $R^1$ represents a methyl group, Q represents a group represented by Q1-181, and Het represents any substituents indicated in [Table 1A] to [Table 20A] (hereinafter, referred to as Compound Class SX181).

A compound (L-1) wherein n is 0, $R^1$ represents a methyl group, Q represents a group represented by Q1-182, and Het represents any substituents indicated in [Table 1A] to [Table 20A] (hereinafter, referred to as Compound Class SX182).

A compound (L-1) wherein n is 0, $R^1$ represents a methyl group, Q represents a group represented by Q1-183, and Het represents any substituents indicated in [Table 1A] to [Table 20A] (hereinafter, referred to as Compound Class SX183).

A compound (L-1) wherein n is 0, $R^1$ represents a methyl group, Q represents a group represented by Q1-184, and Het represents any substituents indicated in [Table 1A] to [Table 20A] (hereinafter, referred to as Compound Class SX184).

A compound (L-1) wherein n is 0, $R^1$ represents a methyl group, Q represents a group represented by Q1-185, and Het represents any substituents indicated in [Table 1A] to [Table 20A] (hereinafter, referred to as Compound Class SX185).

A compound (L-1) wherein n is 0, $R^1$ represents a methyl group, Q represents a group represented by Q1-186, and Het represents any substituents indicated in [Table 1A] to [Table 20A] (hereinafter, referred to as Compound Class SX186).

A compound (L-1) wherein n is 0, $R^1$ represents a methyl group, Q represents a group represented by Q1-187, and Het represents any substituents indicated in [Table 1A] to [Table 20A] (hereinafter, referred to as Compound Class SX187).

A compound (L-1) wherein n is 0, $R^1$ represents a methyl group, Q represents a group represented by Q1-188, and Het represents any substituents indicated in [Table 1A] to [Table 20A] (hereinafter, referred to as Compound Class SX188).

A compound (L-1) wherein n is 0, $R^1$ represents a methyl group, Q represents a group represented by Q1-189, and Het represents any substituents indicated in [Table 1A] to [Table 20A] (hereinafter, referred to as Compound Class SX189).

A compound (L-1) wherein n is 0, $R^1$ represents a methyl group, Q represents a group represented by Q1-190, and Het represents any substituents indicated in [Table 1A] to [Table 20A] (hereinafter, referred to as Compound Class SX190).

A compound (L-1) wherein n is 0, $R^1$ represents a methyl group, Q represents a group represented by Q1-191, and Het represents any substituents indicated in [Table 1A] to [Table 20A] (hereinafter, referred to as Compound Class SX191).

A compound (L-1) wherein n is 0, $R^1$ represents a methyl group, Q represents a group represented by Q1-192, and Het represents any substituents indicated in [Table 1A] to [Table 20A] (hereinafter, referred to as Compound Class SX192).

A compound (L-1) wherein n is 0, $R^1$ represents a methyl group, Q represents a group represented by Q1-193, and Het represents any substituents indicated in [Table 1A] to [Table 20A] (hereinafter, referred to as Compound Class SX193).

A compound (L-1) wherein n is 0, $R^1$ represents a methyl group, Q represents a group represented by Q1-194, and Het represents any substituents indicated in [Table 1A] to [Table 20A] (hereinafter, referred to as Compound Class SX194).

A compound (L-1) wherein n is 0, $R^1$ represents a methyl group, Q represents a group represented by Q1-195,

and Het represents any substituents indicated in [Table 1A] to [Table 20A] (hereinafter, referred to as Compound Class SX195).

A compound (L-1) wherein n is 0, $R^1$ represents a methyl group, Q represents a group represented by Q1-196, and Het represents any substituents indicated in [Table 1A] to [Table 20A] (hereinafter, referred to as Compound Class SX196).

A compound (L-1) wherein n is 0, $R^1$ represents a methyl group, Q represents a group represented by Q1-197, and Het represents any substituents indicated in [Table 1A] to [Table 20A] (hereinafter, referred to as Compound Class SX197).

A compound (L-1) wherein n is 0, $R^1$ represents a methyl group, Q represents a group represented by Q1-198, and Het represents any substituents indicated in [Table 1A] to [Table 20A] (hereinafter, referred to as Compound Class SX198).

A compound (L-1) wherein n is 0, $R^1$ represents a methyl group, Q represents a group represented by Q1-199, and Het represents any substituents indicated in [Table 1A] to [Table 20A] (hereinafter, referred to as Compound Class SX199).

A compound (L-1) wherein n is 0, $R^1$ represents a methyl group, Q represents a group represented by Q1-200, and Het represents any substituents indicated in [Table 1A] to [Table 20A] (hereinafter, referred to as Compound Class SX200).

A compound (L-1) wherein n is 0, $R^1$ represents a methyl group, Q represents a group represented by Q1-201, and Het represents any substituents indicated in [Table 1A] to [Table 20A] (hereinafter, referred to as Compound Class SX201).

A compound (L-1) wherein n is 0, $R^1$ represents a methyl group, Q represents a group represented by Q1-202, and Het represents any substituents indicated in [Table 1A] to [Table 20A] (hereinafter, referred to as Compound Class SX202).

A compound (L-1) wherein n is 0, $R^1$ represents a methyl group, Q represents a group represented by Q1-203, and Het represents any substituents indicated in [Table 1A] to [Table 20A] (hereinafter, referred to as Compound Class SX203).

A compound (L-1) wherein n is 0, $R^1$ represents a methyl group, Q represents a group represented by Q1-204, and Het represents any substituents indicated in [Table 1A] to [Table 20A] (hereinafter, referred to as Compound Class SX204).

A compound (L-1) wherein n is 0, $R^1$ represents a methyl group, Q represents a group represented by Q1-205, and Het represents any substituents indicated in [Table 1A] to [Table 20A] (hereinafter, referred to as Compound Class SX205).

A compound (L-1) wherein n is 0, $R^1$ represents a methyl group, Q represents a group represented by Q1-206, and Het represents any substituents indicated in [Table 1A] to [Table 20A] (hereinafter, referred to as Compound Class SX206).

A compound (L-1) wherein n is 0, $R^1$ represents a methyl group, Q represents a group represented by Q1-207, and Het represents any substituents indicated in [Table 1A] to [Table 20A] (hereinafter, referred to as Compound Class SX207).

A compound (L-1) wherein n is 0, $R^1$ represents a methyl group, Q represents a group represented by Q1-208, and Het represents any substituents indicated in [Table 1A] to [Table 20A] (hereinafter, referred to as Compound Class SX208).

A compound (L-1) wherein n is 0, $R^1$ represents a methyl group, Q represents a group represented by Q1-209, and Het represents any substituents indicated in [Table 1A] to [Table 20A] (hereinafter, referred to as Compound Class SX209).

A compound (L-1) wherein n is 0, $R^1$ represents a methyl group, Q represents a group represented by Q5-1, and Het represents any substituents indicated in [Table 1A] to [Table 20A] (hereinafter, referred to as Compound Class SX210).

A compound (L-1) wherein n is 0, $R^1$ represents a methyl group, Q represents a group represented by Q5-2, and Het represents any substituents indicated in [Table 1A] to [Table 20A] (hereinafter, referred to as Compound Class SX211).

A compound (L-1) wherein n is 0, $R^1$ represents a methyl group, Q represents a group represented by Q5-3, and Het represents any substituents indicated in [Table 1A] to [Table 20A] (hereinafter, referred to as Compound Class SX212).

A compound (L-1) wherein n is 0, $R^1$ represents a methyl group, Q represents a group represented by Q5-4, and Het represents any substituents indicated in [Table 1A] to [Table 20A] (hereinafter, referred to as Compound Class SX213).

A compound (L-1) wherein n is 0, $R^1$ represents a methyl group, Q represents a group represented by Q5-5, and Het represents any substituents indicated in [Table 1A] to [Table 20A] (hereinafter, referred to as Compound Class SX214).

A compound (L-1) wherein n is 0, $R^1$ represents a methyl group, Q represents a group represented by Q5-6, and Het represents any substituents indicated in [Table 1A] to [Table 20A] (hereinafter, referred to as Compound Class SX215).

A compound (L-1) wherein n is 0, $R^1$ represents a methyl group, Q represents a group represented by Q5-7, and Het represents any substituents indicated in [Table 1A] to [Table 20A] (hereinafter, referred to as Compound Class SX216).

A compound (L-1) wherein n is 0, $R^1$ represents a methyl group, Q represents a group represented by Q5-7, and Het represents any substituents indicated in [Table 1A] to [Table 20A] (hereinafter, referred to as Compound Class SX216).

A compound (L-1) wherein n is 0, $R^1$ represents a methyl group, Q represents a group represented by Q5-7, and Het represents any substituents indicated in [Table 1A] to [Table 20A] (hereinafter, referred to as Compound Class SX216).

A compound (L-1) wherein n is 0, $R^1$ represents a methyl group, Q represents a group represented by Q5-8, and Het represents any substituents indicated in [Table 1A] to [Table 20A] (hereinafter, referred to as Compound Class SX216).

A compound (L-1) wherein n is 0, $R^1$ represents a methyl group, Q represents a group represented by Q5-9, and Het represents any substituents indicated in [Table 1A] to [Table 20A] (hereinafter, referred to as Compound Class SX217).

A compound (L-1) wherein n is 0, $R^1$ represents a methyl group, Q represents a group represented by Q5-10, and Het represents any substituents indicated in [Table 1A] to [Table 20A] (hereinafter, referred to as Compound Class SX218).

A compound (L-1) wherein n is 0, $R^1$ represents a methyl group, Q represents a group represented by Q5-11, and Het represents any substituents indicated in [Table 1A] to [Table 20A] (hereinafter, referred to as Compound Class SX219).

A compound (L-1) wherein n is 0, $R^1$ represents a methyl group, Q represents a group represented by Q5-11, and Het represents any substituents indicated in [Table 1A] to [Table 20A] (hereinafter, referred to as Compound Class SX220).

A compound (L-1) wherein n is 0, $R^1$ represents a methyl group, Q represents a group represented by Q5-12, and Het represents any substituents indicated in [Table 1A] to [Table 20A] (hereinafter, referred to as Compound Class SX221).

A compound (L-1) wherein n is 0, $R^1$ represents a methyl group, Q represents a group represented by Q5-13, and Het represents any substituents indicated in [Table 1A] to [Table 20A] (hereinafter, referred to as Compound Class SX222).

A compound (L-1) wherein n is 0, $R^1$ represents a methyl group, Q represents a group represented by Q5-14, and Het represents any substituents indicated in [Table 1A] to [Table 20A] (hereinafter, referred to as Compound Class SX223).

A compound (L-1) wherein n is 0, $R^1$ represents a methyl group, Q represents a group represented by Q5-15, and Het represents any substituents indicated in [Table 1A] to [Table 20A] (hereinafter, referred to as Compound Class SX224).

A compound (L-1) wherein n is 0, $R^1$ represents a methyl group, Q represents a group represented by Q5-16, and Het represents any substituents indicated in [Table 1A] to [Table 20A] (hereinafter, referred to as Compound Class SX225).

A compound (L-1) wherein n is 0, $R^1$ represents a methyl group, Q represents a group represented by Q5-17, and Het represents any substituents indicated in [Table 1A] to [Table 20A] (hereinafter, referred to as Compound Class SX226).

A compound (L-1) wherein n is 0, $R^1$ represents a methyl group, Q represents a group represented by Q5-18, and Het represents any substituents indicated in [Table 1A] to [Table 20A] (hereinafter, referred to as Compound Class SX227).

A compound (L-1) wherein n is 0, $R^1$ represents a methyl group, Q represents a group represented by Q5-19, and Het represents any substituents indicated in [Table 1A] to [Table 20A] (hereinafter, referred to as Compound Class SX228).

A compound (L-1) wherein n is 0, $R^1$ represents a methyl group, Q represents a group represented by Q5-20, and Het represents any substituents indicated in [Table 1A] to [Table 20A] (hereinafter, referred to as Compound Class SX229).

A compound (L-1) wherein n is 0, $R^1$ represents a methyl group, Q represents a group represented by Q5-21, and Het represents any substituents indicated in [Table 1A] to [Table 20A] (hereinafter, referred to as Compound Class SX230).

A compound (L-1) wherein n is 0, $R^1$ represents a methyl group, Q represents a group represented by Q5-22, and Het represents any substituents indicated in [Table 1A] to [Table 20A] (hereinafter, referred to as Compound Class SX231).

A compound (L-1) wherein n is 2, $R^1$ represents a methyl group, Q represents a group represented by Q1-1, and Het represents any substituents indicated in [Table 1A] to [Table 20A] (hereinafter, referred to as Compound Class SX232).

A compound (L-1) wherein n is 2, $R^1$ represents a methyl group, Q represents a group represented by Q1-2, and Het represents any substituents indicated in [Table 1A] to [Table 20A] (hereinafter, referred to as Compound Class SX233).

A compound (L-1) wherein n is 2, $R^1$ represents a methyl group, Q represents a group represented by Q1-3, and Het represents any substituents indicated in [Table 1A] to [Table 20A] (hereinafter, referred to as Compound Class SX234).

A compound (L-1) wherein n is 2, $R^1$ represents a methyl group, Q represents a group represented by Q1-4, and Het represents any substituents indicated in [Table 1A] to [Table 20A] (hereinafter, referred to as Compound Class SX235).

A compound (L-1) wherein n is 2, $R^1$ represents a methyl group, Q represents a group represented by Q1-5, and Het represents any substituents indicated in [Table 1A] to [Table 20A] (hereinafter, referred to as Compound Class SX236).

A compound (L-1) wherein n is 2, $R^1$ represents a methyl group, Q represents a group represented by Q1-6, and Het represents any substituents indicated in [Table 1A] to [Table 20A] (hereinafter, referred to as Compound Class SX237).

A compound (L-1) wherein n is 2, $R^1$ represents a methyl group, Q represents a group represented by Q1-7, and Het represents any substituents indicated in [Table 1A] to [Table 20A] (hereinafter, referred to as Compound Class SX238).

A compound (L-1) wherein n is 2, $R^1$ represents a methyl group, Q represents a group represented by Q1-8, and Het represents any substituents indicated in [Table 1A] to [Table 20A] (hereinafter, referred to as Compound Class SX239).

A compound (L-1) wherein n is 2, $R^1$ represents a methyl group, Q represents a group represented by Q1-9, and Het represents any substituents indicated in [Table 1A] to [Table 20A] (hereinafter, referred to as Compound Class SX240).

A compound (L-1) wherein n is 2, $R^1$ represents a methyl group, Q represents a group represented by Q1-10, and Het represents any substituents indicated in [Table 1A] to [Table 20A] (hereinafter, referred to as Compound Class SX241).

A compound (L-1) wherein n is 2, $R^1$ represents a methyl group, Q represents a group represented by Q1-11, and Het represents any substituents indicated in [Table 1A] to [Table 20A] (hereinafter, referred to as Compound Class SX242).

A compound (L-1) wherein n is 2, $R^1$ represents a methyl group, Q represents a group represented by Q1-12, and Het represents any substituents indicated in [Table 1A] to [Table 20A] (hereinafter, referred to as Compound Class SX243).
A compound (L-1) wherein n is 2, $R^1$ represents a methyl group, Q represents a group represented by Q1-13, and Het represents any substituents indicated in [Table 1A] to [Table 20A] (hereinafter, referred to as Compound Class SX244).
A compound (L-1) wherein n is 2, $R^1$ represents a methyl group, Q represents a group represented by Q1-14, and Het represents any substituents indicated in [Table 1A] to [Table 20A] (hereinafter, referred to as Compound Class SX245).
A compound (L-1) wherein n is 2, $R^1$ represents a methyl group, Q represents a group represented by Q1-15, and Het represents any substituents indicated in [Table 1A] to [Table 20A] (hereinafter, referred to as Compound Class SX246).
A compound (L-1) wherein n is 2, $R^1$ represents a methyl group, Q represents a group represented by Q1-16, and Het represents any substituents indicated in [Table 1A] to [Table 20A] (hereinafter, referred to as Compound Class SX247).
A compound (L-1) wherein n is 2, $R^1$ represents a methyl group, Q represents a group represented by Q1-17, and Het represents any substituents indicated in [Table 1A] to [Table 20A] (hereinafter, referred to as Compound Class SX248).
A compound (L-1) wherein n is 2, $R^1$ represents a methyl group, Q represents a group represented by Q1-18, and Het represents any substituents indicated in [Table 1A] to [Table 20A] (hereinafter, referred to as Compound Class SX249).
A compound (L-1) wherein n is 2, $R^1$ represents a methyl group, Q represents a group represented by Q1-19, and Het represents any substituents indicated in [Table 1A] to [Table 20A] (hereinafter, referred to as Compound Class SX250).
A compound (L-1) wherein n is 2, $R^1$ represents a methyl group, Q represents a group represented by Q1-20, and Het represents any substituents indicated in [Table 1A] to [Table 20A] (hereinafter, referred to as Compound Class SX251).
A compound (L-1) wherein n is 2, $R^1$ represents a methyl group, Q represents a group represented by Q1-21, and Het represents any substituents indicated in [Table 1A] to [Table 20A] (hereinafter, referred to as Compound Class SX252).
A compound (L-1) wherein n is 2, $R^1$ represents a methyl group, Q represents a group represented by Q1-22, and Het represents any substituents indicated in [Table 1A] to [Table 20A] (hereinafter, referred to as Compound Class SX253).
A compound (L-1) wherein n is 2, $R^1$ represents a methyl group, Q represents a group represented by Q1-23, and Het represents any substituents indicated in [Table 1A] to [Table 20A] (hereinafter, referred to as Compound Class SX254).
A compound (L-1) wherein n is 2, $R^1$ represents a methyl group, Q represents a group represented by Q1-24, and Het represents any substituents indicated in [Table 1A] to [Table 20A] (hereinafter, referred to as Compound Class SX255).
A compound (L-1) wherein n is 2, $R^1$ represents a methyl group, Q represents a group represented by Q1-25, and Het represents any substituents indicated in [Table 1A] to [Table 20A] (hereinafter, referred to as Compound Class SX256).
A compound (L-1) wherein n is 2, $R^1$ represents a methyl group, Q represents a group represented by Q1-26, and Het represents any substituents indicated in [Table 1A] to [Table 20A] (hereinafter, referred to as Compound Class SX257).
A compound (L-1) wherein n is 2, $R^1$ represents a methyl group, Q represents a group represented by Q1-27, and Het represents any substituents indicated in [Table 1A] to [Table 20A] (hereinafter, referred to as Compound Class SX258).
A compound (L-1) wherein n is 2, $R^1$ represents a methyl group, Q represents a group represented by Q1-28, and Het represents any substituents indicated in [Table 1A] to [Table 20A] (hereinafter, referred to as Compound Class SX259).
A compound (L-1) wherein n is 2, $R^1$ represents a methyl group, Q represents a group represented by Q1-29, and Het represents any substituents indicated in [Table 1A] to [Table 20A] (hereinafter, referred to as Compound Class SX260).
A compound (L-1) wherein n is 2, $R^1$ represents a methyl group, Q represents a group represented by Q1-30, and Het represents any substituents indicated in [Table 1A] to [Table 20A] (hereinafter, referred to as Compound Class SX261).
A compound (L-1) wherein n is 2, $R^1$ represents a methyl group, Q represents a group represented by Q1-31, and Het represents any substituents indicated in [Table 1A] to [Table 20A] (hereinafter, referred to as Compound Class SX262).
A compound (L-1) wherein n is 2, $R^1$ represents a methyl group, Q represents a group represented by Q1-32, and Het represents any substituents indicated in [Table 1A] to [Table 20A] (hereinafter, referred to as Compound Class SX263).
A compound (L-1) wherein n is 2, $R^1$ represents a methyl group, Q represents a group represented by Q1-33, and Het represents any substituents indicated in [Table 1A] to [Table 20A] (hereinafter, referred to as Compound Class SX264).
A compound (L-1) wherein n is 2, $R^1$ represents a methyl group, Q represents a group represented by Q1-34, and Het represents any substituents indicated in [Table 1A] to [Table 20A] (hereinafter, referred to as Compound Class SX265).
A compound (L-1) wherein n is 2, $R^1$ represents a methyl group, Q represents a group represented by Q1-35, and Het represents any substituents indicated in [Table 1A] to [Table 20A] (hereinafter, referred to as Compound Class SX266).
A compound (L-1) wherein n is 2, $R^1$ represents a methyl group, Q represents a group represented by Q1-36, and Het represents any substituents indicated in [Table 1A] to [Table 20A] (hereinafter, referred to as Compound Class SX267).
A compound (L-1) wherein n is 2, $R^1$ represents a methyl group, Q represents a group represented by Q1-37, and Het represents any substituents indicated in [Table 1A] to [Table 20A] (hereinafter, referred to as Compound Class SX268).
A compound (L-1) wherein n is 2, $R^1$ represents a methyl group, Q represents a group represented by Q1-38, and Het represents any substituents indicated in [Table 1A] to [Table 20A] (hereinafter, referred to as Compound Class SX269).
A compound (L-1) wherein n is 2, $R^1$ represents a methyl group, Q represents a group represented by Q1-39, and Het represents any substituents indicated in [Table 1A] to [Table 20A] (hereinafter, referred to as Compound Class SX270).
A compound (L-1) wherein n is 2, $R^1$ represents a methyl group, Q represents a group represented by Q1-40, and Het represents any substituents indicated in [Table 1A] to [Table 20A] (hereinafter, referred to as Compound Class SX271).

A compound (L-1) wherein n is 2, $R^1$ represents a methyl group, Q represents a group represented by Q1-41, and Het represents any substituents indicated in [Table 1A] to [Table 20A] (hereinafter, referred to as Compound Class SX272).
A compound (L-1) wherein n is 2, $R^1$ represents a methyl group, Q represents a group represented by Q1-42, and Het represents any substituents indicated in [Table 1A] to [Table 20A] (hereinafter, referred to as Compound Class SX273).
A compound (L-1) wherein n is 2, $R^1$ represents a methyl group, Q represents a group represented by Q1-43, and Het represents any substituents indicated in [Table 1A] to [Table 20A] (hereinafter, referred to as Compound Class SX274).
A compound (L-1) wherein n is 2, $R^1$ represents a methyl group, Q represents a group represented by Q1-44, and Het represents any substituents indicated in [Table 1A] to [Table 20A] (hereinafter, referred to as Compound Class SX275).
A compound (L-1) wherein n is 2, $R^1$ represents a methyl group, Q represents a group represented by Q1-45, and Het represents any substituents indicated in [Table 1A] to [Table 20A] (hereinafter, referred to as Compound Class SX276).
A compound (L-1) wherein n is 2, $R^1$ represents a methyl group, Q represents a group represented by Q1-46, and Het represents any substituents indicated in [Table 1A] to [Table 20A] (hereinafter, referred to as Compound Class SX277).
A compound (L-1) wherein n is 2, $R^1$ represents a methyl group, Q represents a group represented by Q1-47, and Het represents any substituents indicated in [Table 1A] to [Table 20A] (hereinafter, referred to as Compound Class SX278).
A compound (L-1) wherein n is 2, $R^1$ represents a methyl group, Q represents a group represented by Q1-48, and Het represents any substituents indicated in [Table 1A] to [Table 20A] (hereinafter, referred to as Compound Class SX279).
A compound (L-1) wherein n is 2, $R^1$ represents a methyl group, Q represents a group represented by Q1-49, and Het represents any substituents indicated in [Table 1A] to [Table 20A] (hereinafter, referred to as Compound Class SX280).
A compound (L-1) wherein n is 2, $R^1$ represents a methyl group, Q represents a group represented by Q1-50, and Het represents any substituents indicated in [Table 1A] to [Table 20A] (hereinafter, referred to as Compound Class SX281).
A compound (L-1) wherein n is 2, $R^1$ represents a methyl group, Q represents a group represented by Q1-51, and Het represents any substituents indicated in [Table 1A] to [Table 20A] (hereinafter, referred to as Compound Class SX282).
A compound (L-1) wherein n is 2, $R^1$ represents a methyl group, Q represents a group represented by Q1-52, and Het represents any substituents indicated in [Table 1A] to [Table 20A] (hereinafter, referred to as Compound Class SX283).
A compound (L-1) wherein n is 2, $R^1$ represents a methyl group, Q represents a group represented by Q1-53, and Het represents any substituents indicated in [Table 1A] to [Table 20A] (hereinafter, referred to as Compound Class SX284).
A compound (L-1) wherein n is 2, $R^1$ represents a methyl group, Q represents a group represented by Q1-54, and Het represents any substituents indicated in [Table 1A] to [Table 20A] (hereinafter, referred to as Compound Class SX285).
A compound (L-1) wherein n is 2, $R^1$ represents a methyl group, Q represents a group represented by Q1-55, and Het represents any substituents indicated in [Table 1A] to [Table 20A] (hereinafter, referred to as Compound Class SX286).
A compound (L-1) wherein n is 2, $R^1$ represents a methyl group, Q represents a group represented by Q1-56, and Het represents any substituents indicated in [Table 1A] to [Table 20A] (hereinafter, referred to as Compound Class SX287).
A compound (L-1) wherein n is 2, $R^1$ represents a methyl group, Q represents a group represented by Q1-57, and Het represents any substituents indicated in [Table 1A] to [Table 20A] (hereinafter, referred to as Compound Class SX288).
A compound (L-1) wherein n is 2, $R^1$ represents a methyl group, Q represents a group represented by Q1-58, and Het represents any substituents indicated in [Table 1A] to [Table 20A] (hereinafter, referred to as Compound Class SX289).
A compound (L-1) wherein n is 2, $R^1$ represents a methyl group, Q represents a group represented by Q1-58, and Het represents any substituents indicated in [Table 1A] to [Table 20A] (hereinafter, referred to as Compound Class SX289).
A compound (L-1) wherein n is 2, $R^1$ represents a methyl group, Q represents a group represented by Q1-59, and Het represents any substituents indicated in [Table 1A] to [Table 20A] (hereinafter, referred to as Compound Class SX290).
A compound (L-1) wherein n is 2, $R^1$ represents a methyl group, Q represents a group represented by Q1-60, and Het represents any substituents indicated in [Table 1A] to [Table 20A] (hereinafter, referred to as Compound Class SX291).
A compound (L-1) wherein n is 2, $R^1$ represents a methyl group, Q represents a group represented by Q1-61, and Het represents any substituents indicated in [Table 1A] to [Table 20A] (hereinafter, referred to as Compound Class SX292).
A compound (L-1) wherein n is 2, $R^1$ represents a methyl group, Q represents a group represented by Q1-62, and Het represents any substituents indicated in [Table 1A] to [Table 20A] (hereinafter, referred to as Compound Class SX293).
A compound (L-1) wherein n is 2, $R^1$ represents a methyl group, Q represents a group represented by Q1-63, and Het represents any substituents indicated in [Table 1A] to [Table 20A] (hereinafter, referred to as Compound Class SX294).
A compound (L-1) wherein n is 2, $R^1$ represents a methyl group, Q represents a group represented by Q1-64, and Het represents any substituents indicated in [Table 1A] to [Table 20A] (hereinafter, referred to as Compound Class SX295).
A compound (L-1) wherein n is 2, $R^1$ represents a methyl group, Q represents a group represented by Q1-65, and Het represents any substituents indicated in [Table 1A] to [Table 20A] (hereinafter, referred to as Compound Class SX296).
A compound (L-1) wherein n is 2, $R^1$ represents a methyl group, Q represents a group represented by Q1-66, and Het represents any substituents indicated in [Table 1A] to [Table 20A] (hereinafter, referred to as Compound Class SX297).
A compound (L-1) wherein n is 2, $R^1$ represents a methyl group, Q represents a group represented by Q1-67, and Het represents any substituents indicated in [Table 1A] to [Table 20A] (hereinafter, referred to as Compound Class SX298).
A compound (L-1) wherein n is 2, $R^1$ represents a methyl group, Q represents a group represented by Q1-68, and Het represents any substituents indicated in [Table 1A] to [Table 20A] (hereinafter, referred to as Compound Class SX299).

A compound (L-1) wherein n is 2, $R^1$ represents a methyl group, Q represents a group represented by Q1-69, and Het represents any substituents indicated in [Table 1A] to [Table 20A] (hereinafter, referred to as Compound Class SX300).
A compound (L-1) wherein n is 2, $R^1$ represents a methyl group, Q represents a group represented by Q1-70, and Het represents any substituents indicated in [Table 1A] to [Table 20A] (hereinafter, referred to as Compound Class SX301).
A compound (L-1) wherein n is 2, $R^1$ represents a methyl group, Q represents a group represented by Q1-71, and Het represents any substituents indicated in [Table 1A] to [Table 20A] (hereinafter, referred to as Compound Class SX302).
A compound (L-1) wherein n is 2, $R^1$ represents a methyl group, Q represents a group represented by Q1-72, and Het represents any substituents indicated in [Table 1A] to [Table 20A] (hereinafter, referred to as Compound Class SX303).
A compound (L-1) wherein n is 2, $R^1$ represents a methyl group, Q represents a group represented by Q1-73, and Het represents any substituents indicated in [Table 1A] to [Table 20A] (hereinafter, referred to as Compound Class SX304).
A compound (L-1) wherein n is 2, $R^1$ represents a methyl group, Q represents a group represented by Q1-74, and Het represents any substituents indicated in [Table 1A] to [Table 20A] (hereinafter, referred to as Compound Class SX305).
A compound (L-1) wherein n is 2, $R^1$ represents a methyl group, Q represents a group represented by Q1-75, and Het represents any substituents indicated in [Table 1A] to [Table 20A] (hereinafter, referred to as Compound Class SX306).
A compound (L-1) wherein n is 2, $R^1$ represents a methyl group, Q represents a group represented by Q1-76, and Het represents any substituents indicated in [Table 1A] to [Table 20A] (hereinafter, referred to as Compound Class SX307).
A compound (L-1) wherein n is 2, $R^1$ represents a methyl group, Q represents a group represented by Q1-77, and Het represents any substituents indicated in [Table 1A] to [Table 20A] (hereinafter, referred to as Compound Class SX308).
A compound (L-1) wherein n is 2, $R^1$ represents a methyl group, Q represents a group represented by Q1-78, and Het represents any substituents indicated in [Table 1A] to [Table 20A] (hereinafter, referred to as Compound Class SX309).
A compound (L-1) wherein n is 2, $R^1$ represents a methyl group, Q represents a group represented by Q1-79, and Het represents any substituents indicated in [Table 1A] to [Table 20A] (hereinafter, referred to as Compound Class SX310).
A compound (L-1) wherein n is 2, $R^1$ represents a methyl group, Q represents a group represented by Q1-80, and Het represents any substituents indicated in [Table 1A] to [Table 20A] (hereinafter, referred to as Compound Class SX311).
A compound (L-1) wherein n is 2, $R^1$ represents a methyl group, Q represents a group represented by Q1-81, and Het represents any substituents indicated in [Table 1A] to [Table 20A] (hereinafter, referred to as Compound Class SX312).
A compound (L-1) wherein n is 2, $R^1$ represents a methyl group, Q represents a group represented by Q1-82, and Het represents any substituents indicated in [Table 1A] to [Table 20A] (hereinafter, referred to as Compound Class SX313).
A compound (L-1) wherein n is 2, $R^1$ represents a methyl group, Q represents a group represented by Q1-83, and Het represents any substituents indicated in [Table 1A] to [Table 20A] (hereinafter, referred to as Compound Class SX314).
A compound (L-1) wherein n is 2, $R^1$ represents a methyl group, Q represents a group represented by Q1-84, and Het represents any substituents indicated in [Table 1A] to [Table 20A] (hereinafter, referred to as Compound Class SX315).
A compound (L-1) wherein n is 2, $R^1$ represents a methyl group, Q represents a group represented by Q1-85, and Het represents any substituents indicated in [Table 1A] to [Table 20A] (hereinafter, referred to as Compound Class SX316).
A compound (L-1) wherein n is 2, $R^1$ represents a methyl group, Q represents a group represented by Q1-86, and Het represents any substituents indicated in [Table 1A] to [Table 20A] (hereinafter, referred to as Compound Class SX317).
A compound (L-1) wherein n is 2, $R^1$ represents a methyl group, Q represents a group represented by Q1-87, and Het represents any substituents indicated in [Table 1A] to [Table 20A] (hereinafter, referred to as Compound Class SX318).
A compound (L-1) wherein n is 2, $R^1$ represents a methyl group, Q represents a group represented by Q1-88, and Het represents any substituents indicated in [Table 1A] to [Table 20A] (hereinafter, referred to as Compound Class SX319).
A compound (L-1) wherein n is 2, $R^1$ represents a methyl group, Q represents a group represented by Q1-89, and Het represents any substituents indicated in [Table 1A] to [Table 20A] (hereinafter, referred to as Compound Class SX320).
A compound (L-1) wherein n is 2, $R^1$ represents a methyl group, Q represents a group represented by Q1-90, and Het represents any substituents indicated in [Table 1A] to [Table 20A] (hereinafter, referred to as Compound Class SX321).
A compound (L-1) wherein n is 2, $R^1$ represents a methyl group, Q represents a group represented by Q1-91, and Het represents any substituents indicated in [Table 1A] to [Table 20A] (hereinafter, referred to as Compound Class SX322).
A compound (L-1) wherein n is 2, $R^1$ represents a methyl group, Q represents a group represented by Q1-92, and Het represents any substituents indicated in [Table 1A] to [Table 20A] (hereinafter, referred to as Compound Class SX323).
A compound (L-1) wherein n is 2, $R^1$ represents a methyl group, Q represents a group represented by Q1-93, and Het represents any substituents indicated in [Table 1A] to [Table 20A] (hereinafter, referred to as Compound Class SX324).
A compound (L-1) wherein n is 2, $R^1$ represents a methyl group, Q represents a group represented by Q1-94, and Het represents any substituents indicated in [Table 1A] to [Table 20A] (hereinafter, referred to as Compound Class SX325).
A compound (L-1) wherein n is 2, $R^1$ represents a methyl group, Q represents a group represented by Q1-95, and Het represents any substituents indicated in [Table 1A] to [Table 20A] (hereinafter, referred to as Compound Class SX326).
A compound (L-1) wherein n is 2, $R^1$ represents a methyl group, Q represents a group represented by Q1-96, and Het represents any substituents indicated in [Table 1A] to [Table 20A] (hereinafter, referred to as Compound Class SX327).
A compound (L-1) wherein n is 2, $R^1$ represents a methyl group, Q represents a group represented by Q1-97, and Het represents any substituents indicated in [Table 1A] to [Table 20A] (hereinafter, referred to as Compound Class SX328).

A compound (L-1) wherein n is 2, $R^1$ represents a methyl group, Q represents a group represented by Q1-98, and Het represents any substituents indicated in [Table 1A] to [Table 20A] (hereinafter, referred to as Compound Class SX329).

A compound (L-1) wherein n is 2, $R^1$ represents a methyl group, Q represents a group represented by Q1-99, and Het represents any substituents indicated in [Table 1A] to [Table 20A] (hereinafter, referred to as Compound Class SX330).

A compound (L-1) wherein n is 2, $R^1$ represents a methyl group, Q represents a group represented by Q1-100, and Het represents any substituents indicated in [Table 1A] to [Table 20A] (hereinafter, referred to as Compound Class SX331).

A compound (L-1) wherein n is 2, $R^1$ represents a methyl group, Q represents a group represented by Q1-101, and Het represents any substituents indicated in [Table 1A] to [Table 20A] (hereinafter, referred to as Compound Class SX332).

A compound (L-1) wherein n is 2, $R^1$ represents a methyl group, Q represents a group represented by Q1-102, and Het represents any substituents indicated in [Table 1A] to [Table 20A] (hereinafter, referred to as Compound Class SX333).

A compound (L-1) wherein n is 2, $R^1$ represents a methyl group, Q represents a group represented by Q1-103, and Het represents any substituents indicated in [Table 1A] to [Table 20A] (hereinafter, referred to as Compound Class SX334).

A compound (L-1) wherein n is 2, $R^1$ represents a methyl group, Q represents a group represented by Q1-104, and Het represents any substituents indicated in [Table 1A] to [Table 20A] (hereinafter, referred to as Compound Class SX335).

A compound (L-1) wherein n is 2, $R^1$ represents a methyl group, Q represents a group represented by Q1-105, and Het represents any substituents indicated in [Table 1A] to [Table 20A] (hereinafter, referred to as Compound Class SX336).

A compound (L-1) wherein n is 2, $R^1$ represents a methyl group, Q represents a group represented by Q1-106, and Het represents any substituents indicated in [Table 1A] to [Table 20A] (hereinafter, referred to as Compound Class SX337).

A compound (L-1) wherein n is 2, $R^1$ represents a methyl group, Q represents a group represented by Q1-107, and Het represents any substituents indicated in [Table 1A] to [Table 20A] (hereinafter, referred to as Compound Class SX338).

A compound (L-1) wherein n is 2, $R^1$ represents a methyl group, Q represents a group represented by Q1-108, and Het represents any substituents indicated in [Table 1A] to [Table 20A] (hereinafter, referred to as Compound Class SX339).

A compound (L-1) wherein n is 2, $R^1$ represents a methyl group, Q represents a group represented by Q1-109, and Het represents any substituents indicated in [Table 1A] to [Table 20A] (hereinafter, referred to as Compound Class SX340).

A compound (L-1) wherein n is 2, $R^1$ represents a methyl group, Q represents a group represented by Q1-110, and Het represents any substituents indicated in [Table 1A] to [Table 20A] (hereinafter, referred to as Compound Class SX341).

A compound (L-1) wherein n is 2, $R^1$ represents a methyl group, Q represents a group represented by Q1-111, and Het represents any substituents indicated in [Table 1A] to [Table 20A] (hereinafter, referred to as Compound Class SX342).

A compound (L-1) wherein n is 2, $R^1$ represents a methyl group, Q represents a group represented by Q1-112, and Het represents any substituents indicated in [Table 1A] to [Table 20A] (hereinafter, referred to as Compound Class SX343).

A compound (L-1) wherein n is 2, $R^1$ represents a methyl group, Q represents a group represented by Q1-113, and Het represents any substituents indicated in [Table 1A] to [Table 20A] (hereinafter, referred to as Compound Class SX344).

A compound (L-1) wherein n is 2, $R^1$ represents a methyl group, Q represents a group represented by Q1-114, and Het represents any substituents indicated in [Table 1A] to [Table 20A] (hereinafter, referred to as Compound Class SX345).

A compound (L-1) wherein n is 2, $R^1$ represents a methyl group, Q represents a group represented by Q1-115, and Het represents any substituents indicated in [Table 1A] to [Table 20A] (hereinafter, referred to as Compound Class SX346).

A compound (L-1) wherein n is 2, $R^1$ represents a methyl group, Q represents a group represented by Q1-116, and Het represents any substituents indicated in [Table 1A] to [Table 20A] (hereinafter, referred to as Compound Class SX347).

A compound (L-1) wherein n is 2, $R^1$ represents a methyl group, Q represents a group represented by Q1-117, and Het represents any substituents indicated in [Table 1A] to [Table 20A] (hereinafter, referred to as Compound Class SX348).

A compound (L-1) wherein n is 2, $R^1$ represents a methyl group, Q represents a group represented by Q1-118, and Het represents any substituents indicated in [Table 1A] to [Table 20A] (hereinafter, referred to as Compound Class SX349).

A compound (L-1) wherein n is 2, $R^1$ represents a methyl group, Q represents a group represented by Q1-119, and Het represents any substituents indicated in [Table 1A] to [Table 20A] (hereinafter, referred to as Compound Class SX350).

A compound (L-1) wherein n is 2, $R^1$ represents a methyl group, Q represents a group represented by Q1-120, and Het represents any substituents indicated in [Table 1A] to [Table 20A] (hereinafter, referred to as Compound Class SX351).

A compound (L-1) wherein n is 2, $R^1$ represents a methyl group, Q represents a group represented by Q1-121, and Het represents any substituents indicated in [Table 1A] to [Table 20A] (hereinafter, referred to as Compound Class SX352).

A compound (L-1) wherein n is 2, $R^1$ represents a methyl group, Q represents a group represented by Q1-122, and Het represents any substituents indicated in [Table 1A] to [Table 20A] (hereinafter, referred to as Compound Class SX353).

A compound (L-1) wherein n is 2, $R^1$ represents a methyl group, Q represents a group represented by Q1-123, and Het represents any substituents indicated in [Table 1A] to [Table 20A] (hereinafter, referred to as Compound Class SX354).

A compound (L-1) wherein n is 2, $R^1$ represents a methyl group, Q represents a group represented by Q1-124, and Het represents any substituents indicated in [Table 1A] to [Table 20A] (hereinafter, referred to as Compound Class SX355).

A compound (L-1) wherein n is 2, $R^1$ represents a methyl group, Q represents a group represented by Q1-125, and Het represents any substituents indicated in [Table 1A] to [Table 20A] (hereinafter, referred to as Compound Class SX356).

A compound (L-1) wherein n is 2, $R^1$ represents a methyl group, Q represents a group represented by Q1-126, and Het represents any substituents indicated in [Table 1A] to [Table 20A] (hereinafter, referred to as Compound Class SX357).

A compound (L-1) wherein n is 2, $R^1$ represents a methyl group, Q represents a group represented by Q1-127, and Het represents any substituents indicated in [Table 1A] to [Table 20A] (hereinafter, referred to as Compound Class SX358).

A compound (L-1) wherein n is 2, $R^1$ represents a methyl group, Q represents a group represented by Q1-128, and Het represents any substituents indicated in [Table 1A] to [Table 20A] (hereinafter, referred to as Compound Class SX359).

A compound (L-1) wherein n is 2, $R^1$ represents a methyl group, Q represents a group represented by Q1-129, and Het represents any substituents indicated in [Table 1A] to [Table 20A] (hereinafter, referred to as Compound Class SX360).

A compound (L-1) wherein n is 2, $R^1$ represents a methyl group, Q represents a group represented by Q1-130, and Het represents any substituents indicated in [Table 1A] to [Table 20A] (hereinafter, referred to as Compound Class SX361).

A compound (L-1) wherein n is 2, $R^1$ represents a methyl group, Q represents a group represented by Q1-131, and Het represents any substituents indicated in [Table 1A] to [Table 20A] (hereinafter, referred to as Compound Class SX362).

A compound (L-1) wherein n is 2, $R^1$ represents a methyl group, Q represents a group represented by Q1-132, and Het represents any substituents indicated in [Table 1A] to [Table 20A] (hereinafter, referred to as Compound Class SX363).

A compound (L-1) wherein n is 2, $R^1$ represents a methyl group, Q represents a group represented by Q1-133, and Het represents any substituents indicated in [Table 1A] to [Table 20A] (hereinafter, referred to as Compound Class SX364).

A compound (L-1) wherein n is 2, $R^1$ represents a methyl group, Q represents a group represented by Q1-134, and Het represents any substituents indicated in [Table 1A] to [Table 20A] (hereinafter, referred to as Compound Class SX365).

A compound (L-1) wherein n is 2, $R^1$ represents a methyl group, Q represents a group represented by Q1-135, and Het represents any substituents indicated in [Table 1A] to [Table 20A] (hereinafter, referred to as Compound Class SX366).

A compound (L-1) wherein n is 2, $R^1$ represents a methyl group, Q represents a group represented by Q1-136, and Het represents any substituents indicated in [Table 1A] to [Table 20A] (hereinafter, referred to as Compound Class SX367).

A compound (L-1) wherein n is 2, $R^1$ represents a methyl group, Q represents a group represented by Q1-137,

and Het represents any substituents indicated in [Table 1A] to [Table 20A] (hereinafter, referred to as Compound Class SX368).

A compound (L-1) wherein n is 2, $R^1$ represents a methyl group, Q represents a group represented by Q1-138, and Het represents any substituents indicated in [Table 1A] to [Table 20A] (hereinafter, referred to as Compound Class SX369).

A compound (L-1) wherein n is 2, $R^1$ represents a methyl group, Q represents a group represented by Q1-139, and Het represents any substituents indicated in [Table 1A] to [Table 20A] (hereinafter, referred to as Compound Class SX370).

A compound (L-1) wherein n is 2, $R^1$ represents a methyl group, Q represents a group represented by Q1-140, and Het represents any substituents indicated in [Table 1A] to [Table 20A] (hereinafter, referred to as Compound Class SX371).

A compound (L-1) wherein n is 2, $R^1$ represents a methyl group, Q represents a group represented by Q1-141, and Het represents any substituents indicated in [Table 1A] to [Table 20A] (hereinafter, referred to as Compound Class SX372).

A compound (L-1) wherein n is 2, $R^1$ represents a methyl group, Q represents a group represented by Q1-142, and Het represents any substituents indicated in [Table 1A] to [Table 20A] (hereinafter, referred to as Compound Class SX373).

A compound (L-1) wherein n is 2, $R^1$ represents a methyl group, Q represents a group represented by Q1-143, and Het represents any substituents indicated in [Table 1A] to [Table 20A] (hereinafter, referred to as Compound Class SX374).

A compound (L-1) wherein n is 2, $R^1$ represents a methyl group, Q represents a group represented by Q1-144, and Het represents any substituents indicated in [Table 1A] to [Table 20A] (hereinafter, referred to as Compound Class SX375).

A compound (L-1) wherein n is 2, $R^1$ represents a methyl group, Q represents a group represented by Q1-145, and Het represents any substituents indicated in [Table 1A] to [Table 20A] (hereinafter, referred to as Compound Class SX376).

A compound (L-1) wherein n is 2, $R^1$ represents a methyl group, Q represents a group represented by Q1-145, and Het represents any substituents indicated in [Table 1A] to [Table 20A] (hereinafter, referred to as Compound Class SX376).

A compound (L-1) wherein n is 2, $R^1$ represents a methyl group, Q represents a group represented by Q1-146, and Het represents any substituents indicated in [Table 1A] to [Table 20A] (hereinafter, referred to as Compound Class SX377).

A compound (L-1) wherein n is 2, $R^1$ represents a methyl group, Q represents a group represented by Q1-147, and Het represents any substituents indicated in [Table 1A] to [Table 20A] (hereinafter, referred to as Compound Class SX378).

A compound (L-1) wherein n is 2, $R^1$ represents a methyl group, Q represents a group represented by Q1-148, and Het represents any substituents indicated in [Table 1A] to [Table 20A] (hereinafter, referred to as Compound Class SX379).

A compound (L-1) wherein n is 2, $R^1$ represents a methyl group, Q represents a group represented by Q1-149, and Het represents any substituents indicated in [Table 1A] to [Table 20A] (hereinafter, referred to as Compound Class SX380).

A compound (L-1) wherein n is 2, $R^1$ represents a methyl group, Q represents a group represented by Q1-150, and Het represents any substituents indicated in [Table 1A] to [Table 20A] (hereinafter, referred to as Compound Class SX381).

A compound (L-1) wherein n is 2, $R^1$ represents a methyl group, Q represents a group represented by Q1-151, and Het represents any substituents indicated in [Table 1A] to [Table 20A] (hereinafter, referred to as Compound Class SX382).

A compound (L-1) wherein n is 2, $R^1$ represents a methyl group, Q represents a group represented by Q1-152, and Het represents any substituents indicated in [Table 1A] to [Table 20A] (hereinafter, referred to as Compound Class SX383).

A compound (L-1) wherein n is 2, $R^1$ represents a methyl group, Q represents a group represented by Q1-153, and Het represents any substituents indicated in [Table 1A] to [Table 20A] (hereinafter, referred to as Compound Class SX384).

A compound (L-1) wherein n is 2, $R^1$ represents a methyl group, Q represents a group represented by Q1-154, and Het represents any substituents indicated in [Table 1A] to [Table 20A] (hereinafter, referred to as Compound Class SX385).

A compound (L-1) wherein n is 2, $R^1$ represents a methyl group, Q represents a group represented by Q1-155, and Het represents any substituents indicated in [Table 1A] to [Table 20A] (hereinafter, referred to as Compound Class

SX386).

A compound (L-1) wherein n is 2, $R^1$ represents a methyl group, Q represents a group represented by Q1-156, and Het represents any substituents indicated in [Table 1A] to [Table 20A] (hereinafter, referred to as Compound Class SX387).

A compound (L-1) wherein n is 2, $R^1$ represents a methyl group, Q represents a group represented by Q1-157, and Het represents any substituents indicated in [Table 1A] to [Table 20A] (hereinafter, referred to as Compound Class SX388).

A compound (L-1) wherein n is 2, $R^1$ represents a methyl group, Q represents a group represented by Q1-158, and Het represents any substituents indicated in [Table 1A] to [Table 20A] (hereinafter, referred to as Compound Class SX389).

A compound (L-1) wherein n is 2, $R^1$ represents a methyl group, Q represents a group represented by Q1-159, and Het represents any substituents indicated in [Table 1A] to [Table 20A] (hereinafter, referred to as Compound Class SX390).

A compound (L-1) wherein n is 2, $R^1$ represents a methyl group, Q represents a group represented by Q1-160, and Het represents any substituents indicated in [Table 1A] to [Table 20A] (hereinafter, referred to as Compound Class SX391).

A compound (L-1) wherein n is 2, $R^1$ represents a methyl group, Q represents a group represented by Q1-161, and Het represents any substituents indicated in [Table 1A] to [Table 20A] (hereinafter, referred to as Compound Class SX392).

A compound (L-1) wherein n is 2, $R^1$ represents a methyl group, Q represents a group represented by Q1-161, and Het represents any substituents indicated in [Table 1A] to [Table 20A] (hereinafter, referred to as Compound Class SX392).

A compound (L-1) wherein n is 2, $R^1$ represents a methyl group, Q represents a group represented by Q1-162, and Het represents any substituents indicated in [Table 1A] to [Table 20A] (hereinafter, referred to as Compound Class SX393).

A compound (L-1) wherein n is 2, $R^1$ represents a methyl group, Q represents a group represented by Q1-163, and Het represents any substituents indicated in [Table 1A] to [Table 20A] (hereinafter, referred to as Compound Class SX394).

A compound (L-1) wherein n is 2, $R^1$ represents a methyl group, Q represents a group represented by Q1-164, and Het represents any substituents indicated in [Table 1A] to [Table 20A] (hereinafter, referred to as Compound Class SX395).

A compound (L-1) wherein n is 2, $R^1$ represents a methyl group, Q represents a group represented by Q1-165, and Het represents any substituents indicated in [Table 1A] to [Table 20A] (hereinafter, referred to as Compound Class SX396).

A compound (L-1) wherein n is 2, $R^1$ represents a methyl group, Q represents a group represented by Q1-166, and Het represents any substituents indicated in [Table 1A] to [Table 20A] (hereinafter, referred to as Compound Class SX397).

A compound (L-1) wherein n is 2, $R^1$ represents a methyl group, Q represents a group represented by Q1-167, and Het represents any substituents indicated in [Table 1A] to [Table 20A] (hereinafter, referred to as Compound Class SX398).

A compound (L-1) wherein n is 2, $R^1$ represents a methyl group, Q represents a group represented by Q1-168, and Het represents any substituents indicated in [Table 1A] to [Table 20A] (hereinafter, referred to as Compound Class SX399).

A compound (L-1) wherein n is 2, $R^1$ represents a methyl group, Q represents a group represented by Q1-169, and Het represents any substituents indicated in [Table 1A] to [Table 20A] (hereinafter, referred to as Compound Class SX400).

A compound (L-1) wherein n is 2, $R^1$ represents a methyl group, Q represents a group represented by Q1-170, and Het represents any substituents indicated in [Table 1A] to [Table 20A] (hereinafter, referred to as Compound Class SX401).

A compound (L-1) wherein n is 2, $R^1$ represents a methyl group, Q represents a group represented by Q1-171, and Het represents any substituents indicated in [Table 1A] to [Table 20A] (hereinafter, referred to as Compound Class SX402).

A compound (L-1) wherein n is 2, $R^1$ represents a methyl group, Q represents a group represented by Q1-172, and Het represents any substituents indicated in [Table 1A] to [Table 20A] (hereinafter, referred to as Compound Class SX403).

A compound (L-1) wherein n is 2, $R^1$ represents a methyl group, Q represents a group represented by Q1-173, and Het represents any substituents indicated in [Table 1A] to [Table 20A] (hereinafter, referred to as Compound Class SX404).

A compound (L-1) wherein n is 2, $R^1$ represents a methyl group, Q represents a group represented by Q1-174, and Het represents any substituents indicated in [Table 1A] to [Table 20A] (hereinafter, referred to as Compound Class SX405).

A compound (L-1) wherein n is 2, $R^1$ represents a methyl group, Q represents a group represented by Q1-175, and Het represents any substituents indicated in [Table 1A] to [Table 20A] (hereinafter, referred to as Compound Class SX406).

A compound (L-1) wherein n is 2, $R^1$ represents a methyl group, Q represents a group represented by Q1-176, and Het represents any substituents indicated in [Table 1A] to [Table 20A] (hereinafter, referred to as Compound Class SX407).

A compound (L-1) wherein n is 2, $R^1$ represents a methyl group, Q represents a group represented by Q1-177, and Het represents any substituents indicated in [Table 1A] to [Table 20A] (hereinafter, referred to as Compound Class SX408).

A compound (L-1) wherein n is 2, $R^1$ represents a methyl group, Q represents a group represented by Q1-178, and Het represents any substituents indicated in [Table 1A] to [Table 20A] (hereinafter, referred to as Compound Class SX409).

A compound (L-1) wherein n is 2, $R^1$ represents a methyl group, Q represents a group represented by Q1-179, and Het represents any substituents indicated in [Table 1A] to [Table 20A] (hereinafter, referred to as Compound Class SX410).

A compound (L-1) wherein n is 2, $R^1$ represents a methyl group, Q represents a group represented by Q1-180, and Het represents any substituents indicated in [Table 1A] to [Table 20A] (hereinafter, referred to as Compound Class SX411).

A compound (L-1) wherein n is 2, $R^1$ represents a methyl group, Q represents a group represented by Q1-181, and Het represents any substituents indicated in [Table 1A] to [Table 20A] (hereinafter, referred to as Compound Class SX412).

A compound (L-1) wherein n is 2, $R^1$ represents a methyl group, Q represents a group represented by Q1-181, and Het represents any substituents indicated in [Table 1A] to [Table 20A] (hereinafter, referred to as Compound Class SX412).

A compound (L-1) wherein n is 2, $R^1$ represents a methyl group, Q represents a group represented by Q1-182, and Het represents any substituents indicated in [Table 1A] to [Table 20A] (hereinafter, referred to as Compound Class SX413).

A compound (L-1) wherein n is 2, $R^1$ represents a methyl group, Q represents a group represented by Q1-183, and Het represents any substituents indicated in [Table 1A] to [Table 20A] (hereinafter, referred to as Compound Class SX414).

A compound (L-1) wherein n is 2, $R^1$ represents a methyl group, Q represents a group represented by Q1-184, and Het represents any substituents indicated in [Table 1A] to [Table 20A] (hereinafter, referred to as Compound Class SX415).

A compound (L-1) wherein n is 2, $R^1$ represents a methyl group, Q represents a group represented by Q1-185, and Het represents any substituents indicated in [Table 1A] to [Table 20A] (hereinafter, referred to as Compound Class SX416).

A compound (L-1) wherein n is 2, $R^1$ represents a methyl group, Q represents a group represented by Q1-186, and Het represents any substituents indicated in [Table 1A] to [Table 20A] (hereinafter, referred to as Compound Class SX417).

A compound (L-1) wherein n is 2, $R^1$ represents a methyl group, Q represents a group represented by Q1-187, and Het represents any substituents indicated in [Table 1A] to [Table 20A] (hereinafter, referred to as Compound Class SX418).

A compound (L-1) wherein n is 2, $R^1$ represents a methyl group, Q represents a group represented by Q1-188, and Het represents any substituents indicated in [Table 1A] to [Table 20A] (hereinafter, referred to as Compound Class SX419).

A compound (L-1) wherein n is 2, $R^1$ represents a methyl group, Q represents a group represented by Q1-189, and Het represents any substituents indicated in [Table 1A] to [Table 20A] (hereinafter, referred to as Compound Class SX420).

A compound (L-1) wherein n is 2, $R^1$ represents a methyl group, Q represents a group represented by Q1-190, and Het represents any substituents indicated in [Table 1A] to [Table 20A] (hereinafter, referred to as Compound Class SX421).

A compound (L-1) wherein n is 2, $R^1$ represents a methyl group, Q represents a group represented by Q1-191, and Het represents any substituents indicated in [Table 1A] to [Table 20A] (hereinafter, referred to as Compound Class SX422).

A compound (L-1) wherein n is 2, $R^1$ represents a methyl group, Q represents a group represented by Q1-192,

and Het represents any substituents indicated in [Table 1A] to [Table 20A] (hereinafter, referred to as Compound Class SX423).

A compound (L-1) wherein n is 2, $R^1$ represents a methyl group, Q represents a group represented by Q1-193, and Het represents any substituents indicated in [Table 1A] to [Table 20A] (hereinafter, referred to as Compound Class SX424).

A compound (L-1) wherein n is 2, $R^1$ represents a methyl group, Q represents a group represented by Q1-194, and Het represents any substituents indicated in [Table 1A] to [Table 20A] (hereinafter, referred to as Compound Class SX425).

A compound (L-1) wherein n is 2, $R^1$ represents a methyl group, Q represents a group represented by Q1-195, and Het represents any substituents indicated in [Table 1A] to [Table 20A] (hereinafter, referred to as Compound Class SX426).

A compound (L-1) wherein n is 2, $R^1$ represents a methyl group, Q represents a group represented by Q1-196, and Het represents any substituents indicated in [Table 1A] to [Table 20A] (hereinafter, referred to as Compound Class SX427).

A compound (L-1) wherein n is 2, $R^1$ represents a methyl group, Q represents a group represented by Q1-197, and Het represents any substituents indicated in [Table 1A] to [Table 20A] (hereinafter, referred to as Compound Class SX428).

A compound (L-1) wherein n is 2, $R^1$ represents a methyl group, Q represents a group represented by Q1-198, and Het represents any substituents indicated in [Table 1A] to [Table 20A] (hereinafter, referred to as Compound Class SX429).

A compound (L-1) wherein n is 2, $R^1$ represents a methyl group, Q represents a group represented by Q1-199, and Het represents any substituents indicated in [Table 1A] to [Table 20A] (hereinafter, referred to as Compound Class SX430).

A compound (L-1) wherein n is 2, $R^1$ represents a methyl group, Q represents a group represented by Q1-200, and Het represents any substituents indicated in [Table 1A] to [Table 20A] (hereinafter, referred to as Compound Class SX431).

A compound (L-1) wherein n is 2, $R^1$ represents a methyl group, Q represents a group represented by Q1-201, and Het represents any substituents indicated in [Table 1A] to [Table 20A] (hereinafter, referred to as Compound Class SX432).

A compound (L-1) wherein n is 2, $R^1$ represents a methyl group, Q represents a group represented by Q1-202, and Het represents any substituents indicated in [Table 1A] to [Table 20A] (hereinafter, referred to as Compound Class SX433).

A compound (L-1) wherein n is 2, $R^1$ represents a methyl group, Q represents a group represented by Q1-203, and Het represents any substituents indicated in [Table 1A] to [Table 20A] (hereinafter, referred to as Compound Class SX434).

A compound (L-1) wherein n is 2, $R^1$ represents a methyl group, Q represents a group represented by Q1-204, and Het represents any substituents indicated in [Table 1A] to [Table 20A] (hereinafter, referred to as Compound Class SX435).

A compound (L-1) wherein n is 2, $R^1$ represents a methyl group, Q represents a group represented by Q1-205, and Het represents any substituents indicated in [Table 1A] to [Table 20A] (hereinafter, referred to as Compound Class SX436).

A compound (L-1) wherein n is 2, $R^1$ represents a methyl group, Q represents a group represented by Q1-206, and Het represents any substituents indicated in [Table 1A] to [Table 20A] (hereinafter, referred to as Compound Class SX437).

A compound (L-1) wherein n is 2, $R^1$ represents a methyl group, Q represents a group represented by Q1-207, and Het represents any substituents indicated in [Table 1A] to [Table 20A] (hereinafter, referred to as Compound Class SX438).

A compound (L-1) wherein n is 2, $R^1$ represents a methyl group, Q represents a group represented by Q1-208, and Het represents any substituents indicated in [Table 1A] to [Table 20A] (hereinafter, referred to as Compound Class SX439).

A compound (L-1) wherein n is 2, $R^1$ represents a methyl group, Q represents a group represented by Q1-209, and Het represents any substituents indicated in [Table 1A] to [Table 20A] (hereinafter, referred to as Compound Class SX440).

A compound (L-1) wherein n is 2, $R^1$ represents a methyl group, Q represents a group represented by Q2-1, and Het represents any substituents indicated in [Table 1A] to [Table 20A] (hereinafter, referred to as Compound Class SX441).

A compound (L-1) wherein n is 2, $R^1$ represents a methyl group, Q represents a group represented by Q2-2, and Het represents any substituents indicated in [Table 1A] to [Table 20A] (hereinafter, referred to as Compound Class SX442).

A compound (L-1) wherein n is 2, $R^1$ represents a methyl group, Q represents a group represented by Q2-3, and

Het represents any substituents indicated in [Table 1A] to [Table 20A] (hereinafter, referred to as Compound Class SX443).
A compound (L-1) wherein n is 2, $R^1$ represents a methyl group, Q represents a group represented by Q2-4, and Het represents any substituents indicated in [Table 1A] to [Table 20A] (hereinafter, referred to as Compound Class SX444).
A compound (L-1) wherein n is 2, $R^1$ represents a methyl group, Q represents a group represented by Q2-5, and Het represents any substituents indicated in [Table 1A] to [Table 20A] (hereinafter, referred to as Compound Class SX445).
A compound (L-1) wherein n is 2, $R^1$ represents a methyl group, Q represents a group represented by Q2-6, and Het represents any substituents indicated in [Table 1A] to [Table 20A] (hereinafter, referred to as Compound Class SX446).
A compound (L-1) wherein n is 2, $R^1$ represents a methyl group, Q represents a group represented by Q2-7, and Het represents any substituents indicated in [Table 1A] to [Table 20A] (hereinafter, referred to as Compound Class SX447).
A compound (L-1) wherein n is 2, $R^1$ represents a methyl group, Q represents a group represented by Q2-8, and Het represents any substituents indicated in [Table 1A] to [Table 20A] (hereinafter, referred to as Compound Class SX448).
A compound (L-1) wherein n is 2, $R^1$ represents a methyl group, Q represents a group represented by Q2-9, and Het represents any substituents indicated in [Table 1A] to [Table 20A] (hereinafter, referred to as Compound Class SX449).
A compound (L-1) wherein n is 2, $R^1$ represents a methyl group, Q represents a group represented by Q2-10, and Het represents any substituents indicated in [Table 1A] to [Table 20A] (hereinafter, referred to as Compound Class SX450).
A compound (L-1) wherein n is 2, $R^1$ represents a methyl group, Q represents a group represented by Q2-11, and Het represents any substituents indicated in [Table 1A] to [Table 20A] (hereinafter, referred to as Compound Class SX451).
A compound (L-1) wherein n is 2, $R^1$ represents a methyl group, Q represents a group represented by Q2-12, and Het represents any substituents indicated in [Table 1A] to [Table 20A] (hereinafter, referred to as Compound Class SX452).
A compound (L-1) wherein n is 2, $R^1$ represents a methyl group, Q represents a group represented by Q2-13, and Het represents any substituents indicated in [Table 1A] to [Table 20A] (hereinafter, referred to as Compound Class SX453).
A compound (L-1) wherein n is 2, $R^1$ represents a methyl group, Q represents a group represented by Q2-14, and Het represents any substituents indicated in [Table 1A] to [Table 20A] (hereinafter, referred to as Compound Class SX454).
A compound (L-1) wherein n is 2, $R^1$ represents a methyl group, Q represents a group represented by Q2-15, and Het represents any substituents indicated in [Table 1A] to [Table 20A] (hereinafter, referred to as Compound Class SX455).
A compound (L-1) wherein n is 2, $R^1$ represents a methyl group, Q represents a group represented by Q2-16, and Het represents any substituents indicated in [Table 1A] to [Table 20A] (hereinafter, referred to as Compound Class SX456).
A compound (L-1) wherein n is 2, $R^1$ represents a methyl group, Q represents a group represented by Q2-17, and Het represents any substituents indicated in [Table 1A] to [Table 20A] (hereinafter, referred to as Compound Class SX457).
A compound (L-1) wherein n is 2, $R^1$ represents a methyl group, Q represents a group represented by Q2-18, and Het represents any substituents indicated in [Table 1A] to [Table 20A] (hereinafter, referred to as Compound Class SX458).
A compound (L-1) wherein n is 2, $R^1$ represents a methyl group, Q represents a group represented by Q2-19, and Het represents any substituents indicated in [Table 1A] to [Table 20A] (hereinafter, referred to as Compound Class SX459).
A compound (L-1) wherein n is 2, $R^1$ represents a methyl group, Q represents a group represented by Q2-20, and Het represents any substituents indicated in [Table 1A] to [Table 20A] (hereinafter, referred to as Compound Class SX460).
A compound (L-1) wherein n is 2, $R^1$ represents a methyl group, Q represents a group represented by Q2-21, and Het represents any substituents indicated in [Table 1A] to [Table 20A] (hereinafter, referred to as Compound Class SX461).
A compound (L-1) wherein n is 2, $R^1$ represents a methyl group, Q represents a group represented by Q2-22, and Het represents any substituents indicated in [Table 1A] to [Table 20A] (hereinafter, referred to as Compound Class SX462).
A compound (L-1) wherein n is 2, $R^1$ represents a methyl group, Q represents a group represented by Q2-23, and Het represents any substituents indicated in [Table 1A] to [Table 20A] (hereinafter, referred to as Compound Class SX463).
A compound (L-1) wherein n is 2, $R^1$ represents a methyl group, Q represents a group represented by Q2-24, and Het represents any substituents indicated in [Table 1A] to [Table 20A] (hereinafter, referred to as Compound Class SX464).
A compound (L-1) wherein n is 2, $R^1$ represents a methyl group, Q represents a group represented by Q2-25, and Het represents any substituents indicated in [Table 1A] to [Table 20A] (hereinafter, referred to as Compound Class SX465).
A compound (L-1) wherein n is 2, $R^1$ represents a methyl group, Q represents a group represented by Q2-26, and Het represents any substituents indicated in [Table 1A] to [Table 20A] (hereinafter, referred to as Compound Class SX466).
A compound (L-1) wherein n is 2, $R^1$ represents a methyl group, Q represents a group represented by Q2-27, and Het represents any substituents indicated in [Table 1A] to [Table 20A] (hereinafter, referred to as Compound Class SX467).
A compound (L-1) wherein n is 2, $R^1$ represents a methyl group, Q represents a group represented by Q2-28, and Het represents any substituents indicated in [Table 1A] to [Table 20A] (hereinafter, referred to as Compound Class SX468).
A compound (L-1) wherein n is 2, $R^1$ represents a methyl group, Q represents a group represented by Q2-29, and Het represents any substituents indicated in [Table 1A] to [Table 20A] (hereinafter, referred to as Compound Class SX469).
A compound (L-1) wherein n is 2, $R^1$ represents a methyl group, Q represents a group represented by Q2-30, and Het represents any substituents indicated in [Table 1A] to [Table 20A] (hereinafter, referred to as Compound Class SX470).
A compound (L-1) wherein n is 2, $R^1$ represents a methyl group, Q represents a group represented by Q2-31, and Het represents any substituents indicated in [Table 1A] to [Table 20A] (hereinafter, referred to as Compound Class SX471).
A compound (L-1) wherein n is 2, $R^1$ represents a methyl group, Q represents a group represented by Q5-1, and

Het represents any substituents indicated in [Table 1A] to [Table 20A] (hereinafter, referred to as Compound Class SX472).

A compound (L-1) wherein n is 2, $R^1$ represents a methyl group, Q represents a group represented by Q5-2, and Het represents any substituents indicated in [Table 1A] to [Table 20A] (hereinafter, referred to as Compound Class SX473).

A compound (L-1) wherein n is 2, $R^1$ represents a methyl group, Q represents a group represented by Q5-3, and Het represents any substituents indicated in [Table 1A] to [Table 20A] (hereinafter, referred to as Compound Class SX474).

A compound (L-1) wherein n is 2, $R^1$ represents a methyl group, Q represents a group represented by Q5-4, and Het represents any substituents indicated in [Table 1A] to [Table 20A] (hereinafter, referred to as Compound Class SX475).

A compound (L-1) wherein n is 2, $R^1$ represents a methyl group, Q represents a group represented by Q5-5, and Het represents any substituents indicated in [Table 1A] to [Table 20A] (hereinafter, referred to as Compound Class SX476).

A compound (L-1) wherein n is 2, $R^1$ represents a methyl group, Q represents a group represented by Q5-6, and Het represents any substituents indicated in [Table 1A] to [Table 20A] (hereinafter, referred to as Compound Class SX477).

A compound (L-1) wherein n is 2, $R^1$ represents a methyl group, Q represents a group represented by Q5-7, and Het represents any substituents indicated in [Table 1A] to [Table 20A] (hereinafter, referred to as Compound Class SX478).

A compound (L-1) wherein n is 2, $R^1$ represents a methyl group, Q represents a group represented by Q5-8, and Het represents any substituents indicated in [Table 1A] to [Table 20A] (hereinafter, referred to as Compound Class SX479).

A compound (L-1) wherein n is 2, $R^1$ represents a methyl group, Q represents a group represented by Q5-9, and Het represents any substituents indicated in [Table 1A] to [Table 20A] (hereinafter, referred to as Compound Class SX480).

A compound (L-1) wherein n is 2, $R^1$ represents a methyl group, Q represents a group represented by Q5-10, and Het represents any substituents indicated in [Table 1A] to [Table 20A] (hereinafter, referred to as Compound Class SX481).

A compound (L-1) wherein n is 2, $R^1$ represents a methyl group, Q represents a group represented by Q5-11, and Het represents any substituents indicated in [Table 1A] to [Table 20A] (hereinafter, referred to as Compound Class SX482).

A compound (L-1) wherein n is 2, $R^1$ represents a methyl group, Q represents a group represented by Q5-12, and Het represents any substituents indicated in [Table 1A] to [Table 20A] (hereinafter, referred to as Compound Class SX483).

A compound (L-1) wherein n is 2, $R^1$ represents a methyl group, Q represents a group represented by Q5-13, and Het represents any substituents indicated in [Table 1A] to [Table 20A] (hereinafter, referred to as Compound Class SX484).

A compound (L-1) wherein n is 2, $R^1$ represents a methyl group, Q represents a group represented by Q5-14, and Het represents any substituents indicated in [Table 1A] to [Table 20A] (hereinafter, referred to as Compound Class SX485).

A compound (L-1) wherein n is 2, $R^1$ represents a methyl group, Q represents a group represented by Q5-15, and Het represents any substituents indicated in [Table 1A] to [Table 20A] (hereinafter, referred to as Compound Class SX486).

A compound (L-1) wherein n is 2, $R^1$ represents a methyl group, Q represents a group represented by Q5-16, and Het represents any substituents indicated in [Table 1A] to [Table 20A] (hereinafter, referred to as Compound Class SX487).

A compound (L-1) wherein n is 2, $R^1$ represents a methyl group, Q represents a group represented by Q5-17, and Het represents any substituents indicated in [Table 1A] to [Table 20A] (hereinafter, referred to as Compound Class SX488).

A compound (L-1) wherein n is 2, $R^1$ represents a methyl group, Q represents a group represented by Q5-18, and Het represents any substituents indicated in [Table 1A] to [Table 20A] (hereinafter, referred to as Compound Class SX489).

A compound (L-1) wherein n is 2, $R^1$ represents a methyl group, Q represents a group represented by Q5-19, and Het represents any substituents indicated in [Table 1A] to [Table 20A] (hereinafter, referred to as Compound Class SX490).

A compound (L-1) wherein n is 2, $R^1$ represents a methyl group, Q represents a group represented by Q5-20, and Het represents any substituents indicated in [Table 1A] to [Table 20A] (hereinafter, referred to as Compound Class SX491).

A compound (L-1) wherein n is 2, $R^1$ represents a methyl group, Q represents a group represented by Q5-21, and Het represents any substituents indicated in [Table 1A] to [Table 20A] (hereinafter, referred to as Compound Class SX492).

A compound (L-1) wherein n is 2, $R^1$ represents a methyl group, Q represents a group represented by Q5-22, and Het represents any substituents indicated in [Table 1A] to [Table 20A] (hereinafter, referred to as Compound Class SX493).

A compound (L-1) wherein n is 2, $R^1$ represents a methyl group, Q represents a group represented by Q6-1, and Het represents any substituents indicated in [Table 1A] to [Table 20A] (hereinafter, referred to as Compound Class SX494).

A compound (L-1) wherein n is 2, $R^1$ represents a methyl group, Q represents a group represented by Q6-2, and Het represents any substituents indicated in [Table 1A] to [Table 20A] (hereinafter, referred to as Compound Class SX495).

A compound (L-1) wherein n is 2, $R^1$ represents a methyl group, Q represents a group represented by Q6-3, and Het represents any substituents indicated in [Table 1A] to [Table 20A] (hereinafter, referred to as Compound Class SX496).

A compound (L-1) wherein n is 2, $R^1$ represents a methyl group, Q represents a group represented by Q6-4, and Het represents any substituents indicated in [Table 1A] to [Table 20A] (hereinafter, referred to as Compound Class SX497).

A compound (L-1) wherein n is 2, $R^1$ represents a methyl group, Q represents a group represented by Q6-5, and Het represents any substituents indicated in [Table 1A] to [Table 20A] (hereinafter, referred to as Compound Class SX498).

A compound (L-1) wherein n is 2, $R^1$ represents a methyl group, Q represents a group represented by Q6-6, and Het represents any substituents indicated in [Table 1A] to [Table 20A] (hereinafter, referred to as Compound Class SX499).

A compound (L-1) wherein n is 2, $R^1$ represents a methyl group, Q represents a group represented by Q6-7, and Het represents any substituents indicated in [Table 1A] to [Table 20A] (hereinafter, referred to as Compound Class SX500).

A compound (L-1) wherein n is 2, $R^1$ represents a methyl group, Q represents a group represented by Q6-8, and

Het represents any substituents indicated in [Table 1A] to [Table 20A] (hereinafter, referred to as Compound Class SX501).

A compound (L-1) wherein n is 2, $R^1$ represents a methyl group, Q represents a group represented by Q6-9, and Het represents any substituents indicated in [Table 1A] to [Table 20A] (hereinafter, referred to as Compound Class SX502).

A compound (L-1) wherein n is 2, $R^1$ represents a methyl group, Q represents a group represented by Q6-10, and Het represents any substituents indicated in [Table 1A] to [Table 20A] (hereinafter, referred to as Compound Class SX503).

A compound (L-1) wherein n is 2, $R^1$ represents a methyl group, Q represents a group represented by Q6-11, and Het represents any substituents indicated in [Table 1A] to [Table 20A] (hereinafter, referred to as Compound Class SX504).

A compound (L-1) wherein n is 2, $R^1$ represents a methyl group, Q represents a group represented by Q6-12, and Het represents any substituents indicated in [Table 1A] to [Table 20A] (hereinafter, referred to as Compound Class SX505).

A compound (L-1) wherein n is 2, $R^1$ represents a methyl group, Q represents a group represented by Q6-13, and Het represents any substituents indicated in [Table 1A] to [Table 20A] (hereinafter, referred to as Compound Class SX506).

A compound (L-1) wherein n is 2, $R^1$ represents a methyl group, Q represents a group represented by Q6-14, and Het represents any substituents indicated in [Table 1A] to [Table 20A] (hereinafter, referred to as Compound Class SX507).

A compound (L-1) wherein n is 2, $R^1$ represents a methyl group, Q represents a group represented by Q6-15, and Het represents any substituents indicated in [Table 1A] to [Table 20A] (hereinafter, referred to as Compound Class SX508).

A compound (L-1) wherein n is 2, $R^1$ represents a methyl group, Q represents a group represented by Q6-16, and Het represents any substituents indicated in [Table 1A] to [Table 20A] (hereinafter, referred to as Compound Class SX509).

A compound (L-1) wherein n is 2, $R^1$ represents a methyl group, Q represents a group represented by Q6-17, and Het represents any substituents indicated in [Table 1A] to [Table 20A] (hereinafter, referred to as Compound Class SX510).

A compound (L-1) wherein n is 2, $R^1$ represents a methyl group, Q represents a group represented by Q6-18, and Het represents any substituents indicated in [Table 1A] to [Table 20A] (hereinafter, referred to as Compound Class SX511).

A compound (L-1) wherein n is 2, $R^1$ represents a methyl group, Q represents a group represented by Q6-19, and Het represents any substituents indicated in [Table 1A] to [Table 20A] (hereinafter, referred to as Compound Class SX512).

A compound (L-1) wherein n is 2, $R^1$ represents a methyl group, Q represents a group represented by Q6-20, and Het represents any substituents indicated in [Table 1A] to [Table 20A] (hereinafter, referred to as Compound Class SX513).

A compound (L-1) wherein n is 2, $R^1$ represents a methyl group, Q represents a group represented by Q6-21, and Het represents any substituents indicated in [Table 1A] to [Table 20A] (hereinafter, referred to as Compound Class SX514).

A compound (L-1) wherein n is 2, $R^1$ represents a methyl group, Q represents a group represented by Q6-22, and Het represents any substituents indicated in [Table 1A] to [Table 20A] (hereinafter, referred to as Compound Class SX515).

A compound (L-1) wherein n is 0, $R^1$ represents an ethyl group, Q represents a group represented by Q1-1, and Het represents any substituents indicated in [Table 1A] to [Table 20A] (hereinafter, referred to as Compound Class SX516).

A compound (L-1) wherein n is 0, $R^1$ represents an ethyl group, Q represents a group represented by Q1-2, and Het represents any substituents indicated in [Table 1A] to [Table 20A] (hereinafter, referred to as Compound Class SX517).

A compound (L-1) wherein n is 0, $R^1$ represents an ethyl group, Q represents a group represented by Q1-3, and Het represents any substituents indicated in [Table 1A] to [Table 20A] (hereinafter, referred to as Compound Class SX518).

A compound (L-1) wherein n is 0, $R^1$ represents an ethyl group, Q represents a group represented by Q1-4, and Het represents any substituents indicated in [Table 1A] to [Table 20A] (hereinafter, referred to as Compound Class SX519).

A compound (L-1) wherein n is 0, $R^1$ represents an ethyl group, Q represents a group represented by Q1-5, and Het represents any substituents indicated in [Table 1A] to [Table 20A] (hereinafter, referred to as Compound Class SX520).

A compound (L-1) wherein n is 0, $R^1$ represents an ethyl group, Q represents a group represented by Q1-6, and Het represents any substituents indicated in [Table 1A] to [Table 20A] (hereinafter, referred to as Compound Class SX521).

A compound (L-1) wherein n is 0, $R^1$ represents an ethyl group, Q represents a group represented by Q1-7, and Het represents any substituents indicated in [Table 1A] to [Table 20A] (hereinafter, referred to as Compound Class SX522).

A compound (L-1) wherein n is 0, $R^1$ represents an ethyl group, Q represents a group represented by Q1-8, and Het represents any substituents indicated in [Table 1A] to [Table 20A] (hereinafter, referred to as Compound Class SX523).

A compound (L-1) wherein n is 0, $R^1$ represents an ethyl group, Q represents a group represented by Q1-9, and Het represents any substituents indicated in [Table 1A] to [Table 20A] (hereinafter, referred to as Compound Class SX524).

A compound (L-1) wherein n is 0, $R^1$ represents an ethyl group, Q represents a group represented by Q1-10, and Het represents any substituents indicated in [Table 1A] to [Table 20A] (hereinafter, referred to as Compound Class SX525).

A compound (L-1) wherein n is 0, $R^1$ represents an ethyl group, Q represents a group represented by Q1-11, and Het represents any substituents indicated in [Table 1A] to [Table 20A] (hereinafter, referred to as Compound Class SX526).

A compound (L-1) wherein n is 0, $R^1$ represents an ethyl group, Q represents a group represented by Q1-12, and Het represents any substituents indicated in [Table 1A] to [Table 20A] (hereinafter, referred to as Compound Class SX527).

A compound (L-1) wherein n is 0, $R^1$ represents an ethyl group, Q represents a group represented by Q1-13, and Het represents any substituents indicated in [Table 1A] to [Table 20A] (hereinafter, referred to as Compound Class SX528).

A compound (L-1) wherein n is 0, $R^1$ represents an ethyl group, Q represents a group represented by Q1-14, and Het represents any substituents indicated in [Table 1A] to [Table 20A] (hereinafter, referred to as Compound Class SX529).

A compound (L-1) wherein n is 0, $R^1$ represents an ethyl group, Q represents a group represented by Q1-15, and

Het represents any substituents indicated in [Table 1A] to [Table 20A] (hereinafter, referred to as Compound Class SX530).

A compound (L-1) wherein n is 0, $R^1$ represents an ethyl group, Q represents a group represented by Q1-16, and Het represents any substituents indicated in [Table 1A] to [Table 20A] (hereinafter, referred to as Compound Class SX531).

A compound (L-1) wherein n is 0, $R^1$ represents an ethyl group, Q represents a group represented by Q1-17, and Het represents any substituents indicated in [Table 1A] to [Table 20A] (hereinafter, referred to as Compound Class SX532).

A compound (L-1) wherein n is 0, $R^1$ represents an ethyl group, Q represents a group represented by Q1-18, and Het represents any substituents indicated in [Table 1A] to [Table 20A] (hereinafter, referred to as Compound Class SX533).

A compound (L-1) wherein n is 0, $R^1$ represents an ethyl group, Q represents a group represented by Q1-19, and Het represents any substituents indicated in [Table 1A] to [Table 20A] (hereinafter, referred to as Compound Class SX534).

A compound (L-1) wherein n is 0, $R^1$ represents an ethyl group, Q represents a group represented by Q1-20, and Het represents any substituents indicated in [Table 1A] to [Table 20A] (hereinafter, referred to as Compound Class SX535).

A compound (L-1) wherein n is 0, $R^1$ represents an ethyl group, Q represents a group represented by Q1-21, and Het represents any substituents indicated in [Table 1A] to [Table 20A] (hereinafter, referred to as Compound Class SX536).

A compound (L-1) wherein n is 0, $R^1$ represents an ethyl group, Q represents a group represented by Q1-22, and Het represents any substituents indicated in [Table 1A] to [Table 20A] (hereinafter, referred to as Compound Class SX537).

A compound (L-1) wherein n is 0, $R^1$ represents an ethyl group, Q represents a group represented by Q1-23, and Het represents any substituents indicated in [Table 1A] to [Table 20A] (hereinafter, referred to as Compound Class SX538).

A compound (L-1) wherein n is 0, $R^1$ represents an ethyl group, Q represents a group represented by Q1-24, and Het represents any substituents indicated in [Table 1A] to [Table 20A] (hereinafter, referred to as Compound Class SX539).

A compound (L-1) wherein n is 0, $R^1$ represents an ethyl group, Q represents a group represented by Q1-25, and Het represents any substituents indicated in [Table 1A] to [Table 20A] (hereinafter, referred to as Compound Class SX540).

A compound (L-1) wherein n is 0, $R^1$ represents an ethyl group, Q represents a group represented by Q1-26, and Het represents any substituents indicated in [Table 1A] to [Table 20A] (hereinafter, referred to as Compound Class SX541).

A compound (L-1) wherein n is 0, $R^1$ represents an ethyl group, Q represents a group represented by Q1-27, and Het represents any substituents indicated in [Table 1A] to [Table 20A] (hereinafter, referred to as Compound Class SX542).

A compound (L-1) wherein n is 0, $R^1$ represents an ethyl group, Q represents a group represented by Q1-28, and Het represents any substituents indicated in [Table 1A] to [Table 20A] (hereinafter, referred to as Compound Class SX543).

A compound (L-1) wherein n is 0, $R^1$ represents an ethyl group, Q represents a group represented by Q1-29, and Het represents any substituents indicated in [Table 1A] to [Table 20A] (hereinafter, referred to as Compound Class SX544).

A compound (L-1) wherein n is 0, $R^1$ represents an ethyl group, Q represents a group represented by Q1-30, and Het represents any substituents indicated in [Table 1A] to [Table 20A] (hereinafter, referred to as Compound Class SX545).

A compound (L-1) wherein n is 0, $R^1$ represents an ethyl group, Q represents a group represented by Q1-31, and Het represents any substituents indicated in [Table 1A] to [Table 20A] (hereinafter, referred to as Compound Class SX546).

A compound (L-1) wherein n is 0, $R^1$ represents an ethyl group, Q represents a group represented by Q1-32, and Het represents any substituents indicated in [Table 1A] to [Table 20A] (hereinafter, referred to as Compound Class SX547).

A compound (L-1) wherein n is 0, $R^1$ represents an ethyl group, Q represents a group represented by Q1-33, and Het represents any substituents indicated in [Table 1A] to [Table 20A] (hereinafter, referred to as Compound Class SX548).

A compound (L-1) wherein n is 0, $R^1$ represents an ethyl group, Q represents a group represented by Q1-34, and Het represents any substituents indicated in [Table 1A] to [Table 20A] (hereinafter, referred to as Compound Class SX549).

A compound (L-1) wherein n is 0, $R^1$ represents an ethyl group, Q represents a group represented by Q1-35, and Het represents any substituents indicated in [Table 1A] to [Table 20A] (hereinafter, referred to as Compound Class SX550).

A compound (L-1) wherein n is 0, $R^1$ represents an ethyl group, Q represents a group represented by Q1-36, and Het represents any substituents indicated in [Table 1A] to [Table 20A] (hereinafter, referred to as Compound Class SX551).

A compound (L-1) wherein n is 0, $R^1$ represents an ethyl group, Q represents a group represented by Q1-37, and Het represents any substituents indicated in [Table 1A] to [Table 20A] (hereinafter, referred to as Compound Class SX552).

A compound (L-1) wherein n is 0, $R^1$ represents an ethyl group, Q represents a group represented by Q1-38, and Het represents any substituents indicated in [Table 1A] to [Table 20A] (hereinafter, referred to as Compound Class SX553).

A compound (L-1) wherein n is 0, $R^1$ represents an ethyl group, Q represents a group represented by Q1-39, and Het represents any substituents indicated in [Table 1A] to [Table 20A] (hereinafter, referred to as Compound Class SX554).

A compound (L-1) wherein n is 0, $R^1$ represents an ethyl group, Q represents a group represented by Q1-40, and Het represents any substituents indicated in [Table 1A] to [Table 20A] (hereinafter, referred to as Compound Class SX555).

A compound (L-1) wherein n is 0, $R^1$ represents an ethyl group, Q represents a group represented by Q1-41, and Het represents any substituents indicated in [Table 1A] to [Table 20A] (hereinafter, referred to as Compound Class SX556).

A compound (L-1) wherein n is 0, $R^1$ represents an ethyl group, Q represents a group represented by Q1-42, and Het represents any substituents indicated in [Table 1A] to [Table 20A] (hereinafter, referred to as Compound Class SX557).

A compound (L-1) wherein n is 0, $R^1$ represents an ethyl group, Q represents a group represented by Q1-43, and Het represents any substituents indicated in [Table 1A] to [Table 20A] (hereinafter, referred to as Compound Class SX558).

A compound (L-1) wherein n is 0, $R^1$ represents an ethyl group, Q represents a group represented by Q1-44, and

Het represents any substituents indicated in [Table 1A] to [Table 20A] (hereinafter, referred to as Compound Class SX559).

A compound (L-1) wherein n is 0, $R^1$ represents an ethyl group, Q represents a group represented by Q1-45, and Het represents any substituents indicated in [Table 1A] to [Table 20A] (hereinafter, referred to as Compound Class SX560).

A compound (L-1) wherein n is 0, $R^1$ represents an ethyl group, Q represents a group represented by Q1-46, and Het represents any substituents indicated in [Table 1A] to [Table 20A] (hereinafter, referred to as Compound Class SX561).

A compound (L-1) wherein n is 0, $R^1$ represents an ethyl group, Q represents a group represented by Q1-47, and Het represents any substituents indicated in [Table 1A] to [Table 20A] (hereinafter, referred to as Compound Class SX562).

A compound (L-1) wherein n is 0, $R^1$ represents an ethyl group, Q represents a group represented by Q1-48, and Het represents any substituents indicated in [Table 1A] to [Table 20A] (hereinafter, referred to as Compound Class SX563).

A compound (L-1) wherein n is 0, $R^1$ represents an ethyl group, Q represents a group represented by Q1-49, and Het represents any substituents indicated in [Table 1A] to [Table 20A] (hereinafter, referred to as Compound Class SX564).

A compound (L-1) wherein n is 0, $R^1$ represents an ethyl group, Q represents a group represented by Q1-50, and Het represents any substituents indicated in [Table 1A] to [Table 20A] (hereinafter, referred to as Compound Class SX565).

A compound (L-1) wherein n is 0, $R^1$ represents an ethyl group, Q represents a group represented by Q1-51, and Het represents any substituents indicated in [Table 1A] to [Table 20A] (hereinafter, referred to as Compound Class SX566).

A compound (L-1) wherein n is 0, $R^1$ represents an ethyl group, Q represents a group represented by Q1-52, and Het represents any substituents indicated in [Table 1A] to [Table 20A] (hereinafter, referred to as Compound Class SX567).

A compound (L-1) wherein n is 0, $R^1$ represents an ethyl group, Q represents a group represented by Q1-53, and Het represents any substituents indicated in [Table 1A] to [Table 20A] (hereinafter, referred to as Compound Class SX568).

A compound (L-1) wherein n is 0, $R^1$ represents an ethyl group, Q represents a group represented by Q1-54, and Het represents any substituents indicated in [Table 1A] to [Table 20A] (hereinafter, referred to as Compound Class SX569).

A compound (L-1) wherein n is 0, $R^1$ represents an ethyl group, Q represents a group represented by Q1-55, and Het represents any substituents indicated in [Table 1A] to [Table 20A] (hereinafter, referred to as Compound Class SX570).

A compound (L-1) wherein n is 0, $R^1$ represents an ethyl group, Q represents a group represented by Q1-56, and Het represents any substituents indicated in [Table 1A] to [Table 20A] (hereinafter, referred to as Compound Class SX571).

A compound (L-1) wherein n is 0, $R^1$ represents an ethyl group, Q represents a group represented by Q1-57, and Het represents any substituents indicated in [Table 1A] to [Table 20A] (hereinafter, referred to as Compound Class SX572).

A compound (L-1) wherein n is 0, $R^1$ represents an ethyl group, Q represents a group represented by Q1-58, and Het represents any substituents indicated in [Table 1A] to [Table 20A] (hereinafter, referred to as Compound Class SX573).

A compound (L-1) wherein n is 0, $R^1$ represents an ethyl group, Q represents a group represented by Q1-59, and Het represents any substituents indicated in [Table 1A] to [Table 20A] (hereinafter, referred to as Compound Class SX574).

A compound (L-1) wherein n is 0, $R^1$ represents an ethyl group, Q represents a group represented by Q1-60, and Het represents any substituents indicated in [Table 1A] to [Table 20A] (hereinafter, referred to as Compound Class SX575).

A compound (L-1) wherein n is 0, $R^1$ represents an ethyl group, Q represents a group represented by Q1-61, and Het represents any substituents indicated in [Table 1A] to [Table 20A] (hereinafter, referred to as Compound Class SX576).

A compound (L-1) wherein n is 0, $R^1$ represents an ethyl group, Q represents a group represented by Q1-62, and Het represents any substituents indicated in [Table 1A] to [Table 20A] (hereinafter, referred to as Compound Class SX577).

A compound (L-1) wherein n is 0, $R^1$ represents an ethyl group, Q represents a group represented by Q1-63, and Het represents any substituents indicated in [Table 1A] to [Table 20A] (hereinafter, referred to as Compound Class SX578).

A compound (L-1) wherein n is 0, $R^1$ represents an ethyl group, Q represents a group represented by Q1-64, and Het represents any substituents indicated in [Table 1A] to [Table 20A] (hereinafter, referred to as Compound Class SX579).

A compound (L-1) wherein n is 0, $R^1$ represents an ethyl group, Q represents a group represented by Q1-65, and Het represents any substituents indicated in [Table 1A] to [Table 20A] (hereinafter, referred to as Compound Class SX580).

A compound (L-1) wherein n is 0, $R^1$ represents an ethyl group, Q represents a group represented by Q1-66, and Het represents any substituents indicated in [Table 1A] to [Table 20A] (hereinafter, referred to as Compound Class SX581).

A compound (L-1) wherein n is 0, $R^1$ represents an ethyl group, Q represents a group represented by Q1-67, and Het represents any substituents indicated in [Table 1A] to [Table 20A] (hereinafter, referred to as Compound Class SX582).

A compound (L-1) wherein n is 0, $R^1$ represents an ethyl group, Q represents a group represented by Q1-68, and Het represents any substituents indicated in [Table 1A] to [Table 20A] (hereinafter, referred to as Compound Class SX583).

A compound (L-1) wherein n is 0, $R^1$ represents an ethyl group, Q represents a group represented by Q1-69, and Het represents any substituents indicated in [Table 1A] to [Table 20A] (hereinafter, referred to as Compound Class SX584).

A compound (L-1) wherein n is 0, $R^1$ represents an ethyl group, Q represents a group represented by Q1-70, and Het represents any substituents indicated in [Table 1A] to [Table 20A] (hereinafter, referred to as Compound Class SX585).

A compound (L-1) wherein n is 0, $R^1$ represents an ethyl group, Q represents a group represented by Q1-71, and Het represents any substituents indicated in [Table 1A] to [Table 20A] (hereinafter, referred to as Compound Class SX586).

A compound (L-1) wherein n is 0, $R^1$ represents an ethyl group, Q represents a group represented by Q1-72, and Het represents any substituents indicated in [Table 1A] to [Table 20A] (hereinafter, referred to as Compound Class SX587).

A compound (L-1) wherein n is 0, $R^1$ represents an ethyl group, Q represents a group represented by Q1-73, and

Het represents any substituents indicated in [Table 1A] to [Table 20A] (hereinafter, referred to as Compound Class SX588).

A compound (L-1) wherein n is 0, $R^1$ represents an ethyl group, Q represents a group represented by Q1-74, and Het represents any substituents indicated in [Table 1A] to [Table 20A] (hereinafter, referred to as Compound Class SX589).

A compound (L-1) wherein n is 0, $R^1$ represents an ethyl group, Q represents a group represented by Q1-75, and Het represents any substituents indicated in [Table 1A] to [Table 20A] (hereinafter, referred to as Compound Class SX590).

A compound (L-1) wherein n is 0, $R^1$ represents an ethyl group, Q represents a group represented by Q1-76, and Het represents any substituents indicated in [Table 1A] to [Table 20A] (hereinafter, referred to as Compound Class SX591).

A compound (L-1) wherein n is 0, $R^1$ represents an ethyl group, Q represents a group represented by Q1-77, and Het represents any substituents indicated in [Table 1A] to [Table 20A] (hereinafter, referred to as Compound Class SX592).

A compound (L-1) wherein n is 0, $R^1$ represents an ethyl group, Q represents a group represented by Q1-77, and Het represents any substituents indicated in [Table 1A] to [Table 20A] (hereinafter, referred to as Compound Class SX592).

A compound (L-1) wherein n is 0, $R^1$ represents an ethyl group, Q represents a group represented by Q1-78, and Het represents any substituents indicated in [Table 1A] to [Table 20A] (hereinafter, referred to as Compound Class SX593).

A compound (L-1) wherein n is 0, $R^1$ represents an ethyl group, Q represents a group represented by Q1-79, and Het represents any substituents indicated in [Table 1A] to [Table 20A] (hereinafter, referred to as Compound Class SX594).

A compound (L-1) wherein n is 0, $R^1$ represents an ethyl group, Q represents a group represented by Q1-80, and Het represents any substituents indicated in [Table 1A] to [Table 20A] (hereinafter, referred to as Compound Class SX595).

A compound (L-1) wherein n is 0, $R^1$ represents an ethyl group, Q represents a group represented by Q1-81, and Het represents any substituents indicated in [Table 1A] to [Table 20A] (hereinafter, referred to as Compound Class SX596).

A compound (L-1) wherein n is 0, $R^1$ represents an ethyl group, Q represents a group represented by Q1-82, and Het represents any substituents indicated in [Table 1A] to [Table 20A] (hereinafter, referred to as Compound Class SX597).

A compound (L-1) wherein n is 0, $R^1$ represents an ethyl group, Q represents a group represented by Q1-83, and Het represents any substituents indicated in [Table 1A] to [Table 20A] (hereinafter, referred to as Compound Class SX598).

A compound (L-1) wherein n is 0, $R^1$ represents an ethyl group, Q represents a group represented by Q1-84, and Het represents any substituents indicated in [Table 1A] to [Table 20A] (hereinafter, referred to as Compound Class SX599).

A compound (L-1) wherein n is 0, $R^1$ represents an ethyl group, Q represents a group represented by Q1-85, and Het represents any substituents indicated in [Table 1A] to [Table 20A] (hereinafter, referred to as Compound Class SX600).

A compound (L-1) wherein n is 0, $R^1$ represents an ethyl group, Q represents a group represented by Q1-86, and Het represents any substituents indicated in [Table 1A] to [Table 20A] (hereinafter, referred to as Compound Class SX601).

A compound (L-1) wherein n is 0, $R^1$ represents an ethyl group, Q represents a group represented by Q1-87, and Het represents any substituents indicated in [Table 1A] to [Table 20A] (hereinafter, referred to as Compound Class SX602).

A compound (L-1) wherein n is 0, $R^1$ represents an ethyl group, Q represents a group represented by Q1-88, and Het represents any substituents indicated in [Table 1A] to [Table 20A] (hereinafter, referred to as Compound Class SX603).

A compound (L-1) wherein n is 0, $R^1$ represents an ethyl group, Q represents a group represented by Q1-89, and Het represents any substituents indicated in [Table 1A] to [Table 20A] (hereinafter, referred to as Compound Class SX604).

A compound (L-1) wherein n is 0, $R^1$ represents an ethyl group, Q represents a group represented by Q1-90, and Het represents any substituents indicated in [Table 1A] to [Table 20A] (hereinafter, referred to as Compound Class SX605).

A compound (L-1) wherein n is 0, $R^1$ represents an ethyl group, Q represents a group represented by Q1-91, and Het represents any substituents indicated in [Table 1A] to [Table 20A] (hereinafter, referred to as Compound Class SX606).

A compound (L-1) wherein n is 0, $R^1$ represents an ethyl group, Q represents a group represented by Q1-92, and Het represents any substituents indicated in [Table 1A] to [Table 20A] (hereinafter, referred to as Compound Class SX607).

A compound (L-1) wherein n is 0, $R^1$ represents an ethyl group, Q represents a group represented by Q1-93, and Het represents any substituents indicated in [Table 1A] to [Table 20A] (hereinafter, referred to as Compound Class SX608).

A compound (L-1) wherein n is 0, $R^1$ represents an ethyl group, Q represents a group represented by Q1-94, and Het represents any substituents indicated in [Table 1A] to [Table 20A] (hereinafter, referred to as Compound Class SX609).

A compound (L-1) wherein n is 0, $R^1$ represents an ethyl group, Q represents a group represented by Q1-95, and Het represents any substituents indicated in [Table 1A] to [Table 20A] (hereinafter, referred to as Compound Class SX610).

A compound (L-1) wherein n is 0, $R^1$ represents an ethyl group, Q represents a group represented by Q1-96, and Het represents any substituents indicated in [Table 1A] to [Table 20A] (hereinafter, referred to as Compound Class SX611).

A compound (L-1) wherein n is 0, $R^1$ represents an ethyl group, Q represents a group represented by Q1-97, and Het represents any substituents indicated in [Table 1A] to [Table 20A] (hereinafter, referred to as Compound Class SX612).

A compound (L-1) wherein n is 0, $R^1$ represents an ethyl group, Q represents a group represented by Q1-98, and Het represents any substituents indicated in [Table 1A] to [Table 20A] (hereinafter, referred to as Compound Class SX613).

A compound (L-1) wherein n is 0, $R^1$ represents an ethyl group, Q represents a group represented by Q1-99, and Het represents any substituents indicated in [Table 1A] to [Table 20A] (hereinafter, referred to as Compound Class SX614).

A compound (L-1) wherein n is 0, $R^1$ represents an ethyl group, Q represents a group represented by Q1-100, and Het represents any substituents indicated in [Table 1A] to [Table 20A] (hereinafter, referred to as Compound Class SX615).

A compound (L-1) wherein n is 0, $R^1$ represents an ethyl group, Q represents a group represented by Q1-101, and

Het represents any substituents indicated in [Table 1A] to [Table 20A] (hereinafter, referred to as Compound Class SX616).

A compound (L-1) wherein n is 0, $R^1$ represents an ethyl group, Q represents a group represented by Q1-102, and Het represents any substituents indicated in [Table 1A] to [Table 20A] (hereinafter, referred to as Compound Class SX617).

A compound (L-1) wherein n is 0, $R^1$ represents an ethyl group, Q represents a group represented by Q1-103, and Het represents any substituents indicated in [Table 1A] to [Table 20A] (hereinafter, referred to as Compound Class SX618).

A compound (L-1) wherein n is 0, $R^1$ represents an ethyl group, Q represents a group represented by Q1-104, and Het represents any substituents indicated in [Table 1A] to [Table 20A] (hereinafter, referred to as Compound Class SX619).

A compound (L-1) wherein n is 0, $R^1$ represents an ethyl group, Q represents a group represented by Q1-105, and Het represents any substituents indicated in [Table 1A] to [Table 20A] (hereinafter, referred to as Compound Class SX620).

A compound (L-1) wherein n is 0, $R^1$ represents an ethyl group, Q represents a group represented by Q1-106, and Het represents any substituents indicated in [Table 1A] to [Table 20A] (hereinafter, referred to as Compound Class SX621).

A compound (L-1) wherein n is 0, $R^1$ represents an ethyl group, Q represents a group represented by Q1-107, and Het represents any substituents indicated in [Table 1A] to [Table 20A] (hereinafter, referred to as Compound Class SX622).

A compound (L-1) wherein n is 0, $R^1$ represents an ethyl group, Q represents a group represented by Q1-108, and Het represents any substituents indicated in [Table 1A] to [Table 20A] (hereinafter, referred to as Compound Class SX623).

A compound (L-1) wherein n is 0, $R^1$ represents an ethyl group, Q represents a group represented by Q1-109, and Het represents any substituents indicated in [Table 1A] to [Table 20A] (hereinafter, referred to as Compound Class SX624).

A compound (L-1) wherein n is 0, $R^1$ represents an ethyl group, Q represents a group represented by Q1-110, and Het represents any substituents indicated in [Table 1A] to [Table 20A] (hereinafter, referred to as Compound Class SX625).

A compound (L-1) wherein n is 0, $R^1$ represents an ethyl group, Q represents a group represented by Q1-111, and Het represents any substituents indicated in [Table 1A] to [Table 20A] (hereinafter, referred to as Compound Class SX626).

A compound (L-1) wherein n is 0, $R^1$ represents an ethyl group, Q represents a group represented by Q1-112, and Het represents any substituents indicated in [Table 1A] to [Table 20A] (hereinafter, referred to as Compound Class SX627).

A compound (L-1) wherein n is 0, $R^1$ represents an ethyl group, Q represents a group represented by Q1-113, and Het represents any substituents indicated in [Table 1A] to [Table 20A] (hereinafter, referred to as Compound Class SX628).

A compound (L-1) wherein n is 0, $R^1$ represents an ethyl group, Q represents a group represented by Q1-114, and Het represents any substituents indicated in [Table 1A] to [Table 20A] (hereinafter, referred to as Compound Class SX629).

A compound (L-1) wherein n is 0, $R^1$ represents an ethyl group, Q represents a group represented by Q1-115, and Het represents any substituents indicated in [Table 1A] to [Table 20A] (hereinafter, referred to as Compound Class SX630).

A compound (L-1) wherein n is 0, $R^1$ represents an ethyl group, Q represents a group represented by Q1-116, and Het represents any substituents indicated in [Table 1A] to [Table 20A] (hereinafter, referred to as Compound Class SX631).

A compound (L-1) wherein n is 0, $R^1$ represents an ethyl group, Q represents a group represented by Q1-117, and Het represents any substituents indicated in [Table 1A] to [Table 20A] (hereinafter, referred to as Compound Class SX632).

A compound (L-1) wherein n is 0, $R^1$ represents an ethyl group, Q represents a group represented by Q1-118, and Het represents any substituents indicated in [Table 1A] to [Table 20A] (hereinafter, referred to as Compound Class SX633).

A compound (L-1) wherein n is 0, $R^1$ represents an ethyl group, Q represents a group represented by Q1-119, and Het represents any substituents indicated in [Table 1A] to [Table 20A] (hereinafter, referred to as Compound Class SX634).

A compound (L-1) wherein n is 0, $R^1$ represents an ethyl group, Q represents a group represented by Q1-120, and Het represents any substituents indicated in [Table 1A] to [Table 20A] (hereinafter, referred to as Compound Class SX635).

A compound (L-1) wherein n is 0, $R^1$ represents an ethyl group, Q represents a group represented by Q1-121, and Het represents any substituents indicated in [Table 1A] to [Table 20A] (hereinafter, referred to as Compound Class SX636).

A compound (L-1) wherein n is 0, $R^1$ represents an ethyl group, Q represents a group represented by Q1-122, and Het represents any substituents indicated in [Table 1A] to [Table 20A] (hereinafter, referred to as Compound Class SX637).

A compound (L-1) wherein n is 0, $R^1$ represents an ethyl group, Q represents a group represented by Q1-123, and Het represents any substituents indicated in [Table 1A] to [Table 20A] (hereinafter, referred to as Compound Class SX638).

A compound (L-1) wherein n is 0, $R^1$ represents an ethyl group, Q represents a group represented by Q1-124, and Het represents any substituents indicated in [Table 1A] to [Table 20A] (hereinafter, referred to as Compound Class SX639).

A compound (L-1) wherein n is 0, $R^1$ represents an ethyl group, Q represents a group represented by Q1-125, and Het represents any substituents indicated in [Table 1A] to [Table 20A] (hereinafter, referred to as Compound Class SX640).

A compound (L-1) wherein n is 0, $R^1$ represents an ethyl group, Q represents a group represented by Q1-126, and Het represents any substituents indicated in [Table 1A] to [Table 20A] (hereinafter, referred to as Compound Class SX641).

A compound (L-1) wherein n is 0, $R^1$ represents an ethyl group, Q represents a group represented by Q1-127, and Het represents any substituents indicated in [Table 1A] to [Table 20A] (hereinafter, referred to as Compound Class SX642).

A compound (L-1) wherein n is 0, $R^1$ represents an ethyl group, Q represents a group represented by Q1-128, and Het represents any substituents indicated in [Table 1A] to [Table 20A] (hereinafter, referred to as Compound Class SX643).

A compound (L-1) wherein n is 0, $R^1$ represents an ethyl group, Q represents a group represented by Q1-129, and Het represents any substituents indicated in [Table 1A] to [Table 20A] (hereinafter, referred to as Compound Class SX644).

A compound (L-1) wherein n is 0, $R^1$ represents an ethyl group, Q represents a group represented by Q1-130, and

Het represents any substituents indicated in [Table 1A] to [Table 20A] (hereinafter, referred to as Compound Class SX645).

A compound (L-1) wherein n is 0, $R^1$ represents an ethyl group, Q represents a group represented by Q1-131, and Het represents any substituents indicated in [Table 1A] to [Table 20A] (hereinafter, referred to as Compound Class SX646).

A compound (L-1) wherein n is 0, $R^1$ represents an ethyl group, Q represents a group represented by Q1-132, and Het represents any substituents indicated in [Table 1A] to [Table 20A] (hereinafter, referred to as Compound Class SX647).

A compound (L-1) wherein n is 0, $R^1$ represents an ethyl group, Q represents a group represented by Q1-133, and Het represents any substituents indicated in [Table 1A] to [Table 20A] (hereinafter, referred to as Compound Class SX648).

A compound (L-1) wherein n is 0, $R^1$ represents an ethyl group, Q represents a group represented by Q1-134, and Het represents any substituents indicated in [Table 1A] to [Table 20A] (hereinafter, referred to as Compound Class SX649).

A compound (L-1) wherein n is 0, $R^1$ represents an ethyl group, Q represents a group represented by Q1-135, and Het represents any substituents indicated in [Table 1A] to [Table 20A] (hereinafter, referred to as Compound Class SX650).

A compound (L-1) wherein n is 0, $R^1$ represents an ethyl group, Q represents a group represented by Q1-136, and Het represents any substituents indicated in [Table 1A] to [Table 20A] (hereinafter, referred to as Compound Class SX651).

A compound (L-1) wherein n is 0, $R^1$ represents an ethyl group, Q represents a group represented by Q1-137, and Het represents any substituents indicated in [Table 1A] to [Table 20A] (hereinafter, referred to as Compound Class SX652).

A compound (L-1) wherein n is 0, $R^1$ represents an ethyl group, Q represents a group represented by Q1-138, and Het represents any substituents indicated in [Table 1A] to [Table 20A] (hereinafter, referred to as Compound Class SX653).

A compound (L-1) wherein n is 0, $R^1$ represents an ethyl group, Q represents a group represented by Q1-139, and Het represents any substituents indicated in [Table 1A] to [Table 20A] (hereinafter, referred to as Compound Class SX654).

A compound (L-1) wherein n is 0, $R^1$ represents an ethyl group, Q represents a group represented by Q1-140, and Het represents any substituents indicated in [Table 1A] to [Table 20A] (hereinafter, referred to as Compound Class SX655).

A compound (L-1) wherein n is 0, $R^1$ represents an ethyl group, Q represents a group represented by Q1-141, and Het represents any substituents indicated in [Table 1A] to [Table 20A] (hereinafter, referred to as Compound Class SX656).

A compound (L-1) wherein n is 0, $R^1$ represents an ethyl group, Q represents a group represented by Q1-142, and Het represents any substituents indicated in [Table 1A] to [Table 20A] (hereinafter, referred to as Compound Class SX657).

A compound (L-1) wherein n is 0, $R^1$ represents an ethyl group, Q represents a group represented by Q1-143, and Het represents any substituents indicated in [Table 1A] to [Table 20A] (hereinafter, referred to as Compound Class SX658).

A compound (L-1) wherein n is 0, $R^1$ represents an ethyl group, Q represents a group represented by Q1-144, and Het represents any substituents indicated in [Table 1A] to [Table 20A] (hereinafter, referred to as Compound Class SX659).

A compound (L-1) wherein n is 0, $R^1$ represents an ethyl group, Q represents a group represented by Q1-145, and Het represents any substituents indicated in [Table 1A] to [Table 20A] (hereinafter, referred to as Compound Class SX660).

A compound (L-1) wherein n is 0, $R^1$ represents an ethyl group, Q represents a group represented by Q1-146, and Het represents any substituents indicated in [Table 1A] to [Table 20A] (hereinafter, referred to as Compound Class SX661).

A compound (L-1) wherein n is 0, $R^1$ represents an ethyl group, Q represents a group represented by Q1-147, and Het represents any substituents indicated in [Table 1A] to [Table 20A] (hereinafter, referred to as Compound Class SX662).

A compound (L-1) wherein n is 0, $R^1$ represents an ethyl group, Q represents a group represented by Q1-148, and Het represents any substituents indicated in [Table 1A] to [Table 20A] (hereinafter, referred to as Compound Class SX663).

A compound (L-1) wherein n is 0, $R^1$ represents an ethyl group, Q represents a group represented by Q1-149, and Het represents any substituents indicated in [Table 1A] to [Table 20A] (hereinafter, referred to as Compound Class SX664).

A compound (L-1) wherein n is 0, $R^1$ represents an ethyl group, Q represents a group represented by Q1-150, and Het represents any substituents indicated in [Table 1A] to [Table 20A] (hereinafter, referred to as Compound Class SX665).

A compound (L-1) wherein n is 0, $R^1$ represents an ethyl group, Q represents a group represented by Q1-151, and Het represents any substituents indicated in [Table 1A] to [Table 20A] (hereinafter, referred to as Compound Class SX666).

A compound (L-1) wherein n is 0, $R^1$ represents an ethyl group, Q represents a group represented by Q1-152, and Het represents any substituents indicated in [Table 1A] to [Table 20A] (hereinafter, referred to as Compound Class SX667).

A compound (L-1) wherein n is 0, $R^1$ represents an ethyl group, Q represents a group represented by Q1-153, and Het represents any substituents indicated in [Table 1A] to [Table 20A] (hereinafter, referred to as Compound Class SX668).

A compound (L-1) wherein n is 0, $R^1$ represents an ethyl group, Q represents a group represented by Q1-154, and Het represents any substituents indicated in [Table 1A] to [Table 20A] (hereinafter, referred to as Compound Class SX669).

A compound (L-1) wherein n is 0, $R^1$ represents an ethyl group, Q represents a group represented by Q1-155, and Het represents any substituents indicated in [Table 1A] to [Table 20A] (hereinafter, referred to as Compound Class SX670).

A compound (L-1) wherein n is 0, $R^1$ represents an ethyl group, Q represents a group represented by Q1-156, and Het represents any substituents indicated in [Table 1A] to [Table 20A] (hereinafter, referred to as Compound Class SX671).

A compound (L-1) wherein n is 0, $R^1$ represents an ethyl group, Q represents a group represented by Q1-157, and Het represents any substituents indicated in [Table 1A] to [Table 20A] (hereinafter, referred to as Compound Class SX672).

A compound (L-1) wherein n is 0, $R^1$ represents an ethyl group, Q represents a group represented by Q1-158, and Het represents any substituents indicated in [Table 1A] to [Table 20A] (hereinafter, referred to as Compound Class SX673).

A compound (L-1) wherein n is 0, $R^1$ represents an ethyl group, Q represents a group represented by Q1-159, and

Het represents any substituents indicated in [Table 1A] to [Table 20A] (hereinafter, referred to as Compound Class SX674).

A compound (L-1) wherein n is 0, $R^1$ represents an ethyl group, Q represents a group represented by Q1-160, and Het represents any substituents indicated in [Table 1A] to [Table 20A] (hereinafter, referred to as Compound Class SX675).

A compound (L-1) wherein n is 0, $R^1$ represents an ethyl group, Q represents a group represented by Q1-161, and Het represents any substituents indicated in [Table 1A] to [Table 20A] (hereinafter, referred to as Compound Class SX676).

A compound (L-1) wherein n is 0, $R^1$ represents an ethyl group, Q represents a group represented by Q1-162, and Het represents any substituents indicated in [Table 1A] to [Table 20A] (hereinafter, referred to as Compound Class SX677).

A compound (L-1) wherein n is 0, $R^1$ represents an ethyl group, Q represents a group represented by Q1-163, and Het represents any substituents indicated in [Table 1A] to [Table 20A] (hereinafter, referred to as Compound Class SX678).

A compound (L-1) wherein n is 0, $R^1$ represents an ethyl group, Q represents a group represented by Q1-164, and Het represents any substituents indicated in [Table 1A] to [Table 20A] (hereinafter, referred to as Compound Class SX679).

A compound (L-1) wherein n is 0, $R^1$ represents an ethyl group, Q represents a group represented by Q1-165, and Het represents any substituents indicated in [Table 1A] to [Table 20A] (hereinafter, referred to as Compound Class SX680).

A compound (L-1) wherein n is 0, $R^1$ represents an ethyl group, Q represents a group represented by Q1-166, and Het represents any substituents indicated in [Table 1A] to [Table 20A] (hereinafter, referred to as Compound Class SX681).

A compound (L-1) wherein n is 0, $R^1$ represents an ethyl group, Q represents a group represented by Q1-167, and Het represents any substituents indicated in [Table 1A] to [Table 20A] (hereinafter, referred to as Compound Class SX682).

A compound (L-1) wherein n is 0, $R^1$ represents an ethyl group, Q represents a group represented by Q1-168, and Het represents any substituents indicated in [Table 1A] to [Table 20A] (hereinafter, referred to as Compound Class SX683).

A compound (L-1) wherein n is 0, $R^1$ represents an ethyl group, Q represents a group represented by Q1-169, and Het represents any substituents indicated in [Table 1A] to [Table 20A] (hereinafter, referred to as Compound Class SX684).

A compound (L-1) wherein n is 0, $R^1$ represents an ethyl group, Q represents a group represented by Q1-170, and Het represents any substituents indicated in [Table 1A] to [Table 20A] (hereinafter, referred to as Compound Class SX685).

A compound (L-1) wherein n is 0, $R^1$ represents an ethyl group, Q represents a group represented by Q1-171, and Het represents any substituents indicated in [Table 1A] to [Table 20A] (hereinafter, referred to as Compound Class SX686).

A compound (L-1) wherein n is 0, $R^1$ represents an ethyl group, Q represents a group represented by Q1-172, and Het represents any substituents indicated in [Table 1A] to [Table 20A] (hereinafter, referred to as Compound Class SX687).

A compound (L-1) wherein n is 0, $R^1$ represents an ethyl group, Q represents a group represented by Q1-173, and Het represents any substituents indicated in [Table 1A] to [Table 20A] (hereinafter, referred to as Compound Class SX688).

A compound (L-1) wherein n is 0, $R^1$ represents an ethyl group, Q represents a group represented by Q1-174, and Het represents any substituents indicated in [Table 1A] to [Table 20A] (hereinafter, referred to as Compound Class SX689).

A compound (L-1) wherein n is 0, $R^1$ represents an ethyl group, Q represents a group represented by Q1-175, and Het represents any substituents indicated in [Table 1A] to [Table 20A] (hereinafter, referred to as Compound Class SX690).

A compound (L-1) wherein n is 0, $R^1$ represents an ethyl group, Q represents a group represented by Q1-176, and Het represents any substituents indicated in [Table 1A] to [Table 20A] (hereinafter, referred to as Compound Class SX691).

A compound (L-1) wherein n is 0, $R^1$ represents an ethyl group, Q represents a group represented by Q1-177, and Het represents any substituents indicated in [Table 1A] to [Table 20A] (hereinafter, referred to as Compound Class SX692).

A compound (L-1) wherein n is 0, $R^1$ represents an ethyl group, Q represents a group represented by Q1-178, and Het represents any substituents indicated in [Table 1A] to [Table 20A] (hereinafter, referred to as Compound Class SX693).

A compound (L-1) wherein n is 0, $R^1$ represents an ethyl group, Q represents a group represented by Q1-179, and Het represents any substituents indicated in [Table 1A] to [Table 20A] (hereinafter, referred to as Compound Class SX694).

A compound (L-1) wherein n is 0, $R^1$ represents an ethyl group, Q represents a group represented by Q1-180, and Het represents any substituents indicated in [Table 1A] to [Table 20A] (hereinafter, referred to as Compound Class SX695).

A compound (L-1) wherein n is 0, $R^1$ represents an ethyl group, Q represents a group represented by Q1-181, and Het represents any substituents indicated in [Table 1A] to [Table 20A] (hereinafter, referred to as Compound Class SX696).

A compound (L-1) wherein n is 0, $R^1$ represents an ethyl group, Q represents a group represented by Q1-182, and Het represents any substituents indicated in [Table 1A] to [Table 20A] (hereinafter, referred to as Compound Class SX697).

A compound (L-1) wherein n is 0, $R^1$ represents an ethyl group, Q represents a group represented by Q1-183, and Het represents any substituents indicated in [Table 1A] to [Table 20A] (hereinafter, referred to as Compound Class SX698).

A compound (L-1) wherein n is 0, $R^1$ represents an ethyl group, Q represents a group represented by Q1-184, and Het represents any substituents indicated in [Table 1A] to [Table 20A] (hereinafter, referred to as Compound Class SX699).

A compound (L-1) wherein n is 0, $R^1$ represents an ethyl group, Q represents a group represented by Q1-185, and Het represents any substituents indicated in [Table 1A] to [Table 20A] (hereinafter, referred to as Compound Class SX700).

A compound (L-1) wherein n is 0, $R^1$ represents an ethyl group, Q represents a group represented by Q1-186, and Het represents any substituents indicated in [Table 1A] to [Table 20A] (hereinafter, referred to as Compound Class SX701).

A compound (L-1) wherein n is 0, $R^1$ represents an ethyl group, Q represents a group represented by Q1-187, and Het represents any substituents indicated in [Table 1A] to [Table 20A] (hereinafter, referred to as Compound Class SX702).

A compound (L-1) wherein n is 0, $R^1$ represents an ethyl group, Q represents a group represented by Q1-188, and

Het represents any substituents indicated in [Table 1A] to [Table 20A] (hereinafter, referred to as Compound Class SX703).

A compound (L-1) wherein n is 0, $R^1$ represents an ethyl group, Q represents a group represented by Q1-189, and Het represents any substituents indicated in [Table 1A] to [Table 20A] (hereinafter, referred to as Compound Class SX704).

A compound (L-1) wherein n is 0, $R^1$ represents an ethyl group, Q represents a group represented by Q1-190, and Het represents any substituents indicated in [Table 1A] to [Table 20A] (hereinafter, referred to as Compound Class SX705).

A compound (L-1) wherein n is 0, $R^1$ represents an ethyl group, Q represents a group represented by Q1-191, and Het represents any substituents indicated in [Table 1A] to [Table 20A] (hereinafter, referred to as Compound Class SX706).

A compound (L-1) wherein n is 0, $R^1$ represents an ethyl group, Q represents a group represented by Q1-192, and Het represents any substituents indicated in [Table 1A] to [Table 20A] (hereinafter, referred to as Compound Class SX707).

A compound (L-1) wherein n is 0, $R^1$ represents an ethyl group, Q represents a group represented by Q1-193, and Het represents any substituents indicated in [Table 1A] to [Table 20A] (hereinafter, referred to as Compound Class SX708).

A compound (L-1) wherein n is 0, $R^1$ represents an ethyl group, Q represents a group represented by Q1-194, and Het represents any substituents indicated in [Table 1A] to [Table 20A] (hereinafter, referred to as Compound Class SX709).

A compound (L-1) wherein n is 0, $R^1$ represents an ethyl group, Q represents a group represented by Q1-195, and Het represents any substituents indicated in [Table 1A] to [Table 20A] (hereinafter, referred to as Compound Class SX710).

A compound (L-1) wherein n is 0, $R^1$ represents an ethyl group, Q represents a group represented by Q1-196, and Het represents any substituents indicated in [Table 1A] to [Table 20A] (hereinafter, referred to as Compound Class SX711).

A compound (L-1) wherein n is 0, $R^1$ represents an ethyl group, Q represents a group represented by Q1-197, and Het represents any substituents indicated in [Table 1A] to [Table 20A] (hereinafter, referred to as Compound Class SX712).

A compound (L-1) wherein n is 0, $R^1$ represents an ethyl group, Q represents a group represented by Q1-198, and Het represents any substituents indicated in [Table 1A] to [Table 20A] (hereinafter, referred to as Compound Class SX713).

A compound (L-1) wherein n is 0, $R^1$ represents an ethyl group, Q represents a group represented by Q1-199, and Het represents any substituents indicated in [Table 1A] to [Table 20A] (hereinafter, referred to as Compound Class SX714).

A compound (L-1) wherein n is 0, $R^1$ represents an ethyl group, Q represents a group represented by Q1-200, and Het represents any substituents indicated in [Table 1A] to [Table 20A] (hereinafter, referred to as Compound Class SX715).

A compound (L-1) wherein n is 0, $R^1$ represents an ethyl group, Q represents a group represented by Q1-201, and Het represents any substituents indicated in [Table 1A] to [Table 20A] (hereinafter, referred to as Compound Class SX716).

A compound (L-1) wherein n is 0, $R^1$ represents an ethyl group, Q represents a group represented by Q1-202, and Het represents any substituents indicated in [Table 1A] to [Table 20A] (hereinafter, referred to as Compound Class SX717).

A compound (L-1) wherein n is 0, $R^1$ represents an ethyl group, Q represents a group represented by Q1-203, and Het represents any substituents indicated in [Table 1A] to [Table 20A] (hereinafter, referred to as Compound Class SX718).

A compound (L-1) wherein n is 0, $R^1$ represents an ethyl group, Q represents a group represented by Q1-204, and Het represents any substituents indicated in [Table 1A] to [Table 20A] (hereinafter, referred to as Compound Class SX719).

A compound (L-1) wherein n is 0, $R^1$ represents an ethyl group, Q represents a group represented by Q1-205, and Het represents any substituents indicated in [Table 1A] to [Table 20A] (hereinafter, referred to as Compound Class SX720).

A compound (L-1) wherein n is 0, $R^1$ represents an ethyl group, Q represents a group represented by Q1-206, and Het represents any substituents indicated in [Table 1A] to [Table 20A] (hereinafter, referred to as Compound Class SX721).

A compound (L-1) wherein n is 0, $R^1$ represents an ethyl group, Q represents a group represented by Q1-207, and Het represents any substituents indicated in [Table 1A] to [Table 20A] (hereinafter, referred to as Compound Class SX722).

A compound (L-1) wherein n is 0, $R^1$ represents an ethyl group, Q represents a group represented by Q1-208, and Het represents any substituents indicated in [Table 1A] to [Table 20A] (hereinafter, referred to as Compound Class SX723).

A compound (L-1) wherein n is 0, $R^1$ represents an ethyl group, Q represents a group represented by Q1-209, and Het represents any substituents indicated in [Table 1A] to [Table 20A] (hereinafter, referred to as Compound Class SX724).

A compound (L-1) wherein n is 0, $R^1$ represents an ethyl group, Q represents a group represented by Q5-1, and Het represents any substituents indicated in [Table 1A] to [Table 20A] (hereinafter, referred to as Compound Class SX725).

A compound (L-1) wherein n is 0, $R^1$ represents an ethyl group, Q represents a group represented by Q5-2, and Het represents any substituents indicated in [Table 1A] to [Table 20A] (hereinafter, referred to as Compound Class SX726).

A compound (L-1) wherein n is 0, $R^1$ represents an ethyl group, Q represents a group represented by Q5-3, and Het represents any substituents indicated in [Table 1A] to [Table 20A] (hereinafter, referred to as Compound Class SX727).

A compound (L-1) wherein n is 0, $R^1$ represents an ethyl group, Q represents a group represented by Q5-4, and Het represents any substituents indicated in [Table 1A] to [Table 20A] (hereinafter, referred to as Compound Class SX728).

A compound (L-1) wherein n is 0, $R^1$ represents an ethyl group, Q represents a group represented by Q5-5, and Het represents any substituents indicated in [Table 1A] to [Table 20A] (hereinafter, referred to as Compound Class SX729).

A compound (L-1) wherein n is 0, $R^1$ represents an ethyl group, Q represents a group represented by Q5-6, and Het represents any substituents indicated in [Table 1A] to [Table 20A] (hereinafter, referred to as Compound Class SX730).

A compound (L-1) wherein n is 0, $R^1$ represents an ethyl group, Q represents a group represented by Q5-7, and Het represents any substituents indicated in [Table 1A] to [Table 20A] (hereinafter, referred to as Compound Class SX731).

A compound (L-1) wherein n is 0, $R^1$ represents an ethyl group, Q represents a group represented by Q5-8, and

Het represents any substituents indicated in [Table 1A] to [Table 20A] (hereinafter, referred to as Compound Class SX732).

A compound (L-1) wherein n is 0, $R^1$ represents an ethyl group, Q represents a group represented by Q5-9, and Het represents any substituents indicated in [Table 1A] to [Table 20A] (hereinafter, referred to as Compound Class SX733).

A compound (L-1) wherein n is 0, $R^1$ represents an ethyl group, Q represents a group represented by Q5-10, and Het represents any substituents indicated in [Table 1A] to [Table 20A] (hereinafter, referred to as Compound Class SX734).

A compound (L-1) wherein n is 0, $R^1$ represents an ethyl group, Q represents a group represented by Q5-11, and Het represents any substituents indicated in [Table 1A] to [Table 20A] (hereinafter, referred to as Compound Class SX735).

A compound (L-1) wherein n is 0, $R^1$ represents an ethyl group, Q represents a group represented by Q5-12, and Het represents any substituents indicated in [Table 1A] to [Table 20A] (hereinafter, referred to as Compound Class SX736).

A compound (L-1) wherein n is 0, $R^1$ represents an ethyl group, Q represents a group represented by Q5-13, and Het represents any substituents indicated in [Table 1A] to [Table 20A] (hereinafter, referred to as Compound Class SX737).

A compound (L-1) wherein n is 0, $R^1$ represents an ethyl group, Q represents a group represented by Q5-14, and Het represents any substituents indicated in [Table 1A] to [Table 20A] (hereinafter, referred to as Compound Class SX738).

A compound (L-1) wherein n is 0, $R^1$ represents an ethyl group, Q represents a group represented by Q5-15, and Het represents any substituents indicated in [Table 1A] to [Table 20A] (hereinafter, referred to as Compound Class SX739).

A compound (L-1) wherein n is 0, $R^1$ represents an ethyl group, Q represents a group represented by Q5-16, and Het represents any substituents indicated in [Table 1A] to [Table 20A] (hereinafter, referred to as Compound Class SX740).

A compound (L-1) wherein n is 0, $R^1$ represents an ethyl group, Q represents a group represented by Q5-17, and Het represents any substituents indicated in [Table 1A] to [Table 20A] (hereinafter, referred to as Compound Class SX741).

A compound (L-1) wherein n is 0, $R^1$ represents an ethyl group, Q represents a group represented by Q5-18, and Het represents any substituents indicated in [Table 1A] to [Table 20A] (hereinafter, referred to as Compound Class SX742).

A compound (L-1) wherein n is 0, $R^1$ represents an ethyl group, Q represents a group represented by Q5-19, and Het represents any substituents indicated in [Table 1A] to [Table 20A] (hereinafter, referred to as Compound Class SX743).

A compound (L-1) wherein n is 0, $R^1$ represents an ethyl group, Q represents a group represented by Q5-20, and Het represents any substituents indicated in [Table 1A] to [Table 20A] (hereinafter, referred to as Compound Class SX744).

A compound (L-1) wherein n is 0, $R^1$ represents an ethyl group, Q represents a group represented by Q5-21, and Het represents any substituents indicated in [Table 1A] to [Table 20A] (hereinafter, referred to as Compound Class SX745).

A compound (L-1) wherein n is 0, $R^1$ represents an ethyl group, Q represents a group represented by Q5-22, and Het represents any substituents indicated in [Table 1A] to [Table 20A] (hereinafter, referred to as Compound Class SX746).

A compound (L-1) wherein n is 2, $R^1$ represents an ethyl group, Q represents a group represented by Q1-1, and Het represents any substituents indicated in [Table 1A] to [Table 20A] (hereinafter, referred to as Compound Class SX747).

A compound (L-1) wherein n is 2, $R^1$ represents an ethyl group, Q represents a group represented by Q1-2, and Het represents any substituents indicated in [Table 1A] to [Table 20A] (hereinafter, referred to as Compound Class SX748).

A compound (L-1) wherein n is 2, $R^1$ represents an ethyl group, Q represents a group represented by Q1-3, and Het represents any substituents indicated in [Table 1A] to [Table 20A] (hereinafter, referred to as Compound Class SX749).

A compound (L-1) wherein n is 2, $R^1$ represents an ethyl group, Q represents a group represented by Q1-4, and Het represents any substituents indicated in [Table 1A] to [Table 20A] (hereinafter, referred to as Compound Class SX750).

A compound (L-1) wherein n is 2, $R^1$ represents an ethyl group, Q represents a group represented by Q1-5, and Het represents any substituents indicated in [Table 1A] to [Table 20A] (hereinafter, referred to as Compound Class SX751).

A compound (L-1) wherein n is 2, $R^1$ represents an ethyl group, Q represents a group represented by Q1-6, and Het represents any substituents indicated in [Table 1A] to [Table 20A] (hereinafter, referred to as Compound Class SX752).

A compound (L-1) wherein n is 2, $R^1$ represents an ethyl group, Q represents a group represented by Q1-7, and Het represents any substituents indicated in [Table 1A] to [Table 20A] (hereinafter, referred to as Compound Class SX753).

A compound (L-1) wherein n is 2, $R^1$ represents an ethyl group, Q represents a group represented by Q1-8, and Het represents any substituents indicated in [Table 1A] to [Table 20A] (hereinafter, referred to as Compound Class SX754).

A compound (L-1) wherein n is 2, $R^1$ represents an ethyl group, Q represents a group represented by Q1-9, and Het represents any substituents indicated in [Table 1A] to [Table 20A] (hereinafter, referred to as Compound Class SX755).

A compound (L-1) wherein n is 2, $R^1$ represents an ethyl group, Q represents a group represented by Q1-10, and Het represents any substituents indicated in [Table 1A] to [Table 20A] (hereinafter, referred to as Compound Class SX756).

A compound (L-1) wherein n is 2, $R^1$ represents an ethyl group, Q represents a group represented by Q1-11, and Het represents any substituents indicated in [Table 1A] to [Table 20A] (hereinafter, referred to as Compound Class SX757).

A compound (L-1) wherein n is 2, $R^1$ represents an ethyl group, Q represents a group represented by Q1-12, and Het represents any substituents indicated in [Table 1A] to [Table 20A] (hereinafter, referred to as Compound Class SX758).

A compound (L-1) wherein n is 2, $R^1$ represents an ethyl group, Q represents a group represented by Q1-13, and Het represents any substituents indicated in [Table 1A] to [Table 20A] (hereinafter, referred to as Compound Class SX759).

A compound (L-1) wherein n is 2, $R^1$ represents an ethyl group, Q represents a group represented by Q1-14, and Het represents any substituents indicated in [Table 1A] to [Table 20A] (hereinafter, referred to as Compound Class SX760).

A compound (L-1) wherein n is 2, $R^1$ represents an ethyl group, Q represents a group represented by Q1-15, and

Het represents any substituents indicated in [Table 1A] to [Table 20A] (hereinafter, referred to as Compound Class SX761).

A compound (L-1) wherein n is 2, $R^1$ represents an ethyl group, Q represents a group represented by Q1-16, and Het represents any substituents indicated in [Table 1A] to [Table 20A] (hereinafter, referred to as Compound Class SX762).

A compound (L-1) wherein n is 2, $R^1$ represents an ethyl group, Q represents a group represented by Q1-17, and Het represents any substituents indicated in [Table 1A] to [Table 20A] (hereinafter, referred to as Compound Class SX763).

A compound (L-1) wherein n is 2, $R^1$ represents an ethyl group, Q represents a group represented by Q1-18, and Het represents any substituents indicated in [Table 1A] to [Table 20A] (hereinafter, referred to as Compound Class SX764).

A compound (L-1) wherein n is 2, $R^1$ represents an ethyl group, Q represents a group represented by Q1-19, and Het represents any substituents indicated in [Table 1A] to [Table 20A] (hereinafter, referred to as Compound Class SX765).

A compound (L-1) wherein n is 2, $R^1$ represents an ethyl group, Q represents a group represented by Q1-20, and Het represents any substituents indicated in [Table 1A] to [Table 20A] (hereinafter, referred to as Compound Class SX766).

A compound (L-1) wherein n is 2, $R^1$ represents an ethyl group, Q represents a group represented by Q1-21, and Het represents any substituents indicated in [Table 1A] to [Table 20A] (hereinafter, referred to as Compound Class SX767).

A compound (L-1) wherein n is 2, $R^1$ represents an ethyl group, Q represents a group represented by Q1-22, and Het represents any substituents indicated in [Table 1A] to [Table 20A] (hereinafter, referred to as Compound Class SX768).

A compound (L-1) wherein n is 2, $R^1$ represents an ethyl group, Q represents a group represented by Q1-23, and Het represents any substituents indicated in [Table 1A] to [Table 20A] (hereinafter, referred to as Compound Class SX769).

A compound (L-1) wherein n is 2, $R^1$ represents an ethyl group, Q represents a group represented by Q1-24, and Het represents any substituents indicated in [Table 1A] to [Table 20A] (hereinafter, referred to as Compound Class SX770).

A compound (L-1) wherein n is 2, $R^1$ represents an ethyl group, Q represents a group represented by Q1-25, and Het represents any substituents indicated in [Table 1A] to [Table 20A] (hereinafter, referred to as Compound Class SX771).

A compound (L-1) wherein n is 2, $R^1$ represents an ethyl group, Q represents a group represented by Q1-26, and Het represents any substituents indicated in [Table 1A] to [Table 20A] (hereinafter, referred to as Compound Class SX772).

A compound (L-1) wherein n is 2, $R^1$ represents an ethyl group, Q represents a group represented by Q1-27, and Het represents any substituents indicated in [Table 1A] to [Table 20A] (hereinafter, referred to as Compound Class SX773).

A compound (L-1) wherein n is 2, $R^1$ represents an ethyl group, Q represents a group represented by Q1-28, and Het represents any substituents indicated in [Table 1A] to [Table 20A] (hereinafter, referred to as Compound Class SX774).

A compound (L-1) wherein n is 2, $R^1$ represents an ethyl group, Q represents a group represented by Q1-29, and Het represents any substituents indicated in [Table 1A] to [Table 20A] (hereinafter, referred to as Compound Class SX775).

A compound (L-1) wherein n is 2, $R^1$ represents an ethyl group, Q represents a group represented by Q1-30, and Het represents any substituents indicated in [Table 1A] to [Table 20A] (hereinafter, referred to as Compound Class SX776).

A compound (L-1) wherein n is 2, $R^1$ represents an ethyl group, Q represents a group represented by Q1-31, and Het represents any substituents indicated in [Table 1A] to [Table 20A] (hereinafter, referred to as Compound Class SX777).

A compound (L-1) wherein n is 2, $R^1$ represents an ethyl group, Q represents a group represented by Q1-32, and Het represents any substituents indicated in [Table 1A] to [Table 20A] (hereinafter, referred to as Compound Class SX778).

A compound (L-1) wherein n is 2, $R^1$ represents an ethyl group, Q represents a group represented by Q1-33, and Het represents any substituents indicated in [Table 1A] to [Table 20A] (hereinafter, referred to as Compound Class SX779).

A compound (L-1) wherein n is 2, $R^1$ represents an ethyl group, Q represents a group represented by Q1-34, and Het represents any substituents indicated in [Table 1A] to [Table 20A] (hereinafter, referred to as Compound Class SX780).

A compound (L-1) wherein n is 2, $R^1$ represents an ethyl group, Q represents a group represented by Q1-35, and Het represents any substituents indicated in [Table 1A] to [Table 20A] (hereinafter, referred to as Compound Class SX781).

A compound (L-1) wherein n is 2, $R^1$ represents an ethyl group, Q represents a group represented by Q1-36, and Het represents any substituents indicated in [Table 1A] to [Table 20A] (hereinafter, referred to as Compound Class SX782).

A compound (L-1) wherein n is 2, $R^1$ represents an ethyl group, Q represents a group represented by Q1-37, and Het represents any substituents indicated in [Table 1A] to [Table 20A] (hereinafter, referred to as Compound Class SX783).

A compound (L-1) wherein n is 2, $R^1$ represents an ethyl group, Q represents a group represented by Q1-38, and Het represents any substituents indicated in [Table 1A] to [Table 20A] (hereinafter, referred to as Compound Class SX784).

A compound (L-1) wherein n is 2, $R^1$ represents an ethyl group, Q represents a group represented by Q1-39, and Het represents any substituents indicated in [Table 1A] to [Table 20A] (hereinafter, referred to as Compound Class SX785).

A compound (L-1) wherein n is 2, $R^1$ represents an ethyl group, Q represents a group represented by Q1-40, and Het represents any substituents indicated in [Table 1A] to [Table 20A] (hereinafter, referred to as Compound Class SX786).

A compound (L-1) wherein n is 2, $R^1$ represents an ethyl group, Q represents a group represented by Q1-41, and Het represents any substituents indicated in [Table 1A] to [Table 20A] (hereinafter, referred to as Compound Class SX787).

A compound (L-1) wherein n is 2, $R^1$ represents an ethyl group, Q represents a group represented by Q1-42, and Het represents any substituents indicated in [Table 1A] to [Table 20A] (hereinafter, referred to as Compound Class SX788).

A compound (L-1) wherein n is 2, $R^1$ represents an ethyl group, Q represents a group represented by Q1-43, and Het represents any substituents indicated in [Table 1A] to [Table 20A] (hereinafter, referred to as Compound Class SX789).

A compound (L-1) wherein n is 2, $R^1$ represents an ethyl group, Q represents a group represented by Q1-44, and

Het represents any substituents indicated in [Table 1A] to [Table 20A] (hereinafter, referred to as Compound Class SX790).

A compound (L-1) wherein n is 2, $R^1$ represents an ethyl group, Q represents a group represented by Q1-45, and Het represents any substituents indicated in [Table 1A] to [Table 20A] (hereinafter, referred to as Compound Class SX791).

A compound (L-1) wherein n is 2, $R^1$ represents an ethyl group, Q represents a group represented by Q1-46, and Het represents any substituents indicated in [Table 1A] to [Table 20A] (hereinafter, referred to as Compound Class SX792).

A compound (L-1) wherein n is 2, $R^1$ represents an ethyl group, Q represents a group represented by Q1-47, and Het represents any substituents indicated in [Table 1A] to [Table 20A] (hereinafter, referred to as Compound Class SX793).

A compound (L-1) wherein n is 2, $R^1$ represents an ethyl group, Q represents a group represented by Q1-48, and Het represents any substituents indicated in [Table 1A] to [Table 20A] (hereinafter, referred to as Compound Class SX794).

A compound (L-1) wherein n is 2, $R^1$ represents an ethyl group, Q represents a group represented by Q1-49, and Het represents any substituents indicated in [Table 1A] to [Table 20A] (hereinafter, referred to as Compound Class SX795).

A compound (L-1) wherein n is 2, $R^1$ represents an ethyl group, Q represents a group represented by Q1-50, and Het represents any substituents indicated in [Table 1A] to [Table 20A] (hereinafter, referred to as Compound Class SX796).

A compound (L-1) wherein n is 2, $R^1$ represents an ethyl group, Q represents a group represented by Q1-51, and Het represents any substituents indicated in [Table 1A] to [Table 20A] (hereinafter, referred to as Compound Class SX797).

A compound (L-1) wherein n is 2, $R^1$ represents an ethyl group, Q represents a group represented by Q1-52, and Het represents any substituents indicated in [Table 1A] to [Table 20A] (hereinafter, referred to as Compound Class SX798).

A compound (L-1) wherein n is 2, $R^1$ represents an ethyl group, Q represents a group represented by Q1-53, and Het represents any substituents indicated in [Table 1A] to [Table 20A] (hereinafter, referred to as Compound Class SX799).

A compound (L-1) wherein n is 2, $R^1$ represents an ethyl group, Q represents a group represented by Q1-54, and Het represents any substituents indicated in [Table 1A] to [Table 20A] (hereinafter, referred to as Compound Class SX800).

A compound (L-1) wherein n is 2, $R^1$ represents an ethyl group, Q represents a group represented by Q1-55, and Het represents any substituents indicated in [Table 1A] to [Table 20A] (hereinafter, referred to as Compound Class SX801).

A compound (L-1) wherein n is 2, $R^1$ represents an ethyl group, Q represents a group represented by Q1-56, and Het represents any substituents indicated in [Table 1A] to [Table 20A] (hereinafter, referred to as Compound Class SX802).

A compound (L-1) wherein n is 2, $R^1$ represents an ethyl group, Q represents a group represented by Q1-57, and Het represents any substituents indicated in [Table 1A] to [Table 20A] (hereinafter, referred to as Compound Class SX803).

A compound (L-1) wherein n is 2, $R^1$ represents an ethyl group, Q represents a group represented by Q1-58, and Het represents any substituents indicated in [Table 1A] to [Table 20A] (hereinafter, referred to as Compound Class SX804).

A compound (L-1) wherein n is 2, $R^1$ represents an ethyl group, Q represents a group represented by Q1-59, and Het represents any substituents indicated in [Table 1A] to [Table 20A] (hereinafter, referred to as Compound Class SX805).

A compound (L-1) wherein n is 2, $R^1$ represents an ethyl group, Q represents a group represented by Q1-60, and Het represents any substituents indicated in [Table 1A] to [Table 20A] (hereinafter, referred to as Compound Class SX806).

A compound (L-1) wherein n is 2, $R^1$ represents an ethyl group, Q represents a group represented by Q1-61, and Het represents any substituents indicated in [Table 1A] to [Table 20A] (hereinafter, referred to as Compound Class SX807).

A compound (L-1) wherein n is 2, $R^1$ represents an ethyl group, Q represents a group represented by Q1-62, and Het represents any substituents indicated in [Table 1A] to [Table 20A] (hereinafter, referred to as Compound Class SX808).

A compound (L-1) wherein n is 2, $R^1$ represents an ethyl group, Q represents a group represented by Q1-63, and Het represents any substituents indicated in [Table 1A] to [Table 20A] (hereinafter, referred to as Compound Class SX809).

A compound (L-1) wherein n is 2, $R^1$ represents an ethyl group, Q represents a group represented by Q1-64, and Het represents any substituents indicated in [Table 1A] to [Table 20A] (hereinafter, referred to as Compound Class SX810).

A compound (L-1) wherein n is 2, $R^1$ represents an ethyl group, Q represents a group represented by Q1-65, and Het represents any substituents indicated in [Table 1A] to [Table 20A] (hereinafter, referred to as Compound Class SX811).

A compound (L-1) wherein n is 2, $R^1$ represents an ethyl group, Q represents a group represented by Q1-66, and Het represents any substituents indicated in [Table 1A] to [Table 20A] (hereinafter, referred to as Compound Class SX812).

A compound (L-1) wherein n is 2, $R^1$ represents an ethyl group, Q represents a group represented by Q1-67, and Het represents any substituents indicated in [Table 1A] to [Table 20A] (hereinafter, referred to as Compound Class SX813).

A compound (L-1) wherein n is 2, $R^1$ represents an ethyl group, Q represents a group represented by Q1-68, and Het represents any substituents indicated in [Table 1A] to [Table 20A] (hereinafter, referred to as Compound Class SX814).

A compound (L-1) wherein n is 2, $R^1$ represents an ethyl group, Q represents a group represented by Q1-69, and Het represents any substituents indicated in [Table 1A] to [Table 20A] (hereinafter, referred to as Compound Class SX815).

A compound (L-1) wherein n is 2, $R^1$ represents an ethyl group, Q represents a group represented by Q1-70, and Het represents any substituents indicated in [Table 1A] to [Table 20A] (hereinafter, referred to as Compound Class SX816).

A compound (L-1) wherein n is 2, $R^1$ represents an ethyl group, Q represents a group represented by Q1-71, and Het represents any substituents indicated in [Table 1A] to [Table 20A] (hereinafter, referred to as Compound Class SX817).

A compound (L-1) wherein n is 2, $R^1$ represents an ethyl group, Q represents a group represented by Q1-72, and Het represents any substituents indicated in [Table 1A] to [Table 20A] (hereinafter, referred to as Compound Class SX818).

A compound (L-1) wherein n is 2, $R^1$ represents an ethyl group, Q represents a group represented by Q1-73, and

Het represents any substituents indicated in [Table 1A] to [Table 20A] (hereinafter, referred to as Compound Class SX819).

A compound (L-1) wherein n is 2, $R^1$ represents an ethyl group, Q represents a group represented by Q1-74, and Het represents any substituents indicated in [Table 1A] to [Table 20A] (hereinafter, referred to as Compound Class SX820).

A compound (L-1) wherein n is 2, $R^1$ represents an ethyl group, Q represents a group represented by Q1-75, and Het represents any substituents indicated in [Table 1A] to [Table 20A] (hereinafter, referred to as Compound Class SX821).

A compound (L-1) wherein n is 2, $R^1$ represents an ethyl group, Q represents a group represented by Q1-76, and Het represents any substituents indicated in [Table 1A] to [Table 20A] (hereinafter, referred to as Compound Class SX822).

A compound (L-1) wherein n is 2, $R^1$ represents an ethyl group, Q represents a group represented by Q1-77, and Het represents any substituents indicated in [Table 1A] to [Table 20A] (hereinafter, referred to as Compound Class SX823).

A compound (L-1) wherein n is 2, $R^1$ represents an ethyl group, Q represents a group represented by Q1-78, and Het represents any substituents indicated in [Table 1A] to [Table 20A] (hereinafter, referred to as Compound Class SX824).

A compound (L-1) wherein n is 2, $R^1$ represents an ethyl group, Q represents a group represented by Q1-79, and Het represents any substituents indicated in [Table 1A] to [Table 20A] (hereinafter, referred to as Compound Class SX825).

A compound (L-1) wherein n is 2, $R^1$ represents an ethyl group, Q represents a group represented by Q1-80, and Het represents any substituents indicated in [Table 1A] to [Table 20A] (hereinafter, referred to as Compound Class SX826).

A compound (L-1) wherein n is 2, $R^1$ represents an ethyl group, Q represents a group represented by Q1-81, and Het represents any substituents indicated in [Table 1A] to [Table 20A] (hereinafter, referred to as Compound Class SX827).

A compound (L-1) wherein n is 2, $R^1$ represents an ethyl group, Q represents a group represented by Q1-82, and Het represents any substituents indicated in [Table 1A] to [Table 20A] (hereinafter, referred to as Compound Class SX828).

A compound (L-1) wherein n is 2, $R^1$ represents an ethyl group, Q represents a group represented by Q1-83, and Het represents any substituents indicated in [Table 1A] to [Table 20A] (hereinafter, referred to as Compound Class SX829).

A compound (L-1) wherein n is 2, $R^1$ represents an ethyl group, Q represents a group represented by Q1-84, and Het represents any substituents indicated in [Table 1A] to [Table 20A] (hereinafter, referred to as Compound Class SX830).

A compound (L-1) wherein n is 2, $R^1$ represents an ethyl group, Q represents a group represented by Q1-85, and Het represents any substituents indicated in [Table 1A] to [Table 20A] (hereinafter, referred to as Compound Class SX831).

A compound (L-1) wherein n is 2, $R^1$ represents an ethyl group, Q represents a group represented by Q1-86, and Het represents any substituents indicated in [Table 1A] to [Table 20A] (hereinafter, referred to as Compound Class SX832).

A compound (L-1) wherein n is 2, $R^1$ represents an ethyl group, Q represents a group represented by Q1-87, and Het represents any substituents indicated in [Table 1A] to [Table 20A] (hereinafter, referred to as Compound Class SX833).

A compound (L-1) wherein n is 2, $R^1$ represents an ethyl group, Q represents a group represented by Q1-88, and Het represents any substituents indicated in [Table 1A] to [Table 20A] (hereinafter, referred to as Compound Class SX834).

A compound (L-1) wherein n is 2, $R^1$ represents an ethyl group, Q represents a group represented by Q1-89, and Het represents any substituents indicated in [Table 1A] to [Table 20A] (hereinafter, referred to as Compound Class SX835).

A compound (L-1) wherein n is 2, $R^1$ represents an ethyl group, Q represents a group represented by Q1-90, and Het represents any substituents indicated in [Table 1A] to [Table 20A] (hereinafter, referred to as Compound Class SX836).

A compound (L-1) wherein n is 2, $R^1$ represents an ethyl group, Q represents a group represented by Q1-91, and Het represents any substituents indicated in [Table 1A] to [Table 20A] (hereinafter, referred to as Compound Class SX837).

A compound (L-1) wherein n is 2, $R^1$ represents an ethyl group, Q represents a group represented by Q1-92, and Het represents any substituents indicated in [Table 1A] to [Table 20A] (hereinafter, referred to as Compound Class SX838).

A compound (L-1) wherein n is 2, $R^1$ represents an ethyl group, Q represents a group represented by Q1-93, and Het represents any substituents indicated in [Table 1A] to [Table 20A] (hereinafter, referred to as Compound Class SX839).

A compound (L-1) wherein n is 2, $R^1$ represents an ethyl group, Q represents a group represented by Q1-94, and Het represents any substituents indicated in [Table 1A] to [Table 20A] (hereinafter, referred to as Compound Class SX840).

A compound (L-1) wherein n is 2, $R^1$ represents an ethyl group, Q represents a group represented by Q1-95, and Het represents any substituents indicated in [Table 1A] to [Table 20A] (hereinafter, referred to as Compound Class SX841).

A compound (L-1) wherein n is 2, $R^1$ represents an ethyl group, Q represents a group represented by Q1-96, and Het represents any substituents indicated in [Table 1A] to [Table 20A] (hereinafter, referred to as Compound Class SX842).

A compound (L-1) wherein n is 2, $R^1$ represents an ethyl group, Q represents a group represented by Q1-97, and Het represents any substituents indicated in [Table 1A] to [Table 20A] (hereinafter, referred to as Compound Class SX843).

A compound (L-1) wherein n is 2, $R^1$ represents an ethyl group, Q represents a group represented by Q1-98, and Het represents any substituents indicated in [Table 1A] to [Table 20A] (hereinafter, referred to as Compound Class SX844).

A compound (L-1) wherein n is 2, $R^1$ represents an ethyl group, Q represents a group represented by Q1-99, and Het represents any substituents indicated in [Table 1A] to [Table 20A] (hereinafter, referred to as Compound Class SX845).

A compound (L-1) wherein n is 2, $R^1$ represents an ethyl group, Q represents a group represented by Q1-100, and Het represents any substituents indicated in [Table 1A] to [Table 20A] (hereinafter, referred to as Compound Class SX846).

A compound (L-1) wherein n is 2, $R^1$ represents an ethyl group, Q represents a group represented by Q1-101, and Het represents any substituents indicated in [Table 1A] to [Table 20A] (hereinafter, referred to as Compound Class SX847).

A compound (L-1) wherein n is 2, $R^1$ represents an ethyl group, Q represents a group represented by Q1-102, and

Het represents any substituents indicated in [Table 1A] to [Table 20A] (hereinafter, referred to as Compound Class SX848).

A compound (L-1) wherein n is 2, $R^1$ represents an ethyl group, Q represents a group represented by Q1-103, and Het represents any substituents indicated in [Table 1A] to [Table 20A] (hereinafter, referred to as Compound Class SX849).

A compound (L-1) wherein n is 2, $R^1$ represents an ethyl group, Q represents a group represented by Q1-104, and Het represents any substituents indicated in [Table 1A] to [Table 20A] (hereinafter, referred to as Compound Class SX850).

A compound (L-1) wherein n is 2, $R^1$ represents an ethyl group, Q represents a group represented by Q1-105, and Het represents any substituents indicated in [Table 1A] to [Table 20A] (hereinafter, referred to as Compound Class SX851).

A compound (L-1) wherein n is 2, $R^1$ represents an ethyl group, Q represents a group represented by Q1-106, and Het represents any substituents indicated in [Table 1A] to [Table 20A] (hereinafter, referred to as Compound Class SX852).

A compound (L-1) wherein n is 2, $R^1$ represents an ethyl group, Q represents a group represented by Q1-107, and Het represents any substituents indicated in [Table 1A] to [Table 20A] (hereinafter, referred to as Compound Class SX853).

A compound (L-1) wherein n is 2, $R^1$ represents an ethyl group, Q represents a group represented by Q1-108, and Het represents any substituents indicated in [Table 1A] to [Table 20A] (hereinafter, referred to as Compound Class SX854).

A compound (L-1) wherein n is 2, $R^1$ represents an ethyl group, Q represents a group represented by Q1-109, and Het represents any substituents indicated in [Table 1A] to [Table 20A] (hereinafter, referred to as Compound Class SX855).

A compound (L-1) wherein n is 2, $R^1$ represents an ethyl group, Q represents a group represented by Q1-110, and Het represents any substituents indicated in [Table 1A] to [Table 20A] (hereinafter, referred to as Compound Class SX856).

A compound (L-1) wherein n is 2, $R^1$ represents an ethyl group, Q represents a group represented by Q1-111, and Het represents any substituents indicated in [Table 1A] to [Table 20A] (hereinafter, referred to as Compound Class SX857).

A compound (L-1) wherein n is 2, $R^1$ represents an ethyl group, Q represents a group represented by Q1-112, and Het represents any substituents indicated in [Table 1A] to [Table 20A] (hereinafter, referred to as Compound Class SX858).

A compound (L-1) wherein n is 2, $R^1$ represents an ethyl group, Q represents a group represented by Q1-113, and Het represents any substituents indicated in [Table 1A] to [Table 20A] (hereinafter, referred to as Compound Class SX859).

A compound (L-1) wherein n is 2, $R^1$ represents an ethyl group, Q represents a group represented by Q1-114, and Het represents any substituents indicated in [Table 1A] to [Table 20A] (hereinafter, referred to as Compound Class SX860).

A compound (L-1) wherein n is 2, $R^1$ represents an ethyl group, Q represents a group represented by Q1-115, and Het represents any substituents indicated in [Table 1A] to [Table 20A] (hereinafter, referred to as Compound Class SX861).

A compound (L-1) wherein n is 2, $R^1$ represents an ethyl group, Q represents a group represented by Q1-116, and Het represents any substituents indicated in [Table 1A] to [Table 20A] (hereinafter, referred to as Compound Class SX862).

A compound (L-1) wherein n is 2, $R^1$ represents an ethyl group, Q represents a group represented by Q1-117, and Het represents any substituents indicated in [Table 1A] to [Table 20A] (hereinafter, referred to as Compound Class SX863).

A compound (L-1) wherein n is 2, $R^1$ represents an ethyl group, Q represents a group represented by Q1-118, and Het represents any substituents indicated in [Table 1A] to [Table 20A] (hereinafter, referred to as Compound Class SX864).

A compound (L-1) wherein n is 2, $R^1$ represents an ethyl group, Q represents a group represented by Q1-119, and Het represents any substituents indicated in [Table 1A] to [Table 20A] (hereinafter, referred to as Compound Class SX865).

A compound (L-1) wherein n is 2, $R^1$ represents an ethyl group, Q represents a group represented by Q1-120, and Het represents any substituents indicated in [Table 1A] to [Table 20A] (hereinafter, referred to as Compound Class SX866).

A compound (L-1) wherein n is 2, $R^1$ represents an ethyl group, Q represents a group represented by Q1-121, and Het represents any substituents indicated in [Table 1A] to [Table 20A] (hereinafter, referred to as Compound Class SX867).

A compound (L-1) wherein n is 2, $R^1$ represents an ethyl group, Q represents a group represented by Q1-122, and Het represents any substituents indicated in [Table 1A] to [Table 20A] (hereinafter, referred to as Compound Class SX868).

A compound (L-1) wherein n is 2, $R^1$ represents an ethyl group, Q represents a group represented by Q1-123, and Het represents any substituents indicated in [Table 1A] to [Table 20A] (hereinafter, referred to as Compound Class SX869).

A compound (L-1) wherein n is 2, $R^1$ represents an ethyl group, Q represents a group represented by Q1-124, and Het represents any substituents indicated in [Table 1A] to [Table 20A] (hereinafter, referred to as Compound Class SX870).

A compound (L-1) wherein n is 2, $R^1$ represents an ethyl group, Q represents a group represented by Q1-125, and Het represents any substituents indicated in [Table 1A] to [Table 20A] (hereinafter, referred to as Compound Class SX871).

A compound (L-1) wherein n is 2, $R^1$ represents an ethyl group, Q represents a group represented by Q1-126, and Het represents any substituents indicated in [Table 1A] to [Table 20A] (hereinafter, referred to as Compound Class SX872).

A compound (L-1) wherein n is 2, $R^1$ represents an ethyl group, Q represents a group represented by Q1-127, and Het represents any substituents indicated in [Table 1A] to [Table 20A] (hereinafter, referred to as Compound Class SX873).

A compound (L-1) wherein n is 2, $R^1$ represents an ethyl group, Q represents a group represented by Q1-128, and Het represents any substituents indicated in [Table 1A] to [Table 20A] (hereinafter, referred to as Compound Class SX874).

A compound (L-1) wherein n is 2, $R^1$ represents an ethyl group, Q represents a group represented by Q1-129, and Het represents any substituents indicated in [Table 1A] to [Table 20A] (hereinafter, referred to as Compound Class SX875).

A compound (L-1) wherein n is 2, $R^1$ represents an ethyl group, Q represents a group represented by Q1-130, and Het represents any substituents indicated in [Table 1A] to [Table 20A] (hereinafter, referred to as Compound Class SX876).

A compound (L-1) wherein n is 2, $R^1$ represents an ethyl group, Q represents a group represented by Q1-131, and

Het represents any substituents indicated in [Table 1A] to [Table 20A] (hereinafter, referred to as Compound Class SX877).

A compound (L-1) wherein n is 2, $R^1$ represents an ethyl group, Q represents a group represented by Q1-132, and Het represents any substituents indicated in [Table 1A] to [Table 20A] (hereinafter, referred to as Compound Class SX878).

A compound (L-1) wherein n is 2, $R^1$ represents an ethyl group, Q represents a group represented by Q1-133, and Het represents any substituents indicated in [Table 1A] to [Table 20A] (hereinafter, referred to as Compound Class SX879).

A compound (L-1) wherein n is 2, $R^1$ represents an ethyl group, Q represents a group represented by Q1-134, and Het represents any substituents indicated in [Table 1A] to [Table 20A] (hereinafter, referred to as Compound Class SX880).

A compound (L-1) wherein n is 2, $R^1$ represents an ethyl group, Q represents a group represented by Q1-135, and Het represents any substituents indicated in [Table 1A] to [Table 20A] (hereinafter, referred to as Compound Class SX881).

A compound (L-1) wherein n is 2, $R^1$ represents an ethyl group, Q represents a group represented by Q1-136, and Het represents any substituents indicated in [Table 1A] to [Table 20A] (hereinafter, referred to as Compound Class SX882).

A compound (L-1) wherein n is 2, $R^1$ represents an ethyl group, Q represents a group represented by Q1-136, and Het represents any substituents indicated in [Table 1A] to [Table 20A] (hereinafter, referred to as Compound Class SX882).

A compound (L-1) wherein n is 2, $R^1$ represents an ethyl group, Q represents a group represented by Q1-137, and Het represents any substituents indicated in [Table 1A] to [Table 20A] (hereinafter, referred to as Compound Class SX883).

A compound (L-1) wherein n is 2, $R^1$ represents an ethyl group, Q represents a group represented by Q1-138, and Het represents any substituents indicated in [Table 1A] to [Table 20A] (hereinafter, referred to as Compound Class SX884).

A compound (L-1) wherein n is 2, $R^1$ represents an ethyl group, Q represents a group represented by Q1-139, and Het represents any substituents indicated in [Table 1A] to [Table 20A] (hereinafter, referred to as Compound Class SX885).

A compound (L-1) wherein n is 2, $R^1$ represents an ethyl group, Q represents a group represented by Q1-140, and Het represents any substituents indicated in [Table 1A] to [Table 20A] (hereinafter, referred to as Compound Class SX886).

A compound (L-1) wherein n is 2, $R^1$ represents an ethyl group, Q represents a group represented by Q1-141, and Het represents any substituents indicated in [Table 1A] to [Table 20A] (hereinafter, referred to as Compound Class SX887).

A compound (L-1) wherein n is 2, $R^1$ represents an ethyl group, Q represents a group represented by Q1-142, and Het represents any substituents indicated in [Table 1A] to [Table 20A] (hereinafter, referred to as Compound Class SX888).

A compound (L-1) wherein n is 2, $R^1$ represents an ethyl group, Q represents a group represented by Q1-143, and Het represents any substituents indicated in [Table 1A] to [Table 20A] (hereinafter, referred to as Compound Class SX889).

A compound (L-1) wherein n is 2, $R^1$ represents an ethyl group, Q represents a group represented by Q1-144, and Het represents any substituents indicated in [Table 1A] to [Table 20A] (hereinafter, referred to as Compound Class SX890).

A compound (L-1) wherein n is 2, $R^1$ represents an ethyl group, Q represents a group represented by Q1-145, and Het represents any substituents indicated in [Table 1A] to [Table 20A] (hereinafter, referred to as Compound Class SX891).

A compound (L-1) wherein n is 2, $R^1$ represents an ethyl group, Q represents a group represented by Q1-146, and Het represents any substituents indicated in [Table 1A] to [Table 20A] (hereinafter, referred to as Compound Class SX892).

A compound (L-1) wherein n is 2, $R^1$ represents an ethyl group, Q represents a group represented by Q1-147, and Het represents any substituents indicated in [Table 1A] to [Table 20A] (hereinafter, referred to as Compound Class SX893).

A compound (L-1) wherein n is 2, $R^1$ represents an ethyl group, Q represents a group represented by Q1-148, and Het represents any substituents indicated in [Table 1A] to [Table 20A] (hereinafter, referred to as Compound Class SX894).

A compound (L-1) wherein n is 2, $R^1$ represents an ethyl group, Q represents a group represented by Q1-149, and Het represents any substituents indicated in [Table 1A] to [Table 20A] (hereinafter, referred to as Compound Class SX895).

A compound (L-1) wherein n is 2, $R^1$ represents an ethyl group, Q represents a group represented by Q1-150, and Het represents any substituents indicated in [Table 1A] to [Table 20A] (hereinafter, referred to as Compound Class SX896).

A compound (L-1) wherein n is 2, $R^1$ represents an ethyl group, Q represents a group represented by Q1-151, and Het represents any substituents indicated in [Table 1A] to [Table 20A] (hereinafter, referred to as Compound Class SX897).

A compound (L-1) wherein n is 2, $R^1$ represents an ethyl group, Q represents a group represented by Q1-152, and Het represents any substituents indicated in [Table 1A] to [Table 20A] (hereinafter, referred to as Compound Class SX898).

A compound (L-1) wherein n is 2, $R^1$ represents an ethyl group, Q represents a group represented by Q1-153, and Het represents any substituents indicated in [Table 1A] to [Table 20A] (hereinafter, referred to as Compound Class SX899).

A compound (L-1) wherein n is 2, $R^1$ represents an ethyl group, Q represents a group represented by Q1-154, and Het represents any substituents indicated in [Table 1A] to [Table 20A] (hereinafter, referred to as Compound Class SX900).

A compound (L-1) wherein n is 2, $R^1$ represents an ethyl group, Q represents a group represented by Q1-155, and Het represents any substituents indicated in [Table 1A] to [Table 20A] (hereinafter, referred to as Compound Class SX901).

A compound (L-1) wherein n is 2, $R^1$ represents an ethyl group, Q represents a group represented by Q1-156, and Het represents any substituents indicated in [Table 1A] to [Table 20A] (hereinafter, referred to as Compound Class SX902).

A compound (L-1) wherein n is 2, $R^1$ represents an ethyl group, Q represents a group represented by Q1-157, and Het represents any substituents indicated in [Table 1A] to [Table 20A] (hereinafter, referred to as Compound Class SX903).

A compound (L-1) wherein n is 2, $R^1$ represents an ethyl group, Q represents a group represented by Q1-158, and Het represents any substituents indicated in [Table 1A] to [Table 20A] (hereinafter, referred to as Compound Class SX904).

A compound (L-1) wherein n is 2, $R^1$ represents an ethyl group, Q represents a group represented by Q1-159, and

Het represents any substituents indicated in [Table 1A] to [Table 20A] (hereinafter, referred to as Compound Class SX905).

A compound (L-1) wherein n is 2, $R^1$ represents an ethyl group, Q represents a group represented by Q1-160, and Het represents any substituents indicated in [Table 1A] to [Table 20A] (hereinafter, referred to as Compound Class SX906).

A compound (L-1) wherein n is 2, $R^1$ represents an ethyl group, Q represents a group represented by Q1-161, and Het represents any substituents indicated in [Table 1A] to [Table 20A] (hereinafter, referred to as Compound Class SX907).

A compound (L-1) wherein n is 2, $R^1$ represents an ethyl group, Q represents a group represented by Q1-162, and Het represents any substituents indicated in [Table 1A] to [Table 20A] (hereinafter, referred to as Compound Class SX908).

A compound (L-1) wherein n is 2, $R^1$ represents an ethyl group, Q represents a group represented by Q1-163, and Het represents any substituents indicated in [Table 1A] to [Table 20A] (hereinafter, referred to as Compound Class SX909).

A compound (L-1) wherein n is 2, $R^1$ represents an ethyl group, Q represents a group represented by Q1-164, and Het represents any substituents indicated in [Table 1A] to [Table 20A] (hereinafter, referred to as Compound Class SX910).

A compound (L-1) wherein n is 2, $R^1$ represents an ethyl group, Q represents a group represented by Q1-165, and Het represents any substituents indicated in [Table 1A] to [Table 20A] (hereinafter, referred to as Compound Class SX911).

A compound (L-1) wherein n is 2, $R^1$ represents an ethyl group, Q represents a group represented by Q1-166, and Het represents any substituents indicated in [Table 1A] to [Table 20A] (hereinafter, referred to as Compound Class SX912).

A compound (L-1) wherein n is 2, $R^1$ represents an ethyl group, Q represents a group represented by Q1-167, and Het represents any substituents indicated in [Table 1A] to [Table 20A] (hereinafter, referred to as Compound Class SX913).

A compound (L-1) wherein n is 2, $R^1$ represents an ethyl group, Q represents a group represented by Q1-168, and Het represents any substituents indicated in [Table 1A] to [Table 20A] (hereinafter, referred to as Compound Class SX914).

A compound (L-1) wherein n is 2, $R^1$ represents an ethyl group, Q represents a group represented by Q1-169, and Het represents any substituents indicated in [Table 1A] to [Table 20A] (hereinafter, referred to as Compound Class SX915).

A compound (L-1) wherein n is 2, $R^1$ represents an ethyl group, Q represents a group represented by Q1-170, and Het represents any substituents indicated in [Table 1A] to [Table 20A] (hereinafter, referred to as Compound Class SX916).

A compound (L-1) wherein n is 2, $R^1$ represents an ethyl group, Q represents a group represented by Q1-171, and Het represents any substituents indicated in [Table 1A] to [Table 20A] (hereinafter, referred to as Compound Class SX917).

A compound (L-1) wherein n is 2, $R^1$ represents an ethyl group, Q represents a group represented by Q1-172, and Het represents any substituents indicated in [Table 1A] to [Table 20A] (hereinafter, referred to as Compound Class SX918).

A compound (L-1) wherein n is 2, $R^1$ represents an ethyl group, Q represents a group represented by Q1-173, and Het represents any substituents indicated in [Table 1A] to [Table 20A] (hereinafter, referred to as Compound Class SX919).

A compound (L-1) wherein n is 2, $R^1$ represents an ethyl group, Q represents a group represented by Q1-174, and Het represents any substituents indicated in [Table 1A] to [Table 20A] (hereinafter, referred to as Compound Class SX920).

A compound (L-1) wherein n is 2, $R^1$ represents an ethyl group, Q represents a group represented by Q1-175, and Het represents any substituents indicated in [Table 1A] to [Table 20A] (hereinafter, referred to as Compound Class SX921).

A compound (L-1) wherein n is 2, $R^1$ represents an ethyl group, Q represents a group represented by Q1-176, and Het represents any substituents indicated in [Table 1A] to [Table 20A] (hereinafter, referred to as Compound Class SX922).

A compound (L-1) wherein n is 2, $R^1$ represents an ethyl group, Q represents a group represented by Q1-177, and Het represents any substituents indicated in [Table 1A] to [Table 20A] (hereinafter, referred to as Compound Class SX923).

A compound (L-1) wherein n is 2, $R^1$ represents an ethyl group, Q represents a group represented by Q1-178, and Het represents any substituents indicated in [Table 1A] to [Table 20A] (hereinafter, referred to as Compound Class SX924).

A compound (L-1) wherein n is 2, $R^1$ represents an ethyl group, Q represents a group represented by Q1-179, and Het represents any substituents indicated in [Table 1A] to [Table 20A] (hereinafter, referred to as Compound Class SX925).

A compound (L-1) wherein n is 2, $R^1$ represents an ethyl group, Q represents a group represented by Q1-180, and Het represents any substituents indicated in [Table 1A] to [Table 20A] (hereinafter, referred to as Compound Class SX926).

A compound (L-1) wherein n is 2, $R^1$ represents an ethyl group, Q represents a group represented by Q1-181, and Het represents any substituents indicated in [Table 1A] to [Table 20A] (hereinafter, referred to as Compound Class SX927).

A compound (L-1) wherein n is 2, $R^1$ represents an ethyl group, Q represents a group represented by Q1-182, and Het represents any substituents indicated in [Table 1A] to [Table 20A] (hereinafter, referred to as Compound Class SX928).

A compound (L-1) wherein n is 2, $R^1$ represents an ethyl group, Q represents a group represented by Q1-183, and Het represents any substituents indicated in [Table 1A] to [Table 20A] (hereinafter, referred to as Compound Class SX929).

A compound (L-1) wherein n is 2, $R^1$ represents an ethyl group, Q represents a group represented by Q1-184, and Het represents any substituents indicated in [Table 1A] to [Table 20A] (hereinafter, referred to as Compound Class SX930).

A compound (L-1) wherein n is 2, $R^1$ represents an ethyl group, Q represents a group represented by Q1-185, and Het represents any substituents indicated in [Table 1A] to [Table 20A] (hereinafter, referred to as Compound Class SX931).

A compound (L-1) wherein n is 2, $R^1$ represents an ethyl group, Q represents a group represented by Q1-186, and Het represents any substituents indicated in [Table 1A] to [Table 20A] (hereinafter, referred to as Compound Class SX932).

A compound (L-1) wherein n is 2, $R^1$ represents an ethyl group, Q represents a group represented by Q1-187, and Het represents any substituents indicated in [Table 1A] to [Table 20A] (hereinafter, referred to as Compound Class SX933).

A compound (L-1) wherein n is 2, $R^1$ represents an ethyl group, Q represents a group represented by Q1-188, and

Het represents any substituents indicated in [Table 1A] to [Table 20A] (hereinafter, referred to as Compound Class SX934).

A compound (L-1) wherein n is 2, $R^1$ represents an ethyl group, Q represents a group represented by Q1-189, and Het represents any substituents indicated in [Table 1A] to [Table 20A] (hereinafter, referred to as Compound Class SX935).

A compound (L-1) wherein n is 2, $R^1$ represents an ethyl group, Q represents a group represented by Q1-190, and Het represents any substituents indicated in [Table 1A] to [Table 20A] (hereinafter, referred to as Compound Class SX936).

A compound (L-1) wherein n is 2, $R^1$ represents an ethyl group, Q represents a group represented by Q1-191, and Het represents any substituents indicated in [Table 1A] to [Table 20A] (hereinafter, referred to as Compound Class SX937).

A compound (L-1) wherein n is 2, $R^1$ represents an ethyl group, Q represents a group represented by Q1-192, and Het represents any substituents indicated in [Table 1A] to [Table 20A] (hereinafter, referred to as Compound Class SX938).

A compound (L-1) wherein n is 2, $R^1$ represents an ethyl group, Q represents a group represented by Q1-193, and Het represents any substituents indicated in [Table 1A] to [Table 20A] (hereinafter, referred to as Compound Class SX939).

A compound (L-1) wherein n is 2, $R^1$ represents an ethyl group, Q represents a group represented by Q1-194, and Het represents any substituents indicated in [Table 1A] to [Table 20A] (hereinafter, referred to as Compound Class SX940).

A compound (L-1) wherein n is 2, $R^1$ represents an ethyl group, Q represents a group represented by Q1-195, and Het represents any substituents indicated in [Table 1A] to [Table 20A] (hereinafter, referred to as Compound Class SX941).

A compound (L-1) wherein n is 2, $R^1$ represents an ethyl group, Q represents a group represented by Q1-196, and Het represents any substituents indicated in [Table 1A] to [Table 20A] (hereinafter, referred to as Compound Class SX942).

A compound (L-1) wherein n is 2, $R^1$ represents an ethyl group, Q represents a group represented by Q1-197, and Het represents any substituents indicated in [Table 1A] to [Table 20A] (hereinafter, referred to as Compound Class SX943).

A compound (L-1) wherein n is 2, $R^1$ represents an ethyl group, Q represents a group represented by Q1-198, and Het represents any substituents indicated in [Table 1A] to [Table 20A] (hereinafter, referred to as Compound Class SX944).

A compound (L-1) wherein n is 2, $R^1$ represents an ethyl group, Q represents a group represented by Q1-199, and Het represents any substituents indicated in [Table 1A] to [Table 20A] (hereinafter, referred to as Compound Class SX945).

A compound (L-1) wherein n is 2, $R^1$ represents an ethyl group, Q represents a group represented by Q1-200, and Het represents any substituents indicated in [Table 1A] to [Table 20A] (hereinafter, referred to as Compound Class SX946).

A compound (L-1) wherein n is 2, $R^1$ represents an ethyl group, Q represents a group represented by Q1-201, and Het represents any substituents indicated in [Table 1A] to [Table 20A] (hereinafter, referred to as Compound Class SX947).

A compound (L-1) wherein n is 2, $R^1$ represents an ethyl group, Q represents a group represented by 01-202, and Het represents any substituents indicated in [Table 1A] to [Table 20A] (hereinafter, referred to as Compound Class SX948).

A compound (L-1) wherein n is 2, $R^1$ represents an ethyl group, Q represents a group represented by Q1-203, and Het represents any substituents indicated in [Table 1A] to [Table 20A] (hereinafter, referred to as Compound Class SX949).

A compound (L-1) wherein n is 2, $R^1$ represents an ethyl group, Q represents a group represented by Q1-204, and Het represents any substituents indicated in [Table 1A] to [Table 20A] (hereinafter, referred to as Compound Class SX950).

A compound (L-1) wherein n is 2, $R^1$ represents an ethyl group, Q represents a group represented by Q1-205, and Het represents any substituents indicated in [Table 1A] to [Table 20A] (hereinafter, referred to as Compound Class SX951).

A compound (L-1) wherein n is 2, $R^1$ represents an ethyl group, Q represents a group represented by Q1-206, and Het represents any substituents indicated in [Table 1A] to [Table 20A] (hereinafter, referred to as Compound Class SX952).

A compound (L-1) wherein n is 2, $R^1$ represents an ethyl group, Q represents a group represented by Q1-207, and Het represents any substituents indicated in [Table 1A] to [Table 20A] (hereinafter, referred to as Compound Class SX953).

A compound (L-1) wherein n is 2, $R^1$ represents an ethyl group, Q represents a group represented by Q1-208, and Het represents any substituents indicated in [Table 1A] to [Table 20A] (hereinafter, referred to as Compound Class SX954).

A compound (L-1) wherein n is 2, $R^1$ represents an ethyl group, Q represents a group represented by Q1-209, and Het represents any substituents indicated in [Table 1A] to [Table 20A] (hereinafter, referred to as Compound Class SX955).

A compound (L-1) wherein n is 2, $R^1$ represents an ethyl group, Q represents a group represented by Q2-1, and Het represents any substituents indicated in [Table 1A] to [Table 20A] (hereinafter, referred to as Compound Class SX956).

A compound (L-1) wherein n is 2, $R^1$ represents an ethyl group, Q represents a group represented by Q2-2, and Het represents any substituents indicated in [Table 1A] to [Table 20A] (hereinafter, referred to as Compound Class SX957).

A compound (L-1) wherein n is 2, $R^1$ represents an ethyl group, Q represents a group represented by Q2-3, and Het represents any substituents indicated in [Table 1A] to [Table 20A] (hereinafter, referred to as Compound Class SX958).

A compound (L-1) wherein n is 2, $R^1$ represents an ethyl group, Q represents a group represented by Q2-4, and Het represents any substituents indicated in [Table 1A] to [Table 20A] (hereinafter, referred to as Compound Class SX959).

A compound (L-1) wherein n is 2, $R^1$ represents an ethyl group, Q represents a group represented by Q2-5, and Het represents any substituents indicated in [Table 1A] to [Table 20A] (hereinafter, referred to as Compound Class SX960).

A compound (L-1) wherein n is 2, $R^1$ represents an ethyl group, Q represents a group represented by Q2-6, and Het represents any substituents indicated in [Table 1A] to [Table 20A] (hereinafter, referred to as Compound Class SX961).

A compound (L-1) wherein n is 2, $R^1$ represents an ethyl group, Q represents a group represented by Q2-7, and Het represents any substituents indicated in [Table 1A] to [Table 20A] (hereinafter, referred to as Compound Class SX962).

A compound (L-1) wherein n is 2, $R^1$ represents an ethyl group, Q represents a group represented by Q2-8, and

Het represents any substituents indicated in [Table 1A] to [Table 20A] (hereinafter, referred to as Compound Class SX963).

A compound (L-1) wherein n is 2, $R^1$ represents an ethyl group, Q represents a group represented by Q2-9, and Het represents any substituents indicated in [Table 1A] to [Table 20A] (hereinafter, referred to as Compound Class SX964).

A compound (L-1) wherein n is 2, $R^1$ represents an ethyl group, Q represents a group represented by Q2-10, and Het represents any substituents indicated in [Table 1A] to [Table 20A] (hereinafter, referred to as Compound Class SX965).

A compound (L-1) wherein n is 2, $R^1$ represents an ethyl group, Q represents a group represented by Q2-11, and Het represents any substituents indicated in [Table 1A] to [Table 20A] (hereinafter, referred to as Compound Class SX966).

A compound (L-1) wherein n is 2, $R^1$ represents an ethyl group, Q represents a group represented by Q2-12, and Het represents any substituents indicated in [Table 1A] to [Table 20A] (hereinafter, referred to as Compound Class SX967).

A compound (L-1) wherein n is 2, $R^1$ represents an ethyl group, Q represents a group represented by Q2-13, and Het represents any substituents indicated in [Table 1A] to [Table 20A] (hereinafter, referred to as Compound Class SX968).

A compound (L-1) wherein n is 2, $R^1$ represents an ethyl group, Q represents a group represented by Q2-14, and Het represents any substituents indicated in [Table 1A] to [Table 20A] (hereinafter, referred to as Compound Class SX969).

A compound (L-1) wherein n is 2, $R^1$ represents an ethyl group, Q represents a group represented by Q2-15, and Het represents any substituents indicated in [Table 1A] to [Table 20A] (hereinafter, referred to as Compound Class SX970).

A compound (L-1) wherein n is 2, $R^1$ represents an ethyl group, Q represents a group represented by Q2-16, and Het represents any substituents indicated in [Table 1A] to [Table 20A] (hereinafter, referred to as Compound Class SX971).

A compound (L-1) wherein n is 2, $R^1$ represents an ethyl group, Q represents a group represented by Q2-17, and Het represents any substituents indicated in [Table 1A] to [Table 20A] (hereinafter, referred to as Compound Class SX972).

A compound (L-1) wherein n is 2, $R^1$ represents an ethyl group, Q represents a group represented by Q2-18, and Het represents any substituents indicated in [Table 1A] to [Table 20A] (hereinafter, referred to as Compound Class SX973).

A compound (L-1) wherein n is 2, $R^1$ represents an ethyl group, Q represents a group represented by Q2-19, and Het represents any substituents indicated in [Table 1A] to [Table 20A] (hereinafter, referred to as Compound Class SX974).

A compound (L-1) wherein n is 2, $R^1$ represents an ethyl group, Q represents a group represented by Q2-20, and Het represents any substituents indicated in [Table 1A] to [Table 20A] (hereinafter, referred to as Compound Class SX975).

A compound (L-1) wherein n is 2, $R^1$ represents an ethyl group, Q represents a group represented by Q2-21, and Het represents any substituents indicated in [Table 1A] to [Table 20A] (hereinafter, referred to as Compound Class SX976).

A compound (L-1) wherein n is 2, $R^1$ represents an ethyl group, Q represents a group represented by Q2-22, and Het represents any substituents indicated in [Table 1A] to [Table 20A] (hereinafter, referred to as Compound Class SX977).

A compound (L-1) wherein n is 2, $R^1$ represents an ethyl group, Q represents a group represented by Q2-23, and Het represents any substituents indicated in [Table 1A] to [Table 20A] (hereinafter, referred to as Compound Class SX978).

A compound (L-1) wherein n is 2, $R^1$ represents an ethyl group, Q represents a group represented by Q2-24, and Het represents any substituents indicated in [Table 1A] to [Table 20A] (hereinafter, referred to as Compound Class SX979).

A compound (L-1) wherein n is 2, $R^1$ represents an ethyl group, Q represents a group represented by Q2-25, and Het represents any substituents indicated in [Table 1A] to [Table 20A] (hereinafter, referred to as Compound Class SX980).

A compound (L-1) wherein n is 2, $R^1$ represents an ethyl group, Q represents a group represented by Q2-26, and Het represents any substituents indicated in [Table 1A] to [Table 20A] (hereinafter, referred to as Compound Class SX981).

A compound (L-1) wherein n is 2, $R^1$ represents an ethyl group, Q represents a group represented by Q2-27, and Het represents any substituents indicated in [Table 1A] to [Table 20A] (hereinafter, referred to as Compound Class SX982).

A compound (L-1) wherein n is 2, $R^1$ represents an ethyl group, Q represents a group represented by Q2-28, and Het represents any substituents indicated in [Table 1A] to [Table 20A] (hereinafter, referred to as Compound Class SX983).

A compound (L-1) wherein n is 2, $R^1$ represents an ethyl group, Q represents a group represented by Q2-29, and Het represents any substituents indicated in [Table 1A] to [Table 20A] (hereinafter, referred to as Compound Class SX984).

A compound (L-1) wherein n is 2, $R^1$ represents an ethyl group, Q represents a group represented by Q2-30, and Het represents any substituents indicated in [Table 1A] to [Table 20A] (hereinafter, referred to as Compound Class SX985).

A compound (L-1) wherein n is 2, $R^1$ represents an ethyl group, Q represents a group represented by Q2-31, and Het represents any substituents indicated in [Table 1A] to [Table 20A] (hereinafter, referred to as Compound Class SX986).

A compound (L-1) wherein n is 2, $R^1$ represents an ethyl group, Q represents a group represented by Q5-1, and Het represents any substituents indicated in [Table 1A] to [Table 20A] (hereinafter, referred to as Compound Class SX987).

A compound (L-1) wherein n is 2, $R^1$ represents an ethyl group, Q represents a group represented by Q5-2, and Het represents any substituents indicated in [Table 1A] to [Table 20A] (hereinafter, referred to as Compound Class SX988).

A compound (L-1) wherein n is 2, $R^1$ represents an ethyl group, Q represents a group represented by Q5-3, and Het represents any substituents indicated in [Table 1A] to [Table 20A] (hereinafter, referred to as Compound Class SX989).

A compound (L-1) wherein n is 2, $R^1$ represents an ethyl group, Q represents a group represented by Q5-4, and Het represents any substituents indicated in [Table 1A] to [Table 20A] (hereinafter, referred to as Compound Class SX990).

A compound (L-1) wherein n is 2, $R^1$ represents an ethyl group, Q represents a group represented by Q5-5, and Het represents any substituents indicated in [Table 1A] to [Table 20A] (hereinafter, referred to as Compound Class SX991).

A compound (L-1) wherein n is 2, $R^1$ represents an ethyl group, Q represents a group represented by Q5-6, and

Het represents any substituents indicated in [Table 1A] to [Table 20A] (hereinafter, referred to as Compound Class SX992).

A compound (L-1) wherein n is 2, $R^1$ represents an ethyl group, Q represents a group represented by Q5-7, and Het represents any substituents indicated in [Table 1A] to [Table 20A] (hereinafter, referred to as Compound Class SX993).

A compound (L-1) wherein n is 2, $R^1$ represents an ethyl group, Q represents a group represented by Q5-8, and Het represents any substituents indicated in [Table 1A] to [Table 20A] (hereinafter, referred to as Compound Class SX994).

A compound (L-1) wherein n is 2, $R^1$ represents an ethyl group, Q represents a group represented by Q5-9, and Het represents any substituents indicated in [Table 1A] to [Table 20A] (hereinafter, referred to as Compound Class SX995).

A compound (L-1) wherein n is 2, $R^1$ represents an ethyl group, Q represents a group represented by Q5-10, and Het represents any substituents indicated in [Table 1A] to [Table 20A] (hereinafter, referred to as Compound Class SX996).

A compound (L-1) wherein n is 2, $R^1$ represents an ethyl group, Q represents a group represented by Q5-11, and Het represents any substituents indicated in [Table 1A] to [Table 20A] (hereinafter, referred to as Compound Class SX997).

A compound (L-1) wherein n is 2, $R^1$ represents an ethyl group, Q represents a group represented by Q5-12, and Het represents any substituents indicated in [Table 1A] to [Table 20A] (hereinafter, referred to as Compound Class SX998).

A compound (L-1) wherein n is 2, $R^1$ represents an ethyl group, Q represents a group represented by Q5-13, and Het represents any substituents indicated in [Table 1A] to [Table 20A] (hereinafter, referred to as Compound Class SX999).

A compound (L-1) wherein n is 2, $R^1$ represents an ethyl group, Q represents a group represented by Q5-14, and Het represents any substituents indicated in [Table 1A] to [Table 20A] (hereinafter, referred to as Compound Class SX1000).

A compound (L-1) wherein n is 2, $R^1$ represents an ethyl group, Q represents a group represented by Q5-15, and Het represents any substituents indicated in [Table 1A] to [Table 20A] (hereinafter, referred to as Compound Class SX1001).

A compound (L-1) wherein n is 2, $R^1$ represents an ethyl group, Q represents a group represented by Q5-16, and Het represents any substituents indicated in [Table 1A] to [Table 20A] (hereinafter, referred to as Compound Class SX1002).

A compound (L-1) wherein n is 2, $R^1$ represents an ethyl group, Q represents a group represented by Q5-17, and Het represents any substituents indicated in [Table 1A] to [Table 20A] (hereinafter, referred to as Compound Class SX1003).

A compound (L-1) wherein n is 2, $R^1$ represents an ethyl group, Q represents a group represented by Q5-18, and Het represents any substituents indicated in [Table 1A] to [Table 20A] (hereinafter, referred to as Compound Class SX1004).

A compound (L-1) wherein n is 2, $R^1$ represents an ethyl group, Q represents a group represented by Q5-19, and Het represents any substituents indicated in [Table 1A] to [Table 20A] (hereinafter, referred to as Compound Class SX1005).

A compound (L-1) wherein n is 2, $R^1$ represents an ethyl group, Q represents a group represented by Q5-20, and Het represents any substituents indicated in [Table 1A] to [Table 20A] (hereinafter, referred to as Compound Class SX1006).

A compound (L-1) wherein n is 2, $R^1$ represents an ethyl group, Q represents a group represented by Q5-21, and Het represents any substituents indicated in [Table 1A] to [Table 20A] (hereinafter, referred to as Compound Class SX1007).

A compound (L-1) wherein n is 2, $R^1$ represents an ethyl group, Q represents a group represented by Q5-22, and Het represents any substituents indicated in [Table 1A] to [Table 20A] (hereinafter, referred to as Compound Class SX1008).

A compound (L-1) wherein n is 2, $R^1$ represents an ethyl group, Q represents a group represented by Q6-1, and Het represents any substituents indicated in [Table 1A] to [Table 20A] (hereinafter, referred to as Compound Class SX1009).

A compound (L-1) wherein n is 2, $R^1$ represents an ethyl group, Q represents a group represented by Q6-2, and Het represents any substituents indicated in [Table 1A] to [Table 20A] (hereinafter, referred to as Compound Class SX1010).

A compound (L-1) wherein n is 2, $R^1$ represents an ethyl group, Q represents a group represented by Q6-3, and Het represents any substituents indicated in [Table 1A] to [Table 20A] (hereinafter, referred to as Compound Class SX1011).

A compound (L-1) wherein n is 2, $R^1$ represents an ethyl group, Q represents a group represented by Q6-4, and Het represents any substituents indicated in [Table 1A] to [Table 20A] (hereinafter, referred to as Compound Class SX1012).

A compound (L-1) wherein n is 2, $R^1$ represents an ethyl group, Q represents a group represented by Q6-5, and Het represents any substituents indicated in [Table 1A] to [Table 20A] (hereinafter, referred to as Compound Class SX1013).

A compound (L-1) wherein n is 2, $R^1$ represents an ethyl group, Q represents a group represented by Q6-6, and

Het represents any substituents indicated in [Table 1A] to [Table 20A] (hereinafter, referred to as Compound Class SX1014).

A compound (L-1) wherein n is 2, $R^1$ represents an ethyl group, Q represents a group represented by Q6-7, and Het represents any substituents indicated in [Table 1A] to [Table 20A] (hereinafter, referred to as Compound Class SX1015).

A compound (L-1) wherein n is 2, $R^1$ represents an ethyl group, Q represents a group represented by Q6-8, and Het represents any substituents indicated in [Table 1A] to [Table 20A] (hereinafter, referred to as Compound Class SX1016).

A compound (L-1) wherein n is 2, $R^1$ represents an ethyl group, Q represents a group represented by Q6-9, and Het represents any substituents indicated in [Table 1A] to [Table 20A] (hereinafter, referred to as Compound Class SX1017).

A compound (L-1) wherein n is 2, $R^1$ represents an ethyl group, Q represents a group represented by Q6-10, and Het represents any substituents indicated in [Table 1A] to [Table 20A] (hereinafter, referred to as Compound Class SX1018).

A compound (L-1) wherein n is 2, $R^1$ represents an ethyl group, Q represents a group represented by Q6-11, and Het represents any substituents indicated in [Table 1A] to [Table 20A] (hereinafter, referred to as Compound Class SX1019).

A compound (L-1) wherein n is 2, $R^1$ represents an ethyl group, Q represents a group represented by Q6-12, and Het represents any substituents indicated in [Table 1A] to [Table 20A] (hereinafter, referred to as Compound Class SX1020).

A compound (L-1) wherein n is 2, $R^1$ represents an ethyl group, Q represents a group represented by Q6-13, and Het represents any substituents indicated in [Table 1A] to [Table 20A] (hereinafter, referred to as Compound Class SX1021).

A compound (L-1) wherein n is 2, $R^1$ represents an ethyl group, Q represents a group represented by Q6-14, and Het represents any substituents indicated in [Table 1A] to [Table 20A] (hereinafter, referred to as Compound Class SX1022).

A compound (L-1) wherein n is 2, $R^1$ represents an ethyl group, Q represents a group represented by Q6-15, and Het represents any substituents indicated in [Table 1A] to [Table 20A] (hereinafter, referred to as Compound Class SX1023).

A compound (L-1) wherein n is 2, $R^1$ represents an ethyl group, Q represents a group represented by Q6-16, and Het represents any substituents indicated in [Table 1A] to [Table 20A] (hereinafter, referred to as Compound Class SX1024).

A compound (L-1) wherein n is 2, $R^1$ represents an ethyl group, Q represents a group represented by Q6-17, and Het represents any substituents indicated in [Table 1A] to [Table 20A] (hereinafter, referred to as Compound Class SX1025).

A compound (L-1) wherein n is 2, $R^1$ represents an ethyl group, Q represents a group represented by Q6-18, and Het represents any substituents indicated in [Table 1A] to [Table 20A] (hereinafter, referred to as Compound Class SX1026).

A compound (L-1) wherein n is 2, $R^1$ represents an ethyl group, Q represents a group represented by Q6-19, and Het represents any substituents indicated in [Table 1A] to [Table 20A] (hereinafter, referred to as Compound Class SX1027).

A compound (L-1) wherein n is 2, $R^1$ represents an ethyl group, Q represents a group represented by Q6-20, and Het represents any substituents indicated in [Table 1A] to [Table 20A] (hereinafter, referred to as Compound Class SX1028).

A compound (L-1) wherein n is 2, $R^1$ represents an ethyl group, Q represents a group represented by Q6-21, and Het represents any substituents indicated in [Table 1A] to [Table 20A] (hereinafter, referred to as Compound Class SX1029).

A compound (L-1) wherein n is 2, $R^1$ represents an ethyl group, Q represents a group represented by Q6-22, and Het represents any substituents indicated in [Table 1A] to [Table 20A] (hereinafter, referred to as Compound Class SX1030).

A compound (L-1) wherein n is 2, $R^1$ represents a cyclopropylmethyl group, Q represents a group represented by Q1-1, and Het represents any substituents indicated in [Table 1A] to [Table 20A] (hereinafter, referred to as Compound Class SX1031).

A compound (L-1) wherein n is 2, $R^1$ represents a cyclopropylmethyl group, Q represents a group represented by Q1-2, and Het represents any substituents indicated in [Table 1A] to [Table 20A] (hereinafter, referred to as Compound Class SX1032).

A compound (L-1) wherein n is 2, $R^1$ represents a cyclopropylmethyl group, Q represents a group represented by Q1-3, and Het represents any substituents indicated in [Table 1A] to [Table 20A] (hereinafter, referred to as Compound

Class SX1033).

A compound (L-1) wherein n is 2, $R^1$ represents a cyclopropylmethyl group, Q represents a group represented by Q1-4, and Het represents any substituents indicated in [Table 1A] to [Table 20A] (hereinafter, referred to as Compound Class SX1034).

A compound (L-1) wherein n is 2, $R^1$ represents a cyclopropylmethyl group, Q represents a group represented by Q1-5, and Het represents any substituents indicated in [Table 1A] to [Table 20A] (hereinafter, referred to as Compound Class SX1035).

A compound (L-1) wherein n is 2, $R^1$ represents a cyclopropylmethyl group, Q represents a group represented by Q1-6, and Het represents any substituents indicated in [Table 1A] to [Table 20A] (hereinafter, referred to as Compound Class SX1036).

A compound (L-1) wherein n is 2, $R^1$ represents a cyclopropylmethyl group, Q represents a group represented by Q1-7, and Het represents any substituents indicated in [Table 1A] to [Table 20A] (hereinafter, referred to as Compound Class SX1037).

A compound (L-1) wherein n is 2, $R^1$ represents a cyclopropylmethyl group, Q represents a group represented by Q1-8, and Het represents any substituents indicated in [Table 1A] to [Table 20A] (hereinafter, referred to as Compound Class SX1038).

A compound (L-1) wherein n is 2, $R^1$ represents a cyclopropylmethyl group, Q represents a group represented by Q1-9, and Het represents any substituents indicated in [Table 1A] to [Table 20A] (hereinafter, referred to as Compound Class SX1039).

A compound (L-1) wherein n is 2, $R^1$ represents a cyclopropylmethyl group, Q represents a group represented by Q1-10, and Het represents any substituents indicated in [Table 1A] to [Table 20A] (hereinafter, referred to as Compound Class SX1040).

A compound (L-1) wherein n is 2, $R^1$ represents a cyclopropylmethyl group, Q represents a group represented by Q1-11, and Het represents any substituents indicated in [Table 1A] to [Table 20A] (hereinafter, referred to as Compound Class SX1041).

A compound (L-1) wherein n is 2, $R^1$ represents a cyclopropylmethyl group, Q represents a group represented by Q1-12, and Het represents any substituents indicated in [Table 1A] to [Table 20A] (hereinafter, referred to as Compound Class SX1042).

A compound (L-1) wherein n is 2, $R^1$ represents a cyclopropylmethyl group, Q represents a group represented by Q1-13, and Het represents any substituents indicated in [Table 1A] to [Table 20A] (hereinafter, referred to as Compound Class SX1043).

A compound (L-1) wherein n is 2, $R^1$ represents a cyclopropylmethyl group, Q represents a group represented by Q1-14, and Het represents any substituents indicated in [Table 1A] to [Table 20A] (hereinafter, referred to as Compound Class SX1044).

A compound (L-1) wherein n is 2, $R^1$ represents a cyclopropylmethyl group, Q represents a group represented by Q1-15, and Het represents any substituents indicated in [Table 1A] to [Table 20A] (hereinafter, referred to as Compound Class SX1045).

A compound (L-1) wherein n is 2, $R^1$ represents a cyclopropylmethyl group, Q represents a group represented by Q1-16, and Het represents any substituents indicated in [Table 1A] to [Table 20A] (hereinafter, referred to as Compound Class SX1046).

A compound (L-1) wherein n is 2, $R^1$ represents a cyclopropylmethyl group, Q represents a group represented by Q1-17, and Het represents any substituents indicated in [Table 1A] to [Table 20A] (hereinafter, referred to as Compound Class SX1047).

A compound (L-1) wherein n is 2, $R^1$ represents a cyclopropylmethyl group, Q represents a group represented by Q1-18, and Het represents any substituents indicated in [Table 1A] to [Table 20A] (hereinafter, referred to as Compound Class SX1048).

A compound (L-1) wherein n is 2, $R^1$ represents a cyclopropylmethyl group, Q represents a group represented by Q1-19, and Het represents any substituents indicated in [Table 1A] to [Table 20A] (hereinafter, referred to as Compound Class SX1049).

A compound (L-1) wherein n is 2, $R^1$ represents a cyclopropylmethyl group, Q represents a group represented by Q1-20, and Het represents any substituents indicated in [Table 1A] to [Table 20A] (hereinafter, referred to as Compound Class SX1050).

A compound (L-1) wherein n is 2, $R^1$ represents a cyclopropylmethyl group, Q represents a group represented by Q1-21, and Het represents any substituents indicated in [Table 1A] to [Table 20A] (hereinafter, referred to as Compound Class SX1051).

A compound (L-1) wherein n is 2, $R^1$ represents a cyclopropylmethyl group, Q represents a group represented by Q1-22, and Het represents any substituents indicated in [Table 1A] to [Table 20A] (hereinafter, referred to as Compound Class SX1052).

A compound (L-1) wherein n is 2, $R^1$ represents a cyclopropylmethyl group, Q represents a group represented by Q1-23, and Het represents any substituents indicated in [Table 1A] to [Table 20A] (hereinafter, referred to as Compound Class SX1053).

A compound (L-1) wherein n is 2, $R^1$ represents a cyclopropylmethyl group, Q represents a group represented by Q1-24, and Het represents any substituents indicated in [Table 1A] to [Table 20A] (hereinafter, referred to as Compound Class SX1054).

A compound (L-1) wherein n is 2, $R^1$ represents a cyclopropylmethyl group, Q represents a group represented by Q1-25, and Het represents any substituents indicated in [Table 1A] to [Table 20A] (hereinafter, referred to as Compound Class SX1055).

A compound (L-1) wherein n is 2, $R^1$ represents a cyclopropylmethyl group, Q represents a group represented by Q1-26, and Het represents any substituents indicated in [Table 1A] to [Table 20A] (hereinafter, referred to as Compound Class SX1056).

A compound (L-1) wherein n is 2, $R^1$ represents a cyclopropylmethyl group, Q represents a group represented by Q1-27, and Het represents any substituents indicated in [Table 1A] to [Table 20A] (hereinafter, referred to as Compound Class SX1057).

A compound (L-1) wherein n is 2, $R^1$ represents a cyclopropylmethyl group, Q represents a group represented by Q1-28, and Het represents any substituents indicated in [Table 1A] to [Table 20A] (hereinafter, referred to as Compound Class SX1058).

A compound (L-1) wherein n is 2, $R^1$ represents a cyclopropylmethyl group, Q represents a group represented by Q1-29, and Het represents any substituents indicated in [Table 1A] to [Table 20A] (hereinafter, referred to as Compound Class SX1059).

A compound (L-1) wherein n is 2, $R^1$ represents a cyclopropylmethyl group, Q represents a group represented by Q1-30, and Het represents any substituents indicated in [Table 1A] to [Table 20A] (hereinafter, referred to as Compound Class SX1060).

A compound (L-1) wherein n is 2, $R^1$ represents a cyclopropylmethyl group, Q represents a group represented by Q1-31, and Het represents any substituents indicated in [Table 1A] to [Table 20A] (hereinafter, referred to as Compound Class SX1061).

A compound (L-1) wherein n is 2, $R^1$ represents a cyclopropylmethyl group, Q represents a group represented by Q1-32, and Het represents any substituents indicated in [Table 1A] to [Table 20A] (hereinafter, referred to as Compound Class SX1062).

A compound (L-1) wherein n is 2, $R^1$ represents a cyclopropylmethyl group, Q represents a group represented by Q1-33, and Het represents any substituents indicated in [Table 1A] to [Table 20A] (hereinafter, referred to as Compound Class SX1063).

A compound (L-1) wherein n is 2, $R^1$ represents a cyclopropylmethyl group, Q represents a group represented by Q1-34, and Het represents any substituents indicated in [Table 1A] to [Table 20A] (hereinafter, referred to as Compound Class SX1064).

A compound (L-1) wherein n is 2, $R^1$ represents a cyclopropylmethyl group, Q represents a group represented by Q1-35, and Het represents any substituents indicated in [Table 1A] to [Table 20A] (hereinafter, referred to as Compound Class SX1065).

A compound (L-1) wherein n is 2, $R^1$ represents a cyclopropylmethyl group, Q represents a group represented by Q1-36, and Het represents any substituents indicated in [Table 1A] to [Table 20A] (hereinafter, referred to as Compound Class SX1066).

A compound (L-1) wherein n is 2, $R^1$ represents a cyclopropylmethyl group, Q represents a group represented by Q1-37, and Het represents any substituents indicated in [Table 1A] to [Table 20A] (hereinafter, referred to as Compound Class SX1067).

A compound (L-1) wherein n is 2, $R^1$ represents a cyclopropylmethyl group, Q represents a group represented by Q1-38, and Het represents any substituents indicated in [Table 1A] to [Table 20A] (hereinafter, referred to as Compound Class SX1068).

A compound (L-1) wherein n is 2, $R^1$ represents a cyclopropylmethyl group, Q represents a group represented by Q1-39, and Het represents any substituents indicated in [Table 1A] to [Table 20A] (hereinafter, referred to as Compound Class SX1069).

A compound (L-1) wherein n is 2, $R^1$ represents a cyclopropylmethyl group, Q represents a group represented by Q1-40, and Het represents any substituents indicated in [Table 1A] to [Table 20A] (hereinafter, referred to as Compound Class SX1070).

A compound (L-1) wherein n is 2, $R^1$ represents a cyclopropylmethyl group, Q represents a group represented by Q1-41, and Het represents any substituents indicated in [Table 1A] to [Table 20A] (hereinafter, referred to as Compound Class SX1071).

A compound (L-1) wherein n is 2, $R^1$ represents a cyclopropylmethyl group, Q represents a group represented by

Q1-42, and Het represents any substituents indicated in [Table 1A] to [Table 20A] (hereinafter, referred to as Compound Class SX1072).

A compound (L-1) wherein n is 2, $R^1$ represents a cyclopropylmethyl group, Q represents a group represented by Q1-43, and Het represents any substituents indicated in [Table 1A] to [Table 20A] (hereinafter, referred to as Compound Class SX1073).

A compound (L-1) wherein n is 2, $R^1$ represents a cyclopropylmethyl group, Q represents a group represented by Q1-44, and Het represents any substituents indicated in [Table 1A] to [Table 20A] (hereinafter, referred to as Compound Class SX1074).

A compound (L-1) wherein n is 2, $R^1$ represents a cyclopropylmethyl group, Q represents a group represented by Q1-45, and Het represents any substituents indicated in [Table 1A] to [Table 20A] (hereinafter, referred to as Compound Class SX1075).

A compound (L-1) wherein n is 2, $R^1$ represents a cyclopropylmethyl group, Q represents a group represented by Q1-46, and Het represents any substituents indicated in [Table 1A] to [Table 20A] (hereinafter, referred to as Compound Class SX1076).

A compound (L-1) wherein n is 2, $R^1$ represents a cyclopropylmethyl group, Q represents a group represented by Q1-47, and Het represents any substituents indicated in [Table 1A] to [Table 20A] (hereinafter, referred to as Compound Class SX1077).

A compound (L-1) wherein n is 2, $R^1$ represents a cyclopropylmethyl group, Q represents a group represented by Q1-48, and Het represents any substituents indicated in [Table 1A] to [Table 20A] (hereinafter, referred to as Compound Class SX1078).

A compound (L-1) wherein n is 2, $R^1$ represents a cyclopropylmethyl group, Q represents a group represented by Q1-49, and Het represents any substituents indicated in [Table 1A] to [Table 20A] (hereinafter, referred to as Compound Class SX1079).

A compound (L-1) wherein n is 2, $R^1$ represents a cyclopropylmethyl group, Q represents a group represented by Q1-50, and Het represents any substituents indicated in [Table 1A] to [Table 20A] (hereinafter, referred to as Compound Class SX1080).

A compound (L-1) wherein n is 2, $R^1$ represents a cyclopropylmethyl group, Q represents a group represented by Q1-51, and Het represents any substituents indicated in [Table 1A] to [Table 20A] (hereinafter, referred to as Compound Class SX1081).

A compound (L-1) wherein n is 2, $R^1$ represents a cyclopropylmethyl group, Q represents a group represented by Q1-52, and Het represents any substituents indicated in [Table 1A] to [Table 20A] (hereinafter, referred to as Compound Class SX1082).

A compound (L-1) wherein n is 2, $R^1$ represents a cyclopropylmethyl group, Q represents a group represented by Q1-53, and Het represents any substituents indicated in [Table 1A] to [Table 20A] (hereinafter, referred to as Compound Class SX1083).

A compound (L-1) wherein n is 2, $R^1$ represents a cyclopropylmethyl group, Q represents a group represented by Q1-54, and Het represents any substituents indicated in [Table 1A] to [Table 20A] (hereinafter, referred to as Compound Class SX1084).

A compound (L-1) wherein n is 2, $R^1$ represents a cyclopropylmethyl group, Q represents a group represented by Q1-55, and Het represents any substituents indicated in [Table 1A] to [Table 20A] (hereinafter, referred to as Compound Class SX1085).

A compound (L-1) wherein n is 2, $R^1$ represents a cyclopropylmethyl group, Q represents a group represented by Q1-56, and Het represents any substituents indicated in [Table 1A] to [Table 20A] (hereinafter, referred to as Compound Class SX1086).

A compound (L-1) wherein n is 2, $R^1$ represents a cyclopropylmethyl group, Q represents a group represented by Q1-57, and Het represents any substituents indicated in [Table 1A] to [Table 20A] (hereinafter, referred to as Compound Class SX1087).

A compound (L-1) wherein n is 2, $R^1$ represents a cyclopropylmethyl group, Q represents a group represented by Q1-58, and Het represents any substituents indicated in [Table 1A] to [Table 20A] (hereinafter, referred to as Compound Class SX1088).

A compound (L-1) wherein n is 2, $R^1$ represents a cyclopropylmethyl group, Q represents a group represented by Q1-59, and Het represents any substituents indicated in [Table 1A] to [Table 20A] (hereinafter, referred to as Compound Class SX1089).

A compound (L-1) wherein n is 2, $R^1$ represents a cyclopropylmethyl group, Q represents a group represented by Q1-60, and Het represents any substituents indicated in [Table 1A] to [Table 20A] (hereinafter, referred to as Compound Class SX1090).

A compound (L-1) wherein n is 2, $R^1$ represents a cyclopropylmethyl group, Q represents a group represented by Q1-61, and Het represents any substituents indicated in [Table 1A] to [Table 20A] (hereinafter, referred to as Compound

Class SX1091).

A compound (L-1) wherein n is 2, $R^1$ represents a cyclopropylmethyl group, Q represents a group represented by Q1-62, and Het represents any substituents indicated in [Table 1A] to [Table 20A] (hereinafter, referred to as Compound Class SX1092).

A compound (L-1) wherein n is 2, $R^1$ represents a cyclopropylmethyl group, Q represents a group represented by Q1-63, and Het represents any substituents indicated in [Table 1A] to [Table 20A] (hereinafter, referred to as Compound Class SX1093).

A compound (L-1) wherein n is 2, $R^1$ represents a cyclopropylmethyl group, Q represents a group represented by Q1-64, and Het represents any substituents indicated in [Table 1A] to [Table 20A] (hereinafter, referred to as Compound Class SX1094).

A compound (L-1) wherein n is 2, $R^1$ represents a cyclopropylmethyl group, Q represents a group represented by Q1-65, and Het represents any substituents indicated in [Table 1A] to [Table 20A] (hereinafter, referred to as Compound Class SX1095).

A compound (L-1) wherein n is 2, $R^1$ represents a cyclopropylmethyl group, Q represents a group represented by Q1-66, and Het represents any substituents indicated in [Table 1A] to [Table 20A] (hereinafter, referred to as Compound Class SX1096).

A compound (L-1) wherein n is 2, $R^1$ represents a cyclopropylmethyl group, Q represents a group represented by Q1-67, and Het represents any substituents indicated in [Table 1A] to [Table 20A] (hereinafter, referred to as Compound Class SX1097).

A compound (L-1) wherein n is 2, $R^1$ represents a cyclopropylmethyl group, Q represents a group represented by Q1-68, and Het represents any substituents indicated in [Table 1A] to [Table 20A] (hereinafter, referred to as Compound Class SX1098).

A compound (L-1) wherein n is 2, $R^1$ represents a cyclopropylmethyl group, Q represents a group represented by Q1-69, and Het represents any substituents indicated in [Table 1A] to [Table 20A] (hereinafter, referred to as Compound Class SX1099).

A compound (L-1) wherein n is 2, $R^1$ represents a cyclopropylmethyl group, Q represents a group represented by Q1-70, and Het represents any substituents indicated in [Table 1A] to [Table 20A] (hereinafter, referred to as Compound Class SX1100).

A compound (L-1) wherein n is 2, $R^1$ represents a cyclopropylmethyl group, Q represents a group represented by Q1-71, and Het represents any substituents indicated in [Table 1A] to [Table 20A] (hereinafter, referred to as Compound Class SX1101).

A compound (L-1) wherein n is 2, $R^1$ represents a cyclopropylmethyl group, Q represents a group represented by Q1-72, and Het represents any substituents indicated in [Table 1A] to [Table 20A] (hereinafter, referred to as Compound Class SX1102).

A compound (L-1) wherein n is 2, $R^1$ represents a cyclopropylmethyl group, Q represents a group represented by Q1-73, and Het represents any substituents indicated in [Table 1A] to [Table 20A] (hereinafter, referred to as Compound Class SX1103).

A compound (L-1) wherein n is 2, $R^1$ represents a cyclopropylmethyl group, Q represents a group represented by Q1-74, and Het represents any substituents indicated in [Table 1A] to [Table 20A] (hereinafter, referred to as Compound Class SX1104).

A compound (L-1) wherein n is 2, $R^1$ represents a cyclopropylmethyl group, Q represents a group represented by Q1-75, and Het represents any substituents indicated in [Table 1A] to [Table 20A] (hereinafter, referred to as Compound Class SX1105).

A compound (L-1) wherein n is 2, $R^1$ represents a cyclopropylmethyl group, Q represents a group represented by Q1-76, and Het represents any substituents indicated in [Table 1A] to [Table 20A] (hereinafter, referred to as Compound Class SX1106).

A compound (L-1) wherein n is 2, $R^1$ represents a cyclopropylmethyl group, Q represents a group represented by Q1-77, and Het represents any substituents indicated in [Table 1A] to [Table 20A] (hereinafter, referred to as Compound Class SX1107).

A compound (L-1) wherein n is 2, $R^1$ represents a cyclopropylmethyl group, Q represents a group represented by Q1-78, and Het represents any substituents indicated in [Table 1A] to [Table 20A] (hereinafter, referred to as Compound Class SX1108).

A compound (L-1) wherein n is 2, $R^1$ represents a cyclopropylmethyl group, Q represents a group represented by Q1-79, and Het represents any substituents indicated in [Table 1A] to [Table 20A] (hereinafter, referred to as Compound Class SX1109).

A compound (L-1) wherein n is 2, $R^1$ represents a cyclopropylmethyl group, Q represents a group represented by Q1-80, and Het represents any substituents indicated in [Table 1A] to [Table 20A] (hereinafter, referred to as Compound Class SX1110).

A compound (L-1) wherein n is 2, $R^1$ represents a cyclopropylmethyl group, Q represents a group represented by Q1-81, and Het represents any substituents indicated in [Table 1A] to [Table 20A] (hereinafter, referred to as Compound Class SX1111).

A compound (L-1) wherein n is 2, $R^1$ represents a cyclopropylmethyl group, Q represents a group represented by Q1-82, and Het represents any substituents indicated in [Table 1A] to [Table 20A] (hereinafter, referred to as Compound Class SX1112).

A compound (L-1) wherein n is 2, $R^1$ represents a cyclopropylmethyl group, Q represents a group represented by Q1-83, and Het represents any substituents indicated in [Table 1A] to [Table 20A] (hereinafter, referred to as Compound Class SX1113).

A compound (L-1) wherein n is 2, $R^1$ represents a cyclopropylmethyl group, Q represents a group represented by Q1-84, and Het represents any substituents indicated in [Table 1A] to [Table 20A] (hereinafter, referred to as Compound Class SX1114).

A compound (L-1) wherein n is 2, $R^1$ represents a cyclopropylmethyl group, Q represents a group represented by Q1-85, and Het represents any substituents indicated in [Table 1A] to [Table 20A] (hereinafter, referred to as Compound Class SX1115).

A compound (L-1) wherein n is 2, $R^1$ represents a cyclopropylmethyl group, Q represents a group represented by Q1-86, and Het represents any substituents indicated in [Table 1A] to [Table 20A] (hereinafter, referred to as Compound Class SX1116).

A compound (L-1) wherein n is 2, $R^1$ represents a cyclopropylmethyl group, Q represents a group represented by Q1-87, and Het represents any substituents indicated in [Table 1A] to [Table 20A] (hereinafter, referred to as Compound Class SX1117).

A compound (L-1) wherein n is 2, $R^1$ represents a cyclopropylmethyl group, Q represents a group represented by Q1-88, and Het represents any substituents indicated in [Table 1A] to [Table 20A] (hereinafter, referred to as Compound Class SX1118).

A compound (L-1) wherein n is 2, $R^1$ represents a cyclopropylmethyl group, Q represents a group represented by Q1-89, and Het represents any substituents indicated in [Table 1A] to [Table 20A] (hereinafter, referred to as Compound Class SX1119).

A compound (L-1) wherein n is 2, $R^1$ represents a cyclopropylmethyl group, Q represents a group represented by Q1-90, and Het represents any substituents indicated in [Table 1A] to [Table 20A] (hereinafter, referred to as Compound Class SX1120).

A compound (L-1) wherein n is 2, $R^1$ represents a cyclopropylmethyl group, Q represents a group represented by Q1-91, and Het represents any substituents indicated in [Table 1A] to [Table 20A] (hereinafter, referred to as Compound Class SX1121).

A compound (L-1) wherein n is 2, $R^1$ represents a cyclopropylmethyl group, Q represents a group represented by Q1-92, and Het represents any substituents indicated in [Table 1A] to [Table 20A] (hereinafter, referred to as Compound Class SX1122).

A compound (L-1) wherein n is 2, $R^1$ represents a cyclopropylmethyl group, Q represents a group represented by Q1-93, and Het represents any substituents indicated in [Table 1A] to [Table 20A] (hereinafter, referred to as Compound Class SX1123).

A compound (L-1) wherein n is 2, $R^1$ represents a cyclopropylmethyl group, Q represents a group represented by Q1-94, and Het represents any substituents indicated in [Table 1A] to [Table 20A] (hereinafter, referred to as Compound Class SX1124).

A compound (L-1) wherein n is 2, $R^1$ represents a cyclopropylmethyl group, Q represents a group represented by Q1-95, and Het represents any substituents indicated in [Table 1A] to [Table 20A] (hereinafter, referred to as Compound Class SX1125).

A compound (L-1) wherein n is 2, $R^1$ represents a cyclopropylmethyl group, Q represents a group represented by Q1-96, and Het represents any substituents indicated in [Table 1A] to [Table 20A] (hereinafter, referred to as Compound Class SX1126).

A compound (L-1) wherein n is 2, $R^1$ represents a cyclopropylmethyl group, Q represents a group represented by Q1-97, and Het represents any substituents indicated in [Table 1A] to [Table 20A] (hereinafter, referred to as Compound Class SX1127).

A compound (L-1) wherein n is 2, $R^1$ represents a cyclopropylmethyl group, Q represents a group represented by Q1-98, and Het represents any substituents indicated in [Table 1A] to [Table 20A] (hereinafter, referred to as Compound Class SX1128).

A compound (L-1) wherein n is 2, $R^1$ represents a cyclopropylmethyl group, Q represents a group represented by Q1-99, and Het represents any substituents indicated in [Table 1A] to [Table 20A] (hereinafter, referred to as Compound Class SX1129).

A compound (L-1) wherein n is 2, $R^1$ represents a cyclopropylmethyl group, Q represents a group represented by

Q1-100, and Het represents any substituents indicated in [Table 1A] to [Table 20A] (hereinafter, referred to as Compound Class SX1130).

A compound (L-1) wherein n is 2, $R^1$ represents a cyclopropylmethyl group, Q represents a group represented by Q1-101, and Het represents any substituents indicated in [Table 1A] to [Table 20A] (hereinafter, referred to as Compound Class SX1131).

A compound (L-1) wherein n is 2, $R^1$ represents a cyclopropylmethyl group, Q represents a group represented by Q1-102, and Het represents any substituents indicated in [Table 1A] to [Table 20A] (hereinafter, referred to as Compound Class SX1132).

A compound (L-1) wherein n is 2, $R^1$ represents a cyclopropylmethyl group, Q represents a group represented by Q1-103, and Het represents any substituents indicated in [Table 1A] to [Table 20A] (hereinafter, referred to as Compound Class SX1133).

A compound (L-1) wherein n is 2, $R^1$ represents a cyclopropylmethyl group, Q represents a group represented by Q1-104, and Het represents any substituents indicated in [Table 1A] to [Table 20A] (hereinafter, referred to as Compound Class SX1134).

A compound (L-1) wherein n is 2, $R^1$ represents a cyclopropylmethyl group, Q represents a group represented by Q1-105, and Het represents any substituents indicated in [Table 1A] to [Table 20A] (hereinafter, referred to as Compound Class SX1135).

A compound (L-1) wherein n is 2, $R^1$ represents a cyclopropylmethyl group, Q represents a group represented by Q1-106, and Het represents any substituents indicated in [Table 1A] to [Table 20A] (hereinafter, referred to as Compound Class SX1136).

A compound (L-1) wherein n is 2, $R^1$ represents a cyclopropylmethyl group, Q represents a group represented by Q1-107, and Het represents any substituents indicated in [Table 1A] to [Table 20A] (hereinafter, referred to as Compound Class SX1137).

A compound (L-1) wherein n is 2, $R^1$ represents a cyclopropylmethyl group, Q represents a group represented by Q1-108, and Het represents any substituents indicated in [Table 1A] to [Table 20A] (hereinafter, referred to as Compound Class SX1138).

A compound (L-1) wherein n is 2, $R^1$ represents a cyclopropylmethyl group, Q represents a group represented by Q1-109, and Het represents any substituents indicated in [Table 1A] to [Table 20A] (hereinafter, referred to as Compound Class SX1139).

A compound (L-1) wherein n is 2, $R^1$ represents a cyclopropylmethyl group, Q represents a group represented by Q1-110, and Het represents any substituents indicated in [Table 1A] to [Table 20A] (hereinafter, referred to as Compound Class SX1140).

A compound (L-1) wherein n is 2, $R^1$ represents a cyclopropylmethyl group, Q represents a group represented by Q1-111, and Het represents any substituents indicated in [Table 1A] to [Table 20A] (hereinafter, referred to as Compound Class SX1141).

A compound (L-1) wherein n is 2, $R^1$ represents a cyclopropylmethyl group, Q represents a group represented by Q1-112, and Het represents any substituents indicated in [Table 1A] to [Table 20A] (hereinafter, referred to as Compound Class SX1142).

A compound (L-1) wherein n is 2, $R^1$ represents a cyclopropylmethyl group, Q represents a group represented by Q1-113, and Het represents any substituents indicated in [Table 1A] to [Table 20A] (hereinafter, referred to as Compound Class SX1143).

A compound (L-1) wherein n is 2, $R^1$ represents a cyclopropylmethyl group, Q represents a group represented by Q1-114, and Het represents any substituents indicated in [Table 1A] to [Table 20A] (hereinafter, referred to as Compound Class SX1144).

A compound (L-1) wherein n is 2, $R^1$ represents a cyclopropylmethyl group, Q represents a group represented by Q1-115, and Het represents any substituents indicated in [Table 1A] to [Table 20A] (hereinafter, referred to as Compound Class SX1145).

A compound (L-1) wherein n is 2, $R^1$ represents a cyclopropylmethyl group, Q represents a group represented by Q1-116, and Het represents any substituents indicated in [Table 1A] to [Table 20A] (hereinafter, referred to as Compound Class SX1146).

A compound (L-1) wherein n is 2, $R^1$ represents a cyclopropylmethyl group, Q represents a group represented by Q1-117, and Het represents any substituents indicated in [Table 1A] to [Table 20A] (hereinafter, referred to as Compound Class SX1147).

A compound (L-1) wherein n is 2, $R^1$ represents a cyclopropylmethyl group, Q represents a group represented by Q1-118, and Het represents any substituents indicated in [Table 1A] to [Table 20A] (hereinafter, referred to as Compound Class SX1148).

A compound (L-1) wherein n is 2, $R^1$ represents a cyclopropylmethyl group, Q represents a group represented by Q1-119, and Het represents any substituents indicated in [Table 1A] to [Table 20A] (hereinafter, referred to as Compound

Class SX1149).

A compound (L-1) wherein n is 2, $R^1$ represents a cyclopropylmethyl group, Q represents a group represented by Q1-120, and Het represents any substituents indicated in [Table 1A] to [Table 20A] (hereinafter, referred to as Compound Class SX1150).

A compound (L-1) wherein n is 2, $R^1$ represents a cyclopropylmethyl group, Q represents a group represented by Q1-121, and Het represents any substituents indicated in [Table 1A] to [Table 20A] (hereinafter, referred to as Compound Class SX1151).

A compound (L-1) wherein n is 2, $R^1$ represents a cyclopropylmethyl group, Q represents a group represented by Q1-122, and Het represents any substituents indicated in [Table 1A] to [Table 20A] (hereinafter, referred to as Compound Class SX1152).

A compound (L-1) wherein n is 2, $R^1$ represents a cyclopropylmethyl group, Q represents a group represented by Q1-123, and Het represents any substituents indicated in [Table 1A] to [Table 20A] (hereinafter, referred to as Compound Class SX1153).

A compound (L-1) wherein n is 2, $R^1$ represents a cyclopropylmethyl group, Q represents a group represented by Q1-124, and Het represents any substituents indicated in [Table 1A] to [Table 20A] (hereinafter, referred to as Compound Class SX1154).

A compound (L-1) wherein n is 2, $R^1$ represents a cyclopropylmethyl group, Q represents a group represented by Q1-125, and Het represents any substituents indicated in [Table 1A] to [Table 20A] (hereinafter, referred to as Compound Class SX1155).

A compound (L-1) wherein n is 2, $R^1$ represents a cyclopropylmethyl group, Q represents a group represented by Q1-126, and Het represents any substituents indicated in [Table 1A] to [Table 20A] (hereinafter, referred to as Compound Class SX1156).

A compound (L-1) wherein n is 2, $R^1$ represents a cyclopropylmethyl group, Q represents a group represented by Q1-127, and Het represents any substituents indicated in [Table 1A] to [Table 20A] (hereinafter, referred to as Compound Class SX1157).

A compound (L-1) wherein n is 2, $R^1$ represents a cyclopropylmethyl group, Q represents a group represented by Q1-128, and Het represents any substituents indicated in [Table 1A] to [Table 20A] (hereinafter, referred to as Compound Class SX1158).

A compound (L-1) wherein n is 2, $R^1$ represents a cyclopropylmethyl group, Q represents a group represented by Q1-129, and Het represents any substituents indicated in [Table 1A] to [Table 20A] (hereinafter, referred to as Compound Class SX1159).

A compound (L-1) wherein n is 2, $R^1$ represents a cyclopropylmethyl group, Q represents a group represented by Q1-130, and Het represents any substituents indicated in [Table 1A] to [Table 20A] (hereinafter, referred to as Compound Class SX1160).

A compound (L-1) wherein n is 2, $R^1$ represents a cyclopropylmethyl group, Q represents a group represented by Q1-131, and Het represents any substituents indicated in [Table 1A] to [Table 20A] (hereinafter, referred to as Compound Class SX1161).

A compound (L-1) wherein n is 2, $R^1$ represents a cyclopropylmethyl group, Q represents a group represented by Q1-132, and Het represents any substituents indicated in [Table 1A] to [Table 20A] (hereinafter, referred to as Compound Class SX1162).

A compound (L-1) wherein n is 2, $R^1$ represents a cyclopropylmethyl group, Q represents a group represented by Q1-133, and Het represents any substituents indicated in [Table 1A] to [Table 20A] (hereinafter, referred to as Compound Class SX1163).

A compound (L-1) wherein n is 2, $R^1$ represents a cyclopropylmethyl group, Q represents a group represented by Q1-134, and Het represents any substituents indicated in [Table 1A] to [Table 20A] (hereinafter, referred to as Compound Class SX1164).

A compound (L-1) wherein n is 2, $R^1$ represents a cyclopropylmethyl group, Q represents a group represented by Q1-135, and Het represents any substituents indicated in [Table 1A] to [Table 20A] (hereinafter, referred to as Compound Class SX1165).

A compound (L-1) wherein n is 2, $R^1$ represents a cyclopropylmethyl group, Q represents a group represented by Q1-136, and Het represents any substituents indicated in [Table 1A] to [Table 20A] (hereinafter, referred to as Compound Class SX1166).

A compound (L-1) wherein n is 2, $R^1$ represents a cyclopropylmethyl group, Q represents a group represented by Q1-137, and Het represents any substituents indicated in [Table 1A] to [Table 20A] (hereinafter, referred to as Compound Class SX1167).

A compound (L-1) wherein n is 2, $R^1$ represents a cyclopropylmethyl group, Q represents a group represented by Q1-138, and Het represents any substituents indicated in [Table 1A] to [Table 20A] (hereinafter, referred to as Compound Class SX1168).

A compound (L-1) wherein n is 2, $R^1$ represents a cyclopropylmethyl group, Q represents a group represented by Q1-139, and Het represents any substituents indicated in [Table 1A] to [Table 20A] (hereinafter, referred to as Compound Class SX1169).

A compound (L-1) wherein n is 2, $R^1$ represents a cyclopropylmethyl group, Q represents a group represented by Q1-140, and Het represents any substituents indicated in [Table 1A] to [Table 20A] (hereinafter, referred to as Compound Class SX1170).

A compound (L-1) wherein n is 2, $R^1$ represents a cyclopropylmethyl group, Q represents a group represented by Q1-141, and Het represents any substituents indicated in [Table 1A] to [Table 20A] (hereinafter, referred to as Compound Class SX1171).

A compound (L-1) wherein n is 2, $R^1$ represents a cyclopropylmethyl group, Q represents a group represented by Q1-142, and Het represents any substituents indicated in [Table 1A] to [Table 20A] (hereinafter, referred to as Compound Class SX1172).

A compound (L-1) wherein n is 2, $R^1$ represents a cyclopropylmethyl group, Q represents a group represented by Q1-143, and Het represents any substituents indicated in [Table 1A] to [Table 20A] (hereinafter, referred to as Compound Class SX1173).

A compound (L-1) wherein n is 2, $R^1$ represents a cyclopropylmethyl group, Q represents a group represented by Q1-144, and Het represents any substituents indicated in [Table 1A] to [Table 20A] (hereinafter, referred to as Compound Class SX1174).

A compound (L-1) wherein n is 2, $R^1$ represents a cyclopropylmethyl group, Q represents a group represented by Q1-145, and Het represents any substituents indicated in [Table 1A] to [Table 20A] (hereinafter, referred to as Compound Class SX1175).

A compound (L-1) wherein n is 2, $R^1$ represents a cyclopropylmethyl group, Q represents a group represented by Q1-146, and Het represents any substituents indicated in [Table 1A] to [Table 20A] (hereinafter, referred to as Compound Class SX1176).

A compound (L-1) wherein n is 2, $R^1$ represents a cyclopropylmethyl group, Q represents a group represented by Q1-147, and Het represents any substituents indicated in [Table 1A] to [Table 20A] (hereinafter, referred to as Compound Class SX1177).

A compound (L-1) wherein n is 2, $R^1$ represents a cyclopropylmethyl group, Q represents a group represented by Q1-148, and Het represents any substituents indicated in [Table 1A] to [Table 20A] (hereinafter, referred to as Compound Class SX1178).

A compound (L-1) wherein n is 2, $R^1$ represents a cyclopropylmethyl group, Q represents a group represented by Q1-149, and Het represents any substituents indicated in [Table 1A] to [Table 20A] (hereinafter, referred to as Compound Class SX1179).

A compound (L-1) wherein n is 2, $R^1$ represents a cyclopropylmethyl group, Q represents a group represented by Q1-150, and Het represents any substituents indicated in [Table 1A] to [Table 20A] (hereinafter, referred to as Compound Class SX1180).

A compound (L-1) wherein n is 2, $R^1$ represents a cyclopropylmethyl group, Q represents a group represented by Q1-151, and Het represents any substituents indicated in [Table 1A] to [Table 20A] (hereinafter, referred to as Compound Class SX1181).

A compound (L-1) wherein n is 2, $R^1$ represents a cyclopropylmethyl group, Q represents a group represented by Q1-152, and Het represents any substituents indicated in [Table 1A] to [Table 20A] (hereinafter, referred to as Compound Class SX1182).

A compound (L-1) wherein n is 2, $R^1$ represents a cyclopropylmethyl group, Q represents a group represented by Q1-153, and Het represents any substituents indicated in [Table 1A] to [Table 20A] (hereinafter, referred to as Compound Class SX1183).

A compound (L-1) wherein n is 2, $R^1$ represents a cyclopropylmethyl group, Q represents a group represented by Q1-154, and Het represents any substituents indicated in [Table 1A] to [Table 20A] (hereinafter, referred to as Compound Class SX1184).

A compound (L-1) wherein n is 2, $R^1$ represents a cyclopropylmethyl group, Q represents a group represented by Q1-155, and Het represents any substituents indicated in [Table 1A] to [Table 20A] (hereinafter, referred to as Compound Class SX1185).

A compound (L-1) wherein n is 2, $R^1$ represents a cyclopropylmethyl group, Q represents a group represented by Q1-156, and Het represents any substituents indicated in [Table 1A] to [Table 20A] (hereinafter, referred to as Compound Class SX1186).

A compound (L-1) wherein n is 2, $R^1$ represents a cyclopropylmethyl group, Q represents a group represented by Q1-157, and Het represents any substituents indicated in [Table 1A] to [Table 20A] (hereinafter, referred to as Compound Class SX1187).

A compound (L-1) wherein n is 2, $R^1$ represents a cyclopropylmethyl group, Q represents a group represented by

Q1-158, and Het represents any substituents indicated in [Table 1A] to [Table 20A] (hereinafter, referred to as Compound Class SX1188).

A compound (L-1) wherein n is 2, $R^1$ represents a cyclopropylmethyl group, Q represents a group represented by Q1-159, and Het represents any substituents indicated in [Table 1A] to [Table 20A] (hereinafter, referred to as Compound Class SX1189).

A compound (L-1) wherein n is 2, $R^1$ represents a cyclopropylmethyl group, Q represents a group represented by Q1-160, and Het represents any substituents indicated in [Table 1A] to [Table 20A] (hereinafter, referred to as Compound Class SX1190).

A compound (L-1) wherein n is 2, $R^1$ represents a cyclopropylmethyl group, Q represents a group represented by Q1-161, and Het represents any substituents indicated in [Table 1A] to [Table 20A] (hereinafter, referred to as Compound Class SX1191).

A compound (L-1) wherein n is 2, $R^1$ represents a cyclopropylmethyl group, Q represents a group represented by Q1-162, and Het represents any substituents indicated in [Table 1A] to [Table 20A] (hereinafter, referred to as Compound Class SX1192).

A compound (L-1) wherein n is 2, $R^1$ represents a cyclopropylmethyl group, Q represents a group represented by Q1-163, and Het represents any substituents indicated in [Table 1A] to [Table 20A] (hereinafter, referred to as Compound Class SX1193).

A compound (L-1) wherein n is 2, $R^1$ represents a cyclopropylmethyl group, Q represents a group represented by Q1-164, and Het represents any substituents indicated in [Table 1A] to [Table 20A] (hereinafter, referred to as Compound Class SX1194).

A compound (L-1) wherein n is 2, $R^1$ represents a cyclopropylmethyl group, Q represents a group represented by Q1-165, and Het represents any substituents indicated in [Table 1A] to [Table 20A] (hereinafter, referred to as Compound Class SX1195).

A compound (L-1) wherein n is 2, $R^1$ represents a cyclopropylmethyl group, Q represents a group represented by Q1-166, and Het represents any substituents indicated in [Table 1A] to [Table 20A] (hereinafter, referred to as Compound Class SX1196).

A compound (L-1) wherein n is 2, $R^1$ represents a cyclopropylmethyl group, Q represents a group represented by Q1-167, and Het represents any substituents indicated in [Table 1A] to [Table 20A] (hereinafter, referred to as Compound Class SX1197).

A compound (L-1) wherein n is 2, $R^1$ represents a cyclopropylmethyl group, Q represents a group represented by Q1-168, and Het represents any substituents indicated in [Table 1A] to [Table 20A] (hereinafter, referred to as Compound Class SX1198).

A compound (L-1) wherein n is 2, $R^1$ represents a cyclopropylmethyl group, Q represents a group represented by Q1-169, and Het represents any substituents indicated in [Table 1A] to [Table 20A] (hereinafter, referred to as Compound Class SX1199).

A compound (L-1) wherein n is 2, $R^1$ represents a cyclopropylmethyl group, Q represents a group represented by Q1-170, and Het represents any substituents indicated in [Table 1A] to [Table 20A] (hereinafter, referred to as Compound Class SX1200).

A compound (L-1) wherein n is 2, $R^1$ represents a cyclopropylmethyl group, Q represents a group represented by Q1-171, and Het represents any substituents indicated in [Table 1A] to [Table 20A] (hereinafter, referred to as Compound Class SX1201).

A compound (L-1) wherein n is 2, $R^1$ represents a cyclopropylmethyl group, Q represents a group represented by Q1-172, and Het represents any substituents indicated in [Table 1A] to [Table 20A] (hereinafter, referred to as Compound Class SX1202).

A compound (L-1) wherein n is 2, $R^1$ represents a cyclopropylmethyl group, Q represents a group represented by Q1-173, and Het represents any substituents indicated in [Table 1A] to [Table 20A] (hereinafter, referred to as Compound Class SX1203).

A compound (L-1) wherein n is 2, $R^1$ represents a cyclopropylmethyl group, Q represents a group represented by Q1-174, and Het represents any substituents indicated in [Table 1A] to [Table 20A] (hereinafter, referred to as Compound Class SX1204).

A compound (L-1) wherein n is 2, $R^1$ represents a cyclopropylmethyl group, Q represents a group represented by Q1-175, and Het represents any substituents indicated in [Table 1A] to [Table 20A] (hereinafter, referred to as Compound Class SX1205).

A compound (L-1) wherein n is 2, $R^1$ represents a cyclopropylmethyl group, Q represents a group represented by Q1-176, and Het represents any substituents indicated in [Table 1A] to [Table 20A] (hereinafter, referred to as Compound Class SX1206).

A compound (L-1) wherein n is 2, $R^1$ represents a cyclopropylmethyl group, Q represents a group represented by Q1-177, and Het represents any substituents indicated in [Table 1A] to [Table 20A] (hereinafter, referred to as Compound

Class SX1207).

A compound (L-1) wherein n is 2, $R^1$ represents a cyclopropylmethyl group, Q represents a group represented by Q1-178, and Het represents any substituents indicated in [Table 1A] to [Table 20A] (hereinafter, referred to as Compound Class SX1208).

A compound (L-1) wherein n is 2, $R^1$ represents a cyclopropylmethyl group, Q represents a group represented by Q1-179, and Het represents any substituents indicated in [Table 1A] to [Table 20A] (hereinafter, referred to as Compound Class SX1209).

A compound (L-1) wherein n is 2, $R^1$ represents a cyclopropylmethyl group, Q represents a group represented by Q1-180, and Het represents any substituents indicated in [Table 1A] to [Table 20A] (hereinafter, referred to as Compound Class SX1210).

A compound (L-1) wherein n is 2, $R^1$ represents a cyclopropylmethyl group, Q represents a group represented by Q1-181, and Het represents any substituents indicated in [Table 1A] to [Table 20A] (hereinafter, referred to as Compound Class SX1211).

A compound (L-1) wherein n is 2, $R^1$ represents a cyclopropylmethyl group, Q represents a group represented by Q1-182, and Het represents any substituents indicated in [Table 1A] to [Table 20A] (hereinafter, referred to as Compound Class SX1212).

A compound (L-1) wherein n is 2, $R^1$ represents a cyclopropylmethyl group, Q represents a group represented by Q1-183, and Het represents any substituents indicated in [Table 1A] to [Table 20A] (hereinafter, referred to as Compound Class SX1213).

A compound (L-1) wherein n is 2, $R^1$ represents a cyclopropylmethyl group, Q represents a group represented by Q1-184, and Het represents any substituents indicated in [Table 1A] to [Table 20A] (hereinafter, referred to as Compound Class SX1214).

A compound (L-1) wherein n is 2, $R^1$ represents a cyclopropylmethyl group, Q represents a group represented by Q1-185, and Het represents any substituents indicated in [Table 1A] to [Table 20A] (hereinafter, referred to as Compound Class SX1215).

A compound (L-1) wherein n is 2, $R^1$ represents a cyclopropylmethyl group, Q represents a group represented by Q1-186, and Het represents any substituents indicated in [Table 1A] to [Table 20A] (hereinafter, referred to as Compound Class SX1216).

A compound (L-1) wherein n is 2, $R^1$ represents a cyclopropylmethyl group, Q represents a group represented by Q1-187, and Het represents any substituents indicated in [Table 1A] to [Table 20A] (hereinafter, referred to as Compound Class SX1217).

A compound (L-1) wherein n is 2, $R^1$ represents a cyclopropylmethyl group, Q represents a group represented by Q1-188, and Het represents any substituents indicated in [Table 1A] to [Table 20A] (hereinafter, referred to as Compound Class SX1218).

A compound (L-1) wherein n is 2, $R^1$ represents a cyclopropylmethyl group, Q represents a group represented by Q1-189, and Het represents any substituents indicated in [Table 1A] to [Table 20A] (hereinafter, referred to as Compound Class SX1219).

A compound (L-1) wherein n is 2, $R^1$ represents a cyclopropylmethyl group, Q represents a group represented by Q1-190, and Het represents any substituents indicated in [Table 1A] to [Table 20A] (hereinafter, referred to as Compound Class SX1220).

A compound (L-1) wherein n is 2, $R^1$ represents a cyclopropylmethyl group, Q represents a group represented by Q1-191, and Het represents any substituents indicated in [Table 1A] to [Table 20A] (hereinafter, referred to as Compound Class SX1221).

A compound (L-1) wherein n is 2, $R^1$ represents a cyclopropylmethyl group, Q represents a group represented by Q1-192, and Het represents any substituents indicated in [Table 1A] to [Table 20A] (hereinafter, referred to as Compound Class SX1222).

A compound (L-1) wherein n is 2, $R^1$ represents a cyclopropylmethyl group, Q represents a group represented by Q1-193, and Het represents any substituents indicated in [Table 1A] to [Table 20A] (hereinafter, referred to as Compound Class SX1223).

A compound (L-1) wherein n is 2, $R^1$ represents a cyclopropylmethyl group, Q represents a group represented by Q1-194, and Het represents any substituents indicated in [Table 1A] to [Table 20A] (hereinafter, referred to as Compound Class SX1224).

A compound (L-1) wherein n is 2, $R^1$ represents a cyclopropylmethyl group, Q represents a group represented by Q1-195, and Het represents any substituents indicated in [Table 1A] to [Table 20A] (hereinafter, referred to as Compound Class SX1225).

A compound (L-1) wherein n is 2, $R^1$ represents a cyclopropylmethyl group, Q represents a group represented by Q1-196, and Het represents any substituents indicated in [Table 1A] to [Table 20A] (hereinafter, referred to as Compound Class SX1226).

A compound (L-1) wherein n is 2, $R^1$ represents a cyclopropylmethyl group, Q represents a group represented by Q1-197, and Het represents any substituents indicated in [Table 1A] to [Table 20A] (hereinafter, referred to as Compound Class SX1227).

A compound (L-1) wherein n is 2, $R^1$ represents a cyclopropylmethyl group, Q represents a group represented by Q1-198, and Het represents any substituents indicated in [Table 1A] to [Table 20A] (hereinafter, referred to as Compound Class SX1228).

A compound (L-1) wherein n is 2, $R^1$ represents a cyclopropylmethyl group, Q represents a group represented by Q1-199, and Het represents any substituents indicated in [Table 1A] to [Table 20A] (hereinafter, referred to as Compound Class SX1229).

A compound (L-1) wherein n is 2, $R^1$ represents a cyclopropylmethyl group, Q represents a group represented by Q1-200, and Het represents any substituents indicated in [Table 1A] to [Table 20A] (hereinafter, referred to as Compound Class SX1230).

A compound (L-1) wherein n is 2, $R^1$ represents a cyclopropylmethyl group, Q represents a group represented by Q1-201, and Het represents any substituents indicated in [Table 1A] to [Table 20A] (hereinafter, referred to as Compound Class SX1231).

A compound (L-1) wherein n is 2, $R^1$ represents a cyclopropylmethyl group, Q represents a group represented by Q1-202, and Het represents any substituents indicated in [Table 1A] to [Table 20A] (hereinafter, referred to as Compound Class SX1232).

A compound (L-1) wherein n is 2, $R^1$ represents a cyclopropylmethyl group, Q represents a group represented by Q1-203, and Het represents any substituents indicated in [Table 1A] to [Table 20A] (hereinafter, referred to as Compound Class SX1233).

A compound (L-1) wherein n is 2, $R^1$ represents a cyclopropylmethyl group, Q represents a group represented by Q1-204, and Het represents any substituents indicated in [Table 1A] to [Table 20A] (hereinafter, referred to as Compound Class SX1234).

A compound (L-1) wherein n is 2, $R^1$ represents a cyclopropylmethyl group, Q represents a group represented by Q1-205, and Het represents any substituents indicated in [Table 1A] to [Table 20A] (hereinafter, referred to as Compound Class SX1235).

A compound (L-1) wherein n is 2, $R^1$ represents a cyclopropylmethyl group, Q represents a group represented by Q1-206, and Het represents any substituents indicated in [Table 1A] to [Table 20A] (hereinafter, referred to as Compound Class SX1236).

A compound (L-1) wherein n is 2, $R^1$ represents a cyclopropylmethyl group, Q represents a group represented by Q1-207, and Het represents any substituents indicated in [Table 1A] to [Table 20A] (hereinafter, referred to as Compound Class SX1237).

A compound (L-1) wherein n is 2, $R^1$ represents a cyclopropylmethyl group, Q represents a group represented by Q1-208, and Het represents any substituents indicated in [Table 1A] to [Table 20A] (hereinafter, referred to as Compound Class SX1238).

A compound (L-1) wherein n is 2, $R^1$ represents a cyclopropylmethyl group, Q represents a group represented by Q1-209, and Het represents any substituents indicated in [Table 1A] to [Table 20A] (hereinafter, referred to as Compound Class SX1239).

A compound (L-1) wherein n is 2, $R^1$ represents a cyclopropylmethyl group, Q represents a group represented by Q2-1, and Het represents any substituents indicated in [Table 1A] to [Table 20A] (hereinafter, referred to as Compound Class SX1240).

A compound (L-1) wherein n is 2, $R^1$ represents a cyclopropylmethyl group, Q represents a group represented by Q2-2, and Het represents any substituents indicated in [Table 1A] to [Table 20A] (hereinafter, referred to as Compound Class SX1241).

A compound (L-1) wherein n is 2, $R^1$ represents a cyclopropylmethyl group, Q represents a group represented by Q2-3, and Het represents any substituents indicated in [Table 1A] to [Table 20A] (hereinafter, referred to as Compound Class SX1242).

A compound (L-1) wherein n is 2, $R^1$ represents a cyclopropylmethyl group, Q represents a group represented by Q2-4, and Het represents any substituents indicated in [Table 1A] to [Table 20A] (hereinafter, referred to as Compound Class SX1243).

A compound (L-1) wherein n is 2, $R^1$ represents a cyclopropylmethyl group, Q represents a group represented by Q2-5, and Het represents any substituents indicated in [Table 1A] to [Table 20A] (hereinafter, referred to as Compound Class SX1244).

A compound (L-1) wherein n is 2, $R^1$ represents a cyclopropylmethyl group, Q represents a group represented by Q2-6, and Het represents any substituents indicated in [Table 1A] to [Table 20A] (hereinafter, referred to as Compound Class SX1245).

A compound (L-1) wherein n is 2, $R^1$ represents a cyclopropylmethyl group, Q represents a group represented by

Q2-7, and Het represents any substituents indicated in [Table 1A] to [Table 20A] (hereinafter, referred to as Compound Class SX1246).

A compound (L-1) wherein n is 2, $R^1$ represents a cyclopropylmethyl group, Q represents a group represented by Q2-8, and Het represents any substituents indicated in [Table 1A] to [Table 20A] (hereinafter, referred to as Compound Class SX1247).

A compound (L-1) wherein n is 2, $R^1$ represents a cyclopropylmethyl group, Q represents a group represented by Q2-9, and Het represents any substituents indicated in [Table 1A] to [Table 20A] (hereinafter, referred to as Compound Class SX1248).

A compound (L-1) wherein n is 2, $R^1$ represents a cyclopropylmethyl group, Q represents a group represented by Q2-10, and Het represents any substituents indicated in [Table 1A] to [Table 20A] (hereinafter, referred to as Compound Class SX1249).

A compound (L-1) wherein n is 2, $R^1$ represents a cyclopropylmethyl group, Q represents a group represented by Q2-11, and Het represents any substituents indicated in [Table 1A] to [Table 20A] (hereinafter, referred to as Compound Class SX1250).

A compound (L-1) wherein n is 2, $R^1$ represents a cyclopropylmethyl group, Q represents a group represented by Q2-12, and Het represents any substituents indicated in [Table 1A] to [Table 20A] (hereinafter, referred to as Compound Class SX1251).

A compound (L-1) wherein n is 2, $R^1$ represents a cyclopropylmethyl group, Q represents a group represented by Q2-13, and Het represents any substituents indicated in [Table 1A] to [Table 20A] (hereinafter, referred to as Compound Class SX1252).

A compound (L-1) wherein n is 2, $R^1$ represents a cyclopropylmethyl group, Q represents a group represented by Q2-14, and Het represents any substituents indicated in [Table 1A] to [Table 20A] (hereinafter, referred to as Compound Class SX1253).

A compound (L-1) wherein n is 2, $R^1$ represents a cyclopropylmethyl group, Q represents a group represented by Q2-15, and Het represents any substituents indicated in [Table 1A] to [Table 20A] (hereinafter, referred to as Compound Class SX1254).

A compound (L-1) wherein n is 2, $R^1$ represents a cyclopropylmethyl group, Q represents a group represented by Q2-16, and Het represents any substituents indicated in [Table 1A] to [Table 20A] (hereinafter, referred to as Compound Class SX1255).

A compound (L-1) wherein n is 2, $R^1$ represents a cyclopropylmethyl group, Q represents a group represented by Q2-17, and Het represents any substituents indicated in [Table 1A] to [Table 20A] (hereinafter, referred to as Compound Class SX1256).

A compound (L-1) wherein n is 2, $R^1$ represents a cyclopropylmethyl group, Q represents a group represented by Q2-18, and Het represents any substituents indicated in [Table 1A] to [Table 20A] (hereinafter, referred to as Compound Class SX1257).

A compound (L-1) wherein n is 2, $R^1$ represents a cyclopropylmethyl group, Q represents a group represented by Q2-19, and Het represents any substituents indicated in [Table 1A] to [Table 20A] (hereinafter, referred to as Compound Class SX1258).

A compound (L-1) wherein n is 2, $R^1$ represents a cyclopropylmethyl group, Q represents a group represented by Q2-20, and Het represents any substituents indicated in [Table 1A] to [Table 20A] (hereinafter, referred to as Compound Class SX1259).

A compound (L-1) wherein n is 2, $R^1$ represents a cyclopropylmethyl group, Q represents a group represented by Q2-21, and Het represents any substituents indicated in [Table 1A] to [Table 20A] (hereinafter, referred to as Compound Class SX1260).

A compound (L-1) wherein n is 2, $R^1$ represents a cyclopropylmethyl group, Q represents a group represented by Q2-22, and Het represents any substituents indicated in [Table 1A] to [Table 20A] (hereinafter, referred to as Compound Class SX1261).

A compound (L-1) wherein n is 2, $R^1$ represents a cyclopropylmethyl group, Q represents a group represented by Q2-23, and Het represents any substituents indicated in [Table 1A] to [Table 20A] (hereinafter, referred to as Compound Class SX1262).

A compound (L-1) wherein n is 2, $R^1$ represents a cyclopropylmethyl group, Q represents a group represented by Q2-24, and Het represents any substituents indicated in [Table 1A] to [Table 20A] (hereinafter, referred to as Compound Class SX1263).

A compound (L-1) wherein n is 2, $R^1$ represents a cyclopropylmethyl group, Q represents a group represented by Q2-25, and Het represents any substituents indicated in [Table 1A] to [Table 20A] (hereinafter, referred to as Compound Class SX1264).

A compound (L-1) wherein n is 2, $R^1$ represents a cyclopropylmethyl group, Q represents a group represented by Q2-26, and Het represents any substituents indicated in [Table 1A] to [Table 20A] (hereinafter, referred to as Compound

Class SX1265).

A compound (L-1) wherein n is 2, $R^1$ represents a cyclopropylmethyl group, Q represents a group represented by Q2-27, and Het represents any substituents indicated in [Table 1A] to [Table 20A] (hereinafter, referred to as Compound Class SX1266).

A compound (L-1) wherein n is 2, $R^1$ represents a cyclopropylmethyl group, Q represents a group represented by Q2-28, and Het represents any substituents indicated in [Table 1A] to [Table 20A] (hereinafter, referred to as Compound Class SX1267).

A compound (L-1) wherein n is 2, $R^1$ represents a cyclopropylmethyl group, Q represents a group represented by Q2-29, and Het represents any substituents indicated in [Table 1A] to [Table 20A] (hereinafter, referred to as Compound Class SX1268).

A compound (L-1) wherein n is 2, $R^1$ represents a cyclopropylmethyl group, Q represents a group represented by Q2-30, and Het represents any substituents indicated in [Table 1A] to [Table 20A] (hereinafter, referred to as Compound Class SX1269).

A compound (L-1) wherein n is 2, $R^1$ represents a cyclopropylmethyl group, Q represents a group represented by Q2-31, and Het represents any substituents indicated in [Table 1A] to [Table 20A] (hereinafter, referred to as Compound Class SX1270).

A compound (L-1) wherein n is 2, $R^1$ represents a cyclopropylmethyl group, Q represents a group represented by Q5-1, and Het represents any substituents indicated in [Table 1A] to [Table 20A] (hereinafter, referred to as Compound Class SX1271).

A compound (L-1) wherein n is 2, $R^1$ represents a cyclopropylmethyl group, Q represents a group represented by Q5-2, and Het represents any substituents indicated in [Table 1A] to [Table 20A] (hereinafter, referred to as Compound Class SX1272).

A compound (L-1) wherein n is 2, $R^1$ represents a cyclopropylmethyl group, Q represents a group represented by Q5-3, and Het represents any substituents indicated in [Table 1A] to [Table 20A] (hereinafter, referred to as Compound Class SX1273).

A compound (L-1) wherein n is 2, $R^1$ represents a cyclopropylmethyl group, Q represents a group represented by Q5-4, and Het represents any substituents indicated in [Table 1A] to [Table 20A] (hereinafter, referred to as Compound Class SX1274).

A compound (L-1) wherein n is 2, $R^1$ represents a cyclopropylmethyl group, Q represents a group represented by Q5-5, and Het represents any substituents indicated in [Table 1A] to [Table 20A] (hereinafter, referred to as Compound Class SX1275).

A compound (L-1) wherein n is 2, $R^1$ represents a cyclopropylmethyl group, Q represents a group represented by Q5-6, and Het represents any substituents indicated in [Table 1A] to [Table 20A] (hereinafter, referred to as Compound Class SX1276).

A compound (L-1) wherein n is 2, $R^1$ represents a cyclopropylmethyl group, Q represents a group represented by Q5-7, and Het represents any substituents indicated in [Table 1A] to [Table 20A] (hereinafter, referred to as Compound Class SX1277).

A compound (L-1) wherein n is 2, $R^1$ represents a cyclopropylmethyl group, Q represents a group represented by Q5-8, and Het represents any substituents indicated in [Table 1A] to [Table 20A] (hereinafter, referred to as Compound Class SX1278).

A compound (L-1) wherein n is 2, $R^1$ represents a cyclopropylmethyl group, Q represents a group represented by Q5-9, and Het represents any substituents indicated in [Table 1A] to [Table 20A] (hereinafter, referred to as Compound Class SX1279).

A compound (L-1) wherein n is 2, $R^1$ represents a cyclopropylmethyl group, Q represents a group represented by Q5-10, and Het represents any substituents indicated in [Table 1A] to [Table 20A] (hereinafter, referred to as Compound Class SX1280).

A compound (L-1) wherein n is 2, $R^1$ represents a cyclopropylmethyl group, Q represents a group represented by Q5-11, and Het represents any substituents indicated in [Table 1A] to [Table 20A] (hereinafter, referred to as Compound Class SX1281).

A compound (L-1) wherein n is 2, $R^1$ represents a cyclopropylmethyl group, Q represents a group represented by Q5-12, and Het represents any substituents indicated in [Table 1A] to [Table 20A] (hereinafter, referred to as Compound Class SX1282).

A compound (L-1) wherein n is 2, $R^1$ represents a cyclopropylmethyl group, Q represents a group represented by Q5-13, and Het represents any substituents indicated in [Table 1A] to [Table 20A] (hereinafter, referred to as Compound Class SX1283).

A compound (L-1) wherein n is 2, $R^1$ represents a cyclopropylmethyl group, Q represents a group represented by Q5-14, and Het represents any substituents indicated in [Table 1A] to [Table 20A] (hereinafter, referred to as Compound Class SX1284).

A compound (L-1) wherein n is 2, $R^1$ represents a cyclopropylmethyl group, Q represents a group represented by Q5-15, and Het represents any substituents indicated in [Table 1A] to [Table 20A] (hereinafter, referred to as Compound Class SX1285).

A compound (L-1) wherein n is 2, $R^1$ represents a cyclopropylmethyl group, Q represents a group represented by Q5-16, and Het represents any substituents indicated in [Table 1A] to [Table 20A] (hereinafter, referred to as Compound Class SX1286).

A compound (L-1) wherein n is 2, $R^1$ represents a cyclopropylmethyl group, Q represents a group represented by Q5-17, and Het represents any substituents indicated in [Table 1A] to [Table 20A] (hereinafter, referred to as Compound Class SX1287).

A compound (L-1) wherein n is 2, $R^1$ represents a cyclopropylmethyl group, Q represents a group represented by Q5-18, and Het represents any substituents indicated in [Table 1A] to [Table 20A] (hereinafter, referred to as Compound Class SX1288).

A compound (L-1) wherein n is 2, $R^1$ represents a cyclopropylmethyl group, Q represents a group represented by Q5-19, and Het represents any substituents indicated in [Table 1A] to [Table 20A] (hereinafter, referred to as Compound Class SX1289).

A compound (L-1) wherein n is 2, $R^1$ represents a cyclopropylmethyl group, Q represents a group represented by Q5-20, and Het represents any substituents indicated in [Table 1A] to [Table 20A] (hereinafter, referred to as Compound Class SX1290).

A compound (L-1) wherein n is 2, $R^1$ represents a cyclopropylmethyl group, Q represents a group represented by Q5-21, and Het represents any substituents indicated in [Table 1A] to [Table 20A] (hereinafter, referred to as Compound Class SX1291).

A compound (L-1) wherein n is 2, $R^1$ represents a cyclopropylmethyl group, Q represents a group represented by Q5-22, and Het represents any substituents indicated in [Table 1A] to [Table 20A] (hereinafter, referred to as Compound Class SX1292).

A compound (L-1) wherein n is 2, $R^1$ represents a cyclopropylmethyl group, Q represents a group represented by Q6-1, and Het represents any substituents indicated in [Table 1A] to [Table 20A] (hereinafter, referred to as Compound Class SX1293).

A compound (L-1) wherein n is 2, $R^1$ represents a cyclopropylmethyl group, Q represents a group represented by Q6-2, and Het represents any substituents indicated in [Table 1A] to [Table 20A] (hereinafter, referred to as Compound Class SX1294).

A compound (L-1) wherein n is 2, $R^1$ represents a cyclopropylmethyl group, Q represents a group represented by Q6-3, and Het represents any substituents indicated in [Table 1A] to [Table 20A] (hereinafter, referred to as Compound Class SX1295).

A compound (L-1) wherein n is 2, $R^1$ represents a cyclopropylmethyl group, Q represents a group represented by Q6-4, and Het represents any substituents indicated in [Table 1A] to [Table 20A] (hereinafter, referred to as Compound Class SX1296).

A compound (L-1) wherein n is 2, $R^1$ represents a cyclopropylmethyl group, Q represents a group represented by Q6-5, and Het represents any substituents indicated in [Table 1A] to [Table 20A] (hereinafter, referred to as Compound Class SX1297).

A compound (L-1) wherein n is 2, $R^1$ represents a cyclopropylmethyl group, Q represents a group represented by Q6-6, and Het represents any substituents indicated in [Table 1A] to [Table 20A] (hereinafter, referred to as Compound Class SX1298).

A compound (L-1) wherein n is 2, $R^1$ represents a cyclopropylmethyl group, Q represents a group represented by Q6-7, and Het represents any substituents indicated in [Table 1A] to [Table 20A] (hereinafter, referred to as Compound Class SX1299).

A compound (L-1) wherein n is 2, $R^1$ represents a cyclopropylmethyl group, Q represents a group represented by Q6-8, and Het represents any substituents indicated in [Table 1A] to [Table 20A] (hereinafter, referred to as Compound Class SX1300).

A compound (L-1) wherein n is 2, $R^1$ represents a cyclopropylmethyl group, Q represents a group represented by Q6-9, and Het represents any substituents indicated in [Table 1A] to [Table 20A] (hereinafter, referred to as Compound Class SX1301).

A compound (L-1) wherein n is 2, $R^1$ represents a cyclopropylmethyl group, Q represents a group represented by Q6-10, and Het represents any substituents indicated in [Table 1A] to [Table 20A] (hereinafter, referred to as Compound Class SX1302).

A compound (L-1) wherein n is 2, $R^1$ represents a cyclopropylmethyl group, Q represents a group represented by Q6-11, and Het represents any substituents indicated in [Table 1A] to [Table 20A] (hereinafter, referred to as Compound Class SX1303).

A compound (L-1) wherein n is 2, $R^1$ represents a cyclopropylmethyl group, Q represents a group represented by

Q6-12, and Het represents any substituents indicated in [Table 1A] to [Table 20A] (hereinafter, referred to as Compound Class SX1304).

A compound (L-1) wherein n is 2, $R^1$ represents a cyclopropylmethyl group, Q represents a group represented by Q6-13, and Het represents any substituents indicated in [Table 1A] to [Table 20A] (hereinafter, referred to as Compound Class SX1305).

A compound (L-1) wherein n is 2, $R^1$ represents a cyclopropylmethyl group, Q represents a group represented by Q6-14, and Het represents any substituents indicated in [Table 1A] to [Table 20A] (hereinafter, referred to as Compound Class SX1306).

A compound (L-1) wherein n is 2, $R^1$ represents a cyclopropylmethyl group, Q represents a group represented by Q6-15, and Het represents any substituents indicated in [Table 1A] to [Table 20A] (hereinafter, referred to as Compound Class SX1307).

A compound (L-1) wherein n is 2, $R^1$ represents a cyclopropylmethyl group, Q represents a group represented by Q6-16, and Het represents any substituents indicated in [Table 1A] to [Table 20A] (hereinafter, referred to as Compound Class SX1308).

A compound (L-1) wherein n is 2, $R^1$ represents a cyclopropylmethyl group, Q represents a group represented by Q6-17, and Het represents any substituents indicated in [Table 1A] to [Table 20A] (hereinafter, referred to as Compound Class SX1309).

A compound (L-1) wherein n is 2, $R^1$ represents a cyclopropylmethyl group, Q represents a group represented by Q6-18, and Het represents any substituents indicated in [Table 1A] to [Table 20A] (hereinafter, referred to as Compound Class SX1310).

A compound (L-1) wherein n is 2, $R^1$ represents a cyclopropylmethyl group, Q represents a group represented by Q6-19, and Het represents any substituents indicated in [Table 1A] to [Table 20A] (hereinafter, referred to as Compound Class SX1311).

A compound (L-1) wherein n is 2, $R^1$ represents a cyclopropylmethyl group, Q represents a group represented by Q6-20, and Het represents any substituents indicated in [Table 1A] to [Table 20A] (hereinafter, referred to as Compound Class SX1312).

A compound (L-1) wherein n is 2, $R^1$ represents a cyclopropylmethyl group, Q represents a group represented by Q6-21, and Het represents any substituents indicated in [Table 1A] to [Table 20A] (hereinafter, referred to as Compound Class SX1313).

A compound (L-1) wherein n is 2, $R^1$ represents a cyclopropylmethyl group, Q represents a group represented by Q6-22, and Het represents any substituents indicated in [Table 1A] to [Table 20A] (hereinafter, referred to as Compound Class SX1314).

A compound (L-1) wherein n is 0, $R^1$ represents a methyl group, Q represents a group represented by Q1-210, and Het represents any substituents indicated in [Table 1A] to [Table 20A] (hereinafter, referred to as Compound Class SX1315).

A compound (L-1) wherein n is 0, $R^1$ represents a methyl group, Q represents a group represented by Q1-211, and Het represents any substituents indicated in [Table 1A] to [Table 20A] (hereinafter, referred to as Compound Class SX1316).

A compound (L-1) wherein n is 0, $R^1$ represents a methyl group, Q represents a group represented by Q5-23, and Het represents any substituents indicated in [Table 1A] to [Table 20A] (hereinafter, referred to as Compound Class SX1317).

A compound (L-1) wherein n is 0, $R^1$ represents a methyl group, Q represents a group represented by Q5-24, and Het represents any substituents indicated in [Table 1A] to [Table 20A] (hereinafter, referred to as Compound Class SX1318).

A compound (L-1) wherein n is 0, $R^1$ represents a methyl group, Q represents a group represented by Q5-25, and Het represents any substituents indicated in [Table 1A] to [Table 20A] (hereinafter, referred to as Compound Class SX1319).

A compound (L-1) wherein n is 0, $R^1$ represents a methyl group, Q represents a group represented by Q5-26, and Het represents any substituents indicated in [Table 1A] to [Table 20A] (hereinafter, referred to as Compound Class SX1320).

A compound (L-1) wherein n is 2, $R^1$ represents a methyl group, Q represents a group represented by Q1-210, and Het represents any substituents indicated in [Table 1A] to [Table 20A] (hereinafter, referred to as Compound Class SX1321).

A compound (L-1) wherein n is 2, $R^1$ represents a methyl group, Q represents a group represented by Q1-211, and Het represents any substituents indicated in [Table 1A] to [Table 20A] (hereinafter, referred to as Compound Class SX1322).

A compound (L-1) wherein n is 2, $R^1$ represents a methyl group, Q represents a group represented by Q2-32, and Het represents any substituents indicated in [Table 1A] to [Table 20A] (hereinafter, referred to as Compound Class

SX1323).

A compound (L-1) wherein n is 2, $R^1$ represents a methyl group, Q represents a group represented by Q2-33, and Het represents any substituents indicated in [Table 1A] to [Table 20A] (hereinafter, referred to as Compound Class SX1324).

A compound (L-1) wherein n is 2, $R^1$ represents a methyl group, Q represents a group represented by Q2-34, and Het represents any substituents indicated in [Table 1A] to [Table 20A] (hereinafter, referred to as Compound Class SX1325).

A compound (L-1) wherein n is 2, $R^1$ represents a methyl group, Q represents a group represented by Q2-35, and Het represents any substituents indicated in [Table 1A] to [Table 20A] (hereinafter, referred to as Compound Class SX1326).

A compound (L-1) wherein n is 2, $R^1$ represents a methyl group, Q represents a group represented by Q2-36, and Het represents any substituents indicated in [Table 1A] to [Table 20A] (hereinafter, referred to as Compound Class SX1327).

A compound (L-1) wherein n is 2, $R^1$ represents a methyl group, Q represents a group represented by Q5-23, and Het represents any substituents indicated in [Table 1A] to [Table 20A] (hereinafter, referred to as Compound Class SX1328).

A compound (L-1) wherein n is 2, $R^1$ represents a methyl group, Q represents a group represented by Q5-24, and Het represents any substituents indicated in [Table 1A] to [Table 20A] (hereinafter, referred to as Compound Class SX1329).

A compound (L-1) wherein n is 2, $R^1$ represents a methyl group, Q represents a group represented by Q5-25, and Het represents any substituents indicated in [Table 1A] to [Table 20A] (hereinafter, referred to as Compound Class SX1330).

A compound (L-1) wherein n is 2, $R^1$ represents a methyl group, Q represents a group represented by Q5-26, and Het represents any substituents indicated in [Table 1A] to [Table 20A] (hereinafter, referred to as Compound Class SX1331).

A compound (L-1) wherein n is 2, $R^1$ represents a methyl group, Q represents a group represented by Q6-23, and Het represents any substituents indicated in [Table 1A] to [Table 20A] (hereinafter, referred to as Compound Class SX1332).

A compound (L-1) wherein n is 2, $R^1$ represents a methyl group, Q represents a group represented by Q6-24, and Het represents any substituents indicated in [Table 1A] to [Table 20A] (hereinafter, referred to as Compound Class SX1333).

A compound (L-1) wherein n is 2, $R^1$ represents a methyl group, Q represents a group represented by Q6-25, and Het represents any substituents indicated in [Table 1A] to [Table 20A] (hereinafter, referred to as Compound Class SX1334).

A compound (L-1) wherein n is 2, $R^1$ represents a methyl group, Q represents a group represented by Q6-26, and Het represents any substituents indicated in [Table 1A] to [Table 20A] (hereinafter, referred to as Compound Class SX1335).

A compound (L-1) wherein n is 0, $R^1$ represents an ethyl group, Q represents a group represented by Q1-210, and Het represents any substituents indicated in [Table 1A] to [Table 20A] (hereinafter, referred to as Compound Class SX1336).

A compound (L-1) wherein n is 0, $R^1$ represents an ethyl group, Q represents a group represented by Q1-211, and Het represents any substituents indicated in [Table 1A] to [Table 20A] (hereinafter, referred to as Compound Class SX1337).

A compound (L-1) wherein n is 0, $R^1$ represents an ethyl group, Q represents a group represented by Q5-23, and Het represents any substituents indicated in [Table 1A] to [Table 20A] (hereinafter, referred to as Compound Class SX1338).

A compound (L-1) wherein n is 0, $R^1$ represents an ethyl group, Q represents a group represented by Q5-24, and Het represents any substituents indicated in [Table 1A] to [Table 20A] (hereinafter, referred to as Compound Class SX1339).

A compound (L-1) wherein n is 0, $R^1$ represents an ethyl group, Q represents a group represented by Q5-25, and Het represents any substituents indicated in [Table 1A] to [Table 20A] (hereinafter, referred to as Compound Class SX1340).

A compound (L-1) wherein n is 0, $R^1$ represents an ethyl group, Q represents a group represented by Q5-26, and Het represents any substituents indicated in [Table 1A] to [Table 20A] (hereinafter, referred to as Compound Class SX1341).

A compound (L-1) wherein n is 2, $R^1$ represents an ethyl group, Q represents a group represented by Q1-210, and Het represents any substituents indicated in [Table 1A] to [Table 20A] (hereinafter, referred to as Compound Class SX1342).

A compound (L-1) wherein n is 2, $R^1$ represents an ethyl group, Q represents a group represented by Q1-211, and Het represents any substituents indicated in [Table 1A] to [Table 20A] (hereinafter, referred to as Compound Class SX1343).

A compound (L-1) wherein n is 2, $R^1$ represents an ethyl group, Q represents a group represented by Q2-32, and Het represents any substituents indicated in [Table 1A] to [Table 20A] (hereinafter, referred to as Compound Class SX1344).

A compound (L-1) wherein n is 2, $R^1$ represents an ethyl group, Q represents a group represented by Q2-33, and Het represents any substituents indicated in [Table 1A] to [Table 20A] (hereinafter, referred to as Compound Class SX1345).

A compound (L-1) wherein n is 2, $R^1$ represents an ethyl group, Q represents a group represented by Q2-34, and Het represents any substituents indicated in [Table 1A] to [Table 20A] (hereinafter, referred to as Compound Class SX1346).

A compound (L-1) wherein n is 2, $R^1$ represents an ethyl group, Q represents a group represented by Q2-35, and Het represents any substituents indicated in [Table 1A] to [Table 20A] (hereinafter, referred to as Compound Class SX1347).

A compound (L-1) wherein n is 2, $R^1$ represents an ethyl group, Q represents a group represented by Q2-36, and Het represents any substituents indicated in [Table 1A] to [Table 20A] (hereinafter, referred to as Compound Class SX1348).

A compound (L-1) wherein n is 2, $R^1$ represents an ethyl group, Q represents a group represented by Q5-23, and Het represents any substituents indicated in [Table 1A] to [Table 20A] (hereinafter, referred to as Compound Class SX1349).

A compound (L-1) wherein n is 2, $R^1$ represents an ethyl group, Q represents a group represented by Q5-24, and Het represents any substituents indicated in [Table 1A] to [Table 20A] (hereinafter, referred to as Compound Class SX1350).

A compound (L-1) wherein n is 2, $R^1$ represents an ethyl group, Q represents a group represented by Q5-25, and Het represents any substituents indicated in [Table 1A] to [Table 20A] (hereinafter, referred to as Compound Class SX1351).

A compound (L-1) wherein n is 2, $R^1$ represents an ethyl group, Q represents a group represented by Q5-26, and Het represents any substituents indicated in [Table 1A] to [Table 20A] (hereinafter, referred to as Compound Class SX1352).

A compound (L-1) wherein n is 2, $R^1$ represents an ethyl group, Q represents a group represented by Q6-23, and Het represents any substituents indicated in [Table 1A] to [Table 20A] (hereinafter, referred to as Compound Class SX1353).

A compound (L-1) wherein n is 2, $R^1$ represents an ethyl group, Q represents a group represented by Q6-24, and Het represents any substituents indicated in [Table 1A] to [Table 20A] (hereinafter, referred to as Compound Class SX1354).

A compound (L-1) wherein n is 2, $R^1$ represents an ethyl group, Q represents a group represented by Q6-25, and Het represents any substituents indicated in [Table 1A] to [Table 20A] (hereinafter, referred to as Compound Class SX1355).

A compound (L-1) wherein n is 2, $R^1$ represents an ethyl group, Q represents a group represented by Q6-26, and Het represents any substituents indicated in [Table 1A] to [Table 20A] (hereinafter, referred to as Compound Class SX1356).

A compound (L-1) wherein n is 2, $R^1$ represents a cyclopropylmethyl group, Q represents a group represented by Q1-210, and Het represents any substituents indicated in [Table 1A] to [Table 20A] (hereinafter, referred to as Compound Class SX1357).

A compound (L-1) wherein n is 2, $R^1$ represents a cyclopropylmethyl group, Q represents a group represented by Q1-211, and Het represents any substituents indicated in [Table 1A] to [Table 20A] (hereinafter, referred to as Compound Class SX1358).

A compound (L-1) wherein n is 2, $R^1$ represents a cyclopropylmethyl group, Q represents a group represented by Q2-32, and Het represents any substituents indicated in [Table 1A] to [Table 20A] (hereinafter, referred to as Compound Class SX1359).

A compound (L-1) wherein n is 2, $R^1$ represents a cyclopropylmethyl group, Q represents a group represented by Q2-33, and Het represents any substituents indicated in [Table 1A] to [Table 20A] (hereinafter, referred to as Compound Class SX1360).

A compound (L-1) wherein n is 2, $R^1$ represents a cyclopropylmethyl group, Q represents a group represented by Q2-34, and Het represents any substituents indicated in [Table 1A] to [Table 20A] (hereinafter, referred to as Compound Class SX1361).

A compound (L-1) wherein n is 2, $R^1$ represents a cyclopropylmethyl group, Q represents a group represented by

Q2-35, and Het represents any substituents indicated in [Table 1A] to [Table 20A] (hereinafter, referred to as Compound Class SX1362).

A compound (L-1) wherein n is 2, $R^1$ represents a cyclopropylmethyl group, Q represents a group represented by Q2-36, and Het represents any substituents indicated in [Table 1A] to [Table 20A] (hereinafter, referred to as Compound Class SX1363).

A compound (L-1) wherein n is 2, $R^1$ represents a cyclopropylmethyl group, Q represents a group represented by Q5-23, and Het represents any substituents indicated in [Table 1A] to [Table 20A] (hereinafter, referred to as Compound Class SX1364).

A compound (L-1) wherein n is 2, $R^1$ represents a cyclopropylmethyl group, Q represents a group represented by Q5-24, and Het represents any substituents indicated in [Table 1A] to [Table 20A] (hereinafter, referred to as Compound Class SX1365).

A compound (L-1) wherein n is 2, $R^1$ represents a cyclopropylmethyl group, Q represents a group represented by Q5-25, and Het represents any substituents indicated in [Table 1A] to [Table 20A] (hereinafter, referred to as Compound Class SX1366).

A compound (L-1) wherein n is 2, $R^1$ represents a cyclopropylmethyl group, Q represents a group represented by Q5-26, and Het represents any substituents indicated in [Table 1A] to [Table 20A] (hereinafter, referred to as Compound Class SX1367).

A compound (L-1) wherein n is 2, $R^1$ represents a cyclopropylmethyl group, Q represents a group represented by Q6-23, and Het represents any substituents indicated in [Table 1A] to [Table 20A] (hereinafter, referred to as Compound Class SX1368).

A compound (L-1) wherein n is 2, $R^1$ represents a cyclopropylmethyl group, Q represents a group represented by Q6-24, and Het represents any substituents indicated in [Table 1A] to [Table 20A] (hereinafter, referred to as Compound Class SX1369).

A compound (L-1) wherein n is 2, $R^1$ represents a cyclopropylmethyl group, Q represents a group represented by Q6-25, and Het represents any substituents indicated in [Table 1A] to [Table 20A] (hereinafter, referred to as Compound Class SX1370).

A compound (L-1) wherein n is 2, $R^1$ represents a cyclopropylmethyl group, Q represents a group represented by Q6-26, and Het represents any substituents indicated in [Table 1A] to [Table 20A] (hereinafter, referred to as Compound Class SX1371).

Next, Formulation Examples of the Present compound are shown below. The "part(s)" represents "part(s) by weight". Also, the expression of "Present compound S" represents the compounds described in the Compound classes SX1 to SX1371.

Formulation Example 1

A mixture of polyoxyethylene alkyl ether sulfate ammonium salt and silica (weight ratio of 1 : 1) (35 parts), any one of the Present compound S (10 parts), and water (55 parts) are mixed, and the resulting mixture is subjected to fine grinding according to a wet grinding method to obtain each formulation.

Formulation Example 2

Any one of the Present compound S (50 parts), calcium lignin sulfonate (3 parts), sodium lauryl sulfate (2 parts), and silica (45 parts) are ground and mixed to obtain each formulation.

Formulation Example 3

Any one of the Present compound S (5 parts), polyoxyethylene styryl phenyl ether (9 parts), polyoxyethylene decyl ether (number of added ethyleneoxide: 5) (5 parts), calcium dodecylbenzene sulfonate (6 parts), and xylene (75 parts) are mixed to obtain each formulation.

Formulation Example 4

Any one of the Present compound S (2 parts), silica (1 part), calcium lignin sulfonate (2 parts), bentonite (30 parts), and kaolin clay (65 parts) are ground and mixed, an appropriate amount of water is added thereto, the resulting mixture is kneaded, subjected to granulation with a granulator, and then dried to obtain each formulation.

Formulation Example 5

Any one of the Present compound S (10 parts), and a mixture of benzyl alcohol (18 parts) and DMSO (9 parts) are mixed, GERONOL (registered trademark) TE250 (6.3 parts), Ethylan (registered trademark) NS-500LQ (2.7 parts), and solvent naphtha (54 parts) are added thereto, and the resulting mixture is mixed to obtain each formulation.

Formulation Example 6

Any one of the Present compound S (0.1 part) is mixed with kerosene (39.9 parts) and dissolved therein, the resulting solution is placed into an aerosol container, and the container is filled with liquefied petroleum gas (a mixture of propane, butane, and isobutane; saturated vapor pressure: 0.47 MPa (25°C)) (60 parts) to obtain each formulation.

Formulation Example 7

Any one of the Present compound S (0.2 part), lees powder extracted from pyrethrum (50 parts), Tabu powder (30 parts), and wood powder (19.8 parts) are mixed, an appropriate amount of water is added thereto, the resulting mixture is kneaded, then subjected to an extruder to obtain a plate sheet, and the plate sheet is subjected to a punching machine to be converted into a spiral shape to obtain each formulation.

Next, an efficacy of the present compound on controlling harmful arthropods is shown by Test examples. The following tests in the test examples below were conducted at 25°C.

Test Method 1

Test compounds are made to a formulation according to a similar method to that described in the Formulation example 1, and thereto is added water containing 0.03 v/v% of Shindain (registered trademark) to prepare a diluted solution containing a prescribed concentration of the test compound.

Cucumber (*cucumber sativus*) seedling (on the developmental stage of the second true leaf) is planted in a cup, and approximately 30 cotton aphids (*Aphis gossypii*) (all stages of life) are released onto the seedling. After one day, the diluted solutions are sprayed into the seedling at a ratio of 10 mL/seedling. After 5 days, the number of the surviving insects is examined and the controlling value is calculated by the following equation.

$$\text{Controlling value (\%)} = \{1 - (Cb \times Tai)/(Cai \times Tb)\} \times 100$$

wherein the symbols in the equation represent the following descriptions.

Cb: Number of the test insects in untreated group;
Cai: Number of the surviving insects at the time of the examination in untreated group;
Tb: Number of the test insects in treated group;
Tai: Number of the surviving insects at the time of the examination in treated group;

Here the "untreated group" represents a group where a similar treatment procedure to that of treated group except not using the test compound is done.

Test Example 1-1

The test was conducted according to the Test method 1 by making the prescribed concentration 500 ppm and using the below-mentioned present compounds as a test compound, and, as the result of the test, the below-mentioned present compounds showed 90% or more as the controlling value. Present compound Nos: 2, 18, 22, 23, 24, 26, 27, 29, 31, 33, 34, 35, 40, 41, 42, 44, 52, 54, 55, 56, 69, 70, 75, 76, 78, 79, 96, 102, 109, 111, 115, 125, 128, 130, 134, 138, 144

Test Method 2

Test compounds are made to a formulation according to a similar method to that described in the Formulation example 5, and thereto is added water to prepare a diluted solution containing a prescribed concentration of the test compound.

Cucumber (*cucumber sativus*) seedling (on the developmental stage of the second true leaf) is planted in a cup, and the diluted solutions are drenched to the foot of the seedling at a ratio of 5 mL/seedling. After 7 days, approximately 30

cotton aphids (*Aphis gossypii*) (all stages of life) are released onto the leaf of the seedling. After 6 days, the number of the surviving insects is examined and the controlling value is calculated by the following equation.

$$\text{Controlling value (\%)} = \{1 - (Cb \times Tai)/(Cai \times Tb)\} \times 100$$

wherein the symbols in the equation represent the following descriptions.

Cb: Number of the test insects in untreated group;
Cai: Number of the surviving insects at the time of the examination in untreated group;
Tb: Number of the test insects in treated group;
Tai: Number of the surviving insects at the time of the examination in treated group;

Here the "untreated group" represents a group where a similar treatment procedure to that of treated group except not using the test compound is done.

Test Example 2-1

The test is conducted according to the Test method 2 by making the prescribed concentration 200 ppm and using the present compound as a test compound to confirm a pesticidal effect against cotton aphids.

Test Method 3

Test compounds are made to a formulation according to a similar method to that described in the Formulation example 1, and thereto is added water containing 0.03 v/v% of Shindain (registered trademark) to prepare a diluted solution containing a prescribed concentration of the test compound.

Cabbage (*Brassicae oleracea*) seedling (on the developmental stage of the second to third true leaf) is planted in a cup, and the diluted solutions are sprayed into the seedling at a ratio of 20 mL/seedling. Thereafter, the stem and leaf thereof are cut out and then is installed into a cup that is covered with filter paper on the bed of the cup. Five (5) cotton worm (*Spodoptera litura*) at the second instar larval stage are released into the cup. After 5 days, the number of the surviving insects is counted, and the mortality of insects is calculated by the following equation.

$$\text{Mortality (\%)} = (1 - \text{Number of surviving insects}/5) \times 100$$

Test Example 3-1

The test was conducted according to the Test method 3 by making the prescribed concentration 500 ppm and using the below-mentioned present compounds as a test compound, and, as the result of the test, the below-mentioned present compounds showed 80% or more as the mortality of insects. Present compound Nos: 2, 3, 22, 23, 24, 27, 29, 31, 32, 33, 34, 35, 36, 42, 43, 44, 49, 74, 75, 76, 77, 78, 79, 80, 87, 96, 102, 109, 110, 111, 112, 114, 115, 120, 125, 126, 128, 129, 130, 131, 133, 134, 135, 138, 139, 143, 144

Test Method 4

Test compounds are made to a formulation according to a similar method to that described in the Formulation example 1, and thereto is added water containing 0.03 v/v% of Shindain (registered trademark) to prepare a diluted solution containing a prescribed concentration of the test compound.

Cabbage (*Brassicae oleracea*) seedling (on the developmental stage of the second to third true leaf) is planted in a cup, and the diluted solutions are sprayed into the seedling at a ratio of 20 mL/seedling. Thereafter, the stem and leaf thereof are cut out and then is installed into a cup that is covered with filter paper on the bed of the cup. Five (5) diamondback moth (*Plutella xylostella*) at the second instar larval stage are released into the cup. After 5 days, the number of the surviving insects is counted, and the mortality of insects is calculated by the following equation.

$$\text{Mortality (\%)} = (1 - \text{Number of surviving insects}/5) \times 100$$

Test Example 4-1

The test was conducted according to the Test method 4 by making the prescribed concentration 500 ppm and

using the below-mentioned present compounds as a test compound, and, as the result of the test, the below-mentioned present compounds showed 80% or more as the mortality of insects.
Present compound Nos: 2, 3, 18, 22, 23, 24, 26, 29, 31, 32, 33, 34, 35, 36, 39, 40, 41, 43, 44, 49, 54, 55, 56, 60, 69, 75, 76, 77, 78, 79, 80, 81, 87, 96, 98, 99, 101, 102, 109, 110, 111, 112, 114, 115, 120, 125, 126, 128, 129, 130, 131, 133, 134, 135, 136, 137, 138, 139, 140, 143, 144, 145

Test Method 5

Each 1 mg of the test compounds is dissolved into 50 μL of a mixed solution of polyoxyethylene sorbitan monolaurate and acetone (polyoxyethylene sorbitan monolaurate: acetone = 5 : 95 (v/v ratio)). Thereto is added water containing 0.03% by volume of Shindain (registered trademark) to prepare a diluted solution containing a prescribed concentration of the test compound.
A young entire seedling of Corns (*Zea mays*) is immersed into the diluted solution for 30 seconds. Thereafter, each two grains of the seedlings are installed in a plastic petri dish (90 mm radius), and 10 Western corn rootworms (*Diabrotica virgifera virgifera*) at the second instar larval stage are released into the dish. After 5 days, the number of the dead insects is counted, and the mortality of insects is calculated by the following equation.

Mortality (%) = (Number of dead insects/10} × 100

Test Example 5-1

The test was conducted according to the Test method 5 by making the prescribed concentration 200 ppm and using the below-mentioned present compounds as a test compound, and, as the result of the test, the below-mentioned present compounds showed 80% or more as the mortality of insects.
Present compound Nos: 26, 33, 34, 36, 77, 88, 101

Test Method 6

Acetone solution in which test compounds are adjusted to 800 ppm are prepared, and the solution is poured into a container having an internal volume of 50 mL, and the solution is coated uniformly on an inner face of the container such that the test compound is made to be 40 $mg/m^2$, and the container is then dried.
Five (5) German cockroach (*Blattella germanica*) male adults are placed in the container, and the cup is covered with the lid. After the prescribed timing is passed, the state of the German cockroach is examined, and the mortality of insects is calculated by the following equation.

Mortality (%) = (Number of dead insects/Number of tested insects) × 100

Test Example 6-1

The prescribed timing was set to three (3) days, and the test was conducted by using the below-mentioned present compounds as a test compound according to the Test method 6 and as the result of the test, the below-mentioned present compounds showed 80% or more as the mortality of insects.
Present compound Nos: 44

Test Method 7

Acetone solution in which test compounds are adjusted to 2000 ppm are prepared, and the solution is poured into a container having an internal volume of 20 mL, and the solution is coated uniformly on an inner face of the container such that the test compound is made to be 100 $mg/m^2$, and the container is then dried.
Five (5) *Haemaphysalis longicornis* nymphs are placed in the container, and the cup is covered with the lid. After the prescribed timing is passed, the state of the *Haemaphysalis longicornis* is examined, and the mortality of insects is calculated by the following equation.

Mortality (%) = (Number of dead insects/Number of tested insects) × 100

Test Example 7-1

The prescribed timing was set to two (2) days, and the test was conducted by using the below-mentioned present compounds as a test compound according to the Test method 7 to confirm a pesticidal effect against *Haemaphysalis longicornis.*

Test Method 8

Acetone solution in which test compounds are adjusted to 800 ppm are prepared, and the solution is poured into a container having an internal volume of 20 mL, and the solution is coated uniformly on an inner face of the container such that the test compound is made to be 40 mg/$m^2$, and the container is then dried.

Five (5) housefly (*Musca domestica*) female adults are placed in the container, and the cup is covered with the lid. After the prescribed timing is passed, the state of the housefly is examined, and the mortality of insects is calculated by the following equation.

Mortality (%) = (Number of dead insects/Number of tested insects) $\times$ 100

Test Example 8-1

The prescribed timing was set to one (1) day, and the test was conducted by using the below-mentioned present compounds as a test compound according to the Test method 8 and as the result of the test, the below-mentioned present compounds showed 80% or more as the mortality of insects.
Present compound Nos: 33, 34, 36, 43, 44, 77, 138, 139

Test Method 9

Test compounds are made to a formulation according to a similar method to that described in the Formulation example 5, and thereto is added water containing 0.03 v/v% of Shindain (registered trademark) to prepare a diluted solution containing a prescribed concentration of the test compound.

Cabbage (*Brassicae oleracea*) seedling (on the developmental stage of the third to fourth true leaf) is planted in a cup, and the diluted solutions are sprayed into the seedling at a ratio of 20 mL/seedling. Thereafter, ten (10) cotton worm (*Spodoptera litura*) at the third instar larval stage are released onto the seedling. After 6 days, the number of the surviving insects is counted, and the mortality of insects is calculated by the following equation.

Mortality (%) = (1 - Number of surviving insects/10) $\times$ 100

Test Example 9-1

The test was conducted according to the Test method 9 by making the prescribed concentration 200 ppm and using the below-mentioned present compounds as a test compound, and, as the result of the test, the below-mentioned present compounds showed 80% or more as the controlling value.
Present compound Nos: 22, 23, 24, 27, 28, 29, 31, 33, 34, 35, 36, 43, 44, 57, 62, 75, 76, 77, 79, 80, 90, 101, 102, 103, 109, 110, 111, 112, 113, 115, 116, 120, 122, 125, 126, 128, 129, 130, 131, 133, 134, 138, 139, 142

Test Example 9-2

The test was conducted according to the Test method 9 by making the prescribed concentration 50 ppm and using the below-mentioned present compounds as a test compound, and, as the result of the test, the below-mentioned present compounds showed 80% or more as the controlling value.
Present compound Nos: 22, 23, 24, 27, 28, 29, 31, 33, 34, 35, 36, 43, 44, 57, 76, 77, 101, 102, 103, 109, 110, 111, 112, 115, 120, 122, 125, 126, 128, 130, 133, 134, 138, 139

Test Method 10

Test compounds are made to a formulation according to a similar method to that described in the Formulation example 5, and thereto is added water containing 0.03 v/v% of Shindain (registered trademark) to prepare a diluted solution containing a prescribed concentration of the test compound.

Cabbage (*Brassicae oleracea*) seedling (on the developmental stage of the third to fourth true leaf) is planted in a

cup, and the diluted solutions are sprayed into the seedling at a ratio of 20 mL/seedling. Thereafter, ten (10) diamondback moth (*Plutella xylostella*) at the third instar larval stage are released onto the seedling. After 6 days, the number of the surviving insects is counted, and the mortality of insects is calculated by the following equation.

Mortality (%) = (1 - Number of surviving insects/10) × 100

Test Example 10-1

The test was conducted according to the Test method 10 by making the prescribed concentration 200 ppm and using the below-mentioned present compounds as a test compound, and, as the result of the test, the below-mentioned present compounds showed 80% or more as the controlling value.
Present compound Nos: 22, 24, 26, 29, 31, 32, 33, 34, 35, 36, 41, 44, 57, 62, 63, 75, 76, 78, 79, 80, 91, 101, 102, 103, 109, 110, 111, 112, 113, 115, 116, 120, 122, 125, 126, 128, 129, 130, 131, 132, 133, 134, 135, 136, 138, 139, 140, 144, 145

Test Example 10-2

The test was conducted according to the Test method 10 by making the prescribed concentration 50 ppm and using the below-mentioned present compounds as a test compound, and, as the result of the test, the below-mentioned present compounds showed 80% or more as the controlling value.
Present compound Nos: 22, 23, 24, 26, 29, 31, 32, 33, 34, 35, 36, 41, 44, 57, 62, 76, 101, 102, 103, 109, 110, 111, 115, 116, 120, 122, 125, 128, 130, 131, 134, 135, 138, 139, 144

Test Method 11

Test compounds are made to a formulation according to a similar method to that described in the Formulation example 5, and thereto is added water containing 0.03 v/v% of Shindain (registered trademark) to prepare a diluted solution containing a prescribed concentration of the test compound.
Cucumber (*cucumber sativus*) seedling (on the developmental stage of the second true leaf) is planted in a cup, and approximately 30 cotton aphids (*Aphis gossypii*) (all stages of life) are released onto the seedling. After one day, the diluted solutions are sprayed into the seedling at a ratio of 10 mL/seedling. After 5 days, the number of the surviving insects is examined and the controlling value is calculated by the following equation.

$$\text{Controlling value (\%)} = \{1 - (Cb \times Tai)/(Cai \times Tb)\} \times 100$$

wherein the symbols in the equation represent the following descriptions.

Cb: Number of the test insects in untreated group;
Cai: Number of the surviving insects at the time of the examination in untreated group;
Tb: Number of the test insects in treated group;
Tai: Number of the surviving insects at the time of the examination in treated group;

Here the "untreated group" represents a group where a similar treatment procedure to that of treated group except not using the test compound is done.

Test Example 11-1

The test was conducted according to the Test method 11 by making the prescribed concentration 200 ppm and using the below-mentioned present compounds as a test compound, and, as the result of the test, the below-mentioned present compounds showed 90% or more as the controlling value.
Present compound Nos: 22, 23, 24, 26, 27, 29, 31, 33, 34, 40, 41, 43, 44, 69, 70, 75, 76, 78, 79, 96, 102, 103, 109, 111, 115, 125, 126, 128, 130, 133, 134, 135, 138, 139, 144

Test Example 11-2

The test was conducted according to the Test method 11 by making the prescribed concentration 50 ppm and using the below-mentioned present compounds as a test compound, and, as the result of the test, the below-mentioned present compounds showed 90% or more as the controlling value. Present compound Nos: 22, 23, 24, 26, 27, 31, 33, 34, 36, 41, 69, 70, 78, 79, 96, 102, 103, 109, 111, 115, 125, 130, 134, 135, 138, 139

Test Method 12

Test compounds are made to a formulation according to a similar method to that described in the Formulation example 1, and thereto is added water to prepare a diluted solution containing a prescribed concentration of the test compound.

Into the diluted solution, 30 common house mosquitos (*Culex pipiens pallens*) at the last instar larval stage are released, and after 1 day, the state of the house mosquito larvae is examined, and the mortality of insects is calculated by the following equation.

Mortality (%) = (Number of dead insects/Number of tested insects) × 100

Test Example 12-1

The test was conducted according to the Test method 12 by making the prescribed concentration 3.5 ppm and using the below-mentioned present compounds as a test compound, and, as the result of the test, the below-mentioned present compounds showed 90% or more as the controlling value.
Present compound Nos: 22, 23, 26, 29, 31, 33, 34, 35, 36, 40, 41, 42, 44, 56, 70, 76, 84, 109, 110, 111, 115, 125, 129, 130, 131, 134, 135, 136, 138, 139, 144

Test Method 13

Each 1 mg of the present compound X is dissolved into 10 μL of a mixed solution of xylene, DMF, and a surfactant (xylene : DMF: surfactant = 4 : 4: 1 (v/v ratio)). Thereto is added water containing 0.02% by volume of a spreader to prepare diluted solution A containing a prescribed concentration of the present compound.

Each 1 mg of the present ingredients is dissolved into 10 μL of a mixed solution of xylene, DMF, and a surfactant (xylene : DMF: surfactant = 4 : 4: 1 (v/v ratio)). Thereto is added water containing 0.02% by volume of a spreader to prepare diluted solution B containing a prescribed concentration of the present ingredient.

The diluted solution A is mixed with the diluted solution B to prepare diluted solution C.

Leaf discs of Cucumber (*cucumber sativus*) cotyledon (length 1.5 cm) are placed in each well of 24-well microplate. Two (2) apterous adults and 8 larvae of cotton aphids (*Aphis gossypii*) per one well are released and the diluted solution C is sprayed at 20 μL per one well. The group is defined as "treated group". A well that is sprayed with 20 μL of water containing 0.02% by volume of a spreader instead of the diluted solution C is defined as "untreated group".

After drying the diluted solution C, the upper microplate is covered with a film sheet. After 5 days, the number of the surviving insects in each well is examined.

The controlling value is calculated by the following equation.

$$\text{Controlling value (\%)} = \{1 - (Tai)/(Cai)\} \times 100$$

wherein the symbols in the equation represent the following descriptions.

Cai: Number of the surviving insects at the time of the examination in untreated group;
Tai: Number of the surviving insects at the time of the examination in treated group.

Specific diluted solutions C, which can confirm their effect according to the Test method 13, are described in the following 1) to 5).

1) The diluted solution C comprises the combination recited in List A wherein a concentration of the present compound X is 200 ppm and a concentration of the present ingredient is 2000 ppm. In List A, Comp X represents any one compound selected from the compound classes SX1 to SX1371. List A:
Comp X + Clothianidin; Comp X + thiamethoxam; Comp X + imidacloprid; Comp X + thiacloprid; Comp X + flupyradifurone; Comp X + sulfoxaflor; Comp X + triflumezopyrim; Comp X + dicloromezotiaz; Comp X + beta-cyfluthrin; Comp X + tefluthrin; Comp X + fipronil; Comp X + chlorantraniliprole; Comp X + cyantraniliprole; Comp X + tetraniliprole; Comp X + thiodicarb; Comp X + carbofuran; Comp X + fluxametamide; Comp X + afoxolaner; Comp X + fluralaner; Comp X + broflanilide; Comp X + abamectin; Comp X + fluopyram; Comp X + fluensulfone; Comp X + fluazaindolizine; Comp X + tioxazafen; Comp X + flupyrimin; Comp X + Mycorrhizal Fungi; Comp X + Bradyrhizobium japonicum TA-11; Comp X + Bacillus firmus; Comp X + Bacillus firmus 1-1582; Comp X + Bacillus amyloliquefaciens; Comp X + Bacillus amyloliquefaciens FZB42; Comp X + Bacillus amyloliquefaciens PTA4838; Comp X + Pasteuria

nishizawae; Comp X + Pasteuria nishizawae Pn1; Comp X + Pasteuria penetrans; Comp X + tebuconazole; Comp X + prothioconazole; Comp X + metconazole; Comp X + ipconazole; Comp X + triticonazole; Comp X + difenoconazole; Comp X + imazalil; Comp X + triadimenol; Comp X + tetraconazole; Comp X + flutriafol; Comp X + mandestrobin; Comp X + azoxystrobin; Comp X + pyraclostrobin; Comp X + trifloxystrobin; Comp X + fluoxastrobin; Comp X + picoxystrobin; Comp X + fenamidone; Comp X + metalaxyl; Comp X + metalaxyl-M; Comp X + fludioxonil; Comp X + sedaxane; Comp X + penflufen; Comp X + fluxapyroxad; Comp X + benzovindiflupyr; Comp X + boscalid; Comp X + carboxin; Comp X + penthiopyrad; Comp X + flutolanil; Comp X + captan; Comp X + thiram; Comp X + tolclofos-methyl; Comp X + thiabendazole; Comp X + ethaboxam; Comp X + mancozeb; Comp X + picarbutrazox; Comp X + oxathiapiprolin; Comp X + silthiofam; Comp X + inpyrfluxam.

2) The diluted solution C comprises the combination recited in List A wherein a concentration of the present compound is 200 ppm, and a concentration of the present ingredient is 200 ppm.

3) The diluted solution C comprises the combination recited in List A wherein a concentration of the present compound X is 500 ppm, and a concentration of the present ingredient is 50 ppm.

4) The diluted solution C comprises the combination recited in List A wherein a concentration of the present compound X is 500 ppm, and a concentration of the present ingredient is 5 ppm.

5) The diluted solution C comprises the combination recited in List A wherein a concentration of the present compound X is 500 ppm, and a concentration of the present ingredient is 0.5 ppm.

Industrial Applicability

The present compound shows an excellent control effect against a harmful arthropod.

## Claims

**1.** A compound represented by formula (I):

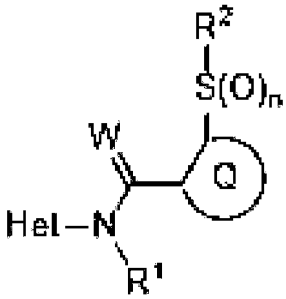

(I)

[wherein

Q represented by the following formula:

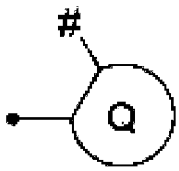

represents a group represented by formula Q1, a group represented by formula Q2, a group represented by formula Q3, a group represented by formula Q4, a group represented by formula Q5, a group represented by formula Q6, or a group represented by formula Q7 (# represents a binding site to a sulfur atom, and • represents a binding site to a carbon atom to which W is attached),

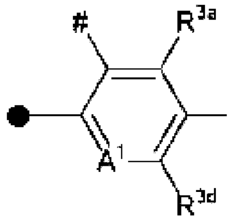
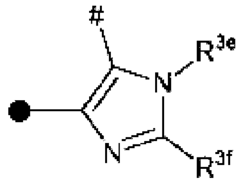
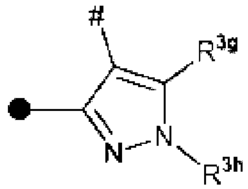
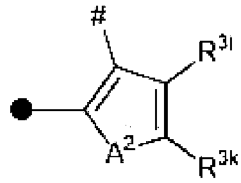

Q1 Q2 Q3 Q4

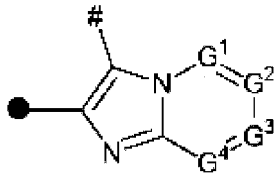
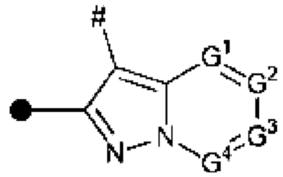
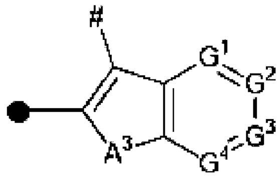

Q5 Q6 Q7

$A^1$ represents a nitrogen atom or $CR^{3c}$,
$A^2$ represents an oxygen atom, a sulfur atom or $NR^6$,
$A^3$ represents an oxygen atom or a sulfur atom,
$G^1$ represents a nitrogen atom or $CR^{10a}$,
$G^2$ represents a nitrogen atom or $CR^{10b}$,
$G^3$ represents a nitrogen atom or $CR^{10c}$,
$G^4$ represents a nitrogen atom or $CR^{10d}$,
$R^{3a}$, $R^{3b}$, $R^{3c}$, $R^{3d}$, $R^{3f}$, $R^{3g}$, $R^{3i}$, $R^{3k}$, $R^{10a}$, $R^{10b}$, $R^{10c}$ and $R^{10d}$ are identical to or different from each other and each represents a C1-C6 chain hydrocarbon group which may be optionally substituted with one or more substituents selected from Group B, a C3-C7 cycloalkyl group which may be optionally substituted with one or more substituents selected from Group K, a phenyl group which may be optionally substituted with one or more substituents selected from Group M, a five(5) or six(6) membered aromatic heterocyclic group which may be optionally substituted with one or more substituents selected from Group M, $OR^{12}$ , $NR^{11}R^{12}$ , $NR^{11a}R^{12a}$, $NR^{19}NR^{11}R^{12}$, $NR^{19}OR^{11}$, $NR^{11}C(O)R^{13}$, $NR^{19}NR^{11}C(O)R^{13}$, $NR^{11}C(O)OR^{14}$, $NR^{19}NR^{11}C(O)OR^{14}$, $NR^{11}C(O)NR^{21}R^{22}$, $NR^{19}NR^{11}C(O)NR^{21}R^{22}$, $N{=}CHNR^{21}R^{22}$, $N{=}S(O)_pR^{15}R^{16}$, $C(O)R^{13}$, $C(O)OR^{17}$, $C(O)NR^{21}R^{22}$, $C(O)NR^{11}S(O)_2R^{18}$, $CR^{20}{=}NOR^{17}$, $NR^{11}CR^{19}{=}NOR^{17}$, $S(O)_mR^{18}$, a cyano group, a nitro group, a hydrogen atom or a halogen atom (with the proviso that when Q represents a group represented by formula Q1 and $A^1$ represents a $CR^{3c}$, all of the existing $R^{3a}$, $R^{3b}$, $R^{3c}$ and $R^{3d}$ don't represent a hydrogen atom),
$R^{3e}$ and $R^{3h}$ are identical to or different from each other and each represents a C1-C6 alkyl group which may be optionally substituted with one or more halogen atoms, or a phenyl group which may be optionally substituted with one or more substituents selected from Group M,
when Q represents a group represented by formula Q1, neighboring two substituents among $R^{3a}$, $R^{3b}$ and $R^{3d}$ may combine with two carbon atoms to which they are attached to form a benzene ring, a pyrrole ring, a furan ring, a thiophene ring, a pyrazole ring, an imidazole ring, an oxazole ring, an isoxazole ring, a thiazole ring, an isothiazole ring, an oxadiazole ring, a thiadiazole ring, a pyridine ring, a pyridazine ring, a pyrimidine ring, a pyrazine ring {the benzene ring, the pyrrole ring, the furan ring, the thiophene ring, the pyrazole ring, the imidazole ring, the oxazole ring, the isoxazole ring, the thiazole ring, the isothiazole ring, the pyridine ring, the pyridazine ring, the pyrimidine ring, and the pyrazine ring may be optionally substituted with one or more substituents selected from Group M}, or a triazole ring which may be optionally substituted with one or more substituents selected from Group P,
p is 0 or 1,
m is 0, 1 or 2,
$R^{20}$ represents a C1-C6 chain hydrocarbon group which may be optionally substituted with one or more halogen atoms, a halogen atom, $OR^{23}$, $NR^{24}R^{25}$, or a hydrogen atom,
$R^{17}$ represents a C1-C6 chain hydrocarbon group which may be optionally substituted with one or more halogen atoms, a phenyl group which may be optionally substituted with one or more substituents selected from Group J, or a hydrogen atom,
$R^{12}$ represents a C1-C6 chain hydrocarbon group which may be optionally substituted with one or more

substituents selected from Group L, a C3-C7 cycloalkyl group which may be optionally substituted with one or more substituents selected from Group T, a C3-C7 cycloalkenyl group which may be optionally substituted with one or more substituents selected from Group T, a phenyl group which may be optionally substituted with one or more substituents selected from Group J, a six(6) membered aromatic heterocyclic group which may be optionally substituted with one or more substituents selected from Group J, a hydrogen atom, or $S(O)_2R^{18}$,

$R^{18}$ represents a C1-C6 chain hydrocarbon group which may be optionally substituted with one or more halogen atoms, or a phenyl group which may be optionally substituted with one or more substituents selected from Group J,

$R^{11a}$ and $R^{12a}$ combine with a nitrogen atom to which they are attached to form a three(3) to seven(7) membered nonaromatic heterocyclic group which may be optionally substituted with one or more substituents selected from Group K,

$R^{13}$ represents a C1-C6 chain hydrocarbon group which may be optionally substituted with one or more halogen atoms, a C3-C7 cycloalkyl group which may be optionally substituted with one or more halogen atoms, a (C3-C6 cycloalkyl) C1-C3 alkyl group which may be optionally substituted with one or more halogen atoms, a phenyl group which may be optionally substituted with one or more substituents selected from Group J, a five(5) or six(6) membered aromatic heterocyclic group which may be optionally substituted with one or more substituents selected from Group J, or a hydrogen atom,

$R^{14}$ represents a C1-C6 chain hydrocarbon group which may be optionally substituted with one or more halogen atoms, a C3-C7 cycloalkyl group which may be optionally substituted with one or more halogen atoms, a (C3-C6 cycloalkyl) C1-C3 alkyl group which may be optionally substituted with one or more halogen atoms, or a phenyl C1-C3 alkyl group {the phenyl moiety in the phenyl C1-C3 alkyl group may be optionally substituted with one or more substituents selected from Group J},

$R^{15}$ and $R^{16}$ are identical to or different from each other and each represents a C1-C6 alkyl group which may be optionally substituted with one or more halogen atoms,

$R^{21}$ represents a C1-C6 alkyl group which may be optionally substituted with one or more halogen atoms, or a hydrogen atom,

$R^{22}$ represents a C1-C6 chain hydrocarbon group which may be optionally substituted with one or more substituents selected from Group L, a C3-C7 cycloalkyl group which may be optionally substituted with one or more substituents selected from Group T, or a hydrogen atom,

$R^{23}$ represents a C1-C6 chain hydrocarbon group which may be optionally substituted with one or more halogen atoms,

$R^{11}$, $R^{19}$, $R^{24}$, and $R^{25}$ are identical to or different from each other and each represents a C1-C6 chain hydrocarbon group which may be optionally substituted with one or more halogen atoms, or a hydrogen atom,

W represents an oxygen atom or a sulfur atom,

$R^1$ represents a C1-C6 chain hydrocarbon group which may be optionally substituted with one or more substituents selected from Group D, a C3-C7 cycloalkyl group which may be optionally substituted with one or more substituents selected from Group E, $C(O)R^4$, $C(O)OR^4$, or $C(O)NR^4R^3$,

$R^4$ and $R^5$ are identical to or different from each other and each represents a C1-C6 chain hydrocarbon group which may be optionally substituted with one or more halogen atoms, a phenyl group which may be optionally substituted with one or more substituents selected from Group F, a five(5) or six(6) membered heterocyclic group which may be optionally substituted with one or more substituents selected from Group F, or a hydrogen atom,

$R^2$ represents a C1-C6 alkyl group which may be optionally substituted with one or more halogen atoms, a cyclopropyl group, or a cyclopropylmethyl group,

n is 0, 1 or 2,

Het represents a group represented by formula Het1, a group represented by formula Het2, a group represented by formula Het3, a group represented by formula Het4, a group represented by formula Het5, or a group represented by formula Het6,

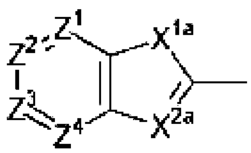

Het1

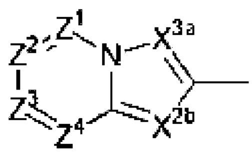

Het2

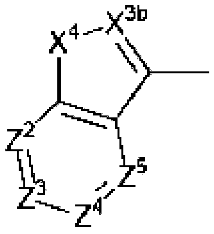
Het3

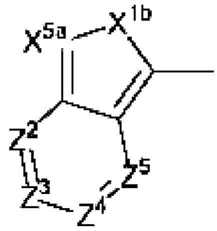
Het4

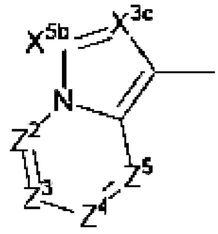
Het5

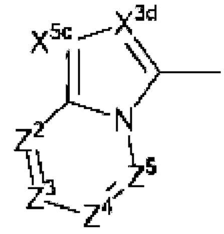
Het6

$X^{1a}$ and $X^{1b}$ are identical to or different from each other and each represents $NR^{7a}$, an oxygen atom or a sulfur atom,
$X^4$ represents $NR^{7b}$, an oxygen atom or a sulfur atom,
$X^{2a}$ and $X^{2b}$ are identical to or different from each other and each represents a nitrogen atom or $CR^{8a}$,
$X^{3a}$, $X^{3b}$, $X^{3c}$ and $X^{3d}$ are identical to or different from each other and each represents a nitrogen atom or $CR^{8b}$,
$X^{5a}$, $X^{5b}$ and $X^{5c}$ are identical to or different from each other and each represents a nitrogen atom or $CR^{8c}$,
$R^{7a}$ and $R^{7b}$ are identical to or different from each other and each represents a C1-C6 chain hydrocarbon group which may be optionally substituted with one or more halogen atoms, a C1-C6 alkoxy group which may be optionally substituted with one or more halogen atoms, a C3-C7 cycloalkyl group which may be optionally substituted with one or more halogen atoms, a (C3-C7 cycloalkyl) C1-C6 alkyl group which may be optionally substituted with one or more halogen atoms, or a hydrogen atom,
$R^{8a}$, $R^{8b}$ and $R^{8c}$ are identical to or different from each other and each represents a C1-C6 chain hydrocarbon group which may be optionally substituted with one or more substituents selected from Group $B^2$, a C3-C7 cycloalkyl group which may be optionally substituted with one or more substituents selected from Group $K^2$, a phenyl group which may be optionally substituted with one or more substituents selected from Group $M^2$, a five(5) or six(6) membered aromatic heterocyclic group which may be optionally substituted with one or more substituents selected from Group $M^2$, $OR^{32}$, $NR^{31}R^{32}$, $NR^{31a}R^{32a}$, $NR^{39}NR^{31}R^{32}$, $NR^{39}OR^{31}$, $NR^{31}C(O)R^{33}$, $NR^{39}NR^{31}C(O)R^{33}$, $NR^{31}C(O)OR^{34}$, $NR^{39}NR^{31}C(O)OR^{34}$, $NR^{31}C(O)NR^{41}R^{42}$, $NR^{39}NR^{31}C(O)NR^{41}R^{42}$, $N=CHNR^{41}R^{42}$, $N=S(O)_kR^{35}R^{36}$, $C(O)R^{33}$, $C(O)OR^{37}$, $C(O)NR^{41}R^{42}$, $C(O)NR^{31}S(O)_2R^{38}$, $CR^{40}=NOR^{37}$, $NR^{31}CR^{39}=NOR^{37}$, $S(O)_tR^{38}$, a cyano group, a nitro group, a hydrogen atom, or a halogen atom,
k is 0 or 1,
t is 0, 1 or 2,
$R^{40}$ represents a C1-C6 chain hydrocarbon group which may be optionally substituted with one or more halogen atoms, a halogen atom, $OR^{43}$, $NR^{44}R^{45}$, or a hydrogen atom,
$R^{37}$ represents a C1-C6 chain hydrocarbon group which may be optionally substituted with one or more halogen atoms, a phenyl group which may be optionally substituted with one or more substituents selected from Group $J^2$, or a hydrogen atom,
$R^{32}$ represents a C1-C6 chain hydrocarbon group which may be optionally substituted with one or more substituents selected from Group $L^2$, a C3-C7 cycloalkyl group which may be optionally substituted with one or more substituents selected from Group $T^2$, a C3-C7 cycloalkenyl group which may be optionally substituted with one or more substituents selected from Group $T^2$, a phenyl group which may be optionally substituted with one or more substituents selected from Group $J^2$, a six(6) membered aromatic heterocyclic group which may be optionally substituted with one or more substituents selected from Group $J^2$, a hydrogen atom, or $S(O)_2R^{38}$,
$R^{38}$ represents a C1-C6 chain hydrocarbon group which may be optionally substituted with one or more halogen atoms, or a phenyl group which may be optionally substituted with one or more substituents selected from Group $J^2$,
$R^{31a}$ and $R^{32a}$ combine with a nitrogen atom to which they are attached to form a three(3) to seven(7) membered nonaromatic heterocyclic group which may be optionally substituted with one or more substituents selected from Group $K^2$,
$R^{33}$ represents a C1-C6 chain hydrocarbon group which may be optionally substituted with one or more halogen atoms, a C3-C7 cycloalkyl group which may be optionally substituted with one or more halogen atoms, a (C3-C6 cycloalkyl) C1-C3 alkyl group which may be optionally substituted with one or more halogen atoms, a phenyl group which may be optionally substituted with one or more substituents selected from Group $J^2$, a five(5) or six(6) membered aromatic heterocyclic group which may be optionally substituted with one or more substituents selected from Group $J^2$, or a hydrogen atom,
$R^{34}$ represents a C1-C6 chain hydrocarbon group which may be optionally substituted with one or more halogen atoms, a C3-C7 cycloalkyl group which may be optionally substituted with one or more halogen atoms, a (C3-C6

cycloalkyl) C1-C3 alkyl group which may be optionally substituted with one or more halogen atoms, or a phenyl C1-C3 alkyl group {the phenyl moiety in the phenyl C1-C3 alkyl group may be optionally substituted with one or more substituents selected from Group $J^2$},

$R^{35}$ and $R^{36}$ are identical to or different from each other and each represents a C1-C6 alkyl group which may be optionally substituted with one or more halogen atoms,

$R^{41}$ represents a C1-C6 alkyl group which may be optionally substituted with one or more halogen atoms, or a hydrogen atom,

$R^{42}$ represents a C1-C6 chain hydrocarbon group which may be optionally substituted with one or more substituents selected from Group $L^2$, a C3-C7 cycloalkyl group which may be optionally substituted with one or more substituents selected from Group $T^2$, or a hydrogen atom,

$R^{43}$ represents a C1-C6 chain hydrocarbon group which may be optionally substituted with one or more halogen atoms,

$R^{31}$, $R^{39}$, $R^{44}$ and $R^{45}$ are identical to or different from each other and each represents a C1-C6 chain hydrocarbon group which may be optionally substituted with one or more halogen atoms, or a hydrogen atom,

$Z^1$ represents a nitrogen atom or $CR^{9a}$,

$Z^2$ represents a nitrogen atom or $CR^{9b}$,

$Z^3$ represents a nitrogen atom or $CR^{9c}$,

$Z^4$ represents a nitrogen atom or $CR^{9d}$,

$Z^5$ represents a nitrogen atom or $CR^{9e}$,

$R^{9a}$, $R^{9d}$ and $R^{9e}$ are identical to or different from each other and each represents a C1-C6 chain hydrocarbon group which may be optionally substituted with one or more substituents selected from Group $B^3$, a C3-C7 cycloalkyl group which may be optionally substituted with one or more substituents selected from Group $K^3$, a phenyl group which may be optionally substituted with one or more substituents selected from Group $M^3$, a five(5) or six(6) membered aromatic heterocyclic group which may be optionally substituted with one or more substituents selected from Group $M^3$, $OR^{52}$, $NR^{51}R^{52}$, $NR^{51a}R^{52a}$, $NR^{59}NR^{51}R^{52}$, $NR^{59}OR^{51}$, $NR^{51}C(O)R^{53}$, $NR^{59}NR^{51}C(O)R^{53}$, $NR^{51}C(O)OR^{54}$, $NR^{59}NR^{51}C(O)OR^{54}$, $NR^{51}C(O)NR^{51}R^{52}$, $NR^{59}NR^{51}C(O)NR^{61}R^{62}$, $N=CHNR^{61}R^{62}$, $N=S\ (O)_qR^{55}R^{56}$, $C(O)R^{53}$, $C(O)OR^{57}$, $C(O)NR^{61}R^{62}$, $C(O)NR^{51}S(O)_2R^{58}$, $CR^{60}=NOR^{57}$, $NR^{51}CR^{59}=NOR^{57}$, $S(O)_vR^{58}$, a cyano group, a nitro group, a hydrogen atom, or a halogen atom,

q is 0 or 1,

v is 0, 1 or 2,

$R^{9b}$ and $R^{9c}$ are identical to or different from each other and each represents a C1-C6 chain hydrocarbon group which may be optionally substituted with one or more substituents selected from Group $B^3$, a C3-C7 cycloalkyl group which may be optionally substituted with one or more substituents selected from Group $K^3$, a phenyl group which may be optionally substituted with one or more substituents selected from Group $M^3$, a five(5) or six(6) membered aromatic heterocyclic group which may be optionally substituted with one or more substituents selected from Group $M^3$, $OR^{72}$, $NR^{71}R^{72}$, $NR^{71a}R^{72a}$, $NR^{79}NR^{71}R^{72}$, $NR^{79}OR^{71}$, $NR^{71}C(O)R^{73}$, $NR^{79}NR^{71}C(O)R^{73}$, $NR^{71}C(O)OR^{74}$, $NR^{79}NR^{71}C(O)OR^{74}$, $NR^{71}C(O)NR^{71}R^{72}$, $NR^{79}NR^{71}C(O)NR^{81}R^{82}$, $N=CHNR^{81}R^{82}$, $N=S(O)_uR^{75}R^{76}$, $C(O)R^{73}$, $C(O)OR^{77}$, $C(O)NR^{81}R^{82}$, $C(O)NR^{71}S(O)_2R^{78}$, $CR^{80}=NOR^{77}$, $NR^{71}CR^{79}=NOR^{77}$, $S(O)_wR^{78}$, a cyano group, a nitro group, a hydrogen atom, or a halogen atom,

u is 0 or 1,

w is 0, 1 or 2,

$R^{60}$ and $R^{80}$ are identical to or different from each other and each represents a C1-C6 chain hydrocarbon group which may be optionally substituted with one or more halogen atoms, a halogen atom, $OR^{63}$, $NR^{64}R^{65}$, or a hydrogen atom,

$R^{57}$ and $R^{77}$ are identical to or different from each other and each represents a C1-C6 chain hydrocarbon group which may be optionally substituted with one or more halogen atoms, a phenyl group which may be optionally substituted with one or more substituents selected from Group $J^3$, or a hydrogen atom,

$R^{52}$ represents a C1-C6 chain hydrocarbon group which may be optionally substituted with one or more substituents selected from Group $L^3$, a C3-C7 cycloalkyl group which may be optionally substituted with one or more substituents selected from Group $T^3$, a C3-C7 cycloalkenyl group which may be optionally substituted with one or more substituents selected from Group $T^3$, a phenyl group which may be optionally substituted with one or more substituents selected from Group $J^3$, a six(6) membered aromatic heterocyclic group which may be optionally substituted with one or more substituents selected from Group $J^3$, or $S(O)_2R^{58}$,

$R^{72}$ represents a C1-C6 chain hydrocarbon group which may be optionally substituted with one or more substituents selected from Group $L^3$, a C3-C7 cycloalkyl group which may be optionally substituted with one or more substituents selected from Group $T^3$, a C3-C7 cycloalkenyl group which may be optionally substituted with one or more substituents selected from Group $T^3$, a hydrogen atom, or $S(O)_2R^{78}$,

$R^{58}$ represents a C1-C6 chain hydrocarbon group which may be optionally substituted with one or more halogen

atoms, or a phenyl group which may be optionally substituted with one or more substituents selected from Group $J^3$,

$R^{78}$ represents a C1-C6 chain hydrocarbon group which may be optionally substituted with one or more halogen atoms,

$R^{51a}$ and $R^{52a}$ combine with a nitrogen atom to which they are attached to form a three(3) to seven(7) membered non-aromatic heterocyclic group which may be optionally substituted with one or more substituents selected from Group $K^3$,

$R^{71a}$ and $R^{72a}$ combine with a nitrogen atom to which they are attached to form a three(3) to seven(7) membered non-aromatic heterocyclic group which may be optionally substituted with one or more substituents selected from Group $K^3$,

$R^{53}$ and $R^{73}$ are identical to or different from each other and each represents a C1-C6 chain hydrocarbon group which may be optionally substituted with one or more halogen atoms, a C3-C7 cycloalkyl group which may be optionally substituted with one or more halogen atoms, a (C3-C6 cycloalkyl) C1-C3 alkyl group which may be optionally substituted with one or more halogen atoms, a phenyl group which may be optionally substituted with one or more substituents selected from Group $J^3$, a five(5) or six(6) membered aromatic heterocyclic group which may be optionally substituted with one or more substituents selected from Group $J^3$, or a hydrogen atom,

$R^{54}$ and $R^{74}$ are identical to or different from each other and each represents a C1-C6 chain hydrocarbon group which may be optionally substituted with one or more halogen atoms, a C3-C7 cycloalkyl group which may be optionally substituted with one or more halogen atoms, a (C3-C6 cycloalkyl) C1-C3 alkyl group which may be optionally substituted with one or more halogen atoms, or a phenyl C1-C3 alkyl group {the phenyl moiety in the phenyl C1-C3 alkyl group may be optionally substituted with one or more substituents selected from Group $J^3$},

$R^{55}$, $R^{56}$, $R^{75}$ and $R^{76}$ are identical to or different from each other and each represents a C1-C6 alkyl group which may be optionally substituted with one or more halogen atoms,

$R^{61}$ and $R^{81}$ are identical to or different from each other and each represents a C1-C6 alkyl group which may be optionally substituted with one or more halogen atoms, or a hydrogen atom,

$R^{62}$ and $R^{82}$ are identical to or different from each other and each represents a C1-C6 chain hydrocarbon group which may be optionally substituted with one or more substituents selected from Group $L^3$, a C3-C7 cycloalkyl group which may be optionally substituted with one or more substituents selected from Group $T^3$, or a hydrogen atom,

$R^{63}$ represents a C1-C6 chain hydrocarbon group which may be optionally substituted with one or more halogen atoms,

$R^{51}$, $R^{59}$, $R^{64}$, $R^{65}$, $R^{71}$, and $R^{79}$ are identical to or different from each other and each represents a C1-C6 chain hydrocarbon group which may be optionally substituted with one or more halogen atoms, or a hydrogen atom,

Group D: a group consisting of a C3-C6 cycloalkyl group which may be optionally substituted with one or more substituents selected from halogen atom and C1-C3 alkyl group, a C1-C6 alkoxy group which may be optionally substituted with one or more halogen atoms, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, a C2-C6 alkylcarbonyl group which may be optionally substituted with one or more halogen atoms, a C2-C6 alkoxycarbonyl group which may be optionally substituted with one or more halogen atoms, a phenyl group which may be optionally substituted with one or more substituents selected from Group F, a five(5) or six(6) membered aromatic heterocyclic group which may be optionally substituted with one or more substituents selected from Group F, a hydroxy group, a cyano group and a halogen atom,

Group E: a group consisting of a C1-C6 alkyl group which may be optionally substituted with one or more halogen atoms, a C1-C6 alkoxy group which may be optionally substituted with one or more halogen atoms, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, a cyano group, and a halogen atom,

Group F: a group consisting of a C1-C6 alkyl group which may be optionally substituted with one or more halogen atoms, a C1-C6 alkoxy group which may be optionally substituted with one or more halogen atoms, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, a C1-C6 alkylamino group which may be optionally substituted with one or more halogen atoms, di(C1-C6 alkyl which may be optionally substituted with one or more halogen atoms) amino group, a C2-C6 alkylcarbonyl group which may be optionally substituted with one or more halogen atoms, a C2-C6 alkoxycarbonyl group which may be optionally substituted with one or more halogen atoms, a hydroxy group, a sulfanyl group, an amino group, a cyano group, a nitro group, and a halogen atom,

Group B: a group consisting of a C1-C6 alkoxy group which may be optionally substituted with one or more

halogen atoms, a C3-C6 alkenyloxy group which may be optionally substituted with one or more halogen atoms, a C3-C6 alkynyloxy group which may be optionally substituted with one or more halogen atoms, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, a C3-C6 cycloalkyl group which may be optionally substituted with one or more halogen atoms, a cyano group, hydroxy group, and a halogen atom,

Group G: a group consisting of a C1-C6 chain hydrocarbon group which may be optionally substituted with one or more halogen atoms, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, and a halogen atom,

Group J: a group consisting of a C1-C6 chain hydrocarbon group which may be optionally substituted with one or more halogen atoms, a hydroxy group, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a sulfanyl group, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, an amino group, $NHR^{91}$, $NR^{91}R^{92}$, $C(O)R^{91}$, $OC(O)R^{91}$, $C(O)OR^{91}$, a cyano group, a nitro group, and a halogen atom,

$R^{91}$ and $R^{92}$ are identical to or different from each other and each represents a C1-C6 alkyl group which may be optionally substituted with one or more halogen atoms,

Group K: a group consisting of a C1-C6 chain hydrocarbon group which may be optionally substituted with one or more halogen atoms, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a halogen atom, an oxo group, a hydroxy group, a cyano group, and a nitro group,

Group L: a group consisting of a C1-C6 alkoxy group which may be optionally substituted with one or more halogen atoms, a phenyl group which may be optionally substituted with one or more substituents selected from Group J, a five(5) or six(6) membered aromatic heterocyclic group which may be optionally substituted with one or more substituents selected from Group J, a C3-C7 cycloalkyl group which may be optionally substituted with one or more halogen atoms, a three(3) to seven(7) membered non-aromatic heterocyclic group which may be optionally substituted with one or more substituents selected from Group G, an amino group, $NHR^{91}$, $NR^{91}R^{92}$, a halogen atom, and a cyano group,

Group M: a group consisting of a C1-C6 alkyl group which may be optionally substituted with one or more halogen atoms, $OR^{94}$, $NR^{93}R^{94}$, $C(O)R^{94}$, $C(O)NR^{93}R^{94}$, $OC(O)R^{93}$, $OC(O)OR^{93}$, $NR^{94}C(O)R^{93}$, $NR^{94}C(O)OR^{93}$, $C(O)OR^{94}$, a halogen atom, a nitro group, a cyano group, an amino group, and five(5) or six(6) membered aromatic heterocyclic group,

$R^{93}$ represents a C1-C6 alkyl group which may be optionally substituted with one or more halogen atoms, or a C3-C6 cycloalkyl group which may be optionally substituted with one or more halogen atoms,

$R^{94}$ represents a C1-C6 alkyl group which may be optionally substituted with one or more halogen atoms, a C3-C6 cycloalkyl group which may be optionally substituted with one or more halogen atoms, or a hydrogen atom,

Group P: a group consisting of a C2-C6 chain hydrocarbon group which may be optionally substituted with one or more halogen atoms, a C3-C6 cycloalkyl group which may be optionally substituted with one or more halogen atoms, a phenyl group which may be optionally substituted with one or more substituents selected from Group J, a five(5) or six(6) membered aromatic heterocyclic group which may be optionally substituted with one or more substituents selected from Group J, a C2-C6 alkylcarbonyl group which may be optionally substituted with one or more halogen atoms, a C2-C6 alkoxycarbonyl group which may be optionally substituted with one or more halogen atoms, an aminocarbonyl group, a (C1-C6 alkyl) aminocarbonyl group which may be optionally substituted with one or more halogen atoms, a methyl group, and a di(C1-C4 alkyl) aminocarbonyl group which may be optionally substituted with one or more halogen atoms,

Group T: a group consisting of a C1-C6 alkyl group which may be optionally substituted with one or more halogen atoms, a halogen atom, and a cyano group,

Group $B^2$: a group consisting of a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, a C3-C6 cycloalkyl group which may be optionally substituted with one or more halogen atoms, a cyano group, a hydroxy group, and a halogen atom,

Group $G^2$: a group consisting of a C1-C6 chain hydrocarbon group which may be optionally substituted with one or more halogen atoms, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, and a halogen atom,
Group $J^2$: a group consisting of a C1-C6 chain hydrocarbon group which may be optionally substituted with one or more halogen atoms, a hydroxy group, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a sulfanyl group, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, an amino group, $NHR^{95}$, $NR^{95}R^{96}$, $C(O)R^{95}$, $OC(O)R^{95}$, $C(O)OR^{95}$, a cyano group, a nitro group, and a halogen atom,
$R^{95}$ and $R^{96}$ are identical to or different from each other and each represents a C1-C6 alkyl group which may be optionally substituted with one or more halogen atoms,
Group $K^2$: a group consisting of a C1-C6 chain hydrocarbon group which may be optionally substituted with one or more halogen atoms, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a halogen atom, an oxo group, a hydroxy group, a cyano group, and a nitro group,
Group $L^2$: a group consisting of a C1-C6 alkoxy group which may be optionally substituted with one or more halogen atoms, a phenyl group which may be optionally substituted with one or more substituents selected from Group $J^2$, a five(5) or six(6) membered aromatic heterocyclic group which may be optionally substituted with one or more substituents selected from Group $J^2$, a C3-C7 cycloalkyl group which may be optionally substituted with one or more halogen atoms, a three(3) to seven(7) membered non-aromatic heterocyclic group which may be optionally substituted with one or more substituents selected from Group $G^2$, an amino group, $NHR^{95}$, $NR^{95}R^{96}$, a halogen atom, and a cyano group,
Group $M^2$: a group consisting of a C1-C6 alkyl group which may be optionally substituted with one or more halogen atoms, $OR^{98}$, $NR^{97}R^{98}$, $C(O)R^{98}$, $C(O)NR^{97}R^{98}$, $OC(O)R^{97}$, $OC(O)OR^{97}$, $NR^{98}C(O)R^{97}$, $NR^{98}C(O)OR^{97}$, $C(O)OR^{98}$, a halogen atom, a nitro group, a cyano group, an amino group, and a five(5) or six (6) membered aromatic heterocyclic group,
$R^{97}$ represents a C1-C6 alkyl group which may be optionally substituted with one or more halogen atoms, or a C3-C6 cycloalkyl group which may be optionally substituted with one or more halogen atoms,
$R^{98}$ represents a C1-C6 alkyl group which may be optionally substituted with one or more halogen atoms, a C3-C6 cycloalkyl group which may be optionally substituted with one or more halogen atoms, or a hydrogen atom,
Group $T^2$: a group consisting of a C1-C6 alkyl group which may be optionally substituted with one or more halogen atoms, a halogen atom, and a cyano group,
Group $B^3$: a group consisting of a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, a C3-C6 cycloalkyl group which may be optionally substituted with one or more halogen atoms, a cyano group, a hydroxy group, and a halogen atom,
Group $G^3$: a group consisting of a C1-C6 chain hydrocarbon group which may be optionally substituted with one or more halogen atoms, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, and a halogen atom,
Group $J^3$: a group consisting of a C1-C6 chain hydrocarbon group which may be optionally substituted with one or more halogen atoms, a hydroxy group, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a sulfanyl group, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, an amino group, $NHR^{99}$, $NR^{99}R^{100}$, $C(O)R^{99}$, $OC(O)R^{99}$, $C(O)OR^{99}$, a cyano group, a nitro group, and a halogen atom,
$R^{99}$ and $R^{100}$ are identical to or different from each other and each represents a C1-C6 alkyl group which may be optionally substituted with one or more halogen atoms,
Group $K^3$: a group consisting of a C1-C6 chain hydrocarbon group which may be optionally substituted with one or

more halogen atoms, a C1-C6 alkoxy group which may be optionally substituted with one or more halogen atoms, a C3-C6 alkenyloxy group which may be optionally substituted with one or more halogen atoms, a C3-C6 alkynyloxy group which may be optionally substituted with one or more halogen atoms, a halogen atom, an oxo group, a hydroxy group, a cyano group, and a nitro group,

Group $L^3$: a group consisting of a C1-C6 alkoxy group which may be optionally substituted with one or more halogen atoms, a phenyl group which may be optionally substituted with one or more substituents selected from Group $J^3$, a five(5) or six(6) membered aromatic heterocyclic group which may be optionally substituted with one or more substituents selected from Group $J^3$, a C3-C7 cycloalkyl group which may be optionally substituted with one or more halogen atoms, a three(3) to seven(7) membered non-aromatic heterocyclic group which may be optionally substituted with one or more substituents selected from Group $G^3$, an amino group, $NHR^{99}$, $NR^{99}R^{100}$, a halogen atom, and a cyano group,

Group $M^3$: a group consisting of a C1-C6 alkyl group which may be optionally substituted with one or more halogen atoms, $OR^{102}$, $NR^{101}R^{102}$, $C(O)R^{102}$, $C(O)NR^{101}R^{102}$, $OC(O)R^{101}$, $OC(O)OR^{101}$, $NR^{102}C(O)R^{101}$, $NR^{102}C(O)OR^{101}$, $C(O)OR^{102}$, a halogen atom, a nitro group, a cyano group, an amino group, and a five(5) or six(6) membered aromatic heterocyclic group,

$R^{101}$ represents a C1-C6 alkyl group which may be optionally substituted with one or more halogen atoms, or a C3-C6 cycloalkyl group which may be optionally substituted with one or more halogen atoms,

$R^{102}$ represents a C1-C6 alkyl group which may be optionally substituted with one or more halogen atoms, a C3-C6 cycloalkyl group which may be optionally substituted with one or more halogen atoms, or a hydrogen atom,

Group $T^3$: a group consisting of a C1-C6 alkyl group which may be optionally substituted with one or more halogen atoms, a halogen atom, and a cyano group]

or N-oxide thereof.

2. The compound according to claim 1 or N-oxide thereof wherein Q represents a group represented by formula Q1 or a group represented by formula Q5.

3. The compound according to claim 1 or N-oxide thereof wherein Q represents a group represented by formula Q1 and $A^1$ represents a nitrogen atom.

4. The compound according to claim 1 or N-oxide thereof wherein Q represents a group represented by formula Q5, $G^1$ represents $CR^{10a}$, $G^2$ represents $CR^{10b}$, $G^3$ represents $CR^{10c}$, and $G^4$ represents a nitrogen atom or $CR^{10d}$.

5. The compound according to claim 1 or N-oxide thereof wherein Q represents a group represented by formula Q5, $G^1$ represents $CR^{10a}$, $G^2$ represents $CR^{10b}$, $G^3$ represents $CR^{10c}$, and $G^4$ represents $CR^{10d}$.

6. The compound according to any one of claims 1 to 5 or N-oxide thereof wherein Het represents a group represented by formula Het1, $Z^1$ represents $CR^{9a}$, $Z^2$ represents $CR^{9b}$, $Z^3$ represents $CR^{9c}$, and $Z^4$ represents $CR^{9d}$.

7. The compound according to any one of claims 1 to 5 or N-oxide thereof wherein Het represents a group represented by formula Het2, $Z^1$ represents $CR^{9a}$, $Z^2$ represents $CR^{9b}$, $Z^3$ represents $CR^{9c}$, and $Z^4$ represents $CR^{9d}$.

8. The compound according to any one of claims 1 to 7 or N-oxide thereof wherein $R^2$ represents an ethyl group.

9. The compound according to any one of claims 1 to 7 or N-oxide thereof wherein W represents an oxygen atom.

10. A composition for controlling harmful arthropod which comprises the compound according to any one of claims 1 to 9 or N-oxide thereof and an inert career.

11. A composition comprising one or more ingredients selected from the group consisting of the following Group (a), Group (b), Group (c), and Group (d), and the compound according to any one of claims 1 to 9 or N-oxide thereof

Group (a): a group consisting of insecticidal active ingredients, miticidal active ingredients, and nematicidal active ingredients;
Group (b): fungicidal active ingredients,
Group (c): plant growth regulatory ingredients; and
Group (d): repellent ingredients.

**12.** A method for controlling harmful arthropod which comprises applying an effective amount of the compound according to any one of claims 1 to 9 or N-oxide thereof, or an effective amount of the composition according to claim 11 to a harmful arthropod or a habitat where a harmful arthropod lives.

**13.** A seed or vegetative reproductive organ carrying an effective amount of the compound according to any one of claims 1 to 9 or N-oxide thereof, or an effective amount of the composition according to claim 11.

**INTERNATIONAL SEARCH REPORT**

International application No. **PCT/JP2024/019624**

**A. CLASSIFICATION OF SUBJECT MATTER**

***C07D 401/12***(2006.01)i; ***A01N 25/00***(2006.01)i; ***A01N 37/24***(2006.01)i; ***A01N 37/32***(2006.01)i; ***A01N 37/38***(2006.01)i; ***A01N 37/46***(2006.01)i; ***A01N 37/50***(2006.01)i; ***A01N 43/32***(2006.01)i; ***A01N 43/36***(2006.01)i; ***A01N 43/40***(2006.01)i; ***A01N 43/50***(2006.01)i; ***A01N 43/52***(2006.01)i; ***A01N 43/54***(2006.01)i; ***A01N 43/56***(2006.01)i; ***A01N 43/76***(2006.01)i; ***A01N 43/78***(2006.01)i; ***A01N 43/80***(2006.01)i; ***A01N 43/88***(2006.01)i; ***A01N 43/90***(2006.01)i; ***A01N 43/653***(2006.01)i; ***A01N 43/713***(2006.01)i; ***A01N 43/836***(2006.01)i; ***A01N 47/02***(2006.01)i; ***A01N 47/04***(2006.01)i; ***A01N 47/14***(2006.01)i; ***A01N 47/18***(2006.01)i; ***A01N 47/22***(2006.01)i; ***A01N 47/24***(2006.01)i; ***A01N 47/26***(2006.01)i; ***A01N 47/40***(2006.01)i; ***A01N 51/00***(2006.01)i; ***A01N 53/06***(2006.01)i; ***A01N 53/08***(2006.01)i; ***A01N 55/10***(2006.01)i; ***A01N 57/14***(2006.01)i; ***A01N 59/16***(2006.01)i; ***A01N 63/20***(2020.01)i; ***A01N 65/12***(2009.01)i; ***A01P 7/00***(2006.01)i; ***A01P 7/02***(2006.01)i; ***A01P 7/04***(2006.01)i; ***A61K 31/437***(2006.01)i; ***A61K 31/4439***(2006.01)i; ***A61K 45/00***(2006.01)i; ***A61P 33/14***(2006.01)i; ***C07D 413/12***(2006.01)i; ***C07D 417/12***(2006.01)i; ***C07D 471/04***(2006.01)i; ***C07D 519/00***(2006.01)i

FI: C07D401/12 CSP; A01N25/00 102; A01N37/24 101; A01N37/32 101; A01N37/38; A01N37/46; A01N37/50; A01N43/32; A01N43/36 A; A01N43/40 101C; A01N43/40 101D; A01N43/40 101E; A01N43/40 101Q; A01N43/50 C; A01N43/50 Q; A01N43/52; A01N43/54 A; A01N43/56 C; A01N43/56 D; A01N43/653 C; A01N43/653 G; A01N43/653 J; A01N43/653 Q; A01N43/713; A01N43/76 101; A01N43/78 A; A01N43/78 C; A01N43/78 D; A01N43/78 101; A01N43/80 101; A01N43/836; A01N43/88; A01N43/90 101; A01N43/90 103; A01N47/02; A01N47/04 101; A01N47/14 C; A01N47/18 101A; A01N47/22 G; A01N47/24 G; A01N47/24 J; A01N47/26; A01N47/40 Z; A01N51/00; A01N53/06 110; A01N53/08 125; A01N55/10 300; A01N57/14 C; A01N59/16 Z; A01N63/20; A01N65/12; A01P7/00; A01P7/02; A01P7/04; A61K31/437; A61K31/4439; A61K45/00; A61P33/14; C07D413/12; C07D417/12 CSP; C07D471/04 108A; C07D519/00 301; C07D519/00 311

According to International Patent Classification (IPC) or to both national classification and IPC

**B. FIELDS SEARCHED**

Minimum documentation searched (classification system followed by classification symbols)

C07D401/12; A01N25/00; A01N37/24; A01N37/32; A01N37/38; A01N37/46; A01N37/50; A01N43/32; A01N43/36; A01N43/40; A01N43/50; A01N43/52; A01N43/54; A01N43/56; A01N43/76; A01N43/78; A01N43/80; A01N43/88; A01N43/90; A01N43/653; A01N43/713; A01N43/836; A01N47/02; A01N47/04; A01N47/14; A01N47/18; A01N47/22; A01N47/24; A01N47/26; A01N47/40; A01N51/00; A01N53/06; A01N53/08; A01N55/10; A01N57/14; A01N59/16; A01N63/20; A01N65/12; A01P7/00; A01P7/02; A01P7/04; A61K31/437; A61K31/4439; A61K45/00; A61P33/14; C07D413/12; C07D417/12; C07D471/04; C07D519/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 August 2024** | **27 August 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/019624**

**C. DOCUMENTS CONSIDERED TO BE RELEVANT**

| Category* | Citation of document, with indication, where appropriate, of the relevant passages | Relevant to claim No. |
|---|---|---|
| A | JP 2022-69685 A (SUMITOMO CHEMICAL COMPANY, LIMITED) 11 May 2022 (2022-05-11)<br>entire text | 1-3, 6, 8-13 |
| A | WO 2019/200310 A1 (HUTCHINSON FRED CANCER RES) 17 October 2019 (2019-10-17)<br>entire text | 1-3, 6, 8-13 |
| A | JP 2011-507910 A (UNIV ROCHESTER) 10 March 2011 (2011-03-10)<br>entire text | 1-3, 6, 8-13 |

Form PCT/ISA/210 (second sheet) (July 2022)

**INTERNATIONAL SEARCH REPORT**

International application No.
**PCT/JP2024/019624**

**Box No. III Observations where unity of invention is lacking (Continuation of item 3 of first sheet)**

This International Searching Authority found multiple inventions in this international application, as follows:

The inventions in claim 1 and claims 2-13 that refer back to said claim have the technical feature of relating to compounds represented by formula (I) set forth in claim 1. However, even compounds of the present application that are not just simply compounds represented by formula (I) but are further limited to those in which Q is a group represented by formulas Q1 or Q5 (see claims 2-5) and Het is a group represented by formulas Het1 or Het2 (see claims 6-7) are set forth in many documents such as document 1 (see paragraphs [0365]-[0371], etc.) and are publicly known. Thus, said technical feature does not make a contribution over the prior art and is not a special technical feature. Moreover, since no other same or corresponding special technical features can be found among the invention in claim 1 and the inventions in claims 1-13, the group of inventions set forth in claims 1-13 are not found to be linked so as to form a single general inventive concept. Thus, the number of inventions set forth in claim 1 and claims 2-13 that refer back to said claim is at least 42 from the combinations of the 7 kinds of Q × the 6 kinds of Het, or more.

Document 1: JP 2022-069685 A

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims: it is covered by claims Nos.: **Portion relating to the compound represented by formula (I), wherein Q is a group represented by the formula Q1 and Het is a group represented by the formula Het1 (part of claims 1-3, 6, 8-13)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/JP2024/019624**

| Patent document cited in search report | Publication date (day/month/year) | Patent family member(s) | Publication date (day/month/year) |
|---|---|---|---|
| JP 2022-69685 A | 11 May 2022 | (Family: none) | |
| WO 2019/200310 A1 | 17 October 2019 | US 2021/0047278 A1 | |
| | | US 2021/0041439 A1 | |
| | | WO 2019/200305 A1 | |
| | | EP 3774766 A1 | |
| JP 2011-507910 A | 10 March 2011 | US 2009/0163545 A1 | |
| | | WO 2009/086303 A2 | |
| | | EP 2219646 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2023088493 A
- JP 2023218891 A
- WO 2013191041 A
- WO 2015117912 A
- WO 2022043576 A1
- WO 2022250069 A1


### Non-patent literature cited in the description


- the FAO/WHO Joint Meeting on Pesticide Specifications. Manual on development and use of FAO and WHO Specifications for pesticides, FAO Plant Production and Protection Papers. 2016, 271-276